(19) Europäisches Patentamt
European Patent Office
Office européen des brevets

(11) EP 2 894 473 B1

# (12) EUROPEAN PATENT SPECIFICATION

(45) Date of publication and mention
of the grant of the patent:
**21.09.2016 Bulletin 2016/38**

(51) Int Cl.:
**G01N 33/53** *(2006.01)*   **G01N 33/68** *(2006.01)*
**G01N 33/92** *(2006.01)*

(21) Application number: **15150059.2**

(22) Date of filing: **27.08.2010**

(54) **Methods and compositions for diagnosis and prognosis of renal injury and renal failure**

Verfahren und Zusammensetzungen zur Diagnose und Prognose von Nierenläsion und Niereninsuffizienz

Procédés et compositions pour le diagnostic et le pronostic de lésion rénale et d'insuffisance rénale

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: 28.08.2009 US 238134 P
28.08.2009 US 238120 P
28.08.2009 US 238129 P
28.08.2009 US 238118 P
28.08.2009 US 238123 P
28.08.2009 US 238127 P
28.08.2009 US 238121 P
28.08.2009 US 238128 P
28.08.2009 US 238125 P
28.08.2009 US 238115 P
18.09.2009 US 244002 P
18.09.2009 US 243993 P
18.09.2009 US 243991 P
18.09.2009 US 243997 P

(43) Date of publication of application:
**15.07.2015 Bulletin 2015/29**

(62) Document number(s) of the earlier application(s) in accordance with Art. 76 EPC:
**10812639.2 / 2 470 905**

(73) Proprietor: **Astute Medical, Inc.**
**San Diego, CA 92121 (US)**

(72) Inventors:
• **Anderberg, Joseph**
**Encinitas, CA 92024 (US)**
• **Gray, Jeff**
**Solana Beach, CA 92075 (US)**
• **McPherson, Paul**
**Encinitas, CA 92024 (US)**
• **Nakamura, Kevin**
**Cardiff by the Sea, CA 92007 (US)**
• **Kampf, James Patrick**
**San Diego, CA 92130 (US)**

(74) Representative: **Schiweck, Weinzierl & Koch**
**European Patent Attorneys**
**Landsberger Straße 98**
**80339 München (DE)**

(56) References cited:
**US-A1- 2006 069 056     US-A1- 2007 248 989**

• **S G COCA ET AL: "Biomarkers for the diagnosis and risk stratification of acute kidney injury: A systematic review", KIDNEY INTERNATIONAL, vol. 73, no. 9, 19 December 2007 (2007-12-19), pages 1008-1016, XP055027810, ISSN: 0085-2538, DOI: 10.1038/sj.ki.5002729**

**Description**

BACKGROUND OF THE INVENTION

The following discussion of the background of the invention is merely provided to aid the reader in understanding the invention and is not admitted to describe or constitute prior art to the present invention.

The kidney is responsible for water and solute excretion from the body. Its functions include maintenance of acid-base balance, regulation of electrolyte concentrations, control of blood volume, and regulation of blood pressure. As such, loss of kidney function through injury and/or disease results in substantial morbidity and mortality. A detailed discussion of renal injuries is provided in Harrison's Principles of Internal Medicine, 17th Ed., McGraw Hill, New York, pages 1741-1830, which are hereby incorporated by reference in their entirety. Renal disease and/or injury may be acute or chronic. Acute and chronic kidney disease are described as follows (from Current Medical Diagnosis & Treatment 2008, 47th Ed, McGraw Hill, New York, pages 785-815, which are hereby incorporated by reference in their entirety): "Acute renal failure is worsening of renal function over hours to days, resulting in the retention of nitrogenous wastes (such as urea nitrogen) and creatinine in the blood. Retention of these substances is called azotemia. Chronic renal failure (chronic kidney disease) results from an abnormal loss of renal function over months to years".

Acute renal failure (ARF, also known as acute kidney injury, or AKI) is an abrupt (typically detected within about 48 hours to 1 week)reduction in glomerular filtration. This loss of filtration capacity results in retention of nitrogenous (urea and creatinine) and non-nitrogenous waste products that are normally excreted by the kidney, a reduction in urine output, or both. It is reported that ARF complicates about 5% of hospital admissions, 4-15% of cardiopulmonary bypass surgeries, and up to 30% of intensive care admissions. ARF may be categorized as prerenal, intrinsic renal, or postrenal in causation. Intrinsic renal disease can be further divided into glomerular, tubular, interstitial, and vascular abnormalities. Major causes of ARF are described in the following table, which is adapted from the Merck Manual, 17th ed., Chapter 222:

| Type | Risk Factors |
|---|---|
| **Prerenal** | |
| ECF volume depletion | Excessive diuresis, hemorrhage, GI losses, loss of intravascular fluid into the extravascular space (due to ascites, peritonitis, pancreatitis, or burns), loss of skin and mucus membranes, renal salt- and water-wasting states |
| Low cardiac output | Cardiomyopathy, MI, cardiac tamponade, pulmonary embolism, pulmonary hypertension, positive-pressure mechanical ventilation |
| Low systemic vascular resistance | Septic shock, liver failure, antihypertensive drugs |
| Increased renal vascular resistance | NSAIDs, cyclosporines, tacrolimus, hypercalcemia, anaphylaxis, anesthetics, renal artery obstruction, renal vein thrombosis, sepsis, hepatorenal syndrome |
| Decreased efferent arteriolar tone (leading to decreased GFR from reduced glomerular transcapillary pressure, especially in patients with bilateral renal artery stenosis) | ACE inhibitors or angiotensin II receptor blockers |
| **Intrinsic Renal** | |
| Acute tubular injury | Ischemia (prolonged or severe prerenal state): surgery, hemorrhage, arterial or venous obstruction; Toxins: NSAIDs, cyclosporines, tacrolimus, aminoglycosides, foscarnet, ethylene glycol, hemoglobin, myoglobin, ifosfamide, heavy metals, methotrexate, radiopaque contrast agents, streptozotocin |
| Acute glomerulonephritis | ANCA-associated: Crescentic glomerulonephritis, polyarteritis nodosa, Wegener's granulomatosis; Anti-GBM glomerulonephritis: Goodpasture's syndrome; Immune-complex: Lupus glomerulonephritis, postinfectious glomerulonephritis, cryoglobulinemic glomerulonephritis |

(continued)

| Intrinsic Renal | |
|---|---|
| Acute tubulo interstitial nephritis | Drug reaction (eg, β-lactams, NSAIDs, sulfonamides, ciprofloxacin, thiazide diuretics, furosemide, phenytoin, allopurinol, pyelonephritis, papillary necrosis |
| Acute vascular nephropathy | Vasculitis, malignant hypertension, thrombotic microangiopathies, scleroderma, atheroembolism |
| Infiltrative diseases | Lymphoma, sarcoidosis, leukemia |
| Postrenal | |
| Tubular precipitation | Uric acid (tumor lysis), sulfonamides, triamterene, acyclovir, indinavir, methotrexate, ethylene glycol ingestion, myeloma protein, myoglobin |
| Ureteral obstruction | Intrinsic: Calculi, clots, sloughed renal tissue, fungus ball, edema, malignancy, congenital defects; Extrinsic: Malignancy, retroperitoneal fibrosis, ureteral trauma during surgery or high impact injury |
| Bladder obstruction | Mechanical: Benign prostatic hyperplasia, prostate cancer, bladder cancer, urethral strictures, phimosis, paraphimosis, urethral valves, obstructed indwelling urinary catheter; Neurogenic: Anticholinergic drugs, upper or lower motor neuron lesion |

In the case of ischemic ARF, the course of the disease may be divided into four phases. During an initiation phase, which lasts hours to days, reduced perfusion of the kidney is evolving into injury. Glomerular ultrafiltration reduces, the flow of filtrate is reduced due to debris within the tubules, and back leakage of filtrate through injured epithelium occurs. Renal injury can be mediated during this phase by reperfusion of the kidney. Initiation is followed by an extension phase which is characterized by continued ischemic injury and inflammation and may involve endothelial damage and vascular congestion. During the maintenance phase, lasting from 1 to 2 weeks, renal cell injury occurs, and glomerular filtration and urine output reaches a minimum. A recovery phase can follow in which the renal epithelium is repaired and GFR gradually recovers. Despite this, the survival rate of subjects with ARF may be as low as about 60%.

Acute kidney injury caused by radiocontrast agents (also called contrast media) and other nephrotoxins such as cyclosporine, antibiotics including aminoglycosides and anticancer drugs such as cisplatin manifests over a period of days to about a week. Contrast induced nephropathy (CIN, which is AKI caused by radiocontrast agents) is thought to be caused by intrarenal vasoconstriction (leading to ischemic injury) and from the generation of reactive oxygen species that are directly toxic to renal tubular epithelial cells. CIN classically presents as an acute (onset within 24-48h) but reversible (peak 3-5 days, resolution within 1 week) rise in blood urea nitrogen and serum creatinine.

A commonly reported criteria for defining and detecting AKI is an abrupt (typically within about 2-7 days or within a period of hospitalization) elevation of serum creatinine. Although the use of serum creatinine elevation to define and detect AKI is well established, the magnitude of the serum creatinine elevation and the time over which it is measured to define AKI varies considerably among publications. Traditionally, relatively large increases in serum creatinine such as 100%, 200%, an increase of at least 100% to a value over 2 mg/dL and other definitions were used to define AKI. However, the recent trend has been towards using smaller serum creatinine rises to define AKI. The relationship between serum creatinine rise, AKI and the associated health risks are reviewed in Praught and Shlipak, Curr Opin Nephrol Hypertens 14:265-270, 2005 and Chertow et al, J Am Soc Nephrol 16: 3365-3370, 2005. As described in these publications, acute worsening renal function (AKI) and increased risk of death and other detrimental outcomes are now known to be associated with very small increases in serum creatinine. These increases may be determined as a relative (percent) value or a nominal value. Relative increases in serum creatinine as small as 20% from the pre-injury value have been reported to indicate acutely worsening renal function (AKI) and increased health risk, but the more commonly reported value to define AKI and increased health risk is a relative increase of at least 25%. Nominal increases as small as 0.3 mg/dL, 0.2 mg/dL or even 0.1 mg/dL have been reported to indicate worsening renal function and increased risk of death. Various time periods for the serum creatinine to rise to these threshold values have been used to define AKI, for example, ranging from 2 days, 3 days, 7 days, or a variable period defined as the time the patient is in the hospital or intensive care unit. These studies indicate there is not a particular threshold serum creatinine rise (or time period for the rise) for worsening renal function or AKI, but rather a continuous increase in risk with increasing magnitude of serum creatinine rise.

One study (Lassnigg et all, J Am Soc Nephrol 15:1597-1605, 2004) investigated both increases and decreases in serum creatinine. Patients with a mild fall in serum creatinine of -0.1 to -0.3 mg/dL following heart surgery had the lowest mortality rate. Patients with a larger fall in serum creatinine (more than or equal to -0.4 mg/dL) or any increase in serum creatinine had a larger mortality rate. These findings caused the authors to conclude that even very subtle changes in renal function (as detected by small creatinine changes within 48 hours of surgery) seriously effect patient's outcomes. In an effort to reach consensus on a unified classification system for using serum creatinine to define AKI in clinical trials and in clinical practice, Bellomo et al., Crit Care. 8(4):R204-12, 2004, proposes the following classifications for stratifying AKI patients:

"Risk": serum creatinine increased 1.5 fold from baseline OR urine production of <0.5 ml/kg body weight/hr for 6 hours;
"Injury": serum creatinine increased 2.0 fold from baseline OR urine production <0.5 ml/kg/hr for 12 h;
"Failure": serum creatinine increased 3.0 fold from baseline OR creatinine >355 $\mu$mol/l (with a rise of >44) or urine output below 0.3 ml/kg/hr for 24 h or anuria for at least 12 hours;

And included two clinical outcomes:

"Loss": persistent need for renal replacement therapy for more than four weeks.
"ESRD": end stage renal disease-the need for dialysis for more than 3 months.

These criteria are called the RIFLE criteria, which provide a useful clinical tool to classify renal status. As discussed in Kellum, Crit. Care Med. 36: S141-45, 2008 and Ricci et al., Kidney Int. 73, 538-546, 2008, the RIFLE criteria provide a uniform definition of AKI which has been validated in numerous studies.

More recently, Mehta et al., Crit. Care 11:R31 (doi:10.1186.cc5713), 2007, proposes the following similar classifications for stratifying AKI patients, which have been modified from RIFLE:

"Stage I": increase in serum creatinine of more than or equal to 0.3 mg/dL ($\geq$ 26.4 $\mu$mol/L) or increase to more than or equal to 150% (1.5-fold) from baseline OR urine output less than 0.5 mL/kg per hour for more than 6 hours;
"Stage II": increase in serum creatinine to more than 200% (> 2-fold) from baseline OR urine output less than 0.5 mL/kg per hour for more than 12 hours;
"Stage III": increase in serum creatinine to more than 300% (> 3-fold) from baseline OR serum creatinine $\geq$ 354 $\mu$mol/L accompanied by an acute increase of at least 44 $\mu$mol/L OR urine output less than 0.3 mL/kg per hour for 24 hours or anuria for 12 hours.

The CIN Consensus Working Panel (McCollough et al, Rev Cardiovasc Med. 2006; 7(4):177-197) uses a serum creatinine rise of 25% to define Contrast induced nephropathy (which is a type of AKI).Although various groups propose slightly different criteria for using serum creatinine to detect AKI, the consensus is that small changes in serum creatinine, such as 0.3 mg/dL or 25%, are sufficient to detect AKI (worsening renal function) and that the magnitude of the serum creatinine change is an indicator of the severity of the AKI and mortality risk.

Although serial measurement of serum creatinine over a period of days is an accepted method of detecting and diagnosing AKI and is considered one of the most important tools to evaluate AKI patients, serum creatinine is generally regarded to have several limitations in the diagnosis, assessment and monitoring of AKI patients. The time period for serum creatinine to rise to values (e.g., a 0.3 mg/dL or 25% rise) considered diagnostic for AKI can be 48 hours or longer depending on the definition used. Since cellular injury in AKI can occur over a period of hours, serum creatinine elevations detected at 48 hours or longer can be a late indicator of injury, and relying on serum creatinine can thus delay diagnosis of AKI. Furthermore, serum creatinine is not a good indicator of the exact kidney status and treatment needs during the most acute phases of AKI when kidney function is changing rapidly. Some patients with AKI will recover fully, some will need dialysis (either short term or long term) and some will have other detrimental outcomes including death, major adverse cardiac events and chronic kidney disease. Because serum creatinine is a marker of filtration rate, it does not differentiate between the causes of AKI (pre-renal, intrinsic renal, post-renal obstruction, atheroembolic, etc) or the category or location of injury in intrinsic renal disease (for example, tubular, glomerular or interstitial in origin). Urine output is similarly limited, Knowing these things can be of vital importance in managing and treating patients with AKI.

These limitations underscore the need for better methods to detect and assess AKI, particularly in the early and subclinical stages, but also in later stages when recovery and repair of the kidney can occur. Furthermore, there is a need to better identify patients who are at risk of having an AKI.

BRIEF SUMMARY OF THE INVENTION

It is an object of the invention to provide methods and compositions for evaluating renal function in a subject. As described herein, measurement of one or more biomarkers selected from the group consisting of Tumor necrosis factor receptor superfamily member 10B, Cadherin-16, Caspase-9, Bc12 antagonist of cell death, Caspase-1, Cadherin-1, Poly [ADP-ribose] polymerase 1, Cyclin-dependent kinase inhibitor 1, Cadherin-5, Myoglobin, Apolipoprotein A-II, Mucin-16, Carcinoembryonic antigen-related cell adhesion molecule 5, and Cellular tumor antigen p53 (referred to herein as a "kidney injury marker") can be used for diagnosis, prognosis, risk stratification, staging, monitoring, categorizing and determination of further diagnosis and treatment regimens in subjects suffering or at risk of suffering from an injury to renal function, reduced renal function, and/or acute renal failure (also called acute kidney injury).

US application US 2007/248989 discloses a method for evaluating renal status in a subject, comprising performing one or more assays configured to detect one or more biomarkers selected from the group consisting of aprotinin, alpha-1-microglobulin, alpha-1-acid-glycoprotein, and micro-albumin on a body fluid sample obtained from the subject to provide an assay result; andc orrelating the assay result(s) to the renal status of the subject.

The kidney injury markers of the present invention may be used, individually or in panels comprising a plurality of kidney injury markers, for risk stratification (that is, to identify subjects at risk for a future injury to renal function, for future progression to reduced renal function, for future progression to ARF, for future improvement in renal function, *etc.*); for diagnosis of existing disease (that is, to identify subjects who have suffered an injury to renal function, who have progressed to reduced renal function, who have progressed to ARF, *etc.*); for monitoring for deterioration or improvement of renal function; and for predicting a future medical outcome, such as improved or worsening renal function, a decreased or increased mortality risk, a decreased or increased risk that a subject will require renal replacement therapy (*i.e.*, hemodialysis, peritoneal dialysis, hemofiltration, and/or renal transplantation, a decreased or increased risk that a subject will recover from an injury to renal function, a decreased or increased risk that a subject will recover from ARF, a decreased or increased risk that a subject will progress to end stage renal disease, a decreased or increased risk that a subject will progress to chronic renal failure, a decreased or increased risk that a subject will suffer rejection of a transplanted kidney, *etc.*

In a first aspect, the present invention relates to methods for evaluating renal status in a subject. These methods comprise performing an assay method that is configured to detect one or more biomarkers selected from the group consisting of Tumor necrosis factor receptor superfamily member 10B, Cadherin-16, Caspase-9, Bc12 antagonist of cell death, Caspase-1, Cadherin-1, Poly [ADP-ribose] polymerase 1, Cyclin-dependent kinase inhibitor 1, Cadherin-5, Myoglobin, Apolipoprotein A-II, Mucin-16, Carcinoembryonic antigen-related cell adhesion molecule 5, and Cellular tumor antigen p53 in a body fluid sample obtained from the subject. The assay result(s), for example measured concentration(s) of one or more biomarkers selected from the group consisting of Tumor necrosis factor receptor superfamily member 10B, Cadherin-16, Caspase-9, Bc12 antagonist of cell death, Caspase-1, Cadherin-1, Poly [ADP-ribose] polymerase 1, Cyclin-dependent kinase inhibitor 1, Cadherin-5, Myoglobin, Apolipoprotein A-II, Mucin-16, is/are then correlated to the renal status of the subject. This correlation to renal status may include correlating the assay result(s) to one or more of risk stratification, diagnosis, prognosis, staging, classifying and monitoring of the subject as described herein. Thus, the present invention utilizes one or more kidney injury markers of the present invention for the evaluation of renal injury.

In certain embodiments, the methods for evaluating renal status described herein are methods for risk stratification of the subject; that is, assigning a likelihood of one or more future changes in renal status to the subject. In these embodiments, the assay result(s) is/are correlated to one or more such future changes. The following are preferred risk stratification embodiments.

In preferred risk stratification embodiments, these methods comprise determining a subject's risk for a future injury to renal function, and the assay result(s) is/are correlated to a likelihood of such a future injury to renal function. For example, the measured concentration(s) may each be compared to a threshold value. For a "positive going" kidney injury marker, an increased likelihood of suffering a future injury to renal function is assigned to the subject when the measured concentration is above the threshold, relative to a likelihood assigned when the measured concentration is below the threshold. For a "negative going" kidney injury marker, an increased likelihood of suffering a future injury to renal function is assigned to the subject when the measured concentration is below the threshold, relative to a likelihood assigned when the measured concentration is above the threshold.

In other preferred risk stratification embodiments, these methods comprise determining a subject's risk for future reduced renal function, and the assay result(s) is/are correlated to a likelihood of such reduced renal function. For example, the measured concentrations may each be compared to a threshold value. For a "positive going" kidney injury marker, an increased likelihood of suffering a future reduced renal function is assigned to the subject when the measured concentration is above the threshold, relative to a likelihood assigned when the measured concentration is below the threshold. For a "negative going" kidney injury marker, an increased likelihood of future reduced renal function is assigned to the subject when the measured concentration is below the threshold, relative to a likelihood assigned when the measured concentration is above the threshold.

In still other preferred risk stratification embodiments, these methods comprise determining a subject's likelihood for a future improvement in renal function, and the assay result(s) is/are correlated to a likelihood of such a future improvement in renal function. For example, the measured concentration(s) may each be compared to a threshold value. For a "positive going" kidney injury marker, an increased likelihood of a future improvement in renal function is assigned to the subject when the measured concentration is below the threshold, relative to a likelihood assigned when the measured concentration is above the threshold. For a "negative going" kidney injury marker, an increased likelihood of a future improvement in renal function is assigned to the subject when the measured concentration is above the threshold, relative to a likelihood assigned when the measured concentration is below the threshold.

In yet other preferred risk stratification embodiments, these methods comprise determining a subject's risk for progression to ARF, and the result(s) is/are correlated to a likelihood of such progression to ARF. For example, the measured concentration(s) may each be compared to a threshold value. For a "positive going" kidney injury marker, an increased likelihood of progression to ARF is assigned to the subject when the measured concentration is above the threshold, relative to a likelihood assigned when the measured concentration is below the threshold. For a "negative going" kidney injury marker, an increased likelihood of progression to ARF is assigned to the subject when the measured concentration is below the threshold, relative to a likelihood assigned when the measured concentration is above the threshold.

And in other preferred risk stratification embodiments, these methods comprise determining a subject's outcome risk, and the assay result(s) is/are correlated to a likelihood of the occurrence of a clinical outcome related to a renal injury suffered by the subject. For example, the measured concentration(s) may each be compared to a threshold value. For a "positive going" kidney injury marker, an increased likelihood of one or more of: acute kidney injury, progression to a worsening stage of AKI, mortality, a requirement for renal replacement therapy, a requirement for withdrawal of renal toxins, end stage renal disease, heart failure, stroke, myocardial infarction, progression to chronic kidney disease, *etc.*, is assigned to the subject when the measured concentration is above the threshold, relative to a likelihood assigned when the measured concentration is below the threshold. For a "negative going" kidney injury marker, an increased likelihood of one or more of: acute kidney injury, progression to a worsening stage of AKI, mortality, a requirement for renal replacement therapy, a requirement for withdrawal of renal toxins, end stage renal disease, heart failure, stroke, myocardial infarction, progression to chronic kidney disease, *etc.*, is assigned to the subject when the measured concentration is below the threshold, relative to a likelihood assigned when the measured concentration is above the threshold.

In such risk stratification embodiments, preferably the likelihood or risk assigned is that an event of interest is more or less likely to occur within 180 days of the time at which the body fluid sample is obtained from the subject. In particularly preferred embodiments, the likelihood or risk assigned relates to an event of interest occurring within a shorter time period such as 18 months, 120 days, 90 days, 60 days, 45 days, 30 days, 21 days, 14 days, 7 days, 5 days, 96 hours, 72 hours, 48 hours, 36 hours, 24 hours, 12 hours, or less. A risk at 0 hours of the time at which the body fluid sample is obtained from the subject is equivalent to diagnosis of a current condition.

In preferred risk stratification embodiments, the subject is selected for risk stratification based on the pre-existence in the subject of one or more known risk factors for prerenal, intrinsic renal, or postrenal ARF. For example, a subject undergoing or having undergone major vascular surgery, coronary artery bypass, or other cardiac surgery; a subject having pre-existing congestive heart failure, preeclampsia, eclampsia, diabetes mellitus, hypertension, coronary artery disease, proteinuria, renal insufficiency, glomerular filtration below the normal range, cirrhosis, serum creatinine above the normal range, or sepsis; or a subject exposed to NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin are all preferred subjects for monitoring risks according to the methods described herein. This list is not meant to be limiting. By "pre-existence" in this context is meant that the risk factor exists at the time the body fluid sample is obtained from the subject. In particularly preferred embodiments, a subject is chosen for risk stratification based on an existing diagnosis of injury to renal function, reduced renal function, or ARF.

In other embodiments, the methods for evaluating renal status described herein are methods for diagnosing a renal injury in the subject; that is, assessing whether or not a subject has suffered from an injury to renal function, reduced renal function, or ARF. In these embodiments, the assay result(s), for example measured concentration(s) of one or more biomarkers selected from the group consisting of Tumor necrosis factor receptor superfamily member 10B, Cadherin-16, Caspase-9, Bc12 antagonist of cell death, Caspase-1, Cadherin-1, Poly [ADP-ribose] polymerase 1, Cyclin-dependent kinase inhibitor 1, Cadherin-5, Myoglobin, Apolipoprotein A-II, Mucin-16, is/are correlated to the occurrence or nonoccurrence of a change in renal status. The following are preferred diagnostic embodiments.

In preferred diagnostic embodiments, these methods comprise diagnosing the occurrence or nonoccurrence of an injury to renal function, and the assay result(s) is/are correlated to the occurrence or nonoccurrence of such an injury. For example, each of the measured concentration(s) may be compared to a threshold value. For a positive going marker, an increased likelihood of the occurrence of an injury to renal function is assigned to the subject when the measured concentration is above the threshold (relative to the likelihood assigned when the measured concentration is below the threshold); alternatively, when the measured concentration is below the threshold, an increased likelihood of the nonoccurrence of an injury to renal function may be assigned to the subject (relative to the likelihood assigned when the measured concentration is above the threshold). For a negative going marker, an increased likelihood of the occurrence of an injury to renal function is assigned to the subject when the measured concentration is below the threshold (relative to the likelihood assigned when the measured concentration is above the threshold); alternatively, when the measured concentration is above the threshold, an increased likelihood of the nonoccurrence of an injury to renal function may be assigned to the subject (relative to the likelihood assigned when the measured concentration is below the threshold).

In other preferred diagnostic embodiments, these methods comprise diagnosing the occurrence or nonoccurrence of reduced renal function, and the assay result(s) is/are correlated to the occurrence or nonoccurrence of an injury causing reduced renal function. For example, each of the measured concentration(s) may be compared to a threshold value. For a positive going marker, an increased likelihood of the occurrence of an injury causing reduced renal function is assigned to the subject when the measured concentration is above the threshold (relative to the likelihood assigned when the measured concentration is below the threshold); alternatively, when the measured concentration is below the threshold, an increased likelihood of the nonoccurrence of an injury causing reduced renal function may be assigned to the subject (relative to the likelihood assigned when the measured concentration is above the threshold). For a negative going marker, an increased likelihood of the occurrence of an injury causing reduced renal function is assigned to the subject when the measured concentration is below the threshold (relative to the likelihood assigned when the measured concentration is above the threshold); alternatively, when the measured concentration is above the threshold, an increased likelihood of the nonoccurrence of an injury causing reduced renal function may be assigned to the subject (relative to the likelihood assigned when the measured concentration is below the threshold).

In yet other preferred diagnostic embodiments, these methods comprise diagnosing the occurrence or nonoccurrence of ARF, and the assay result(s) is/are correlated to the occurrence or nonoccurrence of an injury causing ARF. For example, each of the measured concentration(s) may be compared to a threshold value. For a positive going marker, an increased likelihood of the occurrence of ARF is assigned to the subject when the measured concentration is above the threshold (relative to the likelihood assigned when the measured concentration is below the threshold); alternatively, when the measured concentration is below the threshold, an increased likelihood of the nonoccurrence of ARF may be assigned to the subject (relative to the likelihood assigned when the measured concentration is above the threshold). For a negative going marker, an increased likelihood of the occurrence of ARF is assigned to the subject when the measured concentration is below the threshold (relative to the likelihood assigned when the measured concentration is above the threshold); alternatively, when the measured concentration is above the threshold, an increased likelihood of the nonoccurrence of ARF may be assigned to the subject (relative to the likelihood assigned when the measured concentration is below the threshold).

In still other preferred diagnostic embodiments, these methods comprise diagnosing a subject as being in need of renal replacement therapy, and the assay result(s) is/are correlated to a need for renal replacement therapy. For example, each of the measured concentration(s) may be compared to a threshold value. For a positive going marker, an increased likelihood of the occurrence of an injury creating a need for renal replacement therapy is assigned to the subject when the measured concentration is above the threshold (relative to the likelihood assigned when the measured concentration is below the threshold); alternatively, when the measured concentration is below the threshold, an increased likelihood of the nonoccurrence of an injury creating a need for renal replacement therapy may be assigned to the subject (relative to the likelihood assigned when the measured concentration is above the threshold). For a negative going marker, an increased likelihood of the occurrence of an injury creating a need for renal replacement therapy is assigned to the subject when the measured concentration is below the threshold (relative to the likelihood assigned when the measured concentration is above the threshold); alternatively, when the measured concentration is above the threshold, an increased likelihood of the nonoccurrence of an injury creating a need for renal replacement therapy may be assigned to the subject (relative to the likelihood assigned when the measured concentration is below the threshold).

In still other preferred diagnostic embodiments, these methods comprise diagnosing a subject as being in need of renal transplantation, and the assay result(s0 is/are correlated to a need for renal transplantation. For example, each of the measured concentration(s) may be compared to a threshold value. For a positive going marker, an increased likelihood of the occurrence of an injury creating a need for renal transplantation is assigned to the subject when the measured concentration is above the threshold (relative to the likelihood assigned when the measured concentration is below the threshold); alternatively, when the measured concentration is below the threshold, an increased likelihood of the nonoccurrence of an injury creating a need for renal transplantation may be assigned to the subject (relative to the likelihood assigned when the measured concentration is above the threshold). For a negative going marker, an increased likelihood of the occurrence of an injury creating a need for renal transplantation is assigned to the subject when the measured concentration is below the threshold (relative to the likelihood assigned when the measured concentration is above the threshold); alternatively, when the measured concentration is above the threshold, an increased likelihood of the nonoccurrence of an injury creating a need for renal transplantation may be assigned to the subject (relative to the

EP 2 894 473 B1

likelihood assigned when the measured concentration is below the threshold).

In still other embodiments, the methods for evaluating renal status described herein are methods for monitoring a renal injury in the subject; that is, assessing whether or not renal function is improving or worsening in a subject who has suffered from an injury to renal function, reduced renal function, or ARF. In these embodiments, the assay result(s), for example measured concentration(s) of one or more biomarkers selected from the group consisting of Tumor necrosis factor receptor superfamily member 10B, Cadherin-16, Caspase-9, Bc12 antagonist of cell death, Caspase-1, Cadherin-1, Poly [ADP-ribose] polymerase 1, Cyclin-dependent kinase inhibitor 1, Cadherin-5, Myoglobin, Apolipoprotein A-II, Mucin-16, is/are correlated to the occurrence or nonoccurrence of a change in renal status. The following are preferred monitoring embodiments.

In preferred monitoring embodiments, these methods comprise monitoring renal status in a subject suffering from an injury to renal function, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In other preferred monitoring embodiments, these methods comprise monitoring renal status in a subject suffering from reduced renal function, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In yet other preferred monitoring embodiments, these methods comprise monitoring renal status in a subject suffering from acute renal failure, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In other additional preferred monitoring embodiments, these methods comprise monitoring renal status in a subject at risk of an injury to renal function due to the pre-existence of one or more known risk factors for prerenal, intrinsic renal, or postrenal ARF, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In still other embodiments, the methods for evaluating renal status described herein are methods for classifying a renal injury in the subject; that is, determining whether a renal injury in a subject is prerenal, intrinsic renal, or postrenal; and/or further subdividing these classes into subclasses such as acute tubular injury, acute glomerulonephritis acute tubulointerstitial nephritis, acute vascular nephropathy, or infiltrative disease; and/or assigning a likelihood that a subject will progress to a particular RIFLE stage. In these embodiments, the assay result(s), for example measured concentration(s) of one or more biomarkers selected from the group consisting of Tumor necrosis factor receptor superfamily member 10B, Cadherin-16, Caspase-9, Bc12 antagonist of cell death, Caspase-1, Cadherin-1, Poly [ADP-ribose] polymerase 1, Cyclin-dependent kinase inhibitor 1, Cadherin-5, Myoglobin, Apolipoprotein A-II, Mucin-16, is/are correlated to a particular class and/or subclass. The following are preferred classification embodiments.

In preferred classification embodiments, these methods comprise determining whether a renal injury in a subject is prerenal, intrinsic renal, or postrenal; and/or further subdividing these classes into subclasses such as acute tubular injury, acute glomerulonephritis acute tubulo interstitial nephritis, acute vascular nephropathy, or infiltrative disease; and/or assigning a likelihood that a subject will progress to a particular RIFLE stage, and the assay result(s) is/are correlated to the injury classification for the subject. For example, the measured concentration may be compared to a threshold value, and when the measured concentration is above the threshold, a particular classification is assigned; alternatively, when the measured concentration is below the threshold, a different classification may be assigned to the subject.

A variety of methods may be used by the skilled artisan to arrive at a desired threshold value for use in these methods. For example, the threshold value may be determined from a population of normal subjects by selecting a concentration representing the 75th, 85th, 90th, 95th, or 99th percentile of a kidney injury marker measured in such normal subjects. Alternatively, the threshold value may be determined from a "diseased" population of subjects, e.g., those suffering from an injury or having a predisposition for an injury (e.g., progression to ARF or some other clinical outcome such as death, dialysis, renal transplantation, etc.), by selecting a concentration representing the 75th, 85th, 90th, 95th, or 99th percentile of a kidney injury marker measured in such subjects. In another alternative, the threshold value may be determined from a prior measurement of a kidney injury marker in the same subject; that is, a temporal change in the level of a kidney injury marker in the subject may be used to assign risk to the subject.

The foregoing discussion is not meant to imply, however, that the kidney injury markers of the present invention must be compared to corresponding individual thresholds. Methods for combining assay results can comprise the use of multivariate logistical regression, loglinear modeling, neural network analysis, n-of-m analysis, decision tree analysis, calculating ratios of markers, etc. This list is not meant to be limiting. In these methods, a composite result which is determined by combining individual markers may be treated as if it is itself a marker; that is, a threshold may be determined for the composite result as described herein for individual markers, and the composite result for an individual patient compared to this threshold.

The ability of a particular test to distinguish two populations can be established using ROC analysis. For example, ROC curves established from a "first" subpopulation which is predisposed to one or more future changes in renal status, and a "second" subpopulation which is not so predisposed can be used to calculate a ROC curve, and the area under the curve provides a measure of the quality of the test. Preferably, the tests described herein provide a ROC curve area greater than 0.5, preferably at least 0.6, more preferably 0.7, still more preferably at least 0.8, even more preferably at least 0.9, and most preferably at least 0.95.

In certain aspects, the measured concentration of one or more kidney injury markers, or a composite of such markers, may be treated as continuous variables. For example, any particular concentration can be converted into a corresponding probability of a future reduction in renal function for the subject, the occurrence of an injury, a classification, etc. In yet another alternative, a threshold that can provide an acceptable level of specificity and sensitivity in separating a population of subjects into "bins" such as a "first" subpopulation (e.g., which is predisposed to one or more future changes in renal status, the occurrence of an injury, a classification, etc.) and a "second" subpopulation which is not so predisposed. A threshold value is selected to separate this first and second population by one or more of the following measures of test accuracy:

an odds ratio greater than 1, preferably at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less;

a specificity of greater than 0.5, preferably at least about 0.6, more preferably at least about 0.7, still more preferably at least about 0.8, even more preferably at least about 0.9 and most preferably at least about 0.95, with a corresponding sensitivity greater than 0.2, preferably greater than about 0.3, more preferably greater than about 0.4, still more preferably at least about 0.5, even more preferably about 0.6, yet more preferably greater than about 0.7, still more preferably greater than about 0.8, more preferably greater than about 0.9, and most preferably greater than about 0.95;

a sensitivity of greater than 0.5, preferably at least about 0.6, more preferably at least about 0.7, still more preferably at least about 0.8, even more preferably at least about 0.9 and most preferably at least about 0.95, with a corresponding specificity greater than 0.2, preferably greater than about 0.3, more preferably greater than about 0.4, still more preferably at least about 0.5, even more preferably about 0.6, yet more preferably greater than about 0.7, still more preferably greater than about 0.8, more preferably greater than about 0.9, and most preferably greater than about 0.95;

at least about 75% sensitivity, combined with at least about 75% specificity;

a positive likelihood ratio (calculated as sensitivity/(1-specificity)) of greater than 1, at least about 2, more preferably at least about 3, still more preferably at least about 5, and most preferably at least about 10; or

a negative likelihood ratio (calculated as (1-sensitivity)/specificity) of less than 1, less than or equal to about 0.5, more preferably less than or equal to about 0.3, and most preferably less than or equal to about 0.1.

The term "about" in the context of any of the above measurements refers to +/-5% of a given measurement.

Multiple thresholds may also be used to assess renal status in a subject. For example, a "first" subpopulation which is predisposed to one or more future changes in renal status, the occurrence of an injury, a classification, etc., and a "second" subpopulation which is not so predisposed can be combined into a single group. This group is then subdivided into three or more equal parts (known as tertiles, quartiles, quintiles, etc., depending on the number of subdivisions). An odds ratio is assigned to subjects based on which subdivision they fall into. If one considers a tertile, the lowest or highest tertile can be used as a reference for comparison of the other subdivisions. This reference subdivision is assigned an odds ratio of 1. The second tertile is assigned an odds ratio that is relative to that first tertile. That is, someone in the second tertile might be 3 times more likely to suffer one or more future changes in renal status in comparison to someone in the first tertile. The third tertile is also assigned an odds ratio that is relative to that first tertile.

In certain embodiments, the assay method is an immunoassay. Antibodies for use in such assays will specifically bind a full length kidney injury marker of interest, and may also bind one or more polypeptides that are "related" thereto, as that term is defined hereinafter. Numerous immunoassay formats are known to those of skill in the art. Preferred body fluid samples are selected from the group consisting of urine, blood, serum, saliva, tears, and plasma.

The foregoing method steps should not be interpreted to mean that the kidney injury marker assay result(s) is/are used in isolation in the methods described herein. Rather, additional variables or other clinical indicia may be included in the methods described herein. For example, a risk stratification, diagnostic, classification, monitoring, etc. method may combine the assay result(s) with one or more variables measured for the subject selected from the group consisting of demographic information (e.g., weight, sex, age, race), medical history (e.g., family history, type of surgery, pre-existing disease such as aneurism, congestive heart failure, preeclampsia, eclampsia, diabetes mellitus, hypertension, coronary artery disease, proteinuria, renal insufficiency, or sepsis, type of toxin exposure such as NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin), clinical variables (e.g., blood pressure, temperature, respiration rate), risk scores (APACHE score, PREDICT score, TIMI Risk Score for UA/NSTEMI, Framingham Risk Score), a glomerular filtration rate, an estimated glomerular filtration rate, a urine production rate, a serum or plasma creatinine concentration, a urine creatinine concentration, a fractional excretion of sodium, a urine sodium concentration, a urine creatinine to serum or plasma creatinine ratio, a urine specific gravity, a urine osmolality, a urine urea nitrogen to plasma urea nitrogen ratio, a plasma BUN to creatnine ratio, a renal failure index calculated as urine sodium / (urine creatinine / plasma creatinine), a serum or plasma neutrophil gelatinase (NGAL) concentration, a urine NGAL concentration, a serum or plasma cystatin C concentration, a serum or plasma cardiac troponin concentration, a serum or plasma BNP concentration, a serum or plasma NTproBNP concentration, and a serum or plasma proBNP concentration. Other measures of renal function which may be combined with one or more kidney injury marker assay result(s) are described hereinafter and in Harrison's Principles of Internal Medicine, 17th Ed., McGraw Hill, New York, pages 1741-1830, and Current Medical Diagnosis & Treatment 2008, 47th Ed, McGraw Hill, New York, pages 785- 815.

When more than one marker is measured, the individual markers may be measured in samples obtained at the same time, or may be determined from samples obtained at different (e.g., an earlier or later) times. The individual markers may also be measured on the same or different body fluid samples. For example, one kidney injury marker may be measured in a serum or plasma sample and another kidney injury marker may be measured in a urine sample. In addition, assignment of a likelihood may combine an individual kidney injury marker assay result with temporal changes in one or more additional variables.

In various related aspects, the present invention also relates to devices and kits for performing the methods described herein. Suitable kits comprise reagents sufficient for performing an assay for at least one of the described kidney injury markers, together with instructions for performing the described threshold comparisons.

In certain embodiments, reagents for performing such assays are provided in an assay device, and such assay devices may be included in such a kit. Preferred reagents can comprise one or more solid phase antibodies, the solid phase antibody comprising antibody that detects the intended biomarker target(s) bound to a solid support. In the case of sandwich immunoassays, such reagents can also include one or more detectably labeled antibodies, the detectably labeled antibody comprising antibody that detects the intended biomarker target(s) bound to a detectable label. Additional optional elements that may be provided as part of an assay device are described hereinafter.

Detectable labels may include molecules that are themselves detectable (e.g., fluorescent moieties, electrochemical labels, ecl (electrochemical luminescence) labels, metal chelates, colloidal metal particles, *etc.*) as well as molecules that may be indirectly detected by production of a detectable reaction product (*e.g.*, enzymes such as horseradish peroxidase, alkaline phosphatase, *etc.*) or through the use of a specific binding molecule which itself may be detectable (*e.g.*, a labeled antibody that binds to the second antibody, biotin, digoxigenin, maltose, oligohistidine, 2,4-dintrobenzene, phenylarsenate, ssDNA, dsDNA, etc.).

Generation of a signal from the signal development element can be performed using various optical, acoustical, and electrochemical methods well known in the art. Examples of detection modes include fluorescence, radiochemical detection, reflectance, absorbance, amperometry, conductance, impedance, interferometry, ellipsometry, *etc.* In certain of these methods, the solid phase antibody is coupled to a transducer (*e.g.*, a diffraction grating, electrochemical sensor, etc) for generation of a signal, while in others, a signal is generated by a transducer that is spatially separate from the solid phase antibody (*e.g.*, a fluorometer that employs an excitation light source and an optical detector). This list is not meant to be limiting. Antibody-based biosensors may also be employed to determine the presence or amount of analytes that optionally eliminate the need for a labeled molecule.

DETAILED DESCRIPTION OF THE INVENTION

The present invention relates to methods and compositions for diagnosis, differential diagnosis, risk stratification, monitoring, classifying and determination of treatment regimens in subjects suffering or at risk of suffering from injury to renal function, reduced renal function and/or acute renal failure through measurement of one or more kidney injury markers. In various embodiments, a measured concentration of one or more biomarkers selected from the group consisting of Tumor necrosis factor receptor superfamily member 10B, Cadherin-16, Caspase-9, Bcl2 antagonist of cell death, Caspase-1, Cadherin-1, Poly [ADP-ribose] polymerase 1, Cyclin-dependent kinase inhibitor 1, Cadherin-5, Myoglobin, Apolipoprotein A-II, Mucin-16, or one or more markers related thereto, are correlated to the renal status of the subject.

For purposes of this document, the following definitions apply:

As used herein, an "injury to renal function" is an abrupt (within 14 days, preferably within 7 days, more preferably within 72 hours, and still more preferably within 48 hours) measurable reduction in a measure of renal function. Such an injury may be identified, for example, by a decrease in glomerular filtration rate or estimated GFR, a reduction in urine output, an increase in serum creatinine, an increase in serum cystatin C, a requirement for renal replacement therapy, *etc.* "Improvement in Renal Function" is an abrupt (within 14 days, preferably within 7 days, more preferably within 72 hours, and still more preferably within 48 hours) measurable increase in a measure of renal function. Preferred methods for measuring and/or estimating GFR are described hereinafter.

As used herein, "reduced renal function" is an abrupt (within 14 days, preferably within 7 days, more preferably within 72 hours, and still more preferably within 48 hours) reduction in kidney function identified by an absolute increase in serum creatinine of greater than or equal to 0.1 mg/dL ($\geq$ 8.8 $\mu$mol/L), a percentage increase in serum creatinine of greater than or equal to 20% (1.2-fold from baseline), or a reduction in urine output (documented oliguria of less than 0. 5 ml/kg per hour).

As used herein, "acute renal failure" or "ARF" is an abrupt (within 14 days, preferably within 7 days, more preferably within 72 hours, and still more preferably within 48 hours) reduction in kidney function identified by an absolute increase in serum creatinine of greater than or equal to 0.3 mg/dl ($\geq$ 26.4 $\mu$mol/l), a percentage increase in serum creatinine of greater than or equal to 50% (1. 5-fold from baseline), or a reduction in urine output (documented oliguria of less than 0.5 ml/kg per hour for at least 6 hours). This term is synonymous with "acute kidney injury" or "AKI."

As used herein, the term "tumor necrosis factor receptor superfamily member 10B" refers to one or more polypeptides present in a biological sample that are derived from the tumor necrosis factor receptor superfamily member 10B precursor (Swiss-Prot 014763 (SEQ ID NO: 1)).

```
                10          20          30          40          50          60

    MEQRGQNAPA  ASGARKRHGP  GPREARGARP  GPRVPKTLVL  VVAAVLLLVS  AESALITQQD

                70          80          90         100         110         120

    LAPQQRAAPQ  QKRSSPSEGL  CPPGHHISED  GRDCISCKYG  QDYSTHWNDL  LFCLRCTRCD

               130         140         150         160         170         180

    SGEVELSPCT  TTRNTVCQCE  EGTFREEDSP  EMCRKCRTGC  PRGMVKVGDC  TPWSDIECVH

               190         200         210         220         230         240

    KESGTKHSGE  APAVEETVTS  SPGTPASPCS  LSGIIIGVTV  AAVVLIVAVF  VCKSLLWKKV

               250         260         270         280         290         300

    LPYLKGICSG  GGGDPERVDR  SSQRPGAEDN  VLNEIVSILQ  PTQVPEQEME  VQEPAEPTGV

               310         320         330         340         350         360

    NMLSPGESEH  LLEPAEAERS  QRRRLLVPAN  EGDPTETLRQ  CFDDFADLVP  FDSWEPLMRK

               370         380         390         400         410         420

    LGLMDNEIKV  AKAEAAGHRD  TLYTMLIKWV  NKTGRDASVH  TLLDALETLG  ERLAKQKIED

               430         440

    HLLSSGKFMY  LEGNADSAMS
```

Most preferably, the tumor necrosis factor receptor superfamily member 10B assay detects one or more soluble forms of tumor necrosis factor receptor superfamily member 10B. Tumor necrosis factor receptor superfamily member 10B is a single-pass type I membrane protein having a large extracellular domain, most or all of which is present in soluble forms of tumor necrosis factor receptor superfamily member 10B generated either through alternative splicing event which deletes all or a portion of the transmembrane domain, or by proteolysis of the membrane-bound form. In the case of an immunoassay, one or more antibodies that bind to epitopes within this extracellular domain may be used to detect these soluble form(s). The following domains have been identified in tumor necrosis factor receptor superfamily member 10B:

| Residues | Length | Domain ID |
|---|---|---|
| 1-55 | 55 | signal sequence |
| 56-440 | 385 | tumor necrosis factor receptor superfamily member 10B |
| 56-210 | 155 | extracellular |
| 211-231 | 21 | transmembrane |
| 232-440 | 209 | cytoplasmic |

As used herein, the term "cadherin-16" refers to one or more polypeptides present in a biological sample that are derived from the cadherin-16 precursor (Swiss-Prot 075309 (SEQ ID NO: 2)).

|  | 10 | 20 | 30 | 40 | 50 | 60 |
|---|---|---|---|---|---|---|
| | MVPAWLWLLC | VSVPQALPKA | QPAELSVEVP | ENYGGNFPLY | LTKLPLPREG | AEGQIVLSGD |

|  | 70 | 80 | 90 | 100 | 110 | 120 |
|---|---|---|---|---|---|---|
| | SGKATEGPFA | MDPDSGFLLV | TRALDREEQA | EYQLQVTLEM | QDGHVLWGPQ | PVLVHVKDEN |

| 130 | 140 | 150 | 160 | 170 | 180 |
|---|---|---|---|---|---|
| DQVPHFSQAI | YRARLSRGTR | PGIPFLFLEA | SDRDEPGTAN | SDLRFHILSQ | APAQPSPDMF |

| 190 | 200 | 210 | 220 | 230 | 240 |
|---|---|---|---|---|---|
| QLEPRLGALA | LSPKGSTSLD | HALERTYQLL | VQVKDMGDQA | SGHQATATVE | VSIIESTWVS |

| 250 | 260 | 270 | 280 | 290 | 300 |
|---|---|---|---|---|---|
| LEPIHLAENL | KVLYPHHMAQ | VHWSGGDVHY | HLESHPPGPF | EVNAEGNLYV | TRELDREAQA |

| 310 | 320 | 330 | 340 | 350 | 360 |
|---|---|---|---|---|---|
| EYLLQVRAQN | SHGEDYAAPL | ELHVLVMDEN | DNVPICPPRD | PTVSIPELSP | PGTEVTRLSA |

| 370 | 380 | 390 | 400 | 410 | 420 |
|---|---|---|---|---|---|
| EDADAPGSPN | SHVVYQLLSP | EPEDGVEGRA | FQVDPTSGSV | TLGVLPLRAG | QNILLLVLAM |

| 430 | 440 | 450 | 460 | 470 | 480 |
|---|---|---|---|---|---|
| DLAGAEGGFS | STCEVEVAVT | DINDHAPEFI | TSQIGPISLP | EDVEPGTLVA | MLTAIDADLE |

| 490 | 500 | 510 | 520 | 530 | 540 |
|---|---|---|---|---|---|
| PAFRLMDFAI | ERGDTEGTFG | LDWEPDSGHV | RLRLCKNLSY | EAAPSHEVVV | VVQSVAKLVG |

| 550 | 560 | 570 | 580 | 590 | 600 |
|---|---|---|---|---|---|
| PGPGPGATAT | VTVLVERVMP | PPKLDQESYE | ASVPISAPAG | SFLLTIQPSD | PISRTLRFSL |

| 610 | 620 | 630 | 640 | 650 | 660 |
|---|---|---|---|---|---|
| VNDSEGWLCI | EKFSGEVHTA | QSLQGAQPGD | TYTVLVEAQD | TDEPRLSASA | PLVIHFLKAP |

| 670 | 680 | 690 | 700 | 710 | 720 |
|---|---|---|---|---|---|
| PAPALTLAPV | PSQYLCTPRQ | DHGLIVSGPS | KDPDLASGHG | PYSFTLGPNP | TVQRDWRLQT |

| 730 | 740 | 750 | 760 | 770 | 780 |
|---|---|---|---|---|---|
| LNGSHAYLTL | ALHWVEPREH | IIPVVSHNA | QMWQLLVRVI | VCRCNVEGQC | MRKVGRMKGM |

| 790 | 800 | 810 | 820 |  |  |
|---|---|---|---|---|---|
| PTKLSAVGIL | VGTLVAIGIF | LILIFTHWTM | SRKKDPDQPA | DSVPLKATV | |

Most preferably, the cadherin-16 assay detects one or more soluble forms of cadherin-16. Cadherin-16 is a single-pass type I membrane protein having a large extracellular domain, most or all of which is present in soluble forms of cadherin-16 generated either through alternative splicing event which deletes all or a portion of the transmembrane domain, or by proteolysis of the membrane-bound form. In the case of an immunoassay, one or more antibodies that bind to epitopes within this extracellular domain may be used to detect these soluble form(s). The following domains have been identified in cadherin-16:

| Residues | Length | Domain ID |
|---|---|---|
| 1-18 | 18 | signal sequence |
| 19 | 829 | cadherin-16 |
| 19-786 | 768 | extracellular |
| 787-807 | 22 | transmembrane |
| 808-829 | 22 | cytoplasmic |

As used herein, the term "caspase-9" refers to one or more polypeptides present in a biological sample that are derived from the caspase-9 precursor (Swiss-Prot P55211 (SEQ ID NO: 3)).

```
          10         20         30         40         50         60
MDEADRRLLR RCRLRLVEEL QVDQLWDALL SRELFRPHMI EDIQRAGSGS RRDQARQLII

          70         80         90        100        110        120
DLETRGSQAL PLFISCLEDT GQDMLASFLR TNRQAAKLSK PTLENLTPVV LRPEIRKPEV

         130        140        150        160        170        180
LRPETPRPVD IGSGGFGDVG ALESLRGNAD LAYILSMEPC GHCLIINNVN FCRESGLRTR

         190        200        210        220        230        240
TGSNIDCEKL RRRFSSLHFM VEVKGDLTAK KMVLALLELA QQDHGALDCC VVVILSHGCQ

         250        260        270        280        290        300
ASHLQFPGAV YGTDGCPVSV EKIVNIFNGT SCPSLGGKPK LFFIQACGGE QKDHGFEVAS

         310        320        330        340        350        360
TSPEDESPGS NPEPDATPFQ EGLRTFDQLD AISSLPTPSD IFVSYSTFPG FVSWRDPKSG

         370        380        390        400        410
SWYVETLDDI FEQWAHSEDL QSLLLRVANA VSVKGIYKQM PGCFNFLRKK LFFKTS
```

The following domains have been identified in caspase-9:

| Residues | Length | Domain ID |
|---|---|---|
| 1-315 | 315 | caspase-9 p35 subunit |
| 316-330 | 15 | pro-peptide |
| 331-416 | 86 | caspase-9 subunit p10 |

As used herein, the term "Bcl2 antagonist of cell death" refers to one or more polypeptides present in a biological sample that are derived from the Bc12 antagonist of cell death precursor (Swiss-Prot Q92934 (SEQ ID NO: 4)).

```
          10         20         30         40         50         60

MFQIPEFEPS EQEDSSSAER GLGPSPAGDG PSGSGKHHRQ APGLLWDASH QQEQPTSSSH

          70         80         90        100        110        120

HGGAGAVEIR SRHSSYPAGT EDDEGMGEEP SPFRGRSRSA PPNLWAAQRY GRELRRMSDE

         130        140        150        160

FVDSFKKGLP RPKSAGTATQ MRQSSSWTRV FQSWWDRNLG RGSSAPSQ
```

As used herein, the term "caspase-1" refers to one or more polypeptides present in a biological sample that are derived from the caspase-1 precursor (Swiss-Prot P29466 (SEQ ID NO: 5)).

```
          10         20         30         40         50         60

MADKVLKEKR KLFIRSMGEG TINGLLDELL QTRVLNKEEM EKVKRENATV MDKTRALIDS

          70         80         90        100        110        120

VIPKGAQACQ ICITYICEED SYLAGTLGLS ADQTSGNYLN MQDSQGVLSS FPAPQAVQDN

         130        140        150        160        170        180

PAMPTSSGSE GNVKLCSLEE AQRIWKQKSA EIYPIMDKSS RTRLALIICN EEFDSIPRRT

         190        200        210        220        230        240

GAEVDITGMT MLLQNLGYSV DVKKNLTASD MTTELEAFAH RPEHKTSDST FLVFMSHGIR

         250        260        270        280        290        300

EGICGKKHSE QVPDILQLNA IFNMLNTKNC PSLKDKPKVI IIQACRGDSP GVVWFKDSVG

         310        320        330        340        350        360

VSGNLSLPTT EEFEDDAIKK AHIEKDFIAF CSSTPDNVSW RHPTMGSVFI GRLIEHMQEY

         370        380        390        400

ACSCDVEEIF RKVRFSFEQP DGRAQMPTTE RVTLTRCFYL FPGH
```

The following domains have been identified in caspase-1:

| Residues | Length | Domain ID |
|---|---|---|
| 1-119 | 119 | pro-peptide |
| 120-297 | 178 | caspase-1 p20 subunit |
| 298-316 | 19 | pro-peptide |
| 317-404 | 88 | caspase-1 p10 subunit |

As used herein, the terms "epithelial cadherin" or "Cadherin-1" refers to one or more polypeptides present in a biological sample that are derived from the epithelial cadherin precursor (Swiss-Prot P12830 (SEQ ID NO: 6)).

```
                10              20              30              40              50              60

  MGPWSRSLSA  LLLLLQVSSW  LCQEPEPCHP  GFDAESYTFT  VPRRHLERGR  VLGRVNFEDC

                70              80              90             100             110             120

  TGRQRTAYFS  LDTRFKVGTD  GVITVKRPLR  FHNPQIHFLV  YAWDSTYRKF  STKVTLNTVG

               130             140             150             160             170             180

  HHHRPPPHQA  SVSGIQAELL  TFPNSSPGLR  RQKRDWVIPP  ISCPENEKGP  FPKNLVQIKS

               190             200             210             220             230             240

  NKDKEGKVFY  SITGQGADTP  PVGVFIIERE  TGWLKVTEPL  DRERIATYTL  FSHAVSSNGN

               250             260             270             280             290             300

  AVEDPMEILI  TVTDQNDNKP  EFTQEVFKGS  VMEGALPGTS  VMEVTATDAD  DDVNTYNAAI

               310             320             330             340             350             360

  AYTILSQDPE  LPDKNMFTIN  RNTGVISVVT  TGLDRESFPT  YTLVVQAADL  QGEGLSTTAT

               370             380             390             400             410             420

  AVITVTDTND  NPPIFNPTTY  KGQVPENEAN  VVITTLKVTD  ADAPNTPAWE  AVYTILNDDG

               430             440             450             460             470             480

  GQFVVTTNPV  NNDGILKTAK  GLDFEAKQQY  ILHVAVTNVV  PFEVSLTTST  ATVTVDVLDV

               490             500             510             520             530             540

  NEAPIFVPPE  KRVEVSEDFG  VGQEITSYTA  QEPDTFMEQK  ITYRIWRDTA  NWLEINPDTG

               550             560             570             580             590             600

  AISTRAELDR  EDFEHVKNST  YTALIIATDN  GSPVATGTGT  LLLILSDVND  NAPIPEPRTI

               610             620             630             640             650             660

  FFCERNPKPQ  VINIIDADLP  PNTSPFTAEL  THGASANWTI  QYNDPTQESI  ILKPKMALEV
```

```
        670        680        690        700        710        720

GDYKINLKLM DNQNKDQVTT LEVSVCDCEG AAGVCRKAQP VEAGLQIPAI LGILGGILAL

        730        740        750        760        770        780

LILILLLLLF LRRRAVVKEP LLPPEDDTRD NVYYYDEEGG GEEDQDFDLS QLHRGLDARP

        790        800        810        820        830        840

EVTRNDVAPT LMSVPRYLPR PANPDEIGNF IDENLKAADT DPTAPPYDSL LVFDYEGSGS

        850        860        870        880

EAASLSSLNS SESDKDQDYD YLNEWGNRFK KLADMYGGGE DD
```

Most preferably, the epithelial cadherin assay detects one or more soluble forms of epithelial cadherin. Epithelial cadherin is a single-pass type I membrane protein having a large extracellular domain, most or all of which is present in soluble forms of epithelial cadherin generated either through alternative splicing event which deletes all or a portion of the transmembrane domain, or by proteolysis of the membrane-bound form. In the case of an immunoassay, one or more antibodies that bind to epitopes within this extracellular domain may be used to detect these soluble form(s). The following domains have been identified in epithelial cadherin:

| Residues | Length | Domain ID |
|---|---|---|
| 1-22 | 22 | signal sequence |
| 23-154 | 132 | pro-peptide |
| 155-882 | 728 | epithelial cadherin |
| 155-709 | 555 | extracellular |
| 710-730 | 21 | transmembrane |
| 731-882 | 152 | cytoplasmic |

As used herein, the term "poly [ADP-ribose] polymerase 1" refers to one or more polypeptides present in a biological sample that are derived from the poly [ADP-ribose] polymerase 1 precursor (Swiss-Prot 09874 (SEQ ID NO: 7)).

```
        10         20         30         40         50         60

MAESSDKLYR VEYAKSGRAS CKKCSESIPK DSLRMAIMVQ SPMFDGKVPH WYHFSCFWKV

        70         80         90        100        110        120
```

GHSIRHPDVE VDGFSELRWD DQQKVKKTAE AGGVTGKGQD GIGSKAEKTL GDFAAEYAKS

NRSTCKGCME KIEKGQVRLS KKMVDPEKPQ LGMIDRWYHP GCFVKNREEL GFRPEYSASQ

LKGFSLLATE DKEALKKQLP GVKSEGKRKG DEVDGVDEVA KKKSKKEKDK DSKLEKALKA

QNDLIWNIKD ELKKVCSTND LKELLIFNKQ QVPSGESAIL DRVADGMVFG ALLPCEECSG

QLVFKSDAYY CTGDVTAWTK CMVKTQTPNR KEWVTPKEFR EISYLKKLKV KKQDRIFPPE

TSASVAATPP PSTASAPAAV NSSASADKPL SNMKILTLGK LSRNKDEVKA MIEKLGGKLT

GTANKASLCI STKKEVEKMN KKMEEVKEAN IRVVSEDFLQ DVSASTKSLQ ELFLAHILSP

WGAEVKAEPV EVVAPRGKSG AALSKKSKGQ VKEEGINKSE KRMKLTLKGG AAVDPDSGLE

HSAHVLEKGG KVFSATLGLV DIVKGTNSYY KLQLLEDDKE NRYWIFRSWG RVGTVIGSNK

LEQMPSKEDA IEHFMKLYEE KTGNAWHSKN FTKYPKKFYP LEIDYGQDEE AVKKLTVNPG

TKSKLPKPVQ DLIKMIFDVE SMKKAMVEYE IDLQKMPLGK LSKRQIQAAY SILSEVQQAV

SQGSSDSQIL DLSNRFYTLI PHDFGMKKPP LLNNADSVQA KVEMLDNLLD IEVAYSLLRG

GSDDSSKDPI DVNYEKLKTD IKVVDRDSEE AEIIRKYVKN THATTHNAYD LEVIDIFKIE

REGECQRYKP FKQLHNRRLL WHGSRTTNFA GILSQGLRIA PPEAPVTGYM FGKGIYFADM

```
VSKSANYCHT SQGDPIGLIL LGEVALGNMY ELKHASHISK LPKGKHSVKG LGKTTPDPSA
           970        980        990        1000       1010
NISLDGVDVP LGTGISSGVN DTSLLYNEYI VYDIAQVNLK YLLKLKFNFK TSLW
```

The following domains have been identified in poly [ADP-ribose] polymerase 1:

| Residues | Length | Domain ID |
|---|---|---|
| 1 | 1 | initiator methionine |
| 2-1014 | 1013 | poly [ADP-ribose] polymerase 1 |

Poly [ADP-ribose] polymerase 1can be cleaved by many caspases in vitro and is one of the main cleavage targets of caspase-3 in vivo. The cleavage occurs between Asp(214) and Gly(215), which separates PARP's N-terminal DNA binding domain (24 kDa) from its C-terminal catalytic domain (89 kDa). Suitable assays may recognize only the large fragment of poly [ADP-ribose] polymerase 1 (89 kDa) but not the full length poly [ADP-ribose] polymerase 1, may recognize only the small fragment of poly [ADP-ribose] polymerase 1 (24 kDa) but not the full length poly [ADP-ribose] polymerase 1, may recognize only full length poly [ADP-ribose] polymerase 1, or may recognize one fragment and the full length full length poly [ADP-ribose] polymerase 1.

As used herein, the term "cyclin-dependent kinase inhibitor 1" refers to one or more polypeptides present in a biological sample that are derived from the cyclin-dependent kinase inhibitor 1 precursor (Swiss-Prot P38936 (SEQ ID NO: 8)).

```
           10         20         30         40         50         60
MSEPAGDVRQ NPCGSKACRR LFGPVDSEQL SRDCDALMAG CIQEARERWN FDFVTETPLE
           70         80         90         100        110        120
GDFAWERVRG LGLPKLYLPT GPRRGRDELG GGRRPGTSPA LLQGTAEEDH VDLSLSCTLV
           130        140        150        160
PRSGEQAEGS PGGPGDSQGR KRRQTSMTDF YHSKRRLIFS KRKP
```

The following domains have been identified in cyclin-dependent kinase inhibitor 1:

| Residues | Length | Domain |
|---|---|---|
| 1 | 1 | initiator methionine |
| 2-164 | 163 | cyclin-dependent kinase inhibitor 1 |

As used herein, the term "cadherin-5" refers to one or more polypeptides present in a biological sample that are derived from the cadherin-5 precursor (Swiss-Prot P33151 (SEQ ID NO: 9)).

```
            10          20          30          40          50          60

MQRLMMLLAT  SGACLGLLAV  AAVAAAGANP  AQRDTHSLLP  THRRQKRDWI  WNQMHIDEEK

            70          80          90         100         110         120

NTSLPHHVGK  IKSSVSRKNA  KYLLKGEYVG  KVFRVDAETG  DVFAIERLDR  ENISEYHLTA

           130         140         150         160         170         180

VIVDKDTGEN  LETPSSFTIK  VHDVNDNWPV  FTHRLFNASV  PESSAVGTSV  ISVTAVDADD

           190         200         210         220         230         240

PTVGDHASVM  YQILKGKEYF  AIDNSGRIIT  ITKSLDREKQ  ARYEIVVEAR  DAQGLRGDSG

           250         260         270         280         290         300

TATVLVTLQD  INDNFPFFTQ  TKYTFVVPED  TRVGTSVGSL  FVEDPDEPQN  RMTKYSILRG

           310         320         330         340         350         360

DYQDAFTIET  NPAHNEGIIK  PMKPLDYEYI  QQYSFIVEAT  DPTIDLRYMS  PPAGNRAQVI

           370         380         390         400         410         420

INITDVDEPP  IFQQPFYHFQ  LKENQKKPLI  GTVLAMDPDA  ARHSIGYSIR  RTSDKGQFFR

           430         440         450         460         470         480

VTKKGDIYNE  KELDREVYPW  YNLTVEAKEL  DSTGTPTGKE  SIVQVHIEVL  DENDNAPEFA

           490         500         510         520         530         540

KPYQPKVCEN  AVHGQLVLQI  SAIDKDITPR  NVKFKFTLNT  ENNFTLTDNH  DNTANITVKY

           550         560         570         580         590         600

GQFDREHTKV  HFLPVVISDN  GMPSRTGTST  LTVAVCKCNE  QGEFTFCEDM  AAQVGVSIQA

           610         620         630         640         650         660

VVAILLCILT  ITVITLLIFL  RRRLRKQARA  HGKSVPEIHE  QLVTYDEEGG  GEMDTTSYDV

           670         680         690         700         710         720
```

```
SVLNSVRRGG AKPPRPALDA RPSLYAQVQK PPRHAPGAHG GPGEMAAMIE VKKDEADHDG

      730        740        750        760        770        780

DGPPYDTLHI YGYEGSESIA ESLSSLGTDS SDSDVDYDFL NDWGPRFKML AELYGSDPRE


ELLY
```

Most preferably, the cadherin-5 assay detects one or more soluble forms of cadherin-5. Cadherin-5 is a single-pass type I membrane protein having a large extracellular domain, most or all of which is present in soluble forms of cadherin-5 generated either through alternative splicing event which deletes all or a portion of the transmembrane domain, or by proteolysis of the membrane-bound form. In the case of an immunoassay, one or more antibodies that bind to epitopes within this extracellular domain may be used to detect these soluble form(s). The following domains have been identified in cadherin-5:

| Residues | Length | Domain ID |
| --- | --- | --- |
| 1-25 | 251 | signal sequence |
| 26-47 | 22 | pro-peptide |
| 48-784 | 737 | cadherin-5 |
| 48-599 | 522 | extracellular |
| 600-620 | 21 | transmembrane |
| 621-784 | 164 | cytoplasmic |

As used herein, the term "Myoglobin" refers to one or polypeptides present in a biological sample that are derived from the Myoglobin precursor (Swiss-Prot P02144 (SEQ ID NO: 10)).

```
          10         20         30         40         50         60

MGLSDGEWQL VLNVWGKVEA DIPGHGQEVL IRLFKGHPET LEKFDKFKHL KSEDEMKASE

          70         80         90        100        110        120

DLKKHGATVL TALGGILKKK GHHEAEIKPL AQSHATKHKI PVKYLEFISE CIIQVLQSKH

         130        140        150

PGDFGADAQG AMNKALELFR KDMASNYKEL GFQG
```

The following domains have been identified in Myoglobin:

| Residues | Length | Domain ID |
| --- | --- | --- |
| 1 | 1 | Initiator Methionine |
| 2-154 | 153 | Myoglobin |

As used herein, the term "Apolipoprotein A-II" refers to one or polypeptides present in a biological sample that are derived from the Apolipoprotein A-II precursor (Swiss-Prot P02652 (SEQ ID NO: 11)).

```
            10          20          30          40          50          60

MKLLAATVLL  LTICSLEGAL  VRRQAKEPCV  ESLVSQYFQT  VTDYGKDLME  KVKSPELQAE

            70          80          90         100

AKSYFEKSKE  QLTPLIKKAG  TELVNFLSYF  VELGTQPATQ
```

The following domains have been identified in Apolipoprotein A-II:

| Residues | Length | Domain ID |
|----------|--------|-----------|
| 1-18 | 18 | Signal sequence |
| 19-23 | 5 | Propeptide |
| 24-100 | 77 | Apolipoprotein A-II |
| 24-99 | 76 | Apolipoprotein A-II(1-76) |

As used herein, the term "Mucin-16" refers to one or polypeptides present in a biological sample that are derived from the Mucin-16 precursor (Swiss-Prot Q8WXI7 (SEQ ID NO: 12)).

```
            10          20          30          40          50          60

MLKPSGLPGS  SSPTRSLMTG  SRSTKATPEM  DSGLTGATLS  PKTSTGAIVV  TEHTLPFTSP

            70          80          90         100         110         120

DKTLASPTSS  VVGRTTQSLG  VMSSALPEST  SRGMTHSEQR  TSPSLSPQVN  GTPSRNYPAT

           130         140         150         160         170         180

SMVSGLSSPR  TRTSSTEGNF  TKEASTYTLT  VETTSGPVTE  KYTVPTETST  TEGDSTETPW
```

```
        190         200         210         220         230         240
DTRYIPVKIT SPMKTFADST ASKENAPVSM TPAETTVTDS HTPGRTNPSF GTLYSSFLDL

        250         260         270         280         290         300
SPKGTPNSRG ETSLELILST TGYPFSSPEP GSAGHSRIST SAPLSSSASV LDNKISETSI

        310         320         330         340         350         360
FSGQSLTSPL SPGVPEARAS TMPNSAIPFS MTLSNAETSA ERVRSTISSL GTPSISTKQT

        370         380         390         400         410         420
AETILTFHAF AETMDIPSTH IAKTLASEWL GSPGTLGGTS TSALTTTSPS TTLVSEETNT

        430         440         450         460         470         480
HHSTSGKETE GTLNTSMTPL ETSAPGEESE MTATLVPTLG FTTLDSKIRS PSQVSSSHPT

        490         500         510         520         530         540
RELRTTGSTS GRQSSSTAAH GSSDILRATT SSTSKASSWT SESTAQQFSE PQHTQWVETS

        550         560         570         580         590         600
PSMKTERPPA STSVAAPITT SVPSVVSGFT TLKTSSTKGI WLEETSADTL IGESTAGPTT

        610         620         630         640         650         660
HQFAVPTGIS MTGGSSTRGS QGTTHLLTRA TASSETSADL TLATNGVPVS VSPAVSKTAA

        670         680         690         700         710         720
GSSPPGGTKP SYTMVSSVIP ETSSLQSSAF REGTSLGLTP LNTRHPFSSP EPDSAGHTKI

        730         740         750         760         770         780
STSIPLLSSA SVLEDKVSAT STFSHHKATS SITTGTPEIS TKTKPSSAVL SSMTLSNAAT

        790         800         810         820         830         840
SPERVRNATS PLTHPSPSGE ETAGSVLTLS TSAETTDSPN IHPTGTLTSE SSESPSTLSL

        850         860         870         880         890         900
PSVSGVKTTF SSSTPSTHLF TSGEETEETS NPSVSQPETS VSRVRTTLAS TSVPTPVFPT

        910         920         930         940         950         960
MDTWPTRSAQ FSSSHLVSEL RATSSTSVTN STGSALPKIS HLTGTATMSQ TNRDTFNDSA

        970         980         990        1000        1010        1020
APQSTTWPET SPRFKTGLPS ATTTVSTSAT SLSATVMVSK FTSPATSSME ATSIREPSTT
```

```
        1030        1040        1050        1060        1070        1080
ILTTETTNGP  GSMAVASTNI  PIGKGYITEG  RLDTSHLPIG  TTASSETSMD  FTMAKESVSM

        1090        1100        1110        1120        1130        1140
SVSPSQSMDA  AGSSTPGRTS  QFVDTFSDDV  YHLTSREITI  PRDGTSSALT  PQMTATHPPS

        1150        1160        1170        1180        1190        1200
PDPGSARSTW  LGILSSSPSS  PTPKVTMSST  FSTQRVTTSM  IMDTVETSRW  NMPNLPSTTS

        1210        1220        1230        1240        1250        1260
LTPSNIPTSG  AIGKSTLVPL  DTPSPATSLE  ASEGGLPTLS  TYPESTNTPS  IHLGAHASSE

        1270        1280        1290        1300        1310        1320
SPSTIKLTMA  SVVKPGSYTP  LTFPSIETHI  HVSTARMAYS  SGSSPEMTAP  GETNTGSTWD

        1330        1340        1350        1360        1370        1380
PTTYITTTDP  KDTSSAQVST  PHSVRTLRTT  ENHPKTESAT  PAAYSGSPKI  SSSPNLTSPA

        1390        1400        1410        1420        1430        1440
TKAWTITDTT  EHSTQLHYTK  LAEKSSGFET  QSAPGPVSVV  IPTSPTIGSS  TLELTSDVPG

        1450        1460        1470        1480        1490        1500
EPLVLAPSEQ  TTITLPMATW  LSTSLTEEMA  STDLDISSPS  SPMSTFAIFP  PMSTPSHELS

        1510        1520        1530        1540        1550        1560
KSEADTSAIR  NTDSTTLDQH  LGIRSLGRTG  DLTTVPITPL  TTTWTSVIEH  STQAQDTLSA

        1570        1580        1590        1600        1610        1620
TMSPTHVTQS  LKDQTSIPAS  ASPSHLTEVY  PELGTQGRSS  SEATTFWKPS  TDTLSREIET

        1630        1640        1650        1660        1670        1680
GPTNIQSTPP  MDNTTTGSSS  SGVTLGIAHL  PIGTSSPAET  STNMALERRS  STATVSMAGT

        1690        1700        1710        1720        1730        1740
MGLLVTSAPG  RSISQSLGRV  SSVLSESTTE  GVTDSSKGSS  PRLNTQGNTA  LSSSLEPSYA

        1750        1760        1770        1780        1790        1800
EGSQMSTSIP  LTSSPTTPDV  EFIGGSTFWT  KEVTTVMTSD  ISKSSARTES  SSATLMSTAL

        1810        1820        1830        1840        1850        1860
GSTENTGKEK  LRTASMDLPS  PTPSMEVTPW  ISLTLSNAPN  TTDSLDLSHG  VHTSSAGTLA
```

```
       1870        1880        1890        1900        1910        1920
TDRSLNTGVT  RASRLENGSD  TSSKSLSMGN  STHTSMTDTE  KSEVSSSIHP  RPETSAPGAE

       1930        1940        1950        1960        1970        1980
TTLTSTPGNR  AISLTLPFSS  IPVEEVISTG  ITSGPDINSA  PMTHSPITPP  TIVWTSTGTI

       1990        2000        2010        2020        2030        2040
EQSTQPLHAV  SSEKVSVQTQ  STPYVNSVAV  SASPTHENSV  SSGSSTSSPY  SSASLESLDS

       2050        2060        2070        2080        2090        2100
TISRRNAITS  WLWDLTTSLP  TTTWPSTSLS  EALSSGHSGV  SNPSSTTTEF  PLFSAASTSA

       2110        2120        2130        2140        2150        2160
AKQRNPETET  HGPQNTAAST  LNTDASSVTG  LSETPVGASI  SSEVPLPMAI  TSRSDVSGLT

       2170        2180        2190        2200        2210        2220
SESTANPSLG  TASSAGTKLT  RTISLPTSES  LVSFRMNKDP  WTVSIPLGSH  PTTNTETSIP

       2230        2240        2250        2260        2270        2280
VNSAGPPGLS  TVASDVIDTP  SDGAESIPTV  SFSPSPDTEV  TTISHFPEKT  THSFRTISSL

       2290        2300        2310        2320        2330        2340
THELTSRVTP  IPGDWMSSAM  STKPTGASPS  ITLGERRTIT  SAAPTTSPIV  LTASFTETST

       2350        2360        2370        2380        2390        2400
VSLDNETTVK  TSDILDARKT  NELPSDSSSS  SDLINTSIAS  STMDVTKTAS  ISPTSISGMT

       2410        2420        2430        2440        2450        2460
ASSSPSLFSS  DRPQVPTSTT  ETNTATSPSV  SSNTYSLDGG  SNVGGTPSTL  PPFTITHPVE

       2470        2480        2490        2500        2510        2520
TSSALLAWSR  PVRTFSTMVS  TDTASGENPT  SSNSVVTSVP  APGTWASVGS  TTDLPAMGFL

       2530        2540        2550        2560        2570        2580
KTSPAGEAHS  LLASTIEPAT  AFTPHLSAAV  VTGSSATSEA  SLLTTSESKA  IHSSPQTPTT

       2590        2600        2610        2620        2630        2640
PTSGANWETS  ATPESLLVVT  ETSDTTLTSK  ILVTDTILFS  TVSTPPSKFP  STGTLSGASF

       2650        2660        2670        2680        2690        2700
PTLLPDTPAI  PLTATEPTSS  LATSFDSTPL  VTIASDSLGT  VPETTLTMSE  TSNGDALVLK
```

```
        2710       2720       2730       2740       2750       2760

TVSNPDRSIP GITIQGVTES PLHPSSTSPS KIVAPRNTTY EGSITVALST LPAGTTGSLV

        2770       2780       2790       2800       2810       2820

FSQSSENSET TALVDSSAGL ERASVMPLTT GSQGMASSGG IRSGSTHSTG TKTFSSLPLT

        2830       2840       2850       2860       2870       2880

MNPGEVTAMS EITTNRLTAT QSTAPKGIPV KPTSAESGLL TPVSASSSPS KAFASLTTAP

        2890       2900       2910       2920       2930       2940

PSTWGIPQST LTFEFSEVPS LDTKSASLPT PGQSLNTIPD SDASTASSSL SKSPEKNPRA

        2950       2960       2970       2980       2990       3000

RMMTSTKAIS ASSFQSTGFT ETPEGSASPS MAGHEPRVPT SGTGDPRYAS ESMSYPDPSK

        3010       3020       3030       3040       3050       3060

ASSAMTSTSL ASKLTTLFST GQAARSGSSS SPISLSTEKE TSFLSPTAST SRKTSLFLGP

        3070       3080       3090       3100       3110       3120

SMARQPNILV HLQTSALTLS PTSTLNMSQE EPPELTSSQT IAEEEGTTAE TQTLTFTPSE

        3130       3140       3150       3160       3170       3180

TPTSLLPVSS PTEPTARRKS SPETWASSIS VPAKTSLVET TDGTLVTTIK MSSQAAQGNS

        3190       3200       3210       3220       3230       3240

TWPAPAEETG TSPAGTSPGS PEVSTTLKIM SSKEPSISPE IRSTVRNSPW KTPETTVPME

        3250       3260       3270       3280       3290       3300

TTVEPVTLQS TALGSGSTSI SHLPTGTTSP TKSPTENMLA TERVSLSPSP PEAWTNLYSG

        3310       3320       3330       3340       3350       3360

TPGGTRQSLA TMSSVSLESP TARSITGTGQ QSSPELVSKT TGMEFSMWHG STGGTTGDTH

        3370       3380       3390       3400       3410       3420

VSLSTSSNIL EDPVTSPNSV SSLTDKSKHK TETWVSTTAI PSTVLNNKIM AAEQQTSRSV

        3430       3440       3450       3460       3470       3480

DEAYSSTSSW SDQTSGSDIT LGASPDVTNT LYITSTAQTT SLVSLPSGDQ GITSLTNPSG

        3490       3500       3510       3520       3530       3540

GKTSSASSVT SPSIGLETLR ANVSAVKSDI APTAGHLSQT SSPAEVSILD VTTAPTPGIS
```

```
        3550       3560       3570       3580       3590       3600
TTITTMGTNS ISTTTPNPEV GMSTMDSTPA TERRTTSTEH PSTWSSTAAS DSWTVTDMTS

        3610       3620       3630       3640       3650       3660
NLKVARSPGT ISTMHTTSFL ASSTELDSMS TPHGRITVIG TSLVTPSSDA SAVKTETSTS

        3670       3680       3690       3700       3710       3720
ERTLSPSDTT ASTPISTFSR VQRMSISVPD ILSTSWTPSS TEAEDVPVSM VSTDHASTKT

        3730       3740       3750       3760       3770       3780
DPNTPLSTFL FDSLSTLDWD TGRSLSSATA TTSAPQGATT PQELTLETMI SPATSQLPFS

        3790       3800       3810       3820       3830       3840
IGHITSAVTP AAMARSSGVT FSRPDPTSKK AEQTSTQLPT TTSAHPGQVP RSAATTLDVI

        3850       3860       3870       3880       3890       3900
PHTAKTPDAT FQRQGQTALT TEARATSDSW NEKEKSTPSA PWITEMMNSV SEDTIKEVTS

        3910       3920       3930       3940       3950       3960
SSSVLKDPEY AGHKLGIWDD FIPKFGKAAH MRELPLLSPP QDKEAIHPST NTVETTGWVT

        3970       3980       3990       4000       4010       4020
SSEHASHSTI PAHSASSKLT SPVVTTSTRE QAIVSMSTTT WPESTRARTE PNSFLTIELR

        4030       4040       4050       4060       4070       4080
DVSPYMDTSS TTQTSIISSP GSTAITKGPR TEITSSKRIS SSFLAQSMRS SDSPSEAITR

        4090       4100       4110       4120       4130       4140
LSNFPAMTES GGMILAMQTS PPGATSLSAP TLDTSATASW TGTPLATTQR FTYSEKTTLF

        4150       4160       4170       4180       4190       4200
SKGPEDTSQP SPPSVEETSS SSSLVPIHAT TSPSNILLTS QGHSPSSTPP VTSVFLSETS

        4210       4220       4230       4240       4250       4260
GLGKTTDMSR ISLEPGTSLP PNLSSTAGEA LSTYEASRDT KAIHHSADTA VTNMEATSSE

        4270       4280       4290       4300       4310       4320
YSPIPGHTKP SKATSPLVTS HIMGDITSST SVFGSSETTE IETVSSVNQG LQERSTSQVA

        4330       4340       4350       4360       4370       4380
SSATETSTVI THVSSGDATT HVTKTQATFS SGTSISSPHQ FITSTNTFTD VSTNPSTSLI
```

```
            4390        4400        4410        4420        4430        4440
MTESSGVTIT  TQTGPTGAAT  QGPYLLDTST  MPYLTETPLA  VTPDFMQSEK  TTLISKGPKD
            4450        4460        4470        4480        4490        4500
VTWTSPPSVA  ETSYPSSLTP  FLVTTIPPAT  STLQGQHTSS  PVSATSVLTS  GLVKTTDMLN
            4510        4520        4530        4540        4550        4560
TSMEPVTNSP  QNLNNPSNEI  LATLAATTDI  ETIHPSINKA  VTNMGTASSA  HVLHSTLPVS
            4570        4580        4590        4600        4610        4620
SEPSTATSPM  VPASSMGDAL  ASISIPGSET  TDIEGEPTSS  LTAGRKENST  LQEMNSTTES
            4630        4640        4650        4660        4670        4680
NIILSNVSVG  AITEATKMEV  PSFDATFIPT  PAQSTKFPDI  FSVASSRLSN  SPPMTISTHM
            4690        4700        4710        4720        4730        4740
TTTQTGSSGA  TSKIPLALDT  STLETSAGTP  SVVTEGFAHS  KITTAMNNDV  KDVSQTNPPF
            4750        4760        4770        4780        4790        4800
QDEASSPSSQ  APVLVTTLPS  SVAFTPQWHS  TSSPVSMSSV  LTSSLVKTAG  KVDTSLETVT
            4810        4820        4830        4840        4850        4860
SSPQSMSNTL  DDISVTSAAT  TDIETTHPSI  NTVVTNVGTT  GSAFESHSTV  SAYPEPSKVT
            4870        4880        4890        4900        4910        4920
SPNVTTSTME  DTTISRSIPK  SSKTTRTETE  TTSSLTPKLR  ETSISQEITS  STETSTVPYK
            4930        4940        4950        4960        4970        4980
ELTGATTEVS  RTDVTSSSST  SFPGPDQSTV  SLDISTETNT  RLSTSPIMTE  SAEITITTQT
            4990        5000        5010        5020        5030        5040
GPHGATSQDT  FTMDPSNTTP  QAGIHSAMTH  GFSQLDVTTL  MSRIPQDVSW  TSPPSVDKTS
            5050        5060        5070        5080        5090        5100
SPSSFLSSPA  MTTPSLISST  LPEDKLSSPM  TSLLTSGLVK  ITDILRTRLE  PVTSSLPNFS
            5110        5120        5130        5140        5150        5160
STSDKILATS  KDSKDTKEIF  PSINTEETNV  KANNSGHESH  SPALADSETP  KATTQMVITT
            5170        5180        5190        5200        5210        5220
TVGDPAPSTS  MPVHGSSETT  NIKREPTYFL  TPRLRETSTS  QESSFPTDTS  FLLSKVPTGT
```

```
        5230       5240       5250       5260       5270       5280

ITEVSSTGVN SSSKISTPDH DKSTVPPDTF TGEIPRVFTS SIKTKSAEMT ITTQASPPES

        5290       5300       5310       5320       5330       5340

ASHSTLPLDT STTLSQGGTH STVTQGFPYS EVTTLMGMGP GNVSWMTTPP VEETSSVSSL

        5350       5360       5370       5380       5390       5400

MSSPAMTSPS PVSSTSPQSI PSSPLPVTAL PTSVLVTTTD VLGTTSPESV TSSPPNLSSI

        5410       5420       5430       5440       5450       5460

THERPATYKD TAHTEAAMHH STNTAVTNVG TSGSGHKSQS SVLADSETSK ATPLMSTTST

        5470       5480       5490       5500       5510       5520

LGDTSVSTST PNISQTNQIQ TEPTASLSPR LRESSTSEKT SSTTETNTAF SYVPTGAITQ

        5530       5540       5550       5560       5570       5580

ASRTEISSSR TSISDLDRPT IAPDISTGMI TRLFTSPIMT KSAEMTVTTQ TTTPGATSQG

        5590       5600       5610       5620       5630       5640

ILPWDTSTTL FQGGTHSTVS QGFPHSEITT LRSRTPGDVS WMTTPPVEET SSGFSLMSPS

        5650       5660       5670       5680       5690       5700

MTSPSPVSST SPESIPSSPL PVTALLTSVL VTTTNVLGTT SPETVTSSPP NLSSPTQERL

        5710       5720       5730       5740       5750       5760

TTYKDTAHTE AMHASMHTNT AVANVGTSIS GHESQSSVPA DSHTSKATSP MGITFAMGDT

        5770       5780       5790       5800       5810       5820

SVSTSTPAFF ETRIQTESTS SLIPGLRDTR TSEEINTVTE TSTVLSEVPT TTTTEVSRTE

        5830       5840       5850       5860       5870       5880

VITSSRTTIS GPDHSKMSPY ISTETITRLS TFPFVTGSTE MAITNQTGPI GTISQATLTL

        5890       5900       5910       5920       5930       5940

DTSSTASWEG THSPVTQRFP HSEETTMSR STKGVSWQSP PSVEETSSPS SPVPLPAITS

        5950       5960       5970       5980       5990       6000

HSSLYSAVSG SSPTSALPVT SLLTSGRRKT IDMLDTHSEL VTSSLPSASS FSGEILTSEA

        6010       6020       6030       6040       6050       6060

STNTETIHFS ENTAETNMGT TNSMHKLHSS VSIHSQPSGH TPPKVTGSMM EDAIVSTSTP
```

```
        6070        6080        6090        6100        6110        6120

GSPETKNVDR  DSTSPLTPEL  KEDSTALVMN  STTESNTVFS  SVSLDAATEV  SRAEVTYYDP

        6130        6140        6150        6160        6170        6180

TFMPASAQST  KSPDISPEAS  SSHSNSPPLT  ISTHKTIATQ  TGPSGVTSLG  QLTLDTSTIA

        6190        6200        6210        6220        6230        6240

TSAGTPSART  QDFVDSETTS  VMNNDLNDVL  KTSPFSAEEA  NSLSSQAPLL  VTTSPSPVTS

        6250        6260        6270        6280        6290        6300

TLQEHSTSSL  VSVTSVPTPT  LAKITDMDTN  LEPVTRSPQN  LRNTLATSEA  TTDTHTMHPS

        6310        6320        6330        6340        6350        6360

INTAMANVGT  TSSPNEFYFT  VSPDSDPYKA  TSAVVITSTS  GDSIVSTSMP  RSSAMKKIES

        6370        6380        6390        6400        6410        6420

ETTFSLIFRL  RETSTSQKIG  SSSDTSTVFD  KAFTAATTEV  SRTELTSSSR  TSIQGTEKPT

        6430        6440        6450        6460        6470        6480

MSPDTSTRSV  TMLSTFAGLT  KSEERTIATQ  TGPHRATSQG  TLTWDTSITT  SQAGTHSAMT

        6490        6500        6510        6520        6530        6540

HGFSQLDLST  LTSRVPEYIS  GTSPPSVEKT  SSSSSLLSLP  AITSPSPVPT  TLPESRPSSP

        6550        6560        6570        6580        6590        6600

VHLTSLPTSG  LVKTTDMLAS  VASLPPNLGS  TSHKIPTTSE  DIKDTEKMYP  STNIAVTNVG

        6610        6620        6630        6640        6650        6660

TTTSEKESYS  SVPAYSEPPK  VTSPMVTSFN  IRDTIVSTSM  PGSSEITRIE  MESTFSVAHG

        6670        6680        6690        6700        6710        6720

LKGTSTSQDP  IVSTEKSAVL  HKLTTGATET  SRTEVASSRR  TSIPGPDHST  ESPDISTEVI

        6730        6740        6750        6760        6770        6780

PSLPISLGIT  ESSNMTIITR  TGPPLGSTSQ  GTFTLDTPTT  SSRAGTHSMA  TQEFPHSEMT

        6790        6800        6810        6820        6830        6840

TVMNKDPEIL  SWTIPPSIEK  TSFSSSLMPS  PAMTSPPVSS  TLPKTIHTTP  SPMTSLLTPS

        6850        6860        6870        6880        6890        6900

LVMTTDTLGT  SPEPTTSSPP  NLSSTSHVIL  TTDEDTTAIE  AMHPSTSTAA  TNVETTCSGH
```

```
        6910       6920       6930       6940       6950       6960
GSQSSVLTDS EKTKATAPMD TTSTMGHTTV STSMSVSSET TKIKRESTYS LTPGLRETSI

        6970       6980       6990       7000       7010       7020
SQNASFSTDT SIVLSEVPTG TTAEVSRTEV TSSGRTSIPG PSQSTVLPEI STRTMTRLFA

        7030       7040       7050       7060       7070       7080
SPTMTESAEM TIPTQTGPSG STSQDTLTLD TSTTKSQAKT HSTLTQRFPH SEMTTLMSRG

        7090       7100       7110       7120       7130       7140
PGDMSWQSSP SLENPSSLPS LLSLPATTSP PPISSTLPVT ISSSPLPVTS LLTSSPVTTT

        7150       7160       7170       7180       7190       7200
DMLHTSPELV TSSPPKLSHT SDERLTTGKD TTNTEAVHPS TNTAASNVEI PSFGHESPSS

        7210       7220       7230       7240       7250       7260
ALADSETSKA TSPMFITSTQ EDTTVAISTP HFLETSRIQK ESISSLSPKL RETGSSVETS

        7270       7280       7290       7300       7310       7320
SAIETSAVLS EVSIGATTEI SRTEVTSSSR TSISGSAEST MLPEISTTRK IIKFPTSPIL

        7330       7340       7350       7360       7370       7380
AESSEMTIKT QTSPPGSTSE STFTLDTSTT PSLVITHSTM TQRLPHSEIT TLVSRGAGDV

        7390       7400       7410       7420       7430       7440
PRPSSLPVEE TSPPSSQLSL SAMISPSPVS STLPASSHSS SASVTSPLTP GQVKTTEVLD

        7450       7460       7470       7480       7490       7500
ASAEPETSSP PSLSSTSVEI LATSEVTTDT EKIHPFPNTA VTKVGTSSSG HESPSSVLPD

        7510       7520       7530       7540       7550       7560
SETTKATSAM GTISIMGDTS VSTLTPALSN TRKIQSEPAS SLTTRLRETS TSEETSLATE

        7570       7580       7590       7600       7610       7620
ANTVLSKVST GATTEVSRTE AISFSRTSMS GPEQSTMSQD ISIGTIPRIS ASSVLTESAK

        7630       7640       7650       7660       7670       7680
MTITTQTGPS ESTLESTLNL NTATTPSWVE THSIVIQGFP HPEMTTSMGR GPGGVSWPSP

        7690       7700       7710       7720       7730       7740
PFVKETSPPS SPLSLPAVTS PHPVSTTFLA HIPPSPLPVT SLLTSGPATT TDILGTSTEP
```

```
        7750        7760        7770        7780        7790        7800
GTSSSSSLST TSHERLTTYK DTAHTEAVHP STNTGGTNVA TTSSGYKSQS SVLADSSPMC

        7810        7820        7830        7840        7850        7860
TTSTMGDTSV LTSTPAFLET RRIQTELASS LTPGLRESSG SEGTSSGTKM STVLSKVPTG

        7870        7880        7890        7900        7910        7920
ATTEISKEDV TSIPGPAQST ISPDISTRTV SWFSTSPVMT ESAEITMNTH TSPLGATTQG

        7930        7940        7950        7960        7970        7980
TSTLATSSTT SLTMTHSTIS QGFSHSQMST LMRRGPEDVS WMSPPLLEKT RPSFSLMSSP

        7990        8000        8010        8020        8030        8040
ATTSPSPVSS TLPESISSSP LPVTSLLTSG LAKTTDMLHK SSEPVTNSPA NLSSTSVEIL

        8050        8060        8070        8080        8090        8100
ATSEVTTDTE KTHPSSNRTV TDVGTSSSGH ESTSFVLADS QTSKVTSPMV ITSTMEDTSV

        8110        8120        8130        8140        8150        8160
STSTPGFFET SRIQTEPTSS LTLGLRKTSS SEGTSLATEM STVLSGVPTG ATAEVSRTEV

        8170        8180        8190        8200        8210        8220
TSSSRTSISG FAQLTVSPET STETITRLPT SSIMTESAEM MIKTQTDPPG STPESTHTVD

        8230        8240        8250        8260        8270        8280
ISTTPNWVET HSTVTQRFSH SEMTTLVSRS PGDMLWPSQS SVEETSSASS LLSLPATTSP

        8290        8300        8310        8320        8330        8340
SPVSSTLVED FPSASLPVTS LLTPGLVITT DRMGISREPG TSSTSNLSST SHERLTTLED

        8350        8360        8370        8380        8390        8400
TVDTEDMQPS THTAVTNVRT SISGHESQSS VLSDSETPKA TSPMGTTYTM GETSVSISTS

        8410        8420        8430        8440        8450        8460
DFFETSRIQI EPTSSLTSGL RETSSSERIS SATEGSTVLS EVPSGATTEV SRTEVISSRG

        8470        8480        8490        8500        8510        8520
TSMSGPDQFT ISPDISTEAI TRLSTSPIMT ESAESAITIE TGSPGATSEG TLTLDTSTTT

        8530        8540        8550        8560        8570        8580
FWSGTHSTAS PGFSHSEMTT LMSRTPGDVP WPSLPSVEEA SSVSSSLSSP AMTSTSFFSA
```

| 8590 | 8600 | 8610 | 8620 | 8630 | 8640 |
|------|------|------|------|------|------|
| LPESISSSPH | PVTALLTLGP | VKTTDMLRTS | SEPETSSPPN | LSSTSAEILA | TSEVTKDREK |

| 8650 | 8660 | 8670 | 8680 | 8690 | 8700 |
|------|------|------|------|------|------|
| IHPSSNTPVV | NVGTVIYKHL | SPSSVLADLV | TTKPTSPMAT | TSTLGNTSVS | TSTPAFPETM |

| 8710 | 8720 | 8730 | 8740 | 8750 | 8760 |
|------|------|------|------|------|------|
| MTQPTSSLTS | GLREISTSQE | TSSATERSAS | LSGMPTGATT | KVSRTEALSL | GRTSTPGPAQ |

| 8770 | 8780 | 8790 | 8800 | 8810 | 8820 |
|------|------|------|------|------|------|
| STISPEISTE | TITRISTPLT | TTGSAEMTIT | PKTGHSGASS | QGTFTLDTSS | RASWPGTHSA |

| 8830 | 8840 | 8850 | 8860 | 8870 | 8880 |
|------|------|------|------|------|------|
| ATHRSPHSGM | TTPMSRGPED | VSWPSRPSVE | KTSPPSSLVS | LSAVTSPSPL | YSTPSESSHS |

| 8890 | 8900 | 8910 | 8920 | 8930 | 8940 |
|------|------|------|------|------|------|
| SPLRVTSLFT | PVMMKTTDML | DTSLEPVTTS | PPSMNITSDE | SLATSKATME | TEAIQLSENT |

| 8950 | 8960 | 8970 | 8980 | 8990 | 9000 |
|------|------|------|------|------|------|
| AVTQMGTISA | RQEFYSSYPG | LPEPSKVTSP | VVTSSTIKDI | VSTTIPASSE | ITRIEMESTS |

| 9010 | 9020 | 9030 | 9040 | 9050 | 9060 |
|------|------|------|------|------|------|
| TLTPTPRETS | TSQEIHSATK | PSTVPYKALT | SATIEDSMTQ | VMSSSRGPSP | DQSTMSQDIS |

| 9070 | 9080 | 9090 | 9100 | 9110 | 9120 |
|------|------|------|------|------|------|
| SEVITRLSTS | PIKAESTEMT | ITTQTGSPGA | TSRGTLTLDT | STTFMSGTHS | TASQGFSHSQ |

| 9130 | 9140 | 9150 | 9160 | 9170 | 9180 |
|------|------|------|------|------|------|
| MTALMSRTPG | DVPWLSHPSV | EEASSASFSL | SSPVMTSSSP | VSSTLPDSIH | SSSLPVTSLL |

| 9190 | 9200 | 9210 | 9220 | 9230 | 9240 |
|------|------|------|------|------|------|
| TSGLVKTTEL | LGTSSEPETS | SPPNLSSTSA | EILATTEVTT | DTEKLEMTNV | VTSGYTHESP |

| 9250 | 9260 | 9270 | 9280 | 9290 | 9300 |
|------|------|------|------|------|------|
| SSVLADSVTT | KATSSMGITY | PTGDTNVLTS | TPAFSDTSRI | QTKSKLSLTP | GLMETSISEE |

| 9310 | 9320 | 9330 | 9340 | 9350 | 9360 |
|------|------|------|------|------|------|
| TSSATEKSTV | LSSVPTGATT | EVSRTEAISS | SRTSIPGPAQ | STMSSDTSME | TITRISTPLT |

| 9370 | 9380 | 9390 | 9400 | 9410 | 9420 |
|------|------|------|------|------|------|
| RKESTDMAIT | PKTGPSGATS | QGTFTLDSSS | TASWPGTHSA | TTQRFPQSVV | TTPMSRGPED |

```
        9430        9440        9450        9460        9470        9480

VSWPSPLSVE  KNSPPSSLVS  SSSVTSPSPL  YSTPSGSSHS  SPVPVTSLFT  SIMMKATDML

        9490        9500        9510        9520        9530        9540

DASLEPETTS  APNMNITSDE  SLATSKATTE  TEAIHVFENT  AASHVETTSA  TEELYSSSPG

        9550        9560        9570        9580        9590        9600

FSEPTKVISP  VVTSSSIRDN  MVSTTMPGSS  GITRIEIESM  SSLTPGLRET  RTSQDITSST

        9610        9620        9630        9640        9650        9660

ETSTVLYKMS  SGATPEVSRT  EVMPSSRTSI  PGPAQSTMSL  DISDEVVTRL  STSPIMTESA

        9670        9680        9690        9700        9710        9720

EITITTQTGY  SLATSQVTLP  LGTSMTFLSG  THSTMSQGLS  HSEMTNLMSR  GPESLSWTSP

        9730        9740        9750        9760        9770        9780

RFVETTRSSS  SLTSLPLTTS  LSPVSSTLLD  SSPSSPLPVT  SLILPGLVKT  TEVLDTSSEP

        9790        9800        9810        9820        9830        9840

KTSSSPNLSS  TSVEIPATSE  IMTDTEKIHP  SSNTAVAKVR  TSSSVHESHS  SVLADSETTI

        9850        9860        9870        9880        9890        9900

TIPSMGITSA  VDDTTVFTSN  PAFSETRRIP  TEPTFSLTPG  FRETSTSEET  TSITETSAVL

        9910        9920        9930        9940        9950        9960

YGVPTSATTE  VSMTEIMSSN  RTHIPDSDQS  TMSPDIITEV  ITRLSSSSMM  SESTQMTITT

        9970        9980        9990       10000       10010       10020

QKSSPGATAQ  STLTLATTTA  PLARTHSTVP  PRFLHSEMTT  LMSRSPENPS  WKSSPFVEKT

       10030       10040       10050       10060       10070       10080

SSSSSLLSLP  VTTSPSVSST  LPQSIPSSSF  SVTSLLTPGM  VKTTDTSTEP  GTSLSPNLSG

       10090       10100       10110       10120       10130       10140

TSVEILAASE  VTTDTEKIHP  SSSMAVTNVG  TTSSGHELYS  SVSIHSEPSK  ATYPVGTPSS

       10150       10160       10170       10180       10190       10200

MAETSISTSM  PANFETTGFE  AEPFSHLTSG  FRKTNMSLDT  SSVTPTNTPS  SPGSTHLLQS

       10210       10220       10230       10240       10250       10260

SKTDFTSSAK  TSSPDWPPAS  QYTEIPVDII  TPFNASPSIT  ESTGITSFPE  SRFTMSVTES
```

34

```
        10270        10280        10290        10300        10310        10320
THHLSTDLLP SAETISTGTV MPSLSEAMTS FATTGVPRAI SGSGSPFSRT ESGPGDATLS

        10330        10340        10350        10360        10370        10380
TIAESLPSST PVPFSSSTFT TTDSSTIPAL HEITSSSATP YRVDTSLGTE SSTTEGRLVM

        10390        10400        10410        10420        10430        10440
VSTLDTSSQP GRTSSTPILD TRMTESVELG TVTSAYQVPS LSTRLTRTDG IMEHITKIPN

        10450        10460        10470        10480        10490        10500
EAAHRGTIRP VKGPQTSTSP ASPKGLHTGG TKRMETTTTA LKTTTTALKT TSRATLTTSV

        10510        10520        10530        10540        10550        10560
YTPTLGTLTP LNASRQMAST ILTEMMITTP YVFPDVPETT SSLATSLGAE TSTALPRTTP

        10570        10580        10590        10600        10610        10620
SVLNRESETT ASLVSRSGAE RSPVIQTLDV SSSEPDTTAS WVIHPAETIP TVSKTTPNFF

        10630        10640        10650        10660        10670        10680
HSELDTVSST ATSHGADVSS AIPTNISPSE LDALTPLVTI SGTDTSTTFP TLTKSPHETE

        10690        10700        10710        10720        10730        10740
TRTTWLTHPA ETSSTIPRTI PNFSHHESDA TPSIATSPGA ETSSAIPIMT VSPGAEDLVT

        10750        10760        10770        10780        10790        10800
SQVTSSGTDR NMTIPTLTLS PGEPKTIASL VTHPEAQTSS AIPTSTISPA VSRLVTSMVT

        10810        10820        10830        10840        10850        10860
SLAAKTSTTN RALTNSPGEP ATTVSLVTHP AQTSPTVPWT TSIFFHSKSD TTPSMTTSHG

        10870        10880        10890        10900        10910        10920
AESSSAVPTP TVSTEVPGVV TPLVTSSRAV ISTTIPILTL SPGEPETTPS MATSHGEEAS

        10930        10940        10950        10960        10970        10980
SAIPTPTVSP GVPGVVTSLV TSSRAVTSTT IPILTFSLGE PETTPSMATS HGTEAGSAVP

        10990        11000        11010        11020        11030        11040
TVLPEVPGMV TSLVASSRAV TSTTLPTLTL SPGEPETTPS MATSHGAEAS STVPTVSPEV

        11050        11060        11070        11080        11090        11100
PGVVTSLVTS SSGVNSTSIP TLILSPGELE TTPSMATSHG AEASSAVPTP TVSPGVSGVV
```

```
        11110        11120        11130        11140        11150        11160
TPLVTSSRAV TSTTIPILTL SSSEPETTPS MATSHGVEAS SAVLTVSPEV PGMVTSLVTS
        11170        11180        11190        11200        11210        11220
SRAVTSTTIP TLTISSDEPE TTTSLVTHSE AKMISAIPTL AVSPTVQGLV TSLVTSSGSE
        11230        11240        11250        11260        11270        11280
TSAFSNLTVA SSQPETIDSW VAHPGTEASS VVPTLTVSTG EPFTNISLVT HPAESSSTLP
        11290        11300        11310        11320        11330        11340
RTTSRFSHSE LDTMPSTVTS PEAESSSAIS TTISPGIPGV LTSLVTSSGR DISATFPTVP
        11350        11360        11370        11380        11390        11400
ESPHESEATA SWVTHPAVTS TTVPRTTPNY SHSEPDTTPS IATSPGAEAT SDFPTITVSP
        11410        11420        11430        11440        11450        11460
DVPDMVTSQV TSSGTDTSIT IPTLTLSSGE PETTTSFITY SETHTSSAIP TLPVSPGASK
        11470        11480        11490        11500        11510        11520
MLTSLVISSG TDSTTTFPTL TETPYEPETT AIQLIHPAET NTMVPKTTPK FSHSKSDTTL
        11530        11540        11550        11560        11570        11580
PVAITSPGPE ASSAVSTTTI SPDMSDLVTS LVPSSGTDTS TTFPTLSETP YEPETTVTWL
        11590        11600        11610        11620        11630        11640
THPAETSTTV SGTIPNFSHR GSDTAPSMVT SPGVDTRSGV PTTTIPPSIP GVVTSQVTSS
        11650        11660        11670        11680        11690        11700
ATDTSTAIPT LTPSPGEPET TASSATHPGT QTGFTVPIRT VPSSEPDTMA SWVTHPPQTS
        11710        11720        11730        11740        11750        11760
TPVSRTTSSF SHSSPDATPV MATSPRTEAS SAVLTTISPG APEMVTSQIT SSGAATSTTV
        11770        11780        11790        11800        11810        11820
PTLTHSPGMP ETTALLSTHP RTGTSKTFPA STVFPQVSET TASLTIRPGA ETSTALPTQT
        11830        11840        11850        11860        11870        11880
TSSLFTLLVT GTSRVDLSPT ASPGVSAKTA PLSTHPGTET STMIPTSTLS LGLLETTGLL
        11890        11900        11910        11920        11930        11940
ATSSSAETST STLTLTVSPA VSGLSSASIT TDKPQTVTSW NTETSPSVTS VGPPEFSRTV
```

```
        11950      11960      11970      11980      11990      12000

TGTTMTLIPS EMPTPPKTSH GEGVSPTTIL RTTMVEATNL ATTGSSPTVA KTTTTFNTLA

        12010      12020      12030      12040      12050      12060

GSLFTPLTTP GMSTLASESV TSRTSYNHRS WISTTSSYNR RYWTPATSTP VTSTFSPGIS

        12070      12080      12090      12100      12110      12120

TSSIPSSTAA TVPFMVPFTL NFTITNLQYE EDMRHPGSRK FNATERELQG LLKPLFRNSS

        12130      12140      12150      12160      12170      12180

LEYLYSGCRL ASLRPEKDSS AMAVDAICTH RPDPEDLGLD RERLYWELSN LTNGIQELGP

        12190      12200      12210      12220      12230      12240

YTLDRNSLYV NGFTHRSSMP TTSTPGTSTV DVGTSGTPSS SPSPTAAGPL LMPFTLNFTI

        12250      12260      12270      12280      12290      12300

TNLQYEEDMR RTGSRKFNTM ESVLQGLLKP LFKNTSVGPL YSGCRLTLLR PEKDGAATGV

        12310      12320      12330      12340      12350      12360

DAICTHRLDP KSPGLNREQL YWELSKLTND IEELGPYTLD RNSLYVNGFT HQSSVSTTST

        12370      12380      12390      12400      12410      12420

PGTSTVDLRT SGTPSSLSSP TIMAAGPLLV PFTLNFTITN LQYGEDMGHP GSRKFNTTER

        12430      12440      12450      12460      12470      12480

VLQGLLGPIF KNTSVGPLYS GCRLTSLRSE KDGAATGVDA ICIHHLDPKS PGLNRERLYW

        12490      12500      12510      12520      12530      12540

ELSQLTNGIK ELGPYTLDRN SLYVNGFTHR TSVPTTSTPG TSTVDLGTSG TPFSLPSPAT

        12550      12560      12570      12580      12590      12600

AGPLLVLFTL NFTITNLKYE EDMHRPGSRK FNTTERVLQT LLGPMFKNTS VGLLYSGCRL

        12610      12620      12630      12640      12650      12660

TLLRSEKDGA ATGVDAICTH RLDPKSPGLD REQLYWELSQ LTNGIKELGP YTLDRNSLYV

        12670      12680      12690      12700      12710      12720

NGFTHWIPVP TSSTPGTSTV DLGSGTPSSL PSPTAAGPLL VPFTLNFTIT NLQYEEDMHH

        12730      12740      12750      12760      12770      12780

PGSRKFNTTE RVLQGLLGPM FKNTSVGLLY SGCRLTLLRS EKDGAATGVD AICTHRLDPK
```

```
       12790       12800       12810       12820       12830       12840

SPGVDREQLY  WELSQLTNGI  KELGPYTLDR  NSLYVNGFTH  QTSAPNTSTP  GTSTVDLGTS

       12850       12860       12870       12880       12890       12900

GTPSSLPSPT  SAGPLLVPFT  LNFTITNLQY  EEDMRHPGSR  KFNTTERVLQ  GLLKPLFKST

       12910       12920       12930       12940       12950       12960

SVGPLYSGCR  LTLLRSEKDG  AATGVDAICT  HRLDPKSPGV  DREQLYWELS  QLTNGIKELG

       12970       12980       12990       13000       13010       13020

PYTLDRNSLY  VNGFTHQTSA  PNTSTPGTST  VDLGTSGTPS  SLPSPTSAGP  LLVPFTLNFT

       13030       13040       13050       13060       13070       13080

ITNLQYEEDM  HHPGSRKFNT  TERVLQGLLG  PMFKNTSVGL  LYSGCRLTLL  RPEKNGAATG

       13090       13100       13110       13120       13130       13140

MDAICSHRLD  PKSPGLNREQ  LYWELSQLTH  GIKELGPYTL  DRNSLYVNGF  THRSSVAPTS

       13150       13160       13170       13180       13190       13200

TPGTSTVDLG  TSGTPSSLPS  PTTAVPLLVP  FTLNFTITNL  QYGEDMRHPG  SRKFNTTERV

       13210       13220       13230       13240       13250       13260

LQGLLGPLFK  NSSVGPLYSG  CRLISLRSEK  DGAATGVDAI  CTHHLNPQSP  GLDREQLYWQ

       13270       13280       13290       13300       13310       13320

LSQMTNGIKE  LGPYTLDRNS  LYVNGFTHRS  SGLTTSTPWT  STVDLGTSGT  PSPVPSPTTA

       13330       13340       13350       13360       13370       13380

GPLLVPFTLN  FTITNLQYEE  DMHRPGSRKF  NTTERVLQGL  LSPIFKNSSV  GPLYSGCRLT

       13390       13400       13410       13420       13430       13440

SLRPEKDGAA  TGMDAVCLYH  PNPKRPGLDR  EQLYWELSQL  THNITELGPY  SLDRDSLYVN

       13450       13460       13470       13480       13490       13500

GFTHQNSVPT  TSTPGTSTVY  WATTGTPSSF  PGHTEPGPLL  IPFTFNFTIT  NLHYEENMQH

       13510       13520       13530       13540       13550       13560

PGSRKFNTTE  RVLQGLLKPL  FKNTSVGPLY  SGCRLTSLRP  EKDGAATGMD  AVCLYHPNPK

       13570       13580       13590       13600       13610       13620

RPGLDREQLY  WELSQLTHNI  TELGPYSLDR  DSLYVNGFTH  QNSVPTTSTP  GTSTVYWATT
```

```
        13630       13640       13650       13660       13670       13680
GTPSSFPGHT  EPGPLLIPFT  FNFTITNLHY  EENMQHPGSR  KFNTTERVLQ  GLLKPLFKNT
        13690       13700       13710       13720       13730       13740
SVGPLYSGCR  LTLLRPEKHE  AATGVDTICT  HRVDPIGPGL  DRERLYWELS  QLTNSITELG
        13750       13760       13770       13780       13790       13800
PYTLDRDSLY  VNGFNPRSSV  PTTSTPGTST  VHLATSGTPS  SLPGHTAPVP  LLIPFTLNFT
        13810       13820       13830       13840       13850       13860
ITNLHYEENM  QHPGSRKFNT  TERVLQGLLK  PLFKNTSVGP  LYSGCRLTLL  RPEKHEAATG
        13870       13880       13890       13900       13910       13920
VDTICTHRVD  PIGPGLXXEX  LYWELSXLTX  XIXELGPYTL  DRXSLYVNGF  THXXSXPTTS
        13930       13940       13950       13960       13970       13980
TPGTSTVXXG  TSGTPSSXPX  XTSAGPLLVP  FTLNFTITNL  QYEEDMHHPG  SRKFNTTERV
        13990       14000       14010       14020       14030       14040
LQGLLGPMFK  NTSVGLLYSG  CRLTLLRPEK  NGAATGMDAI  CSHRLDPKSP  GLDREQLYWE
        14050       14060       14070       14080       14090       14100
LSQLTHGIKE  LGPYTLDRNS  LYVNGFTHRS  SVAPTSTPGT  STVDLGTSGT  PSSLPSPTTA
        14110       14120       14130       14140       14150       14160
VPLLVPFTLN  FTITNLQYGE  DMRHPGSRKF  NTTERVLQGL  LGPLFKNSSV  GPLYSGCRLI
        14170       14180       14190       14200       14210       14220
SLRSEKDGAA  TGVDAICTHH  LNPQSPGLDR  EQLYWQLSQM  TNGIKELGPY  TLDRNSLYVN
        14230       14240       14250       14260       14270       14280
GFTHRSSGLT  TSTPWTSTVD  LGTSGTPSPV  PSPTTAGPLL  VPFTLNFTIT  NLQYEEDMHR
        14290       14300       14310       14320       14330       14340
PGSRKFNATE  RVLQGLLSPI  FKNSSVGPLY  SGCRLTSLRP  EKDGAATGMD  AVCLYHPNPK
        14350       14360       14370       14380       14390       14400
RPGLDREQLY  WELSQLTHNI  TELGPYSLDR  DSLYVNGFTH  QSSMTTTRTP  DTSTMHLATS
        14410       14420       14430       14440       14450       14460
RTPASLSGPT  TASPLLVLFT  INCTITNLQY  EEDMRRTGSR  KFNTMESVLQ  GLLKPLFKNT
```

```
     14470      14480      14490      14500      14510      14520

SVGPLYSGCR LTLLRPKKDG AATGVDAICT HRLDPKSPGL NREQLYWELS KLTNDIEELG

     14530      14540      14550      14560      14570      14580

PYTLDRNSLY VNGFTHQSSV STTSTPGTST VDLRTSGTPS SLSSPTIMXX XPLLXPFTXN

     14590      14600      14610      14620      14630      14640

XTITNLXXXX XMXXPGSRKF NTTERVLQGL LRPLFKNTSV SSLYSGCRLT LLRPEKDGAA

     14650      14660      14670      14680      14690      14700

TRVDAACTYR PDPKSPGLDR EQLYWELSQL THSITELGPY TLDRVSLYVN GFNPRSSVPT

     14710      14720      14730      14740      14750      14760

TSTPGTSTVH LATSGTPSSL PGHTXXXPLL XPFTXNXTIT NLXXXXXMXX PGSRKFNTTE

     14770      14780      14790      14800      14810      14820

RVLQGLLKPL FRNSSLEYLY SGCRLASLRP EKDSSAMAVD AICTHRPDPE DLGLDRERLY

     14830      14840      14850      14860      14870      14880

WELSNLTNGI QELGPYTLDR NSLYVNGFTH RSSGLTTSTP WTSTVDLGTS GTPSPVPSPT

     14890      14900      14910      14920      14930      14940

TAGPLLVPFT LNFTITNLQY EEDMHRPGSR RFNTTERVLQ GLLTPLFKNT SVGPLYSGCR

     14950      14960      14970      14980      14990      15000

LTLLRPEKQE AATGVDTICT HRVDPIGPGL DRERLYWELS QLTNSITELG PYTLDRDSLY

     15010      15020      15030      15040      15050      15060

VNGFNPWSSV PTTSTPGTST VHLATSGTPS SLPGHTAPVP LLIPFTLNFT ITDLHYEENM

     15070      15080      15090      15100      15110      15120

QHPGSRKFNT TERVLQGLLK PLFKSTSVGP LYSGCRLTLL RPEKHGAATG VDAICTLRLD

     15130      15140      15150      15160      15170      15180

PTGPGLDRER LYWELSQLTN SVTELGPYTL DRDSLYVNGF THRSSVPTTS IPGTSAVHLE

     15190      15200      15210      15220      15230      15240

TSGTPASLPG HTAPGPLLVP FTLNFTITNL QYEEDMRHPG SRKFSTTERV LQGLLKPLFK

     15250      15260      15270      15280      15290      15300

NTSVSSLYSG CRLTLLRPEK DGAATRVDAV CTHRPDPKSP GLDRERLYWK LSQLTHGITE
```

40

```
      15310      15320      15330      15340      15350      15360

LGPYTLDRHS LYVNGFTHQS SMTTTRTPDT STMHLATSRT PASLSGPTTA SPLLVLFTIN

      15370      15380      15390      15400      15410      15420

FTITNLRYEE NMHHPGSRKF NTTERVLQGL LRPVFKNTSV GPLYSGCRLT TLRPKKDGAA

      15430      15440      15450      15460      15470      15480

TKVDAICTYR PDPKSPGLDR EQLYWELSQL THSITELGPY TQDRDSLYVN GFTHRSSVPT

      15490      15500      15510      15520      15530      15540

TSIPGTSAVH LETSGTPASL PGHTAPGPLL VPFTLNFTIT NLQYEEDMRH PGSRKFNTTE

      15550      15560      15570      15580      15590      15600

RVLQGLLKPL FKSTSVGPLY SGCRLTLLRP EKRGAATGVD TICTHRLDPL NPGLDREQLY

      15610      15620      15630      15640      15650      15660

WELSKLTRGI IELGPYLLDR GSLYVNGFTH RTSVPTTSTP GTSTVDLGTS GTPFSLPSPA

      15670      15680      15690      15700      15710      15720

XXXPLLXPFT XNXTITNLXX XXXMXXPGSR KFNTTERVLQ TLLGPMFKNT SVGLLYSGCR

      15730      15740      15750      15760      15770      15780

LTLLRSEKDG AATGVDAICT HRLDPKSPGV DREQLYWELS QLTNGIKELG PYTLDRNSLY

      15790      15800      15810      15820      15830      15840

VNGFTHWIPV PTSSTPGTST VDLGSGTPSS LPSPTTAGPL LVPFTLNFTI TNLKYEEDMH

      15850      15860      15870      15880      15890      15900

CPGSRKFNTT ERVLQSLLGP MFKNTSVGPL YSGCRLTLLR SEKDGAATGV DAICTHRLDP

      15910      15920      15930      15940      15950      15960

KSPGVDREQL YWELSQLTNG IKELGPYTLD RNSLYVNGFT HQTSAPNTST PGTSTVDLGT

      15970      15980      15990      16000      16010      16020

SGTPSSLPSP TXXXPLLXPF TXNXTITNLX XXXXMXXPGS RKFNTTEXVL QGLLXPXFKN

      16030      16040      16050      16060      16070      16080

XSVGXLYSGC RLTXLRXEKX GAATGXDAIC XHXXXPKXPG LXXEXLYWEL SXLTXXIXEL

      16090      16100      16110      16120      16130      16140

GPYTLDRXSL YVNGFTHWIP VPTSSTPGTS TVDLGSGTPS SLPSPTTAGP LLVPFTLNFT
```

41

```
        16150       16160       16170       16180       16190       16200
ITNLKYEEDM  HCPGSRKFNT  TERVLQSLLG  PMFKNTSVGP  LYSGCRLTSL  RSEKDGAATG
        16210       16220       16230       16240       16250       16260
VDAICTHRVD  PKSPGVDREQ  LYWELSQLTN  GIKELGPYTL  DRNSLYVNGF  THQTSAPNTS
        16270       16280       16290       16300       16310       16320
TPGTSTVXXG  TSGTPSSXPX  XTSAGPLLVP  FTLNFTITNL  QYEEDMHHPG  SRKFNTTERV
        16330       16340       16350       16360       16370       16380
LQGLLGPMFK  NTSVGLLYSG  CRLTLLRPEK  NGATTGMDAI  CTHRLDPKSP  GLXXEXLYWE
        16390       16400       16410       16420       16430       16440
LSXLTXXIXE  LGPYTLDRXS  LYVNGFTHXX  SXPTTSTPGT  STVXXGTSGT  PSSXPXXTXX
        16450       16460       16470       16480       16490       16500
XPLLXPFTXN  XTITNLXXXX  XMXXPGSRKF  NTTERVLQGL  LKPLFRNSSL  EYLYSGCRLA
        16510       16520       16530       16540       16550       16560
SLRPEKDSSA  MAVDAICTHR  PDPEDLGLDR  ERLYWELSNL  TNGIQELGPY  TLDRNSLYVN
        16570       16580       16590       16600       16610       16620
GFTHRSSMPT  TSTPGTSTVD  VGTSGTPSSS  PSPTTAGPLL  IPFTLNFTIT  NLQYGEDMGH
        16630       16640       16650       16660       16670       16680
PGSRKFNTTE  RVLQGLLGPI  FKNTSVGPLY  SGCRLTSLRS  EKDGAATGVD  AICIHHLDPK
        16690       16700       16710       16720       16730       16740
SPGLNRERLY  WELSQLTNGI  KELGPYTLDR  NSLYVNGFTH  RTSVPTTSTP  GTSTVDLGTS
        16750       16760       16770       16780       16790       16800
GTPFSLPSPA  TAGPLLVLFT  LNFTITNLKY  EEDMHRPGSR  KFNTTERVLQ  TLLGPMFKNT
        16810       16820       16830       16840       16850       16860
SVGLLYSGCR  LTLLRSEKDG  AATGVDAICT  HRLDPKSPGL  XXEXLYWELS  XLTXXIXELG
        16870       16880       16890       16900       16910       16920
PYTLDRXSLY  VNGFTHXXSX  PTTSTPGTST  VXXGTSGTPS  SXPXXTXXXP  LLXPFTXNXT
        16930       16940       16950       16960       16970       16980
ITNLXXXXXM  XXPGSRKFNT  TERVLQGLLR  PVFKNTSVGP  LYSGCRLTLL  RPKKDGAATK
```

```
        16990        17000        17010        17020        17030        17040

VDAICTYRPD  PKSPGLDREQ  LYWELSQLTH  SITELGPYTQ  DRDSLYVNGF  THRSSVPTTS

        17050        17060        17070        17080        17090        17100

IPGTSAVHLE  TTGTPSSFPG  HTEPGPLLIP  FTFNFTITNL  RYEENMQHPG  SRKFNTTERV

        17110        17120        17130        17140        17150        17160

LQGLLTPLFK  NTSVGPLYSG  CRLTLLRPEK  QEAATGVDTI  CTHRVDPIGP  GLDRERLYWE

        17170        17180        17190        17200        17210        17220

LSQLTNSITE  LGPYTLDRDS  LYVDGFNPWS  SVPTTSTPGT  STVHLATSGT  PSPLPGHTAP

        17230        17240        17250        17260        17270        17280

VPLLIPFTLN  FTITDLHYEE  NMQHPGSRKF  NTTERVLQGL  LKPLFKSTSV  GPLYSGCRLT

        17290        17300        17310        17320        17330        17340

LLRPEKHGAA  TGVDAICTLR  LDPTGPGLDR  ERLYWELSQL  TNSITELGPY  TLDRDSLYVN

        17350        17360        17370        17380        17390        17400

GFNPWSSVPT  TSTPGTSTVH  LATSGTPSSL  PGHTTAGPLL  VPFTLNFTIT  NLKYEEDMHC

        17410        17420        17430        17440        17450        17460

PGSRKFNTTE  RVLQSLHGPM  FKNTSVGPLY  SGCRLTLLRS  EKDGAATGVD  AICTHRLDPK

        17470        17480        17490        17500        17510        17520

SPGLXXEXLY  WELSXLTXXI  XELGPYTLDR  XSLYVNGFTH  XXSXPTTSTP  GTSTVXXGTS

        17530        17540        17550        17560        17570        17580

GTPSSXPXXT  XXXPLLXPFT  XNXTITNLXX  XXXMXXPGSR  KFNTTEXVLQ  GLLXPXFKNX

        17590        17600        17610        17620        17630        17640

SVGXLYSGCR  LTXLRXEKXG  AATGXDAICX  HXXXPKXPGL  XXEXLYWELS  XLTNSITELG

        17650        17660        17670        17680        17690        17700

PYTLDRDSLY  VNGFTHRSSM  PTTSIPGTSA  VHLETSGTPA  SLPGHTAPGP  LLVPFTLNFT

        17710        17720        17730        17740        17750        17760

ITNLQYEEDM  RHPGSRKFNT  TERVLQGLLK  PLFKSTSVGP  LYSGCRLTLL  RPEKRGAATG

        17770        17780        17790        17800        17810        17820

VDTICTHRLD  PLNPGLXXEX  LYWELSXLTX  XIXELGPYTL  DRXSLYVNGF  THXXSXPTTS
```

```
        17830       17840       17850       17860       17870       17880
TPGTSTVXXG  TSGTPSSXPX  XTXXXPLLXP  FTXNXTITNL  XXXXXMXXPG  SRKFNTTEXV
        17890       17900       17910       17920       17930       17940
LQGLLXPXFK  NXSVGXLYSG  CRLTXLRXEK  XGAATGXDAI  CXHXXXPKXP  GLXXEXLYWE
        17950       17960       17970       17980       17990       18000
LSXLTXXIXE  LGPYTLDRXS  LYVNGFHPRS  SVPTTSTPGT  STVHLATSGT  PSSLPGHTAP
        18010       18020       18030       18040       18050       18060
VPLLIPFTLN  FTITNLHYEE  NMQHPGSRKF  NTTERVLQGL  LGPMFKNTSV  GLLYSGCRLT
        18070       18080       18090       18100       18110       18120
LLRPEKNGAA  TGMDAICSHR  LDPKSPGLXX  EXLYWELSXL  TXXIXELGPY  TLDRXSLYVN
        18130       18140       18150       18160       18170       18180
GFTHXXSXPT  TSTPGTSTVX  XGTSGTPSSX  PXXTXXXPLL  XPFTXNXTIT  NLXXXXXMXX
        18190       18200       18210       18220       18230       18240
PGSRKFNTTE  XVLQGLLXPX  FKNXSVGXLY  SGCRLTXLRX  EKXGAATGXD  AICXHXXXPK
        18250       18260       18270       18280       18290       18300
XPGLXXEXLY  WELSXLTXXI  XELGPYTLDR  XSLYVNGFTH  QNSVPTTSTP  GTSTVYWATT
        18310       18320       18330       18340       18350       18360
GTPSSFPGHT  EPGPLLIPFT  FNFTITNLHY  EENMQHPGSR  KFNTTERVLQ  GLLTPLFKNT
        18370       18380       18390       18400       18410       18420
SVGPLYSGCR  LTLLRPEKQE  AATGVDTICT  HRVDPIGPGL  XXEXLYWELS  XLTXXIXELG
        18430       18440       18450       18460       18470       18480
PYTLDRXSLY  VNGFTHXXSX  PTTSTPGTST  VXXGTSGTPS  SXPXXTXXXP  LLXPFTXNXT
        18490       18500       18510       18520       18530       18540
ITNLXXXXXM  XXPGSRKFNT  TEXVLQGLLX  PXFKNXSVGX  LYSGCRLTXL  RXEKXGAATG
        18550       18560       18570       18580       18590       18600
XDAICXHXXX  PKXPGLXXEX  LYWELSXLTX  XIXELGPYTL  DRXSLYVNGF  THRSSVPTTS
        18610       18620       18630       18640       18650       18660
SPGTSTVHLA  TSGTPSSLPG  HTAPVPLLIP  FTLNFTITNL  HYEENMQHPG  SRKFNTTERV
```

```
     18670       18680       18690       18700       18710       18720
LQGLLKPLFK  STSVGPLYSG  CRLTLLRPEK  HGAATGVDAI  CTLRLDPTGP  GLXXEXLYWE

     18730       18740       18750       18760       18770       18780
LSXLTXXIXE  LGPYTLDRXS  LYVNGFTHXX  SXPTTSTPGT  STVXXGTSGT  PSSXPXXTXX

     18790       18800       18810       18820       18830       18840
XPLLXPFTXN  XTITNLXXXX  XMXXPGSRKF  NTTEXVLQGL  LXPXFKNXSV  GXLYSGCRLT

     18850       18860       18870       18880       18890       18900
XLRXEKXGAA  TGXDAICXHX  XXPKXPGLXX  EXLYWELSXL  TXXIXELGPY  TLDRXSLYVN

     18910       18920       18930       18940       18950       18960
GFTHRTSVPT  TSTPGTSTVH  LATSGTPSSL  PGHTAPVPLL  IPFTLNFTIT  NLQYEEDMHR

     18970       18980       18990       19000       19010       19020
PGSRKFNTTE  RVLQGLLSPI  FKNSSVGPLY  SGCRLTSLRP  EKDGAATGMD  AVCLYHPNPK

     19030       19040       19050       19060       19070       19080
RPGLDREQLY  CELSQLTHNI  TELGPYSLDR  DSLYVNGFTH  QNSVPTTSTP  GTSTVYWATT

     19090       19100       19110       19120       19130       19140
GTPSSFPGHT  XXXPLLXPFT  XNXTITNLXX  XXXMXXPGSR  KFNTTEXVLQ  GLLXPXFKNX

     19150       19160       19170       19180       19190       19200
SVGXLYSGCR  LTXLRXEKXG  AATGXDAICX  HXXXPKXPGL  XXEXLYWELS  XLTXXIXELG

     19210       19220       19230       19240       19250       19260
PYTLDRXSLY  VNGFTHWSSG  LTTSTPWTST  VDLGTSGTPS  PVPSPTTAGP  LLVPFTLNFT

     19270       19280       19290       19300       19310       19320
ITNLQYEEDM  HRPGSRKFNA  TERVLQGLLS  PIFKNTSVGP  LYSGCRLTLL  RPEKQEAATG

     19330       19340       19350       19360       19370       19380
VDTICTHRVD  PIGPGLXXEX  LYWELSXLTX  XIXELGPYTL  DRXSLYVNGF  THXXSXPTTS

     19390       19400       19410       19420       19430       19440
TPGTSTVXXG  TSGTPSSXPX  XTXXXPLLXP  FTXNXTITNL  XXXXXMXXPG  SRKFNTTEXV

     19450       19460       19470       19480       19490       19500
LQGLLXPXFK  NXSVGXLYSG  CRLTXLRXEK  XGAATGXDAI  CXHXXXPKXP  GLXXEXLYWE
```

LSXLTXXIXE LGPYTLDRXS LYVNGFTHRS FGLTTSTPWT STVDLGTSGT PSPVPSPTTA

GPLLVPFTLN FTITNLQYEE DMHRPGSRKF NTTERVLQGL LTPLFRNTSV SSLYSGCRLT

LLRPEKDGAA TRVDAVCTHR PDPKSPGLXX EXLYWELSXL TXXIXELGPY TLDRXSLYVN

GFTHXXSXPT TSTPGTSTVX XGTSGTPSSX PXXTXXXPLL XPFTXNXTIT NLXXXXXMXX

PGSRKFNTTE XVLQGLLXPX FKNXSVGXLY SGCRLTXLRX EKXGAATGXD AICXHXXXPK

XPGLXXEXLY WELSXLTXXI XELGPYTLDR XSLYVNGFTH WIPVPTSSTP GTSTVDLGSG

TPSSLPSPTT AGPLLVPFTL NFTITNLQYG EDMGHPGSRK FNTTERVLQG LLGPIFKNTS

VGPLYSGCRL TSLRSEKDGA ATGVDAICIH HLDPKSPGLX XEXLYWELSX LTXXIXELGP

YTLDRXSLYV NGFTHXXSXP TTSTPGTSTV XXGTSGTPSS XPXXTXXXPL LXPFTXNXTI

TNLXXXXXMX XPGSRKFNTT EXVLQGLLXP XFKNXSVGXL YSGCRLTXLR XEKXGAATGX

DAICXHXXXP KXPGLXXEXL YWELSXLTXX IXELGPYTLD RXSLYVNGFT HQTFAPNTST

PGTSTVDLGT SGTPSSLPSP TSAGPLLVPF TLNFTITNLQ YEEDMHHPGS RKFNTTERVL

QGLLGPMFKN TSVGLLYSGC RLTLLRPEKN GAATRVDAVC THRPDPKSPG LXXEXLYWEL

SXLTXXIXEL GPYTLDRXSL YVNGFTHXXS XPTTSTPGTS TVXXGTSGTP SSXPXXTAPV

```
        20350        20360        20370        20380        20390        20400

PLLIPFTLNF   TITNLHYEEN   MQHPGSRKFN   TTERVLQGLL   KPLFKSTSVG   PLYSGCRLTL

        20410        20420        20430        20440        20450        20460

LRPEKHGAAT   GVDAICTLRL   DPTGPGLDRE   RLYWELSQLT   NSVTELGPYT   LDRDSLYVNG

        20470        20480        20490        20500        20510        20520

FTQRSSVPTT   SIPGTSAVHL   ETSGTPASLP   GHTAPGPLLV   PFTLNFTITN   LQYEVDMRHP

        20530        20540        20550        20560        20570        20580

GSRKFNTTER   VLQGLLKPLF   KSTSVGPLYS   GCRLTLLRPE   KRGAATGVDT   ICTHRLDPLN

        20590        20600        20610        20620        20630        20640

PGLDREQLYW   ELSKLTRGII   ELGPYLLDRG   SLYVNGFTHR   NFVPITSTPG   TSTVHLGTSE

        20650        20660        20670        20680        20690        20700

TPSSLPRPIV   PGPLLVPFTL   NFTITNLQYE   EAMRHPGSRK   FNTTERVLQG   LLRPLFKNTS

        20710        20720        20730        20740        20750        20760

IGPLYSSCRL   TLLRPEKDKA   ATRVDAICTH   HPDPQSPGLN   REQLYWELSQ   LTHGITELGP

        20770        20780        20790        20800        20810        20820

YTLDRDSLYV   DGFTHWSPIP   TTSTPGTSIV   NLGTSGIPPS   LPETTXXXPL   LXPFTXNXTI

        20830        20840        20850        20860        20870        20880

TNLXXXXXMX   XPGSRKFNTT   ERVLQGLLKP   LFKSTSVGPL   YSGCRLTLLR   PEKDGVATRV

        20890        20900        20910        20920        20930        20940

DAICTHRPDP   KIPGLDRQQL   YWELSQLTHS   ITELGPYTLD   RDSLYVNGFT   QRSSVPTTST

        20950        20960        20970        20980        20990        21000

PGTFTVQPET   SETPSSLPGP   TATGPVLLPF   TLNFTITNLQ   YEEDMHRPGS   RKFNTTERVL

        21010        21020        21030        21040        21050        21060

QGLLMPLFKN   TSVSSLYSGC   RLTLLRPEKD   GAATRVDAVC   THRPDPKSPG   LDRERLYWKL

        21070        21080        21090        21100        21110        21120

SQLTHGITEL   GPYTLDRHSL   YVNGFTHQSS   MTTTRTPDTS   TMHLATSRTP   ASLSGPTTAS

        21130        21140        21150        21160        21170        21180

PLLVLFTINF   TITNLRYEEN   MHHPGSRKFN   TTERVLQGLL   RPVFKNTSVG   PLYSGCRLTL
```

21190      21200      21210      21220      21230      21240

LRPKKDGAAT KVDAICTYRP DPKSPGLDRE QLYWELSQLT HSITELGPYT LDRDSLYVNG

21250      21260      21270      21280      21290      21300

FTQRSSVPTT SIPGTPTVDL GTSGTPVSKP GPSAASPLLV LFTLNFTITN LRYEENMQHP

21310      21320      21330      21340      21350      21360

GSRKFNTTER VLQGLLRSLF KSTSVGPLYS GCRLTLLRPE KDGTATGVDA ICTHHPDPKS

21370      21380      21390      21400      21410      21420

PRLDREQLYW ELSQLTHNIT ELGHYALDND SLFVNGFTHR SSVSTTSTPG TPTVYLGASK

21430      21440      21450      21460      21470      21480

TPASIFGPSA ASHLLILFTL NFTITNLRYE ENMWPGSRKF NTTERVLQGL LRPLFKNTSV

21490      21500      21510      21520      21530      21540

GPLYSGSRLT LLRPEKDGEA TGVDAICTHR PDPTGPGLDR EQLYLELSQL THSITELGPY

21550      21560      21570      21580      21590      21600

TLDRDSLYVN GFTHRSSVPT TSTGVVSEEP FTLNFTINNL RYMADMGQPG SLKFNITDNV

21610      21620      21630      21640      21650      21660

MKHLLSPLFQ RSSLGARYTG CRVIALRSVK NGAETRVDLL CTYLQPLSGP GLPIKQVFHE

21670      21680      21690      21700      21710      21720

LSQQTHGITR LGPYSLDKDS LYLNGYNEPG LDEPPTTPKP ATTFLPPLSE ATTAMGYHLK

21730      21740      21750      21760      21770      21780

TLTLNFTISN LQYSPDMGKG SATFNSTEGV LQHLLRPLFQ KSSMGPFYLG CQLISLRPEK

21790      21800      21810      21820      21830      21840

DGAATGVDTT CTYHPDPVGP GLDIQQLYWE LSQLTHGVTQ LGFYVLDRDS LFINGYAPQN

21850      21860      21870      21880      21890      21900

LSIRGEYQIN FHIVNWNLSN PDPTSSEYIT LLRDIQDKVT TLYKGSQLHD TFRFCLVTNL

21910      21920      21930      21940      21950      21960

TMDSVLVTVK ALFSSNLDPS LVEQVFLDKT LNASFHWLGS TYQLVDIHVT EMESSVYQPT

21970      21980      21990      22000      22010      22020

SSSSTQHFYL NFTITNLPYS QDKAQPGTTN YQRNKRNIED ALNQLFRNSS IKSYFSDCQV

|  |  |  |  |  |  |
|---|---|---|---|---|---|
| 22030 | 22040 | 22050 | 22060 | 22070 | 22080 |

STFRSVPNRH HTGVDSLCNF SPLARRVDRV AIYEEFLRMT RNGTQLQNFT LDRSSVLVDG

|  |  |  |  |  |  |
|---|---|---|---|---|---|
| 22090 | 22100 | 22110 | 22120 | 22130 | 22140 |

YSPNRNEPLT GNSDLPFWAV ILIGLAGLLG LITCLICGVL VTTRRRKKEG EYNVQQQCPG

22150

YYQSHLDLED LQ

Most preferably, the Mucin-16 assay detects one or more soluble forms of Mucin-16. Mucin-16 is a single-pass type I membrane protein having a large extracellular domain, most or all of which is present in soluble forms of Mucin-16 generated either through alternative splicing event which deletes all or a portion of the transmembrane domain, or by proteolysis of the membrane-bound form. In the case of an immunoassay, one or more antibodies that bind to epitopes within this extracellular domain may be used to detect these soluble form(s). The following domains have been identified in Mucin-16:

| Residues | Length | Domain ID |
|---|---|---|
| 1-22152 | 22152 | Mucin-16 |
| 1-22096 | 22906 | extracellular |
| 22907-22117 | 21 | transmembrane |
| 22128-22152 | 35 | cytoplasmic |

As used herein, the term "Carcinoembryonic antigen-related cell adhesion molecule 5" refers to one or polypeptides present in a biological sample that are derived from the Carcinoembryonic antigen-related cell adhesion molecule 5 precursor (Swiss-Prot P06731 (SEQ ID NO: 13)).

|  |  |  |  |  |  |
|---|---|---|---|---|---|
| 10 | 20 | 30 | 40 | 50 | 60 |

MESPSAPPHR WCIPWQRLLL TASLLTFWNP PTTAKLTIES TPFNVAEGKE VLLLVHNLPQ

|  |  |  |  |  |  |
|---|---|---|---|---|---|
| 70 | 80 | 90 | 100 | 110 | 120 |

HLFGYSWYKG ERVDGNRQII GYVIGTQQAT PGPAYSGREI IYPNASLLIQ NIIQNDTGFY

|  |  |  |  |  |  |
|---|---|---|---|---|---|
| 130 | 140 | 150 | 160 | 170 | 180 |

TLHVIKSDLV NEEATGQFRV YPELPKPSIS SNNSKPVEDK DAVAFTCEPE TQDATYLWWV

|  |  |  |  |  |  |
|---|---|---|---|---|---|
| 190 | 200 | 210 | 220 | 230 | 240 |

```
NNQSLPVSPR LQLSNGNRTL TLFNVTRNDT ASYKCETQNP VSARRSDSVI LNVLYGPDAP
           250        260        270        280        290        300

TISPLNTSYR SGENLNLSCH AASNPPAQYS WFVNGTFQQS TQELFIPNIT VNNSGSYTCQ
           310        320        330        340        350        360

AHNSDTGLNR TTVTTITVYA EPPKPFITSN NSNPVEDEDA VALTCEPEIQ NTTYLWWVNN
           370        380        390        400        410        420

QSLPVSPRLQ LSNDNRTLTL LSVTRNDVGP YECGIQNELS VDHSDPVILN VLYGPDDPTI
           430        440        450        460        470        480

SPSYTYYRPG VNLSLSCHAA SNPPAQYSWL IDGNIQQHTQ ELFISNITEK NSGLYTCQAN
           490        500        510        520        530        540

NSASGHSRTT VKTITVSAEL PKPSISSNNS KPVEDKDAVA FTCEPEAQNT TYLWWVNGQS
           550        560        570        580        590        600

LPVSPRLQLS NGNRTLTLFN VTRNDARAYV CGIQNSVSAN RSDPVTLDVL YGPDTPIISP
           610        620        630        640        650        660

PDSSYLSGAN LNLSCHSASN PSPQYSWRIN GIPQQHTQVL FIAKITPNNN GTYACFVSNL
           670        680        690        700

ATGRNNSIVK SITVSASGTS PGLSAGATVG IMIGVLVGVA LI
```

The following domains have been identified in Carcinoembryonic antigen-related cell adhesion molecule 5:

| Residues | Length | Domain ID |
|----------|--------|-----------|
| 1-34 | 34 | Signal sequence |
| 35-685 | 5 | Carcinoembryonic antigen-related cell adhesion molecule 5 |
| 686-702 | 17 | Propeptide |

As used herein, the term "cellular tumor antigen p53" refers to one or more polypeptides present in a biological sample that are derived from the cellular tumor antigen p53 precursor (Swiss-Prot P04637 (SEQ ID NO: 14)).

```
           10         20         30         40         50         60

MEEPQSDPSV EPPLSQETFS DLWKLLPENN VLSPLPSQAM DDLMLSPDDI EQWFTEDPGP
```

```
        70          80          90         100         110         120

  DEAPRMPEAA  PRVAPAPAAP  TPAAPAPAPS  WPLSSSVPSQ  KTYQGSYGFR  LGFLHSGTAK

       130         140         150         160         170         180

  SVTCTYSPAL  NKMFCQLAKT  CPVQLWVDST  PPPGTRVRAM  AIYKQSQHMT  EVVRRCPHHE

       190         200         210         220         230         240

  RCSDSDGLAP  PQHLIRVEGN  LRVEYLDDRN  TFRHSVVVPY  EPPEVGSDCT  TIHYNYMCNS

       250         260         270         280         290         300

  SCMGGMNRRP  ILTIITLEDS  SGNLLGRNSF  EVRVCACPGR  DRRTEEENLR  KKGEPHHELP

       310         320         330         340         350         360

  PGSTKRALPN  NTSSSPQPKK  KPLDGEYFTL  QIRGRERFEM  FRELNEALEL  KDAQAGKEPG

       370         380         390

  GSRAHSSHLK  SKKGQSTSRH  KKLMFKTEGP  DSD
```

Isoform 2 of cellular tumor antigen p53 has the following changes from this isoform 1 sequence:

332-341: IRGRERFEMF (SEQ ID NO: 15) → DGTSFQKENC (SEQ ID NO: 16)

342-393: Missing

As used herein, the term "relating a signal to the presence or amount" of an analyte reflects this understanding. Assay signals are typically related to the presence or amount of an analyte through the use of a standard curve calculated using known concentrations of the analyte of interest. As the term is used herein, an assay is "configured to detect" an analyte if an assay can generate a detectable signal indicative of the presence or amount of a physiologically relevant concentration of the analyte. Because an antibody epitope is on the order of 8 amino acids, an immunoassay configured to detect a marker of interest will also detect polypeptides related to the marker sequence, so long as those polypeptides contain the epitope(s) necessary to bind to the antibody or antibodies used in the assay. The term "related marker" as used herein with regard to a biomarker such as one of the kidney injury markers described herein refers to one or more fragments, variants, etc., of a particular marker or its biosynthetic parent that may be detected as a surrogate for the marker itself or as independent biomarkers. The term also refers to one or more polypeptides present in a biological sample that are derived from the biomarker precursor complexed to additional species, such as binding proteins, receptors, heparin, lipids, sugars, *etc.*

In this regard, the skilled artisan will understand that the signals obtained from an immunoassay are a direct result of complexes formed between one or more antibodies and the target biomolecule (*i.e.*, the analyte) and polypeptides containing the necessary epitope(s) to which the antibodies bind. While such assays may detect the full length biomarker and the assay result be expressed as a concentration of a biomarker of interest, the signal from the assay is actually a result of all such "immunoreactive" polypeptides present in the sample. Expression of biomarkers may also be determined by means other than immunoassays, including protein measurements (such as dot blots, western blots, chromatographic methods, mass spectrometry, *etc.*) and nucleic acid measurements (mRNA quatitation). This list is not meant to be limiting.

The term "positive going" marker as that term is used herein refer to a marker that is determined to be elevated in subjects suffering from a disease or condition, relative to subjects not suffering from that disease or condition. The term "negative going" marker as that term is used herein refer to a marker that is determined to be reduced in subjects suffering from a disease or condition, relative to subjects not suffering from that disease or condition.

The term "subject" as used herein refers to a human or non-human organism. Thus, the methods and compositions described herein are applicable to both human and veterinary disease. Further, while a subject is preferably a living organism, the invention described herein may be used in post-mortem analysis as well. Preferred subjects are humans, and most preferably "patients," which as used herein refers to living humans that are receiving medical care for a disease or condition. This includes persons with no defined illness who are being investigated for signs of pathology.

Preferably, an analyte is measured in a sample. Such a sample may be obtained from a subject, or may be obtained from biological materials intended to be provided to the subject. For example, a sample may be obtained from a kidney being evaluated for possible transplantation into a subject, and an analyte measurement used to evaluate the kidney for preexisting damage. Preferred samples are body fluid samples.

The term "body fluid sample" as used herein refers to a sample of bodily fluid obtained for the purpose of diagnosis, prognosis, classification or evaluation of a subject of interest, such as a patient or transplant donor. In certain embodiments, such a sample may be obtained for the purpose of determining the outcome of an ongoing condition or the effect of a treatment regimen on a condition. Preferred body fluid samples include blood, serum, plasma, cerebrospinal fluid, urine, saliva, sputum, and pleural effusions. In addition, one of skill in the art would realize that certain body fluid samples would be more readily analyzed following a fractionation or purification procedure, for example, separation of whole blood into serum or plasma components.

The term "diagnosis" as used herein refers to methods by which the skilled artisan can estimate and/or determine the probability ("a likelihood") of whether or not a patient is suffering from a given disease or condition. In the case of the present invention, "diagnosis" includes using the results of an assay, most preferably an immunoassay, for a kidney injury marker of the present invention, optionally together with other clinical characteristics, to arrive at a diagnosis (that is, the occurrence or nonoccurrence) of an acute renal injury or ARF for the subject from which a sample was obtained and assayed. That such a diagnosis is "determined" is not meant to imply that the diagnosis is 100% accurate. Many biomarkers are indicative of multiple conditions. The skilled clinician does not use biomarker results in an informational vacuum, but rather test results are used together with other clinical indicia to arrive at a diagnosis. Thus, a measured biomarker level on one side of a predetermined diagnostic threshold indicates a greater likelihood of the occurrence of disease in the subject relative to a measured level on the other side of the predetermined diagnostic threshold.

Similarly, a prognostic risk signals a probability ("a likelihood") that a given course or outcome will occur. A level or a change in level of a prognostic indicator, which in turn is associated with an increased probability of morbidity (e.g., worsening renal function, future ARF, or death) is referred to as being "indicative of an increased likelihood" of an adverse outcome in a patient.

Marker Assays

In general, immunoassays involve contacting a sample containing or suspected of containing a biomarker of interest with at least one antibody that specifically binds to the biomarker. A signal is then generated indicative of the presence or amount of complexes formed by the binding of polypeptides in the sample to the antibody or other binding species. The signal is then related to the presence or amount of the biomarker in the sample. Numerous methods and devices are well known to the skilled artisan for the detection and analysis of biomarkers. *See, e.g.,* U.S. Patents 6,143,576; 6,113,855; 6,019,944; 5,985,579; 5,947,124; 5,939,272; 5,922,615; 5,885,527; 5,851,776; 5,824,799; 5,679,526; 5,525,524; and 5,480,792, and The Immunoassay Handbook, David Wild, ed. Stockton Press, New York, 1994.

The assay devices and methods known in the art can utilize labeled molecules in various sandwich, competitive, or non-competitive assay formats, to generate a signal that is related to the presence or amount of the biomarker of interest. Suitable assay formats also include chromatographic, mass spectrographic, and protein "blotting" methods. Additionally, certain methods and devices, such as biosensors and optical immunoassays, may be employed to determine the presence or amount of analytes without the need for a labeled molecule. *See, e.g.,* U.S. Patents 5,631,171; and 5,955,377. One skilled in the art also recognizes that robotic instrumentation including but not limited to Beckman ACCESS®, Abbott AXSYM®, Roche ELECSYS®, Dade Behring STRATUS® systems are among the immunoassay analyzers that are capable of performing immunoassays. But any suitable immunoassay may be utilized, for example, enzyme-linked immunoassays (ELISA), radioimmunoassays (RIAs), competitive binding assays, and the like.

Antibodies or other polypeptides may be immobilized onto a variety of solid supports for use in assays. Solid phases that may be used to immobilize specific binding members include include those developed and/or used as solid phases in solid phase binding assays. Examples of suitable solid phases include membrane filters, cellulose-based papers, beads (including polymeric, latex and paramagnetic particles), glass, silicon wafers, microparticles, nanoparticles, TentaGels, AgroGels, PEGA gels, SPOCC gels, and multiple-well plates. An assay strip could be prepared by coating the antibody or a plurality of antibodies in an array on solid support. This strip could then be dipped into the test sample and then processed quickly through washes and detection steps to generate a measurable signal, such as a colored spot. Antibodies or other polypeptides may be bound to specific zones of assay devices either by conjugating directly to an assay device surface, or by indirect binding. In an example of the later case, antibodies or other polypeptides may be immobilized on particles or other solid supports, and that solid support immobilized to the device surface.

Biological assays require methods for detection, and one of the most common methods for quantitation of results is to conjugate a detectable label to a protein or nucleic acid that has affinity for one of the components in the biological system being studied. Detectable labels may include molecules that are themselves detectable (*e.g.*, fluorescent moieties, electrochemical labels, metal chelates, *etc.*) as well as molecules that may be indirectly detected by production of a detectable reaction product (*e.g.*, enzymes such as horseradish peroxidase, alkaline phosphatase, *etc.*) or by a specific binding molecule which itself may be detectable (e.g., biotin, digoxigenin, maltose, oligohistidine, 2,4-dintrobenzene, phenylarsenate, ssDNA, dsDNA, etc.).

Preparation of solid phases and detectable label conjugates often comprise the use of chemical cross-linkers. Cross-linking reagents contain at least two reactive groups, and are divided generally into homo functional cross-linkers (containing identical reactive groups) and heterofunctional cross-linkers (containing non-identical reactive groups). Homobifunctional cross-linkers that couple through amines, sulfhydryls or react non-specifically are available from many commercial sources. Maleimides, alkyl and aryl halides, alpha-haloacyls and pyridyl disulfides are thiol reactive groups. Maleimides, alkyl and aryl halides, and alpha-haloacyls react with sulfhydryls to form thiol ether bonds, while pyridyl disulfides react with sulfhydryls to produce mixed disulfides. The pyridyl disulfide product is cleavable. Imidoesters are also very useful for protein-protein cross-links. A variety of heterobifunctional cross-linkers, each combining different attributes for successful conjugation, are commercially available.

In certain aspects, the present invention provides kits for the analysis of the described kidney injury markers. The kit comprises reagents for the analysis of at least one test sample which comprise at least one antibody that a kidney injury marker. The kit can also include devices and instructions for performing one or more of the diagnostic and/or prognostic correlations described herein. Preferred kits will comprise an antibody pair for performing a sandwich assay, or a labeled species for performing a competitive assay, for the analyte. Preferably, an antibody pair comprises a first antibody conjugated to a solid phase and a second antibody conjugated to a detectable label, wherein each of the first and second antibodies that bind a kidney injury marker. Most preferably each of the antibodies are monoclonal antibodies. The instructions for use of the kit and performing the correlations can be in the form of labeling, which refers to any written or recorded material that is attached to, or otherwise accompanies a kit at any time during its manufacture, transport, sale or use. For example, the term labeling encompasses advertising leaflets and brochures, packaging materials, instructions, audio or video cassettes, computer discs, as well as writing imprinted directly on kits.

Antibodies

The term "antibody" as used herein refers to a peptide or polypeptide derived from, modeled after or substantially encoded by an immunoglobulin gene or immunoglobulin genes, or fragments thereof, capable of specifically binding an antigen or epitope. *See, e.g.* Fundamental Immunology, 3rd Edition, W.E. Paul, ed., Raven Press, N.Y. (1993); Wilson (1994; J. Immunol. Methods 175:267-273; Yarmush (1992) J. Biochem. Biophys. Methods 25:85-97. The term antibody includes antigen-binding portions, i.e., "antigen binding sites," (e.g., fragments, subsequences, complementarity determining regions (CDRs)) that retain capacity to bind antigen, including (i) a Fab fragment, a monovalent fragment consisting of the VL, VH, CL and CHI domains; (ii) a F(ab')2 fragment, a bivalent fragment comprising two Fab fragments linked by a disulfide bridge at the hinge region; (iii) a Fd fragment consisting of the VH and CHI domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR). Single chain antibodies are also included by reference in the term "antibody."

Antibodies used in the immunoassays described herein preferably specifically bind to a kidney injury marker of the present invention. The term "specifically binds" is not intended to indicate that an antibody binds exclusively to its intended target since, as noted above, an antibody binds to any polypeptide displaying the epitope(s) to which the antibody binds. Rather, an antibody "specifically binds" if its affinity for its intended target is about 5-fold greater when compared to its affinity for a non-target molecule which does not display the appropriate epitope(s). Preferably the affinity of the antibody will be at least about 5 fold, preferably 10 fold, more preferably 25-fold, even more preferably 50-fold, and most preferably 100-fold or more, greater for a target molecule than its affinity for a non-target molecule. In preferred embodiments, Preferred antibodies bind with affinities of at least about $10^7$ M$^{-1}$, and preferably between about $10^8$ M$^{-1}$ to about $10^9$ M$^{-1}$, about $10^9$ M$^{-1}$ to about $10^{10}$ M$^{-1}$, or about $10^{10}$ M$^{-1}$ to about $10^{12}$ M$^{-1}$.

Affinity is calculated as $K_d = k_{off}/k_{on}$ ($k_{off}$ is the dissociation rate constant, $K_{on}$ is the association rate constant and $K_d$ is the equilibrium constant). Affinity can be determined at equilibrium by measuring the fraction bound (r) of labeled ligand at various concentrations (c). The data are graphed using the Scatchard equation: r/c = K(n-r): where r = moles of bound ligand/mole of receptor at equilibrium; c = free ligand concentration at equilibrium; K = equilibrium association constant; and n = number of ligand binding sites per receptor molecule. By graphical analysis, r/c is plotted on the Y-axis versus r on the X-axis, thus producing a Scatchard plot. Antibody affinity measurement by Scatchard analysis is well known in the art. *See, e.g.*, van Erp et al., J. Immunoassay 12: 425-43, 1991; Nelson and Griswold, Comput. Methods Programs Biomed. 27: 65-8, 1988.

The term "epitope" refers to an antigenic determinant capable of specific binding to an antibody. Epitopes usually consist of chemically active surface groupings of molecules such as amino acids or sugar side chains and usually have specific three dimensional structural characteristics, as well as specific charge characteristics. Conformational and nonconformational epitopes are distinguished in that the binding to the former but not the latter is lost in the presence of denaturing solvents.

Numerous publications discuss the use of phage display technology to produce and screen libraries of polypeptides for binding to a selected analyte. *See, e.g,* Cwirla et al., Proc. Natl. Acad. Sci. USA 87, 6378-82, 1990; Devlin et al., Science 249, 404-6, 1990, Scott and Smith, Science 249, 386-88, 1990; and Ladner et al., U.S. Pat. No. 5,571,698. A basic concept of phage display methods is the establishment of a physical association between DNA encoding a polypeptide to be screened and the polypeptide. This physical association is provided by the phage particle, which displays a polypeptide as part of a capsid enclosing the phage genome which encodes the polypeptide. The establishment of a physical association between polypeptides and their genetic material allows simultaneous mass screening of very large numbers of phage bearing different polypeptides. Phage displaying a polypeptide with affinity to a target bind to the target and these phage are enriched by affinity screening to the target. The identity of polypeptides displayed from these phage can be determined from their respective genomes. Using these methods a polypeptide identified as having a binding affinity for a desired target can then be synthesized in bulk by conventional means. *See, e.g.,* U.S. Patent No. 6,057,098.

The antibodies that are generated by these methods may then be selected by first screening for affinity and specificity with the purified polypeptide of interest and, if required, comparing the results to the affinity and specificity of the antibodies with polypeptides that are desired to be excluded from binding. The screening procedure can involve immobilization of the purified polypeptides in separate wells of microtiter plates. The solution containing a potential antibody or groups of antibodies is then placed into the respective microtiter wells and incubated for about 30 min to 2 h. The microtiter wells are then washed and a labeled secondary antibody (for example, an anti-mouse antibody conjugated to alkaline phosphatase if the raised antibodies are mouse antibodies) is added to the wells and incubated for about 30 min and then washed. Substrate is added to the wells and a color reaction will appear where antibody to the immobilized polypeptide(s) are present.

The antibodies so identified may then be further analyzed for affinity and specificity in the assay design selected. In the development of immunoassays for a target protein, the purified target protein acts as a standard with which to judge the sensitivity and specificity of the immunoassay using the antibodies that have been selected. Because the binding affinity of various antibodies may differ; certain antibody pairs (e.g., in sandwich assays) may interfere with one another sterically, etc., assay performance of an antibody may be a more important measure than absolute affinity and specificity of an antibody.

While the present application describes antibody-based binding assays in detail, alternatives to antibodies as binding species in assays are well known in the art. These include receptors for a particular target, aptamers, etc. Aptamers are oligonucleic acid or peptide molecules that bind to a specific target molecule. Aptamers are usually created by selecting them from a large random sequence pool, but natural aptamers also exist. High-affinity aptamers containing modified nucleotides conferring improved characteristics on the ligand, such as improved in vivo stability or improved delivery characteristics. Examples of such modifications include chemical substitutions at the ribose and/or phosphate and/or base positions, and may include amino acid side chain functionalities.

Assay Correlations

The term "correlating" as used herein in reference to the use of biomarkers refers to comparing the presence or amount of the biomarker(s) in a patient to its presence or amount in persons known to suffer from, or known to be at risk of, a given condition; or in persons known to be free of a given condition. Often, this takes the form of comparing an assay result in the form of a biomarker concentration to a predetermined threshold selected to be indicative of the occurrence or nonoccurrence of a disease or the likelihood of some future outcome.

Selecting a diagnostic threshold involves, among other things, consideration of the probability of disease, distribution of true and false diagnoses at different test thresholds, and estimates of the consequences of treatment (or a failure to treat) based on the diagnosis. For example, when considering administering a specific therapy which is highly efficacious and has a low level of risk, few tests are needed because clinicians can accept substantial diagnostic uncertainty. On the other hand, in situations where treatment options are less effective and more risky, clinicians often need a higher degree of diagnostic certainty. Thus, cost/benefit analysis is involved in selecting a diagnostic threshold.

Suitable thresholds may be determined in a variety of ways. For example, one recommended diagnostic threshold for the diagnosis of acute myocardial infarction using cardiac troponin is the 97.5th percentile of the concentration seen in a normal population. Another method may be to look at serial samples from the same patient, where a prior "baseline" result is used to monitor for temporal changes in a biomarker level.

Population studies may also be used to select a decision threshold. Reciever Operating Characteristic ("ROC") arose from the field of signal dectection therory developed during World War II for the analysis of radar images, and ROC analysis is often used to select a threshold able to best distinguish a "diseased" subpopulation from a "nondiseased" subpopulation. A false positive in this case occurs when the person tests positive, but actually does not have the disease. A false negative, on the other hand, occurs when the person tests negative, suggesting they are healthy, when they actually do have the disease. To draw a ROC curve, the true positive rate (TPR) and false positive rate (FPR) are determined as the decision threshold is varied continuously. Since TPR is equivalent with sensitivity and FPR is equal to 1 - specificity, the ROC graph is sometimes called the sensitivity vs (1 - specificity) plot. A perfect test will have an area under the ROC curve of 1.0; a random test will have an area of 0.5. A threshold is selected to provide an acceptable level of specificity and sensitivity.

In this context, "diseased" is meant to refer to a population having one characteristic (the presence of a disease or condition or the occurrence of some outcome) and "nondiseased" is meant to refer to a population lacking the characteristic. While a single decision threshold is the simplest application of such a method, multiple decision thresholds may be used. For example, below a first threshold, the absence of disease may be assigned with relatively high confidence, and above a second threshold the presence of disease may also be assigned with relatively high confidence. Between the two thresholds may be considered indeterminate. This is meant to be exemplary in nature only.

In addition to threshold comparisons, other methods for correlating assay results to a patient classification (occurrence or nonoccurrence of disease, likelihood of an outcome, etc.) include decision trees, rule sets, Bayesian methods, and neural network methods. These methods can produce probability values representing the degree to which a subject belongs to one classification out of a plurality of classifications.

Measures of test accuracy may be obtained as described in Fischer et al., Intensive Care Med. 29: 1043-51, 2003, and used to determine the effectiveness of a given biomarker. These measures include sensitivity and specificity, predictive values, likelihood ratios, diagnostic odds ratios, and ROC curve areas. The area under the curve ("AUC") of a ROC plot is equal to the probability that a classifier will rank a randomly chosen positive instance higher than a randomly chosen negative one. The area under the ROC curve may be thought of as equivalent to the Mann-Whitney U test, which tests for the median difference between scores obtained in the two groups considered if the groups are of continuous data, or to the Wilcoxon test of ranks.

As discussed above, suitable tests may exhibit one or more of the following results on these various measures: a specificity of greater than 0.5, preferably at least 0.6, more preferably at least 0.7, still more preferably at least 0.8, even more preferably at least 0.9 and most preferably at least 0.95, with a corresponding sensitivity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95; a sensitivity of greater than 0.5, preferably at least 0.6, more preferably at least 0.7, still more preferably at least 0.8, even more preferably at least 0.9 and most preferably at least 0.95, with a corresponding specificity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95; at least 75% sensitivity, combined with at least 75% specificity; a ROC curve area of greater than 0.5, preferably at least 0.6, more preferably 0.7, still more preferably at least 0.8, even more preferably at least 0.9, and most preferably at least 0.95; an odds ratio different from 1, preferably at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less; a positive likelihood ratio (calculated as sensitivity/(1-specificity)) of greater than 1, at least 2, more preferably at least 3, still more preferably at least 5, and most preferably at least 10; and or a negative likelihood ratio (calculated as (1-sensitivity)/specificity) of less than 1, less than or equal to 0.5, more preferably less than or equal to 0.3, and most preferably less than or equal to 0.1

Additional clinical indicia may be combined with the kidney injury marker assay result(s) of the present invention. These include other biomarkers related to renal status. Examples include the following, which recite the common biomarker name, followed by the Swiss-Prot entry number for that biomarker or its parent: Actin (P68133); Adenosine deaminase binding protein (DPP4, P27487); Alpha-1-acid glycoprotein 1 (P02763); Alpha-1-microglobulin (P02760); Albumin (P02768); Angiotensinogenase (Renin, P00797); Annexin A2 (P07355); Beta-glucuronidase (P08236); B-2-microglobulin (P61679); Beta-galactosidase (P16278); BMP-7 (P18075); Brain natriuretic peptide (proBNP, BNP-32, NTproBNP; P16860); Calcium-binding protein Beta (S100-beta, P04271); Carbonic anhydrase (Q16790); Casein Kinase 2 (P68400); Ceruloplasmin (P00450); Clusterin (P10909); Complement C3 (P01024); Cysteine-rich protein (CYR61, 000622); Cytochrome C (P99999); Epidermal growth factor (EGF, P01133); Endothelin-1 (P05305); Exosomal Fetuin-A (P02765); Fatty acid-binding protein, heart (FABP3, P05413); Fatty acid-binding protein, liver (P07148); Ferritin (light chain, P02793; heavy chain P02794); Fructose-1,6-biphosphatase (P09467); GRO-alpha (CXCL1, (P09341); Growth Hormone (P01241); Hepatocyte growth factor (P14210); Insulin-like growth factor I (P01343); Immunoglobulin G; Immunoglobulin Light Chains (Kappa and Lambda); Interferon gamma (P01308); Lysozyme (P61626); Interleukin-1alpha (P01583); Interleukin-2 (P60568); Interleukin-4 (P60568); Interleukin-9 (P15248); Interleukin-12p40 (P29460); Interleukin-13 (P35225); Interleukin-16 (Q14005); L1 cell adhesion molecule (P32004); Lactate dehydrogenase (P00338);

Leucine Aminopeptidase (P28838); Meprin A-alpha subunit (Q16819); Meprin A-beta subunit (Q16820); Midkine (P21741); MIP2-alpha (CXCL2, P19875); MMP-2 (P08253); MMP-9 (P14780); Netrin-1 (095631); Neutral endopeptidase (P08473); Osteopontin (P10451); Renal papillary antigen 1 (RPA1); Renal papillary antigen 2 (RPA2); Retinol binding protein (P09455); Ribonuclease; S100 calcium-binding protein A6 (P06703); Serum Amyloid P Component (P02743); Sodium/Hydrogen exchanger isoform (NHE3, P48764); Spermidine/spermine N1-acetyltransferase (P21673); TGF-Beta1 (P01137); Transferrin (P02787); Trefoil factor 3 (TFF3, Q07654); Toll-Like protein 4 (000206); Total protein; Tubulointerstitial nephritis antigen (Q9UJW2); Uromodulin (Tamm-Horsfall protein, P07911).

For purposes of risk stratification, Adiponectin (Q15848); Alkaline phosphatase (P05186); Aminopeptidase N (P15144); CalbindinD28k (P05937); Cystatin C (P01034); 8 subunit of FIFO ATPase (P03928); Gamma-glutamyltransferase (P19440); GSTa (alpha-glutathione-S-transferase, P08263); GSTpi (Glutathione-S-transferase P; GST class-pi; P09211); IGFBP-1 (P08833); IGFBP-2 (P18065); IGFBP-6 (P24592); Integral membrane protein 1 (Itm1, P46977); Interleukin-6 (P05231); Interleukin-8 (P10145); Interleukin-18 (Q14116); IP-10 (10 kDa interferon-gamma-induced protein, P02778); IRPR (IFRD1, 000458); Isovaleryl-CoA dehydrogenase (IVD, P26440); I-TAC/CXCL11 (014625); Keratin 19 (P08727); Kim-1 (Hepatitis A virus cellular receptor 1, 043656); L-arginine:glycine amidinotransferase (P50440); Leptin (P41159); Lipocalin2 (NGAL, P80188); MCP-1 (P13500); MIG (Gamma-interferon-induced monokine Q07325); MIP-1a (P10147); MIP-3a (P78556); MIP-1beta (P13236); MIP-1d (Q16663); NAG (N-acetyl-beta-D-glucosaminidase, P54802); Organic ion transporter (OCT2, 015244); Osteoprotegerin (014788); P8 protein (060356); Plasminogen activator inhibitor 1 (PAI-1, P05121); ProANP(1-98) (P01160); Protein phosphatase 1-beta (PPI-beta, P62140); Rab GDI-beta (P50395); Renal kallikrein (Q86U61); RT1.B-1 (alpha) chain of the integral membrane protein (Q5Y7A8); Soluble tumor necrosis factor receptor superfamily member 1A (sTNFR-1, P19438); Soluble tumor necrosis factor receptor superfamily member 1B (sTNFR-II, P20333); Tissue inhibitor of metalloproteinases 3 (TIMP-3, P35625); uPAR (Q03405) may be combined with the kidney injury marker assay result(s) of the present invention.

Other clinical indicia which may be combined with the kidney injury marker assay result(s) of the present invention includes demographic information (e.g., weight, sex, age, race), medical history (e.g., family history, type of surgery, pre-existing disease such as aneurism, congestive heart failure, preeclampsia, eclampsia, diabetes mellitus, hypertension, coronary artery disease, proteinuria, renal insufficiency, or sepsis, type of toxin exposure such as NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin), clinical variables (e.g., blood pressure, temperature, respiration rate), risk scores (APACHE score, PREDICT score, TIMI Risk Score for UA/NSTEMI, Framingham Risk Score), a urine total protein measurement, a glomerular filtration rate, an estimated glomerular filtration rate, a urine production rate, a serum or plasma creatinine concentration, a renal papillary antigen 1 (RPA1) measurement; a renal papillary antigen 2 (RPA2) measurement; a urine creatinine concentration, a fractional excretion of sodium, a urine sodium concentration, a urine creatinine to serum or plasma creatinine ratio, a urine specific gravity, a urine osmolality, a urine urea nitrogen to plasma urea nitrogen ratio, a plasma BUN to creatnine ratio, and/or a renal failure index calculated as urine sodium / (urine creatinine / plasma creatinine). Other measures of renal function which may be combined with the kidney injury marker assay result(s) are described hereinafter and in Harrison's Principles of Internal Medicine, 17th Ed., McGraw Hill, New York, pages 1741-1830, and Current Medical Diagnosis & Treatment 2008, 47th Ed, McGraw Hill, New York, pages 785-815.

Combining assay results/clinical indicia in this manner can comprise the use of multivariate logistical regression, loglinear modeling, neural network analysis, n-of-m analysis, decision tree analysis, etc. This list is not meant to be limiting.

Diagnosis of Acute Renal Failure

As noted above, the terms "acute renal (or kidney) injury" and "acute renal (or kidney) failure" as used herein are defined in part in terms of changes in serum creatinine from a baseline value. Most definitions of ARF have common elements, including the use of serum creatinine and, often, urine output. Patients may present with renal dysfunction without an available baseline measure of renal function for use in this comparison. In such an event, one may estimate a baseline serum creatinine value by assuming the patient initially had a normal GFR. Glomerular filtration rate (GFR) is the volume of fluid filtered from the renal (kidney) glomerular capillaries into the Bowman's capsule per unit time. Glomerular filtration rate (GFR) can be calculated by measuring any chemical that has a steady level in the blood, and is freely filtered but neither reabsorbed nor secreted by the kidneys. GFR is typically expressed in units of ml/min:

$$GFR = \frac{\text{Urine Concentration} \times \text{Urine Flow}}{\text{Plasma Concentration}}$$

By normalizing the GFR to the body surface area, a GFR of approximately 75-100 ml/min per 1.73 m$^2$ can be

assumed. The rate therefore measured is the quantity of the substance in the urine that originated from a calculable volume of blood.

There are several different techniques used to calculate or estimate the glomerular filtration rate (GFR or eGFR). In clinical practice, however, creatinine clearance is used to measure GFR. Creatinine is produced naturally by the body (creatinine is a metabolite of creatine, which is found in muscle). It is freely filtered by the glomerulus, but also actively secreted by the renal tubules in very small amounts such that creatinine clearance overestimates actual GFR by 10-20%. This margin of error is acceptable considering the ease with which creatinine clearance is measured.

Creatinine clearance (CCr) can be calculated if values for creatinine's urine concentration ($U_{Cr}$), urine flow rate (V), and creatinine's plasma concentration ($P_{Cr}$) are known. Since the product of urine concentration and urine flow rate yields creatinine's excretion rate, creatinine clearance is also said to be its excretion rate ($U_{Cr} \times V$) divided by its plasma concentration. This is commonly represented mathematically as:

$$C_{Cr} = \frac{U_{Cr} \times V}{P_{Cr}}$$

Commonly a 24 hour urine collection is undertaken, from empty-bladder one morning to the contents of the bladder the following morning, with a comparative blood test then taken:

$$C_{Cr} = \frac{U_{Cr} \times 24\text{-hour volume}}{P_{Cr} \times 24 \times 60 mins}$$

To allow comparison of results between people of different sizes, the CCr is often corrected for the body surface area (BSA) and expressed compared to the average sized man as ml/min/1.73 m2. While most adults have a BSA that approaches 1.7 (1.6-1.9), extremely obese or slim patients should have their CCr corrected for their actual BSA:

$$C_{Cr-corrected} = \frac{C_{Cr} \times 1.73}{BSA}$$

The accuracy of a creatinine clearance measurement (even when collection is complete) is limited because as glomerular filtration rate (GFR) falls creatinine secretion is increased, and thus the rise in serum creatinine is less. Thus, creatinine excretion is much greater than the filtered load, resulting in a potentially large overestimation of the GFR (as much as a twofold difference). However, for clinical purposes it is important to determine whether renal function is stable or getting worse or better. This is often determined by monitoring serum creatinine alone. Like creatinine clearance, the serum creatinine will not be an accurate reflection of GFR in the non-steady-state condition of ARF. Nonetheless, the degree to which serum creatinine changes from baseline will reflect the change in GFR. Serum creatinine is readily and easily measured and it is specific for renal function.

For purposes of determining urine output on a Urine output on a mL/kg/hr basis, hourly urine collection and measurement is adequate. In the case where, for example, only a cumulative 24-h output was available and no patient weights are provided, minor modifications of the RIFLE urine output criteria have been described. For example, Bagshaw et al., Nephrol. Dial. Transplant. 23: 1203-1210, 2008, assumes an average patient weight of 70 kg, and patients are assigned a RIFLE classification based on the following: <35 mL/h (Risk), <21 mL/h (Injury) or <4 mL/h (Failure).

Selecting a Treatment Regimen

Once a diagnosis is obtained, the clinician can readily select a treatment regimen that is compatible with the diagnosis, such as initiating renal replacement therapy, withdrawing delivery of compounds that are known to be damaging to the kidney, kidney transplantation, delaying or avoiding procedures that are known to be damaging to the kidney, modifying diuretic administration, initiating goal directed therapy, etc. The skilled artisan is aware of appropriate treatments for numerous diseases discussed in relation to the methods of diagnosis described herein. See, e.g., Merck Manual of Diagnosis and Therapy, 17th Ed. Merck Research Laboratories, Whitehouse Station, NJ, 1999. In addition, since the methods and compositions described herein provide prognostic information, the markers of the present invention may be used to monitor a course of treatment. For example, improved or worsened prognostic state may indicate that a particular treatment is or is not efficacious.

One skilled in the art readily appreciates that the present invention is well adapted to carry out the objects and obtain the ends and advantages mentioned, as well as those inherent therein. The examples provided herein are representative of preferred embodiments, are exemplary, and are not intended as limitations on the scope of the invention.

Example 1: Contrast-induced nephropathy sample collection

The objective of this sample collection study is to collect samples of plasma and urine and clinical data from patients before and after receiving intravascular contrast media. Approximately 250 adults undergoing radiographic/angiographic procedures involving intravascular administration of iodinated contrast media are enrolled. To be enrolled in the study, each patient must meet all of the following inclusion criteria and none of the following exclusion criteria:

Inclusion Criteria
males and females 18 years of age or older;
undergoing a radiographic / angiographic procedure (such as a CT scan or coronary intervention) involving the intravascular administration of contrast media;
expected to be hospitalized for at least 48 hours after contrast administration.
able and willing to provide written informed consent for study participation and to comply with all study procedures.
Exclusion Criteria
renal transplant recipients;
acutely worsening renal function prior to the contrast procedure;
already receiving dialysis (either acute or chronic) or in imminent need of dialysis at enrollment;
expected to undergo a major surgical procedure (such as involving cardiopulmonary bypass) or an additional imaging procedure with contrast media with significant risk for further renal insult within the 48 hrs following contrast administration;
participation in an interventional clinical study with an experimental therapy within the previous 30 days;
known infection with human immunodeficiency virus (HIV) or a hepatitis virus.

Immediately prior to the first contrast administration (and after any pre-procedure hydration), an EDTA anti-coagulated blood sample (10 mL) and a urine sample (10 mL) are collected from each patient. Blood and urine samples are then collected at 4 ($\pm$0.5), 8 ($\pm$1), 24 ($\pm$2) 48 ($\pm$2), and 72 ($\pm$2) hrs following the last administration of contrast media during the index contrast procedure. Blood is collected via direct venipuncture or via other available venous access, such as an existing femoral sheath, central venous line, peripheral intravenous line or hep-lock. These study blood samples are processed to plasma at the clinical site, frozen and shipped to Astute Medical, Inc., San Diego, CA. The study urine samples are frozen and shipped to Astute Medical, Inc.

Serum creatinine is assessed at the site immediately prior to the first contrast administration (after any pre-procedure hydration) and at 4 ($\pm$0.5), 8 ($\pm$1), 24 ($\pm$2) and 48 ($\pm$2) ), and 72 ($\pm$2) hours following the last administration of contrast (ideally at the same time as the study samples are obtained). In addition, each patient's status is evaluated through day 30 with regard to additional serum and urine creatinine measurements, a need for dialysis, hospitalization status, and adverse clinical outcomes (including mortality).

Prior to contrast administration, each patient is assigned a risk based on the following assessment: systolic blood pressure <80 mm Hg = 5 points; intra-arterial balloon pump = 5 points; congestive heart failure (Class III-IV or history of pulmonary edema) = 5 points; age >75 yrs = 4 points; hematocrit level <39% for men, <35% for women = 3 points; diabetes = 3 points; contrast media volume = 1 point for each 100 mL; serum creatinine level >1.5 g/dL = 4 points OR estimated GFR 40-60 mL/min/1.73 $m^2$ = 2 points, 20-40 mL/min/1.73 $m^2$ = 4 points, < 20 mL/min/1.73 $m^2$ = 6 points. The risks assigned are as follows: risk for CIN and dialysis: 5 or less total points = risk of CIN - 7.5%, risk of dialysis - 0.04%; 6-10 total points = risk of CIN - 14%, risk of dialysis - 0.12%; 11-16 total points = risk of CIN - 26.1%, risk of dialysis - 1.09%; >16 total points = risk of CIN - 57.3%, risk of dialysis - 12.8%.

Example 2: Cardiac surgery sample collection

The objective of this sample collection study is to collect samples of plasma and urine and clinical data from patients before and after undergoing cardiovascular surgery, a procedure known to be potentially damaging to kidney function. Approximately 900 adults undergoing such surgery are enrolled. To be enrolled in the study, each patient must meet all of the following inclusion criteria and none of the following exclusion criteria:

Inclusion Criteria
males and females 18 years of age or older;

undergoing cardiovascular surgery;
Toronto/Ottawa Predictive Risk Index for Renal Replacement risk score of at least 2 (Wijeysundera et al., JAMA 297: 1801-9, 2007); and
able and willing to provide written informed consent for study participation and to comply with all study procedures.
Exclusion Criteria
known pregnancy;
previous renal transplantation;
acutely worsening renal function prior to enrollment (e.g., any category of RIFLE criteria);
already receiving dialysis (either acute or chronic) or in imminent need of dialysis at enrollment;
currently enrolled in another clinical study or expected to be enrolled in another clinical study within 7 days of cardiac surgery that involves drug infusion or a therapeutic intervention for AKI;
known infection with human immunodeficiency virus (HIV) or a hepatitis virus.

Within 3 hours prior to the first incision (and after any pre-procedure hydration), an EDTA anti-coagulated blood sample (10 mL), whole blood (3 mL), and a urine sample (35 mL) are collected from each patient. Blood and urine samples are then collected at 3 ($\pm$0.5), 6 ($\pm$0.5), 12 ($\pm$1), 24 ($\pm$2) and 48 ($\pm$2) hrs following the procedure and then daily on days 3 through 7 if the subject remains in the hospital. Blood is collected via direct venipuncture or via other available venous access, such as an existing femoral sheath, central venous line, peripheral intravenous line or hep-lock. These study blood samples are frozen and shipped to Astute Medical, Inc., San Diego, CA. The study urine samples are frozen and shipped to Astute Medical, Inc.

Example 3: Acutely ill subject sample collection

The objective of this study is to collect samples from acutely ill patients. Approximately 900 adults expected to be in the ICU for at least 48 hours will be enrolled. To be enrolled in the study, each patient must meet all of the following inclusion criteria and none of the following exclusion criteria:

Inclusion Criteria
males and females 18 years of age or older;

Study population 1: approximately 300 patients that have at least one of:

shock (SBP < 90 mmHg and/or need for vasopressor support to maintain MAP > 60 mmHg and/or documented drop in SBP of at least 40 mmHg); and

sepsis;

Study population 2: approximately 300 patients that have at least one of:

IV antibiotics ordered in computerized physician order entry (CPOE) within 24 hours of enrollment;

contrast media exposure within 24 hours of enrollment;
increased Intra-Abdominal Pressure with acute decompensated heart failure; and severe trauma as the primary reason for ICU admission and likely to be hospitalized in the ICU for 48 hours after enrollment;
Study population 3: approximately 300 patients expected to be hospitalized through acute care setting (ICU or ED) with a known risk factor for acute renal injury (e.g. sepsis, hypotension/shock (Shock = systolic BP < 90 mmHg and/or the need for vasopressor support to maintain a MAP > 60 mmHg and/or a documented drop in SBP > 40 mmHg), major trauma, hemorrhage, or major surgery); and/or expected to be hospitalized to the ICU for at least 24 hours after enrollment.

Exclusion Criteria
known pregnancy;
institutionalized individuals;
previous renal transplantation;
known acutely worsening renal function prior to enrollment (e.g., any category of RIFLE criteria);
received dialysis (either acute or chronic) within 5 days prior to enrollment or in imminent need of dialysis at the time of enrollment;
known infection with human immunodeficiency virus (HIV) or a hepatitis virus;

meets only the SBP < 90 mmHg inclusion criterion set forth above, and does not have shock in the attending physician's or principal investigator's opinion.

After providing informed consent, an EDTA anti-coagulated blood sample (10 mL) and a urine sample (25-30 mL) are collected from each patient. Blood and urine samples are then collected at 4 ($\pm$ 0.5) and 8 ($\pm$ 1) hours after contrast administration (if applicable); at 12 ($\pm$ 1), 24 ($\pm$ 2), and 48 ($\pm$ 2) hours after enrollment, and thereafter daily up to day 7 to day 14 while the subject is hospitalized. Blood is collected via direct venipuncture or via other available venous access, such as an existing femoral sheath, central venous line, peripheral intravenous line or hep-lock. These study blood samples are processed to plasma at the clinical site, frozen and shipped to Astute Medical, Inc., San Diego, CA. The study urine samples are frozen and shipped to Astute Medical, Inc.

Example 4. Immunoassay format

Analytes are measured using standard sandwich enzyme immunoassay techniques. A first antibody which binds the analyte is immobilized in wells of a 96 well polystyrene microplate. Analyte standards and test samples are pipetted into the appropriate wells and any analyte present is bound by the immobilized antibody. After washing away any unbound substances, a horseradish peroxidase-conjugated second antibody which binds the analyte is added to the wells, thereby forming sandwich complexes with the analyte (if present) and the first antibody. Following a wash to remove any unbound antibody-enzyme reagent, a substrate solution comprising tetramethylbenzidine and hydrogen peroxide is added to the wells. Color develops in proportion to the amount of analyte present in the sample. The color development is stopped and the intensity of the color is measured at 540 nm or 570 nm. An analyte concentration is assigned to the test sample by comparison to a standard curve determined from the analyte standards.

Example 5. Apparently Healthy Donor and Chronic Disease Patient Samples

Human urine samples from donors with no known chronic or acute disease ("Apparently Healthy Donors") were purchased from two vendors (Golden West Biologicals, Inc., 27625 Commerce Center Dr., Temecula, CA 92590 and Virginia Medical Research, Inc., 915 First Colonial Rd., Virginia Beach, VA 23454). The urine samples were shipped and stored frozen at less than -20° C. The vendors supplied demographic information for the individual donors including gender, race (Black /White), smoking status and age.

Human urine samples from donors with various chronic diseases ("Chronic Disease Patients") including congestive heart failure, coronary artery disease, chronic kidney disease, chronic obstructive pulmonary disease, diabetes mellitus and hypertension were purchased from Virginia Medical Research, Inc., 915 First Colonial Rd., Virginia Beach, VA 23454. The urine samples were shipped and stored frozen at less than -20 degrees centigrade. The vendor provided a case report form for each individual donor with age, gender, race (Black/White), smoking status and alcohol use, height, weight, chronic disease(s) diagnosis, current medications and previous surgeries.

Example 6. Use of Kidney Injury Markers for evaluating renal status in patients

Patients from the intensive care unit (ICU) were enrolled in the following study. Each patient was classified by kidney status as non-injury (0), risk of injury (R), injury (I), and failure (F) according to the maximum stage reached within 7 days of enrollment as determined by the RIFLE criteria. EDTA anti-coagulated blood samples (10 mL) and a urine samples (25-30 mL) were collected from each patient at enrollment, 4 ($\pm$ 0.5) and 8 ($\pm$ 1) hours after contrast administration (if applicable); at 12 ($\pm$ 1), 24 ($\pm$ 2), and 48 ($\pm$ 2) hours after enrollment, and thereafter daily up to day 7 to day 14 while the subject is hospitalized. Tumor necrosis factor receptor superfamily member 10B, Cadherin-16, Caspase-9, Bcl2 antagonist of cell death, Caspase-1, Cadherin-1, Poly [ADP-ribose] polymerase 1, Cyclin-dependent kinase inhibitor 1, Cadherin-5, Myoglobin, Apolipoprotein A-II, Mucin-16, Carcinoembryonic antigen-related cell adhesion molecule 5, and Cellular tumor antigen p53 were each measured by standard immunoassay methods using commercially available assay reagents in the urine samples and the plasma component of the blood samples collected. Concentrations were reported as follows: Tumor necrosis factor receptor superfamily member 10B - ng/ml, Cadherin-16 - ng/ml, Caspase-9 - ng/ml, Bcl2 antagonist of cell death - absorbance units, Caspase-1 - pg/ml, Cadherin-1 - pg/ml, Poly [ADP-ribose] polymerase 1 - ng/ml, Cyclin-dependent kinase inhibitor 1 - pg/ml, Cadherin-5 - ng/ml, Myoglobin - ng/ml, Apolipoprotein A-II - ng/ml, Mucin-16, Carcinoembryonic antigen-related cell adhesion molecule 5, and Cellular tumor antigen p53 - U/ml, Carcinoembryonic antigen-related cell adhesion molecule 5 - ng/ml, and Cellular tumor antigen p53 - ng/ml.

Two cohorts were defined as described in the introduction to each of the following tables. In the following tables, the time "prior max stage" represents the time at which a sample is collected, relative to the time a particular patient reaches the lowest disease stage as defined for that cohort, binned into three groups which are +/- 12 hours. For example, "24 hr prior" which uses 0 vs R, I, F as the two cohorts would mean 24 hr (+/- 12 hours) prior to reaching stage R (or I if no sample at R, or F if no sample at R or I).

A receiver operating characteristic (ROC) curve was generated for each biomarker measured and the area under each ROC curve (AUC) was determined. Patients in Cohort 2 were also separated according to the reason for adjudication to cohort 2 as being based on serum creatinine measurements (sCr), being based on urine output (UO), or being based on either serum creatinine measurements or urine output. Using the same example discussed above (0 vs R, I, F), for those patients adjudicated to stage R, I, or F on the basis of serum creatinine measurements alone, the stage 0 cohort may have included patients adjudicated to stage R, I, or F on the basis of urine output; for those patients adjudicated to stage R, I, or F on the basis of urine output alone, the stage 0 cohort may have included patients adjudicated to stage R, I, or F on the basis of serum creatinine measurements; and for those patients adjudicated to stage R, I, or F on the basis of serum creatinine measurements or urine output, the stage 0 cohort contains only patients in stage 0 for both serum creatinine measurements and urine output. Also, in the data for patients adjudicated on the basis of serum creatinine measurements or urine output, the adjudication method which yielded the most severe RIFLE stage was used.

The ability to distinguish cohort 1 from Cohort 2 was determined using ROC analysis. SE is the standard error of the AUC, n is the number of sample or individual patients ("pts," as indicated). Standard errors were calculated as described in Hanley, J. A., and McNeil, B.J., The meaning and use of the area under a receiver operating characteristic (ROC) curve. Radiology (1982) 143: 29-36; p values were calculated with a two-tailed Z-test, and are reported as $p<0.05$ in tables 1-6 and $p<0.10$ in tables 7-14. An AUC < 0.5 is indicative of a negative going marker for the comparison, and an AUC > 0.5 is indicative of a positive going marker for the comparison.

Various threshold (or "cutoff") concentrations were selected, and the associated sensitivity and specificity for distinguishing cohort 1 from cohort 2 were determined. OR is the odds ratio calculated for the particular cutoff concentration, and 95% CI is the confidence interval for the odds ratio.

Table 1: Comparison of marker levels in urine samples collected from Cohort 1 (patients that did not progress beyond RIFLE stage 0) and in urine samples collected from subjects at 0, 24 hours, and 48 hours prior to reaching stage R, I or F in Cohort 2.

**Apolipoprotein A-II**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 71.6 | 85.6 | 71.6 | 72.2 | 71.6 | 75.2 |
| Average | 199 | 234 | 199 | 394 | 199 | 136 |
| Stdev | 723 | 763 | 723 | 2590 | 723 | 224 |
| p(t-test) | | 0.70 | | 0.21 | | 0.58 |
| Min | 2.08 | 4.67 | 2.08 | 9.26 | 2.08 | 1.00E-9 |
| Max | 6400 | 6400 | 6400 | 24300 | 6400 | 1420 |
| n (Samp) | 366 | 74 | 366 | 88 | 366 | 41 |
| n (Patient) | 196 | 74 | 196 | 88 | 196 | 41 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 75.8 | 85.0 | 75.8 | 85.9 | 75.8 | 75.2 |
| Average | 201 | 358 | 201 | 131 | 201 | 120 |
| Stdev | 1050 | 1220 | 1050 | 145 | 1050 | 127 |
| p(t-test) | | 0.45 | | 0.69 | | 0.71 |
| Min | 1.00E-9 | 4.67 | 1.00E-9 | 9.44 | 1.00E-9 | 5.58 |
| Max | 24300 | 6400 | 24300 | 677 | 24300 | 488 |
| n (Samp) | 750 | 27 | 750 | 37 | 750 | 23 |
| n (Patient) | 294 | 27 | 294 | 37 | 294 | 23 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 72.9 | 95.4 | 72.9 | 82.5 | 72.9 | 117 |
| Average | 219 | 249 | 219 | 483 | 219 | 174 |

| UO only | 0hr prior to AKI stage | | | 24hr prior to AKI stage | | | 48hr prior to AKI stage | |
|---|---|---|---|---|---|---|---|---|
| | Cohort 1 | | Cohort 2 | Cohort 1 | | Cohort 2 | Cohort 1 | Cohort 2 |
| Stdev | 774 | | 808 | 774 | | 2820 | 774 | 260 |
| p(t-test) | | | 0.78 | 0.16 | | | | 0.73 |
| Min | 2.08 | | 18.9 | 2.08 | | 9.26 | 2.08 | 1.00E-9 |
| Max | 6400 | | 6400 | 6400 | | 24300 | 6400 | 1420 |
| n (Samp) | 297 | | 65 | 297 | | 74 | 297 | 35 |
| n (Patient) | 132 | | 65 | 132 | | 74 | 132 | 35 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.57 | 0.54 | 0.59 | 0.53 | 0.54 | 0.56 | 0.53 | 0.51 | 0.60 |
| SE | 0.038 | 0.058 | 0.040 | 0.035 | 0.050 | 0.038 | 0.048 | 0.062 | 0.053 |
| p | 0.066 | 0.54 | 0.019 | 0.47 | 0.45 | 0.11 | 0.57 | 0.82 | 0.068 |
| nCohort 1 | 366 | 750 | 297 | 366 | 750 | 297 | 366 | 750 | 297 |
| nCohort 2 | 74 | 27 | 65 | 88 | 37 | 74 | 41 | 23 | 35 |
| Cutoff 1 | 53.4 | 58.0 | 64.3 | 46.8 | 58.2 | 56.1 | 46.8 | 47.2 | 68.6 |
| Sens 1 | 70% | 70% | 71% | 70% | 70% | 70% | 71% | 74% | 71% |
| Spec 1 | 38% | 36% | 45% | 32% | 37% | 38% | 32% | 28% | 48% |
| Cutoff 2 | 36.3 | 34.3 | 48.8 | 40.5 | 33.2 | 44.5 | 28.5 | 41.3 | 37.4 |
| Sens 2 | 81% | 81% | 80% | 81% | 81% | 81% | 80% | 83% | 80% |
| Spec 2 | 20% | 16% | 31% | 24% | 15% | 25% | 12% | 23% | 19% |
| Cutoff 3 | 33.5 | 26.3 | 36.2 | 28.5 | 26.3 | 33.2 | 21.0 | 27.1 | 23.1 |
| Sens 3 | 91% | 93% | 91% | 91% | 92% | 91% | 90% | 91% | 91% |
| Spec 3 | 17% | 9% | 16% | 12% | 9% | 14% | 7% | 10% | 8% |
| Cutoff 4 | 107 | 114 | 113 | 107 | 114 | 113 | 107 | 114 | 113 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 4 | 39% | 41% | 42% | 34% | 41% | 41% | 39% | 35% | 54% |
| Spec 4 | 70% | 70% | 70% | 70% | 70% | 70% | 70% | 70% | 70% |
| Cutoff 5 | 135 | 148 | 144 | 135 | 148 | 144 | 135 | 148 | 144 |
| Sens 5 | 32% | 26% | 34% | 26% | 30% | 31% | 32% | 17% | 46% |
| Spec 5 | 80% | 80% | 80% | 80% | 80% | 80% | 80% | 80% | 80% |
| Cutoff 6 | 226 | 227 | 292 | 226 | 227 | 292 | 226 | 227 | 292 |
| Sens 6 | 18% | 15% | 14% | 10% | 11% | 9% | 12% | 13% | 9% |
| Spec 6 | 90% | 90% | 90% | 90% | 90% | 90% | 90% | 90% | 90% |
| OR Quart 2 | 0.67 | 0.42 | 1.2 | 1.4 | 0.58 | 1.1 | 0.46 | 1.4 | 0.60 |
| p Value | 0.32 | 0.21 | 0.68 | 0.33 | 0.31 | 0.87 | 0.14 | 0.56 | 0.39 |
| 95% CI of | 0.30 | 0.11 | 0.51 | 0.72 | 0.21 | 0.50 | 0.17 | 0.44 | 0.19 |
| OR Quart2 | 1.5 | 1.6 | 2.8 | 2.7 | 1.6 | 2.3 | 1.3 | 4.5 | 1.9 |
| OR Quart 3 | 1.2 | 1.1 | 1.7 | 0.88 | 0.79 | 0.99 | 0.72 | 0.80 | 0.60 |
| p Value | 0.59 | 0.79 | 0.22 | 0.72 | 0.62 | 0.97 | 0.48 | 0.74 | 0.39 |
| 95% CI of | 0.60 | 0.41 | 0.73 | 0.43 | 0.30 | 0.46 | 0.29 | 0.21 | 0.19 |
| OR Quart3 | 2.5 | 3.2 | 3.8 | 1.8 | 2.0 | 2.1 | 1.8 | 3.0 | 1.9 |
| OR Quart 4 | 1.6 | 1.3 | 2.6 | 1.5 | 1.3 | 1.7 | 1.2 | 1.4 | 2.4 |
| p Value | 0.17 | 0.62 | 0.018 | 0.20 | 0.53 | 0.12 | 0.69 | 0.57 | 0.056 |
| 95% CI of | 0.81 | 0.47 | 1.2 | 0.80 | 0.56 | 0.86 | 0.52 | 0.44 | 0.98 |
| OR Quart4 | 3.2 | 3.5 | 5.6 | 3.0 | 3.1 | 3.5 | 2.7 | 4.5 | 6.0 |

(continued)

| Bcl2 antagonist of cell death | | | | | | |
|---|---|---|---|---|---|---|
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 0.00173 | 0.00173 | nd | nd | nd | nd |
| Average | 0.0412 | 0.00246 | nd | nd | nd | nd |
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Stdev | 0.150 | 0.00254 | nd | nd | nd | nd |
| p(t-test) | | 0.38 | nd | nd | nd | nd |
| Min | 0.00173 | 0.00173 | nd | nd | nd | nd |
| Max | 0.833 | 0.0105 | nd | nd | nd | nd |
| n (Samp) | 54 | 12 | nd | nd | nd | nd |
| n (Patient) | 36 | 12 | nd | nd | nd | nd |
| | | | | | | |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 0.00173 | 0.00173 | nd | nd | nd | nd |
| Average | 0.0279 | 0.00319 | nd | nd | nd | nd |
| Stdev | 0.122 | 0.00359 | nd | nd | nd | nd |
| p(t-test) | | 0.62 | nd | nd | nd | nd |
| Min | 0.00173 | 0.00173 | nd | nd | nd | nd |
| Max | 0.833 | 0.0105 | nd | nd | nd | nd |
| n (Samp) | 84 | 6 | nd | nd | nd | nd |
| n (Patient) | 59 | 6 | nd | nd | nd | nd |
| | | | | | | |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.00173 | 0.00173 | nd | nd | nd | nd |
| Average | 0.0457 | 0.00173 | nd | nd | nd | nd |
| Stdev | 0.172 | 0 | nd | nd | nd | nd |
| p(t-test) | | 0.38 | nd | nd | nd | nd |
| Min | 0.00173 | 0.00173 | nd | nd | nd | nd |
| Max | 0.833 | 0.00173 | nd | nd | nd | nd |
| n (Samp) | 40 | 12 | nd | nd | nd | nd |
| n (Patient) | 26 | 12 | nd | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.38 | 0.43 | 0.39 | nd | nd | nd | nd | nd | nd |
| SE | 0.094 | 0.13 | 0.097 | nd | nd | nd | nd | nd | nd |
| p | 0.20 | 0.60 | 0.24 | nd | nd | nd | nd | nd | nd |
| nCohort 1 | 54 | 84 | 40 | nd | nd | nd | nd | nd | nd |
| nCohort 2 | 12 | 6 | 12 | nd | nd | nd | nd | nd | nd |
| Cutoff 1 | 0 | 0 | 0 | nd | nd | nd | nd | nd | nd |
| Sens 1 | 100% | 100% | 100% | nd | nd | nd | nd | nd | nd |
| Spec 1 | 0% | 0% | 0% | nd | nd | nd | nd | nd | nd |
| Cutoff 2 | 0 | 0 | 0 | nd | nd | nd | nd | nd | nd |
| Sens 2 | 100% | 100% | 100% | nd | nd | nd | nd | nd | nd |
| Spec 2 | 0% | 0% | 0% | nd | nd | nd | nd | nd | nd |
| Cutoff 3 | 0 | 0 | 0 | nd | nd | nd | nd | nd | nd |
| Sens 3 | 100% | 100% | 100% | nd | nd | nd | nd | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 3 | 0% | 0% | 0% | nd | nd | nd | nd | nd | nd |
| Cutoff 4 | 0.00509 | 0.00173 | 0.00173 | nd | nd | nd | nd | nd | nd |
| Sens 4 | 8% | 17% | 0% | nd | nd | nd | nd | nd | nd |
| Spec 4 | 70% | 71% | 78% | nd | nd | nd | nd | nd | nd |
| Cutoff 5 | 0.0148 | 0.00943 | 0.00509 | nd | nd | nd | nd | nd | nd |
| Sens 5 | 0% | 17% | 0% | nd | nd | nd | nd | nd | nd |
| Spec 5 | 83% | 82% | 80% | nd | nd | nd | nd | nd | nd |
| Cutoff 6 | 0.0354 | 0.0235 | 0.0148 | nd | nd | nd | nd | nd | nd |
| Sens 6 | 0% | 0% | 0% | nd | nd | nd | nd | nd | nd |
| Spec 6 | 91% | 92% | 90% | nd | nd | nd | nd | nd | nd |
| OR Quart 2 | 9.6 | 4.9 | >0 | nd | nd | nd | nd | nd | nd |
| p Value | 0.050 | 0.17 | <na | nd | nd | nd | nd | nd | nd |
| 95% CI of | 1.0 | 0.50 | >na | nd | nd | nd | nd | nd | nd |
| OR Quart2 | 92 | 48 | na | nd | nd | nd | nd | nd | nd |
| OR Quart 3 | 3.4 | 1.0 | >160 | nd | nd | nd | nd | nd | nd |
| p Value | 0.31 | 1.0 | <5.9E-4 | nd | nd | nd | nd | nd | nd |
| 95% CI of | 0.32 | 0.059 | >8.8 | nd | nd | nd | nd | nd | nd |
| OR Quart3 | 37 | 17 | na | nd | nd | nd | nd | nd | nd |
| OR Quart 4 | 2.3 | 0 | >0 | nd | nd | nd | nd | nd | nd |
| p Value | 0.52 | na | <na | nd | nd | nd | nd | nd | nd |
| 95% CI of | 0.19 | na | >na | nd | nd | nd | nd | nd | nd |
| OR Quart4 | 28 | na | na | nd | nd | nd | nd | nd | nd |

**Caspase-9**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.469 | 0.955 | nd | nd | nd | nd |
| Average | 1.11 | 0.937 | nd | nd | nd | nd |
| Stdev | 2.89 | 1.01 | nd | nd | nd | nd |
| p(t-test) | | 0.84 | nd | nd | nd | nd |
| Min | 0.0360 | 0.0360 | nd | nd | nd | nd |
| Max | 19.5 | 3.69 | nd | nd | nd | nd |
| n (Samp) | 54 | 12 | nd | nd | nd | nd |
| n (Patient) | 36 | 12 | nd | nd | nd | nd |
| | | | | | | |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 0.469 | 1.05 | nd | nd | nd | nd |
| Average | 1.08 | 1.44 | nd | nd | nd | nd |
| Stdev | 2.44 | 1.42 | nd | nd | nd | nd |
| p(t-test) | | 0.73 | nd | nd | nd | nd |
| Min | 0.0360 | 0.0360 | nd | nd | nd | nd |
| Max | 19.5 | 3.92 | nd | nd | nd | nd |
| n (Samp) | 86 | 6 | nd | nd | nd | nd |
| n (Patient) | 60 | 6 | nd | nd | nd | nd |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 0.586 | 0.790 | nd | nd | nd | nd |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Average | 1.35 | 0.929 | nd | nd | nd | nd |
| Stdev | 3.31 | 0.998 | nd | nd | nd | nd |
| p(t-test) | | 0.67 | nd | nd | nd | nd |
| Min | 0.0360 | 0.0360 | nd | nd | nd | nd |
| Max | 19.5 | 3.69 | nd | nd | nd | nd |
| n (Samp) | 40 | 12 | nd | nd | nd | nd |
| n (Patient) | 26 | 12 | nd | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.59 | 0.66 | 0.56 | nd | nd | nd | nd | nd | nd |
| SE | 0.094 | 0.13 | 0.097 | nd | nd | nd | nd | nd | nd |
| p | 0.32 | 0.20 | 0.52 | nd | nd | nd | nd | nd | nd |
| nCohort 1 | 54 | 86 | 40 | nd | nd | nd | nd | nd | nd |
| nCohort 2 | 12 | 6 | 12 | nd | nd | nd | nd | nd | nd |
| Cutoff 1 | 0.208 | 0.330 | 0.208 | nd | nd | nd | nd | nd | nd |
| Sens 1 | 75% | 83% | 83% | nd | nd | nd | nd | nd | nd |
| Spec 1 | 37% | 38% | 28% | nd | nd | nd | nd | nd | nd |
| Cutoff 2 | 0 | 0.330 | 0.208 | nd | nd | nd | nd | nd | nd |
| Sens 2 | 100% | 83% | 83% | nd | nd | nd | nd | nd | nd |
| Spec 2 | 0% | 38% | 28% | nd | nd | nd | nd | nd | nd |
| Cutoff 3 | 0 | 0 | 0 | nd | nd | nd | nd | nd | nd |
| Sens 3 | 100% | 100% | 100% | nd | nd | nd | nd | nd | nd |
| Spec 3 | 0% | 0% | 0% | nd | nd | nd | nd | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 4 | 0.703 | 0.811 | 0.781 | nd | nd | nd | nd | nd | nd |
| Sens 4 | 58% | 67% | 50% | nd | nd | nd | nd | nd | nd |
| Spec 4 | 70% | 71% | 72% | nd | nd | nd | nd | nd | nd |
| Cutoff 5 | 1.04 | 1.04 | 0.868 | nd | nd | nd | nd | nd | nd |
| Sens 5 | 33% | 50% | 50% | nd | nd | nd | nd | nd | nd |
| Spec 5 | 81% | 80% | 80% | nd | nd | nd | nd | nd | nd |
| Cutoff 6 | 1.76 | 2.17 | 2.17 | nd | nd | nd | nd | nd | nd |
| Sens 6 | 8% | 17% | 8% | nd | nd | nd | nd | nd | nd |
| Spec 6 | 91% | 91% | 90% | nd | nd | nd | nd | nd | nd |
| OR Quart 2 | 0.58 | 1.0 | 1.6 | nd | nd | nd | nd | nd | nd |
| p Value | 0.58 | 1.0 | 0.62 | nd | nd | nd | nd | nd | nd |
| 95% CI of | 0.083 | 0.059 | 0.23 | nd | nd | nd | nd | nd | nd |
| OR Quart2 | 4.0 | 17 | 12 | nd | nd | nd | nd | nd | nd |
| OR Quart 3 | 0.29 | 1.0 | 1.0 | nd | nd | nd | nd | nd | nd |
| p Value | 0.31 | 1.0 | 1.0 | nd | nd | nd | nd | nd | nd |
| 95% CI of | 0.027 | 0.059 | 0.12 | nd | nd | nd | nd | nd | nd |
| OR Quart3 | 3.1 | 17 | 8.4 | nd | nd | nd | nd | nd | nd |
| OR Quart 4 | 2.4 | 3.3 | 3.4 | nd | nd | nd | nd | nd | nd |
| p Value | 0.29 | 0.32 | 0.20 | nd | nd | nd | nd | nd | nd |
| 95% CI of | 0.48 | 0.32 | 0.53 | nd | nd | nd | nd | nd | nd |
| OR Quart4 | 12 | 34 | 22 | nd | nd | nd | nd | nd | nd |

| Cadherin-1 | | | | | | |
|---|---|---|---|---|---|---|
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 48800 | 32700 | 48800 | 111000 | nd | nd |
| Average | 84900 | 79300 | 84900 | 143000 | nd | nd |
| Stdev | 121000 | 100000 | 121000 | 147000 | nd | nd |
| p(t-test) | | 0.86 | | 0.091 | nd | nd |
| Min | 160 | 1620 | 160 | 1660 | nd | nd |
| Max | 744000 | 363000 | 744000 | 543000 | nd | nd |
| n (Samp) | 52 | 20 | 52 | 20 | nd | nd |
| n (Patient) | 41 | 20 | 41 | 20 | nd | nd |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 37500 | 50200 | 37500 | 111000 | nd | nd |
| Average | 75900 | 91900 | 75900 | 146000 | nd | nd |
| Stdev | 124000 | 107000 | 124000 | 140000 | nd | nd |
| p(t-test) | | 0.65 | | 0.046 | nd | nd |
| Min | 160 | 1620 | 160 | 2220 | nd | nd |
| Max | 744000 | 363000 | 744000 | 543000 | nd | nd |
| n (Samp) | 42 | 16 | 42 | 21 | nd | nd |
| n (Patient) | 33 | 16 | 33 | 21 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.45 | nd | 0.55 | 0.64 | nd | 0.71 | nd | nd | nd |

EP 2 894 473 B1

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| SE | 0.077 | nd | 0.086 | 0.076 | nd | 0.073 | nd | nd | nd |
| p | 0.55 | nd | 0.58 | 0.064 | nd | 0.0041 | nd | nd | nd |
| nCohort 1 | 52 | nd | 42 | 52 | nd | 42 | nd | nd | nd |
| nCohort 2 | 20 | nd | 16 | 20 | nd | 21 | nd | nd | nd |
| Cutoff 1 | 10500 | nd | 21400 | 51100 | nd | 54200 | nd | nd | nd |
| Sens 1 | 70% | nd | 75% | 70% | nd | 71% | nd | nd | nd |
| Spec 1 | 15% | nd | 36% | 54% | nd | 60% | nd | nd | nd |
| Cutoff 2 | 9800 | nd | 10400 | 28500 | nd | 36500 | nd | nd | nd |
| Sens 2 | 80% | nd | 81% | 80% | nd | 81% | nd | nd | nd |
| Spec 2 | 13% | nd | 24% | 38% | nd | 50% | nd | nd | nd |
| Cutoff 3 | 4310 | nd | 3350 | 8380 | nd | 11700 | nd | nd | nd |
| Sens 3 | 90% | nd | 94% | 90% | nd | 90% | nd | nd | nd |
| Spec 3 | 8% | nd | 10% | 12% | nd | 29% | nd | nd | nd |
| Cutoff 4 | 94000 | nd | 80700 | 94000 | nd | 80700 | nd | nd | nd |
| Sens 4 | 25% | nd | 38% | 55% | nd | 62% | nd | nd | nd |
| Spec 4 | 71% | nd | 71% | 71% | nd | 71% | nd | nd | nd |
| Cutoff 5 | 134000 | nd | 122000 | 134000 | nd | 122000 | nd | nd | nd |
| Sens 5 | 20% | nd | 31% | 40% | nd | 48% | nd | nd | nd |
| Spec 5 | 81% | nd | 81% | 81% | nd | 81% | nd | nd | nd |
| Cutoff 6 | 152000 | nd | 144000 | 152000 | nd | 144000 | nd | nd | nd |
| Sens 6 | 15% | nd | 19% | 35% | nd | 33% | nd | nd | nd |
| Spec 6 | 90% | nd | 90% | 90% | nd | 90% | nd | nd | nd |
| OR Quart 2 | 0.74 | nd | 0.62 | 1.4 | nd | 0.92 | nd | nd | nd |
| p Value | 0.70 | nd | 0.59 | 0.67 | nd | 0.93 | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| 95% CI of | 0.16 | nd | 0.11 | 0.27 | nd | 0.16 | nd | nd | nd |
| OR Quart2 | 3.4 | nd | 3.5 | 7.5 | nd | 5.5 | nd | nd | nd |
| OR Quart 3 | 0.74 | nd | 0.68 | 1.9 | nd | 1.8 | nd | nd | nd |
| p Value | 0.70 | nd | 0.66 | 0.43 | nd | 0.48 | nd | nd | nd |
| 95% CI of | 0.16 | nd | 0.12 | 0.38 | nd | 0.35 | nd | nd | nd |
| OR Quart3 | 3.4 | nd | 3.8 | 9.6 | nd | 9.5 | nd | nd | nd |
| OR Quart 4 | 1.7 | nd | 1.7 | 4.0 | nd | 6.7 | nd | nd | nd |
| p Value | 0.48 | nd | 0.52 | 0.080 | nd | 0.022 | nd | nd | nd |
| 95% CI of | 0.41 | nd | 0.35 | 0.85 | nd | 1.3 | nd | nd | nd |
| OR Quart4 | 6.7 | nd | 7.9 | 19 | nd | 34 | nd | nd | nd |

**Cadherin-5**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.00224 | 0.00224 | nd | nd | nd | nd |
| Average | 0.0957 | 0.0142 | nd | nd | nd | nd |
| Stdev | 0.210 | 0.0183 | nd | nd | nd | nd |
| p(t-test) | | 0.28 | nd | nd | nd | nd |
| Min | 0.00224 | 0.00224 | nd | nd | nd | nd |
| Max | 1.10 | 0.0502 | nd | nd | nd | nd |
| n (Samp) | 47 | 8 | nd | nd | nd | nd |
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| n (Patient) | 30 | 8 | nd | nd | nd | nd |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 0.00224 | 0.00224 | nd | nd | nd | nd |
| Average | 0.0806 | 0.00753 | nd | nd | nd | nd |
| Stdev | 0.218 | 0.0108 | nd | nd | nd | nd |
| p(t-test) | | 0.32 | nd | nd | nd | nd |
| Min | 0.00224 | 0.00224 | nd | nd | nd | nd |
| Max | 1.10 | 0.0309 | nd | nd | nd | nd |
| n (Samp) | 34 | 9 | nd | nd | nd | nd |
| n (Patient) | 20 | 9 | nd | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.46 | nd | 0.44 | nd | nd | nd | nd | nd | nd |
| SE | 0.11 | nd | 0.11 | nd | nd | nd | nd | nd | nd |
| p | 0.72 | nd | 0.62 | nd | nd | nd | nd | nd | nd |
| nCohort 1 | 47 | nd | 34 | nd | nd | nd | nd | nd | nd |
| nCohort 2 | 8 | nd | 9 | nd | nd | nd | nd | nd | nd |
| Cutoff 1 | 0 | nd | 0 | nd | nd | nd | nd | nd | nd |
| Sens 1 | 100% | nd | 100% | nd | nd | nd | nd | nd | nd |
| Spec 1 | 0% | nd | 0% | nd | nd | nd | nd | nd | nd |
| Cutoff 2 | 0 | nd | 0 | nd | nd | nd | nd | nd | nd |
| Sens 2 | 100% | nd | 100% | nd | nd | nd | nd | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 2 | 0% | nd | 0% | nd | nd | nd | nd | nd | nd |
| Cutoff 3 | 0 | nd | 0 | nd | nd | nd | nd | nd | nd |
| Sens 3 | 100% | nd | 100% | nd | nd | nd | nd | nd | nd |
| Spec 3 | 0% | nd | 0% | nd | nd | nd | nd | nd | nd |
| Cutoff 4 | 0.0405 | nd | 0.00224 | nd | nd | nd | nd | nd | nd |
| Sens 4 | 12% | nd | 22% | nd | nd | nd | nd | nd | nd |
| Spec 4 | 72% | nd | 71% | nd | nd | nd | nd | nd | nd |
| Cutoff 5 | 0.137 | nd | 0.127 | nd | nd | nd | nd | nd | nd |
| Sens 5 | 0% | nd | 0% | nd | nd | nd | nd | nd | nd |
| Spec 5 | 81% | nd | 82% | nd | nd | nd | nd | nd | nd |
| Cutoff 6 | 0.329 | nd | 0.239 | nd | nd | nd | nd | nd | nd |
| Sens 6 | 0% | nd | 0% | nd | nd | nd | nd | nd | nd |
| Spec 6 | 91% | nd | 91% | nd | nd | nd | nd | nd | nd |
| OR Quart 2 | 2.2 | nd | 0 | nd | nd | nd | nd | nd | nd |
| p Value | 0.55 | nd | na | nd | nd | nd | nd | nd | nd |
| 95% CI of | 0.17 | nd | na | nd | nd | nd | nd | nd | nd |
| OR Quart2 | 27 | nd | na | nd | nd | nd | nd | nd | nd |
| OR Quart 3 | 3.5 | nd | 5.4 | nd | nd | nd | nd | nd | nd |
| p Value | 0.30 | nd | 0.088 | nd | nd | nd | nd | nd | nd |
| 95% CI of | 0.32 | nd | 0.78 | nd | nd | nd | nd | nd | nd |
| OR Quart3 | 39 | nd | 38 | nd | nd | nd | nd | nd | nd |
| OR Quart 4 | 2.4 | nd | 0.50 | nd | nd | nd | nd | nd | nd |
| p Value | 0.51 | nd | 0.60 | nd | nd | nd | nd | nd | nd |
| 95% CI of | 0.19 | nd | 0.038 | nd | nd | nd | nd | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart4 | 30 | nd | 6.5 | nd | nd | nd | nd | nd | nd |

**Cyclin-dependent kinase inhibitor 1**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.713 | 0.116 | 0.713 | 1.98 | 0.713 | 1.09 |
| Average | 103 | 8.88 | 103 | 19.5 | 103 | 5.00 |
| Stdev | 670 | 19.5 | 670 | 49.0 | 670 | 9.16 |
| p(t-test) | | 0.33 | | 0.35 | | 0.47 |
| Min | 1.14E-14 | 1.70E-14 | 1.14E-14 | 1.70E-14 | 1.14E-14 | 1.70E-14 |
| Max | 6950 | 112 | 6950 | 327 | 6950 | 42.0 |
| n (Samp) | 165 | 48 | 165 | 56 | 165 | 25 |
| n (Patient) | 102 | 48 | 102 | 56 | 102 | 25 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.09 | 5.28 | 1.09 | 10.7 | 1.09 | 2.45 |
| Average | 62.4 | 15.7 | 62.4 | 19.5 | 62.4 | 6.76 |
| Stdev | 482 | 28.6 | 482 | 21.2 | 482 | 12.3 |
| p(t-test) | | 0.70 | | 0.69 | | 0.68 |
| Min | 1.14E-14 | 0.116 | 1.14E-14 | 1.70E-14 | 1.14E-14 | 1.70E-14 |
| Max | 6950 | 112 | 6950 | 71.0 | 6950 | 44.1 |
| n (Samp) | 325 | 16 | 325 | 20 | 325 | 13 |
| n (Patient) | 168 | 16 | 168 | 20 | 168 | 13 |

(continued)

| Cyclin-dependent kinase inhibitor 1 | | | | | | | |
|---|---|---|---|---|---|---|---|
| sCr or UO | 0hr prior to AKI stage | | 24hr prior to AKI stage | | 48hr prior to AKI stage | | |
| | Cohort | 1 Cohort 2 | Cohort | 1 Cohort 2 | Cohort 1 | Cohort 2 | |
| | | | | | | | |
| UO only | 0hr prior to AKI stage | | 24hr prior to AKI stage | | 48hr prior to AKI stage | | |
| | Cohort 1 | Cohort 2 | Cohort | 1 Cohort 2 | Cohort 1 | Cohort 2 | |
| Median | 0.850 | 0.116 | 0.850 | 2.45 | 0.850 | 1.09 | |
| Average | 119 | 24.9 | 119 | 23.7 | 119 | 10.5 | |
| Stdev | 729 | 97.5 | 729 | 60.2 | 729 | 24.6 | |
| p(t-test) | | 0.39 | | 0.35 | | 0.48 | |
| Min | 1.14E-14 | 1.70E-14 | 1.14E-14 | 0.116 | 1.14E-14 | 0.116 | |
| Max | 6950 | 630 | 6950 | 327 | 6950 | 112 | |
| n (Samp) | 139 46 | | 139 | 51 | 139 | 23 | |
| n (Patient) | 85 46 | | 85 | 51 | 85 | 23 | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.45 | 0.61 | 0.45 | 0.53 | 0.62 | 0.54 | 0.46 | 0.46 | 0.49 |
| SE | 0.048 | 0.077 | 0.050 | 0.045 | 0.069 | 0.048 | 0.063 | 0.084 | 0.065 |
| p | 0.35 | 0.16 | 0.28 | 0.51 | 0.088 | 0.44 | 0.58 | 0.60 | 0.89 |
| nCohort 1 | 165 | 325 | 139 | 165 | 325 | 139 | 165 | 325 | 139 |
| nCohort 2 | 48 | 16 | 46 | 56 | 20 | 51 | 25 | 13 | 23 |
| Cutoff 1 | 1.70E-14 | 0.850 | 1.70E-14 | 1.70E-14 | 0.850 | 1.14E-14 | 1.70E-14 | 1.70E-14 | 1.14E-14 |
| Sens 1 | 98% | 75% | 98% | 96% | 75% | 100% | 96% | 92% | 100% |
| Spec 1 | 2% | 50% | 2% | 2% | 50% | 2% | 2% | 2% | 2% |
| Cutoff 2 | 1.70E-14 | 0.116 | 1.70E-14 | 1.70E-14 | 1.70E-14 | 1.14E-14 | 1.70E-14 | 1.70E-14 | 1.14E-14 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 2 | 98% | 81% | 98% | 96% | 90% | 100% | 96% | 92% | 100% |
| Spec 2 | 2% | 43% | 2% | 2% | 2% | 2% | 2% | 2% | 2% |
| Cutoff 3 | 1.70E-14 | 1.14E-14 | 1.70E-14 | 1.70E-14 | 1.70E-14 | 1.14E-14 | 1.70E-14 | 1.70E-14 | 1.14E-14 |
| Sens 3 | 98% | 100% | 98% | 96% | 90% | 100% | 96% | 92% | 100% |
| Spec 3 | 2% | 2% | 2% | 2% | 2% | 2% | 2% | 2% | 2% |
| Cutoff 4 | 7.84 | 9.28 | 9.28 | 7.84 | 9.28 | 9.28 | 7.84 | 9.28 | 9.28 |
| Sens 4 | 23% | 25% | 26% | 38% | 50% | 35% | 24% | 15% | 22% |
| Spec 4 | 70% | 71% | 71% | 70% | 71% | 71% | 70% | 71% | 71% |
| Cutoff 5 | 18.3 | 15.5 | 22.7 | 18.3 | 15.5 | 22.7 | 18.3 | 15.5 | 22.7 |
| Sens 5 | 17% | 25% | 15% | 25% | 45% | 20% | 4% | 15% | 13% |
| Spec 5 | 80% | 80% | 81% | 80% | 80% | 81% | 80% | 80% | 81% |
| Cutoff 6 | 60.3 | 44.8 | 73.1 | 60.3 | 44.8 | 73.1 | 60.3 | 44.8 | 73.1 |
| Sens 6 | 2% | 6% | 4% | 7% | 10% | 6% | 0% | 0% | 4% |
| Spec 6 | 90% | 90% | 91% | 90% | 90% | 91% | 90% | 90% | 91% |
| OR Quart 2 | 1.3 | 0.33 | 1.0 | 0.25 | 0.19 | 0.60 | 4.6 | 2.6 | 2.7 |
| p Value | 0.60 | 0.34 | 0.96 | 0.0079 | 0.13 | 0.31 | 0.027 | 0.26 | 0.13 |
| 95% CI of | 0.50 | 0.033 | 0.38 | 0.091 | 0.022 | 0.23 | 1.2 | 0.50 | 0.75 |
| OR Quart2 | 3.3 | 3.2 | 2.8 | 0.70 | 1.7 | 1.6 | 18 | 14 | 9.6 |
| OR Quart 3 | 0.78 | 2.8 | 1.2 | 0.77 | 0.79 | 1.1 | 0 | 0 | 0.23 |
| p Value | 0.64 | 0.13 | 0.76 | 0.53 | 0.73 | 0.82 | na | na | 0.20 |
| 95% CI of | 0.28 | 0.73 | 0.44 | 0.34 | 0.20 | 0.45 | na | na | 0.025 |
| OR Quart3 | 2.2 | 11 | 3.1 | 1.7 | 3.0 | 2.7 | na | na | 2.2 |
| OR Quart 4 | 2.3 | 1.3 | 1.8 | 0.90 | 2.1 | 1.2 | 4.6 | 3.2 | 2.7 |
| p Value | 0.073 | 0.71 | 0.22 | 0.79 | 0.19 | 0.70 | 0.027 | 0.16 | 0.13 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| 95% CI of | 0.93 | 0.29 | 0.71 | 0.40 | 0.69 | 0.49 | 1.2 | 0.63 | 0.75 |
| OR Quart4 | 5.5 | 6.1 | 4.5 | 2.0 | 6.4 | 2.9 | 18 | 16 | 9.6 |

**Carcinoembryonic antigen-related cell adhesion molecule 5**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.646 | 1.40 | 0.646 | 1.30 | 0.646 | 1.38 |
| Average | 2.04 | 4.66 | 2.04 | 4.98 | 2.04 | 4.81 |
| Stdev | 4.06 | 7.77 | 4.06 | 10.7 | 4.06 | 9.92 |
| p(t-test) | | 6.7E-4 | | 7.2E-4 | | 0.0064 |
| Min | 0.00336 | 0.00336 | 0.00336 | 0.0411 | 0.00336 | 0.0844 |
| Max | 43.4 | 29.4 | 43.4 | 54.4 | 43.4 | 47.4 |
| n (Samp) | 253 | 48 | 253 | 57 | 253 | 26 |
| n (Patient) | 102 | 48 | 102 | 57 | 102 | 26 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.947 | 1.48 | 0.947 | 1.66 | 0.947 | 2.29 |
| Average | 12.1 | 3.83 | 12.1 | 7.05 | 12.1 | 3.90 |
| Stdev | 192 | 5.63 | 192 | 12.5 | 192 | 4.53 |
| p(t-test) | | 0.86 | | 0.90 | | 0.88 |
| Min | 0.00336 | 0.00336 | 0.00336 | 0.0411 | 0.00336 | 0.173 |
| Max | 4070 | 21.1 | 4070 | 51.3 | 4070 | 15.3 |
| n (Samp) | 447 | 16 | 447 | 21 | 447 | 13 |

(continued)

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| n (Patient) | 170 | 16 | 170 | 21 | 170 | 13 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.625 | 1.44 | 0.625 | 1.25 | 0.625 | 1.35 |
| Average | 2.35 | 4.89 | 2.35 | 4.76 | 2.35 | 4.02 |
| Stdev | 4.80 | 7.57 | 4.80 | 10.6 | 4.80 | 9.89 |
| p(t-test) |  | 0.0038 | 0.015 |  |  | 0.16 |
| Min | 0.00336 | 0.00336 | 0.00336 | 0.0946 | 0.00336 | 0.00336 |
| Max | 43.4 | 29.4 | 43.4 | 54.4 | 43.4 | 47.4 |
| n (Samp) | 212 | 47 | 212 | 52 | 212 | 25 |
| n (Patient) | 85 | 47 | 85 | 52 | 85 | 25 |

| | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.62 | 0.56 | 0.64 | 0.61 | 0.62 | 0.61 | 0.60 | 0.65 | 0.55 |
| SE | 0.046 | 0.076 | 0.047 | 0.043 | 0.067 | 0.045 | 0.061 | 0.084 | 0.062 |
| p | 0.012 | 0.44 | 0.0036 | 0.0089 | 0.081 | 0.014 | 0.11 | 0.068 | 0.41 |
| nCohort 1 | 253 | 447 | 212 | 253 | 447 | 212 | 253 | 447 | 212 |
| nCohort 2 | 48 | 16 | 47 | 57 | 21 | 52 | 26 | 13 | 25 |
| Cutoff 1 | 0.540 | 0.414 | 0.550 | 0.607 | 1.10 | 0.607 | 0.456 | 1.06 | 0.456 |
| Sens 1 | 71% | 75% | 70% | 70% | 71% | 71% | 73% | 77% | 72% |
| Spec 1 | 45% | 32% | 46% | 48% | 53% | 49% | 42% | 53% | 43% |
| Cutoff 2 | 0.383 | 0.271 | 0.485 | 0.394 | 0.394 | 0.444 | 0.303 | 0.849 | 0.303 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 2 | 81% | 81% | 81% | 81% | 81% | 81% | 81% | 85% | 80% |
| Spec 2 | 37% | 23% | 43% | 38% | 31% | 42% | 31% | 47% | 32% |
| Cutoff 3 | 0.146 | 0.102 | 0.148 | 0.173 | 0.0990 | 0.211 | 0.146 | 0.211 | 0.0990 |
| Sens 3 | 92% | 94% | 91% | 91% | 90% | 90% | 92% | 92% | 92% |
| Spec 3 | 13% | 7% | 12% | 16% | 7% | 20% | 13% | 17% | 8% |
| Cutoff 4 | 1.54 | 1.91 | 1.54 | 1.54 | 1.91 | 1.54 | 1.54 | 1.91 | 1.54 |
| Sens 4 | 38% | 44% | 43% | 44% | 48% | 42% | 42% | 54% | 32% |
| Spec 4 | 70% | 70% | 70% | 70% | 70% | 70% | 70% | 70% | 70% |
| Cutoff 5 | 2.53 | 3.14 | 2.77 | 2.53 | 3.14 | 2.77 | 2.53 | 3.14 | 2.77 |
| Sens 5 | 35% | 44% | 34% | 28% | 33% | 25% | 27% | 31% | 16% |
| Spec 5 | 80% | 80% | 80% | 80% | 80% | 80% | 80% | 80% | 80% |
| Cutoff 6 | 5.50 | 8.51 | 6.40 | 5.50 | 8.51 | 6.40 | 5.50 | 8.51 | 6.40 |
| Sens 6 | 21% | 12% | 23% | 18% | 19% | 17% | 19% | 15% | 12% |
| Spec 6 | 90% | 90% | 90% | 90% | 90% | 90% | 90% | 90% | 90% |
| OR Quart 2 | 2.2 | 0.74 | 1.8 | 1.6 | 0.66 | 1.3 | 0.57 | 0.50 | 0.58 |
| p Value | 0.14 | 0.69 | 0.30 | 0.36 | 0.65 | 0.61 | 0.46 | 0.57 | 0.47 |
| 95% CI of | 0.78 | 0.16 | 0.60 | 0.60 | 0.11 | 0.48 | 0.13 | 0.044 | 0.13 |
| OR Quart2 | 6.2 | 3.4 | 5.2 | 4.1 | 4.0 | 3.5 | 2.5 | 5.5 | 2.5 |
| OR Quart 3 | 2.4 | 0.49 | 2.2 | 3.0 | 2.8 | 3.2 | 2.1 | 2.6 | 2.8 |
| p Value | 0.093 | 0.41 | 0.14 | 0.015 | 0.14 | 0.013 | 0.19 | 0.27 | 0.074 |
| 95% CI of | 0.86 | 0.087 | 0.77 | 1.2 | 0.72 | 1.3 | 0.69 | 0.49 | 0.90 |
| OR Quart3 | 6.7 | 2.7 | 6.2 | 7.4 | 11 | 7.8 | 6.6 | 14 | 8.4 |
| OR Quart 4 | 3.3 | 1.8 | 4.0 | 2.4 | 2.8 | 2.0 | 1.7 | 2.6 | 0.98 |
| p Value | 0.018 | 0.37 | 0.0064 | 0.058 | 0.14 | 0.17 | 0.40 | 0.27 | 0.98 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| 95% CI of | 1.2 | 0.51 | 1.5 | 0.97 | 0.72 | 0.76 | 0.51 | 0.49 | 0.27 |
| OR Quart4 | 8.9 | 6.3 | 11 | 6.0 | 11 | 5.0 | 5.3 | 14 | 3.6 |

**Myoglobin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.21 | 0.965 | 1.21 | 0.546 | 1.21 | 0.330 |
| Average | 26.0 | 38.3 | 26.0 | 70.8 | 26.0 | 47.2 |
| Stdev | 82.5 | 97.7 | 82.5 | 151 | 82.5 | 131 |
| p(t-test) | | 0.36 | | 0.0021 | | 0.24 |
| Min | 0.000105 | 0.000105 | 0.000105 | 0.0276 | 0.000105 | 0.0254 |
| Max | 618 | 469 | 618 | 618 | 618 | 469 |
| n (Samp) | 253 | 48 | 253 | 57 | 253 | 26 |
| n (Patient) | 102 | 48 | 102 | 57 | 102 | 26 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.608 | 0.186 | 0.608 | 0.375 | 0.608 | 0.349 |
| Average | 32.4 | 66.7 | 32.4 | 58.7 | 32.4 | 62.5 |
| Stdev | 99.0 | 156 | 99.0 | 131 | 99.0 | 173 |
| p(t-test) | | 0.18 | | 0.24 | | 0.29 |
| Min | 0.000105 | 0.000105 | 0.000105 | 0.0375 | 0.000105 | 0.0567 |
| Max | 618 | 469 | 618 | 442 | 618 | 618 |
| n (Samp) | 447 | 16 | 447 | 21 | 447 | 13 |

(continued)

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Patient) | 170 | 16 | 170 | 21 | 170 | 13 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.23 | 1.00 | 1.23 | 0.855 | 1.23 | 0.366 |
| Average | 29.6 | 29.5 | 29.6 | 76.5 | 29.6 | 68.8 |
| Stdev | 90.0 | 76.4 | 90.0 | 160 | 90.0 | 155 |
| p(t-test) | | 0.99 | | 0.0051 | | 0.062 |
| Min | 0.00616 | 0.0266 | 0.00616 | 0.000105 | 0.00616 | 0.0254 |
| Max | 618 | 469 | 618 | 618 | 618 | 469 |
| n (Samp) | 212 | 47 | 212 | 52 | 212 | 25 |
| n (Patient) | 85 | 47 | 85 | 52 | 85 | 25 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.51 | 0.48 | 0.51 | 0.50 | 0.53 | 0.51 | 0.45 | 0.52 | 0.50 |
| SE | 0.046 | 0.074 | 0.047 | 0.042 | 0.066 | 0.045 | 0.061 | 0.082 | 0.061 |
| p | 0.85 | 0.84 | 0.87 | 0.94 | 0.66 | 0.84 | 0.44 | 0.76 | 0.97 |
| nCohort 1 | 253 | 447 | 212 | 253 | 447 | 212 | 253 | 447 | 212 |
| nCohort 2 | 48 | 16 | 47 | 57 | 21 | 52 | 26 | 13 | 25 |
| Cutoff 1 | 0.0819 | 0.0422 | 0.0832 | 0.0855 | 0.0849 | 0.0855 | 0.0877 | 0.132 | 0.118 |
| Sens 1 | 71% | 75% | 70% | 70% | 71% | 71% | 73% | 77% | 72% |
| Spec 1 | 19% | 16% | 19% | 19% | 26% | 19% | 19% | 33% | 24% |
| Cutoff 2 | 0.0394 | 0.0333 | 0.0485 | 0.0746 | 0.0556 | 0.0767 | 0.0667 | 0.0667 | 0.0832 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 2 | 81% | 81% | 81% | 81% | 81% | 81% | 81% | 85% | 80% |
| Spec 2 | 11% | 12% | 11% | 18% | 21% | 18% | 17% | 23% | 19% |
| Cutoff 3 | 0.0329 | 0 | 0.0333 | 0.0337 | 0.0399 | 0.0333 | 0.0296 | 0.0584 | 0.0296 |
| Sens 3 | 92% | 100% | 91% | 91% | 90% | 90% | 92% | 92% | 92% |
| Spec 3 | 8% | 0% | 7% | 8% | 15% | 7% | 6% | 22% | 5% |
| Cutoff 4 | 6.79 | 5.77 | 7.09 | 6.79 | 5.77 | 7.09 | 6.79 | 5.77 | 7.09 |
| Sens 4 | 44% | 44% | 43% | 35% | 38% | 37% | 23% | 31% | 32% |
| Spec 4 | 70% | 70% | 70% | 70% | 70% | 70% | 70% | 70% | 70% |
| Cutoff 5 | 16.7 | 16.8 | 18.6 | 16.7 | 16.8 | 18.6 | 16.7 | 16.8 | 18.6 |
| Sens 5 | 31% | 38% | 26% | 32% | 29% | 31% | 19% | 15% | 28% |
| Spec 5 | 80% | 80% | 80% | 80% | 80% | 80% | 80% | 80% | 80% |
| Cutoff 6 | 45.5 | 63.4 | 54.1 | 45.5 | 63.4 | 54.1 | 45.5 | 63.4 | 54.1 |
| Sens 6 | 19% | 12% | 13% | 21% | 19% | 23% | 12% | 15% | 16% |
| Spec 6 | 90% | 90% | 90% | 90% | 90% | 90% | 90% | 90% | 90% |
| OR Quart 2 | 0.33 | 0 | 0.33 | 0.47 | 1.0 | 0.42 | 0.65 | 1.7 | 0.47 |
| p Value | 0.020 | na | 0.025 | 0.068 | 1.0 | 0.056 | 0.51 | 0.48 | 0.24 |
| 95% CI of | 0.13 | na | 0.13 | 0.21 | 0.31 | 0.17 | 0.17 | 0.40 | 0.13 |
| OR Quart2 | 0.84 | na | 0.87 | 1.1 | 3.2 | 1.0 | 2.4 | 7.3 | 1.7 |
| OR Quart 3 | 0.33 | 0.41 | 0.39 | 0.33 | 0.32 | 0.37 | 1.4 | 0.66 | 0.60 |
| p Value | 0.020 | 0.21 | 0.045 | 0.015 | 0.17 | 0.032 | 0.57 | 0.65 | 0.40 |
| 95% CI of | 0.13 | 0.10 | 0.15 | 0.14 | 0.064 | 0.15 | 0.45 | 0.11 | 0.18 |
| OR Quart3 | 0.84 | 1.6 | 0.98 | 0.81 | 1.6 | 0.92 | 4.2 | 4.0 | 2.0 |
| OR Quart 4 | 0.84 | 0.86 | 0.83 | 0.86 | 1.2 | 0.93 | 1.4 | 1.0 | 1.0 |
| p Value | 0.66 | 0.79 | 0.65 | 0.68 | 0.78 | 0.84 | 0.56 | 1.0 | 0.97 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| 95% CI of | 0.39 | 0.28 | 0.37 | 0.41 | 0.38 | 0.43 | 0.46 | 0.20 | 0.36 |
| OR Quart4 | 1.8 | 2.6 | 1.8 | 1.8 | 3.6 | 2.0 | 4.3 | 5.1 | 2.9 |

**Mucin-16**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.619 | 0.753 | 0.619 | 0.547 | nd | nd |
| Average | 1.06 | 1.12 | 1.06 | 23.1 | nd | nd |
| Stdev | 1.28 | 0.983 | 1.28 | 96.4 | nd | nd |
| p(t-test) |  | 0.85 |  | 0.10 | nd | nd |
| Min | 0.141 | 1.00E-9 | 0.141 | 0.223 | nd | nd |
| Max | 6.37 | 3.40 | 6.37 | 433 | nd | nd |
| n (Samp) | 52 | 20 | 52 | 20 | nd | nd |
| n (Patient) | 41 | 20 | 41 | 20 | nd | nd |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.547 | 0.654 | 0.547 | 0.684 | nd | nd |
| Average | 0.951 | 1.00 | 0.951 | 22.0 | nd | nd |
| Stdev | 1.18 | 0.898 | 1.18 | 94.1 | nd | nd |
| p(t-test) |  | 0.87 |  | 0.15 | nd | nd |
| Min | 0.0565 | 1.00E-9 | 0.0565 | 0.223 | nd | nd |
| Max | 6.37 | 2.82 | 6.37 | 433 | nd | nd |
| n (Samp) | 42 | 16 | 42 | 21 | nd | nd |
| n (Patient) | 33 | 16 | 33 | 21 | nd | nd |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.54 | nd | 0.54 | 0.49 | nd | 0.56 | nd | nd | nd |
| SE | 0.077 | nd | 0.086 | 0.077 | nd | 0.078 | nd | nd | nd |
| p | 0.61 | nd | 0.67 | 0.87 | nd | 0.48 | nd | nd | nd |
| nCohort 1 | 52 | nd | 42 | 52 | nd | 42 | nd | nd | nd |
| nCohort 2 | 20 | nd | 16 | 20 | nd | 21 | nd | nd | nd |
| Cutoff 1 | 0.479 | nd | 0.412 | 0.344 | nd | 0.384 | nd | nd | nd |
| Sens 1 | 70% | nd | 75% | 70% | nd | 71% | nd | nd | nd |
| Spec 1 | 37% | nd | 36% | 17% | nd | 29% | nd | nd | nd |
| Cutoff 2 | 0.223 | nd | 0.223 | 0.278 | nd | 0.278 | nd | nd | nd |
| Sens 2 | 85% | nd | 81% | 90% | nd | 90% | nd | nd | nd |
| Spec 2 | 4% | nd | 7% | 10% | nd | 12% | nd | nd | nd |
| Cutoff 3 | 0.146 | nd | 0.0565 | 0.278 | nd | 0.278 | nd | nd | nd |
| Sens 3 | 90% | nd | 94% | 90% | nd | 90% | nd | nd | nd |
| Spec 3 | 4% | nd | 2% | 10% | nd | 12% | nd | nd | nd |
| Cutoff 4 | 0.937 | nd | 0.859 | 0.937 | nd | 0.859 | nd | nd | nd |
| Sens 4 | 45% | nd | 38% | 30% | nd | 43% | nd | nd | nd |
| Spec 4 | 71% | nd | 71% | 71% | nd | 71% | nd | nd | nd |
| Cutoff 5 | 1.24 | nd | 1.09 | 1.24 | nd | 1.09 | nd | nd | nd |
| Sens 5 | 40% | nd | 38% | 30% | nd | 33% | nd | nd | nd |
| Spec 5 | 81% | nd | 81% | 81% | nd | 81% | nd | nd | nd |
| Cutoff 6 | 1.87 | nd | 1.68 | 1.87 | nd | 1.68 | nd | nd | nd |

EP 2 894 473 B1

86

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 6 | 20% | nd | 19% | 25% | nd | 24% | nd | nd | nd |
| Spec 6 | 90% | nd | 90% | 90% | nd | 90% | nd | nd | nd |
| OR Quart 2 | 0.74 | nd | 0.62 | 0.40 | nd | 0.50 | nd | nd | nd |
| p Value | 0.70 | nd | 0.59 | 0.26 | nd | 0.38 | nd | nd | nd |
| 95% CI of | 0.16 | nd | 0.11 | 0.082 | nd | 0.11 | nd | nd | nd |
| OR Quart2 | 3.4 | nd | 3.5 | 1.9 | nd | 2.3 | nd | nd | nd |
| OR Quart 3 | 0.52 | nd | 0.68 | 0.77 | nd | 0.50 | nd | nd | nd |
| p Value | 0.43 | nd | 0.66 | 0.72 | nd | 0.38 | nd | nd | nd |
| 95% CI of | 0.10 | nd | 0.12 | 0.19 | nd | 0.11 | nd | nd | nd |
| OR Quart3 | 2.6 | nd | 3.8 | 3.2 | nd | 2.3 | nd | nd | nd |
| OR Quart 4 | 2.1 | nd | 1.7 | 1.0 | nd | 1.2 | nd | nd | nd |
| p Value | 0.30 | nd | 0.52 | 1.0 | nd | 0.83 | nd | nd | nd |
| 95% CI of | 0.52 | nd | 0.35 | 0.25 | nd | 0.28 | nd | nd | nd |
| OR Quart4 | 8.3 | nd | 7.9 | 4.0 | nd | 4.9 | nd | nd | nd |

**Poly [ADP-ribose] polymerase 1 (cleaved)**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.00E-9 | 1.00E-9 | 1.00E-9 | 1.00E-9 | 1.00E-9 | 0.00439 |
| Average | 0.00474 | 0.00294 | 0.00474 | 0.00432 | 0.00474 | 0.00271 |
| Stdev | 0.00688 | 0.00538 | 0.00688 | 0.00586 | 0.00688 | 0.00261 |
| p(t-test) | | 0.11 | | 0.70 | | 0.16 |
| Min | 1.00E-9 | 1.00E-9 | 1.00E-9 | 1.00E-9 | 1.00E-9 | 1.00E-9 |
| Max | 0.0357 | 0.0144 | 0.0357 | 0.0183 | 0.0357 | 0.00723 |

| Poly [ADP-ribose] polymerase 1 (cleaved) | | | | | | |
|---|---|---|---|---|---|---|
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| n (Samp) | 118 | 47 | 118 | 54 | 118 | 24 |
| n (Patient) | 97 | 47 | 97 | 54 | 97 | 24 |
| | | | | | | |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 1.00E-9 | 1.00E-9 | 1.00E-9 | 1.00E-9 | 1.00E-9 | 1.00E-9 |
| Average | 0.00375 | 0.00338 | 0.00375 | 0.00301 | 0.00375 | 0.00350 |
| Stdev | 0.00582 | 0.00600 | 0.00582 | 0.00648 | 0.00582 | 0.00540 |
| p(t-test) | | 0.82 | | 0.60 | | 0.89 |
| Min | 1.00E-9 | 1.00E-9 | 1.00E-9 | 1.00E-9 | 1.00E-9 | 1.00E-9 |
| Max | 0.0357 | 0.0144 | 0.0357 | 0.0253 | 0.0357 | 0.0144 |
| n (Samp) | 263 | 14 | 263 | 19 | 263 | 12 |
| n (Patient) | 159 | 14 | 159 | 19 | 159 | 12 |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 1.00E-9 | 1.00E-9 | 1.00E-9 | 0.00340 | 1.00E-9 | 0.00471 |
| Average | 0.00356 | 0.00258 | 0.00356 | 0.00527 | 0.00356 | 0.00448 |
| Stdev | 0.00628 | 0.00494 | 0.00628 | 0.00622 | 0.00628 | 0.00455 |
| p(t-test) | | 0.36 | | 0.12 | | 0.51 |
| Min | 1.00E-9 | 1.00E-9 | 1.00E-9 | 1.00E-9 | 1.00E-9 | 1.00E-9 |
| Max | 0.0357 | 0.0144 | 0.0357 | 0.0183 | 0.0357 | 0.0144 |
| n (Samp) | 105 | 45 | 105 | 49 | 105 | 23 |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Patient) | 84 | 45 | 84 | 49 | 84 | 23 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.43 | 0.46 | 0.47 | 0.51 | 0.45 | 0.59 | 0.50 | 0.50 | 0.62 |
| SE | 0.050 | 0.081 | 0.052 | 0.048 | 0.070 | 0.050 | 0.065 | 0.086 | 0.068 |
| p | 0.18 | 0.63 | 0.51 | 0.85 | 0.52 | 0.060 | 0.97 | 0.97 | 0.071 |
| nCohort 1 | 118 | 263 | 105 | 118 | 263 | 105 | 118 | 263 | 105 |
| nCohort 2 | 47 | 14 | 45 | 54 | 19 | 49 | 24 | 12 | 23 |
| Cutoff 1 | 0 | 0 | 0 | 0 | 0 | 0 | 0 | 0 | 0 |
| Sens 1 | 100% | 100% | 100% | 100% | 100% | 100% | 100% | 100% | 100% |
| Spec 1 | 0% | 0% | 0% | 0% | 0% | 0% | 0% | 0% | 0% |
| Cutoff 2 | 0 | 0 | 0 | 0 | 0 | 0 | 0 | 0 | 0 |
| Sens 2 | 100% | 100% | 100% | 100% | 100% | 100% | 100% | 100% | 100% |
| Spec 2 | 0% | 0% | 0% | 0% | 0% | 0% | 0% | 0% | 0% |
| Cutoff 3 | 0 | 0 | 0 | 0 | 0 | 0 | 0 | 0 | 0 |
| Sens 3 | 100% | 100% | 100% | 100% | 100% | 100% | 100% | 100% | 100% |
| Spec 3 | 0% | 0% | 0% | 0% | 0% | 0% | 0% | 0% | 0% |
| Cutoff 4 | 0.00598 | 0.00471 | 0.00406 | 0.00598 | 0.00471 | 0.00406 | 0.00598 | 0.00471 | 0.00406 |
| Sens 4 | 17% | 21% | 24% | 24% | 16% | 47% | 4% | 17% | 61% |
| Spec 4 | 70% | 74% | 70% | 70% | 74% | 70% | 70% | 74% | 70% |
| Cutoff 5 | 0.0141 | 0.00723 | 0.00681 | 0.0141 | 0.00723 | 0.00681 | 0.0141 | 0.00723 | 0.00681 |
| Sens 5 | 11% | 21% | 13% | 19% | 11% | 31% | 0% | 17% | 17% |
| Spec 5 | 81% | 81% | 80% | 81% | 81% | 80% | 81% | 81% | 80% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 6 | 0.0144 | 0.0144 | 0.0144 | 0.0144 | 0.0144 | 0.0144 | 0.0144 | 0.0144 | 0.0144 |
| Sens 6 | 0% | 0% | 0% | 2% | 5% | 2% | 0% | 0% | 0% |
| Spec 6 | 95% | 97% | 99% | 95% | 97% | 99% | 95% | 97% | 99% |
| OR Quart 2 | 0.59 | 0.33 | 1.3 | 2.3 | 1.0 | 1.3 | 18 | 1.5 | 1.0 |
| p Value | 0.39 | 0.34 | 0.56 | 0.073 | 0.99 | 0.63 | 0.0069 | 0.65 | 1.0 |
| 95% CI of | 0.18 | 0.033 | 0.50 | 0.93 | 0.20 | 0.45 | 2.2 | 0.25 | 0.23 |
| OR Quart2 | 2.0 | 3.2 | 3.6 | 5.7 | 5.2 | 3.7 | 150 | 9.4 | 4.4 |
| OR Quart 3 | 7.4 | 3.4 | 1.0 | 1.3 | 4.6 | 2.2 | 5.6 | 3.8 | 2.3 |
| p Value | 8.9E-5 | 0.080 | 1.0 | 0.63 | 0.022 | 0.13 | 0.12 | 0.11 | 0.21 |
| 95% CI of | 2.7 | 0.87 | 0.36 | 0.49 | 1.2 | 0.79 | 0.62 | 0.76 | 0.62 |
| OR Quart3 | 20 | 13 | 2.8 | 3.2 | 17 | 6.1 | 51 | 19 | 8.7 |
| OR Quart 4 | 1.0 | 0.33 | 1.5 | 1.0 | 0.33 | 2.9 | 7.2 | 0 | 2.0 |
| p Value | 0.96 | 0.34 | 0.41 | 1.0 | 0.34 | 0.038 | 0.074 | na | 0.33 |
| 95% CI of | 0.35 | 0.033 | 0.56 | 0.38 | 0.033 | 1.1 | 0.82 | na | 0.51 |
| OR Quart4 | 3.1 | 3.2 | 4.1 | 2.6 | 3.2 | 7.9 | 64 | na | 7.5 |

**KSP-Cadherin**

| sCr or UO | 0hr prior to AKI stage | | 24hr prior to AKI stage | | | 48hr prior to AKI stage | |
|---|---|---|---|---|---|---|---|
| | Cohort 1 | Cohort 2 | Cohort 1 | | Cohort 2 | Cohort 1 | Cohort 2 |
| Median | 1.03 | 1.24 | 1.03 | | 1.15 | nd | nd |
| Average | 1.47 | 1.58 | 1.47 | | 1.90 | nd | nd |
| Stdev | 1.59 | 1.15 | 1.59 | | 1.74 | nd | nd |
| p(t-test) | | 0.71 | | | 0.25 | nd | nd |
| Min | 0.00263 | 0.0646 | 0.00263 | | 0.291 | nd | nd |

| KSP-Cadherin | | | | | | | |
|---|---|---|---|---|---|---|---|
| sCr or UO | 0hr prior to AKI stage | | | 24hr prior to AKI stage | | 48hr prior to AKI stage | |
| | Cohort 1 | Cohort 2 | Cohort 1 | | Cohort 2 | Cohort 1 | Cohort 2 |
| Max | 11.9 | 4.63 | 11.9 | | 7.51 | nd | nd |
| n (Samp) | 85 | 32 | 85 | 25 | | nd | nd |
| n (Patient) | 68 | 32 | 68 | 25 | | nd | nd |
| | | | | | | | |
| sCr only | 0hr prior to AKI stage | | | 24hr prior to AKI stage | | 48hr prior to AKI stage | |
| | Cohort 1 | Cohort 2 | Cohort 1 | | Cohort 2 | Cohort 1 | Cohort 2 |
| Median | 1.10 | 0.683 | 1.10 | | 1.04 | nd | nd |
| Average | 1.58 | 1.36 | 1.58 | | 1.60 | nd | nd |
| Stdev | 1.55 | 1.28 | 1.55 | | 1.45 | nd | nd |
| p(t-test) | | 0.66 | | | 0.98 | nd | nd |
| Min | 0.00263 | 0.0646 | 0.00263 | | 0.291 | nd | nd |
| Max | 11.9 | 3.49 | 11.9 | | 4.08 | nd | nd |
| n (Samp) | 152 | 10 | 152 | | 6 | nd | nd |
| n (Patient) | 114 | 10 | 114 | | 6 | nd | nd |
| UO only | 0hr prior to AKI stage | | | 24hr prior to AKI stage | | 48hr prior to AKI stage | |
| | Cohort 1 | Cohort 2 | Cohort 1 | Cohort 2 | | Cohort 1 | Cohort 2 |
| Median | 1.02 | 1.40 | 1.02 | 1.61 | | nd | nd |
| Average | 1.55 | 1.73 | 1.55 | 2.07 | | nd | nd |
| Stdev | 1.72 | 1.11 | 1.72 | 1.77 | | nd | nd |
| p(t-test) | | 0.61 | | 0.19 | | nd | nd |
| Min | 0.00263 | 0.557 | 0.00263 | 0.371 | | nd | nd |
| Max | 11.9 | 4.63 | 11.9 | 7.51 | | nd | nd |
| n (Samp) | 73 | 27 | 73 | 25 | | nd | nd |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Patient) | 59 | 27 | 59 | 25 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.57 | 0.44 | 0.62 | 0.58 | 0.50 | 0.60 | nd | nd | nd |
| SE | 0.061 | 0.097 | 0.065 | 0.067 | 0.12 | 0.068 | nd | nd | nd |
| p | 0.25 | 0.53 | 0.068 | 0.26 | 1.00 | 0.12 | nd | nd | nd |
| nCohort 1 | 85 | 152 | 73 | 85 | 152 | 73 | nd | nd | nd |
| nCohort 2 | 32 | 10 | 27 | 25 | 6 | 25 | nd | nd | nd |
| Cutoff 1 | 0.946 | 0.622 | 0.996 | 0.845 | 0.569 | 0.845 | nd | nd | nd |
| Sens 1 | 72% | 70% | 70% | 72% | 83% | 72% | nd | nd | nd |
| Spec 1 | 46% | 25% | 48% | 40% | 22% | 41% | nd | nd | nd |
| Cutoff 2 | 0.734 | 0.380 | 0.935 | 0.569 | 0.569 | 0.723 | nd | nd | nd |
| Sens 2 | 81% | 80% | 81% | 80% | 83% | 80% | nd | nd | nd |
| Spec 2 | 35% | 14% | 47% | 22% | 22% | 34% | nd | nd | nd |
| Cutoff 3 | 0.555 | 0.0646 | 0.739 | 0.398 | 0.252 | 0.426 | nd | nd | nd |
| Sens 3 | 91% | 90% | 93% | 92% | 100% | 92% | nd | nd | nd |
| Spec 3 | 21% | 3% | 36% | 15% | 8% | 16% | nd | nd | nd |
| Cutoff 4 | 1.66 | 1.69 | 1.71 | 1.66 | 1.69 | 1.71 | nd | nd | nd |
| Sens 4 | 28% | 40% | 30% | 40% | 33% | 40% | nd | nd | nd |
| Spec 4 | 71% | 70% | 71% | 71% | 70% | 71% | nd | nd | nd |
| Cutoff 5 | 2.10 | 2.45 | 2.19 | 2.10 | 2.45 | 2.19 | nd | nd | nd |
| Sens 5 | 25% | 30% | 26% | 36% | 33% | 36% | nd | nd | nd |
| Spec 5 | 80% | 80% | 81% | 80% | 80% | 81% | nd | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 6 | 2.74 | 3.39 | 3.17 | 2.74 | 3.39 | 3.17 | nd | nd | nd |
| Sens 6 | 16% | 10% | 11% | 16% | 17% | 20% | nd | nd | nd |
| Spec 6 | 91% | 90% | 90% | 91% | 90% | 90% | nd | nd | nd |
| OR Quart 2 | 2.2 | 0.32 | 16 | 0.76 | 0.47 | 0.95 | nd | nd | nd |
| p Value | 0.22 | 0.34 | 0.012 | 0.69 | 0.55 | 0.94 | nd | nd | nd |
| 95% CI of | 0.62 | 0.032 | 1.9 | 0.20 | 0.041 | 0.24 | nd | nd | nd |
| OR Quart2 | 7.5 | 3.3 | 140 | 2.9 | 5.5 | 3.8 | nd | nd | nd |
| OR Quart 3 | 2.5 | 1.0 | 14 | 0.80 | 0.49 | 1.3 | nd | nd | nd |
| p Value | 0.14 | 1.0 | 0.018 | 0.74 | 0.56 | 0.73 | nd | nd | nd |
| 95% CI of | 0.74 | 0.19 | 1.6 | 0.21 | 0.042 | 0.33 | nd | nd | nd |
| OR Quart3 | 8.6 | 5.3 | 120 | 3.0 | 5.6 | 4.9 | nd | nd | nd |
| OR Quart 4 | 1.7 | 1.0 | 9.3 | 1.7 | 0.97 | 2.1 | nd | nd | nd |
| p Value | 0.39 | 0.97 | 0.045 | 0.41 | 0.98 | 0.24 | nd | nd | nd |
| 95% CI of | 0.50 | 0.19 | 1.1 | 0.50 | 0.13 | 0.59 | nd | nd | nd |
| OR Quart4 | 6.1 | 5.4 | 83 | 5.5 | 7.3 | 7.7 | nd | nd | nd |

**Tumor necrosis factor receptor superfamily member 10B**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.124 | 0.573 | 0.124 | 0.395 | nd | nd |
| Average | 0.891 | 1.21 | 0.891 | 1.09 | nd | nd |
| Stdev | 1.49 | 1.55 | 1.49 | 1.82 | nd | nd |

(continued)

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| p(t-test) | | 0.43 | | 0.64 | nd | nd |
| Min | 0.00360 | 0.0182 | 0.00360 | 0.00464 | nd | nd |
| Max | 6.71 | 4.65 | 6.71 | 6.48 | nd | nd |
| n (Samp) | 52 | 20 | 52 | 20 | nd | nd |
| n (Patient) | 41 | 20 | 41 | 20 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.106 | 0.709 | 0.106 | 0.464 | nd | nd |
| Average | 0.595 | 1.40 | 0.595 | 1.25 | nd | nd |
| Stdev | 1.03 | 1.68 | 1.03 | 1.96 | nd | nd |
| p(t-test) | | 0.031 | | 0.087 | nd | nd |
| Min | 0.00360 | 0.0573 | 0.00360 | 0.0172 | nd | nd |
| Max | 4.38 | 4.65 | 4.38 | 6.48 | nd | nd |
| n (Samp) | 42 | 16 | 42 | 21 | nd | nd |
| n (Patient) | 33 | 16 | 33 | 21 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.64 | nd | 0.71 | 0.62 | nd | 0.68 | nd | nd | nd |
| SE | 0.076 | nd | 0.081 | 0.076 | nd | 0.074 | nd | nd | nd |
| p | 0.064 | nd | 0.0099 | 0.12 | nd | 0.014 | nd | nd | nd |
| nCohort 1 | 52 | nd | 42 | 52 | nd | 42 | nd | nd | nd |
| nCohort 2 | 20 | nd | 16 | 20 | nd | 21 | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 1 | 0.182 | nd | 0.107 | 0.154 | nd | 0.154 | nd | nd | nd |
| Sens 1 | 70% | nd | 75% | 70% | nd | 71% | nd | nd | nd |
| Spec 1 | 54% | nd | 52% | 52% | nd | 55% | nd | nd | nd |
| Cutoff 2 | 0.0761 | nd | 0.0761 | 0.141 | nd | 0.141 | nd | nd | nd |
| Sens 2 | 80% | nd | 81% | 80% | nd | 81% | nd | nd | nd |
| Spec 2 | 40% | nd | 40% | 52% | nd | 55% | nd | nd | nd |
| Cutoff 3 | 0.0591 | nd | 0.0591 | 0.0796 | nd | 0.101 | nd | nd | nd |
| Sens 3 | 90% | nd | 94% | 90% | nd | 90% | nd | nd | nd |
| Spec 3 | 38% | nd | 38% | 44% | nd | 48% | nd | nd | nd |
| Cutoff 4 | 0.786 | nd | 0.398 | 0.786 | nd | 0.398 | nd | nd | nd |
| Sens 4 | 45% | nd | 56% | 30% | nd | 52% | nd | nd | nd |
| Spec 4 | 71% | nd | 71% | 71% | nd | 71% | nd | nd | nd |
| Cutoff 5 | 1.56 | nd | 0.819 | 1.56 | nd | 0.819 | nd | nd | nd |
| Sens 5 | 25% | nd | 50% | 15% | nd | 33% | nd | nd | nd |
| Spec 5 | 81% | nd | 81% | 81% | nd | 81% | nd | nd | nd |
| Cutoff 6 | 3.11 | nd | 2.19 | 3.11 | nd | 2.19 | nd | nd | nd |
| Sens 6 | 15% | nd | 25% | 10% | nd | 14% | nd | nd | nd |
| Spec 6 | 90% | nd | 90% | 90% | nd | 90% | nd | nd | nd |
| OR Quart 2 | 8.5 | nd | >7.0 | 11 | nd | 11 | nd | nd | nd |
| p Value | 0.061 | nd | <0.097 | 0.036 | nd | 0.038 | nd | nd | nd |
| 95% CI of | 0.90 | nd | >0.71 | 1.2 | nd | 1.1 | nd | nd | nd |
| OR Quart2 | 80 | nd | na | 100 | nd | 100 | nd | nd | nd |
| OR Quart 3 | 11 | nd | >3.8 | 11 | nd | 8.4 | nd | nd | nd |
| p Value | 0.036 | nd | <0.27 | 0.036 | nd | 0.066 | nd | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| 95% CI of OR Quart3 | 1.2 | nd | >0.35 | 1.2 | nd | 0.87 | nd | nd | nd |
| | 100 | nd | na | 100 | nd | 81 | nd | nd | nd |
| OR Quart 4 | 8.5 | nd | >16 | 6.5 | nd | 11 | nd | nd | nd |
| p Value | 0.061 | nd | <0.017 | 0.10 | nd | 0.038 | nd | nd | nd |
| 95% CI of OR Quart4 | 0.90 | nd | >1.7 | 0.68 | nd | 1.1 | nd | nd | nd |
| | 80 | nd | na | 63 | nd | 100 | nd | nd | nd |

Table 2: Comparison of marker levels in urine samples collected from Cohort 1 (patients that did not progress beyond RIFLE stage 0 or R) and in urine samples collected from subjects at 0, 24 hours, and 48 hours prior to reaching stage I or F in Cohort 2.

| Apolipoprotein A-II | | | | | | |
|---|---|---|---|---|---|---|
| sCr or UO | 0hr prior to AKI stage | | 24hr prior to AKI stage | | 48hr prior to AKI stage | |
|  | Cohort 1 | Cohort 2 | Cohort 1 | Cohort 2 | Cohort 1 | Cohort 2 |
| Median | 73.2 | 102 | 73.2 | 116 | 73.2 | 81.7 |
| Average | 185 | 272 | 185 | 697 | 185 | 87.4 |
| Stdev | 645 | 1020 | 645 | 3530 | 645 | 69.4 |
| p(t-test) |  | 0.44 |  | 0.0018 |  | 0.44 |
| Min | 2.08 | 8.69 | 2.08 | 10.8 | 2.08 | 1.00E-9 |
| Max | 6400 | 6400 | 6400 | 24300 | 6400 | 301 |
| n (Samp) | 685 | 38 | 685 | 47 | 685 | 26 |
| n (Patient) | 283 | 38 | 283 | 47 | 283 | 26 |
|  | | | | | | |
| sCr only | 0hr prior to AKI stage | | 24hr prior to AKI stage | | 48hr prior to AKI stage | |
|  | Cohort 1 | Cohort 2 | Cohort 1 | Cohort 2 | Cohort 1 | Cohort 2 |
| Median | 76.6 | 106 | 76.6 | 127 | 76.6 | 98.8 |
| Average | 198 | 891 | 198 | 176 | 198 | 108 |
| Stdev | 990 | 2230 | 990 | 158 | 990 | 84.1 |
| p(t-test) |  | 0.053 |  | 0.93 |  | 0.74 |
| Min | 1.00E-9 | 34.5 | 1.00E-9 | 24.0 | 1.00E-9 | 6.18 |
| Max | 24300 | 6400 | 24300 | 613 | 24300 | 288 |
| n (Samp) | 891 | 8 | 891 | 13 | 891 | 13 |
| n (Patient) | 334 | 8 | 334 | 13 | 334 | 13 |
|  | | | | | | |
| UO only | 0hr prior to AKI stage | | 24hr prior to AKI stage | | 48hr prior | to AKI stage |
|  | Cohort 1 | Cohort 2 | Cohort 1 | Cohort 2 | Cohort 1 | Cohort 2 |
| Median | 76.6 | 107 | 76.6 | 131 | 76.6 | 98.7 |

(continued)

| UO only | 0hr prior to AKI stage | | 24hr prior to AKI stage | | | 48hr prior | to AKI stage |
|---|---|---|---|---|---|---|---|
| | Cohort 1 | Cohort 2 | Cohort 1 | Cohort 2 | | Cohort 1 | Cohort 2 |
| Average | 195 | 297 | 195 | 789 | | 195 | 105 |
| Stdev | 649 | 1060 | 649 | 3780 | | 649 | 74.2 |
| p(t-test) | | 0.39 | | 0.0017 | | | 0.52 |
| Min | 2.08 | 8.69 | 2.08 | 10.8 | | 2.08 | 1.00E-9 |
| Max | 6400 | 6400 | 6400 | 24300 | | 6400 | 301 |
| n (Samp) | 553 | 35 | 553 | 41 | | 553 | 22 |
| n (Patient) | 202 | 35 | 202 | 41 | | 202 | 22 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.59 | 0.60 | 0.60 | 0.66 | 0.66 | 0.67 | 0.47 | 0.51 | 0.53 |
| SE | 0.050 | 0.11 | 0.052 | 0.045 | 0.083 | 0.048 | 0.059 | 0.081 | 0.064 |
| p | 0.069 | 0.36 | 0.053 | 3.3E-4 | 0.055 | 2.3E-4 | 0.60 | 0.87 | 0.61 |
| nCohort 1 | 685 | 891 | 553 | 685 | 891 | 553 | 685 | 891 | 553 |
| nCohort 2 | 38 | 8 | 35 | 47 | 13 | 41 | 26 | 13 | 22 |
| Cutoff 1 | 70.0 | 59.9 | 76.9 | 85.8 | 85.8 | 99.4 | 39.3 | 39.3 | 56.5 |
| Sens 1 | 71% | 75% | 71% | 70% | 77% | 71% | 73% | 77% | 73% |
| Spec 1 | 48% | 37% | 50% | 59% | 56% | 63% | 22% | 21% | 35% |
| Cutoff 2 | 56.5 | 49.4 | 69.8 | 66.5 | 66.5 | 72.6 | 31.3 | 31.3 | 43.2 |
| Sens 2 | 82% | 88% | 80% | 81% | 85% | 80% | 81% | 85% | 82% |
| Spec 2 | 38% | 30% | 45% | 46% | 43% | 47% | 14% | 13% | 22% |
| Cutoff 3 | 33.5 | 34.3 | 33.5 | 43.2 | 43.2 | 44.8 | 11.0 | 16.2 | 27.1 |
| Sens 3 | 92% | 100% | 91% | 91% | 92% | 90% | 92% | 92% | 91% |
| Spec 3 | 16% | 16% | 14% | 26% | 24% | 24% | 2% | 3% | 9% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 4 | 110 | 122 | 118 | 110 | 122 | 118 | 110 | 122 | 118 |
| Sens 4 | 42% | 50% | 46% | 53% | 62% | 54% | 31% | 38% | 36% |
| Spec 4 | 70% | 70% | 70% | 70% | 70% | 70% | 70% | 70% | 70% |
| Cutoff 5 | 148 | 154 | 154 | 148 | 154 | 154 | 148 | 154 | 154 |
| Sens 5 | 18% | 38% | 20% | 38% | 31% | 41% | 19% | 23% | 14% |
| Spec 5 | 80% | 80% | 80% | 80% | 80% | 80% | 80% | 80% | 80% |
| Cutoff 6 | 226 | 229 | 248 | 226 | 229 | 248 | 226 | 229 | 248 |
| Sens 6 | 11% | 12% | 11% | 21% | 31% | 24% | 4% | 8% | 5% |
| Spec 6 | 90% | 90% | 90% | 90% | 90% | 90% | 90% | 90% | 90% |
| OR Quart 2 | 1.2 | 2.0 | 1.2 | 1.4 | 0.50 | 0.49 | 1.3 | 0 | 0.79 |
| p Value | 0.79 | 0.57 | 0.76 | 0.56 | 0.57 | 0.31 | 0.59 | na | 0.73 |
| 95% CI of | 0.38 | 0.18 | 0.36 | 0.44 | 0.045 | 0.12 | 0.46 | na | 0.21 |
| OR Quart2 | 3.5 | 22 | 4.1 | 4.5 | 5.5 | 2.0 | 4.0 | na | 3.0 |
| OR Quart 3 | 2.2 | 2.0 | 2.5 | 3.2 | 2.5 | 2.3 | 0.49 | 0.59 | 1.2 |
| p Value | 0.11 | 0.57 | 0.090 | 0.028 | 0.27 | 0.10 | 0.32 | 0.48 | 0.77 |
| 95% CI of | 0.83 | 0.18 | 0.87 | 1.1 | 0.49 | 0.84 | 0.12 | 0.14 | 0.36 |
| OR Quart3 | 6.0 | 22 | 7.4 | 8.9 | 13 | 6.2 | 2.0 | 2.5 | 4.0 |
| OR Quart 4 | 2.1 | 3.0 | 2.5 | 4.4 | 2.5 | 3.5 | 1.5 | 1.0 | 1.4 |
| p Value | 0.16 | 0.34 | 0.090 | 0.0040 | 0.27 | 0.010 | 0.43 | 1.0 | 0.57 |
| 95% CI of | 0.76 | 0.31 | 0.87 | 1.6 | 0.49 | 1.3 | 0.53 | 0.29 | 0.44 |
| OR Quart4 | 5.6 | 29 | 7.4 | 12 | 13 | 8.9 | 4.4 | 3.5 | 4.6 |

**Caspase-1**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.02 | 0.826 | nd | nd | nd | nd |
| Average | 1.20 | 0.896 | nd | nd | nd | nd |
| Stdev | 1.22 | 0.687 | nd | nd | nd | nd |
| p(t-test) | | 0.49 | nd | nd | nd | nd |
| Min | 0.0223 | 0.0223 | nd | nd | nd | nd |
| Max | 4.68 | 2.33 | nd | nd | nd | nd |
| n (Samp) | 55 | 8 | nd | nd | nd | nd |
| n (Patient) | 35 | 8 | nd | nd | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.09 | 0.826 | nd | nd | nd | nd |
| Average | 1.20 | 0.896 | nd | nd | nd | nd |
| Stdev | 1.19 | 0.687 | nd | nd | nd | nd |
| p(t-test) | | 0.50 | nd | nd | nd | nd |
| Min | 0.0223 | 0.0223 | nd | nd | nd | nd |
| Max | 4.46 | 2.33 | nd | nd | nd | nd |
| n (Samp) | 41 | 8 | nd | nd | nd | nd |
| n (Patient) | 24 | 8 | nd | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.46 | nd | 0.46 | nd | nd | nd | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| SE | 0.11 | nd | 0.11 | nd | nd | nd | nd | nd | nd |
| p | 0.74 | nd | 0.76 | nd | nd | nd | nd | nd | nd |
| nCohort 1 | 55 | nd | 41 | nd | nd | nd | nd | nd | nd |
| nCohort 2 | 8 | nd | 8 | nd | nd | nd | nd | nd | nd |
| Cutoff 1 | 0.496 | nd | 0.496 | nd | nd | nd | nd | nd | nd |
| Sens 1 | 75% | nd | 75% | nd | nd | nd | nd | nd | nd |
| Spec 1 | 40% | nd | 41% | nd | nd | nd | nd | nd | nd |
| Cutoff 2 | 0.298 | nd | 0.298 | nd | nd | nd | nd | nd | nd |
| Sens 2 | 88% | nd | 88% | nd | nd | nd | nd | nd | nd |
| Spec 2 | 36% | nd | 37% | nd | nd | nd | nd | nd | nd |
| Cutoff 3 | 0 | nd | 0 | nd | nd | nd | nd | nd | nd |
| Sens 3 | 100% | nd | 100% | nd | nd | nd | nd | nd | nd |
| Spec 3 | 0% | nd | 0% | nd | nd | nd | nd | nd | nd |
| Cutoff 4 | 1.54 | nd | 1.54 | nd | nd | nd | nd | nd | nd |
| Sens 4 | 12% | nd | 12% | nd | nd | nd | nd | nd | nd |
| Spec 4 | 71% | nd | 71% | nd | nd | nd | nd | nd | nd |
| Cutoff 5 | 2.07 | nd | 2.07 | nd | nd | nd | nd | nd | nd |
| Sens 5 | 12% | nd | 12% | nd | nd | nd | nd | nd | nd |
| Spec 5 | 82% | nd | 85% | nd | nd | nd | nd | nd | nd |
| Cutoff 6 | 2.59 | nd | 2.59 | nd | nd | nd | nd | nd | nd |
| Sens 6 | 0% | nd | 0% | nd | nd | nd | nd | nd | nd |
| Spec 6 | 91% | nd | 90% | nd | nd | nd | nd | nd | nd |
| OR Quart 2 | 1.0 | nd | 2.4 | nd | nd | nd | nd | nd | nd |
| p Value | 1.0 | nd | 0.50 | nd | nd | nd | nd | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| 95% CI of | 0.057 | nd | 0.19 | nd | nd | nd | nd | nd | nd |
| OR Quart2 | 18 | nd | 31 | nd | nd | nd | nd | nd | nd |
| OR Quart 3 | 6.8 | nd | 6.0 | nd | nd | nd | nd | nd | nd |
| p Value | 0.099 | nd | 0.14 | nd | nd | nd | nd | nd | nd |
| 95% CI of | 0.69 | nd | 0.56 | nd | nd | nd | nd | nd | nd |
| OR Quart3 | 67 | nd | 64 | nd | nd | nd | nd | nd | nd |
| OR Quart 4 | 1.1 | nd | 1.1 | nd | nd | nd | nd | nd | nd |
| p Value | 0.96 | nd | 0.95 | nd | nd | nd | nd | nd | nd |
| 95% CI of | 0.061 | nd | 0.061 | nd | nd | nd | nd | nd | nd |
| OR Quart4 | 19 | nd | 20 | nd | nd | nd | nd | nd | nd |

**Caspase-9**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.469 | 0.547 | nd | nd | nd | nd |
| Average | 0.965 | 0.998 | nd | nd | nd | nd |
| Stdev | 2.44 | 1.52 | nd | nd | nd | nd |
| p(t-test) | | 0.97 | nd | nd | nd | nd |
| Min | 0.0360 | 0.0360 | nd | nd | nd | nd |
| Max | 19.5 | 4.64 | nd | nd | nd | nd |
| n (Samp) | 78 | 8 | nd | nd | nd | nd |
| n (Patient) | 56 | 8 | nd | nd | nd | nd |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.469 | 0.547 | nd | nd | nd | nd |
| Average | 1.12 | 0.998 | nd | nd | nd | nd |
| Stdev | 2.73 | 1.52 | nd | nd | nd | nd |
| p(t-test) | | 0.90 | nd | nd | nd | nd |
| | | | | | | |
| Min | 0.0360 | 0.0360 | nd | nd | nd | nd |
| Max | 19.5 | 4.64 | nd | nd | nd | nd |
| n (Samp) | 61 | 8 | nd | nd | nd | nd |
| n (Patient) | 43 | 8 | nd | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.54 | nd | 0.50 | nd | nd | nd | nd | nd | nd |
| SE | 0.11 | nd | 0.11 | nd | nd | nd | nd | nd | nd |
| p | 0.73 | nd | 0.98 | nd | nd | nd | nd | nd | nd |
| nCohort 1 | 78 | nd | 61 | nd | nd | nd | nd | nd | nd |
| nCohort 2 | 8 | nd | 8 | nd | nd | nd | nd | nd | nd |
| Cutoff 1 | 0.208 | nd | 0.208 | nd | nd | nd | nd | nd | nd |
| Sens 1 | 75% | nd | 75% | nd | nd | nd | nd | nd | nd |
| Spec 1 | 36% | nd | 28% | nd | nd | nd | nd | nd | nd |
| Cutoff 2 | 0 | nd | 0 | nd | nd | nd | nd | nd | nd |
| Sens 2 | 100% | nd | 100% | nd | nd | nd | nd | nd | nd |
| Spec 2 | 0% | nd | 0% | nd | nd | nd | nd | nd | nd |
| Cutoff 3 | 0 | nd | 0 | nd | nd | nd | nd | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 3 | 100% | nd | 100% | nd | nd | nd | nd | nd | nd |
| Spec 3 | 0% | nd | 0% | nd | nd | nd | nd | nd | nd |
| Cutoff 4 | 0.781 | nd | 0.781 | nd | nd | nd | nd | nd | nd |
| Sens 4 | 38% | nd | 38% | nd | nd | nd | nd | nd | nd |
| Spec 4 | 72% | nd | 70% | nd | nd | nd | nd | nd | nd |
| Cutoff 5 | 0.955 | nd | 0.955 | nd | nd | nd | nd | nd | nd |
| Sens 5 | 25% | nd | 25% | nd | nd | nd | nd | nd | nd |
| Spec 5 | 81% | nd | 80% | nd | nd | nd | nd | nd | nd |
| Cutoff 6 | 1.66 | nd | 1.49 | nd | nd | nd | nd | nd | nd |
| Sens 6 | 12% | nd | 12% | nd | nd | nd | nd | nd | nd |
| Spec 6 | 91% | nd | 90% | nd | nd | nd | nd | nd | nd |
| OR Quart 2 | 0.95 | nd | 0.47 | nd | nd | nd | nd | nd | nd |
| p Value | 0.96 | nd | 0.55 | nd | nd | nd | nd | nd | nd |
| 95% CI of OR Quart2 | 0.12 | nd | 0.038 | nd | nd | nd | nd | nd | nd |
| OR Quart2 | 7.4 | nd | 5.7 | nd | nd | nd | nd | nd | nd |
| OR Quart 3 | 1.0 | nd | 1.0 | nd | nd | nd | nd | nd | nd |
| p Value | 1.0 | nd | 1.0 | nd | nd | nd | nd | nd | nd |
| 95% CI of OR Quart3 | 0.13 | nd | 0.12 | nd | nd | nd | nd | nd | nd |
| OR Quart3 | 7.9 | nd | 8.1 | nd | nd | nd | nd | nd | nd |
| OR Quart 4 | 0.95 | nd | 1.5 | nd | nd | nd | nd | nd | nd |
| p Value | 0.96 | nd | 0.68 | nd | nd | nd | nd | nd | nd |
| 95% CI of OR Quart4 | 0.12 | nd | 0.22 | nd | nd | nd | nd | nd | nd |
| OR Quart4 | 7.4 | nd | 10 | nd | nd | nd | nd | nd | nd |

**Cadherin-1**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 57300 | 62400 | nd | nd |
| Average | nd | nd | 102000 | 78500 | nd | nd |
| Stdev | nd | nd | 128000 | 81700 | nd | nd |
| p(t-test) | nd | nd | | 0.47 | nd | nd |
| Min | nd | nd | 160 | 1620 | nd | nd |
| Max | nd | nd | 744000 | 260000 | nd | nd |
| n (Samp) | nd | nd | 96 | 16 | nd | nd |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Patient) | nd | nd | 73 | 16 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 58600 | 59500 | nd | nd |
| Average | nd | nd | 104000 | 82000 | nd | nd |
| Stdev | nd | nd | 134000 | 82800 | nd | nd |
| p(t-test) | nd | nd | | 0.54 | nd | nd |
| Min | nd | nd | 160 | 1620 | nd | nd |
| Max | nd | nd | 744000 | 260000 | nd | nd |
| n (Samp) | nd | nd | 81 | 15 | nd | nd |
| n (Patient) | nd | nd | 61 | 15 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.44 | nd | 0.47 | nd | nd | nd |
| SE | nd | nd | nd | 0.080 | nd | 0.082 | nd | nd | nd |
| p | nd | nd | nd | 0.49 | nd | 0.72 | nd | nd | nd |
| nCohort 1 | nd | nd | nd | 96 | nd | 81 | nd | nd | nd |
| nCohort 2 | nd | nd | nd | 16 | nd | 15 | nd | nd | nd |
| Cutoff 1 | nd | nd | nd | 12500 | nd | 12900 | nd | nd | nd |
| Sens 1 | nd | nd | nd | 75% | nd | 73% | nd | nd | nd |
| Spec 1 | nd | nd | nd | 19% | nd | 22% | nd | nd | nd |
| Cutoff 2 | nd | nd | nd | 9460 | nd | 11700 | nd | nd | nd |
| Sens 2 | nd | nd | nd | 81% | nd | 80% | nd | nd | nd |
| Spec 2 | nd | nd | nd | 11% | nd | 22% | nd | nd | nd |
| Cutoff 3 | nd | nd | nd | 1660 | nd | 1660 | nd | nd | nd |
| Sens 3 | nd | nd | nd | 94% | nd | 93% | nd | nd | nd |
| Spec 3 | nd | nd | nd | 2% | nd | 2% | nd | nd | nd |
| Cutoff 4 | nd | nd | nd | 128000 | nd | 125000 | nd | nd | nd |
| Sens 4 | nd | nd | nd | 25% | nd | 27% | nd | nd | nd |
| Spec 4 | nd | nd | nd | 71% | nd | 70% | nd | nd | nd |
| Cutoff 5 | nd | nd | nd | 141000 | nd | 144000 | nd | nd | nd |
| Sens 5 | nd | nd | nd | 25% | nd | 27% | nd | nd | nd |
| Spec 5 | nd | nd | nd | 80% | nd | 80% | nd | nd | nd |
| Cutoff 6 | nd | nd | nd | 219000 | nd | 219000 | nd | nd | nd |
| Sens 6 | nd | nd | nd | 6% | nd | 7% | nd | nd | nd |
| Spec 6 | nd | nd | nd | 91% | nd | 90% | nd | nd | nd |
| OR Quart 2 | nd | nd | nd | 1.3 | nd | 1.0 | nd | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p Value | nd | nd | nd | 0.72 | nd | 1.0 | nd | nd | nd |
| 95% CI of | nd | nd | nd | 0.31 | nd | 0.22 | nd | nd | nd |
| OR Quart2 | nd | nd | nd | 5.5 | nd | 4.6 | nd | nd | nd |
| OR Quart 3 | nd | nd | nd | 0 | nd | 0.45 | nd | nd | nd |
| p Value | nd | nd | nd | na | nd | 0.39 | nd | nd | nd |
| 95% CI of | nd | nd | nd | na | nd | 0.075 | nd | nd | nd |
| OR Quart3 | nd | nd | nd | na | nd | 2.8 | nd | nd | nd |
| OR Quart 4 | nd | nd | nd | 2.0 | nd | 1.3 | nd | nd | nd |
| p Value | nd | nd | nd | 0.32 | nd | 0.71 | nd | nd | nd |
| 95% CI of | nd | nd | nd | 0.51 | nd | 0.31 | nd | nd | nd |
| OR Quart4 | nd | nd | nd | 7.8 | nd | 5.6 | nd | nd | nd |

**Cyclin-dependent kinase inhibitor 1**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.09 | 3.35 | 1.09 | 4.76 | 1.09 | 2.45 |
| Average | 62.1 | 42.8 | 62.1 | 57.1 | 62.1 | 13.0 |
| Stdev | 497 | 133 | 497 | 173 | 497 | 18.2 |
| p(t-test) | | 0.85 | | 0.95 | | 0.68 |
| Min | 1.14E-14 | 1.70E-14 | 1.14E-14 | 0.116 | 1.14E-14 | 0.116 |
| Max | 6950 | 630 | 6950 | 907 | 6950 | 52.9 |
| n (Samp) | 302 | 24 | 302 | 35 | 302 | 17 |
| n (Patient) | 164 | 24 | 164 | 35 | 164 | 17 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 1.23 | 7.84 | 1.23 | 9.80 |
| Average | nd | nd | 58.3 | 15.2 | 58.3 | 15.4 |
| Stdev | nd | nd | 447 | 17.6 | 447 | 16.5 |
| p(t-test) | nd | nd | | 0.80 | | 0.80 |
| Min | nd | nd | 1.14E-14 | 0.116 | 1.14E-14 | 0.116 |
| Max | nd | nd | 6950 | 42.1 | 6950 | 39.2 |
| n (Samp) | nd | nd | 380 | 7 | 380 | 7 |
| n (Patient) | nd | nd | 196 | 7 | 196 | 7 |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 0.850 | 3.35 | 0.850 | 2.87 | 0.850 | 2.66 |
| Average | 70.1 | 42.8 | 70.1 | 62.4 | 70.1 | 12.3 |
| Stdev | 538 | 133 | 538 | 183 | 538 | 17.6 |
| p(t-test) | | 0.80 | | 0.94 | | 0.67 |
| Min | 1.14E-14 | 1.70E-14 | 1.14E-14 | 0.116 | 1.14E-14 | 0.116 |
| Max | 6950 | 630 | 6950 | 907 | 6950 | 52.9 |
| n (Samp) | 257 | 24 | 257 | 31 | 257 | 16 |
| n (Patient) | 134 | 24 | 134 | 31 | 134 | 16 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.55 | nd | 0.54 | 0.59 | 0.64 | 0.58 | 0.55 | 0.60 | 0.59 |
| SE | 0.063 | nd | 0.063 | 0.053 | 0.11 | 0.056 | 0.074 | 0.11 | 0.077 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p | 0.46 | nd | 0.50 | 0.080 | 0.21 | 0.16 | 0.49 | 0.36 | 0.24 |
| nCohort 1 | 302 | nd | 257 | 302 | 380 | 257 | 302 | 380 | 257 |
| nCohort 2 | 24 | nd | 24 | 35 | 7 | 31 | 17 | 7 | 16 |
| Cutoff 1 | 1.70E-14 | nd | 1.70E-14 | 0.713 | 6.55 | 1.70E-14 | 1.70E-14 | 2.02 | 0.116 |
| Sens 1 | 96% | nd | 96% | 71% | 71% | 100% | 100% | 71% | 75% |
| Spec 1 | 2% | nd | 2% | 47% | 65% | 2% | 2% | 52% | 45% |
| Cutoff 2 | 1.70E-14 | nd | 1.70E-14 | 1.70E-14 | 0.850 | 1.70E-14 | 1.70E-14 | 1.70E-14 | 1.70E-14 |
| Sens 2 | 96% | nd | 96% | 100% | 86% | 100% | 100% | 100% | 100% |
| Spec 2 | 2% | nd | 2% | 2% | 47% | 2% | 2% | 2% | 2% |
| Cutoff 3 | 1.70E-14 | nd | 1.70E-14 | 1.70E-14 | 1.70E-14 | 1.70E-14 | 1.70E-14 | 1.70E-14 | 1.70E-14 |
| Sens 3 | 96% | nd | 96% | 100% | 100% | 100% | 100% | 100% | 100% |
| Spec 3 | 2% | nd | 2% | 2% | 2% | 2% | 2% | 2% | 2% |
| Cutoff 4 | 7.84 | nd | 7.29 | 7.84 | 9.35 | 7.29 | 7.84 | 9.35 | 7.29 |
| Sens 4 | 38% | nd | 38% | 43% | 29% | 42% | 41% | 57% | 44% |
| Spec 4 | 70% | nd | 70% | 70% | 71% | 70% | 70% | 71% | 70% |
| Cutoff 5 | 16.1 | nd | 13.9 | 16.1 | 18.3 | 13.9 | 16.1 | 18.3 | 13.9 |
| Sends 5 | 21% | nd | 21% | 29% | 29% | 32% | 24% | 43% | 31% |
| Spec 5 | 80% | nd | 80% | 80% | 80% | 80% | 80% | 80% | 80% |
| Cutoff 6 | 43.4 | nd | 44.8 | 43.4 | 48.9 | 44.8 | 43.4 | 48.9 | 44.8 |
| Sends 6 | 12% | nd | 12% | 11% | 0% | 13% | 12% | 0% | 12% |
| Spec 6 | 90% | nd | 90% | 90% | 90% | 90% | 90% | 90% | 90% |
| OR Quart 2 | 0.23 | nd | 0 | 1.4 | 0.99 | 2.9 | >7.6 | 0 | 0.24 |
| p Value | 0.067 | nd | na | 0.55 | 0.99 | 0.13 | <0.061 | na | 0.21 |
| 95% CI of | 0.047 | nd | na | 0.44 | 0.061 | 0.73 | >0.91 | na | 0.026 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart2 | 1.1 | nd | na | 4.7 | 16 | 11 | na | na | 2.2 |
| OR Quart | 3 0.86 | nd | 0.87 | 2.6 | 3.0 | 3.3 | >3.1 | 0.99 | 1.3 |
| p Value | 0.79 | nd | 0.80 | 0.082 | 0.34 | 0.084 | <0.33 | 0.99 | 0.73 |
| 95% CI of | 0.30 | nd | 0.32 | 0.88 | 0.31 | 0.85 | >0.31 | 0.14 | 0.33 |
| OR Quart3 | 2.5 | nd | 2.4 | 7.8 | 30 | 13 | na | 7.2 | 4.9 |
| OR Quart | 40.85 | nd | 0.74 | 2.3 | 2.0 | 4.1 | >7.6 | 1.5 | 1.5 |
| p Value | 0.77 | nd | 0.58 | 0.13 | 0.57 | 0.035 | <0.061 | 0.66 | 0.53 |
| 95% CI of | 0.29 | nd | 0.26 | 0.78 | 0.18 | 1.1 | >0.91 | 0.25 | 0.41 |
| OR Quart4 | 2,5 | nd | 2.1 | 7.1 | 22 | 16 | na | 9.2 | 5.7 |

**Carcinoembryonic antigen-related cell adhesion molecule 5**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.852 | 1.70 | 0.852 | 2.56 | 0.852 | 1.46 |
| Average | 12.6 | 4.03 | 12.6 | 5.68 | 12.6 | 4.91 |
| Stdev | 199 | 4.99 | 199 | 9.51 | 199 | 11.3 |
| p(t-test) |  | 0.83 |  | 0.84 |  | 0.87 |
| Min | 0.00336 | 0.00336 | 0.00336 | 0.0950 | 0.00336 | 0.148 |
| Max | 4070 | 19.5 | 4070 | 50.6 | 4070 | 47.4 |
| n (Samp) | 419 | 26 | 419 | 34 | 419 | 17 |
| n (Patient) | 164 | 26 | 164 | 34 | 164 | 17 |

(continued)

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 0.978 | 3.62 | 0.978 | 5.87 |
| Average | nd | nd | 11.1 | 5.25 | 11.1 | 6.39 |
| Stdev | nd | nd | 180 | 7.06 | 180 | 4.92 |
| p(t-test) | nd | nd | | 0.93 | | 0.94 |
| Min | nd | nd | 0.00336 | 0.102 | 0.00336 | 0.173 |
| Max | nd | nd | 4070 | 22.0 | 4070 | 14.6 |
| n(Samp) | nd | nd | 511 | 8 | 511 | 7 |
| n (Patient) | nd | nd | 198 | 8 | 198 | 7 |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 0.852 | 1.48 | 0.852 | 2.42 | 0.852 | 1.12 |
| Average | 14.6 | 3.87 | 14.6 | 5.34 | 14.6 | 4.41 |
| Stdev | 216 | 5.02 | 216 | 9.63 | 216 | 11.3 |
| p(t-test) | | 0.80 | | 0.81 | | 0.85 |
| Min | 0.00336 | 0.0573 | 0.00336 | 0.00336 | 0.00336 | 0.148 |
| Max | 4070 | 19.5 | 4070 | 50.6 | 4070 | 47.4 |
| n (Samp) | 355 | 26 | 355 | 30 | 355 | 17 |
| n (Patient) | 134 | 26 | 134 | 30 | 134 | 17 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.63 | nd | 0.61 | 0.67 | 0.65 | 0.66 | 0.57 | 0.76 | 0.54 |
| SE | 0.060 | nd | 0.061 | 0.052 | 0.11 | 0.056 | 0.074 | 0.11 | 0.073 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| p | 0.029 | nd | 0.059 | 0.0014 | 0.16 | 0.0056 | 0.34 | 0.016 | 0.58 |
| nCohort 1 | 419 | nd | 355 | 419 | 511 | 355 | 419 | 511 | 355 |
| nCohort 2 | 26 | nd | 26 | 34 | 8 | 30 | 17 | 7 | 17 |
| Cutoff 1 | 0.969 | nd | 0.816 | 0.942 | 2.26 | 0.942 | 0.607 | 3.47 | 0.491 |
| Sens 1 | 73% | nd | 73% | 71% | 75% | 70% | 71% | 71% | 71% |
| Spec 1 | 53% | nd | 50% | 52% | 73% | 51% | 43% | 80% | 39% |
| Cutoff 2 | 0.590 | nd | 0.590 | 0.546 | 0.214 | 0.684 | 0.279 | 2.55 | 0.279 |
| Sens 2 | 81% | nd | 81% | 82% | 88% | 80% | 82% | 86% | 82% |
| Spec 2 | 42% | nd | 43% | 41% | 17% | 47% | 25% | 74% | 25% |
| Cutoff 3 | 0.150 | nd | 0.150 | 0.214 | 0.0990 | 0.444 | 0.150 | 0.172 | 0.150 |
| Sens 3 | 92% | nd | 92% | 91% | 100% | 90% | 94% | 100% | 94% |
| Spec 3 | 12% | nd | 12% | 18% | 7% | 37% | 12% | 14% | 12% |
| Cutoff 4 | 1.76 | nd | 1.84 | 1.76 | 1.97 | 1.84 | 1.76 | 1.97 | 1.84 |
| Sens 4 | 50% | nd | 46% | 59% | 75% | 57% | 41% | 86% | 29% |
| Spec 4 | 70% | nd | 70% | 70% | 70% | 70% | 70% | 70% | 70% |
| Cutoff 5 | 3.16 | nd | 3.25 | 3.16 | 3.51 | 3.25 | 3.16 | 3.51 | 3.25 |
| Sens 5 | 38% | nd | 35% | 44% | 50% | 40% | 24% | 57% | 18% |
| Spec 5 | 80% | nd | 80% | 80% | 80% | 80% | 80% | 80% | 80% |
| Cutoff 6 | 8.38 | nd | 8.86 | 8.38 | 8.51 | 8.86 | 8.38 | 8.51 | 8.86 |
| Sens 6 | 15% | nd | 12% | 15% | 12% | 13% | 12% | 29% | 6% |
| Spec 6 | 90% | nd | 90% | 90% | 90% | 90% | 90% | 90% | 90% |
| OR Quart 2 | 1.3 | nd | 1.7 | 1.5 | 0 | 2.4 | 0.74 | 0 | 1.7 |
| p Value | 0.70 | nd | 0.47 | 0.52 | na | 0.21 | 0.70 | na | 0.47 |
| 95% CI of | 0.29 | nd | 0.40 | 0.42 | na | 0.61 | 0.16 | na | 0.40 |

EP 2 894 473 B1

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart2 | 6.2 | nd | 7.3 | 5.6 | na | 9.7 | 3.4 | na | 7.3 |
| OR Quart 3 | 3.2 | nd | 3.2 | 2.1 | 0.99 | 2.4 | 1.3 | 1.0 | 1.7 |
| p Value | 0.090 | nd | 0.088 | 0.24 | 0.99 | 0.21 | 0.73 | 1.0 | 0.47 |
| 95% CI of | 0.84 | nd | 0.84 | 0.61 | 0.14 | 0.61 | 0.33 | 0.062 | 0.40 |
| OR Quart3 | 12 | nd | 12 | 7.1 | 7.2 | 9.7 | 4.8 | 16 | 7.3 |
| OR Quart 4 | 3.5 | nd | 3.2 | 4.4 | 2.0 | 4.8 | 1.3 | 5.1 | 1.3 |
| p Value | 0.061 | nd | 0.091 | 0.0096 | 0.42 | 0.017 | 0.73 | 0.14 | 0.70 |
| 95% CI of | 0.94 | nd | 0.83 | 1.4 | 0.36 | 1.3 | 0.33 | 0.59 | 0.29 |
| OR Quart4 | 13 | nd | 12 | 14 | 11 | 17 | 4.8 | 44 | 6.2 |
| | | | | | | | | | |
| **Myoglobin** | | | | | | | | | |
| sCr or UO | 0hr prior to AKI stage | | 24hr prior to AKI stage | | 48hr prior to AKI stage | | | | |
| | Cohort 1 | Cohort 2 | Cohort 1 | Cohort 2 | Cohort 1 | Cohort 2 | | | |
| Median | 0.860 | 0.477 | 0.860 | 0.126 | 0.860 | 0.176 | | | |
| Average | 35.5 | 18.1 | 35.5 | 44.2 | 35.5 | 6.85 | | | |
| Stdev | 103 | 38.1 | 103 | 130 | 103 | 15.5 | | | |
| p(t-test) | | 0.39 | | 0.64 | | 0.25 | | | |
| Min | 0.000105 | 0.0151 | 0.000105 | 0.000105 | 0.000105 | 0.0254 | | | |
| Max | 618 | 163 | 618 | 469 | 618 | 49.1 | | | |
| n (Samp) | 419 | 26 | 419 | 34 | 419 | 17 | | | |
| n (Patient) | 164 | 26 | 164 | 34 | 164 | 17 | | | |

(continued)

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 0.599 | 4.97 | 0.599 | 0.349 |
| Average | nd | nd | 37.0 | 95.0 | 37.0 | 19.7 |
| Stdev | nd | nd | 109 | 171 | 109 | 28.5 |
| p(t-test) | nd | nd | | 0.14 | | 0.68 |
| Min | nd | nd | 0.000105 | 0.0561 | 0.000105 | 0.0683 |
| Max | nd | nd | 618 | 420 | 618 | 71.6 |
| n (Samp) | nd | nd | 511 | 8 | 511 | 7 |
| n (Patient) | nd | nd | 198 | 8 | 198 | 7 |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 0.885 | 0.354 | 0.885 | 0.108 | 0.885 | 0.176 |
| Average | 40.5 | 17.6 | 40.5 | 36.1 | 40.5 | 5.29 |
| Stdev | 112 | 38.2 | 112 | 119 | 112 | 12.1 |
| p(t-test) | | 0.30 | | 0.84 | | 0.20 |
| Min | 0.000105 | 0.0151 | 0.000105 | 0.000105 | 0.000105 | 0.0254 |
| Max | 618 | 163 | 618 | 469 | 618 | 49.1 |
| n (Samp) | 355 | 26 | 355 | 30 | 355 | 17 |
| n (Patient) | 134 | 26 | 134 | 30 | 134 | 17 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.48 | nd | 0.45 | 0.39 | 0.57 | 0.38 | 0.40 | 0.55 | 0.39 |
| SE | 0.059 | nd | 0.060 | 0.053 | 0.11 | 0.057 | 0.074 | 0.11 | 0.074 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p | 0.72 | nd | 0.36 | 0.045 | 0.53 | 0.031 | 0.16 | 0.67 | 0.15 |
| nCohort 1 | 419 | nd | 355 | 419 | 511 | 355 | 419 | 511 | 355 |
| nCohort 2 | 26 | nd | 26 | 34 | 8 | 30 | 17 | 7 | 17 |
| Cutoff 1 | 0.0772 | nd | 0.0667 | 0.0584 | 0.118 | 0.0562 | 0.0849 | 0.132 | 0.0834 |
| Sens 1 | 73% | nd | 73% | 71% | 75% | 70% | 71% | 71% | 71% |
| Spec 1 | 22% | nd | 20% | 19% | 32% | 18% | 23% | 32% | 22% |
| Cutoff 2 | 0.0511 | nd | 0.0422 | 0.0375 | 0.0589 | 0.0366 | 0.0667 | 0.0849 | 0.0630 |
| Sens 2 | 81% | nd | 81% | 82% | 88% | 80% | 82% | 86% | 82% |
| Spec 2 | 18% | nd | 13% | 12% | 22% | 11% | 21% | 26% | 19% |
| Cutoff 3 | 0.0375 | nd | 0.0366 | 0.0331 | 0.0556 | 0.0331 | 0.0337 | 0.0667 | 0.0337 |
| Sens 3 | 92% | nd | 92% | 91% | 100% | 90% | 94% | 100% | 94% |
| Spec 3 | 12% | nd | 11% | 10% | 21% | 9% | 11% | 23% | 10% |
| Cutoff 4 | 7.09 | nd | 7.24 | 7.09 | 6.05 | 7.24 | 7.09 | 6.05 | 7.24 |
| Sens 4 | 31% | nd | 27% | 21% | 50% | 20% | 18% | 43% | 24% |
| Spec 4 | 70% | nd | 70% | 70% | 70% | 70% | 70% | 70% | 70% |
| Cutoff 5 | 19.0 | nd | 23.8 | 19.0 | 17.9 | 23.8 | 19.0 | 17.9 | 23.8 |
| Sens 5 | 19% | nd | 19% | 15% | 25% | 13% | 12% | 43% | 6% |
| Spec 5 | 80% | nd | 80% | 80% | 80% | 80% | 80% | 80% | 80% |
| Cutoff 6 | 66.4 | nd | 79.4 | 66.4 | 70.5 | 79.4 | 66.4 | 70.5 | 79.4 |
| Sens 6 | 12% | nd | 8% | 12% | 25% | 7% | 0% | 14% | 0% |
| Spec 6 | 90% | nd | 90% | 90% | 90% | 90% | 90% | 90% | 90% |
| OR Quart 2 | 0.83 | nd | 0.66 | 0.83 | 0.99 | 0.66 | 0.66 | 3.0 | 2.0 |
| p Value | 0.77 | nd | 0.53 | 0.77 | 0.99 | 0.53 | 0.65 | 0.34 | 0.42 |
| 95% CI of | 0.25 | nd | 0.18 | 0.25 | 0.14 | 0.18 | 0.11 | 0.31 | 0.37 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart2 | 2.8 | nd | 2.4 | 2.8 | 7.2 | 2.4 | 4.0 | 29 | 11 |
| OR Quart 3 | 1.0 | nd | 1.0 | 1.2 | 0.49 | 0.83 | 2.1 | 0 | 2.0 |
| p Value | 0.99 | nd | 0.99 | 0.76 | 0.56 | 0.77 | 0.32 | na | 0.42 |
| 95% CI of | 0.32 | nd | 0.31 | 0.39 | 0.044 | 0.25 | 0.50 | na | 0.37 |
| OR Quart3 | 3.2 | nd | 3.3 | 3.7 | 5.5 | 2.8 | 8.4 | na | 11 |
| OR Quar 4 | 1.6 | nd | 1.8 | 3.0 | 1.5 | 2.8 | 2.1 | 3.0 | 3.7 |
| p Value | 0.42 | nd | 0.29 | 0.029 | 0.66 | 0.042 | 0.32 | 0.34 | 0.11 |
| 95% CI of | 0.54 | nd | 0.61 | 1.1 | 0.25 | 1.0 | 0.50 | 0.31 | 0.75 |
| OR Quart4 | 4.5 | nd | 5.1 | 7.9 | 9.1 | 7.6 | 8.4 | 29 | 18 |
| | | | | | | | | | |

**Mucin-16**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 0.684 | 0.822 | nd | nd |
| Average | nd | nd | 1.24 | 28.1 | nd | nd |
| Stdev | nd | nd | 1.59 | 108 | nd | nd |
| p(t-test) | nd | nd | | 0.014 | nd | nd |
| Min | nd | nd | 1.00E-9 | 0.304 | nd | nd |
| Max | nd | nd | 10.1 | 433 | nd | nd |
| n (Samp) | nd | nd | 96 | 16 | nd | nd |
| n (Patient) | nd | nd | 73 | 16 | nd | nd |

116

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 0.623 | 0.822 | nd | nd |
| Average | nd | nd | 1.23 | 29.9 | nd | nd |
| Stdev | nd | nd | 1.64 | 111 | nd | nd |
| p(t-test) | nd | nd | | 0.020 | nd | nd |
| Min | nd | nd | 1.00E-9 | 0.304 | nd | nd |
| Max | nd | nd | 10.1 | 433 | nd | nd |
| n (Samp) | nd | nd | 81 | 15 | nd | nd |
| n (Patient) | nd | nd | 61 | 15 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.55 | nd | 0.56 | nd | nd | nd |
| SE | nd | nd | nd | 0.080 | nd | 0.083 | nd | nd | nd |
| p | nd | nd | nd | 0.49 | nd | 0.45 | nd | nd | nd |
| nCohort 1 | nd | nd | nd | 96 | nd | 81 | nd | nd | nd |
| nCohort 2 | nd | nd | nd | 16 | nd | 15 | nd | nd | nd |
| Cutoff 1 | nd | nd | nd | 0.412 | nd | 0.412 | nd | nd | nd |
| Sens 1 | nd | nd | nd | 75% | nd | 73% | nd | nd | nd |
| Spec 1 | nd | nd | nd | 32% | nd | 36% | nd | nd | nd |
| Cutoff 2 | nd | nd | nd | 0.344 | nd | 0.344 | nd | nd | nd |
| Sens 2 | nd | nd | nd | 88% | nd | 87% | nd | nd | nd |
| Spec 2 | nd | nd | nd | 21% | nd | 26% | nd | nd | nd |
| Cutoff 3 | nd | nd | nd | 0.278 | nd | 0.278 | nd | nd | nd |
| Sens 3 | nd | nd | nd | 100% | nd | 100% | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 3 | nd | nd | nd | 11% | nd | 15% | nd | nd | nd |
| Cutoff 4 | nd | nd | nd | 1.17 | nd | 1.09 | nd | nd | nd |
| Sens 4 | nd | nd | nd | 25% | nd | 33% | nd | nd | nd |
| Spec 4 | nd | nd | nd | 71% | nd | 70% | nd | nd | nd |
| Cutoff 5 | nd | nd | nd | 1.41 | nd | 1.44 | nd | nd | nd |
| Sens 5 | nd | nd | nd | 19% | nd | 20% | nd | nd | nd |
| Spec 5 | nd | nd | nd | 80% | nd | 80% | nd | nd | nd |
| Cutoff 6 | nd | nd | nd | 3.40 | nd | 3.26 | nd | nd | nd |
| Sens 6 | nd | nd | nd | 12% | nd | 13% | nd | nd | nd |
| Spec 6 | nd | nd | nd | 91% | nd | 90% | nd | nd | nd |
| OR Quart 2 | nd | nd | nd | 0.72 | nd | 1.0 | nd | nd | nd |
| p Value | nd | nd | nd | 0.69 | nd | 1.0 | nd | nd | nd |
| 95% CI of | nd | nd | nd | 0.15 | nd | 0.18 | nd | nd | nd |
| OR Quart2 | nd | nd | nd | 3.6 | nd | 5.5 | nd | nd | nd |
| OR Quart 3 | nd | nd | nd | 1.3 | nd | 1.8 | nd | nd | nd |
| p Value | nd | nd | nd | 0.72 | nd | 0.44 | nd | nd | nd |
| 95% CI of | nd | nd | nd | 0.31 | nd | 0.39 | nd | nd | nd |
| OR Quart3 | nd | nd | nd | 5.5 | nd | 8.8 | nd | nd | nd |
| OR Quart 4 | nd | nd | nd | 1.0 | nd | 1.4 | nd | nd | nd |
| p Value | nd | nd | nd | 1.0 | nd | 0.68 | nd | nd | nd |
| 95% CI of | nd | nd | nd | 0.22 | nd | 0.28 | nd | nd | nd |
| OR Quart4 | nd | nd | nd | 4.5 | nd | 7.1 | nd | nd | nd |

(continued)

| Tumor necrosis factor receptor superfamily member 10B | | | | | | |
|---|---|---|---|---|---|---|
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | nd | nd | 0.344 | 0.306 | nd | nd |
| Average | nd | nd | 0.956 | 1.45 | nd | nd |
| Stdev | nd | nd | 1.45 | 1.97 | nd | nd |
| p(t-test) | nd | nd | | 0.24 | nd | nd |
| Min | nd | nd | 0.00360 | 0.0172 | nd | nd |
| Max | nd | nd | 6.71 | 5.86 | nd | nd |
| n (Samp) | nd | nd | 96 | 16 | nd | nd |
| n (Patient) | nd | nd | 73 | 16 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 0.343 | 0.287 | nd | nd |
| Average | nd | nd | 0.805 | 1.42 | nd | nd |
| Stdev | nd | nd | 1.26 | 2.00 | nd | nd |
| p(t-test) | nd | nd | | 0.12 | nd | nd |
| Min | nd | nd | 0.00360 | 0.0172 | nd | nd |
| Max | nd | nd | 6.48 | 5.86 | nd | nd |
| n (Samp) | nd | nd | 81 | 15 | nd | nd |
| n (Patient) | nd | nd | 61 | 15 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.56 | nd | 0.55 | nd | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| SE | nd | nd | nd | 0.080 | nd | 0.083 | nd | nd | nd |
| p | nd | nd | nd | 0.46 | nd | 0.53 | nd | nd | nd |
| nCohort 1 | nd | nd | nd | 96 | nd | 81 | nd | nd | nd |
| nCohort 2 | nd | nd | nd | 16 | nd | 15 | nd | nd | nd |
| Cutoff 1 | nd | nd | nd | 0.109 | nd | 0.109 | nd | nd | nd |
| Sens 1 | nd | nd | nd | 75% | nd | 73% | nd | nd | nd |
| Spec 1 | nd | nd | nd | 35% | nd | 36% | nd | nd | nd |
| Cutoff 2 | nd | nd | nd | 0.101 | nd | 0.101 | nd | nd | nd |
| Sens 2 | nd | nd | nd | 81% | nd | 80% | nd | nd | nd |
| Spec 2 | nd | nd | nd | 31% | nd | 32% | nd | nd | nd |
| Cutoff 3 | nd | nd | nd | 0.0198 | nd | 0.0182 | nd | nd | nd |
| Sens 3 | nd | nd | nd | 94% | nd | 93% | nd | nd | nd |
| Spec 3 | nd | nd | nd | 11% | nd | 11% | nd | nd | nd |
| Cutoff 4 | nd | nd | nd | 0.855 | nd | 0.819 | nd | nd | nd |
| Sens 4 | nd | nd | nd | 38% | nd | 33% | nd | nd | nd |
| Spec 4 | nd | nd | nd | 71% | nd | 70% | nd | nd | nd |
| Cutoff 5 | nd | nd | nd | 1.56 | nd | 0.979 | nd | nd | nd |
| Sens 5 | nd | nd | nd | 31% | nd | 33% | nd | nd | nd |
| Spec 5 | nd | nd | nd | 80% | nd | 80% | nd | nd | nd |
| Cutoff 6 | nd | nd | nd | 3.11 | nd | 2.25 | nd | nd | nd |
| Sens 6 | nd | nd | nd | 19% | nd | 27% | nd | nd | nd |
| Spec 6 | nd | nd | nd | 91% | nd | 90% | nd | nd | nd |
| OR Quart 2 | nd | nd | nd | 4.3 | nd | 3.7 | nd | nd | nd |
| p Value | nd | nd | nd | 0.086 | nd | 0.14 | nd | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| 95% CI of | nd | nd | nd | 0.81 | nd | 0.66 | nd | nd | nd |
| OR Quart2 | nd | nd | nd | 23 | nd | 20 | nd | nd | nd |
| OR Quart 3 | nd | nd | nd | 0.48 | nd | 1.0 | nd | nd | nd |
| p Value | nd | nd | nd | 0.56 | nd | 1.0 | nd | nd | nd |
| 95% CI of | nd | nd | nd | 0.041 | nd | 0.13 | nd | nd | nd |
| OR Quart3 | nd | nd | nd | 5.6 | nd | 7.7 | nd | nd | nd |
| OR Quart 4 | nd | nd | nd | 3.5 | nd | 2.9 | nd | nd | nd |
| p Value | nd | nd | nd | 0.14 | nd | 0.23 | nd | nd | nd |
| 95% CI of | nd | nd | nd | 0.65 | nd | 0.50 | nd | nd | nd |
| OR Quart4 | nd | nd | nd | 19 | nd | 17 | nd | nd | nd |

**Cellular tumor antigen p53**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 1.00E-9 | 0.000165 | nd | nd |
| Average | nd | nd | 0.00163 | 0.00180 | nd | nd |
| Stdev | nd | nd | 0.00319 | 0.00245 | nd | nd |
| p(t-test) | nd | nd | | 0.84 | nd | nd |
| Min | nd | nd | 1.00E-9 | 1.00E-9 | nd | nd |
| Max | nd | nd | 0.0202 | 0.00839 | nd | nd |
| n (Samp) | nd | nd | 96 | 16 | nd | nd |
| n (Patient) | nd | nd | 73 | 16 | nd | nd |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 1.00E-9 | 3.53E-5 | nd | nd |
| Average | nd | nd | 0.00186 | 0.00145 | nd | nd |
| Stdev | nd | nd | 0.00355 | 0.00190 | nd | nd |
| p(t-test) | nd | nd | | 0.66 | nd | nd |
| Min | nd | nd | 1.00E-9 | 1.00E-9 | nd | nd |
| Max | nd | nd | 0.0202 | 0.00470 | nd | nd |
| n (Samp) | nd | nd | 81 | 15 | nd | nd |
| n (Patient) | nd | nd | 61 | 15 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.58 | nd | 0.55 | nd | nd | nd |
| SE | nd | nd | nd | 0.080 | nd | 0.083 | nd | nd | nd |
| p | nd | nd | nd | 0.30 | nd | 0.56 | nd | nd | nd |
| nCohort 1 | nd | nd | nd | 96 | nd | 81 | nd | nd | nd |
| nCohort 2 | nd | nd | nd | 16 | nd | 15 | nd | nd | nd |
| Cutoff 1 | nd | nd | nd | 0 | nd | 0 | nd | nd | nd |
| Sens 1 | nd | nd | nd | 100% | nd | 100% | nd | nd | nd |
| Spec 1 | nd | nd | nd | 0% | nd | 0% | nd | nd | nd |
| Cutoff 2 | nd | nd | nd | 0 | nd | 0 | nd | nd | nd |
| Sens 2 | nd | nd | nd | 100% | nd | 100% | nd | nd | nd |
| Spec 2 | nd | nd | nd | 0% | nd | 0% | nd | nd | nd |
| Cutoff 3 | nd | nd | nd | 0 | nd | 0 | nd | nd | nd |
| Sens 3 | nd | nd | nd | 100% | nd | 100% | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 3 | nd | nd | nd | 0% | nd | 0% | nd | nd | nd |
| Cutoff 4 | nd | nd | nd | 0.00160 | nd | 0.00160 | nd | nd | nd |
| Sens 4 | nd | nd | nd | 38% | nd | 33% | nd | nd | nd |
| Spec 4 | nd | nd | nd | 73% | nd | 72% | nd | nd | nd |
| Cutoff 5 | nd | nd | nd | 0.00313 | nd | 0.00343 | nd | nd | nd |
| Sens 5 | nd | nd | nd | 38% | nd | 20% | nd | nd | nd |
| Spec 5 | nd | nd | nd | 81% | nd | 80% | nd | nd | nd |
| Cutoff 6 | nd | nd | nd | 0.00470 | nd | 0.00599 | nd | nd | nd |
| Sens 6 | nd | nd | nd | 6% | nd | 0% | nd | nd | nd |
| Spec 6 | nd | nd | nd | 92% | nd | 91% | nd | nd | nd |
| OR Quart 2 | nd | nd | nd | 0.46 | nd | 0.17 | nd | nd | nd |
| p Value | nd | nd | nd | 0.40 | nd | 0.11 | nd | nd | nd |
| 95% CI of | nd | nd | nd | 0.077 | nd | 0.018 | nd | nd | nd |
| OR Quart2 | nd | nd | nd | 2.8 | nd | 1.5 | nd | nd | nd |
| OR Quart 3 | nd | nd | nd | 1.0 | nd | 0.76 | nd | nd | nd |
| p Value | nd | nd | nd | 1.0 | nd | 0.71 | nd | nd | nd |
| 95% CI of | nd | nd | nd | 0.22 | nd | 0.18 | nd | nd | nd |
| OR Quart3 | nd | nd | nd | 4.5 | nd | 3.3 | nd | nd | nd |
| OR Quart 4 | nd | nd | nd | 1.6 | nd | 1.0 | nd | nd | nd |
| p Value | nd | nd | nd | 0.49 | nd | 1.0 | nd | nd | nd |
| 95% CI of | nd | nd | nd | 0.41 | nd | 0.25 | nd | nd | nd |
| OR Quart4 | nd | nd | nd | 6.6 | nd | 4.0 | nd | nd | nd |

Table 3: Comparison of marker levels in urine samples collected within 12 hours of reaching stage R from Cohort 1 (patients that reached, but did not progress beyond, RIFLE stage R) and from Cohort 2 (patients that reached RIFLE stage I or F).

| Apolipoprotein A-II | | | | | | |
|---|---|---|---|---|---|---|
| | sCr or UO | | sCr only | | UO only | |
| | Cohort 1 | Cohort 2 | Cohort 1 | Cohort 2 | Cohort 1 | Cohort 2 |
| Median | 78.2 | 125 | 77.5 | 247 | 85.8 | 106 |
| Average | 156 | 187 | 167 | 307 | 149 | 132 |
| Stdev | 303 | 193 | 368 | 295 | 238 | 77.5 |
| p(t-test) | | 0.58 | | 0.30 | | 0.74 |
| Min | 4.67 | 18.9 | 4.67 | 23.0 | 21.4 | 18.9 |
| Max | 2000 | 845 | 2000 | 845 | 1790 | 323 |
| n (Samp) | 77 | 33 | 29 | 9 | 61 | 24 |
| n (Patient) | 77 | 33 | 29 | 9 | 61 | 24 |
| | | | | | | |
| | At Enrollment | | | | | |
| | sCr or UO | | sCr only | | UO only | |
| AUC | 0.64 | | 0.76 | | 0.60 | |
| SE | 0.059 | | 0.10 | | 0.070 | |
| D | 0.016 | | 0.011 | | 0.16 | |
| nCohort 1 | 77 | | 29 | | 61 | |
| nCohort 2 | 33 | | 9 | | 24 | |
| | At Enrollment | | | | | |
| | sCr or UO | | sCr only | | UO only | |
| Cutoff 1 | 82.3 | | 125 | | 85.8 | |
| Sens 1 | 73% | | 78% | | 71% | |
| Spec 1 | 53% | | 72% | | 51% | |
| Cutoff 2 | 67.3 | | 78.5 | | 65.3 | |
| Sens 2 | 82% | | 89% | | 83% | |
| Spec 2 | 47% | | 55% | | 43% | |
| Cutoff 3 | 58.1 | | 21.6 | | 62.3 | |
| Sens 3 | 91% | | 100% | | 92% | |
| Spec 3 | 42% | | 10% | | 41% | |
| Cutoff 4 | 126 | | 125 | | 148 | |
| Sens 4 | 48% | | 78% | | 33% | |
| Spec 4 | 70% | | 72% | | 70% | |
| Cutoff 5 | 166 | | 166 | | 180 | |
| Sens 5 | 33% | | 56% | | 21% | |
| Spec 5 | 81% | | 83% | | 80% | |
| Cutoff 6 | 314 | | 195 | | 314 | |

(continued)

| | At Enrollment | | |
|---|---|---|---|
| | sCr or UO | sCr only | UO only |
| Sens 6 | 12% | 56% | 4% |
| Spec 6 | 91% | 93% | 90% |
| OR Quart 2 | 3.2 | 0.89 | 12 |
| p Value | 0.12 | 0.94 | 0.025 |
| 95% CI of | 0.75 | 0.047 | 1.4 |
| OR Quart2 | 14 | 17 | 110 |
| OR Quart 3 | 5.5 | 2.3 | 15 |
| p Value | 0.019 | 0.53 | 0.015 |
| 95% CI of | 1.3 | 0.17 | 1.7 |
| OR Quart3 | 23 | 31 | 130 |
| OR Quart 4 | 5.2 | 8.0 | 7.5 |
| p Value | 0.023 | 0.092 | 0.075 |
| 95% CI of | 1.3 | 0.71 | 0.82 |
| OR Quart4 | 21 | 90 | 69 |

**Myoglobin**

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 0.515 | 0.0847 | nd | nd | 0.758 | 0.0561 |
| Average | 42.9 | 26.9 | nd | nd | 49.3 | 3.71 |
| Stdev | 122 | 94.2 | nd | nd | 131 | 12.0 |
| p(t-test) | | 0.57 | nd | nd | | 0.16 |
| Min | 0.000105 | 0.000105 | nd | nd | 0.0176 | 0.0145 |
| Max | 618 | 460 | nd | nd | 618 | 49.1 |
| n (Samp) | 52 | 24 | nd | nd | 44 | 17 |
| n (Patient) | 52 | 24 | nd | nd | 44 | 17 |

| | At Enrollment | | |
|---|---|---|---|
| | sCr or UO | sCr only | UO only |
| AUC | 0.40 | nd | 0.30 |
| SE | 0.072 | nd | 0.079 |
| p | 0.17 | nd | 0.011 |
| nCohort 1 | 52 | nd | 44 |
| nCohort 2 | 24 | nd | 17 |
| Cutoff 1 | 0.0365 | nd | 0.0347 |
| Sens 1 | 71% | nd | 71% |
| Spec 1 | 15% | nd | 14% |

(continued)

|  | At Enrollment | | |
|---|---|---|---|
|  | sCr or UO | sCr only | UO only |
| Cutoff 2 | 0.0324 | nd | 0.0324 |
| Sens 2 | 83% | nd | 82% |
| Spec 2 | 12% | nd | 11% |
| Cutoff 3 | 0.0176 | nd | 0.0176 |
| Sens 3 | 92% | nd | 94% |
| Spec 3 | 4% | nd | 2% |
| Cutoff 4 | 13.5 | nd | 13.9 |
| Sens 4 | 21% | nd | 6% |
| Spec 4 | 71% | nd | 70% |
| Cutoff 5 | 33.1 | nd | 34.7 |
| Sens 5 | 12% | nd | 6% |
| Spec 5 | 81% | nd | 82% |
| Cutoff 6 | 63.7 | nd | 85.7 |
| Sens 6 | 8% | nd | 0% |
| Spec 6 | 90% | nd | 91% |
| OR Quart 2 | 0.75 | nd | 1.8 |
| p Value | 0.70 | nd | 0.57 |
| 95% CI of | 0.17 | nd | 0.25 |
| OR Quart2 | 3.4 | nd | 12 |
| OR Quart 3 | 1.6 | nd | 3.5 |
| p Value | 0.49 | nd | 0.18 |
| 95% CI of | 0.41 | nd | 0.56 |
| OR Quart3 | 6.5 | nd | 22 |
| OR Quart 4 | 2.0 | nd | 6.1 |
| p Value | 0.31 | nd | 0.048 |
| 95% CI of | 0.52 | nd | 1.0 |
| OR Quart4 | 8.0 | nd | 37 |

**KSP-Cadherin**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 1.48 | 0.809 | nd | nd | 1.46 | 0.996 |
| Average | 1.74 | 1.25 | nd | nd | 1.73 | 1.18 |
| Stdev | 1.18 | 1.36 | nd | nd | 1.10 | 1.26 |
| p(t-test) |  | 0.18 | nd | nd |  | 0.21 |
| Min | 0.131 | 0.00263 | nd | nd | 0.196 | 0.00263 |
| Max | 4.63 | 4.23 | nd | nd | 4.63 | 3.88 |

(continued)

| KSP-Cadherin | | | | | | |
|---|---|---|---|---|---|---|
| | sCr or UO | | sCr only | | UO only | |
| | Cohort 1 | Cohort 2 | Cohort 1 | Cohort 2 | Cohort 1 | Cohort 2 |
| n (Samp) | 40 | 16 | nd | nd | 31 | 9 |
| n (Patient) | 40 | 16 | nd | nd | 31 | 9 |
| | | | | | | |
| | At Enrollment | | | | | |
| | sCr or UO | | sCr only | | UO only | |
| AUC | 0.33 | | nd | | 0.31 | |
| SE | 0.084 | | nd | | 0.11 | |
| p | 0.037 | | nd | | 0.081 | |
| nCohort 1 | 40 | | nd | | 31 | |
| nCohort 2 | 16 | | nd | | 9 | |
| Cutoff 1 | 0.258 | | nd | | 0.196 | |
| Sens 1 | 75% | | nd | | 78% | |
| Spec 1 | 5% | | nd | | 3% | |
| Cutoff 2 | 0.196 | | nd | | 0.00263 | |
| Sens 2 | 81% | | nd | | 89% | |
| Spec 2 | 5% | | nd | | 0% | |
| Cutoff 3 | 0.00263 | | nd | | 0 | |
| Sens 3 | 94% | | nd | | 100% | |
| Spec 3 | 0% | | nd | | 0% | |
| Cutoff 4 | 1.84 | | nd | | 1.84 | |
| Sens 4 | 25% | | nd | | 22% | |
| Spec 4 | 70% | | nd | | 71% | |
| Cutoff 5 | 2.57 | | nd | | 2.54 | |
| Sens 5 | 19% | | nd | | 11% | |
| Spec 5 | 80% | | nd | | 81% | |
| Cutoff 6 | 3.39 | | nd | | 3.11 | |
| Sens 6 | 12% | | nd | | 11% | |
| Spec 6 | 90% | | nd | | 90% | |
| OR Quart 2 | 0 | | nd | | 0 | |
| p Value | na | | nd | | na | |
| 95% CI of | na | | nd | | na | |
| OR Quart2 | na | | nd | | na | |
| OR Quart 3 | 1.0 | | nd | | 1.7 | |
| p Value | 1.0 | | nd | | 0.61 | |
| 95% CI of | 0.19 | | nd | | 0.22 | |
| OR Quart3 | 5.2 | | nd | | 13 | |

(continued)

|  | At Enrollment | | |
|---|---|---|---|
|  | sCr or UO | sCr only | UO only |
| OR Quart 4 | 3.3 | nd | 2.7 |
| p Value | 0.13 | nd | 0.34 |
| 95% CI of | 0.69 | nd | 0.36 |
| OR Quart4 | 16 | nd | 20 |

Table 4: Comparison of the maximum marker levels in urine samples collected from Cohort 1 (patients that did not progress beyond RIFLE stage 0) and the maximum values in urine samples collected from subjects between enrollment and 0, 24 hours, and 48 hours prior to reaching stage F in Cohort 2.

| Apolipoprotein A-II | | | | | | |
|---|---|---|---|---|---|---|
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 76.0 | 265 | 76.0 | 217 | 76.0 | 157 |
| Average | 241 | 1760 | 241 | 1460 | 241 | 225 |
| Stdev | 841 | 5360 | 841 | 5260 | 841 | 193 |
| p(t-test) | | 3.5E-4 | | 0.0035 | | 0.95 |
| Min | 5.33 | 27.6 | 5.33 | 9.51 | 5.33 | 55.6 |
| Max | 6400 | 24300 | 6400 | 24300 | 6400 | 764 |
| n (Samp) | 196 | 21 | 196 | 21 | 196 | 11 |
| n (Patient) | 196 | 21 | 196 | 21 | 196 | 11 |
| | | | | | | |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 97.8 | 268 | 97.8 | 265 | 97.8 | 216 |
| Average | 342 | 954 | 342 | 393 | 342 | 213 |
| Stdev | 1610 | 1880 | 1610 | 510 | 1610 | 68.6 |
| p(t-test) | | 0.22 | | 0.92 | | 0.84 |
| Min | 5.33 | 27.6 | 5.33 | 9.51 | 5.33 | 131 |
| Max | 24300 | 6400 | 24300 | 1860 | 24300 | 288 |
| n (Samp) | 294 | 11 | 294 | 11 | 294 | 6 |
| n (Patient) | 294 | 11 | 294 | 11 | 294 | 6 |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 87.2 | 174 | 87.2 | 174 | 87.2 | 149 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Average | 294 | 2240 | 294 | 1850 | 294 | 227 |
| Stdev | 972 | 6320 | 972 | 6230 | 972 | 214 |
| p(t-test) | | 0.0012 | | 0.0086 | | 0.84 |
| Min | 5.33 | 55.6 | 5.33 | 45.8 | 5.33 | 55.6 |
| Max | 6400 | 24300 | 6400 | 24300 | 6400 | 764 |
| n (Samp) | 132 | 15 | 132 | 15 | 132 | 9 |
| n (Patient) | 132 | 15 | 132 | 15 | 132 | 9 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.80 | 0.78 | 0.79 | 0.77 | 0.76 | 0.76 | 0.78 | 0.78 | 0.73 |
| SE | 0.059 | 0.084 | 0.072 | 0.062 | 0.086 | 0.075 | 0.084 | 0.11 | 0.098 |
| p | 2.8E-7 | 9.4E-4 | 4.7E-5 | 8.7E-6 | 0.0029 | 5.7E-4 | 0.0010 | 0.014 | 0.019 |
| nCohort 1 | 196 | 294 | 132 | 196 | 294 | 132 | 196 | 294 | 132 |
| nCohort 2 | 21 | 11 | 15 | 21 | 11 | 15 | 11 | 6 | 9 |
| Cutoff 1 | 148 | 165 | 146 | 148 | 165 | 146 | 146 | 157 | 111 |
| Sens 1 | 71% | 73% | 73% | 71% | 73% | 73% | 73% | 83% | 78% |
| Spec 1 | 80% | 74% | 77% | 80% | 74% | 77% | 79% | 71% | 63% |
| Cutoff 2 | 129 | 157 | 129 | 129 | 157 | 129 | 111 | 157 | 100 |
| Sens 2 | 81% | 82% | 80% | 81% | 82% | 80% | 82% | 83% | 89% |
| Spec 2 | 74% | 71% | 73% | 74% | 71% | 73% | 67% | 71% | 58% |
| Cutoff 3 | 100 | 135 | 100 | 54.2 | 129 | 54.3 | 100 | 129 | 54.3 |
| Sens 3 | 90% | 91% | 93% | 90% | 91% | 93% | 91% | 100% | 100% |
| Spec 3 | 62% | 65% | 58% | 37% | 64% | 32% | 62% | 64% | 32% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 4 | 124 | 155 | 127 | 124 | 155 | 127 | 124 | 155 | 127 |
| Sens 4 | 81% | 82% | 80% | 81% | 82% | 80% | 73% | 83% | 67% |
| Spec 4 | 70% | 70% | 70% | 70% | 70% | 70% | 70% | 70% | 70% |
| Cutoff 5 | 153 | 200 | 164 | 153 | 200 | 164 | 153 | 200 | 164 |
| Sens 5 | 67% | 64% | 53% | 67% | 64% | 53% | 55% | 50% | 33% |
| Spec 5 | 80% | 80% | 80% | 80% | 80% | 80% | 80% | 80% | 80% |
| Cutoff 6 | 296 | 335 | 312 | 296 | 335 | 312 | 296 | 335 | 312 |
| Sens 6 | 29% | 27% | 20% | 24% | 18% | 20% | 9% | 0% | 11% |
| Spec 6 | 90% | 90% | 90% | 90% | 90% | 90% | 90% | 90% | 90% |
| OR Quart 2 | 1.0 | 0 | >1.0 | 2.0 | 0 | 0.97 | >1.0 | >0 | >1.0 |
| p Value | 1.0 | na | <1.0 | 0.57 | na | 0.98 | <1.0 | <na | <0.98 |
| 95% CI of | 0.061 | na | >0.060 | 0.18 | na | 0.058 | >0.061 | >na | >0.062 |
| OR Quart2 | 16 | na | na | 23 | na | 16 | na | na | na |
| OR Quart 3 | 4.2 | 3.1 | >5.6 | 3.1 | 3.1 | 4.2 | >2.0 | >3.1 | >2.1 |
| p Value | 0.20 | 0.33 | <0.12 | 0.33 | 0.33 | 0.21 | <0.57 | <0.33 | <0.55 |
| 95% CI of | 0.46 | 0.31 | >0.62 | 0.31 | 0.31 | 0.45 | >0.18 | >0.32 | >0.18 |
| OR Quart3 | 39 | 30 | na | 31 | 30 | 40 | na | na | na |
| OR Quart 4 | .20 | 7.5 | >12 | 20 | 7.5 | 11 | >9.3 | >3.1 | >7.0 |
| p Value | 0.0046 | 0.063 | <0.024 | 0.0046 | 0.063 | 0.026 | <0.039 | <0.33 | <0.079 |
| 95% CI of | 2.5 | 0.90 | >1.4 | 2.5 | 0.90 | 1.3 | >1.1 | >0.32 | >0.80 |
| OR Quart4 | 160 | 63 | na | 160 | 63 | 94 | na | na | na |

EP 2 894 473 B1

(continued)

**Caspase-1**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.28 | 4.81 | 1.28 | 2.33 | 1.28 | 0.988 |
| Average | 1.56 | 10.5 | 1.56 | 4.46 | 1.56 | 1.77 |
| Stdev | 1.38 | 15.7 | 1.38 | 6.02 | 1.38 | 2.36 |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| p(t-test) | | 0.0057 | | 0.027 | | 0.78 |
| Min | 0.0223 | 0.0223 | 0.0223 | 0.0223 | 0.0223 | 0.0223 |
| Max | 4.68 | 47.1 | 4.68 | 17.3 | 4.68 | 6.25 |
| n (Samp) | 26 | 8 | 26 | 7 | 26 | 6 |
| n (Patient) | 26 | 8 | 26 | 7 | 26 | 6 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.75 | nd | nd | 0.64 | nd | nd | 0.45 | nd | nd |
| SE | 0.11 | nd | nd | 0.12 | nd | nd | 0.13 | nd | nd |
| D | 0.021 | nd | nd | 0.25 | nd | nd | 0.72 | nd | nd |
| nCohort 1 | 26 | nd | nd | 26 | nd | nd | 26 | nd | nd |
| nCohort 2 | 8 | nd | nd | 7 | nd | nd | 6 | nd | nd |
| Cutoff 1 | 2.08 | nd | nd | 1.02 | nd | nd | 0 | nd | nd |
| Sens 1 | 75% | nd | nd | 71% | nd | nd | 100% | nd | nd |
| Spec 1 | 69% | nd | nd | 46% | nd | nd | 0% | nd | nd |
| Cutoff 2 | 0.496 | nd | nd | 0.496 | nd | nd | 0 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 2 | 88% | nd | nd | 86% | nd | nd | 100% | nd | nd |
| Spec 2 | 31% | nd | nd | 31% | nd | nd | 0% | nd | nd |
| Cutoff 3 | 0 | nd | nd | 0 | nd | nd | 0 | nd | nd |
| Sens 3 | 100% | nd | nd | 100% | nd | nd | 100% | nd | nd |
| Spec 3 | 0% | nd | nd | 0% | nd | nd | 0% | nd | nd |
| Cutoff 4 | 2.33 | nd | nd | 2.33 | nd | nd | 2.33 | nd | nd |
| Sens 4 | 62% | nd | nd | 43% | nd | nd | 17% | nd | nd |
| Spec 4 | 73% | nd | nd | 73% | nd | nd | 73% | nd | nd |
| Cutoff 5 | 2.59 | nd | nd | 2.59 | nd | nd | 2.59 | nd | nd |
| Sens 5 | 62% | nd | nd | 43% | nd | nd | 17% | nd | nd |
| Spec 5 | 85% | nd | nd | 85% | nd | nd | 85% | nd | nd |
| Cutoff 6 | 3.90 | nd | nd | 3.90 | nd | nd | 3.90 | nd | nd |
| Sens 6 | 50% | nd | nd | 29% | nd | nd | 17% | nd | nd |
| Spec 6 | 92% | nd | nd | 92% | nd | nd | 92% | nd | nd |
| OR Quart 2 | 0.88 | nd | nd | 1.0 | nd | nd | 1.0 | nd | nd |
| p Value | 0.93 | nd | nd | 1.0 | nd | nd | 1.0 | nd | nd |
| 95% CI of | 0.046 | nd | nd | 0.052 | nd | nd | 0.052 | nd | nd |
| OR Quart 2 | 17 | nd | nd | 19 | nd | nd | 19 | nd | nd |
| OR Quart 3 | 1.0 | nd | nd | 2.3 | nd | nd | 2.3 | nd | nd |
| p Value | 1.0 | nd | nd | 0.53 | nd | nd | 0.53 | nd | nd |
| 95% CI of | 0.052 | nd | nd | 0.17 | nd | nd | 0.17 | nd | nd |
| OR Quart3 | 19 | nd | nd | 33 | nd | nd | 33 | nd | nd |
| OR Quart 4 | 8.8 | nd | nd | 3.5 | nd | nd | 2.3 | nd | nd |
| p Value | 0.086 | nd | nd | 0.33 | nd | nd | 0.53 | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| 95% CI of | 0.74 | nd | nd | 0.28 | nd | nd | 0.17 | nd | nd |
| OR Quart4 | 100 | nd | nd | 43 | nd | nd | 33 | nd | nd |
| | | | | | | | | | |

**Caspase-9**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.549 | 3.00 | 0.549 | 2.16 | 0.549 | 1.09 |
| Average | 1.25 | 2.65 | 1.25 | 1.79 | 1.25 | 1.35 |
| Stdev | 3.21 | 1.71 | 3.21 | 1.60 | 3.21 | 1.25 |
| p(t-test) | | 0.24 | | 0.67 | | 0.94 |
| Min | 0.0360 | 0.146 | 0.0360 | 0.146 | 0.0360 | 0.146 |
| Max | 19.5 | 4.64 | 19.5 | 4.64 | 19.5 | 3.33 |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Samp) | 36 | 8 | 36 | 7 | 36 | 6 |
| n (Patient) | 36 | 8 | 36 | 7 | 36 | 6 |
| | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.79 | nd | nd | 0.69 | nd | nd | 0.64 | nd | nd |
| SE | 0.10 | nd | nd | 0.12 | nd | nd | 0.13 | nd | nd |
| p | 0.0034 | nd | nd | 0.11 | nd | nd | 0.27 | nd | nd |
| nCohort 1 | 36 | nd | nd | 36 | nd | nd | 36 | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| nCohort 2 | 8 | nd | nd | 7 | nd | nd | 6 | nd | nd |
| Cutoff 1 | 1.76 | nd | nd | 0.473 | nd | nd | 0.208 | nd | nd |
| Sens 1 | 75% | nd | nd | 71% | nd | nd | 83% | nd | nd |
| Spec 1 | 89% | nd | nd | 50% | nd | nd | 33% | nd | nd |
| Cutoff 2 | 0.208 | nd | nd | 0.208 | nd | nd | 0.208 | nd | nd |
| Sens 2 | 88% | nd | nd | 86% | nd | nd | 83% | nd | nd |
| Spec 2 | 33% | nd | nd | 33% | nd | nd | 33% | nd | nd |
| Cutoff 3 | 0.135 | nd | nd | 0.135 | nd | nd | 0.135 | nd | nd |
| Sens 3 | 100% | nd | nd | 100% | nd | nd | 100% | nd | nd |
| Spec 3 | 28% | nd | nd | 28% | nd | nd | 28% | nd | nd |
| Cutoff 4 | 0.868 | nd | nd | 0.868 | nd | nd | 0.868 | nd | nd |
| Sens 4 | 75% | nd | nd | 57% | nd | nd | 50% | nd | nd |
| Spec 4 | 72% | nd | nd | 72% | nd | nd | 72% | nd | nd |
| Cutoff 5 | 1.22 | nd | nd | 1.22 | nd | nd | 1.22 | nd | nd |
| Sens 5 | 75% | nd | nd | 57% | nd | nd | 50% | nd | nd |
| Spec 5 | 81% | nd | nd | 81% | nd | nd | 81% | nd | nd |
| Cutoff 6 | 2.17 | nd | nd | 2.17 | nd | nd | 2.17 | nd | nd |
| Sens 6 | 62% | nd | nd | 29% | nd | nd | 17% | nd | nd |
| Spec 6 | 92% | nd | nd | 92% | nd | nd | 92% | nd | nd |
| OR Quart 2 | 2>2.4 | nd | nd | >2.2 | nd | nd | >2.2 | nd | nd |
| p Value | <0.49 | nd | nd | <0.54 | nd | nd | <0.54 | nd | nd |
| 95% CI of | >0.19 | nd | nd | >0.17 | nd | nd | >0.17 | nd | nd |
| OR Quart2 | na | nd | nd | na | nd | nd | na | nd | nd |
| OR Quart 3 | 3>0 | nd | nd | >1.0 | nd | nd | >1.1 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p Value | <na | nd | nd | <1.0 | nd | nd | <0.94 | nd | nd |
| 95% CI of | >na | nd | nd | >0.055 | nd | nd | >0.060 | nd | nd |
| OR Quart3 | na | nd | nd | na | nd | nd | na | nd | nd |
| OR Quart 4 | 4>13 | nd | nd | >5.7 | nd | nd | >3.8 | nd | nd |
| p Value | <0.033 | nd | nd | <0.15 | nd | nd | <0.29 | nd | nd |
| 95% CI of | >1.2 | nd | nd | >0.52 | nd | nd | >0.32 | nd | nd |
| OR Quart4 | na | nd | nd | na | nd | nd | na | nd | nd |

**Cadherin-1**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 66800 | 66800 | 66800 | 66800 | nd | nd |
| Average | 99700 | 95000 | 99700 | 95000 | nd | nd |
| Stdev | 131000 | 95400 | 131000 | 95400 | nd | nd |
| p(t-test) | | 0.92 | | 0.92 | nd | nd |
| Min | 3290 | 2220 | 3290 | 2220 | nd | nd |
| Max | 744000 | 260000 | 744000 | 260000 | nd | nd |
| n (Samp) | 41 | 8 | 41 | 8 | nd | nd |
| n (Patient) | 41 | 8 | 41 | 8 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.49 | nd | nd | 0.49 | nd | nd | nd | nd | nd |
| SE | 0.11 | nd | nd | 0.11 | nd | nd | nd | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| D | 0.91 | nd | nd | 0.91 | nd | nd | nd | nd | nd |
| nCohort 1 | 41 | nd | nd | 41 | nd | nd | nd | nd | nd |
| nCohort 2 | 8 | nd | nd | 8 | nd | nd | nd | nd | nd |
| Cutoff 1 | 16300 | nd | nd | 16300 | nd | nd | nd | nd | nd |
| Sens 1 | 75% | nd | nd | 75% | nd | nd | nd | nd | nd |
| Spec 1 | 17% | nd | nd | 17% | nd | nd | nd | nd | nd |
| Cutoff 2 | 8380 | nd | nd | 8380 | nd | nd | nd | nd | nd |
| Sens 2 | 88% | nd | nd | 88% | nd | nd | nd | nd | nd |
| Spec 2 | 10% | nd | nd | 10% | nd | nd | nd | nd | nd |
| Cutoff 3 | 0 | nd | nd | 0 | nd | nd | nd | nd | nd |
| Sens 3 | 100% | nd | nd | 100% | nd | nd | nd | nd | nd |
| Spec 3 | 0% | nd | nd | 0% | nd | nd | nd | nd | nd |
| Cutoff 4 | 123000 | nd | nd | 123000 | nd | nd | nd | nd | nd |
| Sens 4 | 38% | nd | nd | 38% | nd | nd | nd | nd | nd |
| Spec 4 | 71% | nd | nd | 71% | nd | nd | nd | nd | nd |
| Cutoff 5 | 138000 | nd | nd | 138000 | nd | nd | nd | nd | nd |
| Sens 5 | 25% | nd | nd | 25% | nd | nd | nd | nd | nd |
| Spec 5 | 80% | nd | nd | 80% | nd | nd | nd | nd | nd |
| Cutoff 6 | 155000 | nd | nd | 155000 | nd | nd | nd | nd | nd |
| Sens 6 | 25% | nd | nd | 25% | nd | nd | nd | nd | nd |
| Spec 6 | 90% | nd | nd | 90% | nd | nd | nd | nd | nd |
| OR Quart 2 | 0.30 | nd | nd | 0.30 | nd | nd | nd | nd | nd |
| p Value | 0.33 | nd | nd | 0.33 | nd | nd | nd | nd | nd |
| 95% CI of | 0.027 | nd | nd | 0.027 | nd | nd | nd | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart2 | 3.4 | nd | nd | 3.4 | nd | nd | nd | nd | nd |
| OR Quart 3 | 0.30 | nd | nd | 0.30 | nd | nd | nd | nd | nd |
| p Value | 0.33 | nd | nd | 0.33 | nd | nd | nd | nd | nd |
| 95% CI of | 0.027 | nd | nd | 0.027 | nd | nd | nd | nd | nd |
| OR Quart3 | 3.4 | nd | nd | 3.4 | nd | nd | nd | nd | nd |
| OR Quart 4 | 1.1 | nd | nd | 1.1 | nd | nd | nd | nd | nd |
| p Value | 0.91 | nd | nd | 0.91 | nd | nd | nd | nd | nd |
| 95% CI of | 0.18 | nd | nd | 0.18 | nd | nd | nd | nd | nd |
| OR Quart4 | 7.0 | nd | nd | 7.0 | nd | nd | nd | nd | nd |

**Cyclin-dependent kinase inhibitor 1**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.09 | 14.9 | 3.09 | 9.35 | 3.09 | 4.76 |
| Average | 88.7 | 37.1 | 88.7 | 36.6 | 88.7 | 15.2 |
| Stdev | 688 | 81.6 | 688 | 81.8 | 688 | 16.5 |
| p(t-test) | | 0.77 | | 0.77 | | 0.75 |
| Min | 0.116 | 0.116 | 0.116 | 0.116 | 0.116 | 1.64 |
| Max | 6950 | 327 | 6950 | 327 | 6950 | 44.1 |
| n (Samp) | 102 | 15 | 102 | 15 | 102 | 9 |
| n (Patient) | 102 | 15 | 102 | 15 | 102 | 9 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 4.90 | 18.8 | 4.90 | 15.7 | nd | nd |
| Average | 69.2 | 21.5 | 69.2 | 20.7 | nd | nd |
| Stdev | 541 | 18.2 | 541 | 18.6 | nd | nd |
| | | | | | | |
| p(t-test) | | 0.80 | | 0.80 | nd | nd |
| Min | 1.14E-14 | 0.116 | 1.14E-14 | 0.116 | nd | nd |
| Max | 6950 | 44.1 | 6950 | 44.1 | nd | nd |
| n (Samp) | 168 | 8 | 168 | 8 | nd | nd |
| n (Patient) | 168 | 8 | 168 | 8 | nd | nd |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 3.29 | 14.9 | 3.29 | 14.9 | 3.29 | 4.76 |
| Average | 101 | 50.0 | 101 | 50.0 | 101 | 14.4 |
| Stdev | 753 | 105 | 753 | 105 | 753 | 16.4 |
| p(t-test) | | 0.84 | | 0.84 | | 0.76 |
| Min | 0.116 | 2.45 | 0.116 | 2.45 | 0.116 | 2.45 |
| Max | 6950 | 327 | 6950 | 327 | 6950 | 44.1 |
| n (Samp) | 85 | 9 | 85 | 9 | 85 | 7 |
| n (Patient) | 85 | 9 | 85 | 9 | 85 | 7 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.66 | 0.66 | 0.69 | 0.65 | 0.65 | 0.68 | 0.63 | nd | 0.63 |

EP 2 894 473 B1

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| SE | 0.081 | 0.11 | 0.10 | 0.081 | 0.11 | 0.10 | 0.10 | nd | 0.12 |
| p | 0.050 | 0.15 | 0.067 | 0.062 | 0.18 | 0.078 | 0.22 | nd | 0.28 |
| nCohort 1 | 102 | 168 | 85 | 102 | 168 | 85 | 102 | nd | 85 |
| nCohort 2 | 15 | 8 | 9 | 15 | 8 | 9 | 9 | nd | 7 |
| Cutoff 1 | 2.45 | 7.35 | 2.45 | 2.45 | 7.35 | 2.45 | 2.02 | nd | 2.45 |
| Sens 1 | 73% | 75% | 78% | 73% | 75% | 78% | 89% | nd | 71% |
| Spec 1 | 46% | 59% | 47% | 46% | 59% | 47% | 46% | nd | 47% |
| Cutoff 2 | 2.02 | 1.36 | 2.02 | 2.02 | 1.36 | 2.02 | 2.02 | nd | 2.02 |
| Sens 2 | 87% | 88% | 100% | 87% | 88% | 100% | 89% | nd | 100% |
| Spec 2 | 46% | 40% | 47% | 46% | 40% | 47% | 46% | nd | 47% |
| Cutoff 3 | 1.09 | 1.14E-14 | 2.02 | 1.09 | 1.14E-14 | 2.02 | 1.09 | nd | 2.02 |
| Sens 3 | 93% | 100% | 100% | 93% | 100% | 100% | 100% | nd | 100% |
| Spec 3 | 44% | 1% | 47% | 44% | 1% | 47% | 44% | nd | 47% |
| Cutoff 4 | 12.3 | 13.6 | 12.3 | 12.3 | 13.6 | 12.3 | 12.3 | nd | 12.3 |
| Sens 4 | 53% | 62% | 56% | 47% | 50% | 56% | 44% | nd | 43% |
| Spec 4 | 71% | 70% | 71% | 71% | 70% | 71% | 71% | nd | 71% |
| Cutoff 5 | 25.7 | 26.7 | 25.7 | 25.7 | 26.7 | 25.7 | 25.7 | nd | 25.7 |
| Sens 5 | 33% | 38% | 33% | 33% | 38% | 33% | 33% | nd | 29% |
| Spec 5 | 80% | 80% | 80% | 80% | 80% | 80% | 80% | nd | 80% |
| Cutoff 6 | 55.7 | 56.1 | 44.8 | 55.7 | 56.1 | 44.8 | 55.7 | nd | 44.8 |
| Sens 6 | 7% | 0% | 11% | 7% | 0% | 11% | 0% | nd | 0% |
| Spec 6 | 90% | 90% | 91% | 90% | 90% | 91% | 90% | nd | 91% |
| OR Quart 2 | 4.5 | >2.1 | >3.3 | 4.5 | >2.1 | >3.3 | >4.5 | nd | >3.4 |
| p Value | 0.19 | <0.55 | <0.32 | 0.19 | <0.55 | <0.32 | <0.19 | nd | <0.30 |

EP 2 894 473 B1

140

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| 95% CI of | 0.47 | >0.18 | >0.32 | 0.47 | >0.18 | >0.32 | >0.47 | nd | >0.33 |
| OR Quart2 | 43 | na | na | 43 | na | na | na | nd | na |
| OR Quart 3 | 4.5 | >3.2 | >3.4 | 4.5 | >3.2 | >3.4 | >2.1 | nd | >2.2 |
| p Value | 0.19 | <0.32 | <0.30 | 0.19 | <0.32 | <0.30 | <0.56 | nd | <0.53 |
| 95% CI of | 0.47 | >0.32 | >0.33 | 0.47 | >0.32 | >0.33 | >0.18 | nd | >0.18 |
| OR Quart3 | 43 | na | na | 43 | na | na | na | nd | na |
| OR Quart 4 | 7.0 | >3.2 | >3.3 | 7.0 | >3.2 | >3.3 | >3.2 | nd | >2.2 |
| p Value | 0.081 | <0.32 | <0.32 | 0.081 | <0.32 | <0.32 | <0.32 | nd | <0.53 |
| 95% CI of | 0.79 | >0.32 | >0.32 | 0.79 | >0.32 | >0.32 | >0.32 | nd | >0.18 |
| OR Quart4 | 62 | na | na | 62 | na | na | na | nd | na |

**Carcinoembryonic antigen-related cell adhesion molecule 5**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.14 | 5.45 | 1.14 | 2.86 | 1.14 | 2.98 |
| Average | 3.19 | 7.89 | 3.19 | 7.48 | 3.19 | 5.77 |
| Stdev | 5.64 | 11.7 | 5.64 | 12.1 | 5.64 | 4.90 |
| p(t-test) | | 0.0094 | | 0.021 | | 0.19 |
| Min | 0.00336 | 0.282 | 0.00336 | 0.219 | 0.00336 | 0.852 |
| Max | 43.4 | 50.1 | 43.4 | 50.1 | 43.4 | 14.6 |
| n (Samp) | 102 | 17 | 102 | 16 | 102 | 9 |
| n (Patient) | 102 | 17 | 102 | 16 | 102 | 9 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.57 | 4.46 | 1.57 | 3.11 | nd | nd |
| Average | 28.3 | 4.33 | 28.3 | 3.91 | nd | nd |
| Stdev | 312 | 3.44 | 312 | 3.51 | nd | nd |
| p(t-test) | | 0.83 | | 0.83 | nd | nd |
| Min | 0.00336 | 0.282 | 0.00336 | 0.219 | nd | nd |
| Max | 4070 | 10.9 | 4070 | 10.9 | nd | nd |
| n (Samp) | 170 | 8 | 170 | 8 | nd | nd |
| n (Patient) | 170 | 8 | 170 | 8 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.19 | 6.79 | 1.19 | 4.88 | 1.19 | 2.98 |
| Average | 3.63 | 10.4 | 3.63 | 10.3 | 3.63 | 6.19 |
| Stdev | 6.41 | 14.1 | 6.41 | 14.7 | 6.41 | 5.50 |
| p(t-test) | | 0.0070 | | 0.011 | | 0.31 |
| Min | 0.00336 | 0.356 | 0.00336 | 0.852 | 0.00336 | 0.852 |
| Max | 43.4 | 50.1 | 43.4 | 50.1 | 43.4 | 14.6 |
| n (Samp) | 85 | 11 | 85 | 10 | 85 | 7 |
| n (Patient) | 85 | 11 | 85 | 10 | 85 | 7 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.70 | 0.62 | 0.75 | 0.69 | 0.57 | 0.77 | 0.76 | nd | 0.74 |
| SE | 0.075 | 0.11 | 0.089 | 0.078 | 0.11 | 0.091 | 0.096 | nd | 0.11 |

EP 2 894 473 B1

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p | 0.0072 | 0.28 | 0.0055 | 0.015 | 0.50 | 0.0030 | 0.0080 | nd | 0.034 |
| nCohort 1 | 102 | 170 | 85 | 102 | 170 | 85 | 102 | nd | 85 |
| nCohort 2 | 17 | 8 | 11 | 16 | 8 | 10 | 9 | nd | 7 |
| Cutoff 1 | 2.56 | 2.59 | 2.88 | 2.26 | 2.26 | 2.55 | 2.53 | nd | 2.53 |
| Sens 1 | 71% | 75% | 73% | 75% | 75% | 70% | 78% | nd | 71% |
| Spec 1 | 71% | 65% | 71% | 67% | 61% | 68% | 70% | nd | 68% |
| Cutoff 2 | 0.816 | 0.450 | 1.40 | 1.44 | 0.238 | 2.53 | 1.44 | nd | 1.40 |
| Sens 2 | 82% | 88% | 82% | 81% | 88% | 80% | 89% | nd | 86% |
| Spec 2 | 42% | 20% | 56% | 58% | 8% | 68% | 58% | nd | 56% |
| Cutoff 3 | 0.339 | 0.281 | 0.816 | 0.238 | 0.214 | 1.40 | 0.816 | nd | 0.816 |
| Sens 3 | 94% | 100% | 91% | 94% | 100% | 90% | 100% | nd | 100% |
| Spec 3 | 19% | 10% | 39% | 9% | 5% | 56% | 42% | nd | 39% |
| Cutoff 4 | 2.56 | 3.33 | 2.88 | 2.56 | 3.33 | 2.88 | 2.56 | nd | 2.88 |
| Sens 4 | 71% | 62% | 73% | 56% | 50% | 60% | 67% | nd | 57% |
| Spec 4 | 71% | 70% | 71% | 71% | 70% | 71% | 71% | nd | 71% |
| Cutoff 5 | 4.03 | 5.77 | 5.67 | 4.03 | 5.77 | 5.67 | 4.03 | nd | 5.67 |
| Sens 5 | 53% | 25% | 55% | 44% | 25% | 50% | 44% | nd | 43% |
| | | | | | | | | | |
| Spec 5 | 80% | 80% | 80% | 80% | 80% | 80% | 80% | nd | 80% |
| Cutoff 6 | 8.86 | 11.0 | 9.55 | 8.86 | 11.0 | 9.55 | 8.86 | nd | 9.55 |
| Sens 6 | 29% | 0% | 45% | 25% | 0% | 40% | 33% | nd | 43% |
| Spec 6 | 90% | 90% | 91% | 90% | 90% | 91% | 90% | nd | 91% |
| OR Quart 2 | 0.30 | 0 | 1.0 | 0.47 | 0 | >1.0 | >1.0 | nd | >1.0 |
| p Value | 0.31 | na | 1.0 | 0.54 | na | <1.0 | <1.0 | nd | <0.98 |

EP 2 894 473 B1

143

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| 95% CI of | 0.029 | na | 0.059 | 0.040 | na | >0.059 | >0.059 | nd | >0.062 |
| OR Quart2 | 3.1 | na | 17 | 5.4 | na | na | na | nd | na |
| OR Quart 3 | 1.3 | 1.0 | 3.3 | 3.5 | 1.5 | >4.6 | >4.5 | nd | >3.4 |
| p Value | 0.72 | 1.0 | 0.32 | 0.15 | 0.65 | <0.19 | <0.19 | nd | <0.30 |
| 95% CI of | 0.27 | 0.13 | 0.32 | 0.65 | 0.24 | >0.47 | >0.47 | nd | >0.33 |
| OR Quart3 | 6.6 | 7.4 | 34 | 19 | 9.7 | na | na | nd | na |
| OR Quart 4 | 3.7 | 2.0 | 7.7 | 4.1 | 1.5 | >6.1 | >4.5 | nd | >3.4 |
| p Value | 0.072 | 0.42 | 0.070 | 0.097 | 0.67 | <0.11 | <0.19 | nd | <0.30 |
| 95% CI of | 0.89 | 0.36 | 0.85 | 0.78 | 0.24 | >0.65 | >0.47 | nd | >0.33 |
| OR Quart4 | 15 | 12 | 70 | 22 | 9.4 | na | na | nd | na |

**Myoglobin**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 5.42 | 18.9 | 5.42 | 11.9 | 5.42 | 44.9 |
| Average | 51.4 | 149 | 51.4 | 155 | 51.4 | 174 |
| Stdev | 121 | 203 | 121 | 208 | 121 | 213 |
| p(t-test) |  | 0.0066 |  | 0.0052 |  | 0.0076 |
| Min | 0.00616 | 0.0439 | 0.00616 | 0.0439 | 0.00616 | 0.0772 |
| Max | 618 | 469 | 618 | 469 | 618 | 469 |
| n (Samp) | 102 | 17 | 102 | 16 | 102 | 9 |
| n (Patient) | 102 | 17 | 102 | 16 | 102 | 9 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.65 | 31.9 | 3.65 | 29.4 | nd | nd |
| Average | 63.1 | 42 | 63.1 | 141 | nd | nd |
| Stdev | 142 | 95 | 142 | 196 | nd | nd |
| p(t-test) | | 0.13 | | 0.14 | nd | nd |
| Min | 0.00616 | 0.0439 | 0.00616 | 0.0439 | nd | nd |
| Max | 618 | 469 | 618 | 469 | nd | nd |
| n (Samp) | 170 | 8 | 170 | 8 | nd | nd |
| n (Patient) | 170 | 8 | 170 | 8 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.83 | 0.377 | 3.83 | 2.87 | 3.83 | 5.36 |
| Average | 56.6 | 143 | 56.6 | 152 | 56.6 | 150 |
| Stdev | 128 | 203 | 128 | 212 | 128 | 207 |
| p(t-test) | | 0.055 | | 0.042 | | 0.081 |
| Min | 0.00616 | 0.0696 | 0.00616 | 0.0772 | 0.00616 | 0.0772 |
| Max | 618 | 469 | 618 | 469 | 618 | 469 |
| n (Samp) | 85 | 11 | 85 | 10 | 85 | 7 |
| n (Patient) | 85 | 11 | 85 | 10 | 85 | 7 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.57 | 0.64 | 0.49 | 0.57 | 0.63 | 0.51 | 0.62 | nd | 0.54 |
| SE | 0.078 | 0.11 | 0.093 | 0.080 | 0.11 | 0.097 | 0.10 | nd | 0.12 |
| p | 0.36 | 0.21 | 0.95 | 0.36 | 0.24 | 0.93 | 0.25 | nd | 0.73 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| nCohort 1 | 102 | 170 | 85 | 102 | 170 | 85 | 102 | nd | 85 |
| nCohort 2 | 17 | 8 | 11 | 16 | 8 | 10 | 9 | nd | 7 |
| Cutoff 1 | 0.288 | 10.2 | 0.117 | 0.288 | 9.39 | 0.288 | 0.288 | nd | 0.288 |
| Sens 1 | 71% | 75% | 73% | 75% | 75% | 70% | 78% | nd | 71% |
| Spec 1 | 21% | 61% | 14% | 21% | 61% | 20% | 21% | nd | 20% |
| Cutoff 2 | 0.0772 | 0.0780 | 0.0772 | 0.117 | 0.0780 | 0.117 | 0.117 | nd | 0.117 |
| Sens 2 | 82% | 88% | 82% | 81% | 88% | 80% | 89% | nd | 86% |
| Spec 2 | 13% | 14% | 11% | 16% | 14% | 14% | 16% | nd | 14% |
| Cutoff 3 | 0.0606 | 0.0411 | 0.0696 | 0.0606 | 0.0411 | 0.0772 | 0.0606 | nd | 0.0661 |
| Sens 3 | 94% | 100% | 91% | 94% | 100% | 90% | 100% | nd | 100% |
| Spec 3 | 13% | 9% | 11% | 13% | 9% | 11% | 13% | nd | 11% |
| Cutoff 4 | 17.2 | 18.6 | 17.9 | 17.2 | 18.6 | 17.9 | 17.2 | nd | 17.9 |
| Sens 4 | 53% | 62% | 45% | 44% | 50% | 40% | 56% | nd | 43% |
| Spec 4 | 71% | 70% | 71% | 71% | 70% | 71% | 71% | nd | 71% |
| Cutoff 5 | 38.9 | 45.3 | 55.8 | 38.9 | 45.3 | 55.8 | 38.9 | nd | 55.8 |
| Sens 5 | 47% | 38% | 36% | 44% | 38% | 40% | 56% | nd | 43% |
| Spec 5 | 80% | 80% | 80% | 80% | 80% | 80% | 80% | nd | 80% |
| Cutoff 6 | 222 | 234 | 232 | 222 | 234 | 232 | 222 | nd | 232 |
| Sens 6 | 29% | 25% | 27% | 31% | 25% | 30% | 33% | nd | 29% |
| Spec 6 | 90% | 90% | 91% | 90% | 90% | 91% | 90% | nd | 91% |
| OR Quart 2 | 0.13 | 0 | 0 | 0.13 | 0 | 0.21 | 0.30 | nd | 0 |
| p Value | 0.070 | na | na | 0.070 | na | 0.17 | 0.31 | nd | na |
| 95% CI of | 0.015 | na | na | 0.015 | na | 0.021 | 0.029 | nd | na |
| OR Quart2 | 1.2 | na | na | 1.2 | na | 2.0 | 3.0 | nd | na |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart 3 | 0.27 | 1.0 | 0.17 | 0.28 | 1.0 | 0.21 | 0 | nd | 0.30 |
| p Value | 0.13 | 1.0 | 0.11 | 0.15 | 1.0 | 0.17 | na | nd | 0.32 |
| 95% CI of | 0.050 | 0.13 | 0.018 | 0.052 | 0.13 | 0.021 | na | nd | 0.029 |
| OR Quart3 | 1.5 | 7.4 | 1.5 | 1.5 | 7.4 | 2.0 | na | nd | 3.2 |
| OR Quart 4 | 1.4 | 2.0 | 1.0 | 1.2 | 2.0 | 0.95 | 1.7 | nd | 1.0 |
| p Value | 0.59 | 0.42 | 1.0 | 0.81 | 0.42 | 0.95 | 0.48 | nd | 1.0 |
| 95% CI of | 0.42 | 0.36 | 0.25 | 0.34 | 0.36 | 0.21 | 0.37 | nd | 0.18 |
| OR Quart4 | 4.7 | 12 | 4.0 | 4.0 | 12 | 4.4 | 8.1 | nd | 5.6 |

**Mucin-16**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.684 | 1.03 | 0.684 | 1.03 | nd | nd |
| Average | 1.18 | 54.9 | 1.18 | 54.9 | nd | nd |
| Stdev | 1.41 | 153 | 1.41 | 153 | nd | nd |
| p(t-test) | | 0.023 | | 0.023 | nd | nd |
| Min | 0.141 | 0.304 | 0.141 | 0.304 | nd | nd |
| Max | 6.37 | 433 | 6.37 | 433 | nd | nd |
| n (Samp) | 41 | 8 | 41 | 8 | nd | nd |
| n (Patient) | 41 | 8 | 41 | 8 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.65 | nd | nd | 0.65 | nd | nd | nd | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| SE | 0.11 | nd | nd | 0.11 | nd | nd | nd | nd | nd |
| p | 0.20 | nd | nd | 0.20 | nd | nd | nd | nd | nd |
| nCohort 1 | 41 | nd | nd | 41 | nd | nd | nd | nd | nd |
| nCohort 2 | 8 | nd | nd | 8 | nd | nd | nd | nd | nd |
| Cutoff 1 | 0.781 | nd | nd | 0.781 | nd | nd | nd | nd | nd |
| Sens 1 | 75% | nd | nd | 75% | nd | nd | nd | nd | nd |
| Spec 1 | 61% | nd | nd | 61% | nd | nd | nd | nd | nd |
| Cutoff 2 | 0.464 | nd | nd | 0.464 | nd | nd | nd | nd | nd |
| Sens 2 | 88% | nd | nd | 88% | nd | nd | nd | nd | nd |
| Spec 2 | 32% | nd | nd | 32% | nd | nd | nd | nd | nd |
| Cutoff 3 | 0.278 | nd | nd | 0.278 | nd | nd | nd | nd | nd |
| Sens 3 | 100% | nd | nd | 100% | nd | nd | nd | nd | nd |
| Spec 3 | 10% | nd | nd | 10% | nd | nd | nd | nd | nd |
| Cutoff 4 | 0.961 | nd | nd | 0.961 | nd | nd | nd | nd | nd |
| Sens 4 | 50% | nd | nd | 50% | nd | nd | nd | nd | nd |
| Spec 4 | 71% | nd | nd | 71% | nd | nd | nd | nd | nd |
| Cutoff 5 | 1.31 | nd | nd | 1.31 | nd | nd | nd | nd | nd |
| Sens 5 | 25% | nd | nd | 25% | nd | nd | nd | nd | nd |
| Spec 5 | 80% | nd | nd | 80% | nd | nd | nd | nd | nd |
| Cutoff 6 | 3.42 | nd | nd | 3.42 | nd | nd | nd | nd | nd |
| Sens 6 | 12% | nd | nd | 12% | nd | nd | nd | nd | nd |
| Spec 6 | 90% | nd | nd | 90% | nd | nd | nd | nd | nd |
| OR Quart 2 | 1.0 | nd | nd | 1.0 | nd | nd | nd | nd | nd |
| p Value | 1.0 | nd | nd | 1.0 | nd | nd | nd | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| 95% CI of | 0.055 | nd | nd | 0.055 | nd | nd | nd | nd | nd |
| OR Quart2 | 18 | nd | nd | 18 | nd | nd | nd | nd | nd |
| OR Quart 3 | 3.7 | nd | nd | 3.7 | nd | nd | nd | nd | nd |
| p Value | 0.29 | nd | nd | 0.29 | nd | nd | nd | nd | nd |
| 95% CI of | 0.32 | nd | nd | 0.32 | nd | nd | nd | nd | nd |
| OR Quart3 | 42 | nd | nd | 42 | nd | nd | nd | nd | nd |
| OR Quart 4 | 3.3 | nd | nd | 3.3 | nd | nd | nd | nd | nd |
| p Value | 0.33 | nd | nd | 0.33 | nd | nd | nd | nd | nd |
| 95% CI of | 0.29 | nd | nd | 0.29 | nd | nd | nd | nd | nd |
| OR Quart4 | 37 | nd | nd | 37 | nd | nd | nd | nd | nd |

**Poly [ADP-ribose] polymerase 1 (cleaved)**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.00E-9 | 0.0140 | 1.00E-9 | 0.0140 | 1.00E-9 | 1.00E-9 |
| Average | 0.00479 | 0.0101 | 0.00479 | 0.00956 | 0.00479 | 0.00605 |
| Stdev | 0.00701 | 0.00857 | 0.00701 | 0.00900 | 0.00701 | 0.00754 |
| p(t-test) | | 0.018 | | 0.034 | | 0.65 |
| Min | 1.00E-9 | 1.00E-9 | 1.00E-9 | 1.00E-9 | 1.00E-9 | 1.00E-9 |
| Max | 0.0357 | 0.0253 | 0.0357 | 0.0253 | 0.0357 | 0.0144 |
| n (Samp) | 97 | 12 | 97 | 12 | 97 | 7 |
| n (Patient) | 97 | 12 | 97 | 12 | 97 | 7 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.00E-9 | 0.00990 | 1.00E-9 | 0.00691 | nd | nd |
| Average | 0.00475 | 0.00992 | 0.00475 | 0.00892 | nd | nd |
| Stdev | 0.00651 | 0.00984 | 0.00651 | 0.0106 | nd | nd |
| p(t-test) | | 0.063 | | 0.13 | nd | nd |
| Min | 1.00E-9 | 1.00E-9 | 1.00E-9 | 1.00E-9 | nd | nd |
| Max | 0.0357 | 0.0253 | 0.0357 | 0.0253 | nd | nd |
| n (Samp) | 159 | 6 | 159 | 6 | nd | nd |
| n (Patient) | 159 | 6 | 159 | 6 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.00E-9 | 0.0140 | 1.00E-9 | 0.0140 | 1.00E-9 | 0.00691 |
| Average | 0.00380 | 0.00938 | 0.00380 | 0.00938 | 0.00380 | 0.00706 |
| Stdev | 0.00658 | 0.00789 | 0.00658 | 0.00789 | 0.00658 | 0.00773 |
| p(t-test) | | 0.027 | | 0.027 | | 0.25 |
| Min | 1.00E-9 | 1.00E-9 | 1.00E-9 | 1.00E-9 | 1.00E-9 | 1.00E-9 |
| Max | 0.0357 | 0.0183 | 0.0357 | 0.0183 | 0.0357 | 0.0144 |
| n (Samp) | 84 | 8 | 84 | 8 | 84 | 6 |
| n (Patient) | 84 | 8 | 84 | 8 | 84 | 6 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.67 | 0.65 | 0.69 | 0.64 | 0.58 | 0.69 | 0.52 | nd | 0.60 |
| SE | 0.090 | 0.12 | 0.11 | 0.091 | 0.12 | 0.11 | 0.11 | nd | 0.13 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p | 0.059 | 0.23 | 0.081 | 0.13 | 0.51 | 0.081 | 0.87 | nd | 0.43 |
| nCohort 1 | 97 | 159 | 84 | 97 | 159 | 84 | 97 | nd | 84 |
| nCohort 2 | 12 | 6 | 8 | 12 | 6 | 8 | 7 | nd | 6 |
| Cutoff 1 | 0 | 0 | 0 | 0 | 0 | 0 | 0 | nd | 0 |
| Sens 1 | 100% | 100% | 100% | 100% | 100% | 100% | 100% | nd | 100% |
| Spec 1 | 0% | 0% | 0% | 0% | 0% | 0% | 0% | nd | 0% |
| Cutoff 2 | 0 | 0 | 0 | 0 | 0 | 0 | 0 | nd | 0 |
| Sens 2 | 100% | 100% | 100% | 100% | 100% | 100% | 100% | nd | 100% |
| Spec 2 | 0% | 0% | 0% | 0% | 0% | 0% | 0% | nd | 0% |
| Cutoff 3 | 0 | 0 | 0 | | | 100% | 0 | nd | 0 |
| Sens 3 | 100% | 100% | 100% | 100% | 100% | | 100% | nd | 100% |
| Spec 3 | 0% | 0% | 0% | 0% | 0% | 0% | 0% | nd | 0% |
| Cutoff 4 | 0.00598 | 0.00598 | 0.00471 | 0.00598 | 0.00598 | 0.00471 | 0.00598 | nd | 0.0047 1 |
| Sens 4 | 58% | 50% | 62% | 58% | 50% | 62% | 43% | nd | 50% |
| Spec 4 | 70% | 71% | 74% | 70% | 71% | 74% | 70% | nd | 74% |
| Cutoff 5 | 0.0144 | 0.0144 | 0.0120 | 0.0144 | 0.0144 | 0.0120 | 0.0144 | nd | 0.0120 |
| Sens 1 5 | 17% | 17% | 62% | 17% | 17% | 62% | 0% | nd | 50% |
| Spec 5 | 94% | 96% | 81% | 94% | 96% | 81% | 94% | nd | 81% |
| Cutoff 6 | 0.0144 | 0.0144 | 0.0144 | 0.0144 | 0.0144 | 0.0144 | 0.0144 | nd | 0.0144 |
| Sens 6 | 17% | 17% | 12% | 17% | 17% | 12% | 0% | nd | 0% |
| Spec 6 | 94% | 96% | 99% | 94% | 96% | 99% | 94% | nd | 99% |
| OR Quart 2 | 1.0 | 1.0 | 2.1 | 1.6 | 2.1 | 2.1 | 3.3 | nd | 2.0 |
| p Value | 1.0 | 1.0 | 0.56 | 0.64 | 0.56 | 0.56 | 0.32 | nd | 0.58 |
| 95% CI of | 0.13 | 0.060 | 0.18 | 0.24 | 0.18 | 0.18 | 0.32 | nd | 0.17 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart2 | 7.7 | 17 | 25 | 10 | 24 | 25 | 34 | nd | 24 |
| OR Quart 3 | 1.0 | 1.0 | 0 | 0 | 0 | 0 | 1.0 | nd | 0 |
| p Value | 1.0 | 1.0 | na | na | na | na | 1.0 | nd | na |
| 95% CI of | 0.13 | 0.060 | na | na | na | na | 0.059 | nd | na |
| OR Quart3 | 7.7 | 17 | na | na | na | na | 17 | nd | na |
| OR Quart 4 | 3.4 | 3.1 | 6.1 | 4.2 | 3.1 | 6.1 | 2.1 | nd | 3.1 |
| p Value | 0.16 | 0.34 | 0.11 | 0.095 | 0.34 | 0.11 | 0.56 | nd | 0.34 |
| 95% CI of | 0.62 | 0.31 | 0.65 | 0.78 | 0.31 | 0.65 | 0.18 | nd | 0.30 |
| OR Quart4 | 19 | 31 | 57 | 22 | 31 | 57 | 25 | nd | 33 |

**KSP-Cadherin**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 1.23 | 2.26 | 1.23 | 2.26 | nd | nd |
| Average | 1.66 | 2.48 | 1.66 | 2.48 | nd | nd |
| Stdev | 1.71 | 1.19 | 1.71 | 1.19 | nd | nd |
| p(t-test) |  | 0.17 |  | 0.17 | nd | nd |
| Min | 0.00263 | 0.562 | 0.00263 | 0.562 | nd | nd |
| Max | 11.9 | 4.23 | 11.9 | 4.23 | nd | nd |
| n (Samp) | 68 | 9 | 68 | 9 | nd | nd |
| n (Patient) | 68 | 9 | 68 | 9 | nd | nd |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.17 | 1.87 | 1.17 | 1.87 | nd | nd |
| Average | 1.76 | 2.07 | 1.76 | 2.07 | nd | nd |
| Stdev | 1.84 | 1.22 | 1.84 | 1.22 | nd | nd |
| p(t-test) | | 0.69 | | 0.69 | nd | nd |
| Min | 0.00263 | 0.562 | 0.00263 | 0.562 | nd | nd |
| Max | 11.9 | 4.23 | 11.9 | 4.23 | nd | nd |
| n (Samp) | 59 | 6 | 59 | 6 | nd | nd |
| n (Patient) | 59 | 6 | 59 | 6 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.74 | nd | 0.64 | 0.74 | nd | 0.64 | nd | nd | nd |
| SE | 0.099 | nd | 0.13 | 0.099 | nd | 0.13 | nd | nd | nd |
| p | 0.017 | nd | 0.29 | 0.017 | nd | 0.29 | nd | nd | nd |
| nCohort 1 | 68 | nd | 59 | 68 | nd | 59 | nd | nd | nd |
| nCohort 2 | 9 | nd | 6 | 9 | nd | 6 | nd | nd | nd |
| Cutoff 1 | 1.61 | nd | 1.51 | 1.61 | nd | 1.51 | nd | nd | nd |
| Sens 1 | 78% | nd | 83% | 78% | nd | 83% | nd | nd | nd |
| Spec 1 | 60% | nd | 59% | 60% | nd | 59% | nd | nd | nd |
| Cutoff 2 | 1.56 | nd | 1.51 | 1.56 | nd | 1.51 | nd | nd | nd |
| Sens 2 | 89% | nd | 83% | 89% | nd | 83% | nd | nd | nd |
| Spec 2 | 60% | nd | 59% | 60% | nd | 59% | nd | nd | nd |
| Cutoff 3 | 0.466 | nd | 0.494 | 0.466 | nd | 0.494 | nd | nd | nd |
| Sens 3 | 100% | nd | 100% | 100% | nd | 100% | nd | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 3 | 19% | nd | 19% | 19% | nd | 19% | nd | nd | nd |
| Cutoff 4 | 1.99 | nd | 2.08 | 1.99 | nd | 2.08 | nd | nd | nd |
| Sens 4 | 67% | nd | 50% | 67% | nd | 50% | nd | nd | nd |
| Spec 4 | 71% | nd | 71% | 71% | nd | 71% | nd | nd | nd |
| Cutoff 5 | 2.24 | nd | 2.54 | 2.24 | nd | 2.54 | nd | nd | nd |
| Sens 5 | 56% | nd | 17% | 56% | nd | 17% | nd | nd | nd |
| Spec 5 | 81% | nd | 81% | 81% | nd | 81% | nd | nd | nd |
| Cutoff 6 | 3.17 | nd | 3.77 | 3.17 | nd | 3.77 | nd | nd | nd |
| Sens 6 | 22% | nd | 17% | 22% | nd | 17% | nd | nd | nd |
| Spec 6 | 91% | nd | 92% | 91% | nd | 92% | nd | nd | nd |
| OR Quart 2 | 0 | nd | 0 | 0 | nd | 0 | nd | nd | nd |
| p Value | na | nd | na | na | nd | na | nd | nd | nd |
| 95% CI of | na | nd | na | na | nd | na | nd | nd | nd |
| OR Quart2 | na | nd | na | na | nd | na | nd | nd | nd |
| OR Quart 3 | 3.4 | nd | 3.5 | 3.4 | nd | 3.5 | nd | nd | nd |
| n Value | 0.31 | nd | 0.31 | 0.31 | nd | 0.31 | nd | nd | nd |
| 95% CI of | 0.32 | nd | 0.32 | 0.32 | nd | 0.32 | nd | nd | nd |
| OR Quart3 | 36 | nd | 37 | 36 | nd | 37 | nd | nd | nd |
| OR Quart 4 | 6.0 | nd | 2.0 | 6.0 | nd | 2.0 | nd | nd | nd |
| p Value | 0.12 | nd | 0.59 | 0.12 | nd | 0.59 | nd | nd | nd |
| 95% CI of | 0.63 | nd | 0.16 | 0.63 | nd | 0.16 | nd | nd | nd |
| OR Quart4 | 57 | nd | 24 | 57 | nd | 24 | nd | nd | nd |

| Tumor necrosis factor receptor superfamily member 10B | | | | | | | |
|---|---|---|---|---|---|---|---|
| sCr or UO | 0hr prior to AKI stage | | | 24hr prior to AKI stage | | 48hr prior to AKI stage | |
| | Cohort 1 | | Cohort 2 | Cohort 1 | Cohort 2 | Cohort 1 | Cohort 2 |
| Median | 0.141 | | 0.904 | 0.141 | 0.904 | nd | nd |
| Average | 0.957 | | 1.95 | 0.957 | 1.95 | nd | nd |
| Stdev | 1.61 | | 2.64 | 1.61 | 2.64 | nd | nd |
| p(t-test) | | | 0.16 | | 0.16 | nd | nd |
| Min | 0.00360 | | 0.104 | 0.00360 | 0.104 | nd | nd |
| Max | 6.71 | | 7.54 | 6.71 | 7.54 | nd | nd |
| n (Samp) | 41 | | 8 | 41 | 8 | nd | nd |
| n (Patient) | 41 | | 8 | 41 | 8 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.69 | nd | nd | 0.69 | nd | nd | nd | nd | nd |
| SE | 0.11 | nd | nd | 0.11 | nd | nd | nd | nd | nd |
| p | 0.092 | nd | nd | 0.092 | nd | nd | nd | nd | nd |
| nCohort 1 | 41 | nd | nd | 41 | nd | nd | nd | nd | nd |
| nCohort 2 | 8 | nd | nd | 8 | nd | nd | nd | nd | nd |
| Cutoff 1 | 0.141 | nd | nd | 0.141 | nd | nd | nd | nd | nd |
| Sens 1 | 75% | nd | nd | 75% | nd | nd | nd | nd | nd |
| Spec 1 | 51% | nd | nd | 51% | nd | nd | nd | nd | nd |
| Cutoff 2 | 0.107 | nd | nd | 0.107 | nd | nd | nd | nd | nd |
| Sens 2 | 88% | nd | nd | 88% | nd | nd | nd | nd | nd |
| Spec 2 | 49% | nd | nd | 49% | nd | nd | nd | nd | nd |
| Cutoff 3 | 0.0796 | nd | nd | 0.0796 | nd | nd | nd | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 3 | 100% | nd | nd | 100% | nd | nd | nd | nd | nd |
| Spec 3 | 41% | nd | nd | 41% | nd | nd | nd | nd | nd |
| Cutoff 4 | 0.693 | nd | nd | 0.693 | nd | nd | nd | nd | nd |
| Sens 4 | 50% | nd | nd | 50% | nd | nd | nd | nd | nd |
| Spec 4 | 71% | nd | nd | 71% | nd | nd | nd | nd | nd |
| Cutoff 5 | 1.56 | nd | nd | 1.56 | nd | nd | nd | nd | nd |
| Sens 5 | 38% | nd | nd | 38% | nd | nd | nd | nd | nd |
| Spec 5 | 80% | nd | nd | 80% | nd | nd | nd | nd | nd |
| Cutoff 6 | 3.31 | nd | nd | 3.31 | nd | nd | nd | nd | nd |
| Sens 6 | 25% | nd | nd | 25% | nd | nd | nd | nd | nd |
| Spec 6 | 90% | nd | nd | 90% | nd | nd | nd | nd | nd |
| OR Quart 2 | >4.0 | nd | nd | >4.0 | nd | nd | nd | nd | nd |
| p Value | <0.26 | nd | nd | <0.26 | nd | nd | nd | nd | nd |
| 95% CI of | >0.35 | nd | nd | >0.35 | nd | nd | nd | nd | nd |
| OR Quart2 | na | nd | nd | na | nd | nd | nd | nd | nd |
| OR Quart 3 | >1.1 | nd | nd | >1.1 | nd | nd | nd | nd | nd |
| p Value | <0.95 | nd | nd | <0.95 | nd | nd | nd | nd | nd |
| 95% CI of | >0.061 | nd | nd | >0.061 | nd | nd | nd | nd | nd |
| OR Quart3 | na | nd | nd | na | nd | nd | nd | nd | nd |
| OR Quart 4 | >5.3 | nd | nd | >5.3 | nd | nd | nd | nd | nd |
| p Value | <0.16 | nd | nd | <0.16 | nd | nd | nd | nd | nd |
| 95% CI of | >0.51 | nd | nd | >0.51 | nd | nd | nd | nd | nd |
| OR Quart4 | na | nd | nd | na | nd | nd | nd | nd | nd |

Table 5: Comparison of marker levels in EDTA samples collected from Cohort 1 (patients that did not progress beyond RIFLE stage 0) and in EDTA samples collected from subjects at 0, 24 hours, and 48 hours prior to reaching stage R, I or F in Cohort 2.

| Apolipoprotein A-II | | | | | | |
|---|---|---|---|---|---|---|
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 50600 | 49600 | 50600 | 44300 | 50600 | 54300 |
| Average | 51700 | 52000 | 51700 | 50700 | 51700 | 57500 |
| Stdev | 22200 | 21100 | 22200 | 36000 | 22200 | 32700 |
| p(t-test) | | 0.94 | | 0.83 | | 0.29 |
| Min | 5450 | 7970 | 5450 | 7680 | 5450 | 8560 |
| Max | 105000 | 97000 | 105000 | 253000 | 105000 | 152000 |
| n (Samp) | 105 | 45 | 105 | 50 | 105 | 24 |
| n (Patient) | 97 | 45 | 97 | 50 | 97 | 24 |
| | | | | | | |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 49500 | 59900 | 49500 | 50300 | 49500 | 57100 |
| Average | 51700 | 60100 | 51700 | 64100 | 51700 | 67500 |
| Stdev | 26400 | 18100 | 26400 | 55400 | 26400 | 44100 |
| p(t-test) | | 0.26 | | 0.097 | | 0.062 |
| Min | 1810 | 34500 | 1810 | 12100 | 1810 | 10900 |
| Max | 253000 | 95300 | 253000 | 251000 | 253000 | 176000 |
| n (Samp) | 246 | 13 | 246 | 16 | 246 | 11 |
| n (Patient) | 160 | 13 | 160 | 16 | 160 | 11 |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 50200 | 49600 | 50200 | 43800 | 50200 | 51400 |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Average | 52500 | 51300 | 52500 | 51600 | 52500 | 58800 |
| Stdev | 20200 | 21600 | 20200 | 38000 | 20200 | 32900 |
| p(t-test) | | 0.76 | | 0.86 | | 0.25 |
| Min | 8680 | 7970 | 8680 | 7680 | 8680 | 8560 |
| Max | 123000 | 97000 | 123000 | 253000 | 123000 | 152000 |
| n (Samp) | 96 | 40 | 96 | 44 | 96 | 21 |
| n (Patient) | 84 | 40 | 84 | 44 | 84 | 21 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.51 | 0.63 | 0.48 | 0.45 | 0.55 | 0.44 | 0.55 | 0.60 | 0.55 |
| SE | 0.052 | 0.085 | 0.055 | 0.050 | 0.076 | 0.053 | 0.066 | 0.092 | 0.071 |
| p | 0.89 | 0.12 | 0.75 | 0.35 | 0.55 | 0.24 | 0.50 | 0.27 | 0.49 |
| nCohort 1 | 105 | 246 | 96 | 105 | 246 | 96 | 105 | 246 | 96 |
| nCohort 2 | 45 | 13 | 40 | 50 | 16 | 44 | 24 | 11 | 21 |
| Cutoff 1 | 39500 | 43000 | 39500 | 35500 | 37700 | 35000 | 40700 | 46100 | 40900 |
| Sens 1 | 71% | 77% | 70% | 70% | 75% | 70% | 71% | 73% | 71% |
| Spec 1 | 33% | 40% | 28% | 24% | 31% | 19% | 35% | 46% | 31% |
| Cutoff 2 | 33000 | 42600 | 32500 | 29600 | 35500 | 25400 | 29600 | 37000 | 35000 |
| Sens 2 | 80% | 85% | 80% | 80% | 81% | 82% | 83% | 82% | 81% |
| Spec 2 | 22% | 39% | 16% | 19% | 24% | 6% | 19% | 29% | 19% |
| Cutoff 3 | 23400 | 37000 | 23400 | 20800 | 14000 | 20800 | 14000 | 36200 | 21600 |
| Sens 3 | 91% | 92% | 90% | 90% | 94% | 91% | 92% | 91% | 90% |
| Spec 3 | 10% | 29% | 5% | 6% | 5% | 3% | 4% | 27% | 4% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 4 | 61300 | 61500 | 61500 | 61300 | 61500 | 61500 | 61300 | 61500 | 61500 |
| Sens 4 | 33% | 46% | 30% | 32% | 44% | 34% | 46% | 36% | 48% |
| Spec 4 | 70% | 70% | 71% | 70% | 70% | 71% | 70% | 70% | 71% |
| Cutoff 5 | 69000 | 69400 | 67400 | 69000 | 69400 | 67400 | 69000 | 69400 | 67400 |
| Sens 5 | 24% | 38% | 22% | 18% | 38% | 16% | 33% | 36% | 43% |
| Spec 5 | 80% | 80% | 80% | 80% | 80% | 80% | 80% | 80% | 80% |
| Cutoff 6 | 82000 | 82800 | 77900 | 82000 | 82800 | 77900 | 82000 | 82800 | 77900 |
| Sens 6 | 9% | 8% | 12% | 8% | 12% | 11% | 21% | 36% | 19% |
| Spec 6 | 90% | 90% | 91% | 90% | 90% | 91% | 90% | 90% | 91% |
| OR Quart | 21.6 | 4.1 | 0.74 | 0.60 | 0.98 | 0.76 | 0.80 | 4.2 | 0.61 |
| p Value | 0.36 | 0.21 | 0.58 | 0.32 | 0.98 | 0.60 | 0.74 | 0.21 | 0.49 |
| 95% CI of | 0.59 | 0.45 | 0.25 | 0.22 | 0.24 | 0.27 | 0.22 | 0.46 | 0.15 |
| OR Quart2 | 4.2 | 38 | 2.2 | 1.6 | 4.1 | 2.1 | 3.0 | 39 | 2.5 |
| OR Quart 3 | 30.87 | 3.0 | 1.1 | 1.1 | 0.48 | 0.65 | 0.62 | 2.0 | 0.28 |
| p Value | 0.79 | 0.34 | 0.79 | 0.81 | 0.41 | 0.42 | 0.49 | 0.57 | 0.15 |
| 95% CI of | 0.31 | 0.31 | 0.41 | 0.44 | 0.086 | 0.22 | 0.16 | 0.18 | 0.052 |
| OR Quart3 | 2.5 | 30 | 3.2 | 2.8 | 2.7 | 1.9 | 2.4 | 23 | 1.5 |
| OR Quart | 41.2 | 5.2 | 1.1 | 1.2 | 1.5 | 1.8 | 1.6 | 4.1 | 1.6 |
| p Value | 0.67 | 0.14 | 0.79 | 0.75 | 0.53 | 0.22 | 0.42 | 0.21 | 0.41 |
| 95% CI of | 0.46 | 0.60 | 0.41 | 0.46 | 0.41 | 0.69 | 0.50 | 0.45 | 0.50 |
| OR Quart4 | 3.4 | 46 | 3.2 | 3.0 | 5.7 | 4.9 | 5.2 | 38 | 5.4 |

(continued)

| Bcl2 antagonist of cell death | | | | | | |
|---|---|---|---|---|---|---|
| sCr or UO | 0hr prior to AKI stage | | 24hr prior to AKI stage | | 48hr prior to AKI stage | |
|  | Cohort 1 | Cohort 2 | Cohort 1 | Cohort 2 | Cohort 1 | Cohort 2 |
| Median | 0.432 | 0.221 | nd | nd | nd | nd |
| Average | 0.590 | 0.255 | nd | nd | nd | nd |
| Stdev | 0.478 | 0.204 | nd | nd | nd | nd |
| D(t-test) |  | 0.10 | nd | nd | nd | nd |
| Min | 0.0873 | 0.0786 | nd | nd | nd | nd |
| Max | 2.49 | 0.651 | nd | nd | nd | nd |
| n (Samp) | 32 | 6 | nd | nd | nd | nd |
| n (Patient) | 20 | 6 | nd | nd | nd | nd |
|  | | | | | | |
| UO only | 0hr prior to AKI stage | | 24hr prior to AKI stage | | 48hr prior to AKI stage | |
|  | Cohort 1 | Cohort 2 | Cohort 1 | Cohort 2 | Cohort 1 | Cohort 2 |
| Median | 0.596 | 0.213 | nd | nd | nd | nd |
| Average | 0.643 | 0.243 | nd | nd | nd | nd |
| Stdev | 0.503 | 0.174 | nd | nd | nd | nd |
| D(t-test) |  | 0.035 | nd | nd | nd | nd |
| Min | 0.0873 | 0.0786 | nd | nd | nd | nd |
| Max | 2.49 | 0.651 | nd | nd | nd | nd |
| UO only | 0hr prior to AKI stage | | 24hr prior to AKI stage | | 48hr prior to AKI stage | |
|  | Cohort 1 | Cohort 2 | Cohort 1 | Cohort 2 | Cohort 1 | Cohort 2 |
| n (Samp) | 27 | 8 | nd | nd | nd | nd |
| n (Patient) | 17 | 8 | nd | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.21 | nd | 0.19 | nd | nd | nd | nd | nd | nd |
| SE | 0.12 | nd | 0.098 | nd | nd | nd | nd | nd | nd |
| D | 0.012 | nd | 0.0013 | nd | nd | nd | nd | nd | nd |
| nCohort 1 | 32 | nd | 27 | nd | nd | nd | nd | nd | nd |
| nCohort 2 | 6 | nd | 8 | nd | nd | nd | nd | nd | nd |
| Cutoff 1 | 0.114 | nd | 0.181 | nd | nd | nd | nd | nd | nd |
| Sens 1 | 83% | nd | 75% | nd | nd | nd | nd | nd | nd |
| Spec 1 | 6% | nd | 15% | nd | nd | nd | nd | nd | nd |
| Cutoff 2 | 0.114 | nd | 0.114 | nd | nd | nd | nd | nd | nd |
| Sens 2 | 83% | nd | 88% | nd | nd | nd | nd | nd | nd |
| Spec 2 | 6% | nd | 7% | nd | nd | nd | nd | nd | nd |
| Cutoff 3 | 0 | nd | 0 | nd | nd | nd | nd | nd | nd |
| Sens 3 | 100% | nd | 100% | nd | nd | nd | nd | nd | nd |
| Spec 3 | 0% | nd | 0% | nd | nd | nd | nd | nd | nd |
| Cutoff 4 | 0.703 | nd | 0.771 | nd | nd | nd | nd | nd | nd |
| Sens 4 | 0% | nd | 0% | nd | nd | nd | nd | nd | nd |
| Spec 4 | 72% | nd | 70% | nd | nd | nd | nd | nd | nd |
| Cutoff 5 | 0.991 | nd | 1.01 | nd | nd | nd | nd | nd | nd |
| Sens 5 | 0% | nd | 0% | nd | nd | nd | nd | nd | nd |
| Spec 5 | 81% | nd | 81% | nd | nd | nd | nd | nd | nd |
| Cutoff 6 | 1.05 | nd | 1.13 | nd | nd | nd | nd | nd | nd |
| Sens 6 | 0% | nd | 0% | nd | nd | nd | nd | nd | nd |
| Spec 6 | 91% | nd | 93% | nd | nd | nd | nd | nd | nd |
| OR Quart 2 | 2>1.2 | nd | >1.1 | nd | nd | nd | nd | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p Value | <0.88 | nd | <0.94 | nd | nd | nd | nd | nd | nd |
| 95% CI of | >0.067 | nd | >0.060 | nd | nd | nd | nd | nd | nd |
| OR Quart2 | na | nd | na | nd | nd | nd | nd | nd | nd |
| OR Quart 3 | >2.5 | nd | >4.5 | nd | nd | nd | nd | nd | nd |
| p Value | <0.49 | nd | <0.24 | nd | nd | nd | nd | nd | nd |
| 95% CI of | >0.19 | nd | >0.37 | nd | nd | nd | nd | nd | nd |
| OR Quart3 | na | nd | na | nd | nd | nd | nd | nd | nd |
| OR Quart 4 | 4>5.0 | nd | >9.0 | nd | nd | nd | nd | nd | nd |
| p Value | <0.20 | nd | <0.083 | nd | nd | nd | nd | nd | nd |
| 95% CI of | >0.42 | nd | >0.75 | nd | nd | nd | nd | nd | nd |
| OR Quart4 | na | nd | na | nd | nd | nd | nd | nd | nd |

**Caspase-9**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 25.5 | 14.8 | nd | nd | nd | nd |
| Average | 44.5 | 24.6 | nd | nd | nd | nd |
| Stdev | 60.6 | 24.9 | nd | nd | nd | nd |
| D(t-test) | | 0.27 | nd | nd | nd | nd |
| Min | 0.400 | 4.59 | nd | nd | nd | nd |
| Max | 366 | 78.6 | nd | nd | nd | nd |
| n (Samp) | 57 | 12 | nd | nd | nd | nd |
| n (Patient) | 37 | 12 | nd | nd | nd | nd |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 22.8 | 13.2 | nd | nd | nd | nd |
| Average | 42.5 | 12.5 | nd | nd | nd | nd |
| Stdev | 5 8.2 | 7.24 | nd | nd | nd | nd |
| D(t-test) | | 0.21 | nd | nd | nd | nd |
| Min | 0.400 | 4.32 | nd | nd | nd | nd |
| Max | 366 | 21.7 | nd | nd | nd | nd |
| n (Samp) | 89 | 6 | nd | nd | nd | nd |
| n (Patient) | 61 | 6 | nd | nd | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 30.1 | 9.06 | nd | nd | nd | nd |
| Average | 50.3 | 20.6 | nd | nd | nd | nd |
| Stdev | 67.6 | 24.4 | nd | nd | nd | nd |
| D(t-test) | | 0.12 | nd | nd | nd | nd |
| Min | 6.05 | 3.79 | nd | nd | nd | nd |
| Max | 366 | 78.6 | nd | nd | nd | nd |
| n (Samp) | 42 | 14 | nd | nd | nd | nd |
| n (Patient) | 27 | 14 | nd | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.39 | 0.28 | 0.26 | nd | nd | nd | nd | nd | nd |
| SE | 0.094 | 0.12 | 0.083 | nd | nd | nd | nd | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| D | 0.25 | 0.077 | 0.0038 | nd | nd | nd | nd | nd | nd |
| nCohort 1 | 57 | 89 | 42 | nd | nd | nd | nd | nd | nd |
| nCohort 2 | 12 | 6 | 14 | nd | nd | nd | nd | nd | nd |
| Cutoff 1 | 8.91 | 3.65 | 6.05 | nd | nd | nd | nd | nd | nd |
| Sens 1 | 75% | 100% | 71% | nd | nd | nd | nd | nd | nd |
| Spec 1 | 21% | 7% | 2% | nd | nd | nd | nd | nd | nd |
| Cutoff 2 | 8.22 | 3.65 | 4.59 | nd | nd | nd | nd | nd | nd |
| Sens 2 | 83% | 100% | 86% | nd | nd | nd | nd | nd | nd |
| Spec 2 | 19% | 7% | 0% | nd | nd | nd | nd | nd | nd |
| Cutoff 3 | 4.59 | 3.65 | 3.79 | nd | nd | nd | nd | nd | nd |
| Sens 3 | 92% | 100% | 93% | nd | nd | nd | nd | nd | nd |
| Spec 3 | 9% | 7% | 0% | nd | nd | nd | nd | nd | nd |
| Cutoff 4 | 46.1 | 45.3 | 49.3 | nd | nd | nd | nd | nd | nd |
| Sens 4 | 17% | 0% | 14% | nd | nd | nd | nd | nd | nd |
| Spec 4 | 70% | 71% | 71% | nd | nd | nd | nd | nd | nd |
| Cutoff 5 | 54.5 | 57.5 | 54.5 | nd | nd | nd | nd | nd | nd |
| Sens 5 | 17% | 0% | 14% | nd | nd | nd | nd | nd | nd |
| Spec 5 | 81% | 81% | 81% | nd | nd | nd | nd | nd | nd |
| Cutoff 6 | 90.6 | 94.1 | 106 | nd | nd | nd | nd | nd | nd |
| Sens 6 | 0% | 0% | 0% | nd | nd | nd | nd | nd | nd |
| Spec 6 | 91% | 91% | 90% | nd | nd | nd | nd | nd | nd |
| OR Quart 2 | 1.1 | >1.0 | 1.0 | nd | nd | nd | nd | nd | nd |
| p Value | 0.95 | <0.98 | 1.0 | nd | nd | nd | nd | nd | nd |
| 95% CI of | 0.13 | >0.062 | 0.12 | nd | nd | nd | nd | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart2 | 8.6 | na | 8.3 | nd | nd | nd | nd | nd | nd |
| OR Quart 3 | 1.7 | >3.4 | 1.0 | nd | nd | nd | nd | nd | nd |
| p Value | 0.58 | <0.30 | 1.0 | nd | nd | nd | nd | nd | nd |
| 95% CI of | 0.25 | >0.33 | 0.12 | nd | nd | nd | nd | nd | nd |
| OR Quart3 | 12 | na | 8.3 | nd | nd | nd | nd | nd | nd |
| OR Quart 4 | 43.3 | >2.3 | 8.0 | nd | nd | nd | nd | nd | nd |
| p Value | 0.19 | <0.51 | 0.026 | nd | nd | nd | nd | nd | nd |
| 95% CI of | 0.55 | >0.19 | 1.3 | nd | nd | nd | nd | nd | nd |
| OR Quart4 | 20 | na | 50 | nd | nd | nd | nd | nd | nd |

**Cadherin-1**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 72700 | 88200 | 72700 | 92800 | 72700 | 98500 |
| Average | 102000 | 129000 | 102000 | 127000 | 102000 | 134000 |
| Stdev | 106000 | 88400 | 106000 | 90200 | 106000 | 91700 |
| D(t-test) | | 0.26 | | 0.28 | | 0.28 |
| Min | 14500 | 34900 | 14500 | 3320 | 14500 | 25800 |
| Max | 621000 | 334000 | 621000 | 340000 | 621000 | 283000 |
| n (Samp) | 52 | 28 | 52 | 31 | 52 | 16 |
| n (Patient) | 50 | 28 | 50 | 31 | 50 | 16 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 86800 | 130000 | 86800 | 73000 | 86800 | 109000 |
| Average | 116000 | 145000 | 116000 | 120000 | 116000 | 116000 |
| Stdev | 94600 | 90700 | 94600 | 94900 | 94600 | 73700 |
| D(t-test) | | 0.35 | | 0.87 | | 0.99 |
| Min | 3320 | 34900 | 3320 | 30700 | 3320 | 56100 |
| Max | 621000 | 334000 | 621000 | 340000 | 621000 | 283000 |
| n (Samp) | 123 | 10 | 123 | 16 | 123 | 8 |
| n (Patient) | 96 | 10 | 96 | 16 | 96 | 8 |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 81200 | 88200 | 81200 | 103000 | 81200 | 89400 |
| Average | 119000 | 122000 | 119000 | 122000 | 119000 | 139000 |
| Stdev | 114000 | 85000 | 114000 | 78600 | 114000 | 94300 |
| D(t-test) | | 0.89 | | 0.89 | | 0.59 |
| Min | 39700 | 38100 | 39700 | 3320 | 39700 | 25800 |
| Max | 621000 | 314000 | 621000 | 300000 | 621000 | 277000 |
| n (Samp) | 51 | 22 | 51 | 27 | 51 | 11 |
| n (Patient) | 44 | 22 | 44 | 27 | 44 | 11 |
| | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.62 | 0.63 | 0.54 | 0.59 | 0.47 | 0.57 | 0.62 | 0.56 | 0.59 |
| SE | 0.067 | 0.098 | 0.075 | 0.065 | 0.078 | 0.069 | 0.083 | 0.11 | 0.098 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| D | 0.066 | 0.18 | 0.57 | 0.15 | 0.72 | 0.32 | 0.13 | 0.57 | 0.38 |
| nCohort 1 | 52 | 123 | 51 | 52 | 123 | 51 | 52 | 123 | 51 |
| nCohort 2 | 28 | 10 | 22 | 31 | 16 | 27 | 16 | 8 | 11 |
| Cutoff 1 | 78500 | 79100 | 75400 | 62800 | 40700 | 67700 | 61700 | 61700 | 79100 |
| Sens 1 | 71% | 70% | 73% | 71% | 75% | 70% | 75% | 75% | 73% |
| Spec 1 | 56% | 46% | 41% | 42% | 9% | 37% | 40% | 31% | 49% |
| Cutoff 2 | 52700 | 78500 | 52700 | 47600 | 39700 | 54300 | 57000 | 57000 | 70100 |
| Sens 2 | 82% | 80% | 82% | 81% | 81% | 81% | 81% | 88% | 82% |
| Spec 2 | 21% | 46% | 12% | 17% | 8% | 14% | 29% | 26% | 39% |
| Cutoff 3 | 40700 | 72100 | 47600 | 39700 | 31800 | 39700 | 48100 | 54700 | 47600 |
| Sens 3 | 93% | 90% | 91% | 90% | 94% | 93% | 94% | 100% | 91% |
| Spec 3 | 10% | 41% | 10% | 8% | 6% | 2% | 19% | 23% | 10% |
| Cutoff 4 | 97000 | 131000 | 100000 | 97000 | 131000 | 100000 | 97000 | 131000 | 100000 |
| Sens 4 | 46% | 50% | 32% | 48% | 38% | 52% | 50% | 25% | 45% |
| Spec 4 | 71% | 71% | 71% | 71% | 71% | 71% | 71% | 71% | 71% |
| Cutoff 5 | 114000 | 165000 | 138000 | 114000 | 165000 | 138000 | 114000 | 165000 | 138000 |
| Sens 5 | 36% | 40% | 27% | 42% | 38% | 33% | 38% | 12% | 36% |
| Spec 5 | 81% | 80% | 80% | 81% | 80% | 80% | 81% | 80% | 80% |
| Cutoff 6 | 153000 | 243000 | 242000 | 153000 | 243000 | 242000 | 153000 | 243000 | 242000 |
| Sens 6 | 32% | 20% | 18% | 39% | 6% | 11% | 31% | 12% | 27% |
| Spec 6 | 90% | 90% | 90% | 90% | 90% | 90% | 90% | 90% | 90% |
| OR Quart 2 | 0.78 | 3.2 | 0.74 | 0.47 | 0.14 | 0.54 | 1.0 | 2.0 | 0.93 |
| p Value | 0.72 | 0.33 | 0.70 | 0.27 | 0.079 | 0.41 | 1.0 | 0.58 | 0.94 |
| 95% CI of | 0.19 | 0.32 | 0.16 | 0.12 | 0.016 | 0.13 | 0.17 | 0.17 | 0.11 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart2 | 3.1 | 32 | 3.4 | 1.8 | 1.3 | 2.3 | 5.8 | 23 | 7.6 |
| OR Quart 3 | 1.3 | 2.1 | 1.7 | 0.60 | 0.62 | 1.6 | 1.4 | 4.3 | 1.6 |
| p Value | 0.74 | 0.56 | 0.48 | 0.44 | 0.50 | 0.50 | 0.67 | 0.21 | 0.63 |
| 95% CI of | 0.33 | 0.18 | 0.41 | 0.16 | 0.16 | 0.42 | 0.27 | 0.45 | 0.23 |
| OR Quart3 | 4.7 | 24 | 6.7 | 2.2 | 2.4 | 5.9 | 7.7 | 41 | 11 |
| OR Quart 4 | 42.3 | 4.3 | 1.2 | 2.0 | 0.83 | 1.8 | 2.5 | 0.97 | 2.2 |
| p Value | 0.20 | 0.21 | 0.80 | 0.27 | 0.78 | 0.39 | 0.25 | 0.98 | 0.42 |
| 95% CI of | 0.64 | 0.45 | 0.29 | 0.58 | 0.23 | 0.48 | 0.52 | 0.058 | 0.33 |
| OR Quart4 | 8.5 | 40 | 4.9 | 6.9 | 3.0 | 6.6 | 13 | 16 | 14 |

**Cadherin-5**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 20.4 | 21.2 | nd | nd | nd | nd |
| Average | 20.5 | 23.9 | nd | nd | nd | nd |
| Stdev | 5.01 | 5.05 | nd | nd | nd | nd |
| D(t-test) | | 0.065 | nd | nd | nd | nd |
| Min | 12.4 | 18.0 | nd | nd | nd | nd |
| Max | 35.9 | 32.1 | nd | nd | nd | nd |
| n (Samp) | 48 | 9 | nd | nd | nd | nd |
| n (Patient) | 30 | 9 | nd | nd | nd | nd |

| UO only | 0hr prior to AKI stage | | 24hr prior to AKI stage | | 48hr prior to AKI stage | |
| --- | --- | --- | --- | --- | --- | --- |
| | Cohort 1 | Cohort 2 | Cohort 1 | Cohort 2 | Cohort 1 | Cohort 2 |
| Median | 20.5 | 21.2 | nd | nd | nd | nd |
| Average | 20.7 | 23.3 | nd | nd | nd | nd |
| Stdev | 4.09 | 5.02 | nd | nd | nd | nd |
| D(t-test) | | 0.080 | nd | nd | nd | nd |
| Min | 12.4 | 17.0 | nd | nd | nd | nd |
| Max | 29.3 | 32.1 | nd | nd | nd | nd |
| n (Samp) | 34 | 11 | nd | nd | nd | nd |
| n (Patient) | 20 | 11 | nd | nd | nd | nd |

| | 0hr prior to AKI stage | | | 24hr prior to AKI stage | | | 48hr prior to AKI stage | | |
| --- | --- | --- | --- | --- | --- | --- | --- | --- | --- |
| | sCr or UO | sCr only | UO only | sCr or UO | sCr only | UO only | sCr or UO | sCr only | UO only |
| AUC | 0.69 | nd | 0.64 | nd | nd | nd | nd | nd | nd |
| SE | 0.10 | nd | 0.10 | nd | nd | nd | nd | nd | nd |
| D | 0.077 | nd | 0.16 | nd | nd | nd | nd | nd | nd |
| nCohort 1 | 48 | nd | 34 | nd | nd | nd | nd | nd | nd |
| nCohort 2 | 9 | nd | 11 | nd | nd | nd | nd | nd | nd |
| Cutoff 1 | 20.6 | nd | 19.5 | nd | nd | nd | nd | nd | nd |
| Sens 1 | 78% | nd | 73% | nd | nd | nd | nd | nd | nd |
| Spec 1 | 54% | nd | 44% | nd | nd | nd | nd | nd | nd |
| Cutoff 2 | 18.9 | nd | 18.9 | nd | nd | nd | nd | nd | nd |
| Sens 2 | 89% | nd | 82% | nd | nd | nd | nd | nd | nd |
| Spec 2 | 42% | nd | 38% | nd | nd | nd | nd | nd | nd |
| Cutoff 3 | 17.1 | nd | 17.1 | nd | nd | nd | nd | nd | nd |
| Sens 3 | 100% | nd | 91% | nd | nd | nd | nd | nd | nd |

EP 2 894 473 B1

169

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 3 | 33% | nd | 26% | nd | nd | nd | nd | nd | nd |
| Cutoff 4 | 22.6 | nd | 22.7 | nd | nd | nd | nd | nd | nd |
| Sens 4 | 44% | nd | 45% | nd | nd | nd | nd | nd | nd |
| Spec 4 | 71% | nd | 71% | nd | nd | nd | nd | nd | nd |
| Cutoff 5 | 24.0 | nd | 24.0 | nd | nd | nd | nd | nd | nd |
| Sens 5 | 44% | nd | 45% | nd | nd | nd | nd | nd | nd |
| Spec 5 | 81% | nd | 82% | nd | nd | nd | nd | nd | nd |
| Cutoff 6 | 26.5 | nd | 26.2 | nd | nd | nd | nd | nd | nd |
| Sens 6 | 33% | nd | 27% | nd | nd | nd | nd | nd | nd |
| Spec 6 | 92% | nd | 91% | nd | nd | nd | nd | nd | nd |
| OR Quart 2 | 2>2.3 | nd | 3.8 | nd | nd | nd | nd | nd | nd |
| p Value | <0.51 | nd | 0.29 | nd | nd | nd | nd | nd | nd |
| 95% CI of | >0.19 | nd | 0.32 | nd | nd | nd | nd | nd | nd |
| OR Quart2 | na | nd | 43 | nd | nd | nd | nd | nd | nd |
| OR Quart 3 | >3.8 | nd | 2.2 | nd | nd | nd | nd | nd | nd |
| p Value | <0.27 | nd | 0.54 | nd | nd | nd | nd | nd | nd |
| 95% CI of | >0.35 | nd | 0.17 | nd | nd | nd | nd | nd | nd |
| OR Quart3 | na | nd | 29 | nd | nd | nd | nd | nd | nd |
| OR Quart 4 | 4>5.1 | nd | 7.1 | nd | nd | nd | nd | nd | nd |
| p Value | <0.17 | nd | 0.10 | nd | nd | nd | nd | nd | nd |
| 95% CI of | >0.50 | nd | 0.68 | nd | nd | nd | nd | nd | nd |
| OR Quart4 | na | nd | 75 | nd | nd | nd | nd | nd | nd |

EP 2 894 473 B1

(continued)

| Cyclin-dependent kinase inhibitor 1 | | | | | | | |
|---|---|---|---|---|---|---|---|
| sCr or UO | 0hr prior to AKI stage | | 24hr prior to AKI stage | | | 48hr prior to AKI stage | |
| | Cohort 1 | Cohort 2 | Cohort 1 | | Cohort 2 | Cohort 1 | Cohort 2 |
| Median | 208 | 138 | nd | | nd | nd | nd |
| Average | 1230 | 1120 | nd | | nd | nd | nd |
| Stdev | 2090 | 1790 | nd | | nd | nd | nd |
| D(t-test) | | 0.87 | nd | | nd | nd | nd |
| Min | 0.116 | 28.5 | nd | | nd | nd | nd |
| Max | 6840 | 5430 | nd | | nd | nd | nd |
| n (Samp) | 56 | 12 | nd | | nd | nd | nd |
| n (Patient) | 37 | 12 | nd | | nd | nd | nd |
| | | | | | | | |
| sCr only | 0hr prior to AKI stage | | 24hr prior to AKI stage | | | 48hr prior to AKI stage | |
| | Cohort 1 | Cohort 2 | Cohort 1 | Cohort 2 | | Cohort 1 | Cohort 2 |
| Median | 197 | 171 | nd | nd | | nd | nd |
| Average | 1140 | 463 | nd | nd | | nd | nd |
| Stdev | 1940 | 716 | nd | nd | | nd | nd |
| D(t-test) | | 0.40 | nd | nd | | nd | nd |
| Min | 0.116 | 73.9 | nd | nd | | nd | nd |
| Max | 6840 | 1910 | nd | nd | | nd | nd |
| n (Samp) | 88 | 6 | nd | nd | | nd | nd |
| n (Patient) | 61 | 6 | nd | nd | | nd | nd |
| | | | | | | | |
| UO only | 0hr prior to AKI stage | | 24hr prior to AKI stage | | | 48hr prior to AKI stage | |
| | Cohort 1 | Cohort 2 | Cohort 1 | Cohort 2 | | Cohort 1 | Cohort 2 |
| Median | 381 | 151 | nd | nd | | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Average | 1460 | 1310 | nd | nd | nd | nd |
| Stdev | 2310 | 2210 | nd | nd | nd | nd |
| p(t-test) | | 0.84 | nd | nd | nd | nd |
| Min | 0.116 | 28.5 | nd | nd | nd | nd |
| Max | 6840 | 6400 | nd | nd | nd | nd |
| n (Samp) | 41 | 14 | nd | nd | nd | nd |
| n (Patient) | 27 | 14 | nd | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.49 | 0.46 | 0.44 | nd | nd | nd | nd | nd | nd |
| SE | 0.093 | 0.12 | 0.091 | nd | nd | nd | nd | nd | nd |
| p | 0.91 | 0.78 | 0.52 | nd | nd | nd | nd | nd | nd |
| nCohort 1 | 56 | 88 | 41 | nd | nd | nd | nd | nd | nd |
| nCohort 2 | 12 | 6 | 14 | nd | nd | nd | nd | nd | nd |
| Cutoff 1 | 116 | 123 | 108 | nd | nd | nd | nd | nd | nd |
| Sens 1 | 75% | 83% | 71% | nd | nd | nd | nd | nd | nd |
| Spec 1 | 32% | 33% | 27% | nd | nd | nd | nd | nd | nd |
| Cutoff 2 | 96.8 | 123 | 73.1 | nd | nd | nd | nd | nd | nd |
| Sens 2 | 83% | 83% | 86% | nd | nd | nd | nd | nd | nd |
| Spec 2 | 30% | 33% | 17% | nd | nd | nd | nd | nd | nd |
| Cutoff 3 | 47.1 | 73.1 | 42.3 | nd | nd | nd | nd | nd | nd |
| Sens 3 | 92% | 100% | 93% | nd | nd | nd | nd | nd | nd |
| Spec 3 | 12% | 17% | 12% | nd | nd | nd | nd | nd | nd |

EP 2 894 473 B1

172

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 4 | 715 | 715 | 902 | nd | nd | nd | nd | nd | nd |
| Sens 4 | 33% | 17% | 21% | nd | nd | nd | nd | nd | nd |
| Spec 4 | 71% | 70% | 71% | nd | nd | nd | nd | nd | nd |
| Cutoff 5 | 1430 | 1430 | 1760 | nd | nd | nd | nd | nd | nd |
| Sens 5 | 25% | 17% | 21% | nd | nd | nd | nd | nd | nd |
| Spec 5 | 80% | 82% | 80% | nd | nd | nd | nd | nd | nd |
| Cutoff 6 | 5560 | 5160 | 6560 | nd | nd | nd | nd | nd | nd |
| Sens 6 | 0% | 0% | 0% | nd | nd | nd | nd | nd | nd |
| Spec 6 | 91% | 91% | 90% | nd | nd | nd | nd | nd | nd |
| OR Quart 2 | 0.62 | 2.2 | 1.0 | nd | nd | nd | nd | nd | nd |
| p Value | 0.63 | 0.53 | 1.0 | nd | nd | nd | nd | nd | nd |
| 95% CI of | 0.090 | 0.18 | 0.16 | nd | nd | nd | nd | nd | nd |
| OR Quart2 | 4.3 | 26 | 6.1 | nd | nd | nd | nd | nd | nd |
| OR Quart 3 | 1.9 | 2.1 | 2.0 | nd | nd | nd | nd | nd | nd |
| p Value | 0.42 | 0.56 | 0.41 | nd | nd | nd | nd | nd | nd |
| 95% CI of | 0.38 | 0.18 | 0.38 | nd | nd | nd | nd | nd | nd |
| OR Quart3 | 9.9 | 25 | 11 | nd | nd | nd | nd | nd | nd |
| OR Quart 4 | 40.62 | 1.0 | 1.1 | nd | nd | nd | nd | nd | nd |
| p Value | 0.63 | 0.98 | 0.92 | nd | nd | nd | nd | nd | nd |
| 95% CI of | 0.090 | 0.062 | 0.18 | nd | nd | nd | nd | nd | nd |
| OR Quart4 | 4.3 | 18 | 6.8 | nd | nd | nd | nd | nd | nd |

(continued)

**Carcinoembryonic antigen-related cell adhesion molecule 5**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.66 | 2.13 | 1.66 | 2.36 | 1.66 | 2.26 |
| Average | 2.32 | 3.66 | 2.32 | 3.88 | 2.32 | 3.00 |
| Stdev | 2.32 | 4.90 | 2.32 | 5.35 | 2.32 | 2.72 |
| D(t-test) | | 0.0028 | | 6.3E-4 | | 0.17 |
| Min | 0.183 | 0.453 | 0.183 | 0.562 | 0.183 | 0.363 |
| Max | 20.8 | 30.1 | 20.8 | 33.6 | 20.8 | 12.5 |
| n (Samp) | 260 | 51 | 260 | 56 | 260 | 25 |
| n (Patient) | 110 | 51 | 110 | 56 | 110 | 25 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.77 | 1.68 | 1.77 | 2.00 | 1.77 | 1.91 |
| Average | 2.76 | 3.12 | 2.76 | 3.22 | 2.76 | 1.68 |
| Stdev | 3.35 | 4.09 | 3.35 | 3.30 | 3.35 | 0.833 |
| D(t-test) | | 0.66 | | 0.54 | | 0.25 |
| Min | 0.183 | 0.245 | 0.183 | 0.355 | 0.183 | 0.363 |
| Max | 33.6 | 17.1 | 33.6 | 14.5 | 33.6 | 2.69 |
| n (Samp) | 466 | 18 | 466 | 21 | 466 | 13 |
| n (Patient) | 180 | 18 | 180 | 21 | 180 | 13 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.62 | 2.22 | 1.62 | 2.36 | 1.62 | 2.26 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Average | 2.47 | 3.81 | 2.47 | 3.82 | 2.47 | 3.39 |
| Stdev | 2.58 | 4.85 | 2.58 | 5.27 | 2.58 | 2.98 |
| D(t-test) | | 0.0067 | | 0.0083 | | 0.11 |
| Min | 0.183 | 0.622 | 0.183 | 0.562 | 0.183 | 0.591 |
| Max | 20.8 | 30.1 | 20.8 | 33.6 | 20.8 | 12.5 |
| n (Samp) | 213 | 51 | 213 | 53 | 213 | 23 |
| n (Patient) | 89 | 51 | 89 | 53 | 89 | 23 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.60 | 0.48 | 0.63 | 0.63 | 0.54 | 0.62 | 0.60 | 0.43 | 0.62 |
| SE | 0.045 | 0.070 | 0.046 | 0.043 | 0.066 | 0.045 | 0.062 | 0.084 | 0.065 |
| p | 0.026 | 0.73 | 0.0052 | 0.0029 | 0.51 | 0.0056 | 0.12 | 0.44 | 0.073 |
| nCohort 1 | 260 | 466 | 213 | 260 | 466 | 213 | 260 | 466 | 213 |
| nCohort 2 | 51 | 18 | 51 | 56 | 21 | 53 | 25 | 13 | 23 |
| Cutoff 1 | 1.35 | 1.17 | 1.52 | 1.70 | 1.22 | 1.77 | 1.43 | 1.05 | 1.57 |
| Sens 1 | 71% | 72% | 71% | 71% | 71% | 72% | 72% | 77% | 74% |
| Spec 1 | 40% | 31% | 47% | 53% | 32% | 58% | 43% | 26% | 49% |
| Cutoff 2 | 1.10 | 0.591 | 1.11 | 1.02 | 1.01 | 1.02 | 0.855 | 0.971 | 0.855 |
| Sens 2 | 80% | 83% | 80% | 80% | 81% | 81% | 80% | 85% | 83% |
| Spec 2 | 32% | 8% | 32% | 30% | 25% | 30% | 23% | 23% | 22% |
| Cutoff 3 | 0.893 | 0.448 | 0.919 | 0.787 | 0.731 | 0.787 | 0.679 | 0.417 | 0.699 |
| Sens 3 | 90% | 94% | 90% | 91% | 90% | 91% | 92% | 92% | 91% |
| Spec 3 | 25% | 4% | 23% | 20% | 13% | 18% | 16% | 3% | 15% |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 4 | 2.42 | 2.71 | 2.52 | 2.42 | 2.71 | 2.52 | 2.42 | 2.71 | 2.52 |
| Sens 4 | 39% | 28% | 45% | 45% | 43% | 42% | 48% | 0% | 48% |
| Spec 4 | 70% | 70% | 71% | 70% | 70% | 71% | 70% | 70% | 71% |
| Cutoff 5 | 3.34 | 3.88 | 3.74 | 3.34 | 3.88 | 3.74 | 3.34 | 3.88 | 3.74 |
| Sens 5 | 31% | 22% | 31% | 32% | 29% | 28% | 32% | 0% | 30% |
| Spec 5 | 80% | 80% | 80% | 80% | 80% | 80% | 80% | 80% | 80% |
| Cutoff 6 | 4.93 | 5.28 | 5.88 | 4.93 | 5.28 | 5.88 | 4.93 | 5.28 | 5.88 |
| Sens 6 | 20% | 17% | 12% | 18% | 14% | 11% | 12% | 0% | 17% |
| Spec 6 | 90% | 90% | 90% | 90% | 90% | 90% | 90% | 90% | 90% |
| OR Quart | 2 2.0 | 0.59 | 1.1 | 0.67 | 0.99 | 0.45 | 0.48 | >7.4 | 0.79 |
| p Value | 0.17 | 0.48 | 0.80 | 0.44 | 0.99 | 0.17 | 0.31 | <0.063 3 | 0.73 |
| 95% CI of | 0.75 | 0.14 | 0.41 | 0.24 | 0.28 | 0.15 | 0.11 | >0.90 | 0.20 |
| OR Quart2 | 5.3 | 2.5 | 3.2 | 1.9 | 3.5 | 1.4 | 2.0 | na | 3.1 |
| OR Quart | 32.2 | 1.0 | 2.5 | 2.3 | 0.58 | 2.4 | 1.2 | >3.1 | 1.0 |
| p Value | 0.11 | 1.0 | 0.050 | 0.046 | 0.47 | 0.041 | 0.77 | <0.33 | 1.0 |
| 95% CI of | 0.83 | 0.28 | 1.0 | 1.0 | 0.14 | 1.0 | 0.38 | >0.32 | 0.27 |
| OR Quart3 | 5.8 | 3.5 | 6.3 | 5.4 | 2.5 | 5.7 | 3.7 | na | 3.7 |
| OR Quart | 42.8 | 1.0 | 2.5 | 2.2 | 1.6 | 2.1 | 1.5 | >3.1 | 1.9 |
| p Value | 0.033 | 1.0 | 0.050 | 0.068 | 0.40 | 0.10 | 0.43 | <0.33 | 0.26 |
| 95% CI of | 1.1 | 0.28 | 1.0 | 0.94 | 0.52 | 0.87 | 0.52 | >0.32 | 0.61 |
| OR Quart4 | 7.2 | 3.5 | 6.3 | 5.1 | 5.1 | 4.9 | 4.6 | na | 6.2 |

EP 2 894 473 B1

176

| Myoglobin | | | | | | |
|---|---|---|---|---|---|---|
| sCr or UO | AKI 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 26.8 | 36.2 | 26.8 | 41.7 | 26.8 | 54.3 |
| Average | 66.0 | 106 | 66.0 | 168 | 66.0 | 113 |
| Stdev | 145 | 166 | 145 | 301 | 145 | 194 |
| p(t-test) | | 0.078 | | 1.8E-4 | | 0.13 |
| Min | 4.60 | 6.86 | 4.60 | 4.40 | 4.60 | 7.57 |
| Max | 1720 | 737 | 1720 | 1470 | 1720 | 988 |
| n(Samp) | 260 | 51 | 260 | 56 | 260 | 25 |
| n (Patient) | 110 | 51 | 110 | 56 | 110 | 25 |
| | | | | | | |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 33.0 | 62.6 | 33.0 | 64.7 | 33.0 | 69.7 |
| Average | 83.7 | 266 | 83.7 | 272 | 83.7 | 94.9 |
| Stdev | 191 | 465 | 191 | 433 | 191 | 93.0 |
| p(t-test) | | 2.8E-4 | | 5.2E-5 | | 0.83 |
| Min | 3.68 | 8.01 | 3.68 | 4.40 | 3.68 | 7.57 |
| Max | 2130 | 1880 | 2130 | 1470 | 2130 | 308 |
| n(Samp) | 466 | 18 | 466 | 21 | 466 | 13 |
| n (Patient) | 180 | 18 | 180 | 21 | 180 | 13 |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 27.4 | 37.3 | 27.4 | 41.0 | 27.4 | 62.1 |

(continued)

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Average | 72.4 | 102 | 72.4 | 147 | 72.4 | 165 |
| Stdev | 153 | 158 | 153 | 260 | 153 | 320 |
| p(t-test) | | 0.22 | | 0.0071 | | 0.017 |
| Min | 4.60 | 6.86 | 4.60 | 8.81 | 4.60 | 8.40 |
| Max | 1720 | 737 | 1720 | 1410 | 1720 | 1310 |
| n(Samp) | 213 | 51 | 213 | 53 | 213 | 23 |
| n (Patient) | 89 | 51 | 89 | 53 | 89 | 23 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.60 | 0.65 | 0.59 | 0.65 | 0.61 | 0.64 | 0.63 | 0.62 | 0.63 |
| SE | 0.045 | 0.072 | 0.046 | 0.043 | 0.067 | 0.045 | 0.062 | 0.084 | 0.065 |
| p | 0.034 | 0.041 | 0.039 | 7.0E-4 | 0.086 | 0.0019 | 0.036 | 0.15 | 0.051 |
| nCohort 1 | 260 | 466 | 213 | 260 | 466 | 213 | 260 | 466 | 213 |
| nCohort 2 | 51 | 18 | 51 | 56 | 21 | 53 | 25 | 13 | 23 |
| Cutoff 1 | 21.3 | 26.6 | 20.9 | 27.5 | 24.5 | 27.5 | 20.6 | 25.6 | 20.4 |
| Sens 1 | 71% | 72% | 71% | 71% | 71% | 72% | 72% | 77% | 74% |
| Spec 1 | 40% | 43% | 40% | 52% | 38% | 50% | 39% | 41% | 40% |
| Cutoff 2 | 18.6 | 25.4 | 18.6 | 18.6 | 19.2 | 18.9 | 17.4 | 17.4 | 16.3 |
| Sens 2 | 80% | 83% | 80% | 80% | 81% | 81% | 80% | 85% | 83% |
| Spec 2 | 33% | 41% | 34% | 33% | 31% | 35% | 30% | 25% | 29% |
| Cutoff 3 | 12.5 | 8.56 | 14.8 | 12.3 | 12.9 | 13.2 | 9.64 | 8.39 | 10.6 |
| Sens 3 | 90% | 94% | 90% | 91% | 90% | 91% | 92% | 92% | 91% |
| Spec 3 | 18% | 8% | 26% | 18% | 17% | 21% | 13% | 8% | 15% |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 4 | 50.2 | 60.4 | 56.0 | 50.2 | 60.4 | 56.0 | 50.2 | 60.4 | 56.0 |
| Sens 4 | 43% | 50% | 41% | 46% | 52% | 45% | 52% | 54% | 52% |
| Spec 4 | 70% | 70% | 70% | 70% | 70% | 70% | 70% | 70% | 70% |
| Cutoff 5 | 74.3 | 93.4 | 84.8 | 74.3 | 93.4 | 84.8 | 74.3 | 93.4 | 84.8 |
| Sens 5 | 35% | 39% | 35% | 43% | 38% | 42% | 44% | 31% | 43% |
| Spec 5 | 80% | 80% | 80% | 80% | 80% | 80% | 80% | 80% | 80% |
| Cutoff 6 | 138 | 171 | 174 | 138 | 171 | 174 | 138 | 171 | 174 |
| Sens 6 | 22% | 33% | 16% | 29% | 29% | 26% | 20% | 15% | 13% |
| Spec 6 | 90% | 90% | 90% | 90% | 90% | 90% | 90% | 90% | 90% |
| OR Quart 2 | 1.6 | 2.6 | 2.9 | 0.88 | 0.99 | 2.0 | 1.0 | 0.99 | 0.74 |
| p Value | 0.36 | 0.27 | 0.038 | 0.80 | 0.99 | 0.16 | 1.0 | 0.99 | 0.70 |
| 95% CI of | 0.60 | 0.49 | 1.1 | 0.32 | 0.24 | 0.75 | 0.24 | 0.14 | 0.16 |
| OR Quart2 | 4.1 | 13 | 8.1 | 2.4 | 4.1 | 5.5 | 4.2 | 7.2 | 3.4 |
| OR Quart 3 | 1.7 | 1.5 | 2.0 | 1.8 | 0.99 | 1.7 | 1.5 | 1.5 | 1.3 |
| p Value | 0.26 | 0.65 | 0.20 | 0.19 | 0.99 | 0.31 | 0.51 | 0.66 | 0.73 |
| 95% CI of | 0.67 | 0.25 | 0.69 | 0.75 | 0.24 | 0.61 | 0.42 | 0.25 | 0.32 |
| OR Quart3 | 4.4 | 9.2 | 5.8 | 4.5 | 4.1 | 4.7 | 5.7 | 9.1 | 5.0 |
| OR Quart 4 | 2.6 | 4.2 | 4.0 | 3.4 | 2.3 | 4.1 | 3.0 | 3.1 | 3.2 |
| p Value | 0.039 | 0.073 | 0.0059 | 0.0045 | 0.17 | 0.0030 | 0.070 | 0.17 | 0.063 |
| 95% CI of | 1.1 | 0.88 | 1.5 | 1.5 | 0.70 | 1.6 | 0.91 | 0.61 | 0.94 |
| OR Quart4 | 6.4 | 20 | 11 | 7.9 | 7.8 | 10 | 10.0 | 16 | 11 |

(continued)

**Mucin-16**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.11 | 1.51 | 1.11 | 2.02 | 1.11 | 1.59 |
| Average | 3.53 | 10.4 | 3.53 | 13.9 | 3.53 | 3.72 |
| Stdev | 14.4 | 41.9 | 14.4 | 46.3 | 14.4 | 6.27 |
| p(t-test) | | 0.19 | | 0.063 | | 0.95 |
| Min | 0.117 | 0.204 | 0.117 | 0.122 | 0.117 | 0.122 |
| Max | 131 | 253 | 131 | 248 | 131 | 31.2 |
| n (Samp) | 82 | 36 | 82 | 45 | 82 | 26 |
| n (Patient) | 75 | 36 | 75 | 45 | 75 | 26 |

| sCr only | 0hr prior to AKI | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.41 | 1.27 | 1.41 | 1.83 | 1.41 | 1.24 |
| Average | 8.87 | 1.81 | 8.87 | 5.69 | 8.87 | 1.52 |
| Stdev | 35.3 | 1.57 | 35.3 | 9.55 | 35.3 | 1.06 |
| p(t-test) | | 0.49 | | 0.74 | | 0.53 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Min | 0.117 | 0.481 | 0.117 | 0.631 | 0.117 | 0.631 |
| Max | 263 | 5.56 | 263 | 37.0 | 263 | 4.16 |
| n (Samp) | 197 | 12 | 197 | 14 | 197 | 9 |
| n (Patient) | 131 | 12 | 131 | 14 | 131 | 9 |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.24 | 1.60 | 1.24 | 2.52 | 1.24 | 2.44 |
| Average | 3.82 | 12.7 | 3.82 | 14.8 | 3.82 | 4.48 |
| Stdev | 15.1 | 46.6 | 15.1 | 47.3 | 15.1 | 6.81 |
| p(t-test) | | 0.14 | | 0.067 | | 0.84 |
| Min | 0.122 | 0.204 | 0.122 | 0.122 | 0.122 | 0.122 |
| Max | 131 | 253 | 131 | 248 | 131 | 31.2 |
| n (Samp) | 75 | 29 | 75 | 43 | 75 | 21 |
| n (Patient) | 62 | 29 | 62 | 43 | 62 | 21 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.62 | 0.47 | 0.61 | 0.65 | 0.61 | 0.64 | 0.62 | 0.45 | 0.66 |
| SE | 0.058 | 0.087 | 0.064 | 0.052 | 0.082 | 0.054 | 0.066 | 0.10 | 0.071 |
| p | 0.036 | 0.72 | 0.084 | 0.0055 | 0.18 | 0.0073 | 0.068 | 0.64 | 0.024 |
| nCohort 1 | 82 | 197 | 75 | 82 | 197 | 75 | 82 | 197 | 75 |
| nCohort 2 | 36 | 12 | 29 | 45 | 14 | 43 | 26 | 9 | 21 |
| Cutoff 1 | 1.06 | 1.09 | 0.999 | 1.06 | 1.33 | 1.12 | 1.06 | 1.01 | 1.25 |
| Sens 1 | 72% | 75% | 72% | 71% | 71% | 72% | 73% | 78% | 71% |
| Spec 1 | 50% | 41% | 44% | 50% | 47% | 47% | 50% | 38% | 52% |
| Cutoff 2 | 0.803 | 0.821 | 0.761 | 0.821 | 1.02 | 0.803 | 0.999 | 0.754 | 1.06 |
| Sens 2 | 81% | 83% | 83% | 80% | 86% | 81% | 85% | 89% | 81% |
| Spec 2 | 46% | 36% | 33% | 46% | 38% | 43% | 48% | 29% | 47% |
| Cutoff 3 | 0.464 | 0.481 | 0.379 | 0.334 | 1.01 | 0.334 | 0.630 | 0.630 | 0.999 |
| Sens 3 | 92% | 92% | 93% | 91% | 93% | 91% | 92% | 100% | 90% |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 3 | 16% | 16% | 11% | 10% | 38% | 8% | 24% | 22% | 44% |
| Cutoff 4 | 2.09 | 3.28 | 1.96 | 2.09 | 3.28 | 1.96 | 2.09 | 3.28 | 1.96 |
| Sens 4 | 39% | 17% | 45% | 49% | 43% | 56% | 38% | 11% | 52% |
| Spec 4 | 72% | 70% | 71% | 72% | 70% | 71% | 72% | 70% | 71% |
| Cutoff 5 | 2.78 | 5.21 | 2.98 | 2.78 | 5.21 | 2.98 | 2.78 | 5.21 | 2.98 |
| Sens 5 | 36% | 8% | 41% | 44% | 29% | 47% | 27% | 0% | 38% |
| Spec 5 | 80% | 80% | 80% | 80% | 80% | 80% | 80% | 80% | 80% |
| Cutoff 6 | 4.88 | 8.80 | 5.21 | 4.88 | 8.80 | 5.21 | 4.88 | 8.80 | 5.21 |
| Sens 6 | 28% | 0% | 31% | 29% | 14% | 28% | 12% | 0% | 14% |
| Spec 6 | 90% | 90% | 91% | 90% | 90% | 91% | 90% | 90% | 91% |
| OR Quart 2 | 2.4 | 1.0 | 1.5 | 2.9 | 5.3 | 2.2 | 3.4 | 1.0 | 2.9 |
| p Value | 0.16 | 0.98 | 0.51 | 0.071 | 0.13 | 0.18 | 0.10 | 0.99 | 0.23 |
| 95% CI of | 0.70 | 0.14 | 0.42 | 0.91 | 0.60 | 0.69 | 0.78 | 0.062 | 0.50 |
| OR Quart2 | 8.2 | 7.5 | 5.7 | 8.9 | 47 | 7.1 | 14 | 17 | 17 |
| OR Quart 3 | 1.8 | 3.3 | 1.0 | 1.2 | 3.1 | 1.7 | 2.3 | 6.7 | 3.7 |
| p Value | 0.35 | 0.15 | 1.0 | 0.80 | 0.34 | 0.37 | 0.28 | 0.085 | 0.14 |
| 95% CI of | 0.52 | 0.64 | 0.25 | 0.34 | 0.31 | 0.52 | 0.51 | 0.77 | 0.66 |
| OR Quart3 | 6.5 | 17 | 4.0 | 4.0 | 30 | 5.7 | 10 | 57 | 20 |
| OR Quart 4 | 3.7 | 1.0 | 3.6 | 6.1 | 5.3 | 5.0 | 4.0 | 1.0 | 5.5 |
| p Value | 0.034 | 0.98 | 0.043 | 0.0018 | 0.13 | 0.0061 | 0.060 | 0.99 | 0.046 |
| 95% CI of | 1.1 | 0.14 | 1.0 | 2.0 | 0.60 | 1.6 | 0.95 | 0.062 | 1.0 |
| OR Quart4 | 12 | 7.5 | 12 | 19 | 47 | 16 | 17 | 17 | 29 |

EP 2 894 473 B1

182

| Tumor necrosis factor receptor superfamily member 10B | | | | | |
|---|---|---|---|---|---|
| sCr or UO | 0hr prior to AKI stage | | 24hr prior to AKI stage | | 48hr prior to AKI stage |
| | Cohort 1 | Cohort 2 | Cohort 1 | Cohort 2 | Cohort 1 | Cohort 2 |
| Median | 1.00E-9 | 1.00E-9 | 1.00E-9 | 0.00240 | 1.00E-9 | 1.00E-9 |
| Average | 0.00715 | 0.0102 | 0.00715 | 0.0133 | 0.00715 | 0.0150 |
| Stdev | 0.0203 | 0.0434 | 0.0203 | 0.0446 | 0.0203 | 0.0622 |
| p(t-test) | | 0.60 | | 0.30 | | 0.34 |
| Min | 1.00E-9 | 1.00E-9 | 1.00E-9 | 1.00E-9 | 1.00E-9 | 1.00E-9 |
| Max | 0.114 | 0.262 | 0.114 | 0.286 | 0.114 | 0.300 |
| n (Samp) | 79 | 36 | 79 | 41 | 79 | 23 |
| n (Patient) | 72 | 36 | 72 | 41 | 72 | 23 |
| | | | | | | |
| sCr only | 0hr prior to AKI stage | | 24hr prior to AKI stage | | 48hr prior to AKI stage |
| | Cohort 1 | Cohort 2 | Cohort 1 | Cohort 2 | Cohort 1 | Cohort 2 |
| Median | 1.00E-9 | 1.00E-9 | 1.00E-9 | 0.00507 | 1.00E-9 | 1.00E-9 |
| Average | 0.00582 | 0.0263 | 0.00582 | 0.0316 | 0.00582 | 0.0334 |
| Stdev | 0.0146 | 0.0783 | 0.0146 | 0.0849 | 0.0146 | 0.0938 |
| p(t-test) | | 0.0040 | | 6.0E-4 | | 7.1E-4 |
| Min | 1.00E-9 | 1.00E-9 | 1.00E-9 | 1.00E-9 | 1.00E-9 | 1.00E-9 |
| Max | 0.114 | 0.262 | 0.114 | 0.286 | 0.114 | 0.300 |
| n(Samp) | 187 | 11 | 187 | 11 | 187 | 10 |
| n (Patient) | 127 | 11 | 127 | 11 | 127 | 10 |
| | | | | | | |
| UO only | 0hr prior to AKI stage | | 24hr prior to AKI stage | | 48hr prior to AKI stage |
| | Cohort 1 | Cohort 2 | Cohort 1 | Cohort 2 | Cohort 1 | Cohort 2 |
| Median | 0.00131 | 1.00E-9 | 0.00131 | 0.00202 | 0.00131 | 1.00E-9 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Average | 0.00817 | 0.00387 | 0.00817 | 0.0104 | 0.00817 | 0.00218 |
| Stdev | 0.0213 | 0.00693 | 0.0213 | 0.0213 | 0.0213 | 0.00328 |
| p(t-test) | | 0.28 | | 0.60 | | 0.25 |
| Min | 1.00E-9 | 1.00E-9 | 1.00E-9 | 1.00E-9 | 1.00E-9 | 1.00E-9 |
| Max | 0.114 | 0.0300 | 0.114 | 0.108 | 0.114 | 0.00903 |
| n (Samp) | 70 | 30 | 70 | 40 | 70 | 17 |
| n (Patient) | 56 | 30 | 56 | 40 | 56 | 17 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.47 | 0.44 | 0.45 | 0.61 | 0.62 | 0.57 | 0.47 | 0.51 | 0.43 |
| SE | 0.059 | 0.092 | 0.064 | 0.055 | 0.093 | 0.058 | 0.069 | 0.094 | 0.080 |
| p | 0.65 | 0.52 | 0.43 | 0.038 | 0.21 | 0.22 | 0.72 | 0.91 | 0.35 |
| nCohort 1 | 79 | 187 | 70 | 79 | 187 | 70 | 79 | 187 | 70 |
| nCohort 2 | 36 | 11 | 30 | 41 | 11 | 40 | 23 | 10 | 17 |
| Cutoff 1 | 0 | 0 | 0 | 0 | 0 | 0 | 0 | 0 | 0 |
| Sens 1 | 100% | 100% | 100% | 100% | 100% | 100% | 100% | 100% | 100% |
| Spec 1 | 0% | 0% | 0% | 0% | 0% | 0% | 0% | 0% | 0% |
| Cutoff 2 | 0 | 0 | 0 | 0 | 0 | 0 | 0 | 0 | 0 |
| Sens 2 | 100% | 100% | 100% | 100% | 100% | 100% | 100% | 100% | 100% |
| Spec 2 | 0% | 0% | 0% | 0% | 0% | 0% | 0% | 0% | 0% |
| Cutoff 3 | 0 | 0 | 0 | 0 | 0 | 0 | 0 | 0 | 0 |
| Sens 3 | 100% | 100% | 100% | 100% | 100% | 100% | 100% | 100% | 100% |
| Spec 3 | 0% | 0% | 0% | 0% | 0% | 0% | 0% | 0% | 0% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 4 | 0.00274 | 0.00377 | 0.00377 | 0.00274 | 0.00377 | 0.00377 | 0.00274 | 0.00377 | 0.00377 |
| Sens 4 | 33% | 27% | 33% | 49% | 64% | 42% | 30% | 40% | 24% |
| Spec 4 | 71% | 71% | 71% | 71% | 71% | 71% | 71% | 71% | 71% |
| Cutoff 5 | 0.00507 | 0.00819 | 0.00813 | 0.00507 | 0.00819 | 0.00813 | 0.00507 | 0.00819 | 0.00813 |
| Sens 5 | 28% | 27% | 17% | 37% | 27% | 35% | 26% | 30% | 12% |
| Spec 5 | 81% | 82% | 80% | 81% | 82% | 80% | 81% | 82% | 80% |
| Cutoff 6 | 0.0166 | 0.0136 | 0.0165 | 0.0166 | 0.0136 | 0.0165 | 0.0166 | 0.0136 | 0.0165 |
| Sends 6 | 6% | 18% | 7% | 17% | 18% | 18% | 4% | 20% | 0% |
| Spec 6 | 91% | 90% | 90% | 91% | 90% | 90% | 91% | 90% | 90% |
| OR Quart 2 | 0.12 | 0 | 0.24 | 6.0 | 0 | 1.1 | 1.1 | 0.48 | 0.71 |
| p Value | 0.011 | na | 0.057 | 0.012 | na | 0.84 | 0.94 | 0.41 | 0.68 |
| 95% CI of | 0.024 | na | 0.056 | 1.5 | na | 0.36 | 0.29 | 0.084 | 0.14 |
| OR Quart2 | 0.61 | na | 1.0 | 24 | na | 3.5 | 3.8 | 2.7 | 3.6 |
| OR Quart 3 | 2.0 | 1.4 | 1.2 | 5.2 | 1.0 | 1.2 | 2.4 | 0 | 1.7 |
| p Value | 0.19 | 0.70 | 0.77 | 0.021 | 1.0 | 0.77 | 0.14 | na | 0.47 |
| 95% CI of | 0.71 | 0.29 | 0.38 | 1.3 | 0.24 | 0.38 | 0.74 | na | 0.40 |
| OR Quart3 | 5.7 | 6.4 | 3.7 | 21 | 4.2 | 3.8 | 8.1 | na | 7.1 |
| OR Quart 4 | 0.55 | 1.4 | 0.84 | 9.0 | 0.72 | 2.4 | 0 | 0.98 | 1.1 |
| p Value | 0.30 | 0.68 | 0.77 | 0.0020 | 0.68 | 0.13 | na | 0.98 | 0.94 |
| 95% CI of | 0.17 | 0.30 | 0.26 | 2.2 | 0.15 | 0.78 | na | 0.23 | 0.23 |
| OR Quart4 | 1.7 | 6.6 | 2.7 | 36 | 3.4 | 7.2 | na | 4.2 | 4.9 |

Table 6: Comparison of marker levels in EDTA samples collected from Cohort 1 (patients that did not progress beyond RIFLE stage 0 or R) and in EDTA samples collected from subjects at 0, 24 hours, and 48 hours prior to reaching stage I or F in Cohort 2.

| Apolipoprotein A-II | | | | | | |
|---|---|---|---|---|---|---|
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 54100 | 49700 | 54100 | 45500 | 54100 | 43300 |
| Average | 55400 | 59300 | 55400 | 51900 | 55400 | 46600 |
| Stdev | 29200 | 19900 | 29200 | 32000 | 29200 | 33800 |
| p(t-test) | | 0.57 | | 0.57 | | 0.26 |
| Min | 5450 | 32800 | 5450 | 10700 | 5450 | 1810 |
| Max | 253000 | 97000 | 253000 | 176000 | 253000 | 152000 |
| n (Samp) | 230 | 19 | 230 | 26 | 230 | 15 |
| n (Patient) | 158 | 19 | 158 | 26 | 158 | 15 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 52600 | 51800 | 52600 | 45500 | 52600 | 43200 |
| Average | 54900 | 58300 | 54900 | 51000 | 54900 | 47000 |
| Stdev | 29800 | 21200 | 29800 | 31800 | 29800 | 35000 |
| p(t-test) | | 0.62 | | 0.54 | | 0.35 |
| Min | 7680 | 23300 | 7680 | 10700 | 7680 | 1810 |
| Max | 253000 | 97000 | 253000 | 176000 | 253000 | 152000 |
| n (Samp) | 201 | 19 | 201 | 26 | 201 | 14 |
| n (Patient) | 133 | 19 | 133 | 26 | 133 | 14 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.56 | nd | 0.56 | 0.44 | nd | 0.43 | 0.36 | nd | 0.37 |
| SE | 0.071 | nd | 0.071 | 0.061 | nd | 0.062 | 0.079 | nd | 0.082 |
| p | 0.38 | nd | 0.40 | 0.30 | nd | 0.24 | 0.069 | nd | 0.11 |
| nCohort 1 | 230 | nd | 201 | 230 | nd | 201 | 230 | nd | 201 |
| nCohort 2 | 19 | nd | 19 | 26 | nd | 26 | 15 | nd | 14 |
| Cutoff 1 | 43600 | nd | 43600 | 35400 | nd | 35000 | 36000 | nd | 36000 |
| Sens 1 | 74% | nd | 74% | 73% | nd | 73% | 73% | nd | 71% |
| Spec 1 | 35% | nd | 36% | 21% | nd | 21% | 21% | nd | 21% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 2 | 43000 | nd | 40500 | 30600 | nd | 30600 | 35900 | nd | 31700 |
| Sens 2 | 84% | nd | 84% | 81% | nd | 81% | 80% | nd | 86% |
| Spec 2 | 34% | nd | 30% | 17% | nd | 18% | 21% | nd | 19% |
| Cutoff 3 | 40200 | nd | 32500 | 28300 | nd | 26600 | 7970 | nd | 7970 |
| Sens 3 | 95% | nd | 95% | 92% | nd | 92% | 93% | nd | 93% |
| Spec 3 | 30% | nd | 20% | 16% | nd | 14% | 1% | nd | 1% |
| Cutoff 4 | 64400 | nd | 64800 | 64400 | nd | 64800 | 64400 | nd | 64800 |
| Sens 4 | 42% | nd | 42% | 19% | nd | 15% | 13% | nd | 14% |
| Spec 4 | 70% | nd | 70% | 70% | nd | 70% | 70% | nd | 70% |
| Cutoff 5 | 72600 | nd | 72300 | 72600 | nd | 72300 | 72600 | nd | 72300 |
| Sens 5 | 26% | nd | 26% | 12% | nd | 12% | 13% | nd | 14% |
| Spec 5 | 80% | nd | 80% | 80% | nd | 80% | 80% | nd | 80% |
| Cutoff 6 | 87100 | nd | 83600 | 87100 | nd | 83600 | 87100 | nd | 83600 |
| Sens 6 | 16% | nd | 16% | 8% | nd | 8% | 7% | nd | 7% |
| Spec 6 | 90% | nd | 90% | 90% | nd | 90% | 90% | nd | 90% |
| ORQuart 2 | 12 | nd | 5.2 | 1.2 | nd | 1.9 | 0 | nd | 0 |
| p Value | 0.021 | nd | 0.041 | 0.75 | nd | 0.35 | na | nd | na |
| 95% CI of | 1.5 | nd | 1.1 | 0.35 | nd | 0.51 | na | nd | na |
| OR Quart2 | 95 | nd | 25 | 4.2 | nd | 6.7 | na | nd | na |
| ORQuart 3 | 1.0 | nd | 0.49 | 1.2 | nd | 1.6 | 3.9 | nd | 3.9 |
| p Value | 1.0 | nd | 0.57 | 0.75 | nd | 0.51 | 0.099 | nd | 0.10 |
| 95% CI of | 0.061 | nd | 0.043 | 0.35 | nd | 0.42 | 0.77 | nd | 0.77 |
| OR Quart3 | 16 | nd | 5.6 | 4.2 | nd | 5.8 | 20 | nd | 20 |
| ORQuart 4 | 7.6 | nd | 3.9 | 1.9 | nd | 2.5 | 3.3 | nd | 2.7 |
| p Value | 0.061 | nd | 0.10 | 0.26 | nd | 0.14 | 0.16 | nd | 0.25 |
| 95% CI of | 0.91 | nd | 0.77 | 0.61 | nd | 0.73 | 0.63 | nd | 0.50 |
| OR Quart4 | 64 | nd | 20 | 6.1 | nd | 8.8 | 17 | nd | 15 |

(continued)

**Caspase-1**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 70.5 | 58.2 | nd | nd | nd | nd |
| Average | 90.5 | 69.4 | nd | nd | nd | nd |
| Stdev | 59.1 | 45.7 | nd | nd | nd | nd |
| p(t-test) | | 0.31 | nd | nd | nd | nd |
| Min | 20.6 | 36.3 | nd | nd | nd | nd |
| Max | 326 | 188 | nd | nd | nd | nd |
| n (Samp) | 60 | 9 | nd | nd | nd | nd |
| n (Patient) | 37 | 9 | nd | nd | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 72.3 | 58.2 | nd | nd | nd | nd |
| Average | 91.4 | 69.4 | nd | nd | nd | nd |
| Stdev | 54.9 | 45.7 | nd | nd | nd | nd |
| p(t-test) | | 0.27 | nd | nd | nd | nd |
| Min | 33.8 | 36.3 | nd | nd | nd | nd |
| Max | 271 | 188 | nd | nd | nd | nd |
| n (Samp) | 45 | 9 | nd | nd | nd | nd |
| n (Patient) | 26 | 9 | nd | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.32 | nd | 0.31 | nd | nd | nd | nd | nd | nd |
| SE | 0.10 | nd | 0.10 | nd | nd | nd | nd | nd | nd |
| p | 0.089 | nd | 0.066 | nd | nd | nd | nd | nd | nd |
| nCohort 1 | 60 | nd | 45 | nd | nd | nd | nd | nd | nd |
| nCohort 2 | 9 | nd | 9 | nd | nd | nd | nd | nd | nd |
| Cutoff 1 | 45.8 | nd | 45.8 | nd | nd | nd | nd | nd | nd |
| Sens 1 | 78% | nd | 78% | nd | nd | nd | nd | nd | nd |
| Spec 1 | 15% | nd | 11% | nd | nd | nd | nd | nd | nd |
| Cutoff 2 | 42.1 | nd | 42.0 | nd | nd | nd | nd | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 2 | 89% | nd | 89% | nd | nd | nd | nd | nd | nd |
| Spec 2 | 13% | nd | 9% | nd | nd | nd | nd | nd | nd |
| Cutoff 3 | 35.1 | nd | 33.8 | nd | nd | nd | nd | nd | nd |
| Sens 3 | 100% | nd | 100% | nd | nd | nd | nd | nd | nd |
| Spec 3 | 5% | nd | 2% | nd | nd | nd | nd | nd | nd |
| Cutoff 4 | 95.6 | nd | 95.9 | nd | nd | nd | nd | nd | nd |
| Sens 4 | 11% | nd | 11% | nd | nd | nd | nd | nd | nd |
| Spec 4 | 70% | nd | 73% | nd | nd | nd | nd | nd | nd |
| Cutoff 5 | 110 | nd | 103 | nd | nd | nd | nd | nd | nd |
| Sens 5 | 11% | nd | 11% | nd | nd | nd | nd | nd | nd |
| Spec 5 | 80% | nd | 80% | nd | nd | nd | nd | nd | nd |
| Cutoff 6 | 147 | nd | 186 | nd | nd | nd | nd | nd | nd |
| Sens 6 | 11% | nd | 11% | nd | nd | nd | nd | nd | nd |
| Spec 6 | 90% | nd | 91% | nd | nd | nd | nd | nd | nd |
| OR Quart 2 | 1.1 | nd | 1.1 | nd | nd | nd | nd | nd | nd |
| p Value | 0.97 | nd | 0.96 | nd | nd | nd | nd | nd | nd |
| 95% CI of | 0.061 | nd | 0.061 | nd | nd | nd | nd | nd | nd |
| OR Quart2 | 18 | nd | 19 | nd | nd | nd | nd | nd | nd |
| OR Quart 3 | 5.2 | nd | 5.2 | nd | nd | nd | nd | nd | nd |
| p Value | 0.16 | nd | 0.17 | nd | nd | nd | nd | nd | nd |
| 95% CI of | 0.52 | nd | 0.50 | nd | nd | nd | nd | nd | nd |
| OR Quart3 | 53 | nd | 54 | nd | nd | nd | nd | nd | nd |
| OR Quart 4 | 3.6 | nd | 3.9 | nd | nd | nd | nd | nd | nd |
| p Value | 0.29 | nd | 0.27 | nd | nd | nd | nd | nd | nd |
| 95% CI of | 0.34 | nd | 0.35 | nd | nd | nd | nd | nd | nd |
| OR Quart4 | 39 | nd | 43 | nd | nd | nd | nd | nd | nd |

**Caspase-9**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 21.3 | 7.75 | nd | nd | nd | nd |

(continued)

| Caspase-9 | | | | | | | |
|---|---|---|---|---|---|---|---|
| sCr or UO | 0hr prior to AKI stage | | 24hr prior to AKI stage | | 48hr prior to AKI stage | | |
| | Cohort 1 | Cohort 2 | Cohort 1 | Cohort 2 | Cohort 1 | Cohort 2 | |
| Average | 42.0 | 23.4 | nd | nd | nd | nd | |
| Stdev | 59.4 | 35.2 | nd | nd | nd | nd | |
| p(t-test) | | 0.36 | nd | nd | nd | nd | |
| Min | 0.400 | 3.79 | nd | nd | nd | nd | |
| Max | 366 | 114 | nd | nd | nd | nd | |
| n (Samp) | 82 | 9 | nd | nd | nd | nd | |
| n (Patient) | 59 | 9 | nd | nd | nd | nd | |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 23.9 | 7.75 | nd | nd | nd | nd |
| Average | 46.6 | 23.4 | nd | nd | nd | nd |
| Stdev | 65.1 | 35.2 | nd | nd | nd | nd |
| p(t-test) | | 0.30 | nd | nd | nd | nd |
| Min | 0.400 | 3.79 | nd | nd | nd | nd |
| Max | 366 | 114 | nd | nd | nd | nd |
| n (Samp) | 64 | 9 | nd | nd | nd | nd |
| n (Patient) | 46 | 9 | nd | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.32 | nd | 0.28 | nd | nd | nd | nd | nd | nd |
| SE | 0.10 | nd | 0.10 | nd | nd | nd | nd | nd | nd |
| p | 0.085 | nd | 0.032 | nd | nd | nd | nd | nd | nd |
| nCohort 1 | 82 | nd | 64 | nd | nd | nd | nd | nd | nd |
| nCohort 2 | 9 | nd | 9 | nd | nd | nd | nd | nd | nd |
| Cutoff 1 | 4.69 | nd | 4.69 | nd | nd | nd | nd | nd | nd |
| Sens 1 | 78% | nd | 78% | nd | nd | nd | nd | nd | nd |
| Spec 1 | 9% | nd | 3% | nd | nd | nd | nd | nd | nd |
| Cutoff 2 | 4.59 | nd | 4.59 | nd | nd | nd | nd | nd | nd |
| Sens 2 | 89% | nd | 89% | nd | nd | nd | nd | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 2 | 9% | nd | 3% | nd | nd | nd | nd | nd | nd |
| Cutoff 3 | 3.65 | nd | 0.400 | nd | nd | nd | nd | nd | nd |
| Sens 3 | 100% | nd | 100% | nd | nd | nd | nd | nd | nd |
| Spec 3 | 6% | nd | 2% | nd | nd | nd | nd | nd | nd |
| Cutoff 4 | 43.6 | nd | 45.3 | nd | nd | nd | nd | nd | nd |
| Sens 4 | 11% | nd | 11% | nd | nd | nd | nd | nd | nd |
| Spec 4 | 71% | nd | 70% | nd | nd | nd | nd | nd | nd |
| Cutoff 5 | 52.2 | nd | 54.5 | nd | nd | nd | nd | nd | nd |
| Sens 5 | 11% | nd | 11% | nd | nd | nd | nd | nd | nd |
| Spec 5 | 80% | nd | 81% | nd | nd | nd | nd | nd | nd |
| Cutoff 6 | 87.8 | nd | 106 | nd | nd | nd | nd | nd | nd |
| Sens 6 | 11% | nd | 11% | nd | nd | nd | nd | nd | nd |
| Spec 6 | 90% | nd | 91% | nd | nd | nd | nd | nd | nd |
| ORQuart 2 | 2.1 | nd | 2.2 | nd | nd | nd | nd | nd | nd |
| p Value | 0.56 | nd | 0.52 | nd | nd | nd | nd | nd | nd |
| 95% CI of | 0.18 | nd | 0.19 | nd | nd | nd | nd | nd | nd |
| OR Quart2 | 25 | nd | 27 | nd | nd | nd | nd | nd | nd |
| ORQuart 3 | 1.0 | nd | 1.1 | nd | nd | nd | nd | nd | nd |
| p Value | 1.0 | nd | 0.97 | nd | nd | nd | nd | nd | nd |
| 95% CI of | 0.059 | nd | 0.061 | nd | nd | nd | nd | nd | nd |
| OR Quart3 | 17 | nd | 18 | nd | nd | nd | nd | nd | nd |
| ORQuart 4 | 6.5 | nd | 6.9 | nd | nd | nd | nd | nd | nd |
| p Value | 0.10 | nd | 0.094 | nd | nd | nd | nd | nd | nd |
| 95% CI of | 0.69 | nd | 0.72 | nd | nd | nd | nd | nd | nd |
| OR Quart4 | 61 | nd | 67 | nd | nd | nd | nd | nd | nd |

**Cadherin-1**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 77700 | 123000 | 77700 | 103000 | 77700 | 117000 |
| Average | 113000 | 130000 | 113000 | 122000 | 113000 | 146000 |

(continued)

| Cadherin-1 | | | | | | |
|---|---|---|---|---|---|---|
| sCr or UO | 0hr prior to AKI stage | | 24hr prior to AKI stage | | 48hr prior to AKI stage | |
| Stdev | 97300 | 57100 | 97300 | 77800 | 97300 | 81700 |
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| p(t-test) | | 0.64 | | 0.70 | | 0.26 |
| Min | 14500 | 51600 | 14500 | 3320 | 14500 | 50500 |
| Max | 621000 | 231000 | 621000 | 340000 | 621000 | 285000 |
| n (Samp) | 123 | 7 | 123 | 20 | 123 | 12 |
| n (Patient) | 97 | 7 | 97 | 20 | 97 | 12 |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | nd | nd | 86600 | 95800 | 86600 | 105000 |
| Average | nd | nd | 123000 | 105000 | 123000 | 128000 |
| Stdev | nd | nd | 101000 | 52100 | 101000 | 80100 |
| p(t-test) | nd | nd | | 0.44 | | 0.88 |
| Min | nd | nd | 25800 | 3320 | 25800 | 50500 |
| Max | nd | nd | 621000 | 209000 | 621000 | 277000 |
| n (Samp) | nd | nd | 104 | 20 | 104 | 10 |
| n (Patient) | nd | nd | 81 | 20 | 81 | 10 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.67 | nd | nd | 0.60 | nd | 0.54 | 0.69 | nd | 0.57 |
| SE | 0.12 | nd | nd | 0.071 | nd | 0.072 | 0.088 | nd | 0.098 |
| p | 0.14 | nd | nd | 0.16 | nd | 0.54 | 0.031 | nd | 0.47 |
| nCohort 1 | 123 | nd | nd | 123 | nd | 104 | 123 | nd | 104 |
| nCohort 2 | 7 | nd | nd | 20 | nd | 20 | 12 | nd | 10 |
| Cutoff 1 | 108000 | nd | nd | 81200 | nd | 85700 | 100000 | nd | 79100 |
| Sens 1 | 71% | nd | nd | 70% | nd | 70% | 75% | nd | 70% |
| Spec 1 | 70% | nd | nd | 53% | nd | 50% | 67% | nd | 45% |
| Cutoff 2 | 91400 | nd | nd | 69300 | nd | 70100 | 79100 | nd | 75400 |
| Sens 2 | 86% | nd | nd | 80% | nd | 80% | 83% | nd | 80% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 2 | 63% | nd | nd | 45% | nd | 38% | 52% | nd | 40% |
| Cutoff 3 | 51000 | nd | nd | 54100 | nd | 63600 | 75400 | nd | 54100 |
| Sens 3 | 100% | nd | nd | 90% | nd | 90% | 92% | nd | 90% |
| Spec 3 | 20% | nd | nd | 24% | nd | 33% | 48% | nd | 18% |
| Cutoff 4 | 110000 | nd | nd | 110000 | nd | 114000 | 110000 | nd | 114000 |
| Sens 4 | 57% | nd | nd | 40% | nd | 30% | 50% | nd | 40% |
| Spec 4 | 71% | nd | nd | 71% | nd | 70% | 71% | nd | 70% |
| Cutoff 5 | 165000 | nd | nd | 165000 | nd | 184000 | 165000 | nd | 184000 |
| Sens 5 | 29% | nd | nd | 25% | nd | 10% | 25% | nd | 20% |
| Spec 5 | 80% | nd | nd | 80% | nd | 81% | 80% | nd | 81% |
| Cutoff 6 | 248000 | nd | nd | 248000 | nd | 257000 | 248000 | nd | 257000 |
| Sens 6 | 0% | nd | nd | 5% | nd | 0% | 25% | nd | 20% |
| Spec 6 | 90% | nd | nd | 90% | nd | 90% | 90% | nd | 90% |
| ORQuart 2 | 0 | nd | nd | 0.97 | nd | 3.5 | 2.0 | nd | 0.96 |
| p Value | na | nd | nd | 0.97 | nd | 0.15 | 0.58 | nd | 0.97 |
| 95% CI of | na | nd | nd | 0.18 | nd | 0.64 | 0.17 | nd | 0.13 |
| OR Quart2 | na | nd | nd | 5.2 | nd | 19 | 23 | nd | 7.4 |
| ORQuart 3 | 4.4 | nd | nd | 3.0 | nd | 5.0 | 6.9 | nd | 2.2 |
| p Value | 0.19 | nd | nd | 0.12 | nd | 0.054 | 0.083 | nd | 0.40 |
| 95% CI of | 0.47 | nd | nd | 0.74 | nd | 0.98 | 0.78 | nd | 0.36 |
| OR Quart3 | 42 | nd | nd | 13 | nd | 26 | 60 | nd | 13 |
| ORQuart 4 | 2.0 | nd | nd | 2.1 | nd | 2.1 | 3.1 | nd | 0.96 |
| p Value | 0.58 | nd | nd | 0.31 | nd | 0.40 | 0.34 | nd | 0.97 |
| 95% CI of | 0.17 | nd | nd | 0.49 | nd | 0.36 | 0.31 | nd | 0.13 |
| OR Quart4 | 23 | nd | nd | 9.3 | nd | 13 | 31 | nd | 7.4 |

**Cyclin-dependent kinase inhibitor 1**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 178 | 415 | nd | nd | nd | nd |
| Average | 989 | 2040 | nd | nd | nd | nd |

(continued)

| Cyclin-dependent kinase inhibitor 1 | | | | | | |
| --- | --- | --- | --- | --- | --- | --- |
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Stdev | 1850 | 2760 | nd | nd | nd | nd |
| p(t-test) | | 0.13 | nd | nd | nd | nd |
| Min | 0.116 | 55.2 | nd | nd | nd | nd |
| Max | 6840 | 6400 | nd | nd | nd | nd |
| n (Samp) | 80 | 9 | nd | nd | nd | nd |
| n (Patient) | 58 | 9 | nd | nd | nd | nd |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 182 | 415 | nd | nd | nd | nd |
| Average | 1090 | 2040 | nd | nd | nd | nd |
| Stdev | 2000 | 2760 | nd | nd | nd | nd |
| p(t-test) | | 0.21 | nd | nd | nd | nd |
| Min | 0.116 | 55.2 | nd | nd | nd | nd |
| Max | 6840 | 6400 | nd | nd | nd | nd |
| n (Samp) | 62 | 9 | nd | nd | nd | nd |
| n (Patient) | 45 | 9 | nd | nd | nd | nd |

| | 0hr prior to AKI stage | | | 24hr prior to AKI stage | | | 48hr prior to AKI stage | | |
| --- | --- | --- | --- | --- | --- | --- | --- | --- | --- |
| | sCr or UO | sCr only | UO only | sCr or UO | sCr only | UO only | sCr or UO | sCr only | UO only |
| AUC | 0.58 | nd | 0.56 | nd | nd | nd | nd | nd | nd |
| SE | 0.10 | nd | 0.11 | nd | nd | nd | nd | nd | nd |
| p | 0.44 | nd | 0.55 | nd | nd | nd | nd | nd | nd |
| nCohort 1 | 80 | nd | 62 | nd | nd | nd | nd | nd | nd |
| nCohort 2 | 9 | nd | 9 | nd | nd | nd | nd | nd | nd |
| Cutoff 1 | 82.7 | nd | 82.7 | nd | nd | nd | nd | nd | nd |
| Sens 1 | 78% | nd | 78% | nd | nd | nd | nd | nd | nd |
| Spec 1 | 20% | nd | 19% | nd | nd | nd | nd | nd | nd |
| Cutoff 2 | 61.7 | nd | 55.2 | nd | nd | nd | nd | nd | nd |
| Sens 2 | 89% | nd | 89% | nd | nd | nd | nd | nd | nd |
| Spec 2 | 18% | nd | 16% | nd | nd | nd | nd | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 3 | 53.6 | nd | 52.0 | nd | nd | nd | nd | nd | nd |
| Sens 3 | 100% | nd | 100% | nd | nd | nd | nd | nd | nd |
| Spec 3 | 15% | nd | 15% | nd | nd | nd | nd | nd | nd |
| Cutoff 4 | 429 | nd | 470 | nd | nd | nd | nd | nd | nd |
| Sens 4 | 44% | nd | 44% | nd | nd | nd | nd | nd | nd |
| Spec 4 | 70% | nd | 71% | nd | nd | nd | nd | nd | nd |
| Cutoff 5 | 1300 | nd | 1410 | nd | nd | nd | nd | nd | nd |
| Sens 5 | 33% | nd | 33% | nd | nd | nd | nd | nd | nd |
| Spec 5 | 80% | nd | 81% | nd | nd | nd | nd | nd | nd |
| Cutoff 6 | 3790 | nd | 5160 | nd | nd | nd | nd | nd | nd |
| Sens 6 | 33% | nd | 22% | nd | nd | nd | nd | nd | nd |
| Spec 6 | 90% | nd | 90% | nd | nd | nd | nd | nd | nd |
| ORQuart 2 | 0.30 | nd | 0.27 | nd | nd | nd | nd | nd | nd |
| p Value | 0.32 | nd | 0.29 | nd | nd | nd | nd | nd | nd |
| 95% CI of | 0.029 | nd | 0.026 | nd | nd | nd | nd | nd | nd |
| OR Quart2 | 3.2 | nd | 2.9 | nd | nd | nd | nd | nd | nd |
| ORQuart 3 | 0.30 | nd | 0.58 | nd | nd | nd | nd | nd | nd |
| p Value | 0.32 | nd | 0.58 | nd | nd | nd | nd | nd | nd |
| 95% CI of | 0.029 | nd | 0.085 | nd | nd | nd | nd | nd | nd |
| OR Quart3 | 3.2 | nd | 4.0 | nd | nd | nd | nd | nd | nd |
| ORQuart 4 | 1.3 | nd | 0.93 | nd | nd | nd | nd | nd | nd |
| p Value | 0.73 | nd | 0.94 | nd | nd | nd | nd | nd | nd |
| 95% CI of | 0.26 | nd | 0.16 | nd | nd | nd | nd | nd | nd |
| OR Quart4 | 6.8 | nd | 5.4 | nd | nd | nd | nd | nd | nd |

**Carcinoembryonic antigen-related cell adhesion molecule 5**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.71 | 2.13 | 1.71 | 2.32 | 1.71 | 2.36 |
| Average | 2.73 | 2.72 | 2.73 | 2.84 | 2.73 | 2.41 |
| Stdev | 3.35 | 2.76 | 3.35 | 2.87 | 3.35 | 1.73 |

(continued)

| Carcinoembryonic antigen-related cell adhesion molecule 5 | | | | | | |
|---|---|---|---|---|---|---|
| sCr or UO | 0hr prior to AKI stage | | 24hr prior to AKI stage | | 48hr prior to AKI stage | |
| p(t-test) | | 0.99 | | 0.86 | | 0.69 |
| Min | 0.183 | 0.324 | 0.183 | 0.245 | 0.183 | 0.355 |
| Max | 33.6 | 14.1 | 33.6 | 16.6 | 33.6 | 7.86 |
| n (Samp) | 434 | 27 | 434 | 34 | 434 | 17 |
| n (Patient) | 173 | 27 | 173 | 34 | 173 | 17 |
| | | | | | | |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 1.74 | 2.90 | 1.74 | 3.41 | 1.74 | 2.55 |
| Average | 2.76 | 4.92 | 2.76 | 2.99 | 2.76 | 3.08 |
| Stdev | 3.28 | 6.27 | 3.28 | 1.97 | 3.28 | 1.69 |
| p(t-test) | | 0.11 | | 0.84 | | 0.80 |
| Min | 0.183 | 0.324 | 0.183 | 0.245 | 0.183 | 0.355 |
| Max | 33.6 | 17.1 | 33.6 | 5.37 | 33.6 | 5.44 |
| n (Samp) | 535 | 6 | 535 | 9 | 535 | 7 |
| n (Patient) | 207 | 6 | 207 | 9 | 207 | 7 |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 1.77 | 2.36 | 1.77 | 2.32 | 1.77 | 1.93 |
| Average | 2.89 | 2.85 | 2.89 | 2.92 | 2.89 | 2.26 |
| Stdev | 3.55 | 2.71 | 3.55 | 2.91 | 3.55 | 1.79 |
| p(t-test) | | 0.95 | | 0.97 | | 0.47 |
| Min | 0.183 | 0.491 | 0.183 | 0.710 | 0.183 | 0.324 |
| Max | 33.6 | 14.1 | 33.6 | 16.6 | 33.6 | 7.86 |
| n (Samp) | 359 | 27 | 359 | 32 | 359 | 17 |
| n (Patient) | 139 | 27 | 139 | 32 | 139 | 17 |
| | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.53 | 0.57 | 0.55 | 0.55 | 0.58 | 0.55 | 0.55 | 0.65 | 0.48 |
| SE | 0.058 | 0.12 | 0.059 | 0.053 | 0.10 | 0.054 | 0.073 | 0.11 | 0.072 |
| p | 0.56 | 0.57 | 0.43 | 0.35 | 0.42 | 0.37 | 0.54 | 0.20 | 0.81 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| nCohort 1 | 434 | 535 | 359 | 434 | 535 | 359 | 434 | 535 | 359 |
| nCohort 2 | 27 | 6 | 27 | 34 | 9 | 32 | 17 | 7 | 17 |
| Cutoff 1 | 1.26 | 0.825 | 1.49 | 1.23 | 1.22 | 1.27 | 1.68 | 2.44 | 1.62 |
| Sens 1 | 70% | 83% | 70% | 71% | 78% | 72% | 71% | 71% | 71% |
| Spec 1 | 35% | 16% | 41% | 34% | 32% | 34% | 48% | 66% | 45% |
| Cutoff 2 | 1.04 | 0.825 | 1.09 | 0.971 | 0.562 | 1.06 | 1.04 | 2.28 | 0.679 |
| Sens 2 | 81% | 83% | 81% | 82% | 89% | 81% | 82% | 86% | 82% |
| Spec 2 | 28% | 16% | 28% | 25% | 7% | 27% | 28% | 63% | 10% |
| Cutoff 3 | 0.768 | 0.314 | 0.883 | 0.795 | 0.241 | 0.872 | 0.611 | 0.314 | 0.562 |
| Sens 3 | 93% | 100% | 93% | 91% | 100% | 91% | 94% | 100% | 94% |
| Spec 3 | 15% | 2% | 18% | 17% | 1% | 18% | 10% | 2% | 6% |
| Cutoff 4 | 2.71 | 2.71 | 3.17 | 2.71 | 2.71 | 3.17 | 2.71 | 2.71 | 3.17 |
| Sens 4 | 26% | 50% | 22% | 38% | 56% | 34% | 29% | 43% | 12% |
| Spec 4 | 70% | 70% | 70% | 70% | 70% | 70% | 70% | 70% | 70% |
| Cutoff 5 | 3.99 | 3.88 | 4.22 | 3.99 | 3.88 | 4.22 | 3.99 | 3.88 | 4.22 |
| Sens 5 | 11% | 33% | 11% | 18% | 33% | 16% | 12% | 29% | 12% |
| Spec 5 | 80% | 80% | 80% | 80% | 80% | 80% | 80% | 80% | 80% |
| Cutoff 6 | 5.44 | 5.46 | 5.97 | 5.44 | 5.46 | 5.97 | 5.44 | 5.46 | 5.97 |
| Sens 6 | 7% | 17% | 7% | 12% | 0% | 3% | 6% | 0% | 6% |
| Spec 6 | 90% | 90% | 90% | 90% | 90% | 90% | 90% | 90% | 90% |
| ORQuart 2 | 1.2 | 0 | 1.5 | 0.85 | 0.50 | 0.99 | 0.99 | 0 | 4.3 |
| p Value | 0.76 | na | 0.53 | 0.78 | 0.57 | 0.99 | 0.99 | na | 0.071 |
| 95% CI of OR Quart2 | 0.36 | na | 0.41 | 0.28 | 0.044 | 0.31 | 0.20 | na | 0.88 |
| | 4.1 | na | 5.6 | 2.6 | 5.5 | 3.2 | 5.0 | na | 21 |
| ORQuart 3 | 2.1 | 0.50 | 3.0 | 1.8 | 0.50 | 2.3 | 3.1 | 3.0 | 1.5 |
| p Value | 0.19 | 0.57 | 0.070 | 0.24 | 0.57 | 0.10 | 0.092 | 0.34 | 0.65 |
| 95% CI of OR Quart3 | 0.69 | 0.044 | 0.91 | 0.68 | 0.044 | 0.84 | 0.83 | 0.31 | 0.25 |
| | 6.3 | 5.5 | 9.7 | 4.7 | 5.5 | 6.4 | 12 | 30 | 9.3 |
| ORQuart 4 | 1.2 | 1.5 | 1.5 | 1.3 | 2.6 | 1.2 | 0.65 | 3.0 | 2.0 |
| p Value | 0.77 | 0.66 | 0.53 | 0.61 | 0.27 | 0.79 | 0.65 | 0.34 | 0.42 |
| 95% CI of | 0.36 | 0.25 | 0.41 | 0.47 | 0.49 | 0.38 | 0.11 | 0.31 | 0.37 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart4 | 4.0 | 9.1 | 5.6 | 3.6 | 13 | 3.6 | 4.0 | 29 | 11 |

**Myoglobin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 28.5 | 65.8 | 28.5 | 80.9 | 28.5 | 82.0 |
| Average | 82.5 | 171 | 82.5 | 252 | 82.5 | 221 |
| Stdev | 182 | 372 | 182 | 471 | 182 | 396 |
| p(t-test) | | 0.024 | | 1.2E-5 | | 0.0040 |
| Min | 3.55 | 4.22 | 3.55 | 3.96 | 3.55 | 5.12 |
| Max | 2130 | 1880 | 2130 | 1880 | 2130 | 1310 |
| n(Samp) | 434 | 27 | 434 | 34 | 434 | 17 |
| n (Patient) | 173 | 27 | 173 | 34 | 173 | 17 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 32.7 | 142 | 32.7 | 125 | 32.7 | 151 |
| Average | 87.4 | 640 | 87.4 | 653 | 87.4 | 396 |
| Stdev | 195 | 849 | 195 | 793 | 195 | 429 |
| p(t-test) | | 3.2E-10 | | 3.3E-14 | | 5.1E-5 |
| Min | 3.55 | 35.2 | 3.55 | 13.0 | 3.55 | 17.5 |
| Max | 2130 | 1880 | 2130 | 1880 | 2130 | 1180 |
| n (Samp) | 535 | 6 | 535 | 9 | 535 | 7 |
| n (Patient) | 207 | 6 | 207 | 9 | 207 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 30.3 | 56.9 | 30.3 | 86.9 | 30.3 | 79.1 |
| | Cohor 1 | Cohort 2 | Cohort 1 | Cohort 2 | Cohort 1 | Cohort 2 |
| Average | 83.3 | 103 | 83.3 | 171 | 83.3 | 261 |
| Stdev | 185 | 149 | 185 | 306 | 185 | 518 |
| p(t-test) | | 0.59 | | 0.016 | | 7.1E-4 |
| Min | 4.60 | 4.22 | 4.60 | 3.96 | 4.60 | 5.12 |

(continued)

| | | Cohor 1 | Cohort 2 | Cohort 1 | Cohort 2 | Cohort 1 | Cohort 2 |
|---|---|---|---|---|---|---|---|
| | Max | 2130 | 646 | 2130 | 1410 | 2130 | 1880 |
| | n (Samp) | 359 | 27 | 359 | 32 | 359 | 17 |
| | n (Patient) | 139 | 27 | 139 | 32 | 139 | 17 |

| | | 0hr prior to AKI stage | | | 24hr prior to AKI stage | | | 48hr prior to AKI stage | | |
|---|---|---|---|---|---|---|---|---|---|---|
| | | sCr or UO | sCr only | UO only | sCr or UO | sCr only | UO only | sCr or UO | sCr only | UO only |
| | AUC | 0.66 | 0.83 | 0.64 | 0.66 | 0.70 | 0.67 | 0.64 | 0.81 | 0.65 |
| | SE | 0.059 | 0.10 | 0.059 | 0.053 | 0.098 | 0.054 | 0.074 | 0.099 | 0.074 |
| | p | 0.0056 | 0.0015 | 0.015 | 0.0030 | 0.041 | 0.0022 | 0.050 | 0.0018 | 0.040 |
| | nCohort 1 | 434 | 535 | 359 | 434 | 535 | 359 | 434 | 535 | 359 |
| | nCohort 2 | 27 | 6 | 27 | 34 | 9 | 32 | 17 | 7 | 17 |
| | Cutoff 1 | 42.7 | 66.9 | 42.7 | 34.8 | 31.4 | 38.6 | 34.8 | 101 | 34.8 |
| | Sens 1 | 70% | 83% | 70% | 71% | 78% | 72% | 71% | 71% | 71% |
| | Spec 1 | 64% | 73% | 63% | 57% | 50% | 59% | 57% | 82% | 55% |
| | Cutoff 2 | 22.9 | 66.9 | 22.9 | 16.2 | 19.2 | 18.9 | 17.5 | 87.6 | 18.7 |
| | Sens 2 | 81% | 83% | 81% | 82% | 89% | 81% | 82% | 86% | 82% |
| | Spec 2 | 40% | 73% | 39% | 25% | 30% | 31% | 27% | 78% | 30% |
| | Cutoff 3 | 18.3 | 34.8 | 18.3 | 12.9 | 12.9 | 12.9 | 11.1 | 17.4 | 11.1 |
| | Sens 3 | 93% | 100% | 93% | 91% | 100% | 91% | 94% | 100% | 94% |
| | Spec 3 | 30% | 53% | 30% | 18% | 17% | 18% | 13% | 25% | 12% |
| | Cutoff 4 | 56.0 | 60.1 | 57.8 | 56.0 | 60.1 | 57.8 | 56.0 | 60.1 | 57.8 |
| | Sens 4 | 52% | 83% | 48% | 62% | 56% | 69% | 65% | 86% | 65% |
| | Spec 4 | 70% | 70% | 70% | 70% | 70% | 70% | 70% | 70% | 70% |
| | Cutoff 5 | 93.4 | 95.1 | 95.1 | 93.4 | 95.1 | 95.1 | 93.4 | 95.1 | 95.1 |
| | Sens 5 | 33% | 67% | 22% | 44% | 56% | 47% | 35% | 71% | 35% |
| | Spec 5 | 80% | 80% | 80% | 80% | 80% | 80% | 80% | 80% | 80% |
| | Cutoff 6 | 182 | 180 | 188 | 182 | 180 | 188 | 182 | 180 | 188 |
| | Sens 6 | 15% | 33% | 7% | 24% | 44% | 19% | 18% | 43% | 18% |
| | Spec 6 | 90% | 90% | 90% | 90% | 90% | 90% | 90% | 90% | 90% |
| | ORQuart 2 | 2.6 | >0 | 2.6 | 0.27 | 2.0 | 0.32 | 1.5 | 0 | 1.0 |
| | p Value | 0.27 | <na | 0.27 | 0.11 | 0.57 | 0.16 | 0.66 | na | 1.0 |
| | 95% CI of | 0.49 | >na | 0.48 | 0.056 | 0.18 | 0.062 | 0.25 | na | 0.14 |
| | OR Quart2 | 14 | na | 13 | 1.3 | 22 | 1.6 | 9.2 | na | 7.3 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart 3 | 4.8 | >2.0 | 4.9 | 1.0 | 1.0 | 1.2 | 0.99 | 0 | 2.0 |
| p Value | 0.048 | <0.57 | 0.047 | 1.0 | 1.0 | 0.79 | 0.99 | na | 0.42 |
| 95% CI of OR Quart3 | 1.0 23 | >0.18 na | 1.0 23 | 0.34 2.9 | 0.062 16 | 0.38 3.6 | 0.14 7.2 | na na | 0.37 11 |
| OR Quart 4 | 5.9 | >4.1 | 6.0 | 2.9 | 5.2 | 3.2 | 5.3 | 6.2 | 4.9 |
| p Value | 0.023 | <0.21 | 0.022 | 0.024 | 0.14 | 0.020 | 0.033 | 0.094 | 0.047 |
| 95% CI of OR Quart4 | 1.3 27 | >0.45 na | 1.3 28 | 1.1 7.1 | 0.59 45 | 1.2 8.5 | 1.1 25 | 0.73 52 | 1.0 23 |

**Mucin-16**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 1.34 | 1.72 | 1.34 | 1.78 | 1.34 | 2.02 |
| Average | 4.05 | 40.6 | 4.05 | 23.6 | 4.05 | 19.4 |
| Stdev | 10.9 | 98.2 | 10.9 | 66.0 | 10.9 | 61.0 |
| p(t-test) |  | 4.2E-6 |  | 2.0E-4 |  | 0.0027 |
| Min | 0.117 | 1.12 | 0.117 | 0.191 | 0.117 | 0.549 |
| Max | 131 | 263 | 131 | 253 | 131 | 248 |
| n (Samp) | 196 | 7 | 196 | 22 | 196 | 16 |
| n (Patient) | 130 | 7 | 130 | 22 | 130 | 16 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | nd | nd | 1.46 | 2.02 | 1.46 | 3.28 |
| Average | nd | nd | 4.51 | 27.2 | 4.51 | 23.4 |
| Stdev | nd | nd | 11.7 | 70.6 | 11.7 | 67.5 |
| p(t-test) | nd | nd |  | 1.8E-4 |  | 0.0018 |
| Min | nd | nd | 0.122 | 0.191 | 0.122 | 0.549 |
| Max | nd | nd | 131 | 253 | 131 | 248 |
| n (Samp) | nd | nd | 170 | 19 | 170 | 13 |
| n (Patient) | nd | nd | 108 | 19 | 108 | 13 |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.66 | nd | nd | 0.60 | nd | 0.59 | 0.64 | nd | 0.66 |
| SE | 0.11 | nd | nd | 0.067 | nd | 0.072 | 0.077 | nd | 0.085 |
| p | 0.16 | nd | nd | 0.12 | nd | 0.23 | 0.078 | nd | 0.059 |
| nCohort 1 | 196 | nd | nd | 196 | nd | 170 | 196 | nd | 170 |
| nCohort 2 | 7 | nd | nd | 22 | nd | 19 | 16 | nd | 13 |
| Cutoff 1 | 1.33 | nd | nd | 1.09 | nd | 1.12 | 1.23 | nd | 1.28 |
| Sens 1 | 71% | nd | nd | 73% | nd | 74% | 75% | nd | 77% |
| Spec 1 | 50% | nd | nd | 41% | nd | 39% | 46% | nd | 44% |
| Cutoff 2 | 1.23 | nd | nd | 1.01 | nd | 0.633 | 1.01 | nd | 1.01 |
| Sens 2 | 86% | nd | nd | 82% | nd | 84% | 81% | nd | 85% |
| Spec 2 | 46% | nd | nd | 38% | nd | 22% | 38% | nd | 35% |
| Cutoff 3 | 1.09 | nd | nd | 0.464 | nd | 0.424 | 0.761 | nd | 0.821 |
| Sens 3 | 100% | nd | nd | 91% | nd | 95% | 94% | nd | 92% |
| Spec 3 | 41% | nd | nd | 14% | nd | 12% | 29% | nd | 32% |
| Cutoff 4 | 2.77 | nd | nd | 2.77 | nd | 3.55 | 2.77 | nd | 3.55 |
| Sens 4 | 43% | nd | nd | 41% | nd | 37% | 44% | nd | 46% |
| Spec 4 | 70% | nd | nd | 70% | nd | 70% | 70% | nd | 70% |
| Cutoff 5 | 4.41 | nd | nd | 4.41 | nd | 4.88 | 4.41 | nd | 4.88 |
| Sens 5 | 29% | nd | nd | 32% | nd | 32% | 38% | nd | 38% |
| Spec 5 | 80% | nd | nd | 80% | nd | 80% | 80% | nd | 80% |
| Cutoff 6 | 6.95 | nd | nd | 6.95 | nd | 7.65 | 6.95 | nd | 7.65 |
| Sens 6 | 29% | nd | nd | 27% | nd | 32% | 31% | nd | 38% |
| Spec 6 | 90% | nd | nd | 90% | nd | 90% | 90% | nd | 90% |
| ORQuart 2 | >2.0 | nd | nd | 0.98 | nd | 1.0 | 6.6 | nd | 3.1 |
| p Value | <0.57 | nd | nd | 0.98 | nd | 1.0 | 0.085 | nd | 0.34 |
| 95% CI of | >0.18 | nd | nd | 0.23 | nd | 0.23 | 0.77 | nd | 0.31 |
| OR Quart2 | na | nd | nd | 4.1 | nd | 4.3 | 57 | nd | 31 |
| ORQuart 3 | >3.1 | nd | nd | 1.6 | nd | 1.0 | 3.1 | nd | 3.1 |
| p Value | <0.33 | nd | nd | 0.51 | nd | 1.0 | 0.33 | nd | 0.34 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| 95% CI of | >0.31 | nd | nd | 0.42 | nd | 0.23 | 0.31 | nd | 0.31 |
| OR Quart3 | na | nd | nd | 5.9 | nd | 4.3 | 31 | nd | 31 |
| ORQuart 4 | >2.0 | nd | nd | 2.1 | nd | 1.8 | 6.6 | nd | 6.6 |
| p Value | <0.57 | nd | nd | 0.24 | nd | 0.36 | 0.085 | nd | 0.087 |
| 95% CI of | >0.18 | nd | nd | 0.60 | nd | 0.50 | 0.77 | nd | 0.76 |
| OR Quart4 | na | nd | nd | 7.5 | nd | 6.7 | 57 | nd | 57 |

**Tumor necrosis factor receptor superfamily member 10B**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 1.00E-9 | 0.00692 | 1.00E-9 | 0.00181 | 1.00E-9 | 0.00572 |
| Average | 0.00543 | 0.0417 | 0.00543 | 0.0242 | 0.00543 | 0.0256 |
| Stdev | 0.0150 | 0.0972 | 0.0150 | 0.0668 | 0.0150 | 0.0736 |
| p(t-test) |  | 4.5E-5 |  | 0.0018 |  | 0.0020 |
| Min | 1.00E-9 | 1.00E-9 | 1.00E-9 | 1.00E-9 | 1.00E-9 | 1.00E-9 |
| Max | 0.114 | 0.262 | 0.114 | 0.286 | 0.114 | 0.300 |
| n (Samp) | 189 | 7 | 189 | 18 | 189 | 16 |
| n (Patient) | 125 | 7 | 125 | 18 | 125 | 16 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | nd | nd | 0.000420 | 0.00142 | 0.000420 | 0.00220 |
| Average | nd | nd | 0.00637 | 0.0151 | 0.00637 | 0.00604 |
| Stdev | nd | nd | 0.0161 | 0.0277 | 0.0161 | 0.00762 |
| p(t-test) | nd | nd |  | 0.053 |  | 0.94 |
| Min | nd | nd | 1.00E-9 | 1.00E-9 | 1.00E-9 | 1.00E-9 |
| Max | nd | nd | 0.114 | 0.108 | 0.114 | 0.0222 |
| n (Samp) | nd | nd | 162 | 17 | 162 | 13 |
| n (Patient) | nd | nd | 101 | 17 | 101 | 13 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.63 | nd | nd | 0.61 | nd | 0.59 | 0.65 | nd | 0.57 |
| SE | 0.12 | nd | nd | 0.073 | nd | 0.076 | 0.077 | nd | 0.086 |
| p | 0.25 | nd | nd | 0.12 | nd | 0.25 | 0.060 | nd | 0.38 |
| nCohort 1 | 189 | nd | nd | 189 | nd | 162 | 189 | nd | 162 |
| nCohort 2 | 7 | nd | nd | 18 | nd | 17 | 16 | nd | 13 |
| Cutoff 1 | 0 | nd | nd | 0 | nd | 0 | 0 | nd | 0 |
| Sens 1 | 100% | nd | nd | 100% | nd | 100% | 100% | nd | 100% |
| Spec 1 | 0% | nd | nd | 0% | nd | 0% | 0% | nd | 0% |
| Cutoff 2 | 0 | nd | nd | 0 | nd | 0 | 0 | nd | 0 |
| Sens 2 | 100% | nd | nd | 100% | nd | 100% | 100% | nd | 100% |
| Spec 2 | 0% | nd | nd | 0% | nd | 0% | 0% | nd | 0% |
| Cutoff 3 | 0 | nd | nd | 0 | nd | 0 | 0 | nd | 0 |
| Sens 3 | 100% | nd | nd | 100% | nd | 100% | 100% | nd | 100% |
| Spec 3 | 0% | nd | nd | 0% | nd | 0% | 0% | nd | 0% |
| Cutoff 4 | 0.00367 | nd | nd | 0.00367 | nd | 0.00444 | 0.00367 | nd | 0.00444 |
| Sens 4 | 57% | nd | nd | 44% | nd | 47% | 50% | nd | 38% |
| Spec 4 | 70% | nd | nd | 70% | nd | 70% | 70% | nd | 70% |
| Cutoff 5 | 0.00692 | nd | nd | 0.00692 | nd | 0.00819 | 0.00692 | nd | 0.00819 |
| Sens 5 | 43% | nd | nd | 39% | nd | 35% | 50% | nd | 31% |
| Spec 5 | 81% | nd | nd | 81% | nd | 82% | 81% | nd | 82% |
| Cutoff 6 | 0.0124 | nd | nd | 0.0124 | nd | 0.0136 | 0.0124 | nd | 0.0136 |
| Sens 6 | 29% | nd | nd | 28% | nd | 29% | 31% | nd | 23% |
| Spec 6 | 91% | nd | nd | 91% | nd | 90% | 91% | nd | 90% |
| OR Quart 2 | >3.2 | nd | nd | >7.9 | nd | 1.3 | >5.5 | nd | 4.2 |
| p Value | <0.32 | nd | nd | <0.057 | nd | 0.72 | <0.12 | nd | 0.21 |
| 95% CI of OR Quart 2 | >0.32 | nd | nd | >0.94 | nd | 0.28 | >0.62 | nd | 0.45 |
| | na | nd | nd | na | nd | 6.3 | na | nd | 39 |
| OR Quart 3 | >0 | nd | nd | >4.2 | nd | 0.98 | >3.2 | nd | 3.1 |
| p Value | <na | nd | nd | <0.20 | nd | 0.98 | <0.32 | nd | 0.34 |
| 95% CI of | >na | nd | nd | >0.46 | nd | 0.19 | >0.32 | nd | 0.31 |
| OR Quart3 | na | nd | nd | na | nd | 5.1 | na | nd | 31 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart 4 | >4.4 | nd | nd | >7.9 | nd | 2.5 | >9.3 | nd | 5.4 |
| p Value | <0.20 | nd | nd | <0.057 | nd | 0.20 | <0.039 | nd | 0.13 |
| 95% CI of | >0.47 | nd | nd | >0.94 | nd | 0.61 | >1.1 | nd | 0.60 |
| OR Quart4 | na | nd | nd | na | nd | 10 | na | nd | 48 |

**Cellular tumor antigen p53**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 1.00E-9 | 1.00E-9 | 1.00E-9 | 1.00E-9 | 1.00E-9 | 1.00E-9 |
| Average | 0.0690 | 0.289 | 0.0690 | 0.143 | 0.0690 | 0.172 |
| Stdev | 0.419 | 0.698 | 0.419 | 0.551 | 0.419 | 0.595 |
| p(t-test) |  | 0.19 |  | 0.49 |  | 0.36 |
| Min | 1.00E-9 | 1.00E-9 | 1.00E-9 | 1.00E-9 | 1.00E-9 | 1.00E-9 |
| Max | 3.70 | 1.87 | 3.70 | 2.35 | 3.70 | 2.39 |
| n (Samp) | 189 | 7 | 189 | 18 | 189 | 16 |
| n (Patient) | 125 | 7 | 125 | 18 | 125 | 16 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | nd | nd | 1.00E-9 | 1.00E-9 | 1.00E-9 | 1.00E-9 |
| Average | nd | nd | 0.0804 | 0.0131 | 0.0804 | 0.0283 |
| Stdev | nd | nd | 0.452 | 0.0316 | 0.452 | 0.0790 |
| p(t-test) | nd | nd |  | 0.54 |  | 0.68 |
| Min | nd | nd | 1.00E-9 | 1.00E-9 | 1.00E-9 | 1.00E-9 |
| Max | nd | nd | 3.70 | 0.117 | 3.70 | 0.285 |
| n (Samp) | nd | nd | 162 | 17 | 162 | 13 |
| n (Patient) | nd | nd | 101 | 17 | 101 | 13 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.64 | nd | nd | 0.62 | nd | 0.59 | 0.54 | nd | 0.52 |
| SE | 0.11 | nd | nd | 0.073 | nd | 0.076 | 0.077 | nd | 0.084 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p | 0.21 | nd | nd | 0.091 | nd | 0.23 | 0.57 | nd | 0.80 |
| nCohort | 189 | nd | nd | 189 | nd | 162 | 189 | nd | 162 |
| nCohort 2 | 7 | nd | nd | 18 | nd | 17 | 16 | nd | 13 |
| Cutoff 1 | 0 | nd | nd | 0 | nd | 0 | 0 | nd | 0 |
| Sens 1 | 100% | nd | nd | 100% | nd | 100% | 100% | nd | 100% |
| Spec 1 | 0% | nd | nd | 0% | nd | 0% | 0% | nd | 0% |
| Cutoff 2 | 0 | nd | nd | 0 | nd | 0 | 0 | nd | 0 |
| Sens 2 | 100% | nd | nd | 100% | nd | 100% | 100% | nd | 100% |
| Spec 2 | 0% | nd | nd | 0% | nd | 0% | 0% | nd | 0% |
| Cutoff 3 | 0 | nd | nd | 0 | nd | 0 | 0 | nd | 0 |
| Sens 3 | 100% | nd | nd | 100% | nd | 100% | 100% | nd | 100% |
| Spec 3 | 0% | nd | nd | 0% | nd | 0% | 0% | nd | 0% |
| Cutoff 4 | 1.00E-9 | nd | nd | 1.00E-9 | nd | 1.00E-9 | 1.00E-9 | nd | 1.00E-9 |
| Sens 4 | 43% | nd | nd | 44% | nd | 41% | 25% | nd | 23% |
| Spec 4 | 81% | nd | nd | 81% | nd | 80% | 81% | nd | 80% |
| Cutoff 5 | 1.00E-9 | nd | nd | 1.00E-9 | nd | 0.000790 | 1.00E-9 | nd | 0.000790 |
| Sens 5 | 43% | nd | nd | 44% | nd | 35% | 25% | nd | 23% |
| Spec 5 | 81% | nd | nd | 81% | nd | 81% | 81% | nd | 81% |
| Cutoff 6 | 0.0225 | nd | nd | 0.0225 | nd | 0.0267 | 0.0225 | nd | 0.0267 |
| Sens 6 | 43% | nd | nd | 17% | nd | 12% | 25% | nd | 15% |
| Spec 6 | 90% | nd | nd | 90% | nd | 90% | 90% | nd | 90% |
| ORQuart 2 | 3.1 | nd | nd | 10 | nd | >13 | >16 | nd | >13 |
| p Value | 0.33 | nd | nd | 0.029 | nd | <0.018 | <0.0096 | nd | <0.018 |
| 95% CI of | 0.31 | nd | nd | 1.3 | nd | >1.5 | >2.0 | nd | >1.5 |
| OR Quart2 | 31 | nd | nd | 86 | nd | na | na | nd | na |
| ORQuart 3 | 0 | nd | nd | 0 | nd | >0 | >0 | nd | >0 |
| p Value | na | nd | nd | na | nd | <na | <na | nd | <na |
| 95% CI of | na | nd | nd | na | nd | >na | >na | nd | >na |
| OR Quart3 | na | nd | nd | na | nd | na | na | nd | na |
| ORQuart 4 | 3.1 | nd | nd | 9.1 | nd | >8.1 | >4.2 | nd | >3.1 |
| p Value | 0.33 | nd | nd | 0.041 | nd | <0.055 | <0.20 | nd | <0.33 |
| 95% CI of | 0.31 | nd | nd | 1.1 | nd | >0.95 | >0.46 | nd | >0.31 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart4 | 31 | nd | nd | 76 | nd | na | na | nd | na |

Table 7: Comparison of marker levels in EDTA samples collected within 12 hours of reaching stage R from Cohort 1 (patients that reached, but did not progress beyond, RIFLE stage R) and from Cohort 2 (patients that reached RIFLE stage I or F).

| **Apolipoprotein A-II** | | | | | | |
|---|---|---|---|---|---|---|
|  | sCr or UO | | sCr only | | UO only | |
|  | Cohort 1 | Cohort 2 | Cohort 1 | Cohort 2 | Cohort 1 | Cohort 2 |
| Median | 57100 | 48800 | nd | nd | 57100 | 45400 |
| Average | 57200 | 51600 | nd | nd | 57000 | 46300 |
| Stdev | 36400 | 24300 | nd | nd | 39000 | 21400 |
| p(t-test) |  | 0.53 | nd | nd |  | 0.34 |
| Min | 7970 | 1810 | nd | nd | 7970 | 1810 |
| Max | 251000 | 100000 | nd | nd | 251000 | 75500 |
| n (Samp) | 50 | 19 | nd | nd | 41 | 14 |
| n (Patient) | 50 | 19 | nd | nd | 41 | 14 |

|  | At Enrollment | | |
|---|---|---|---|
|  | sCr or UO | sCr only | UO only |
| AUC | 0.47 | nd | 0.44 |
| SE | 0.079 | nd | 0.091 |
| p | 0.69 | nd | 0.49 |
| nCohort 1 | 50 | nd | 41 |
| nCohort 2 | 19 | nd | 14 |
| Cutoff 1 | 37400 | nd | 32500 |
| Sens 1 | 74% | nd | 71% |
| Spec 1 | 26% | nd | 24% |
| Cutoff 2 | 28800 | nd | 27600 |
| Sens 2 | 84% | nd | 86% |
| Spec 2 | 18% | nd | 22% |
| Cutoff 3 | 23900 | nd | 23900 |
| Sens 3 | 95% | nd | 93% |
| Spec 3 | 16% | nd | 20% |
| Cutoff 4 | 64400 | nd | 66800 |
| Sens 4 | 26% | nd | 21% |
| Spec 4 | 70% | nd | 71% |

(continued)

| | At Enrollment | | |
|---|---|---|---|
| | sCr or UO | sCr only | UO only |
| Cutoff 5 | 72300 | nd | 72300 |
| Sens 5 | 21% | nd | 14% |
| Spec 5 | 80% | nd | 80% |
| Cutoff 6 | 83600 | nd | 85700 |
| Sens 6 | 11% | nd | 0% |
| Spec 6 | 90% | nd | 90% |
| OR Quart 2 | 0.80 | nd | 1.0 |
| p Value | 0.77 | nd | 1.0 |
| 95% CI of | 0.17 | nd | 0.16 |
| OR Quart2 | 3.7 | nd | 6.1 |
| OR Quart 3 | 1.1 | nd | 1.5 |
| p Value | 0.91 | nd | 0.66 |
| 95% CI of | 0.25 | nd | 0.26 |
| OR Quart3 | 4.7 | nd | 8.2 |
| OR Quart 4 | 1.1 | nd | 1.6 |
| p Value | 0.91 | nd | 0.58 |
| 95% CI of | 0.25 | nd | 0.29 |
| OR Quart4 | 4.7 | nd | 9.3 |

**Myoglobin**

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 40.1 | 64.7 | nd | nd | 35.8 | 79.1 |
| Average | 139 | 182 | nd | nd | 132 | 88.9 |
| Stdev | 314 | 344 | nd | nd | 329 | 98.2 |
| p(t-test) | | 0.60 | nd | nd | | 0.60 |
| Min | 7.19 | 4.16 | nd | nd | 7.19 | 4.16 |
| Max | 2130 | 1310 | nd | nd | 2130 | 397 |
| n (Samp) | 54 | 23 | nd | nd | 46 | 17 |
| n (Patient) | 54 | 23 | nd | nd | 46 | 17 |

| | At Enrollment | | |
|---|---|---|---|
| | sCr or UO | sCr only | UO only |
| AUC | 0.55 | nd | 0.54 |
| SE | 0.073 | nd | 0.083 |

(continued)

| | At Enrollment | | |
|---|---|---|---|
| | sCr or UO | sCr only | UO only |
| p | 0.48 | nd | 0.66 |
| nCohort 1 | 54 | nd | 46 |
| nCohort 2 | 23 | nd | 17 |
| Cutoff 1 | 30.3 | nd | 34.2 |
| Sens 1 | 74% | nd | 71% |
| Spec 1 | 43% | nd | 50% |
| Cutoff 2 | 17.8 | nd | 15.3 |
| Sens 2 | 83% | nd | 82% |
| Spec 2 | 19% | nd | 13% |
| Cutoff 3 | 12.9 | nd | 4.16 |
| Sens 3 | 91% | nd | 94% |
| Spec 3 | 13% | nd | 0% |
| Cutoff 4 | 91.7 | nd | 90.0 |
| Sens 4 | 35% | nd | 41% |
| Spec 4 | 70% | nd | 72% |
| Cutoff 5 | 189 | nd | 141 |
| Sens 5 | 17% | nd | 18% |
| Spec 5 | 81% | nd | 80% |
| Cutoff 6 | 328 | nd | 315 |
| Sens 6 | 13% | nd | 6% |
| Spec 6 | 91% | nd | 91% |
| OR Quart 2 | 0.41 | nd | 0.29 |
| p Value | 0.26 | nd | 0.18 |
| 95% CI of | 0.085 | nd | 0.046 |
| OR Quart2 | 1.9 | nd | 1.8 |
| OR Quart 3 | 1.6 | nd | 0.91 |
| p Value | 0.50 | nd | 0.90 |
| 95% CI of | 0.42 | nd | 0.20 |
| OR Quart3 | 5.9 | nd | 4.1 |
| OR Quart 4 | 0.93 | nd | 0.91 |
| p Value | 0.91 | nd | 0.90 |
| 95% CI of | 0.24 | nd | 0.20 |
| OR Quart4 | 3.6 | nd | 4.1 |

Table 8: Comparison of the maximum marker levels in EDTA samples collected from Cohort 1 (patients that did not progress beyond RIFLE stage 0) and the maximum values in EDTA samples collected from subjects between enrollment and 0, 24 hours, and 48 hours prior to reaching stage F in Cohort 2.

| Apolipoprotein A-II | | | | | | |
|---|---|---|---|---|---|---|
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 52600 | 43600 | 52600 | 41100 | 52600 | 57700 |
| Average | 52700 | 57800 | 52700 | 57100 | 52700 | 72600 |
| Stdev | 22500 | 48900 | 22500 | 51100 | 22500 | 58900 |
| p(t-test) | | 0.55 | | 0.62 | | 0.068 |
| Min | 5450 | 10700 | 5450 | 10700 | 5450 | 9430 |
| Max | 105000 | 176000 | 105000 | 176000 | 105000 | 176000 |
| n (Samp) | 97 | 11 | 97 | 10 | 97 | 6 |
| n (Patient) | 97 | 11 | 97 | 10 | 97 | 6 |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 53400 | 57800 | 53400 | 52600 | 53400 | 57700 |
| Average | 53900 | 66500 | 53900 | 64700 | 53900 | 72600 |
| Stdev | 20600 | 54800 | 20600 | 54700 | 20600 | 58900 |
| p(t-test) | | 0.18 | | 0.25 | | 0.073 |
| Min | 8680 | 10700 | 8680 | 10700 | 8680 | 9430 |
| Max | 123000 | 176000 | 123000 | 176000 | 123000 | 176000 |
| n (Samp) | 84 | 8 | 84 | 8 | 84 | 6 |
| n (Patient) | 84 | 8 | 84 | 8 | 84 | 6 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.46 | nd | 0.53 | 0.45 | nd | 0.51 | 0.57 | nd | 0.56 |
| SE | 0.094 | nd | 0.11 | 0.098 | nd | 0.11 | 0.13 | nd | 0.13 |
| p | 0.69 | nd | 0.78 | 0.61 | nd | 0.94 | 0.59 | nd | 0.66 |
| nCohort 1 | 97 | nd | 84 | 97 | nd | 84 | 97 | nd | 84 |
| nCohort 2 | 11 | nd | 8 | 10 | nd | 8 | 6 | nd | 6 |
| Cutoff 1 | 37600 | nd | 37600 | 37600 | nd | 37600 | 37600 | nd | 37600 |
| Sens 1 | 73% | nd | 75% | 70% | nd | 75% | 83% | nd | 83% |
| Spec 1 | 27% | nd | 19% | 27% | nd | 19% | 27% | nd | 19% |
| Cutoff 2 | 14000 | nd | 10700 | 14000 | nd | 10700 | 37600 | nd | 37600 |
| Sens 2 | 82% | nd | 88% | 80% | nd | 88% | 83% | nd | 83% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 2 | 4% | nd | 1% | 4% | nd | 1% | 27% | nd | 19% |
| Cutoff 3 | 10700 | nd | 8680 | 10700 | nd | 8680 | 8680 | nd | 8680 |
| Sens 3 | 91% | nd | 100% | 90% | nd | 100% | 100% | nd | 100% |
| Spec 3 | 2% | nd | 1% | 2% | nd | 1% | 2% | nd | 1% |
| Cutoff 4 | 61600 | nd | 61700 | 61600 | nd | 61700 | 61600 | nd | 61700 |
| Sens 4 | 36% | nd | 50% | 40% | nd | 50% | 50% | nd | 50% |
| Spec 4 | 70% | nd | 70% | 70% | nd | 70% | 70% | nd | 70% |
| Cutoff 5 | 70000 | nd | 69100 | 70000 | nd | 69100 | 70000 | nd | 69100 |
| Sens 5 | 36% | nd | 50% | 30% | nd | 38% | 50% | nd | 50% |
| Spec 5 | 80% | nd | 81% | 80% | nd | 81% | 80% | nd | 81% |
| Cutoff 6 | 82900 | nd | 77900 | 82900 | nd | 77900 | 82900 | nd | 77900 |
| Sens 6 | 18% | nd | 25% | 20% | nd | 25% | 33% | nd | 33% |
| Spec 6 | 91% | nd | 90% | 91% | nd | 90% | 91% | nd | 90% |
| OR Quart 2 | 0 | nd | 0.30 | 0.31 | nd | 0.30 | 2.0 | nd | 0.45 |
| p Value | na | nd | 0.32 | 0.32 | nd | 0.32 | 0.58 | nd | 0.53 |
| 95% CI of | na | nd | 0.029 | 0.030 | nd | 0.029 | 0.17 | nd | 0.038 |
| OR Quart2 | na | nd | 3.2 | 3.2 | nd | 3.2 | 24 | nd | 5.4 |
| OR Quart 3 | 1.0 | nd | 0 | 1.0 | nd | 0.30 | 0 | nd | 0 |
| p Value | 1.0 | nd | na | 1.0 | nd | 0.32 | na | nd | na |
| 95% CI of | 0.22 | nd | na | 0.18 | nd | 0.029 | na | nd | na |
| OR Quart3 | 4.5 | nd | na | 5.5 | nd | 3.2 | na | nd | na |
| OR Quart 4 | 0.72 | nd | 1.4 | 1.0 | nd | 1.0 | 3.1 | nd | 1.5 |
| p Value | 0.69 | nd | 0.68 | 0.96 | nd | 1.0 | 0.34 | nd | 0.67 |
| 95% CI of | 0.14 | nd | 0.28 | 0.19 | nd | 0.18 | 0.30 | nd | 0.23 |
| OR Quart4 | 3.6 | nd | 7.1 | 5.7 | nd | 5.6 | 32 | nd | 10.0 |

**Caspase-1**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 91.6 | 138 | 91.6 | 90.7 | 91.6 | 83.0 |
| Average | 108 | 154 | 108 | 121 | 108 | 101 |
| Stdev | 74.7 | 96.4 | 74.7 | 71.9 | 74.7 | 53.3 |

(continued)

| Caspase-1 | | | | | | | | | |
|---|---|---|---|---|---|---|---|---|---|
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| p(t-test) | | | 0.17 | | | 0.69 | | | 0.83 |
| Min | 33.8 | | 44.7 | 33.8 | | 44.7 | 33.8 | | 44.7 |
| Max | 326 | | 328 | 326 | | 241 | 326 | | 192 |
| n (Samp) | 26 | | 8 | 26 | | 7 | 26 | | 6 |
| n (Patient) | 26 | | 8 | 26 | | 7 | 26 | | 6 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.67 | nd | nd | 0.56 | nd | nd | 0.51 | nd | nd |
| SE | 0.12 | nd | nd | 0.13 | nd | nd | 0.13 | nd | nd |
| p | 0.15 | nd | nd | 0.62 | nd | nd | 0.94 | nd | nd |
| nCohort 1 | 26 | nd | nd | 26 | nd | nd | 26 | nd | nd |
| nCohort 2 | 8 | nd | nd | 7 | nd | nd | 6 | nd | nd |
| Cutoff 1 | 71.0 | nd | nd | 70.7 | nd | nd | 66.1 | nd | nd |
| Sens 1 | 75% | nd | nd | 71% | nd | nd | 83% | nd | nd |
| Spec 1 | 38% | nd | nd | 38% | nd | nd | 31% | nd | nd |
| Cutoff 2 | 70.7 | nd | nd | 66.1 | nd | nd | 66.1 | nd | nd |
| Sens 2 | 88% | nd | nd | 86% | nd | nd | 83% | nd | nd |
| Spec 2 | 38% | nd | nd | 31% | nd | nd | 31% | nd | nd |
| Cutoff 3 | 42.1 | nd | nd | 42.1 | nd | nd | 42.1 | nd | nd |
| Sens 3 | 100% | nd | nd | 100% | nd | nd | 100% | nd | nd |
| Spec 3 | 15% | nd | nd | 15% | nd | nd | 15% | nd | nd |
| Cutoff 4 | 103 | nd | nd | 103 | nd | nd | 103 | nd | nd |
| Sens 4 | 62% | nd | nd | 43% | nd | nd | 33% | nd | nd |
| Spec 4 | 73% | nd | nd | 73% | nd | nd | 73% | nd | nd |
| Cutoff 5 | 121 | nd | nd | 121 | nd | nd | 121 | nd | nd |
| Sens 5 | 62% | nd | nd | 43% | nd | nd | 33% | nd | nd |
| Spec 5 | 81% | nd | nd | 81% | nd | nd | 81% | nd | nd |
| Cutoff 6 | 254 | nd | nd | 254 | nd | nd | 254 | nd | nd |
| Sens 6 | 12% | nd | nd | 0% | nd | nd | 0% | nd | nd |
| Spec 6 | 92% | nd | nd | 92% | nd | nd | 92% | nd | nd |
| OR Quart 2 | 2.0 | nd | nd | 2.3 | nd | nd | 2.3 | nd | nd |
| p Value | 0.60 | nd | nd | 0.53 | nd | nd | 0.53 | nd | nd |
| 95% CI of | 0.15 | nd | nd | 0.17 | nd | nd | 0.17 | nd | nd |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart2 | 27 | nd | nd | 33 | nd | nd | 33 | nd | nd |
| OR Quart 3 | 0 | nd | nd | 1.0 | nd | nd | 1.0 | nd | nd |
| p Value | na | nd | nd | 1.0 | nd | nd | 1.0 | nd | nd |
| 95% CI of OR Quart3 | na | nd | nd | 0.052 | nd | nd | 0.052 | nd | nd |
|  | na | nd | nd | 19 | nd | nd | 19 | nd | nd |
| OR Quart 4 | 8.8 | nd | nd | 3.5 | nd | nd | 2.3 | nd | nd |
| p Value | 0.086 | nd | nd | 0.33 | nd | nd | 0.53 | nd | nd |
| 95% CI of OR Quart4 | 0.74 | nd | nd | 0.28 | nd | nd | 0.17 | nd | nd |
|  | 100 | nd | nd | 43 | nd | nd | 33 | nd | nd |

**Caspase-9**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 28.7 | 12.2 | 28.7 | 6.83 | 28.7 | 7.20 |
| Average | 50.0 | 16.5 | 50.0 | 13.7 | 50.0 | 14.2 |
| Stdev | 69.0 | 14.3 | 69.0 | 15.0 | 69.0 | 16.2 |
| p(t-test) |  | 0.16 |  | 0.18 |  | 0.22 |
| Min | 2.58 | 4.32 | 2.58 | 3.79 | 2.58 | 3.79 |
| Max | 366 | 46.4 | 366 | 46.4 | 366 | 46.4 |
| n (Samp) | 37 | 9 | 37 | 7 | 37 | 6 |
| n (Patient) | 37 | 9 | 37 | 7 | 37 | 6 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.29 | nd | nd | 0.22 | nd | nd | 0.23 | nd | nd |
| SE | 0.10 | nd | nd | 0.11 | nd | nd | 0.12 | nd | nd |
| p | 0.040 | nd | nd | 0.012 | nd | nd | 0.024 | nd | nd |
| nCohort 1 | 37 | nd | nd | 37 | nd | nd | 37 | nd | nd |
| nCohort 2 | 9 | nd | nd | 7 | nd | nd | 6 | nd | nd |
| Cutoff 1 | 6.05 | nd | nd | 6.05 | nd | nd | 4.34 | nd | nd |
| Sens 1 | 78% | nd | nd | 71% | nd | nd | 83% | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 1 | 11% | nd | nd | 11% | nd | nd | 8% | nd | nd |
| Cutoff 2 | 4.34 | nd | nd | 4.34 | nd | nd | 4.34 | nd | nd |
| Sens 2 | 89% | nd | nd | 86% | nd | nd | 83% | nd | nd |
| Spec 2 | 8% | nd | nd | 8% | nd | nd | 8% | nd | nd |
| Cutoff 3 | 3.30 | nd | nd | 3.30 | nd | nd | 3.30 | nd | nd |
| Sens 3 | 100% | nd | nd | 100% | nd | nd | 100% | nd | nd |
| Spec 3 | 5% | nd | nd | 5% | nd | nd | 5% | nd | nd |
| Cutoff 4 | 50.7 | nd | nd | 50.7 | nd | nd | 50.7 | nd | nd |
| Sens 4 | 0% | nd | nd | 0% | nd | nd | 0% | nd | nd |
| Spec 4 | 70% | nd | nd | 70% | nd | nd | 70% | nd | nd |
| Cutoff 5 | 71.4 | nd | nd | 71.4 | nd | nd | 71.4 | nd | nd |
| Sens 5 | 0% | nd | nd | 0% | nd | nd | 0% | nd | nd |
| Spec 5 | 81% | nd | nd | 81% | nd | nd | 81% | nd | nd |
| Cutoff 6 | 106 | nd | nd | 106 | nd | nd | 106 | nd | nd |
| Sens 6 | 0% | nd | nd | 0% | nd | nd | 0% | nd | nd |
| Spec 6 | 92% | nd | nd | 92% | nd | nd | 92% | nd | nd |
| OR Quart 2 | >4.5 | nd | nd | >1.1 | nd | nd | >1.1 | nd | nd |
| p Value | <0.23 | nd | nd | <0.95 | nd | nd | <0.95 | nd | nd |
| 95% CI of | >0.39 | nd | nd | >0.060 | nd | nd | >0.060 | nd | nd |
| OR Quart2 | na | nd | nd | na | nd | nd | na | nd | nd |
| OR Quart 3 | >2.4 | nd | nd | >2.4 | nd | nd | >1.1 | nd | nd |
| p Value | <0.50 | nd | nd | <0.49 | nd | nd | <0.95 | nd | nd |
| 95% CI of | >0.19 | nd | nd | >0.19 | nd | nd | >0.060 | nd | nd |
| OR Quart3 | na | nd | nd | na | nd | nd | na | nd | nd |
| OR Quart 4 | >6.9 | nd | nd | >6.3 | nd | nd | >7.3 | nd | nd |
| p Value | <0.11 | nd | nd | <0.13 | nd | nd | <0.10 | nd | nd |
| 95% CI of | >0.63 | nd | nd | >0.58 | nd | nd | >0.66 | nd | nd |
| OR Quart4 | na | nd | nd | na | nd | nd | na | nd | nd |

(continued)

| Cadherin-1 | | | | | | |
|---|---|---|---|---|---|---|
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 72700 | 112000 | 72700 | 112000 | 72700 | 162000 |
| Average | 103000 | 157000 | 103000 | 154000 | 103000 | 174000 |
| Stdev | 108000 | 103000 | 108000 | 105000 | 108000 | 96900 |
| p(t-test) | | 0.13 | | 0.15 | | 0.13 |
| Min | 14500 | 38300 | 14500 | 38300 | 14500 | 50500 |
| Max | 621000 | 340000 | 621000 | 340000 | 621000 | 285000 |
| n (Samp) | 50 | 12 | 50 | 12 | 50 | 6 |
| n (Patient) | 50 | 12 | 50 | 12 | 50 | 6 |
| | | | | | | |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 77600 | 151000 | 77600 | 132000 | nd | nd |
| Average | 110000 | 170000 | 110000 | 163000 | nd | nd |
| Stdev | 96000 | 127000 | 96000 | 132000 | nd | nd |
| p(t-test) | | 0.15 | | 0.20 | nd | nd |
| Min | 14500 | 38300 | 14500 | 38300 | nd | nd |
| Max | 621000 | 340000 | 621000 | 340000 | nd | nd |
| n (Samp) | 96 | 6 | 96 | 6 | nd | nd |
| n (Patient) | 96 | 6 | 96 | 6 | nd | nd |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 80200 | 162000 | 80200 | 162000 | 80200 | 162000 |
| Average | 114000 | 170000 | 114000 | 170000 | 114000 | 174000 |
| Stdev | 117000 | 86300 | 117000 | 86300 | 117000 | 96900 |
| p(t-test) | | 0.21 | | 0.21 | | 0.24 |
| Min | 39700 | 64400 | 39700 | 64400 | 39700 | 50500 |
| Max | 621000 | 285000 | 621000 | 285000 | 621000 | 285000 |
| n (Samp) | 44 | 8 | 44 | 8 | 44 | 6 |
| n (Patient) | 44 | 8 | 44 | 8 | 44 | 6 |
| | | | | | | |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.69 | 0.62 | 0.77 | 0.66 | 0.56 | 0.77 | 0.77 | nd | 0.74 |
| SE | 0.092 | 0.13 | 0.10 | 0.093 | 0.13 | 0.10 | 0.12 | nd | 0.12 |
| p | 0.038 | 0.35 | 0.0087 | 0.094 | 0.65 | 0.0087 | 0.021 | nd | 0.050 |
| nCohort 1 | 50 | 96 | 44 | 50 | 96 | 44 | 50 | nd | 44 |
| nCohort 2 | 12 | 6 | 8 | 12 | 6 | 8 | 6 | nd | 6 |
| Cutoff 1 | 88900 | 53300 | 105000 | 62800 | 53300 | 105000 | 105000 | nd | 105000 |
| Sens 1 | 75% | 83% | 75% | 75% | 83% | 75% | 83% | nd | 83% |
| Spec 1 | 62% | 22% | 75% | 42% | 22% | 75% | 76% | nd | 75% |
| Cutoff 2 | 62800 | 53300 | 88900 | 54100 | 53300 | 88900 | 105000 | nd | 105000 |
| Sens 2 | 83% | 83% | 88% | 83% | 83% | 88% | 83% | nd | 83% |
| Spec 2 | 42% | 22% | 59% | 24% | 22% | 59% | 76% | nd | 75% |
| Cutoff 3 | 52600 | 38100 | 62800 | 52600 | 38100 | 62800 | 48100 | nd | 47600 |
| Sens 3 | 92% | 100% | 100% | 92% | 100% | 100% | 100% | nd | 100% |
| Spec 3 | 22% | 6% | 34% | 22% | 6% | 34% | 20% | nd | 11% |
| Cutoff 4 | 97000 | 108000 | 97500 | 97000 | 108000 | 97500 | 97000 | nd | 97500 |
| Sens 4 | 58% | 50% | 75% | 58% | 50% | 75% | 83% | nd | 83% |
| Spec 4 | 70% | 71% | 70% | 70% | 71% | 70% | 70% | nd | 70% |
| Cutoff 5 | 114000 | 151000 | 131000 | 114000 | 151000 | 131000 | 114000 | nd | 131000 |
| Sens 5 | 50% | 50% | 50% | 50% | 50% | 50% | 67% | nd | 50% |
| Spec 5 | 80% | 80% | 82% | 80% | 80% | 82% | 80% | nd | 82% |
| Cutoff 6 | 153000 | 242000 | 153000 | 153000 | 242000 | 153000 | 153000 | nd | 153000 |
| Sens 6 | 42% | 33% | 50% | 42% | 33% | 50% | 50% | nd | 50% |
| Spec 6 | 90% | 91% | 91% | 90% | 91% | 91% | 90% | nd | 91% |
| OR Quart 2 | 0.43 | 0 | >1.1 | 0.27 | 0 | >1.1 | 0 | nd | 0 |
| p Value | 0.51 | na | <0.96 | 0.28 | na | <0.96 | na | nd | na |
| 95% CI of | 0.035 | na | >0.061 | 0.025 | na | >0.061 | na | nd | na |
| OR Quart2 | 5.3 | na | na | 2.9 | na | na | na | nd | na |
| OR Quart 3 | 1.6 | 0.48 | >3.9 | 0.62 | 0 | >3.9 | 1.0 | nd | 1.0 |
| p Value | 0.63 | 0.56 | <0.27 | 0.63 | na | <0.27 | 1.0 | nd | 1.0 |
| 95% CI of | 0.23 | 0.041 | >0.35 | 0.087 | na | >0.35 | 0.056 | nd | 0.055 |
| OR Quart3 | 11 | 5.7 | na | 4.3 | na | na | 18 | nd | 18 |
| OR Quart 4 | 3.9 | 1.5 | >5.8 | 2.4 | 0.96 | >5.8 | 5.2 | nd | 4.9 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p Value | 0.14 | 0.67 | <0.14 | 0.29 | 0.96 | <0.14 | 0.17 | nd | 0.19 |
| 95% CI of | 0.64 | 0.23 | >0.55 | 0.47 | 0.17 | >0.55 | 0.50 | nd | 0.46 |
| OR Quart4 | 24 | 9.8 | na | 12 | 5.3 | na | 54 | nd | 52 |

**Cyclin-dependent kinase inhibitor 1**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 212 | 243 | 212 | 255 | 212 | 299 |
| Average | 1210 | 333 | 1210 | 363 | 1210 | 402 |
| Stdev | 1930 | 337 | 1930 | 383 | 1930 | 405 |
| p(t-test) | | 0.19 | | 0.26 | | 0.32 |
| Min | 42.3 | 73.9 | 42.3 | 68.7 | 42.3 | 68.7 |
| Max | 6840 | 1190 | 6840 | 1190 | 6840 | 1190 |
| n (Samp) | 37 | 9 | 37 | 7 | 37 | 6 |
| n (Patient) | 37 | 9 | 37 | 7 | 37 | 6 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.46 | nd | nd | 0.45 | nd | nd | 0.47 | nd | nd |
| SE | 0.11 | nd | nd | 0.12 | nd | nd | 0.13 | nd | nd |
| p | 0.71 | nd | nd | 0.68 | nd | nd | 0.81 | nd | nd |
| nCohort 1 | 37 | nd | nd | 37 | nd | nd | 37 | nd | nd |
| nCohort 2 | 9 | nd | nd | 7 | nd | nd | 6 | nd | nd |
| Cutoff 1 | 138 | nd | nd | 138 | nd | nd | 138 | nd | nd |
| Sens 1 | 78% | nd | nd | 71% | nd | nd | 83% | nd | nd |
| Spec 1 | 38% | nd | nd | 38% | nd | nd | 38% | nd | nd |
| Cutoff 2 | 125 | nd | nd | 125 | nd | nd | 138 | nd | nd |
| Sens 2 | 89% | nd | nd | 86% | nd | nd | 83% | nd | nd |
| Spec 2 | 32% | nd | nd | 32% | nd | nd | 38% | nd | nd |
| Cutoff 3 | 73.1 | nd | nd | 61.7 | nd | nd | 61.7 | nd | nd |
| Sens 3 | 100% | nd | nd | 100% | nd | nd | 100% | nd | nd |
| Spec 3 | 14% | nd | nd | 11% | nd | nd | 11% | nd | nd |
| Cutoff 4 | 866 | nd | nd | 866 | nd | nd | 866 | nd | nd |
| Sens 4 | 11% | nd | nd | 14% | nd | nd | 17% | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 4 | 70% | nd | nd | 70% | nd | nd | 70% | nd | nd |
| Cutoff 5 | 1760 | nd | nd | 1760 | nd | nd | 1760 | nd | nd |
| Sens 5 | 0% | nd | nd | 0% | nd | nd | 0% | nd | nd |
| Spec 5 | 81% | nd | nd | 81% | nd | nd | 81% | nd | nd |
| Cutoff 6 | 5160 | nd | nd | 5160 | nd | nd | 5160 | nd | nd |
| Sens 6 | 0% | nd | nd | 0% | nd | nd | 0% | nd | nd |
| Spec 6 | 92% | nd | nd | 92% | nd | nd | 92% | nd | nd |
| OR Quart 2 | 6.3 | nd | nd | 3.8 | nd | nd | 3.8 | nd | nd |
| p Value | 0.13 | nd | nd | 0.29 | nd | nd | 0.29 | nd | nd |
| 95% CI of | 0.58 | nd | nd | 0.32 | nd | nd | 0.32 | nd | nd |
| OR Quart2 | 68 | nd | nd | 43 | nd | nd | 43 | nd | nd |
| OR Quart 3 | 3.7 | nd | nd | 2.2 | nd | nd | 1.0 | nd | nd |
| p Value | 0.29 | nd | nd | 0.54 | nd | nd | 1.0 | nd | nd |
| 95% CI of | 0.32 | nd | nd | 0.17 | nd | nd | 0.055 | nd | nd |
| OR Quart3 | 42 | nd | nd | 29 | nd | nd | 18 | nd | nd |
| OR Quart 4 | 1.1 | nd | nd | 1.0 | nd | nd | 1.1 | nd | nd |
| p Value | 0.95 | nd | nd | 1.0 | nd | nd | 0.94 | nd | nd |
| 95% CI of | 0.060 | nd | nd | 0.055 | nd | nd | 0.060 | nd | nd |
| OR Quart4 | 20 | nd | nd | 18 | nd | nd | 20 | nd | nd |

| **Carcinoembryonic antigen-related cell adhesion molecule 5** | | | | | | |
|---|---|---|---|---|---|---|
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 1.72 | 3.47 | 1.72 | 3.47 | 1.72 | 2.26 |
| Average | 2.52 | 4.00 | 2.52 | 3.11 | 2.52 | 2.81 |
| Stdev | 2.90 | 3.87 | 2.90 | 1.92 | 2.90 | 1.94 |
| p(t-test) | | 0.065 | | 0.42 | | 0.77 |
| Min | 0.183 | 0.726 | 0.183 | 0.563 | 0.183 | 0.726 |
| Max | 20.8 | 17.1 | 20.8 | 5.94 | 20.8 | 5.94 |
| n (Samp) | 110 | 17 | 110 | 17 | 110 | 9 |
| n (Patient) | 110 | 17 | 110 | 17 | 110 | 9 |

(continued)

**Carcinoembryonic antigen-related cell adhesion molecule 5**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| | | | | | | |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 1.88 | 2.35 | 1.88 | 2.35 | nd | nd |
| Average | 3.12 | 2.81 | 3.12 | 2.75 | nd | nd |
| Stdev | 4.09 | 2.17 | 4.09 | 2.23 | nd | nd |
| p(t-test) | | 0.83 | | 0.80 | nd | nd |
| Min | 0.183 | 0.726 | 0.183 | 0.563 | nd | nd |
| Max | 33.6 | 5.44 | 33.6 | 5.44 | nd | nd |
| n (Samp) | 180 | 8 | 180 | 8 | nd | nd |
| n (Patient) | 180 | 8 | 180 | 8 | nd | nd |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 1.71 | 5.01 | 1.71 | 3.72 | 1.71 | 3.47 |
| Average | 2.73 | 5.08 | 2.73 | 3.75 | 2.73 | 3.38 |
| Stdev | 3.23 | 4.31 | 3.23 | 1.66 | 3.23 | 1.82 |
| p(t-test) | | 0.030 | | 0.30 | | 0.60 |
| Min | 0.183 | 1.30 | 0.183 | 1.30 | 0.183 | 1.30 |
| Max | 20.8 | 17.1 | 20.8 | 5.94 | 20.8 | 5.94 |
| n (Samp) | 89 | 11 | 89 | 11 | 89 | 7 |
| n (Patient) | 89 | 11 | 89 | 11 | 89 | 7 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.66 | 0.50 | 0.78 | 0.63 | 0.48 | 0.75 | 0.60 | nd | 0.70 |
| SE | 0.076 | 0.10 | 0.086 | 0.077 | 0.11 | 0.088 | 0.10 | nd | 0.11 |
| p | 0.033 | 1.00 | 0.0012 | 0.086 | 0.83 | 0.0047 | 0.34 | nd | 0.085 |
| nCohort 1 | 110 | 180 | 89 | 110 | 180 | 89 | 110 | nd | 89 |
| nCohort 2 | 17 | 8 | 11 | 17 | 8 | 11 | 9 | nd | 7 |
| Cutoff 1 | 1.44 | 0.826 | 2.71 | 1.44 | 0.710 | 2.52 | 1.28 | nd | 2.23 |
| Sens 1 | 71% | 75% | 73% | 71% | 75% | 73% | 78% | nd | 71% |
| Spec 1 | 45% | 16% | 73% | 45% | 12% | 72% | 40% | nd | 64% |
| Cutoff 2 | 0.886 | 0.825 | 2.52 | 0.886 | 0.649 | 2.23 | 0.886 | nd | 1.49 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 2 | 82% | 88% | 82% | 82% | 88% | 82% | 89% | nd | 86% |
| Spec 2 | 25% | 16% | 72% | 25% | 8% | 64% | 25% | nd | 46% |
| Cutoff 3 | 0.825 | 0.710 | 1.49 | 0.649 | 0.504 | 1.49 | 0.710 | nd | 1.28 |
| Sens 3 | 94% | 100% | 91% | 94% | 100% | 91% | 100% | nd | 100% |
| Spec 3 | 23% | 12% | 46% | 14% | 6% | 46% | 17% | nd | 40% |
| Cutoff 4 | 2.42 | 2.71 | 2.52 | 2.42 | 2.71 | 2.52 | 2.42 | nd | 2.52 |
| Sens 4 | 65% | 50% | 82% | 59% | 50% | 73% | 44% | nd | 57% |
| Spec 4 | 70% | 70% | 72% | 70% | 70% | 72% | 70% | nd | 72% |
| Cutoff 5 | 3.25 | 3.88 | 3.74 | 3.25 | 3.88 | 3.74 | 3.25 | nd | 3.74 |
| Sens 5 | 53% | 38% | 55% | 53% | 38% | 45% | 44% | nd | 29% |
| Spec 5 | 80% | 80% | 81% | 80% | 80% | 81% | 80% | nd | 81% |
| Cutoff 6 | 5.56 | 6.28 | 6.77 | 5.56 | 6.28 | 6.77 | 5.56 | nd | 6.77 |
| Sens 6 | 12% | 0% | 9% | 6% | 0% | 0% | 11% | nd | 0% |
| Spec 6 | 90% | 90% | 91% | 90% | 90% | 91% | 90% | nd | 91% |
| OR Quart | 0.97 | 0 | >2.2 | 0.97 | 0 | >2.2 | 3.1 | nd | >2.2 |
| p Value | 0.97 | na | <0.54 | 0.97 | na | <0.54 | 0.34 | nd | <0.54 |
| 95% CI of | 0.18 | na | >0.18 | 0.18 | na | >0.18 | 0.30 | nd | >0.18 |
| OR Quart2 | 5.2 | na | na | 5.2 | na | na | 32 | nd | na |
| OR Quart 3 | 0.62 | 0 | >2.2 | 0.62 | 0 | >2.2 | 0.97 | nd | >1.0 |
| p Value | 0.62 | na | <0.54 | 0.62 | na | <0.54 | 0.98 | nd | <0.98 |
| 95% CI of | 0.097 | na | >0.18 | 0.097 | na | >0.18 | 0.058 | nd | >0.062 |
| OR Quart3 | 4.0 | na | na | 4.0 | na | na | 16 | nd | na |
| OR Quart 4 | 3.7 | 1.0 | >9.7 | 3.7 | 1.0 | >9.7 | 4.3 | nd | >4.8 |
| p Value | 0.073 | 1.0 | <0.041 | 0.073 | 1.0 | <0.041 | 0.20 | nd | <0.18 |
| 95% CI of | 0.88 | 0.23 | >1.1 | 0.88 | 0.23 | >1.1 | 0.45 | nd | >0.50 |
| OR Quart4 | 15 | 4.3 | na | 15 | 4.3 | na | 41 | nd | na |

**Myoglobin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 48.8 | 222 | 48.8 | 145 | 48.8 | 122 |
| Average | 109 | 574 | 109 | 498 | 109 | 407 |

(continued)

| Myoglobin | | | | | | |
|---|---|---|---|---|---|---|
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Stdev | 211 | 653 | 211 | 658 | 211 | 480 |
| D(t-test) | | 4.0E-8 | | 3.3E-6 | | 4.7E-4 |
| Min | 5.55 | 16.2 | 5.55 | 16.2 | 5.55 | 16.2 |
| Max | 1720 | 1880 | 1720 | 1880 | 1720 | 1180 |
| n (Samp) | 110 | 17 | 110 | 17 | 110 | 9 |
| n (Patient) | 110 | 17 | 110 | 17 | 110 | 9 |
| | | | | | | |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 56.7 | 475 | 56.7 | 475 | nd | nd |
| Average | 143 | 694 | 143 | 683 | nd | nd |
| Stdev | 283 | 718 | 283 | 729 | nd | nd |
| p(t-test) | | 2.1E-6 | | 3.5E-6 | nd | nd |
| Min | 5.55 | 16.2 | 5.55 | 16.2 | nd | nd |
| Max | 2130 | 1880 | 2130 | 1880 | nd | nd |
| n (Samp) | 180 | 8 | 180 | 8 | nd | nd |
| n (Patient) | 180 | 8 | 180 | 8 | nd | nd |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 46.2 | 222 | 46.2 | 145 | 46.2 | 122 |
| Average | 120 | 547 | 120 | 438 | 120 | 352 |
| Stdev | 223 | 581 | 223 | 574 | 223 | 423 |
| D(t-test) | | 6.9E-6 | | 5.8E-4 | | 0.016 |
| Min | 5.55 | 86.7 | 5.55 | 69.7 | 5.55 | 69.7 |
| Max | 1720 | 1660 | 1720 | 1660 | 1720 | 1160 |
| n (Samp) | 89 | 11 | 89 | 11 | 89 | 7 |
| n (Patient) | 89 | 11 | 89 | 11 | 89 | 7 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.82 | 0.75 | 0.86 | 0.76 | 0.68 | 0.82 | 0.76 | nd | 0.79 |
| SE | 0.064 | 0.10 | 0.072 | 0.071 | 0.11 | 0.080 | 0.095 | nd | 0.10 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p | 5.2E-7 | 0.016 | 3.9E-7 | 3.1E-4 | 0.096 | 8.3E-5 | 0.0061 | nd | 0.0048 |
| nCohort 1 | 110 | 180 | 89 | 110 | 180 | 89 | 110 | nd | 89 |
| nCohort 2 | 17 | 8 | 11 | 17 | 8 | 11 | 9 | nd | 7 |
| Cutoff 1 | 124 | 101 | 138 | 101 | 30.6 | 105 | 79.5 | nd | 87.6 |
| Sens 1 | 71% | 75% | 73% | 71% | 75% | 73% | 78% | nd | 71% |
| Spec 1 | 82% | 69% | 80% | 77% | 32% | 75% | 71% | nd | 69% |
| Cutoff 2 | 101 | 34.3 | 124 | 66.9 | 19.2 | 101 | 66.9 | nd | 79.5 |
| Sens 2 | 82% | 88% | 82% | 82% | 88% | 82% | 89% | nd | 86% |
| Spec 2 | 77% | 35% | 78% | 62% | 19% | 74% | 62% | nd | 67% |
| Cutoff 3 | 33.6 | 15.7 | 108 | 19.2 | 15.7 | 87.6 | 15.0 | nd | 66.9 |
| Sens 3 | 94% | 100% | 91% | 94% | 100% | 91% | 100% | nd | 100% |
| Spec 3 | 38% | 12% | 76% | 21% | 12% | 69% | 14% | nd | 61% |
| Cutoff 4 | 78.9 | 105 | 97.8 | 78.9 | 105 | 97.8 | 78.9 | nd | 97.8 |
| Sens 4 | 88% | 62% | 91% | 76% | 62% | 82% | 78% | nd | 57% |
| Spec 4 | 70% | 70% | 71% | 70% | 70% | 71% | 70% | nd | 71% |
| Cutoff 5 | 112 | 150 | 146 | 112 | 150 | 146 | 112 | nd | 146 |
| Sens 5 | 71% | 62% | 64% | 59% | 62% | 45% | 56% | nd | 43% |
| Spec 5 | 80% | 80% | 81% | 80% | 80% | 81% | 80% | nd | 81% |
| Cutoff 6 | 212 | 315 | 323 | 212 | 315 | 323 | 212 | nd | 323 |
| Sens 6 | 53% | 50% | 36% | 41% | 50% | 27% | 44% | nd | 29% |
| Spec 6 | 90% | 90% | 91% | 90% | 90% | 91% | 90% | nd | 91% |
| OR Quart 2 | 0.97 | 1.0 | >0 | 0.47 | 0.49 | >0 | 0 | nd | >0 |
| p Value | 0.98 | 1.0 | <na | 0.54 | 0.56 | <na | na | nd | <na |
| 95% CI of | 0.058 | 0.061 | >na | 0.040 | 0.043 | >na | na | nd | >na |
| OR Quart2 | 16 | 16 | na | 5.4 | 5.6 | na | na | nd | na |
| OR Quart 3 | 3.1 | 1.0 | >4.8 | 2.1 | 0 | >6.2 | 3.1 | nd | >4.8 |
| p Value | 0.34 | 1.0 | <0.18 | 0.42 | na | <0.11 | 0.34 | nd | <0.18 |
| 95% CI of | 0.30 | 0.061 | >0.49 | 0.35 | na | >0.67 | 0.30 | nd | >0.50 |
| OR Quart3 | 32 | 16 | na | 12 | na | na | 32 | nd | na |
| OR Quart 4 | 18 | 5.5 | >9.7 | 6.6 | 2.7 | >7.9 | 5.6 | nd | >3.4 |
| p Value | 0.0075 | 0.13 | <0.041 | 0.022 | 0.25 | <0.066 | 0.13 | nd | <0.30 |
| 95% CI of | 2.2 | 0.61 | >1.1 | 1.3 | 0.49 | >0.88 | 0.61 | nd | >0.33 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart4 | 150 | 49 | na | 33 | 15 | na | 51 | nd | na |

**Mucin-16**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.17 | 8.40 | 1.17 | 7.52 | nd | nd |
| Average | 3.77 | 31.2 | 3.77 | 30.6 | nd | nd |
| Stdev | 15.1 | 67.4 | 15.1 | 67.6 | nd | nd |
| D(t-test) | | 0.0035 | | 0.0043 | nd | nd |
| Min | 0.117 | 2.02 | 0.117 | 2.02 | nd | nd |
| Max | 131 | 198 | 131 | 198 | nd | nd |
| n (Samp) | 75 | 8 | 75 | 8 | nd | nd |
| n (Patient) | 75 | 8 | 75 | 8 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.36 | 7.47 | 1.36 | 7.47 | nd | nd |
| Average | 4.38 | 38.3 | 4.38 | 38.3 | nd | nd |
| Stdev | 16.6 | 78.2 | 16.6 | 78.2 | nd | nd |
| D(t-test) | | 0.0042 | | 0.0042 | nd | nd |
| Min | 0.122 | 2.02 | 0.122 | 2.02 | nd | nd |
| Max | 131 | 198 | 131 | 198 | nd | nd |
| n (Samp) | 62 | 6 | 62 | 6 | nd | nd |
| n (Patient) | 62 | 6 | 62 | 6 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.89 | nd | 0.85 | 0.88 | nd | 0.85 | nd | nd | nd |
| SE | 0.079 | nd | 0.100 | 0.080 | nd | 0.100 | nd | nd | nd |
| p | 9.8E-7 | nd | 3.7E-4 | 1.6E-6 | nd | 3.7E-4 | nd | nd | nd |
| nCohort 1 | 75 | nd | 62 | 75 | nd | 62 | nd | nd | nd |
| nCohort 2 | 8 | nd | 6 | 8 | nd | 6 | nd | nd | nd |
| Cutoff 1 | 4.24 | nd | 2.09 | 4.24 | nd | 2.09 | nd | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 1 | 75% | nd | 83% | 75% | nd | 83% | nd | nd | nd |
| Spec 1 | 88% | nd | 68% | 88% | nd | 68% | nd | nd | nd |
| Cutoff 2 | 2.09 | nd | 2.09 | 2.09 | nd | 2.09 | nd | nd | nd |
| Sens 2 | 88% | nd | 83% | 88% | nd | 83% | nd | nd | nd |
| Spec 2 | 69% | nd | 68% | 69% | nd | 68% | nd | nd | nd |
| Cutoff 3 | 1.96 | nd | 1.96 | 1.96 | nd | 1.96 | nd | nd | nd |
| Sens 3 | 100% | nd | 100% | 100% | nd | 100% | nd | nd | nd |
| Spec 3 | 67% | nd | 66% | 67% | nd | 66% | nd | nd | nd |
| Cutoff 4 | 2.20 | nd | 2.56 | 2.20 | nd | 2.56 | nd | nd | nd |
| Sens 4 | 75% | nd | 67% | 75% | nd | 67% | nd | nd | nd |
| Spec 4 | 72% | nd | 71% | 72% | nd | 71% | nd | nd | nd |
| Cutoff 5 | 2.98 | nd | 3.41 | 2.98 | nd | 3.41 | nd | nd | nd |
| Sens 5 | 75% | nd | 67% | 75% | nd | 67% | nd | nd | nd |
| Spec 5 | 80% | nd | 81% | 80% | nd | 81% | nd | nd | nd |
| Cutoff 6 | 5.21 | nd | 6.93 | 5.21 | nd | 6.93 | nd | nd | nd |
| Sens 6 | 62% | nd | 50% | 62% | nd | 50% | nd | nd | nd |
| Spec 6 | 91% | nd | 90% | 91% | nd | 90% | nd | nd | nd |
| OR Quart 2 | >0 | nd | >0 | >0 | nd | >0 | nd | nd | nd |
| p Value | <na | nd | <na | <na | nd | <na | nd | nd | nd |
| 95% CI of | >na | nd | >na | >na | nd | >na | nd | nd | nd |
| OR Quart2 | na | nd | na | na | nd | na | nd | nd | nd |
| OR Quart 3 | >2.1 | nd | >2.3 | >2.1 | nd | >2.3 | nd | nd | nd |
| p Value | <0.56 | nd | <0.52 | <0.56 | nd | <0.52 | nd | nd | nd |
| 95% CI of | >0.18 | nd | >0.19 | >0.18 | nd | >0.19 | nd | nd | nd |
| OR Quart3 | na | nd | na | na | nd | na | nd | nd | nd |
| OR Quart 4 | 4>8.0 | nd | >5.2 | >8.0 | nd | >5.2 | nd | nd | nd |
| p Value | <0.066 | nd | <0.16 | <0.066 | nd | <0.16 | nd | nd | nd |
| 95% CI of | >0.87 | nd | >0.52 | >0.87 | nd | >0.52 | nd | nd | nd |
| OR Quart4 | na | nd | na | na | nd | na | nd | nd | nd |

(continued)

| Tumor necrosis factor receptor superfamily member 10B | | | | | | | |
|---|---|---|---|---|---|---|---|
| sCr or UO | 0hr prior to AKI stage | | 24hr prior to AKI stage | | 48hr prior to AKI stage | | |
| | Cohort 1 | Cohort 2 | Cohort 1 | Cohort 2 | Cohort 1 | Cohort 2 | |
| Median | 1.00E-9 | 0.0138 | 1.00E-9 | 0.0129 | nd | nd | |
| Average | 0.00697 | 0.0265 | 0.00697 | 0.0259 | nd | nd | |
| Stdev | 0.0202 | 0.0355 | 0.0202 | 0.0359 | nd | nd | |
| D(t-test) | | 0.020 | | 0.024 | nd | nd | |
| Min | 1.00E-9 | 1.00E-9 | 1.00E-9 | 1.00E-9 | nd | nd | |
| Max | 0.114 | 0.108 | 0.114 | 0.108 | nd | nd | |
| n (Samp) | 72 | 8 | 72 | 8 | nd | nd | |
| n (Patient) | 72 | 8 | 72 | 8 | nd | nd | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or -UO | sCr only | UO only | sCr or UO | sCr only | UO only |
| AUC | 0.81 | nd | nd | 0.79 | nd | nd | nd | nd | nd |
| SE | 0.095 | nd | nd | 0.098 | nd | nd | nd | nd | nd |
| p | 0.0012 | nd | nd | 0.0031 | nd | nd | nd | nd | nd |
| nCohort 1 | 72 | nd | nd | 72 | nd | nd | nd | nd | nd |
| nCohort 2 | 8 | nd | nd | 8 | nd | nd | nd | nd | nd |
| Cutoff 1 | 0.00462 | nd | nd | 0.00438 | nd | nd | nd | nd | nd |
| Sens 1 | 75% | nd | nd | 75% | nd | nd | nd | nd | nd |
| Spec 1 | 79% | nd | nd | 79% | nd | nd | nd | nd | nd |
| Cutoff 2 | 0.00438 | nd | nd | 0.00207 | nd | nd | nd | nd | nd |
| Sens 2 | 88% | nd | nd | 88% | nd | nd | nd | nd | nd |
| Spec 2 | 79% | nd | nd | 65% | nd | nd | nd | nd | nd |
| Cutoff 3 | 0 | nd | nd | 0 | nd | nd | nd | nd | nd |
| Sens 3 | 100% | nd | nd | 100% | nd | nd | nd | nd | nd |
| Spec 3 | 0% | nd | nd | 0% | nd | nd | nd | nd | nd |
| Cutoff 4 | 0.00274 | nd | nd | 0.00274 | nd | nd | nd | nd | nd |
| Sens 4 | 88% | nd | nd | 75% | nd | nd | nd | nd | nd |
| Spec 4 | 71% | nd | nd | 71% | nd | nd | nd | nd | nd |
| Cutoff 5 | 0.00507 | nd | nd | 0.00507 | nd | nd | nd | nd | nd |
| Sens 5 | 62% | nd | nd | 50% | nd | nd | nd | nd | nd |
| Spec 5 | 81% | nd | nd | 81% | nd | nd | nd | nd | nd |
| Cutoff 6 | 0.0124 | nd | nd | 0.0124 | nd | nd | nd | nd | nd |
| Sens 6 | 50% | nd | nd | 50% | nd | nd | nd | nd | nd |
| Spec 6 | 90% | nd | nd | 90% | nd | nd | nd | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or -UO | sCr only | UO only | sCr or UO | sCr only | UO only |
| OR Quart 2 | >1.1 | nd | nd | >1.1 | nd | nd | nd | nd | nd |
| p Value | <0.97 | nd | nd | <0.97 | nd | nd | nd | nd | nd |
| 95% CI of OR Quart2 | >0.061 | nd | nd | >0.061 | nd | nd | nd | nd | nd |
| | na | nd | nd | na | nd | nd | nd | nd | nd |
| OR Quart 3 | >2.2 | nd | nd | >2.2 | nd | nd | nd | nd | nd |
| p Value | <0.53 | nd | nd | <0.53 | nd | nd | nd | nd | nd |
| 95% CI of OR Quart3 | >0.19 | nd | nd | >0.19 | nd | nd | nd | nd | nd |
| | na | nd | nd | na | nd | nd | nd | nd | nd |
| OR Quart 4 | >6.7 | nd | nd | >6.7 | nd | nd | nd | nd | nd |
| p Value | <0.098 | nd | nd | <0.098 | nd | nd | nd | nd | nd |
| 95% CI of OR Quart4 | >0.70 | nd | nd | >0.70 | nd | nd | nd | nd | nd |
| | na | nd | nd | na | nd | nd | nd | nd | nd |

## Claims

1. A method for evaluating renal status in a subject, comprising:

   performing one or more assays configured to detect one or more biomarkers, in a body fluid sample obtained from the subject,to provide an assay result, the one or more biomarkers comprising Cellular tumor antigen p53 and optionally further comprising Tumor necrosis factor receptor superfamily member 10B, Cadherin-16, Caspase-9, Bcl2 antagonist of cell death, Caspase-1, Cadherin-1, Poly [ADP-ribose] polymerase 1, Cyclin-dependent kinase inhibitor 1, Cadherin-5, Myoglobin, Apolipoprotein A-II, Mucin-16, and/or Carcinoembryonic antigen-related cell adhesion molecule 5, and correlating the assay result(s) to the renal status of the subject.

2. A method according to claim 1, wherein said correlation step comprises

   (i) correlating the assay result(s) to one or more of risk stratification, diagnosis, staging, classifying and monitoring of the renal status of the subject or
   (ii) assigning a likelihood of one or more future changes in renal status to the subject based on the assay result(s).

3. A method according to claim 1, wherein said one or more future changes in renal status comprise one or more of a future injury to renal function, future reduced renal function, future improvement in renal function, and future acute renal failure (ARF).

4. A method according to claim 1, wherein the subject is not in acute renal failure.

5. A method according to claim 1, wherein the subject has not experienced a 1.5-fold or greater increase in serum creatinine over a baseline value determined prior to the time at which the body fluid sample is obtained, or

wherein the subject has a urine output of at least 0.5 ml/kg/hr over the 12 hours preceding the time at which the body fluid sample is obtained, or
wherein the subject has not experienced an increase of 0.3 mg/dL or greater in serum creatinine over a baseline value determined prior to the time at which the body fluid sample is obtained, or
wherein the subject (i) has not experienced a 1.5-fold or greater increase in serum creatinine over a baseline value determined prior to the time at which the body fluid sample is obtained, (ii) has a urine output of at least 0.5 ml/kg/hr over the 12 hours preceding the time at which the body fluid sample is obtained, and (iii) has not experienced an increase of 0.3 mg/dL or greater in serum creatinine over a baseline value determined prior to the time at which the body fluid sample is obtained.

6. A method according to claim 1, wherein the subject is in RIFLE stage 0 or R, wherein the subject is optionally in RIFLE stage 0, and said correlating step comprises assigning a likelihood that the subject will reach RIFLE stage R, I or F within 72 hours, within 48 hours or within 24 hours, or
wherein the subject is optionally in RIFLE stage 0 or R, and said correlating step comprises assigning a likelihood that the subject will reach RIFLE stage I or F within 72 hours, within 48 hours or within 24 hours,
wherein the subject is optionally in RIFLE stage 0, and said correlating step comprises assigning a likelihood that the subject will reach RIFLE stage F within 72 hours, within 48 hours or within 24 hours, or
wherein the subject is optionally in RIFLE stage R, and said correlating step comprises assigning a likelihood that the subject will reach RIFLE stage I or F within 72 hours, within 48 hours or within 24 hours.

7. A method according to claim 1, wherein the subject is in RIFLE stage 0, R, or I, and said correlating step comprises assigning a likelihood that the subject will reach RIFLE stage F within 72 hours,
wherein the subject is optionally in RIFLE stage I, and said correlating step comprises assigning a likelihood that the subject will reach RIFLE stage F within 72 hours, within 48 hours or within 24 hours.

8. A method according to claim 1, wherein said assay result(s) comprise a measured urine or plasma concentration of Cellular tumor antigen p53 and optionally one or more of:

a measured urine or plasma concentration of Tumor necrosis factor receptor superfamily member 10B,
a measured urine or plasma concentration of Cadherin-16,
a measured urine or plasma concentration of Caspase-9,
a measured urine or plasma concentration of Bcl2 antagonist of cell death,
a measured urine or plasma concentration of Caspase-1,
a measured urine or plasma concentration of Cadherin-1,
a measured urine or plasma concentration of Poly [ADP-ribose] polymerase 1,
a measured urine or plasma concentration of Cyclin-dependent kinase inhibitor 1,
a measured urine or plasma concentration of Cadherin-5,
a measured urine or plasma concentration of Myoglobin,
a measured urine or plasma concentration of Apolipoprotein A-II,
a measured urine or plasma concentration of Mucin-16, or
a measured urine or plasma concentration of Carcinoembryonic antigen-related cell adhesion molecule 5,
and said correlation step comprises comparing each measured concentration to a corresponding threshold concentration, and

(A) for a positive going marker, assigning an increased likelihood of progression to a worsening RIFLE stage to the subject, relative to the subject's current RIFLE stage, when the measured concentration is above the threshold, or assigning a decreased likelihood of progressing to a worsening RIFLE stage to the subject, relative to the subject's current RIFLE stage, when the measured concentration is below the threshold, or
for a negative going marker, assigning a decreased likelihood of progression to a worsening RIFLE stage to the subject, relative to the subject's current RIFLE stage, when the measured concentration is above the threshold, or assigning an increased likelihood of progressing to a worsening RIFLE stage to the subject, relative to the subject's current RIFLE stage, when the measured concentration is below the threshold,
(B) for a positive going marker, assigning an increased likelihood of progressing to a need for renal replacement therapy to the subject when the measured concentration is above the threshold, or assigning a decreased likelihood of progressing to a need for renal replacement therapy when the measured concentration is below the threshold, or
for a negative going marker, assigning a decreased likelihood of progressing to a need for renal replacement therapy to the subject when the measured concentration is above the threshold, or assigning an increased likelihood of progressing to a need for renal replacement therapy when the measured concentration is below the threshold,

(C) where the subject is not in acute renal failure,

for a positive going marker, assigning an increased likelihood of progressing to acute renal failure when the measured concentration is above the threshold, or assigning a decreased likelihood of progressing to acute renal failure to the subject when the measured concentration is below the threshold, or

for a negative going marker, assigning a decreased likelihood of progressing to acute renal failure when the measured concentration is above the threshold, or assigning an increased likelihood of progressing to acute renal failure to the subject when the measured concentration is below the threshold,

(D) where the subject is wherein the subject is in RIFLE stage 0, and the correlating step comprises assigning a likelihood that the subject will reach RIFLE stage R, I or F within 72 hours,

for a positive going marker, assigning an increased likelihood of progressing to RIFLE stage R, I or F to the subject, when the measured concentration is above the threshold, or assigning a decreased likelihood of progressing to RIFLE stage R, I or F to the subject when the measured concentration is below the threshold, or

for a negative going marker, assigning a decreased likelihood of progressing to RIFLE stage R, I or F to the subject, when the measured concentration is above the threshold, or assigning an increased likelihood of progressing to RIFLE stage R, I or F to the subject when the measured concentration is below the threshold,

(E) where the subject is in RIFLE stage 0 or R, and the correlating step comprises assigning a likelihood that the subject will reach RIFLE stage I or F within 72 hours, for a positive going marker, assigning an increased likelihood of progressing to RIFLE stage I or F to the subject, when the measured concentration is above the threshold, or assigning a decreased likelihood of progressing to RIFLE stage I or F to the subject when the measured concentration is below the threshold, or

for a negative going marker, assigning a decreased likelihood of progressing to RIFLE stage I or F to the subject, when the measured concentration is above the threshold, or assigning an increased likelihood of progressing to RIFLE stage I or F to the subject when the measured concentration is below the threshold,

(F) where the subject is in RIFLE stage 0, and the correlating step comprises assigning a likelihood that the subject will reach RIFLE stage F within 72 hours,

for a positive going marker, assigning an increased likelihood of progressing to RIFLE stage F to the subject, when the measured concentration is above the threshold, or assigning a decreased likelihood of progressing to RIFLE stage I or F to the subject when the measured concentration is below the threshold, or

for a negative going marker, assigning a decreased likelihood of progressing to RIFLE stage F to the subject, when the measured concentration is above the threshold, or assigning an increased likelihood of progressing to RIFLE stage I or F to the subject when the measured concentration is below the threshold,

(G) where the subject is in RIFLE stage R, and the correlating step comprises assigning a likelihood that the subject will reach RIFLE stage I or F within 72 hours,

for a positive going marker, assigning an increased likelihood of progressing to RIFLE stage F to the subject, when the measured concentration is above the threshold, or assigning a decreased likelihood of progressing to RIFLE stage I or F to the subject when the measured concentration is below the threshold, or

for a negative going marker, assigning a decreased likelihood of progressing to RIFLE stage F to the subject, when the measured concentration is above the threshold, or assigning an increased likelihood of progressing to RIFLE stage I or F to the subject when the measured concentration is below the threshold,

(H) where the subject is in RIFLE stage 0, R, or I, and the correlating step comprises assigning a likelihood that the subject will reach RIFLE stage F within 72 hours, for a positive going marker, assigning an increased likelihood of progressing to RIFLE stage F to the subject, when the measured concentration is above the threshold, or assigning a decreased likelihood of progressing to RIFLE stage F to the subject when the measured concentration is below the threshold, or

for a negative going marker, assigning a decreased likelihood of progressing to RIFLE stage F to the subject, when the measured concentration is above the threshold, or assigning an increased likelihood of progressing to RIFLE stage F to the subject when the measured concentration is below the threshold, or

(I) where the subject is in RIFLE stage I, and the correlating step comprises assigning a likelihood that the subject will reach RIFLE stage F within 72 hours,

for a positive going marker, assigning an increased likelihood of progressing to RIFLE stage F to the subject, when the measured concentration is above the threshold, or assigning a decreased likelihood of progressing to RIFLE stage F to the subject when the measured concentration is below the threshold, or

for a negative going marker, assigning a decreased likelihood of progressing to RIFLE stage F to the subject, when the measured concentration is above the threshold, or assigning an increased likelihood of progressing to RIFLE stage F to the subject when the measured concentration is below the threshold.

9. A method according to claim 1, wherein the subject is selected for evaluation of renal status (i) based on the pre-existence in the subject of one or more known risk factors for prerenal, intrinsic renal, or postrenal ARF, or based on an existing diagnosis of one or more of congestive heart failure, preeclampsia, eclampsia, diabetes mellitus, hypertension, coronary artery disease, proteinuria, renal insufficiency, glomerular filtration below the normal range, cirrhosis, serum creatinine above the normal range, sepsis, injury to renal function, reduced renal function, or ARF, or based on undergoing or having undergone major vascular surgery, coronary artery bypass, or other cardiac surgery, or based on exposure to NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin.

10. The use of Cellular tumor antigen p53 for the in-vitro diagnosis, risk stratification, prognosis, classifying and monitoring of renal status of a subject not receiving renal replacement therapy.

11. The use of one or more biomarkers comprising Cellular tumor antigen p53 and the one or more biomarkers optionally further comprising Tumor necrosis factor receptor superfamily member 10B, Cadherin-16, Caspase-9, Bcl2 antagonist of cell death, Caspase-1, Cadherin-1, Poly [ADP-ribose] polymerase 1, Cyclin-dependent kinase inhibitor 1, Cadherin-5, Myoglobin, Apolipoprotein A-II, Mucin-16, or Carcinoembryonic antigen-related cell adhesion molecule 5, for

    (i) the diagnosis, risk stratification, prognosis, classifying and monitoring of renal status of a subject not in acute renal failure or
    (ii) for assigning an increased likelihood of progressing to a worsening RIFLE stage to a subject, relative to the subject's current RIFLE stage.

**Patentansprüche**

1. Ein Verfahren zur Evaluierung des Nierenstatus eines Subjektes, umfassend:

    Durchführen einer oder mehrerer Untersuchung(en), die dafür konfiguriert sind, einen oder mehrere Biomarker in einer Körperflüssigkeitsprobe, die von dem Subjekt erhalten wurde, nachzuweisen, um ein Untersuchungsergebnis bereitzustellen, wobei der eine oder die mehreren Biomarker das zelluläre Tumor-Antigen p53 umfassen und optional weiterhin das Mitglied 10B der Tumor-Nekrose-Faktor-Rezeptor-Superfamilie, Cadherin-16, Caspase-9, den Bcl2-Antagonisten des Zelltodes, Caspase-1, Cadherin-1, Poly-[ADP-Ribose]-Polymerase 1, den Cyclin-abhängigen Kinase-Inhibitor 1, Cadherin-5, Myoglobin, Apolipoprotein A-II, Mucin-16 und/oder das Carcino-embryonale Antigen-verwandte Zelladhäsionsmolekül 5 umfassen, und
    Korrelieren des/der Untersuchungsergebnis(e) mit dem Nierenstatus des Subjektes.

2. Ein Verfahren nach Anspruch 1, wobei der Korrelierungsschritt umfasst:

    (i) Korrelieren des/der Untersuchungsergebnis(se) mit einem oder mehreren aus Risikostratifizierung, Diagnose, Einstufen, Klassifizieren und Überwachen des Nierenstatus des Subjektes oder
    (ii) Zuweisen einer Wahrscheinlichkeit von einer oder mehreren zukünftigen Veränderung(en) des Nierenstatus zum dem Subjekt, basierend auf dem/den Untersuchungsergebnis(sen).

3. Ein Verfahren nach Anspruch 1, wobei die eine oder die mehreren zukünftige(n) Veränderung(en) im Nierenstatus eines oder mehrere aus einer zukünftigen Verletzung der Nierenfunktion, zukünftiger reduzierter Nierenfunktion, zukünftiger Verbesserung der Nierenfunktion und zukünftiges akutes Nierenversagen (ARF) umfassen.

4. Ein Verfahren nach Anspruch 1, wobei das Subjekt kein akutes Nierenversagen hat.

5. Ein Verfahren nach Anspruch 1, wobei das Subjekt keine 1,5-fache oder größere Zunahme des Serumkreatinins über einen Basiswert, der vor dem Zeitpunkt, an dem die Körperflüssigkeitsprobe erhalten wurde, bestimmt wurde, erfahren hat, oder wobei das Subjekt eine Urinausscheidung von mindestens 0,5 ml/kg/Std. über 12 Stunden vor dem Zeitpunkt hat, an dem die Körperflüssigkeitsprobe erhalten wurde, oder wobei das Subjekt keine Zunahme von 0,3 mg/dL oder mehr des Serumkreatinins über einen Basiswert, der vor dem Zeitpunkt, an dem die Körperflüssigkeitsprobe erhalten wurde, bestimmt wurde, erfahren hat, oder

wobei das Subjekt (i) keine 1,5-fache oder größere Zunahme des Serumkreatinins über einen Basiswert, der vor dem Zeitpunkt, an dem die Körperflüssigkeitsprobe erhalten wurde, bestimmt wurde, erfahren hat, (ii) eine Urinausscheidung von mindestens 0,5 ml/kg/Std. über 12 Stunden vor dem Zeitpunkt hat, an dem die Körperflüssigkeitsprobe erhalten wurde, und (iii) keine Zunahme von 0,3 mg/dL oder mehr des Serumkreatinins über einen Basiswert, der vor dem Zeitpunkt, an dem die Körperflüssigkeitsprobe erhalten wurde, bestimmt wurde, erfahren hat.

6. Ein Verfahren nach Anspruch 1, wobei das Subjekt in RIFLE-Stadium 0 oder R ist, wobei das Subjekt optional in RIFLE-Stadium 0 ist, und der Korrelierungsschritt das Zuweisen einer Wahrscheinlichkeit, dass das Subjekt RIFLE-Stadium R, I oder F innerhalb von 72 Stunden, innerhalb von 48 Stunden oder innerhalb von 24 Stunden erreichen wird, umfasst, oder

wobei das Subjekt optional in RIFLE-Stadium 0 oder R ist, und der Korrelierungsschritt das Zuweisen einer Wahrscheinlichkeit, dass das Subjekt RIFLE-Stadium I oder F innerhalb von 72 Stunden, innerhalb von 48 Stunden, oder innerhalb von 24 Stunden erreichen wird, umfasst,

wobei das Subjekt optional in RIFLE-Stadium 0 ist, und der Korrelierungsschritt das Zuweisen einer Wahrscheinlichkeit, dass das Subjekt RIFLE-Stadium F innerhalb von 72 Stunden, innerhalb von 48 Stunden, oder innerhalb von 24 Stunden erreichen wird, umfasst, oder

wobei das Subjekt optional in RIFLE-Stadium R ist, und der Korrelierungsschritt das Zuweisen einer Wahrscheinlichkeit, dass das Subjekt RIFLE-Stadium I oder F innerhalb von 72 Stunden, innerhalb von 48 Stunden oder innerhalb von 24 Stunden erreichen wird, umfasst.

7. Ein Verfahren nach Anspruch 1, wobei das Subjekt in RIFLE-Stadium 0, R oder I ist, und der Korrelierungsschritt das Zuweisen einer Wahrscheinlichkeit, dass das Subjekt RIFLE-Stadium F innerhalb von 72 Stunden erreichen wird, umfasst,

wobei das Subjekt optional in RIFLE-Stadium I ist, und der Korrelierungsschritt das Zuweisen einer Wahrscheinlichkeit, dass das Subjekt RIFLE-Stadium F innerhalb von 72 Stunden, innerhalb von 48 Stunden oder innerhalb von 24 Stunden erreichen wird, umfasst.

8. Ein Verfahren nach Anspruch 1, wobei das/die Untersuchungsergebnis(se) eine gemessene Urin- oder Plasmakonzentration des zellulären Tumorantigens p53 und optional eines oder mehrere der folgenden umfassen:

eine gemessene Urin- oder Plasmakonzentration des Mitglieds 10B der Tumor-Nekrose-Faktor-Rezeptor-Superfamilie,

eine gemessene Urin- oder Plasmakonzentration von Cadherin-16,

eine gemessene Urin- oder Plasmakonzentration von Caspase-9,

eine gemessene Urin- oder Plasmakonzentration des Bcl2- Antagonisten des Zelltods,

eine gemessene Urin- oder Plasmakonzentration von Caspase-1,

eine gemessene Urin- oder Plasmakonzentration von Cadherin-1,

eine gemessene Urin- oder Plasmakonzentration von Poly-[ADP-Ribose]-Polymerase 1,

eine gemessene Urin- oder Plasmakonzentration des Cyclin-abhängigen Kinase-Inhibitors 1,

eine gemessene Urin- oder Plasmakonzentration von Cadherin-5,

eine gemessene Urin- oder Plasmakonzentration von Myoglobin,

eine gemessene Urin- oder Plasma-Konzentration von Apolipoprotein A-II,

eine gemessene Urin- oder Plasmakonzentration von Mucin-16, oder

eine gemessene Urin- oder Plasmakonzentration des Carcino-embryonalen Antigen-verwandten Zelladhäsions-Moleküls 5,

und der Korrelierungsschritt das Vergleichen jeder gemessenen Konzentration mit einer entsprechenden Schwellenwertkonzentration umfasst, und

(A) bei einem positiv werdenden Marker, wenn die gemessene Konzentration über dem Schwellenwert liegt, das Zuweisen einer erhöhten Wahrscheinlichkeit einer Progression zu einer Verschlechterung des RIFLE-Stadiums zu dem Subjekt, im Vergleich zu dem gegenwärtigen RIFLE-Stadium des Subjektes, oder das Zuweisen einer verringerten Wahrscheinlichkeit einer Progression zu einer Verschlechterung des RIFLE-Stadiums zu dem Subjekt, im Vergleich zu dem gegenwärtigen RIFLE-Stadium des Subjektes, wenn die gemessene Konzentration unter dem Schwellenwert liegt, oder

bei einem negativ werdenden Marker, wenn die gemessene Konzentration über dem Schwellenwert liegt, das Zuweisen einer verringerten Wahrscheinlichkeit einer Progression zu einer Verschlechterung des RIFLE-Stadiums zu dem Subjekt, im Vergleich zu dem gegenwärtigen RIFLE-Stadium des Subjektes, oder das Zuweisen einer erhöhten Wahrscheinlichkeit einer Progression zu einer Verschlechterung des RIFLE-Stadiums zu dem Subjekt, im Vergleich zu dem gegenwärtigen RIFLE-Stadium des Subjektes, wenn die gemessene Konzentration unter dem Schwellenwert liegt,

(B) bei einem positiv werdenden Marker, wenn die gemessene Konzentration über dem Schwellenwert liegt, das Zuweisen einer erhöhten Wahrscheinlichkeit eines Fortschreitens zu einem Bedarf einer Nierenersatztherapie zu dem Subjekt, oder das Zuweisen einer verringerten Wahrscheinlichkeit eines Fortschreitens zu einem Bedarf einer Nierenersatztherapie, wenn die gemessene Konzentration unter dem Schwellenwert liegt, oder

bei einem negativ werdenden Marker, wenn die gemessene Konzentration über dem Schwellenwert liegt, das Zuweisen einer verringerten Wahrscheinlichkeit eines Fortschreitens zu einem Bedarf einer Nierenersatztherapie zu dem Subjekt, oder das Zuweisen einer erhöhten Wahrscheinlichkeit eines Fortschreitens zu einem Bedarf einer Nierenersatztherapie, wenn die gemessene Konzentration unter dem Schwellenwert liegt,

(C) wenn das Subjekt kein akutes Nierenversagen hat,

bei einem positiv werdenden Marker, wenn die gemessene Konzentration über dem Schwellenwert liegt, das Zuweisen einer erhöhten Wahrscheinlichkeit des Fortschreitens zu akutem Nierenversagen, oder das Zuweisen einer verringerten Wahrscheinlichkeit des Fortschreitens zu akutem Nierenversagen zu dem Subjekt, wenn die gemessene Konzentration unter dem Schwellenwert liegt, oder

bei einem negativ werdenden Marker, wenn die gemessene Konzentration über dem Schwellenwert liegt, das Zuweisen einer verringerten Wahrscheinlichkeit des Fortschreitens zu akutem Nierenversagen, oder das Zuweisen einer erhöhten Wahrscheinlichkeit des Fortschreitens zu akutem Nierenversagen zu dem Subjekt, wenn die gemessene Konzentration unter dem Schwellenwert liegt,

(D) wenn das Subjekt in RIFLE-Stadium 0 ist und der Korrelierungsschritt das Zuweisen einer Wahrscheinlichkeit umfasst, dass das Subjekt RIFLE-Stadium R, I oder F innerhalb von 72 Stunden erreichen wird,

bei einem positiv werdenden Marker, wenn die gemessene Konzentration über dem Schwellenwert liegt, das Zuweisen einer erhöhten Wahrscheinlichkeit eines Fortschreitens zu RIFLE-Stadium R, I oder F zu dem Subjekt, oder das Zuweisen einer verringerten Wahrscheinlichkeit eines Fortschreitens zu RIFLE-Stadium R, I oder F zu dem Subjekt, wenn die gemessene Konzentration unter dem Schwellenwert liegt, oder

bei einem negativ werdenden Marker, wenn die gemessene Konzentration über dem Schwellenwert liegt, das Zuweisen einer verringerten Wahrscheinlichkeit eines Fortschreitens zu RIFLE-Stadium R, I oder F zu dem Subjekt, oder das Zuweisen einer erhöhten Wahrscheinlichkeit eines Fortschreitens zu RIFLE-Stadium R, I oder F zu dem Subjekt, wenn die gemessene Konzentration unter dem Schwellenwert liegt,

(E) wenn das Subjekt in RIFLE-Stadium 0 oder R ist, und der Korrelierungsschritt das Zuweisen einer Wahrscheinlichkeit umfasst, dass das Subjekt RIFLE-Stadium I oder F innerhalb von 72 Stunden erreichen wird,

bei einem positiv werdenden Marker, wenn die gemessene Konzentration über dem Schwellenwert liegt, das Zuweisen einer erhöhten Wahrscheinlichkeit eines Fortschreitens zu RIFLE-Stadium I oder F zu dem Subjekt, oder das Zuweisen einer verringerten Wahrscheinlichkeit eines Fortschreitens zu RIFLE-Stadium I oder F zu dem Subjekt, wenn die gemessene Konzentration unter dem Schwellenwert liegt, oder

bei einem negativ werdenden Marker, wenn die gemessene Konzentration über dem Schwellenwert liegt, das Zuweisen einer verringerten Wahrscheinlichkeit eines Fortschreitens zu RIFLE-Stadium I oder F zu dem Subjekt, oder das Zuweisen einer erhöhten Wahrscheinlichkeit eines Fortschreitens zu RIFLE-Stadium I oder F zu dem Subjekt, wenn die gemessene Konzentration unter dem Schwellenwert liegt,

(F) wenn das Subjekt in RIFLE-Stadium 0 ist und der Korrelierungsschritt das Zuweisen einer Wahrscheinlichkeit umfasst, dass das Subjekt RIFLE-Stadium F innerhalb von 72 Stunden erreichen wird,

bei einem positiv werdenden Marker, wenn die gemessene Konzentration über dem Schwellenwert liegt, das Zuweisen einer erhöhten Wahrscheinlichkeit eines Fortschreitens zu RIFLE-Stadium F zu dem Subjekt, oder das Zuweisen einer verringerten Wahrscheinlichkeit eines Fortschreitens zu RIFLE-Stadium I oder F zu dem Subjekt, wenn die gemessene Konzentration unter dem Schwellenwert liegt, oder

bei einem negativ werdenden Marker, wenn die gemessene Konzentration über dem Schwellenwert liegt, das Zuweisen einer verringerten Wahrscheinlichkeit eines Fortschreitens zu RIFLE-Stadium F zu dem Subjekt, oder das Zuweisen einer erhöhten Wahrscheinlichkeit eines Fortschreitens zu RIFLE-Stadium I oder F zu dem Subjekt, wenn die gemessene Konzentration unter dem Schwellenwert liegt,

(G) wenn das Subjekt in RIFLE-Stadium R ist und der Korrelierungsschritt das Zuweisen einer Wahrscheinlichkeit umfasst, dass das Subjekt RIFLE-Stadium I oder F innerhalb von 72 Stunden erreichen wird,

bei einem positiv werdenden Marker, wenn die gemessene Konzentration über dem Schwellenwert liegt, das Zuweisen einer erhöhten Wahrscheinlichkeit eines Fortschreitens zu RIFLE-Stadium F zu dem Subjekt, oder das Zuweisen einer verringerten Wahrscheinlichkeit einer Fortschreitens zu RIFLE-Stadium I oder F zu dem Subjekt, wenn die gemessene Konzentration unter dem Schwellenwert liegt, oder

bei einem negativ werdenden Marker, wenn die gemessene Konzentration über dem Schwellenwert liegt, das Zuweisen einer verringerten Wahrscheinlichkeit eines Fortschreitens zu RIFLE-Stadium F zu dem Subjekt, oder das Zuweisen einer erhöhten Wahrscheinlichkeit eines Fortschreitens zu RIFLE-Stadium I oder F zu dem Subjekt, wenn die gemessene Konzentration unter dem Schwellenwert liegt,

(H) wenn das Subjekt in RIFLE-Stadium 0, R oder I ist und der Korrelierungsschritt das Zuweisen einer Wahrscheinlichkeit umfasst, dass das Subjekt das RIFLE-Stadium F innerhalb von 72 Stunden erreichen wird,

bei einem positiv werdenden Marker, wenn die gemessene Konzentration über dem Schwellenwert liegt, das Zuweisen einer erhöhten Wahrscheinlichkeit eines Fortschreitens zu RIFLE- Stadium F zu dem Subjekt, oder das Zuweisen einer verringerten Wahrscheinlichkeit eines Fortschreitens zu RIFLE-Stadium F zu dem Subjekt, wenn die gemessene Konzentration unter dem Schwellenwert liegt, oder

bei einem negativ werdenden Marker, wenn die gemessene Konzentration über dem Schwellenwert liegt, das Zuweisen einer verringerten Wahrscheinlichkeit eines Fortschreitens zu RIFLE-Stadium F zu dem Subjekt, oder das Zuweisen einer erhöhten Wahrscheinlichkeit eines Fortschreitens zu RIFLE-Stadium F zu dem Subjekt, wenn die gemessene Konzentration unter dem Schwellenwert liegt, oder

(I) wenn das Subjekt in RIFLE-Stadium I ist und der Korrelierungsschritt das Zuweisen einer Wahrscheinlichkeit umfasst, dass das Subjekt RIFLE-Stadium F innerhalb von 72 Stunden erreichen wird,

bei einem positiv werdenden Marker, wenn die gemessene Konzentration über dem Schwellenwert liegt, das Zuweisen einer erhöhten Wahrscheinlichkeit eines Fortschreitens zu RIFLE-Stadium F zu dem Subjekt, oder das Zuweisen einer verringerten Wahrscheinlichkeit eines Fortschreitens zu RIFLE-Stadium F zu dem Subjekt, wenn die gemessene Konzentration unter dem Schwellenwert liegt, oder

bei einem negativ werdenden Marker, wenn die gemessene Konzentration über dem Schwellenwert liegt, das Zuweisen einer verringerten Wahrscheinlichkeit eines Fortschreitens zu RIFLE-Stadium F zu dem Subjekt, oder das Zuweisen einer erhöhten Wahrscheinlichkeit eines Fortschreitens zu RIFLE-Stadium F zu dem Subjekt, wenn die gemessene Konzentration unter dem Schwellenwert liegt.

9. Ein Verfahren nach Anspruch 1, wobei das Subjekt zur Evaluation des Nierenstatus ausgewählt wird (i) auf der Grundlage des vorherigen Bestehens von einem oder mehreren bekannten Risikofaktoren für prärenales, immanent-renales oder postrenales ARF bei dem Subjekt, oder

auf der Grundlage einer bestehenden Diagnose von einem oder mehreren aus kongestiver Herzinsuffizienz, Präeklampsie, Eklampsie, Diabetes mellitus, Hypertonie, koronarer Herzkrankheit, Proteinurie, Niereninsuffizienz, glomerulärer Filtration unterhalb des normalen Bereichs, Zirrhose, Serumkreatinin oberhalb des normalen Bereichs, Sepsis, Verletzung der Nierenfunktion, verringerter Nierenfunktion oder ARF, oder auf der Grundlage eines laufenden oder vorangegangenen größeren gefäßchirurgischen Eingriffs, Koronararterien-Bypassoperation oder einer anderen Herzoperation, oder auf der Grundlage einer Exposition gegenüber NSAIDs, Cyclosporinen, Tacrolimus, Aminoglycosiden, Foscarnet, Ethylenglykol, Hämoglobin, Myoglobin, Ifosfamid, Schwermetallen, Methotrexat, röntgendichten Kontrastmitteln oder Streptozotocin.

10. Die Verwendung von zellulärem Tumor-Antigen p53 zur in-vitro Diagnose, Risikostratifizierung, Prognose, Klassifizierung und Überwachung des Nierenstatus eines Subjektes, das keine Nierenersatztherapie erhält.

11. Die Verwendung von einem oder mehreren Biomarkern umfassend zelluläres Tumor-Antigen p53 und die einen oder mehreren Biomarker optional weiterhin umfassend Mitglied 10B der Tumor-Nekrose-Faktor-Rezeptor-Superfamilie, Cadherin-16, Caspase-9, den Bcl2-Antagonisten des Zelltodes, Caspase-1, Cadherin-1, Poly-[ADP-Ribose]-Polymerase 1, den Cyclin-abhängigen Kinase-Inhibitor 1, Cadherin-5, Myoglobin, Apolipoprotein A-II, Mucin-16 oder das Carcino-embryonale Antigen-verwandte Zelladhäsions-Molekül 5, für

(i) die Diagnose, Risikostratifizierung, Prognose, das Klassifizieren und Überwachen des Nierenstatus eines Subjektes, das kein akutes Nierenversagen hat, oder

(ii) das Zuweisen einer erhöhten Wahrscheinlichkeit eines Fortschreitens zu einer Verschlechterung des RIFLE-Stadiums zu einem Subjekt, im Vergleich zu dem gegenwärtigen RIFLE-Stadium des Subjektes.

## Revendications

1. Procédé d'évaluation de l'état rénal chez un sujet, comprenant: la réalisation d'un ou de plusieurs analyses configurés pour détecter un ou plusieurs marqueurs biologiques sur un échantillon de fluide corporel obtenu d'un sujet pour fournir un résultat d'analyse, l'un ou les plusieurs marqueurs biologiques comprenant l'antigène p53 des tumeurs cellulaires, et facultativement comprenant en outre le membre 10B de la superfamille des récepteurs du facteur de nécrose tumorale, la cadhérine-16, la caspase-9, l'antagoniste de Bcl2 de l'apoptose cellulaire, la caspase-1, la cadhérine-1, la poly-[ADP-ribose]-polymérase 1, l'inhibiteur 1 de kinase dépendant de la cycline, la cadhérine-5, la myoglobine, l'apolipoprotéine A-II, la mucine-16 et/ou la molécule 5 d'adhésion cellulaire liée à l'antigène carcino-embryonnaire, et

la corrélation du/des résultat(s) d'analyse(s) à l'état rénal du sujet.

2. Procédé selon la revendication 1, dans lequel ladite étape de corrélation comprend:

    (i) la corrélation du/des résultat(s) d'analyse(s) à un ou plusieurs parmi une stratification des risques, un diagnostic, une stratification, une classification et une surveillance de l'état rénal du sujet ou
    (ii) l'assignation d'une probabilité d'un ou de plusieurs changements futurs de l'état rénal chez le sujet, en fonction du/des résultat(s) d'analyse(s).

3. Procédé selon la revendication 1, dans lequel lesdits un ou plusieurs changements futurs de l'état rénal comprennent un ou plusieurs parmi une lésion future de la fonction rénale, une fonction rénale réduite future, une amélioration future de la fonction rénale, et une insuffisance rénale aiguë (IRA) future.

4. Procédé selon la revendication 1, dans lequel le sujet n'est pas atteint d'une insuffisance rénale aiguë.

5. Procédé selon la revendication 1, dans lequel le sujet n'a pas connu d'augmentation d'au moins 1,5 fois ou plus de la créatinine sérique par rapport à une valeur de référence déterminée avant le moment auquel l'échantillon de fluide corporel est obtenu, ou
dans lequel le sujet a une production d'urine d'au moins 0,5 ml/kg/ heure sur les 12 heures qui précèdent le moment auquel l'échantillon de fluide corporel est obtenu, ou dans lequel le sujet n'a pas connu d'augmentation de 0,3 mg/dL ou plus de créatinine sérique par rapport à une valeur de référence déterminée avant le moment auquel l'échantillon de fluide corporel est obtenu, ou
dans lequel le sujet (i) n'a pas connu d'augmentation de 1,5 fois ou plus de la créatinine sérique par rapport à une valeur de référence déterminée avant le moment auquel l'échantillon de fluide corporel est obtenu, (ii) a une production d'urine d'au moins 0,5 ml/kg/ heure sur les 12 heures qui précèdent le moment auquel l'échantillon de fluide corporel est obtenu, et (iii) n'a pas connu d'augmentation de 0,3 mg/dL ou plus de créatinine sérique par rapport à une valeur de référence déterminée avant le moment auquel l'échantillon de fluide corporel est obtenu.

6. Procédé selon la revendication 1, dans lequel le sujet est dans la classe 0 ou R de RIFLE, le sujet étant facultativement dans la classe 0 de RIFLE, et ladite étape de corrélation comprenant l'assignation d'une probabilité que le sujet atteindra la classe R, I ou F de RIFLE dans les 72 heures, dans les 48 heures ou dans les 24 heures, ou
le sujet étant facultativement dans la classe 0 ou R de RIFLE, et ladite étape de corrélation comprenant l'assignation d'une probabilité que le sujet atteindra la classe I ou F de RIFLE dans les 72 heures, dans les 48 heures ou dans les 24 heures, ou
dans lequel le sujet est facultativement dans la classe 0 de RIFLE et ladite étape de corrélation comprenant l'assignation d'une probabilité que le sujet atteindra la classe F de RIFLE dans les 72 heures, dans les 48 heures ou dans les 24 heures, ou
le sujet étant facultativement dans la classe R de RIFLE, et ladite étape de corrélation comprenant l'assignation d'une probabilité que le sujet atteindra la classe I ou F de RIFLE dans les 72 heures, dans les 48 heures ou dans les 24 heures.

7. Procédé selon la revendication 1, dans lequel le sujet est dans la classe 0, R ou I de RIFLE, et ladite étape de corrélation comprenant l'assignation d'une probabilité que le sujet atteindra la classe F de RIFLE dans les 72 heures, le sujet étant facultativement dans la classe I de RIFLE, et ladite étape de corrélation comprenant l'assignation d'une probabilité que le sujet atteindra la classe F de RIFLE dans les 72 heures, dans les 48 heures ou dans les 24 heures.

8. Procédé selon la revendication 1, dans lequel le(s) dit(s) résultat(s) d'analyse(s) comprennent une concentration d'urine ou de plasma mesurée d'antigène p53 des tumeurs cellulaires, et facultativement une ou plusieurs parmi:

    une concentration d'urine ou de plasma mesurée du membre 10B de la superfamille des récepteurs du facteur de nécrose tumorale,

une concentration d'urine ou de plasma mesurée de cadhérine-16,

une concentration d'urine ou de plasma mesurée de caspase-9,

une concentration d'urine ou de plasma mesurée d'antagoniste de Bcl2 de l'apoptose cellulaire,

une concentration d'urine ou de plasma mesurée de caspase-1,

une concentration d'urine ou de plasma mesurée de cadhérine-1,

une concentration d'urine ou de plasma mesurée de poly-[ADP-ribose]-polymérase 1,

une concentration d'urine ou de plasma mesurée d'inhibiteur 1 de kinase dépendant de la cycline,

une concentration d'urine ou de plasma mesurée de cadhérine-5,

une concentration d'urine ou de plasma mesurée de myoglobine,

une concentration d'urine ou de plasma mesurée d'apolipoprotéine A-II,

une concentration d'urine ou de plasma mesurée de mucine-16, ou

une concentration d'urine ou de plasma mesurée de molécule 5 d'adhésion cellulaire liée à l'antigène carcino-embryonnaire,

et ladite étape de corrélation comprend la comparaison de chaque concentration mesurée à une concentration seuil correspondante, et

(A) pour un marqueur de sens positif, l'assignation d'une probabilité augmentée de progression du sujet vers une classe RIFLE plus grave, par rapport à la classe RIFLE actuelle du sujet, quand la concentration mesurée est supérieure au seuil, ou l'assignation d'une probabilité diminuée de progression du sujet vers une classe RIFLE plus grave, par rapport à la classe RIFLE actuelle du sujet, quand la concentration mesurée est inférieure au seuil, ou

pour un marqueur de sens négatif, l'assignation d'une probabilité diminuée de progression du sujet vers une classe RIFLE plus grave, par rapport à la classe RIFLE actuelle du sujet, quand la concentration mesurée est supérieure au seuil, ou l'assignation d'une probabilité augmentée de progression du sujet vers une classe RIFLE plus grave, par rapport à la classe RIFLE actuelle du sujet, quand la concentration mesurée est inférieure au seuil,

(B) pour un marqueur de sens positif, l'assignation d'une probabilité augmentée de progression vers un besoin de traitement rénal substitutif pour le sujet, quand la concentration mesurée est supérieure au seuil, ou l'assignation d'une probabilité diminuée de progression vers un besoin de traitement rénal substitutif, quand la concentration mesurée est inférieure au seuil, ou

pour un marqueur de sens négatif, l'assignation d'une probabilité diminuée de progression vers un besoin de traitement rénal substitutif pour le sujet, quand la concentration mesurée est supérieure au seuil, ou l'assignation d'une probabilité augmentée de progression vers un besoin de traitement rénal substitutif, quand la concentration mesurée est inférieure au seuil.

(C) le sujet n'ayant pas atteint d'une insuffisance rénale,

pour un marqueur de sens positif, l'assignation d'une probabilité augmentée de progression vers une insuffisance rénale aiguë, quand la concentration mesurée est supérieure au seuil, ou l'assignation d'une probabilité diminuée de progression du sujet vers une insuffisance rénale aiguë, quand la concentration mesurée est inférieure au seuil, ou

pour un marqueur de sens négatif, l'assignation d'une probabilité diminuée de progression vers une insuffisance rénale aiguë, quand la concentration mesurée est supérieure au seuil, ou l'assignation d'une probabilité augmentée de progression du sujet vers une insuffisance rénale aiguë, quand la concentration mesurée est inférieure au seuil,

(D) le sujet étant dans la classe 0 de RIFLE, et ladite étape de corrélation comprenant l'assignation d'une probabilité que le sujet atteindra la classe R, I ou F de RIFLE dans les 72 heures,

pour un marqueur de sens positif, l'assignation d'une probabilité augmentée de progression du sujet vers la classe R, I ou F de RIFLE, quand la concentration mesurée est supérieure au seuil, ou l'assignation d'une probabilité diminuée de progression du sujet vers la classe R, I ou F de RIFLE, quand la concentration mesurée est inférieure au seuil, ou

pour un marqueur de sens négatif, l'assignation d'une probabilité diminuée de progression du sujet vers la classe R, I ou F de RIFLE, quand la concentration mesurée est supérieure au seuil, ou l'assignation d'une probabilité augmentée de progression du sujet vers la classe R, I ou F de RIFLE, quand la concentration mesurée est inférieure au seuil,

(E) le sujet étant dans la classe 0 ou R de RIFLE, et ladite étape de corrélation comprend l'assignation d'une probabilité que le sujet atteindra la classe I ou F de RIFLE dans les 72 heures,

pour un marqueur de sens positif, l'assignation d'une probabilité augmentée de progression du sujet vers la classe I ou F de RIFLE, quand la concentration mesurée est supérieure au seuil, ou l'assignation d'une probabilité diminuée de progression du sujet vers la classe I ou F de RIFLE, quand la concentration mesurée est inférieure au seuil, ou

pour un marqueur de sens négatif, l'assignation d'une probabilité diminuée de progression du sujet vers la classe I ou F de RIFLE, quand la concentration mesurée est supérieure au seuil, ou l'assignation d'une probabilité augmentée de progression du sujet vers la classe I ou F de RIFLE, quand la concentration mesurée est inférieure au seuil,

(F) le sujet étant dans la classe 0 de RIFLE, et ladite étape de corrélation comprenant l'assignation d'une probabilité que le sujet atteindra la classe F de RIFLE dans les 72 heures,

pour un marqueur de sens positif, l'assignation d'une probabilité augmentée de progression du sujet vers la classe F de RIFLE, quand la concentration mesurée est supérieure au seuil, ou l'assignation d'une probabilité diminuée de progression du sujet vers la classe I ou F de RIFLE, quand la concentration mesurée est inférieure au seuil, ou

pour un marqueur de sens négatif, l'assignation d'une probabilité diminuée de progression du sujet vers la classe F de RIFLE, quand la concentration mesurée est supérieure au seuil, ou l'assignation d'une probabilité augmentée de progression du sujet vers la classe I ou F de RIFLE, quand la concentration mesurée est inférieure au seuil,

(G) le sujet étant dans la classe R de RIFLE, et ladite étape de corrélation comprenant l'assignation d'une probabilité que le sujet atteindra la classe I ou F de RIFLE dans les 72 heures,

pour un marqueur de sens positif, l'assignation d'une probabilité augmentée de progression du sujet vers la classe F de RIFLE, quand la concentration mesurée est supérieure au seuil, ou l'assignation d'une probabilité diminuée de progression du sujet vers la classe I ou F de RIFLE, quand la concentration mesurée est inférieure au seuil, ou

pour un marqueur de sens négatif, l'assignation d'une probabilité diminuée de progression du sujet vers la classe F de RIFLE, quand la concentration mesurée est supérieure au seuil, ou l'assignation d'une probabilité augmentée de progression du sujet vers la classe I ou F de RIFLE, quand la concentration mesurée est inférieure au seuil,

(H) le sujet étant dans la classe 0, R ou I de RIFLE, et ladite étape de corrélation comprenant l'assignation d'une probabilité que le sujet atteindra la classe F de RIFLE dans les 72 heures,

pour un marqueur de sens positif, l'assignation d'une probabilité augmentée de progression du sujet vers la classe F de RIFLE, quand la concentration mesurée est supérieure au seuil, ou l'assignation d'une probabilité diminuée de progression du sujet vers la classe F de RIFLE, quand la concentration mesurée est inférieure au seuil, ou

pour un marqueur de sens négatif, l'assignation d'une probabilité diminuée de progression du sujet vers la classe F de RIFLE, quand la concentration mesurée est supérieure au seuil, ou l'assignation d'une probabilité augmentée de progression du sujet vers la classe F de RIFLE, quand la concentration mesurée est inférieure au seuil,

(I) le sujet étant dans la classe I de RIFLE, et ladite étape de corrélation comprenant l'assignation d'une probabilité que le sujet atteindra la classe F de RIFLE dans les 72 heures,

pour un marqueur de sens positif, l'assignation d'une probabilité augmentée de progression du sujet vers la classe F de RIFLE, quand la concentration mesurée est supérieure au seuil, ou l'assignation d'une probabilité diminuée de progression du sujet vers la classe F de RIFLE, quand la concentration mesurée est inférieure au seuil, ou

pour un marqueur de sens négatif, l'assignation d'une probabilité diminuée de progression du sujet vers la classe F de RIFLE, quand la concentration mesurée est supérieure au seuil, ou l'assignation d'une probabilité augmentée de progression du sujet vers la classe F de RIFLE, quand la concentration mesurée est inférieure au seuil.

9. Procédé selon la revendication 1, dans lequel le sujet est sélectionné pour une évaluation de l'état rénale (i) en fonction de la préexistence chez le sujet d'un ou de plusieurs facteurs de risque connus pour une IRA pré-rénale, rénale intrinsèque, ou post-rénale, ou

en fonction d'un diagnostic existant d'un(e) ou de plusieurs parmi une insuffisance cardiaque congestive, une pré-éclampsie, une éclampsie, un diabète sucré, une hypertension, une coronaropathie, une protéinurie, une insuffisance rénale, une filtration glomérulaire inférieure à la plage normale, une cirrhose, de la créatinine sérique supérieure à la plage normale, une sepsie, une lésion de la fonction rénale, une fonction rénale réduite, ou une IRA, ou en fonction du fait de subir ou d'avoir subi une intervention chirurgicale vasculaire majeure, un pontage aorto-coronarien, ou une autre intervention cardiaque, ou en fonction de l'exposition à des AINS, à des cyclosporines, au tacrolimus, à des aminoglycosides, au foscarnet, à de l'éthylèneglycol, à l'hémoglobine, à la myoglobine, à l'ifosfamide, à des métaux lourds, au méthotrexate, à des agents de contraste radio-opaques, ou à de la streptozotocine.

**10.** Utilisation de l'antigène p53 des tumeurs cellulaires pour le diagnostic in vitro, la stratification des risques, le pronostic, la classification et la surveillance de l'état rénal d'un sujet n'étant pas soumis à un traitement rénal substitutif.

**11.** Utilisation d'un ou de plusieurs marqueurs biologiques comprenant l'antigène p53 des tumeurs cellulaires, et l'un ou plusieurs marqueurs biologiques facultativement comprenant en outre le membre 10B de la superfamille des récepteurs du facteur de nécrose tumorale, la cadhérine-16, la caspase-9, l'antagoniste de Bcl2 de l'apoptose cellulaire, la caspase-1, la cadhérine-1, la poly-[ADP-ribose]-polymérase 1, l'inhibiteur 1 de kinase dépendant de la cycline, la cadhérine-5, la myoglobine, l'apolipoprotéine A-II, la mucine-16 ou la molécule 5 d'adhésion cellulaire liée à l'antigène carcino-embryonnaire, pour

(i) le diagnostic, la stratification des risques, le pronostic, la classification et la surveillance de l'état rénal d'un sujet n'étant pas atteint d'insuffisance rénale aiguë, ou
(ii) pour l'assignation d'une probabilité augmentée de progression du sujet vers une classe de RIFLE plus grave par rapport à la classe de RIFLE actuelle du sujet.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2007248989 A
- US 6143576 A
- US 6113855 A
- US 6019944 A
- US 5985579 A
- US 5947124 A
- US 5939272 A
- US 5922615 A
- US 5885527 A
- US 5851776 A
- US 5824799 A
- US 5679526 A
- US 5525524 A
- US 5480792 A
- US 5631171 A
- US 5955377 A
- US 5571698 A, Ladner
- US 6057098 A
- WO 61243991 A
- WO 61243997 A
- WO 61244002 A
- WO 61243993 A
- WO 61238115 A
- WO 61238118 A
- WO 61238120 A
- WO 61238121 A
- WO 61238123 A
- WO 61238125 A
- WO 61238127 A
- WO 61238129 A
- WO 61238134 A
- WO 61238128 A

### Non-patent literature cited in the description

- Harrison's Principles of Internal Medicine. McGraw Hill, 1741-1830
- Current Medical Diagnosis & Treatment. McGraw Hill, 2008, 785-815
- the Merck Manual
- **PRAUGHT ; SHLIPAK.** *Curr Opin Nephrol Hypertens,* 2005, vol. 14, 265-270
- **CHERTOW et al.** *J Am Soc Nephrol,* 2005, vol. 16, 3365-3370
- **LASSNIGG.** *J Am Soc Nephrol,* 2004, vol. 15, 1597-1605
- **BELLOMO et al.** *Crit Care,* 2004, vol. 8 (4), R204-12
- **KELLUM.** *Crit. Care Med.,* 2008, vol. 36, 141-45
- **RICCI et al.** *Kidney Int.,* 2008, vol. 73, 538-546
- **MEHTA et al.** *Crit. Care,* 2007, vol. 11, R31
- **MCCOLLOUGH et al.** *Rev Cardiovasc Med.,* 2006, vol. 7 (4), 177-197
- Current Medical Diagnosis & Treatment. McGraw Hill, 2008, 785-815
- The Immunoassay Handbook. Stockton Press, 1994
- Fundamental Immunology. Raven Press, 1993
- **WILSON.** *J. Immunol. Methods,* 1994, vol. 175, 267-273
- **YARMUSH.** *J. Biochem. Biophys. Methods,* 1992, vol. 25, 85-97
- **WARD et al.** *Nature,* 1989, vol. 341, 544-546
- **, VAN ERP et al.** *J. Immunoassay,* 1991, vol. 12, 425-43
- **NELSON ; GRISWOLD.** *Comput. Methods Programs Biomed,* 1988, vol. 27, 65-8
- **CWIRLA et al.** *Proc. Natl. Acad. Sci. USA,* 1990, vol. 87, 6378-82
- **DEVLIN E.** *Science,* 1990, vol. 249, 404-6
- **SCOTT ; SMITH.** *Science,* 1990, vol. 249, 386-88
- **FISCHER et al.** *Intensive Care Med.,* 2003, vol. 29, 1043-51
- **BAGSHAW et al.** *Nephrol. Dial. Transplant.,* 2008, vol. 23, 1203-1210
- Merck Manual of Diagnosis and Therapy. Merck Research Laboratories, 1999
- **WIJEYSUNDERA et al.** *JAMA,* 2007, vol. 297, 1801-9
- **HANLEY, J. A. ; MCNEIL, B.J.** The meaning and use of the area under a receiver operating characteristic (ROC) curve. *Radiology,* 1982, vol. 143, 29-36

SEQUENCE LISTING

<110> ASTUTE MEDICAL, INC.

<120> METHODS AND COMPOSITIONS FOR DIAGNOSIS AND PROGNOSIS OF RENAL INJURY AND RENAL FAILURE

<130> BLG14431PCTEPD1

<140> Divisional application based on EP10812639.2
<141> 2010-08-27

<150> 61/243,991
<151> 2009-09-18

<150> 61/243,997
<151> 2009-09-18

<150> 61/244,002
<151> 2009-09-18

<150> 61/243,993
<151> 2009-09-18

<150> 61/238,115
<151> 2009-08-28

<150> 61/238,118
<151> 2009-08-28

<150> 61/238,120
<151> 2009-08-28

<150> 61/238,121
<151> 2009-08-28

<150> 61/238,123
<151> 2009-08-28

<150> 61/238,125
<151> 2009-08-28

<150> 61/238,127
<151> 2009-08-28

<150> 61/238,129
<151> 2009-08-28

<150> 61/238,134
<151> 2009-08-28

<150> 61/238,128
<151> 2009-08-28

<160> 16

<170> PatentIn version 3.5

<210> 1
<211> 440
<212> PRT
<213> Homo sapiens

<400> 1

```
Met Glu Gln Arg Gly Gln Asn Ala Pro Ala Ala Ser Gly Ala Arg Lys
1               5               10              15

Arg His Gly Pro Gly Pro Arg Glu Ala Arg Gly Ala Arg Pro Gly Pro
        20              25              30

Arg Val Pro Lys Thr Leu Val Leu Val Val Ala Ala Val Leu Leu Leu
        35              40              45

Val Ser Ala Glu Ser Ala Leu Ile Thr Gln Gln Asp Leu Ala Pro Gln
        50              55              60

Gln Arg Ala Ala Pro Gln Gln Lys Arg Ser Ser Pro Ser Glu Gly Leu
65              70              75              80

Cys Pro Pro Gly His His Ile Ser Glu Asp Gly Arg Asp Cys Ile Ser
                85              90              95

Cys Lys Tyr Gly Gln Asp Tyr Ser Thr His Trp Asn Asp Leu Leu Phe
                100             105             110

Cys Leu Arg Cys Thr Arg Cys Asp Ser Gly Glu Val Glu Leu Ser Pro
        115             120             125

Cys Thr Thr Thr Arg Asn Thr Val Cys Gln Cys Glu Glu Gly Thr Phe
        130             135             140

Arg Glu Glu Asp Ser Pro Glu Met Cys Arg Lys Cys Arg Thr Gly Cys
145             150             155             160

Pro Arg Gly Met Val Lys Val Gly Asp Cys Thr Pro Trp Ser Asp Ile
                165             170             175

Glu Cys Val His Lys Glu Ser Gly Thr Lys His Ser Gly Glu Ala Pro
                180             185             190

Ala Val Glu Glu Thr Val Thr Ser Ser Pro Gly Thr Pro Ala Ser Pro
        195             200             205

Cys Ser Leu Ser Gly Ile Ile Ile Gly Val Thr Val Ala Ala Val Val
        210             215             220

Leu Ile Val Ala Val Phe Val Cys Lys Ser Leu Leu Trp Lys Lys Val
225             230             235             240

Leu Pro Tyr Leu Lys Gly Ile Cys Ser Gly Gly Gly Gly Asp Pro Glu
                245             250             255
```

```
Arg Val Asp Arg Ser Ser Gln Arg Pro Gly Ala Glu Asp Asn Val Leu
        260             265             270

Asn Glu Ile Val Ser Ile Leu Gln Pro Thr Gln Val Pro Glu Gln Glu
        275             280             285

Met Glu Val Gln Glu Pro Ala Glu Pro Thr Gly Val Asn Met Leu Ser
    290             295             300

Pro Gly Glu Ser Glu His Leu Leu Glu Pro Ala Glu Ala Glu Arg Ser
305             310             315             320

Gln Arg Arg Arg Leu Leu Val Pro Ala Asn Glu Gly Asp Pro Thr Glu
            325             330             335

Thr Leu Arg Gln Cys Phe Asp Asp Phe Ala Asp Leu Val Pro Phe Asp
            340             345             350

Ser Trp Glu Pro Leu Met Arg Lys Leu Gly Leu Met Asp Asn Glu Ile
        355             360             365

Lys Val Ala Lys Ala Glu Ala Ala Gly His Arg Asp Thr Leu Tyr Thr
    370             375             380

Met Leu Ile Lys Trp Val Asn Lys Thr Gly Arg Asp Ala Ser Val His
385             390             395             400

Thr Leu Leu Asp Ala Leu Glu Thr Leu Gly Glu Arg Leu Ala Lys Gln
            405             410             415

Lys Ile Glu Asp His Leu Leu Ser Ser Gly Lys Phe Met Tyr Leu Glu
            420             425             430

Gly Asn Ala Asp Ser Ala Met Ser
            435             440
```

<210> 2
<211> 829
<212> PRT
<213> Homo sapiens

<400> 2

```
Met Val Pro Ala Trp Leu Trp Leu Leu Cys Val Ser Val Pro Gln Ala
1               5               10              15

Leu Pro Lys Ala Gln Pro Ala Glu Leu Ser Val Glu Val Pro Glu Asn
        20              25              30
```

228

Tyr Gly Gly Asn Phe Pro Leu Tyr Leu Thr Lys Leu Pro Leu Pro Arg
        35                      40                  45

Glu Gly Ala Glu Gly Gln Ile Val Leu Ser Gly Asp Ser Gly Lys Ala
        50                      55                  60

Thr Glu Gly Pro Phe Ala Met Asp Pro Asp Ser Gly Phe Leu Leu Val
65                      70                  75                  80

Thr Arg Ala Leu Asp Arg Glu Glu Gln Ala Glu Tyr Gln Leu Gln Val
                85                      90                  95

Thr Leu Glu Met Gln Asp Gly His Val Leu Trp Gly Pro Gln Pro Val
                100                     105                 110

Leu Val His Val Lys Asp Glu Asn Asp Gln Val Pro His Phe Ser Gln
                115                     120                 125

Ala Ile Tyr Arg Ala Arg Leu Ser Arg Gly Thr Arg Pro Gly Ile Pro
        130                     135                 140

Phe Leu Phe Leu Glu Ala Ser Asp Arg Asp Glu Pro Gly Thr Ala Asn
145                     150                     155                 160

Ser Asp Leu Arg Phe His Ile Leu Ser Gln Ala Pro Ala Gln Pro Ser
                165                     170                 175

Pro Asp Met Phe Gln Leu Glu Pro Arg Leu Gly Ala Leu Ala Leu Ser
                180                     185                 190

Pro Lys Gly Ser Thr Ser Leu Asp His Ala Leu Glu Arg Thr Tyr Gln
        195                     200                 205

Leu Leu Val Gln Val Lys Asp Met Gly Asp Gln Ala Ser Gly His Gln
        210                     215                 220

Ala Thr Ala Thr Val Glu Val Ser Ile Ile Glu Ser Thr Trp Val Ser
225                     230                     235                 240

Leu Glu Pro Ile His Leu Ala Glu Asn Leu Lys Val Leu Tyr Pro His
                245                     250                 255

His Met Ala Gln Val His Trp Ser Gly Gly Asp Val His Tyr His Leu
                260                     265                 270

Glu Ser His Pro Pro Gly Pro Phe Glu Val Asn Ala Glu Gly Asn Leu
        275                     280                 285

```
Tyr Val Thr Arg Glu Leu Asp Arg Glu Ala Gln Ala Glu Tyr Leu Leu
    290             295             300

Gln Val Arg Ala Gln Asn Ser His Gly Glu Asp Tyr Ala Ala Pro Leu
305             310             315             320

Glu Leu His Val Leu Val Met Asp Glu Asn Asp Asn Val Pro Ile Cys
            325             330             335

Pro Pro Arg Asp Pro Thr Val Ser Ile Pro Glu Leu Ser Pro Pro Gly
            340             345             350

Thr Glu Val Thr Arg Leu Ser Ala Glu Asp Ala Asp Ala Pro Gly Ser
            355             360             365

Pro Asn Ser His Val Val Tyr Gln Leu Leu Ser Pro Glu Pro Glu Asp
    370             375             380

Gly Val Glu Gly Arg Ala Phe Gln Val Asp Pro Thr Ser Gly Ser Val
385             390             395             400

Thr Leu Gly Val Leu Pro Leu Arg Ala Gly Gln Asn Ile Leu Leu Leu
            405             410             415

Val Leu Ala Met Asp Leu Ala Gly Ala Glu Gly Gly Phe Ser Ser Thr
            420             425             430

Cys Glu Val Glu Val Ala Val Thr Asp Ile Asn Asp His Ala Pro Glu
            435             440             445

Phe Ile Thr Ser Gln Ile Gly Pro Ile Ser Leu Pro Glu Asp Val Glu
    450             455             460

Pro Gly Thr Leu Val Ala Met Leu Thr Ala Ile Asp Ala Asp Leu Glu
465             470             475             480

Pro Ala Phe Arg Leu Met Asp Phe Ala Ile Glu Arg Gly Asp Thr Glu
            485             490             495

Gly Thr Phe Gly Leu Asp Trp Glu Pro Asp Ser Gly His Val Arg Leu
            500             505             510

Arg Leu Cys Lys Asn Leu Ser Tyr Glu Ala Ala Pro Ser His Glu Val
            515             520             525

Val Val Val Val Gln Ser Val Ala Lys Leu Val Gly Pro Gly Pro Gly
```

230

```
                530                      535                         540

Pro Gly Ala Thr Ala Thr Val Thr Val Leu Val Glu Arg Val Met Pro
545             550                 555                     560

Pro Pro Lys Leu Asp Gln Glu Ser Tyr Glu Ala Ser Val Pro Ile Ser
                565                 570                     575

Ala Pro Ala Gly Ser Phe Leu Leu Thr Ile Gln Pro Ser Asp Pro Ile
            580                 585                     590

Ser Arg Thr Leu Arg Phe Ser Leu Val Asn Asp Ser Glu Gly Trp Leu
        595                 600                     605

Cys Ile Glu Lys Phe Ser Gly Glu Val His Thr Ala Gln Ser Leu Gln
    610                 615                     620

Gly Ala Gln Pro Gly Asp Thr Tyr Thr Val Leu Val Glu Ala Gln Asp
625                 630                     635                     640

Thr Asp Glu Pro Arg Leu Ser Ala Ser Ala Pro Leu Val Ile His Phe
                645                 650                     655

Leu Lys Ala Pro Pro Ala Pro Ala Leu Thr Leu Ala Pro Val Pro Ser
            660                 665                     670

Gln Tyr Leu Cys Thr Pro Arg Gln Asp His Gly Leu Ile Val Ser Gly
        675                 680                     685

Pro Ser Lys Asp Pro Asp Leu Ala Ser Gly His Gly Pro Tyr Ser Phe
    690                 695                     700

Thr Leu Gly Pro Asn Pro Thr Val Gln Arg Asp Trp Arg Leu Gln Thr
705                 710                     715                     720

Leu Asn Gly Ser His Ala Tyr Leu Thr Leu Ala Leu His Trp Val Glu
                725                 730                     735

Pro Arg Glu His Ile Ile Pro Val Val Val Ser His Asn Ala Gln Met
            740                 745                     750

Trp Gln Leu Leu Val Arg Val Ile Val Cys Arg Cys Asn Val Glu Gly
        755                 760                     765

Gln Cys Met Arg Lys Val Gly Arg Met Lys Gly Met Pro Thr Lys Leu
    770                 775                     780
```

```
Ser Ala Val Gly Ile Leu Val Gly Thr Leu Val Ala Ile Gly Ile Phe
785                 790             795                 800

Leu Ile Leu Ile Phe Thr His Trp Thr Met Ser Arg Lys Lys Asp Pro
            805             810                 815

Asp Gln Pro Ala Asp Ser Val Pro Leu Lys Ala Thr Val
        820             825
```

<210> 3
<211> 416
<212> PRT
<213> Homo sapiens

<400> 3

```
Met Asp Glu Ala Asp Arg Arg Leu Leu Arg Arg Cys Arg Leu Arg Leu
1               5                   10              15

Val Glu Glu Leu Gln Val Asp Gln Leu Trp Asp Ala Leu Leu Ser Arg
            20                  25              30

Glu Leu Phe Arg Pro His Met Ile Glu Asp Ile Gln Arg Ala Gly Ser
            35                  40              45

Gly Ser Arg Arg Asp Gln Ala Arg Gln Leu Ile Ile Asp Leu Glu Thr
        50                  55                  60

Arg Gly Ser Gln Ala Leu Pro Leu Phe Ile Ser Cys Leu Glu Asp Thr
65                  70                  75                  80

Gly Gln Asp Met Leu Ala Ser Phe Leu Arg Thr Asn Arg Gln Ala Ala
                85                  90                  95

Lys Leu Ser Lys Pro Thr Leu Glu Asn Leu Thr Pro Val Val Leu Arg
            100                 105                 110

Pro Glu Ile Arg Lys Pro Glu Val Leu Arg Pro Glu Thr Pro Arg Pro
            115                 120                 125

Val Asp Ile Gly Ser Gly Gly Phe Gly Asp Val Gly Ala Leu Glu Ser
    130                 135                 140

Leu Arg Gly Asn Ala Asp Leu Ala Tyr Ile Leu Ser Met Glu Pro Cys
145                 150                 155                 160

Gly His Cys Leu Ile Ile Asn Asn Val Asn Phe Cys Arg Glu Ser Gly
                165                 170                 175

Leu Arg Thr Arg Thr Gly Ser Asn Ile Asp Cys Glu Lys Leu Arg Arg
```

|  |  | 180 |  |  |  |  | 185 |  |  |  |  | 190 |  |
|---|---|---|---|---|---|---|---|---|---|---|---|---|---|

Arg Phe Ser Ser Leu His Phe Met Val Glu Val Lys Gly Asp Leu Thr
195 200 205

Ala Lys Lys Met Val Leu Ala Leu Leu Glu Leu Ala Gln Gln Asp His
210 215 220

Gly Ala Leu Asp Cys Cys Val Val Val Ile Leu Ser His Gly Cys Gln
225 230 235 240

Ala Ser His Leu Gln Phe Pro Gly Ala Val Tyr Gly Thr Asp Gly Cys
245 250 255

Pro Val Ser Val Glu Lys Ile Val Asn Ile Phe Asn Gly Thr Ser Cys
260 265 270

Pro Ser Leu Gly Gly Lys Pro Lys Leu Phe Phe Ile Gln Ala Cys Gly
275 280 285

Gly Glu Gln Lys Asp His Gly Phe Glu Val Ala Ser Thr Ser Pro Glu
290 295 300

Asp Glu Ser Pro Gly Ser Asn Pro Glu Pro Asp Ala Thr Pro Phe Gln
305 310 315 320

Glu Gly Leu Arg Thr Phe Asp Gln Leu Asp Ala Ile Ser Ser Leu Pro
325 330 335

Thr Pro Ser Asp Ile Phe Val Ser Tyr Ser Thr Phe Pro Gly Phe Val
340 345 350

Ser Trp Arg Asp Pro Lys Ser Gly Ser Trp Tyr Val Glu Thr Leu Asp
355 360 365

Asp Ile Phe Glu Gln Trp Ala His Ser Glu Asp Leu Gln Ser Leu Leu
370 375 380

Leu Arg Val Ala Asn Ala Val Ser Val Lys Gly Ile Tyr Lys Gln Met
385 390 395 400

Pro Gly Cys Phe Asn Phe Leu Arg Lys Lys Leu Phe Phe Lys Thr Ser
405 410 415

<210> 4
<211> 168
<212> PRT

<213> Homo sapiens

<400> 4

```
        Met Phe Gln Ile Pro Glu Phe Glu Pro Ser Glu Gln Glu Asp Ser Ser
        1               5                   10                  15

        Ser Ala Glu Arg Gly Leu Gly Pro Ser Pro Ala Gly Asp Gly Pro Ser
                        20                  25                  30

        Gly Ser Gly Lys His His Arg Gln Ala Pro Gly Leu Leu Trp Asp Ala
                        35                  40                  45

        Ser His Gln Gln Glu Gln Pro Thr Ser Ser Ser His His Gly Gly Ala
                    50                  55                  60

        Gly Ala Val Glu Ile Arg Ser Arg His Ser Ser Tyr Pro Ala Gly Thr
        65                  70                  75                  80

        Glu Asp Asp Glu Gly Met Gly Glu Glu Pro Ser Pro Phe Arg Gly Arg
                        85                  90                  95

        Ser Arg Ser Ala Pro Pro Asn Leu Trp Ala Ala Gln Arg Tyr Gly Arg
                        100                 105                 110

        Glu Leu Arg Arg Met Ser Asp Glu Phe Val Asp Ser Phe Lys Lys Gly
                    115                 120                 125

        Leu Pro Arg Pro Lys Ser Ala Gly Thr Ala Thr Gln Met Arg Gln Ser
            130                 135                 140

        Ser Ser Trp Thr Arg Val Phe Gln Ser Trp Trp Asp Arg Asn Leu Gly
        145                 150                 155                 160

        Arg Gly Ser Ser Ala Pro Ser Gln
                        165
```

<210> 5
<211> 404
<212> PRT
<213> Homo sapiens

<400> 5

```
Met Ala Asp Lys Val Leu Lys Glu Lys Arg Lys Leu Phe Ile Arg Ser
1               5               10              15

Met Gly Glu Gly Thr Ile Asn Gly Leu Leu Asp Glu Leu Leu Gln Thr
            20              25              30

Arg Val Leu Asn Lys Glu Glu Met Glu Lys Val Lys Arg Glu Asn Ala
            35              40              45
```

Thr Val Met Asp Lys Thr Arg Ala Leu Ile Asp Ser Val Ile Pro Lys
50 55 60

Gly Ala Gln Ala Cys Gln Ile Cys Ile Thr Tyr Ile Cys Glu Glu Asp
65 70 75 80

Ser Tyr Leu Ala Gly Thr Leu Gly Leu Ser Ala Asp Gln Thr Ser Gly
85 90 95

Asn Tyr Leu Asn Met Gln Asp Ser Gln Gly Val Leu Ser Ser Phe Pro
100 105 110

Ala Pro Gln Ala Val Gln Asp Asn Pro Ala Met Pro Thr Ser Ser Gly
115 120 125

Ser Glu Gly Asn Val Lys Leu Cys Ser Leu Glu Glu Ala Gln Arg Ile
130 135 140

Trp Lys Gln Lys Ser Ala Glu Ile Tyr Pro Ile Met Asp Lys Ser Ser
145 150 155 160

Arg Thr Arg Leu Ala Leu Ile Ile Cys Asn Glu Glu Phe Asp Ser Ile
165 170 175

Pro Arg Arg Thr Gly Ala Glu Val Asp Ile Thr Gly Met Thr Met Leu
180 185 190

Leu Gln Asn Leu Gly Tyr Ser Val Asp Val Lys Lys Asn Leu Thr Ala
195 200 205

Ser Asp Met Thr Thr Glu Leu Glu Ala Phe Ala His Arg Pro Glu His
210 215 220

Lys Thr Ser Asp Ser Thr Phe Leu Val Phe Met Ser His Gly Ile Arg
225 230 235 240

Glu Gly Ile Cys Gly Lys Lys His Ser Glu Gln Val Pro Asp Ile Leu
245 250 255

Gln Leu Asn Ala Ile Phe Asn Met Leu Asn Thr Lys Asn Cys Pro Ser
260 265 270

Leu Lys Asp Lys Pro Lys Val Ile Ile Ile Gln Ala Cys Arg Gly Asp
275 280 285

Ser Pro Gly Val Val Trp Phe Lys Asp Ser Val Gly Val Ser Gly Asn
290 295 300

```
Leu Ser Leu Pro Thr Thr Glu Glu Phe Glu Asp Asp Ala Ile Lys Lys
305             310             315             320

Ala His Ile Glu Lys Asp Phe Ile Ala Phe Cys Ser Ser Thr Pro Asp
            325             330             335

Asn Val Ser Trp Arg His Pro Thr Met Gly Ser Val Phe Ile Gly Arg
            340             345             350

Leu Ile Glu His Met Gln Glu Tyr Ala Cys Ser Cys Asp Val Glu Glu
        355             360             365

Ile Phe Arg Lys Val Arg Phe Ser Phe Glu Gln Pro Asp Gly Arg Ala
    370             375             380

Gln Met Pro Thr Thr Glu Arg Val Thr Leu Thr Arg Cys Phe Tyr Leu
385             390             395             400

Phe Pro Gly His
```

<210> 6
<211> 882
<212> PRT
<213> Homo sapiens

<400> 6

```
Met Gly Pro Trp Ser Arg Ser Leu Ser Ala Leu Leu Leu Leu Leu Gln
1               5               10              15

Val Ser Ser Trp Leu Cys Gln Glu Pro Glu Pro Cys His Pro Gly Phe
            20              25              30

Asp Ala Glu Ser Tyr Thr Phe Thr Val Pro Arg Arg His Leu Glu Arg
        35              40              45

Gly Arg Val Leu Gly Arg Val Asn Phe Glu Asp Cys Thr Gly Arg Gln
    50              55              60

Arg Thr Ala Tyr Phe Ser Leu Asp Thr Arg Phe Lys Val Gly Thr Asp
65              70              75              80

Gly Val Ile Thr Val Lys Arg Pro Leu Arg Phe His Asn Pro Gln Ile
            85              90              95

His Phe Leu Val Tyr Ala Trp Asp Ser Thr Tyr Arg Lys Phe Ser Thr
            100             105             110
```

Lys Val Thr Leu Asn Thr Val Gly His His His Arg Pro Pro Pro His
115 120 125

Gln Ala Ser Val Ser Gly Ile Gln Ala Glu Leu Leu Thr Phe Pro Asn
130 135 140

Ser Ser Pro Gly Leu Arg Arg Gln Lys Arg Asp Trp Val Ile Pro Pro
145 150 155 160

Ile Ser Cys Pro Glu Asn Glu Lys Gly Pro Phe Pro Lys Asn Leu Val
165 170 175

Gln Ile Lys Ser Asn Lys Asp Lys Glu Gly Lys Val Phe Tyr Ser Ile
180 185 190

Thr Gly Gln Gly Ala Asp Thr Pro Pro Val Gly Val Phe Ile Ile Glu
195 200 205

Arg Glu Thr Gly Trp Leu Lys Val Thr Glu Pro Leu Asp Arg Glu Arg
210 215 220

Ile Ala Thr Tyr Thr Leu Phe Ser His Ala Val Ser Ser Asn Gly Asn
225 230 235 240

Ala Val Glu Asp Pro Met Glu Ile Leu Ile Thr Val Thr Asp Gln Asn
245 250 255

Asp Asn Lys Pro Glu Phe Thr Gln Glu Val Phe Lys Gly Ser Val Met
260 265 270

Glu Gly Ala Leu Pro Gly Thr Ser Val Met Glu Val Thr Ala Thr Asp
275 280 285

Ala Asp Asp Asp Val Asn Thr Tyr Asn Ala Ala Ile Ala Tyr Thr Ile
290 295 300

Leu Ser Gln Asp Pro Glu Leu Pro Asp Lys Asn Met Phe Thr Ile Asn
305 310 315 320

Arg Asn Thr Gly Val Ile Ser Val Val Thr Thr Gly Leu Asp Arg Glu
325 330 335

Ser Phe Pro Thr Tyr Thr Leu Val Val Gln Ala Ala Asp Leu Gln Gly
340 345 350

Glu Gly Leu Ser Thr Thr Ala Thr Ala Val Ile Thr Val Thr Asp Thr
355 360 365

239

Asn Asp Asn Pro Pro Ile Phe Asn Pro Thr Thr Tyr Lys Gly Gln Val
    370             375             380

Pro Glu Asn Glu Ala Asn Val Val Ile Thr Thr Leu Lys Val Thr Asp
385             390             395             400

Ala Asp Ala Pro Asn Thr Pro Ala Trp Glu Ala Val Tyr Thr Ile Leu
            405             410             415

Asn Asp Asp Gly Gly Gln Phe Val Val Thr Thr Asn Pro Val Asn Asn
            420             425             430

Asp Gly Ile Leu Lys Thr Ala Lys Gly Leu Asp Phe Glu Ala Lys Gln
    435             440             445

Gln Tyr Ile Leu His Val Ala Val Thr Asn Val Val Pro Phe Glu Val
    450             455             460

Ser Leu Thr Thr Ser Thr Ala Thr Val Thr Val Asp Val Leu Asp Val
465             470             475             480

Asn Glu Ala Pro Ile Phe Val Pro Pro Glu Lys Arg Val Glu Val Ser
            485             490             495

Glu Asp Phe Gly Val Gly Gln Glu Ile Thr Ser Tyr Thr Ala Gln Glu
            500             505             510

Pro Asp Thr Phe Met Glu Gln Lys Ile Thr Tyr Arg Ile Trp Arg Asp
    515             520             525

Thr Ala Asn Trp Leu Glu Ile Asn Pro Asp Thr Gly Ala Ile Ser Thr
    530             535             540

Arg Ala Glu Leu Asp Arg Glu Asp Phe Glu His Val Lys Asn Ser Thr
545             550             555             560

Tyr Thr Ala Leu Ile Ile Ala Thr Asp Asn Gly Ser Pro Val Ala Thr
            565             570             575

Gly Thr Gly Thr Leu Leu Leu Ile Leu Ser Asp Val Asn Asp Asn Ala
    580             585             590

Pro Ile Pro Glu Pro Arg Thr Ile Phe Phe Cys Glu Arg Asn Pro Lys
    595             600             605

Pro Gln Val Ile Asn Ile Ile Asp Ala Asp Leu Pro Pro Asn Thr Ser

240

|       | 610   |       |       |       | 615   |       |       |       | 620   |       |       |       |       |       |
|-------|-------|-------|-------|-------|-------|-------|-------|-------|-------|-------|-------|-------|-------|-------|

```
Pro Phe Thr Ala Glu Leu Thr His Gly Ala Ser Ala Asn Trp Thr Ile
625             630             635                 640

Gln Tyr Asn Asp Pro Thr Gln Glu Ser Ile Ile Leu Lys Pro Lys Met
            645             650                 655

Ala Leu Glu Val Gly Asp Tyr Lys Ile Asn Leu Lys Leu Met Asp Asn
            660             665                 670

Gln Asn Lys Asp Gln Val Thr Thr Leu Glu Val Ser Val Cys Asp Cys
            675             680                 685

Glu Gly Ala Ala Gly Val Cys Arg Lys Ala Gln Pro Val Glu Ala Gly
            690             695                 700

Leu Gln Ile Pro Ala Ile Leu Gly Ile Leu Gly Gly Ile Leu Ala Leu
705             710             715                 720

Leu Ile Leu Ile Leu Leu Leu Leu Leu Phe Leu Arg Arg Arg Ala Val
                725                 730                 735

Val Lys Glu Pro Leu Leu Pro Pro Glu Asp Asp Thr Arg Asp Asn Val
                740             745                 750

Tyr Tyr Tyr Asp Glu Glu Gly Gly Gly Glu Glu Asp Gln Asp Phe Asp
                755             760                 765

Leu Ser Gln Leu His Arg Gly Leu Asp Ala Arg Pro Glu Val Thr Arg
                770             775                 780

Asn Asp Val Ala Pro Thr Leu Met Ser Val Pro Arg Tyr Leu Pro Arg
785             790             795                 800

Pro Ala Asn Pro Asp Glu Ile Gly Asn Phe Ile Asp Glu Asn Leu Lys
                805             810                 815

Ala Ala Asp Thr Asp Pro Thr Ala Pro Pro Tyr Asp Ser Leu Leu Val
                820             825                 830

Phe Asp Tyr Glu Gly Ser Gly Ser Glu Ala Ala Ser Leu Ser Ser Leu
                835             840                 845

Asn Ser Ser Glu Ser Asp Lys Asp Gln Asp Tyr Asp Tyr Leu Asn Glu
                850             855                 860
```

```
Trp Gly Asn Arg Phe Lys Lys Leu Ala Asp Met Tyr Gly Gly Gly Glu
    865                 870             875                 880


        Asp Asp
```

<210> 7
<211> 1014
<212> PRT
<213> Homo sapiens

<400> 7

```
Met Ala Glu Ser Ser Asp Lys Leu Tyr Arg Val Glu Tyr Ala Lys Ser
1               5                   10              15

Gly Arg Ala Ser Cys Lys Lys Cys Ser Glu Ser Ile Pro Lys Asp Ser
            20              25              30

Leu Arg Met Ala Ile Met Val Gln Ser Pro Met Phe Asp Gly Lys Val
        35              40              45

Pro His Trp Tyr His Phe Ser Cys Phe Trp Lys Val Gly His Ser Ile
    50              55              60

Arg His Pro Asp Val Glu Val Asp Gly Phe Ser Glu Leu Arg Trp Asp
65              70              75              80

Asp Gln Gln Lys Val Lys Lys Thr Ala Glu Ala Gly Gly Val Thr Gly
            85              90              95

Lys Gly Gln Asp Gly Ile Gly Ser Lys Ala Glu Lys Thr Leu Gly Asp
            100             105             110

Phe Ala Ala Glu Tyr Ala Lys Ser Asn Arg Ser Thr Cys Lys Gly Cys
        115             120             125

Met Glu Lys Ile Glu Lys Gly Gln Val Arg Leu Ser Lys Lys Met Val
    130             135             140

Asp Pro Glu Lys Pro Gln Leu Gly Met Ile Asp Arg Trp Tyr His Pro
145             150             155             160

Gly Cys Phe Val Lys Asn Arg Glu Glu Leu Gly Phe Arg Pro Glu Tyr
            165             170             175

Ser Ala Ser Gln Leu Lys Gly Phe Ser Leu Leu Ala Thr Glu Asp Lys
        180             185             190

Glu Ala Leu Lys Lys Gln Leu Pro Gly Val Lys Ser Glu Gly Lys Arg
```

195 200 205

Lys Gly Asp Glu Val Asp Gly Val Asp Glu Val Ala Lys Lys Lys Ser
210 215 220

Lys Lys Glu Lys Asp Lys Asp Ser Lys Leu Glu Lys Ala Leu Lys Ala
225 230 235 240

Gln Asn Asp Leu Ile Trp Asn Ile Lys Asp Glu Leu Lys Lys Val Cys
245 250 255

Ser Thr Asn Asp Leu Lys Glu Leu Leu Ile Phe Asn Lys Gln Gln Val
260 265 270

Pro Ser Gly Glu Ser Ala Ile Leu Asp Arg Val Ala Asp Gly Met Val
275 280 285

Phe Gly Ala Leu Leu Pro Cys Glu Glu Cys Ser Gly Gln Leu Val Phe
290 295 300

Lys Ser Asp Ala Tyr Tyr Cys Thr Gly Asp Val Thr Ala Trp Thr Lys
305 310 315 320

Cys Met Val Lys Thr Gln Thr Pro Asn Arg Lys Glu Trp Val Thr Pro
325 330 335

Lys Glu Phe Arg Glu Ile Ser Tyr Leu Lys Lys Leu Lys Val Lys Lys
340 345 350

Gln Asp Arg Ile Phe Pro Pro Glu Thr Ser Ala Ser Val Ala Ala Thr
355 360 365

Pro Pro Pro Ser Thr Ala Ser Ala Pro Ala Ala Val Asn Ser Ser Ala
370 375 380

Ser Ala Asp Lys Pro Leu Ser Asn Met Lys Ile Leu Thr Leu Gly Lys
385 390 395 400

Leu Ser Arg Asn Lys Asp Glu Val Lys Ala Met Ile Glu Lys Leu Gly
405 410 415

Gly Lys Leu Thr Gly Thr Ala Asn Lys Ala Ser Leu Cys Ile Ser Thr
420 425 430

Lys Lys Glu Val Glu Lys Met Asn Lys Lys Met Glu Glu Val Lys Glu
435 440 445

```
Ala Asn Ile Arg Val Val Ser Glu Asp Phe Leu Gln Asp Val Ser Ala
    450             455             460

Ser Thr Lys Ser Leu Gln Glu Leu Phe Leu Ala His Ile Leu Ser Pro
465             470             475                 480

Trp Gly Ala Glu Val Lys Ala Glu Pro Val Glu Val Val Ala Pro Arg
            485             490                 495

Gly Lys Ser Gly Ala Ala Leu Ser Lys Lys Ser Lys Gly Gln Val Lys
            500             505             510

Glu Glu Gly Ile Asn Lys Ser Glu Lys Arg Met Lys Leu Thr Leu Lys
        515             520             525

Gly Gly Ala Ala Val Asp Pro Asp Ser Gly Leu Glu His Ser Ala His
    530             535             540

Val Leu Glu Lys Gly Gly Lys Val Phe Ser Ala Thr Leu Gly Leu Val
545             550             555                 560

Asp Ile Val Lys Gly Thr Asn Ser Tyr Tyr Lys Leu Gln Leu Leu Glu
                565             570             575

Asp Asp Lys Glu Asn Arg Tyr Trp Ile Phe Arg Ser Trp Gly Arg Val
            580             585             590

Gly Thr Val Ile Gly Ser Asn Lys Leu Glu Gln Met Pro Ser Lys Glu
        595             600             605

Asp Ala Ile Glu His Phe Met Lys Leu Tyr Glu Glu Lys Thr Gly Asn
    610             615             620

Ala Trp His Ser Lys Asn Phe Thr Lys Tyr Pro Lys Lys Phe Tyr Pro
625             630             635                 640

Leu Glu Ile Asp Tyr Gly Gln Asp Glu Glu Ala Val Lys Lys Leu Thr
            645             650             655

Val Asn Pro Gly Thr Lys Ser Lys Leu Pro Lys Pro Val Gln Asp Leu
            660             665             670

Ile Lys Met Ile Phe Asp Val Glu Ser Met Lys Lys Ala Met Val Glu
        675             680             685

Tyr Glu Ile Asp Leu Gln Lys Met Pro Leu Gly Lys Leu Ser Lys Arg
    690             695             700
```

245

```
Gln Ile Gln Ala Ala Tyr Ser Ile Leu Ser Glu Val Gln Gln Ala Val
705             710             715             720


Ser Gln Gly Ser Ser Asp Ser Gln Ile Leu Asp Leu Ser Asn Arg Phe
                725             730             735


Tyr Thr Leu Ile Pro His Asp Phe Gly Met Lys Lys Pro Pro Leu Leu
            740             745             750


Asn Asn Ala Asp Ser Val Gln Ala Lys Val Glu Met Leu Asp Asn Leu
            755             760             765


Leu Asp Ile Glu Val Ala Tyr Ser Leu Leu Arg Gly Gly Ser Asp Asp
    770             775             780


Ser Ser Lys Asp Pro Ile Asp Val Asn Tyr Glu Lys Leu Lys Thr Asp
785             790             795             800


Ile Lys Val Val Asp Arg Asp Ser Glu Glu Ala Glu Ile Ile Arg Lys
            805             810             815


Tyr Val Lys Asn Thr His Ala Thr Thr His Asn Ala Tyr Asp Leu Glu
            820             825             830


Val Ile Asp Ile Phe Lys Ile Glu Arg Glu Gly Glu Cys Gln Arg Tyr
    835             840             845


Lys Pro Phe Lys Gln Leu His Asn Arg Arg Leu Leu Trp His Gly Ser
    850             855             860


Arg Thr Thr Asn Phe Ala Gly Ile Leu Ser Gln Gly Leu Arg Ile Ala
865             870             875             880


Pro Pro Glu Ala Pro Val Thr Gly Tyr Met Phe Gly Lys Gly Ile Tyr
            885             890             895


Phe Ala Asp Met Val Ser Lys Ser Ala Asn Tyr Cys His Thr Ser Gln
            900             905             910


Gly Asp Pro Ile Gly Leu Ile Leu Leu Gly Glu Val Ala Leu Gly Asn
    915             920             925


Met Tyr Glu Leu Lys His Ala Ser His Ile Ser Lys Leu Pro Lys Gly
    930             935             940


Lys His Ser Val Lys Gly Leu Gly Lys Thr Thr Pro Asp Pro Ser Ala
945             950             955             960
```

246

EP 2 894 473 B1

```
Asn Ile Ser Leu Asp Gly Val Asp Val Pro Leu Gly Thr Gly Ile Ser
            965                 970                 975


Ser Gly Val Asn Asp Thr Ser Leu Leu Tyr Asn Glu Tyr Ile Val Tyr
            980                 985                 990


Asp Ile Ala Gln Val Asn Leu Lys  Tyr Leu Leu Lys Leu  Lys Phe Asn
            995                 1000                1005


Phe Lys  Thr Ser Leu Trp
    1010
```

<210> 8
<211> 164
<212> PRT
<213> Homo sapiens

<400> 8

```
Met Ser Glu Pro Ala Gly Asp Val Arg Gln Asn Pro Cys Gly Ser Lys
1               5               10              15


Ala Cys Arg Arg Leu Phe Gly Pro Val Asp Ser Glu Gln Leu Ser Arg
            20              25              30


Asp Cys Asp Ala Leu Met Ala Gly Cys Ile Gln Glu Ala Arg Glu Arg
            35              40              45


Trp Asn Phe Asp Phe Val Thr Glu Thr Pro Leu Glu Gly Asp Phe Ala
    50              55              60


Trp Glu Arg Val Arg Gly Leu Gly Leu Pro Lys Leu Tyr Leu Pro Thr
65              70              75              80


Gly Pro Arg Arg Gly Arg Asp Glu Leu Gly Gly Gly Arg Arg Pro Gly
            85              90              95


Thr Ser Pro Ala Leu Leu Gln Gly Thr Ala Glu Glu Asp His Val Asp
            100             105             110


Leu Ser Leu Ser Cys Thr Leu Val Pro Arg Ser Gly Glu Gln Ala Glu
            115             120             125


Gly Ser Pro Gly Gly Pro Gly Asp Ser Gln Gly Arg Lys Arg Arg Gln
            130             135             140


Thr Ser Met Thr Asp Phe Tyr His Ser Lys Arg Arg Leu Ile Phe Ser
145             150             155             160
```

247

Lys Arg Lys Pro

<210> 9
<211> 784
<212> PRT
<213> Homo sapiens

<400> 9

Met Gln Arg Leu Met Met Leu Leu Ala Thr Ser Gly Ala Cys Leu Gly
1               5                   10                  15

Leu Leu Ala Val Ala Ala Val Ala Ala Ala Gly Ala Asn Pro Ala Gln
            20                  25                  30

Arg Asp Thr His Ser Leu Leu Pro Thr His Arg Arg Gln Lys Arg Asp
        35                  40                  45

Trp Ile Trp Asn Gln Met His Ile Asp Glu Glu Lys Asn Thr Ser Leu
    50                  55                  60

Pro His His Val Gly Lys Ile Lys Ser Ser Val Ser Arg Lys Asn Ala
65                  70                  75                  80

Lys Tyr Leu Leu Lys Gly Glu Tyr Val Gly Lys Val Phe Arg Val Asp
                85                  90                  95

Ala Glu Thr Gly Asp Val Phe Ala Ile Glu Arg Leu Asp Arg Glu Asn
            100                 105                 110

Ile Ser Glu Tyr His Leu Thr Ala Val Ile Val Asp Lys Asp Thr Gly
            115                 120                 125

Glu Asn Leu Glu Thr Pro Ser Ser Phe Thr Ile Lys Val His Asp Val
        130                 135                 140

Asn Asp Asn Trp Pro Val Phe Thr His Arg Leu Phe Asn Ala Ser Val
145                 150                 155                 160

Pro Glu Ser Ser Ala Val Gly Thr Ser Val Ile Ser Val Thr Ala Val
                165                 170                 175

Asp Ala Asp Asp Pro Thr Val Gly Asp His Ala Ser Val Met Tyr Gln
            180                 185                 190

Ile Leu Lys Gly Lys Glu Tyr Phe Ala Ile Asp Asn Ser Gly Arg Ile
            195                 200                 205

248

Ile Thr Ile Thr Lys Ser Leu Asp Arg Glu Lys Gln Ala Arg Tyr Glu
210                215                220

Ile Val Val Glu Ala Arg Asp Ala Gln Gly Leu Arg Gly Asp Ser Gly
225            230                235                240

Thr Ala Thr Val Leu Val Thr Leu Gln Asp Ile Asn Asp Asn Phe Pro
                245                250                255

Phe Phe Thr Gln Thr Lys Tyr Thr Phe Val Val Pro Glu Asp Thr Arg
            260                265                270

Val Gly Thr Ser Val Gly Ser Leu Phe Val Glu Asp Pro Asp Glu Pro
            275                280                285

Gln Asn Arg Met Thr Lys Tyr Ser Ile Leu Arg Gly Asp Tyr Gln Asp
        290                295                300

Ala Phe Thr Ile Glu Thr Asn Pro Ala His Asn Glu Gly Ile Ile Lys
305                310                315                320

Pro Met Lys Pro Leu Asp Tyr Glu Tyr Ile Gln Gln Tyr Ser Phe Ile
                325                330                335

Val Glu Ala Thr Asp Pro Thr Ile Asp Leu Arg Tyr Met Ser Pro Pro
            340                345                350

Ala Gly Asn Arg Ala Gln Val Ile Ile Asn Ile Thr Asp Val Asp Glu
            355                360                365

Pro Pro Ile Phe Gln Gln Pro Phe Tyr His Phe Gln Leu Lys Glu Asn
    370                375                380

Gln Lys Lys Pro Leu Ile Gly Thr Val Leu Ala Met Asp Pro Asp Ala
385                390                395                400

Ala Arg His Ser Ile Gly Tyr Ser Ile Arg Arg Thr Ser Asp Lys Gly
            405                410                415

Gln Phe Phe Arg Val Thr Lys Lys Gly Asp Ile Tyr Asn Glu Lys Glu
            420                425                430

Leu Asp Arg Glu Val Tyr Pro Trp Tyr Asn Leu Thr Val Glu Ala Lys
            435                440                445

Glu Leu Asp Ser Thr Gly Thr Pro Thr Gly Lys Glu Ser Ile Val Gln
    450                455                460

```
Val His Ile Glu Val Leu Asp Glu Asn Asp Asn Ala Pro Glu Phe Ala
465             470         475             480

Lys Pro Tyr Gln Pro Lys Val Cys Glu Asn Ala Val His Gly Gln Leu
                485             490             495

Val Leu Gln Ile Ser Ala Ile Asp Lys Asp Ile Thr Pro Arg Asn Val
            500             505             510

Lys Phe Lys Phe Thr Leu Asn Thr Glu Asn Asn Phe Thr Leu Thr Asp
            515             520             525

Asn His Asp Asn Thr Ala Asn Ile Thr Val Lys Tyr Gly Gln Phe Asp
            530             535             540

Arg Glu His Thr Lys Val His Phe Leu Pro Val Val Ile Ser Asp Asn
545             550             555             560

Gly Met Pro Ser Arg Thr Gly Thr Ser Thr Leu Thr Val Ala Val Cys
                565             570             575

Lys Cys Asn Glu Gln Gly Glu Phe Thr Phe Cys Glu Asp Met Ala Ala
            580             585             590

Gln Val Gly Val Ser Ile Gln Ala Val Val Ala Ile Leu Leu Cys Ile
            595             600             605

Leu Thr Ile Thr Val Ile Thr Leu Leu Ile Phe Leu Arg Arg Arg Leu
    610             615             620

Arg Lys Gln Ala Arg Ala His Gly Lys Ser Val Pro Glu Ile His Glu
625             630             635             640

Gln Leu Val Thr Tyr Asp Glu Glu Gly Gly Gly Glu Met Asp Thr Thr
                645             650             655

Ser Tyr Asp Val Ser Val Leu Asn Ser Val Arg Arg Gly Gly Ala Lys
            660             665             670

Pro Pro Arg Pro Ala Leu Asp Ala Arg Pro Ser Leu Tyr Ala Gln Val
            675             680             685

Gln Lys Pro Pro Arg His Ala Pro Gly Ala His Gly Gly Pro Gly Glu
    690             695             700

Met Ala Ala Met Ile Glu Val Lys Lys Asp Glu Ala Asp His Asp Gly
705             710             715             720
```

```
Asp Gly Pro Pro Tyr Asp Thr Leu His Ile Tyr Gly Tyr Glu Gly Ser
                725                 730                 735

Glu Ser Ile Ala Glu Ser Leu Ser Ser Leu Gly Thr Asp Ser Ser Asp
            740                 745                 750

Ser Asp Val Asp Tyr Asp Phe Leu Asn Asp Trp Gly Pro Arg Phe Lys
            755                 760                 765

Met Leu Ala Glu Leu Tyr Gly Ser Asp Pro Arg Glu Glu Leu Leu Tyr
    770                 775                 780
```

<210> 10
<211> 154
<212> PRT
<213> Homo sapiens

<400> 10

```
Met Gly Leu Ser Asp Gly Glu Trp Gln Leu Val Leu Asn Val Trp Gly
1               5                   10                  15

Lys Val Glu Ala Asp Ile Pro Gly His Gly Gln Glu Val Leu Ile Arg
            20                  25                  30

Leu Phe Lys Gly His Pro Glu Thr Leu Glu Lys Phe Asp Lys Phe Lys
        35                  40                  45

His Leu Lys Ser Glu Asp Glu Met Lys Ala Ser Glu Asp Leu Lys Lys
    50                  55                  60

His Gly Ala Thr Val Leu Thr Ala Leu Gly Gly Ile Leu Lys Lys Lys
65                  70                  75                  80

Gly His His Glu Ala Glu Ile Lys Pro Leu Ala Gln Ser His Ala Thr
                85                  90                  95

Lys His Lys Ile Pro Val Lys Tyr Leu Glu Phe Ile Ser Glu Cys Ile
            100                 105                 110

Ile Gln Val Leu Gln Ser Lys His Pro Gly Asp Phe Gly Ala Asp Ala
            115                 120                 125

Gln Gly Ala Met Asn Lys Ala Leu Glu Leu Phe Arg Lys Asp Met Ala
    130                 135                 140

Ser Asn Tyr Lys Glu Leu Gly Phe Gln Gly
145                 150
```

<210> 11
<211> 100
<212> PRT
<213> Homo sapiens

<400> 11

```
Met Lys Leu Leu Ala Ala Thr Val Leu Leu Leu Thr Ile Cys Ser Leu
1               5                   10                  15

Glu Gly Ala Leu Val Arg Arg Gln Ala Lys Glu Pro Cys Val Glu Ser
            20                  25                  30

Leu Val Ser Gln Tyr Phe Gln Thr Val Thr Asp Tyr Gly Lys Asp Leu
            35                  40                  45

Met Glu Lys Val Lys Ser Pro Glu Leu Gln Ala Glu Ala Lys Ser Tyr
        50                  55                  60

Phe Glu Lys Ser Lys Glu Gln Leu Thr Pro Leu Ile Lys Lys Ala Gly
65                  70                  75                  80

Thr Glu Leu Val Asn Phe Leu Ser Tyr Phe Val Glu Leu Gly Thr Gln
                85                  90                  95

Pro Ala Thr Gln
                100
```

<210> 12
<211> 22152
<212> PRT
<213> Homo sapiens

<220>
<221> MOD_RES
<222> (13877)..(13878)
<223> Any amino acid

<220>
<221> MOD_RES
<222> (13880)..(13880)
<223> Any amino acid

<220>
<221> MOD_RES
<222> (13887)..(13887)
<223> Any amino acid

<220>
<221> MOD_RES
<222> (13890)..(13891)
<223> Any amino acid

<220>
<221> MOD_RES
<222> (13893)..(13893)
<223> Any amino acid

<220>
<221> MOD_RES
<222> (13903)..(13903)
<223> Any amino acid

<220>
<221> MOD_RES
<222> (13913)..(13914)
<223> Any amino acid

<220>
<221> MOD_RES
<222> (13916)..(13916)
<223> Any amino acid

<220>
<221> MOD_RES
<222> (13928)..(13929)
<223> Any amino acid

<220>
<221> MOD_RES
<222> (13938)..(13938)
<223> Any amino acid

<220>
<221> MOD_RES
<222> (13940)..(13941)
<223> Any amino acid

<220>
<221> MOD_RES
<222> (14569)..(14571)
<223> Any amino acid

<220>
<221> MOD_RES
<222> (14575)..(14575)
<223> Any amino acid

<220>
<221> MOD_RES
<222> (14579)..(14579)
<223> Any amino acid

<220>
<221> MOD_RES
<222> (14581)..(14581)
<223> Any amino acid

<220>
<221> MOD_RES
<222> (14587)..(14591)

<223> Any amino acid

<220>
<221> MOD_RES
<222> (14593)..(14594)
<223> Any amino acid

<220>
<221> MOD_RES
<222> (14725)..(14727)
<223> Any amino acid

<220>
<221> MOD_RES
<222> (14731)..(14731)
<223> Any amino acid

<220>
<221> MOD_RES
<222> (14735)..(14735)
<223> Any amino acid

<220>
<221> MOD_RES
<222> (14737)..(14737)
<223> Any amino acid

<220>
<221> MOD_RES
<222> (14743)..(14747)
<223> Any amino acid

<220>
<221> MOD_RES
<222> (14749)..(14750)
<223> Any amino acid

<220>
<221> MOD_RES
<222> (15661)..(15663)
<223> Any amino acid

<220>
<221> MOD_RES
<222> (15667)..(15667)
<223> Any amino acid

<220>
<221> MOD_RES
<222> (15671)..(15671)
<223> Any amino acid

<220>
<221> MOD_RES
<222> (15673)..(15673)
<223> Any amino acid

<220>

<221> MOD_RES
<222> (15679)..(15683)
<223> Any amino acid

<220>
<221> MOD_RES
<222> (15685)..(15686)
<223> Any amino acid

<220>
<221> MOD_RES
<222> (15972)..(15974)
<223> Any amino acid

<220>
<221> MOD_RES
<222> (15978)..(15978)
<223> Any amino acid

<220>
<221> MOD_RES
<222> (15982)..(15982)
<223> Any amino acid

<220>
<221> MOD_RES
<222> (15984)..(15984)
<223> Any amino acid

<220>
<221> MOD_RES
<222> (15990)..(15994)
<223> Any amino acid

<220>
<221> MOD_RES
<222> (15996)..(15997)
<223> Any amino acid

<220>
<221> MOD_RES
<222> (16008)..(16008)
<223> Any amino acid

<220>
<221> MOD_RES
<222> (16015)..(16015)
<223> Any amino acid

<220>
<221> MOD_RES
<222> (16017)..(16017)
<223> Any amino acid

<220>
<221> MOD_RES
<222> (16021)..(16021)
<223> Any amino acid

<220>
<221> MOD_RES
<222> (16025)..(16025)
<223> Any amino acid

<220>
<221> MOD_RES
<222> (16034)..(16034)
<223> Any amino acid

<220>
<221> MOD_RES
<222> (16037)..(16037)
<223> Any amino acid

<220>
<221> MOD_RES
<222> (16040)..(16040)
<223> Any amino acid

<220>
<221> MOD_RES
<222> (16046)..(16046)
<223> Any amino acid

<220>
<221> MOD_RES
<222> (16051)..(16051)
<223> Any amino acid

<220>
<221> MOD_RES
<222> (16053)..(16055)
<223> Any amino acid

<220>
<221> MOD_RES
<222> (16058)..(16058)
<223> Any amino acid

<220>
<221> MOD_RES
<222> (16062)..(16063)
<223> Any amino acid

<220>
<221> MOD_RES
<222> (16065)..(16065)
<223> Any amino acid

<220>
<221> MOD_RES
<222> (16072)..(16072)
<223> Any amino acid

<220>
<221> MOD_RES
<222> (16075)..(16076)

<223> Any amino acid

<220>
<221> MOD_RES
<222> (16078)..(16078)
<223> Any amino acid

<220>
<221> MOD_RES
<222> (16088)..(16088)
<223> Any amino acid

<220>
<221> MOD_RES
<222> (16268)..(16269)
<223> Any amino acid

<220>
<221> MOD_RES
<222> (16278)..(16278)
<223> Any amino acid

<220>
<221> MOD_RES
<222> (16280)..(16281)
<223> Any amino acid

<220>
<221> MOD_RES
<222> (16373)..(16374)
<223> Any amino acid

<220>
<221> MOD_RES
<222> (16376)..(16376)
<223> Any amino acid

<220>
<221> MOD_RES
<222> (16383)..(16383)
<223> Any amino acid

<220>
<221> MOD_RES
<222> (16386)..(16387)
<223> Any amino acid

<220>
<221> MOD_RES
<222> (16389)..(16389)
<223> Any amino acid

<220>
<221> MOD_RES
<222> (16399)..(16399)
<223> Any amino acid

<220>

<221> MOD_RES
<222> (16409)..(16410)
<223> Any amino acid

<220>
<221> MOD_RES
<222> (16412)..(16412)
<223> Any amino acid

<220>
<221> MOD_RES
<222> (16424)..(16425)
<223> Any amino acid

<220>
<221> MOD_RES
<222> (16434)..(16434)
<223> Any amino acid

<220>
<221> MOD_RES
<222> (16436)..(16437)
<223> Any amino acid

<220>
<221> MOD_RES
<222> (16439)..(16441)
<223> Any amino acid

<220>
<221> MOD_RES
<222> (1645)..(16445)
<223> Any amino acid

<220>
<221> MOD_RES
<222> (16449)..(16449)
<223> Any amino acid

<220>
<221> MOD_RES
<222> (16451)..(16451)
<223> Any amino acid

<220>
<221> MOD_RES
<222> (16457)..(16461)
<223> Any amino acid

<220>
<221> MOD_RES
<222> (16463)..(16464)
<223> Any amino acid

<220>
<221> MOD_RES
<222> (16841)..(16842)
<223> Any amino acid

```
<220>
<221> MOD_RES
<222> (16844)..(16844)
<223> Any amino acid

<220>
<221> MOD_RES
<222> (16851)..(16851)
<223> Any amino acid

<220>
<221> MOD_RES
<222> (16854)..(16855)
<223> Any amino acid

<220>
<221> MOD_RES
<222> (16857)..(16857)
<223> Any amino acid

<220>
<221> MOD_RES
<222> (16867)..(16867)
<223> Any amino acid

<220>
<221> MOD_RES
<222> (16877)..(16878)
<223> Any amino acid

<220>
<221> MOD_RES
<222> (16880)..(16880)
<223> Any amino acid

<220>
<221> MOD_RES
<222> (16892)..(16893)
<223> Any amino acid

<220>
<221> MOD_RES
<222> (16902)..(16902)
<223> Any amino acid

<220>
<221> MOD_RES
<222> (16904)..(16905)
<223> Any amino acid

<220>
<221> MOD_RES
<222> (16907)..(16909)
<223> Any amino acid

<220>
<221> MOD_RES
<222> (16913)..(16913)
```

<223> Any amino acid

<220>
<221> MOD_RES
<222> (16917)..(16917)
<223> Any amino acid

<220>
<221> MOD_RES
<222> (16919)..(16919)
<223> Any amino acid

<220>
<221> MOD_RES
<222> (16925)..(16929)
<223> Any amino acid

<220>
<221> MOD_RES
<222> (16931)..(16932)
<223> Any amino acid

<220>
<221> MOD_RES
<222> (17465)..(17466)
<223> Any amino acid

<220>
<221> MOD_RES
<222> (17468)..(17468)
<223> Any amino acid

<220>
<221> MOD_RES
<222> (17475)..(17475)
<223> Any amino acid

<220>
<221> MOD_RES
<222> (17478)..(17479)
<223> Any amino acid

<220>
<221> MOD_RES
<222> (17481)..(17481)
<223> Any amino acid

<220>
<221> MOD_RES
<222> (17491)..(17491)
<223> Any amino acid

<220>
<221> MOD_RES
<222> (17501)..(17502)
<223> Any amino acid

<220>

<221> MOD_RES
<222> (17504)..(17504)
<223> Any amino acid

<220>
<221> MOD_RES
<222> (17516)..(17517)
<223> Any amino acid

<220>
<221> MOD_RES
<222> (17526)..(17526)
<223> Any amino acid

<220>
<221> MOD_RES
<222> (17528)..(17529)
<223> Any amino acid

<220>
<221> MOD_RES
<222> (17531)..(17533)
<223> Any amino acid

<220>
<221> MOD_RES
<222> (17537)..(17537)
<223> Any amino acid

<220>
<221> MOD_RES
<222> (17541)..(17541)
<223> Any amino acid

<220>
<221> MOD_RES
<222> (17543)..(17543)
<223> Any amino acid

<220>
<221> MOD_RES
<222> (17549)..(17553)
<223> Any amino acid

<220>
<221> MOD_RES
<222> (17555)..(17556)
<223> Any amino acid

<220>
<221> MOD_RES
<222> (17567)..(17567)
<223> Any amino acid

<220>
<221> MOD_RES
<222> (17574)..(17574)
<223> Any amino acid

```
<220>
<221> MOD_RES
<222> (17576)..(17576)
<223> Any amino acid

<220>
<221> MOD_RES
<222> (17580)..(17580)
<223> Any amino acid

<220>
<221> MOD_RES
<222> (17584)..(17584)
<223> Any amino acid

<220>
<221> MOD_RES
<222> (17593)..(17593)
<223> Any amino acid

<220>
<221> MOD_RES
<222> (17596)..(17596)
<223> Any amino acid

<220>
<221> MOD_RES
<222> (17599)..(17599)
<223> Any amino acid

<220>
<221> MOD_RES
<222> (17605)..(17605)
<223> Any amino acid

<220>
<221> MOD_RES
<222> (17610)..(17610)
<223> Any amino acid

<220>
<221> MOD_RES
<222> (17612)..(17614)
<223> Any amino acid

<220>
<221> MOD_RES
<222> (17617)..(17617)
<223> Any amino acid

<220>
<221> MOD_RES
<222> (17621)..(17622)
<223> Any amino acid

<220>
<221> MOD_RES
<222> (17624)..(17624)
```

<223> Any amino acid

<220>
<221> MOD_RES
<222> (17631)..(17631)
<223> Any amino acid

<220>
<221> MOD_RES
<222> (17777)..(17778)
<223> Any amino acid

<220>
<221> MOD_RES
<222> (17780)..(17780)
<223> Any amino acid

<220>
<221> MOD_RES
<222> (17787)..(17787)
<223> Any amino acid

<220>
<221> MOD_RES
<222> (17790)..(17791)
<223> Any amino acid

<220>
<221> MOD_RES
<222> (17793)..(17793)
<223> Any amino acid

<220>
<221> MOD_RES
<222> (17803)..(17803)
<223> Any amino acid

<220>
<221> MOD_RES
<222> (17813)..(17814)
<223> Any amino acid

<220>
<221> MOD_RES
<222> (17816)..(17816)
<223> Any amino acid

<220>
<221> MOD_RES
<222> (17828)..(17829)
<223> Any amino acid

<220>
<221> MOD_RES
<222> (17838)..(17838)
<223> Any amino acid

<220>

<221> MOD_RES
<222> (17840)..(17841)
<223> Any amino acid

<220>
<221> MOD_RES
<222> (17843)..(17845)
<223> Any amino acid

<220>
<221> MOD_RES
<222> (17849)..(17849)
<223> Any amino acid

<220>
<221> MOD_RES
<222> (17853)..(17853)
<223> Any amino acid

<220>
<221> MOD_RES
<222> (17855)..(17855)
<223> Any amino acid

<220>
<221> MOD_RES
<222> (17861)..(17865)
<223> Any amino acid

<220>
<221> MOD_RES
<222> (17867)..(17868)
<223> Any amino acid

<220>
<221> MOD_RES
<222> (17879)..(17879)
<223> Any amino acid

<220>
<221> MOD_RES
<222> (17886)..(17886)
<223> Any amino acid

<220>
<221> MOD_RES
<222> (17888)..(17888)
<223> Any amino acid

<220>
<221> MOD_RES
<222> (17892)..(17892)
<223> Any amino acid

<220>
<221> MOD_RES
<222> (17896)..(17896)
<223> Any amino acid

<220>
<221> MOD_RES
<222> (17905)..(17905)
<223> Any amino acid

<220>
<221> MOD_RES
<222> (17908)..(17908)
<223> Any amino acid

<220>
<221> MOD_RES
<222> (17911)..(17911)
<223> Any amino acid

<220>
<221> MOD_RES
<222> (17917)..(17917)
<223> Any amino acid

<220>
<221> MOD_RES
<222> (17922)..(17922)
<223> Any amino acid

<220>
<221> MOD_RES
<222> (17924)..(17926)
<223> Any amino acid

<220>
<221> MOD_RES
<222> (17929)..(17929)
<223> Any amino acid

<220>
<221> MOD_RES
<222> (17933)..(17934)
<223> Any amino acid

<220>
<221> MOD_RES
<222> (17936)..(17936)
<223> Any amino acid

<220>
<221> MOD_RES
<222> (17943)..(17943)
<223> Any amino acid

<220>
<221> MOD_RES
<222> (17946)..(17947)
<223> Any amino acid

<220>
<221> MOD_RES
<222> (17949)..(17949)

<223> Any amino acid

<220>
<221> MOD_RES
<222> (17959)..(17959)
<223> Any amino acid

<220>
<221> MOD_RES
<222> (18089)..(18090)
<223> Any amino acid

<220>
<221> MOD_RES
<222> (18092)..(18092)
<223> Any amino acid

<220>
<221> MOD_RES
<222> (18099)..(18099)
<223> Any amino acid

<220>
<221> MOD_RES
<222> (18102)..(18103)
<223> Any amino acid

<220>
<221> MOD_RES
<222> (18105)..(18105)
<223> Any amino acid

<220>
<221> MOD_RES
<222> (18115)..(18115)
<223> Any amino acid

<220>
<221> MOD_RES
<222> (18125)..(18126)
<223> Any amino acid

<220>
<221> MOD_RES
<222> (18128)..(18128)
<223> Any amino acid

<220>
<221> MOD_RES
<222> (18140)..(18141)
<223> Any amino acid

<220>
<221> MOD_RES
<222> (18150)..(18150)
<223> Any amino acid

<220>

<221> MOD_RES
<222> (18152)..(18153)
<223> Any amino acid

<220>
<221> MOD_RES
<222> (18155)..(18157)
<223> Any amino acid

<220>
<221> MOD_RES
<222> (18161)..(18161)
<223> Any amino acid

<220>
<221> MOD_RES
<222> (18165)..(18165)
<223> Any amino acid

<220>
<221> MOD_RES
<222> (18167)..(18167)
<223> Any amino acid

<220>
<221> MOD_RES
<222> (18173)..(18177)
<223> Any amino acid

<220>
<221> MOD_RES
<222> (18179)..(18180)
<223> Any amino acid

<220>
<221> MOD_RES
<222> (18191)..(18191)
<223> Any amino acid

<220>
<221> MOD_RES
<222> (18198)..(18198)
<223> Any amino acid

<220>
<221> MOD_RES
<222> (18200)..(18200)
<223> Any amino acid

<220>
<221> MOD_RES
<222> (18204)..(18204)
<223> Any amino acid

<220>
<221> MOD_RES
<222> (18208)..(18208)
<223> Any amino acid

<220>
<221> MOD_RES
<222> (18217)..(18217)
<223> Any amino acid

<220>
<221> MOD_RES
<222> (18220)..(18220)
<223> Any amino acid

<220>
<221> MOD_RES
<222> (18223)..(18223)
<223> Any amino acid

<220>
<221> MOD_RES
<222> (18229)..(18229)
<223> Any amino acid

<220>
<221> MOD_RES
<222> (18234)..(18234)
<223> Any amino acid

<220>
<221> MOD_RES
<222> (18236)..(18238)
<223> Any amino acid

<220>
<221> MOD_RES
<222> (18241)..(18241)
<223> Any amino acid

<220>
<221> MOD_RES
<222> (18245)..(18246)
<223> Any amino acid

<220>
<221> MOD_RES
<222> (18248)..(18248)
<223> Any amino acid

<220>
<221> MOD_RES
<222> (18255)..(18255)
<223> Any amino acid

<220>
<221> MOD_RES
<222> (18258)..(18259)
<223> Any amino acid

<220>
<221> MOD_RES
<222> (18261)..(18261)

<223> Any amino acid

<220>
<221> MOD_RES
<222> (18271)..(18271)
<223> Any amino acid

<220>
<221> MOD_RES
<222> (18401)..(18402)
<223> Any amino acid

<220>
<221> MOD_RES
<222> (18404)..(18404)
<223> Any amino acid

<220>
<221> MOD_RES
<222> (18411)..(18411)
<223> Any amino acid

<220>
<221> MOD_RES
<222> (18414)..(18415)
<223> Any amino acid

<220>
<221> MOD_RES
<222> (18417)..(18417)
<223> Any amino acid

<220>
<221> MOD_RES
<222> (18427)..(18427)
<223> Any amino acid

<220>
<221> MOD_RES
<222> (18437)..(18438)
<223> Any amino acid

<220>
<221> MOD_RES
<222> (18440)..(18440)
<223> Any amino acid

<220>
<221> MOD_RES
<222> (18452)..(18453)
<223> Any amino acid

<220>
<221> MOD_RES
<222> (18462)..(18462)
<223> Any amino acid

<220>

<221> MOD_RES
<222> (18464)..(18465)
<223> Any amino acid

<220>
<221> MOD_RES
<222> (18467)..(18469)
<223> Any amino acid

<220>
<221> MOD_RES
<222> (18473)..(18473)
<223> Any amino acid

<220>
<221> MOD_RES
<222> (18477)..(18477)
<223> Any amino acid

<220>
<221> MOD_RES
<222> (18479)..(18479)
<223> Any amino acid

<220>
<221> MOD_RES
<222> (18485)..(18489)
<223> Any amino acid

<220>
<221> MOD_RES
<222> (18491)..(18492)
<223> Any amino acid

<220>
<221> MOD_RES
<222> (18503)..(18503)
<223> Any amino acid

<220>
<221> MOD_RES
<222> (18510)..(18510)
<223> Any amino acid

<220>
<221> MOD_RES
<222> (18512)..(18512)
<223> Any amino acid

<220>
<221> MOD_RES
<222> (18516)..(18516)
<223> Any amino acid

<220>
<221> MOD_RES
<222> (18520)..(18520)
<223> Any amino acid

<220>
<221> MOD_RES
<222> (18529)..(18529)
<223> Any amino acid

<220>
<221> MOD_RES
<222> (18532)..(18532)
<223> Any amino acid

<220>
<221> MOD_RES
<222> (18535)..(18535)
<223> Any amino acid

<220>
<221> MOD_RES
<222> (18541)..(18541)
<223> Any amino acid

<220>
<221> MOD_RES
<222> (18546)..(18546)
<223> Any amino acid

<220>
<221> MOD_RES
<222> (18548)..(18550)
<223> Any amino acid

<220>
<221> MOD_RES
<222> (18553)..(18553)
<223> Any amino acid

<220>
<221> MOD_RES
<222> (18557)..(18558)
<223> Any amino acid

<220>
<221> MOD_RES
<222> (18560)..(18560)
<223> Any amino acid

<220>
<221> MOD_RES
<222> (18567)..(18567)
<223> Any amino acid

<220>
<221> MOD_RES
<222> (18570)..(18571)
<223> Any amino acid

<220>
<221> MOD_RES
<222> (18573)..(18573)

<223> Any amino acid

<220>
<221> MOD_RES
<222> (18583)..(18583)
<223> Any amino acid

<220>
<221> MOD_RES
<222> (18713)..(18714)
<223> Any amino acid

<220>
<221> MOD_RES
<222> (18716)..(18716)
<223> Any amino acid

<220>
<221> MOD_RES
<222> (18723)..(18723)
<223> Any amino acid

<220>
<221> MOD_RES
<222> (18726)..(18727)
<223> Any amino acid

<220>
<221> MOD_RES
<222> (18729)..(18729)
<223> Any amino acid

<220>
<221> MOD_RES
<222> (18739)..(18739)
<223> Any amino acid

<220>
<221> MOD_RES
<222> (18749)..(18750)
<223> Any amino acid

<220>
<221> MOD_RES
<222> (18752)..(18752)
<223> Any amino acid

<220>
<221> MOD_RES
<222> (18764)..(18765)
<223> Any amino acid

<220>
<221> MOD_RES
<222> (18774)..(18774)
<223> Any amino acid

<220>

<221> MOD_RES
<222> (18776)..(18777)
<223> Any amino acid

<220>
<221> MOD_RES
<222> (18779)..(18781)
<223> Any amino acid

<220>
<221> MOD_RES
<222> (18785)..(18785)
<223> Any amino acid

<220>
<221> MOD_RES
<222> (18789)..(18789)
<223> Any amino acid

<220>
<221> MOD_RES
<222> (18791)..(18791)
<223> Any amino acid

<220>
<221> MOD_RES
<222> (18797)..(18801)
<223> Any amino acid

<220>
<221> MOD_RES
<222> (18803)..(18804)
<223> Any amino acid

<220>
<221> MOD_RES
<222> (18815)..(18815)
<223> Any amino acid

<220>
<221> MOD_RES
<222> (18822)..(18822)
<223> Any amino acid

<220>
<221> MOD_RES
<222> (18824)..(18824)
<223> Any amino acid

<220>
<221> MOD_RES
<222> (18828)..(18828)
<223> Any amino acid

<220>
<221> MOD_RES
<222> (18832)..(18832)
<223> Any amino acid

<220>
<221> MOD_RES
<222> (18841)..(18841)
<223> Any amino acid

<220>
<221> MOD_RES
<222> (18844)..(18844)
<223> Any amino acid

<220>
<221> MOD_RES
<222> (18847)..(18847)
<223> Any amino acid

<220>
<221> MOD_RES
<222> (18853)..(18853)
<223> Any amino acid

<220>
<221> MOD_RES
<222> (18858)..(18858)
<223> Any amino acid

<220>
<221> MOD_RES
<222> (18860)..(18862)
<223> Any amino acid

<220>
<221> MOD_RES
<222> (18865)..(18865)
<223> Any amino acid

<220>
<221> MOD_RES
<222> (18869)..(18870)
<223> Any amino acid

<220>
<221> MOD_RES
<222> (18872).. (18872)
<223> Any amino acid

<220>
<221> MOD_RES
<222> (18879)..(18879)
<223> Any amino acid

<220>
<221> MOD_RES
<222> (18882)..(18883)
<223> Any amino acid

<220>
<221> MOD_RES
<222> (18885)..(18885)

<223> Any amino acid

<220>
<221> MOD_RES
<222> (18895)..(18895)
<223> Any amino acid

<220>
<221> MOD_RES
<222> (19091)..(19093)
<223> Any amino acid

<220>
<221> MOD_RES
<222> (19097)..(19097)
<223> Any amino acid

<220>
<221> MOD_RES
<222> (19101)..(19101)
<223> Any amino acid

<220>
<221> MOD_RES
<222> (19103)..(19103)
<223> Any amino acid

<220>
<221> MOD_RES
<222> (19109)..(19113)
<223> Any amino acid

<220>
<221> MOD_RES
<222> (19115)..(19116)
<223> Any amino acid

<220>
<221> MOD_RES
<222> (19127)..(19127)
<223> Any amino acid

<220>
<221> MOD_RES
<222> (19134)..(19134)
<223> Any amino acid

<220>
<221> MOD_RES
<222> (19136)..(19136)
<223> Any amino acid

<220>
<221> MOD_RES
<222> (19140)..(19140)
<223> Any amino acid

<220>

```
<221> MOD_RES
<222> (19144)..(19144)
<223> Any amino acid

<220>
<221> MOD_RES
<222> (19153)..(19153)
<223> Any amino acid

<220>
<221> MOD_RES
<222> (19156)..(19156)
<223> Any amino acid

<220>
<221> MOD_RES
<222> (19159)..(19159)
<223> Any amino acid

<220>
<221> MOD_RES
<222> (19165)..(19165)
<223> Any amino acid

<220>
<221> MOD_RES
<222> (19170)..(19170)
<223> Any amino acid

<220>
<221> MOD_RES
<222> (19172)..(19174)
<223> Any amino acid

<220>
<221> MOD_RES
<222> (19177)..(19177)
<223> Any amino acid

<220>
<221> MOD_RES
<222> (19181)..(19182)
<223> Any amino acid

<220>
<221> MOD_RES
<222> (19184)..(19184)
<223> Any amino acid

<220>
<221> MOD_RES
<222> (19191)..(19191)
<223> Any amino acid

<220>
<221> MOD_RES
<222> (19194)..(19195)
<223> Any amino acid
```

```
<220>
<221> MOD_RES
<222> (19197)..(19197)
<223> Any amino acid

<220>
<221> MOD_RES
<222> (19207)..(19207)
<223> Any amino acid

<220>
<221> MOD_RES
<222> (19337)..(19338)
<223> Any amino acid

<220>
<221> MOD_RES
<222> (19340)..(19340)
<223> Any amino acid

<220>
<221> MOD_RES
<222> (19347)..(19347)
<223> Any amino acid

<220>
<221> MOD_RES
<222> (19350)..(19351)
<223> Any amino acid

<220>
<221> MOD_RES
<222> (19353)..(19353)
<223> Any amino acid

<220>
<221> MOD_RES
<222> (19363)..(19363)
<223> Any amino acid

<220>
<221> MOD_RES
<222> (19373)..(19374)
<223> Any amino acid

<220>
<221> MOD_RES
<222> (19376)..(19376)
<223> Any amino acid

<220>
<221> MOD_RES
<222> (19388)..(19389)
<223> Any amino acid

<220>
<221> MOD_RES
<222> (19398)..(19398)
```

<223> Any amino acid

<220>
<221> MOD_RES
<222> (19400).. (19401)
<223> Any amino acid

<220>
<221> MOD_RES
<222> (19403)..(19405)
<223> Any amino acid

<220>
<221> MOD_RES
<222> (19409)..(19409)
<223> Any amino acid

<220>
<221> MOD_RES
<222> (19413)..(19413)
<223> Any amino acid

<220>
<221> MOD_RES
<222> (19415)..(19415)
<223> Any amino acid

<220>
<221> MOD_RES
<222> (19421)..(19425)
<223> Any amino acid

<220>
<221> MOD_RES
<222> (19427)..(19428)
<223> Any amino acid

<220>
<221> MOD_RES
<222> (19439)..(19439)
<223> Any amino acid

<220>
<221> MOD_RES
<222> (19446)..(19446)
<223> Any amino acid

<220>
<221> MOD_RES
<222> (19448)..(19448)
<223> Any amino acid

<220>
<221> MOD_RES
<222> (19452)..(19452)
<223> Any amino acid

<220>

<220>
<221> MOD_RES
<222> (19456)..(19456)
<223> Any amino acid

<220>
<221> MOD_RES
<222> (19465)..(19465)
<223> Any amino acid

<220>
<221> MOD_RES
<222> (19468)..(19468)
<223> Any amino acid

<220>
<221> MOD_RES
<222> (19471)..(19471)
<223> Any amino acid

<220>
<221> MOD_RES
<222> (19477)..(19477)
<223> Any amino acid

<220>
<221> MOD_RES
<222> (19482)..(19482)
<223> Any amino acid

<220>
<221> MOD_RES
<222> (19484)..(19486)
<223> Any amino acid

<220>
<221> MOD_RES
<222> (19489)..(19489)
<223> Any amino acid

<220>
<221> MOD_RES
<222> (19493)..(19494)
<223> Any amino acid

<220>
<221> MOD_RES
<222> (19496)..(19496)
<223> Any amino acid

<220>
<221> MOD_RES
<222> (19503)..(19503)
<223> Any amino acid

<220>
<221> MOD_RES
<222> (19506)..(19507)
<223> Any amino acid

<220>
<221> MOD_RES
<222> (19509)..(19509)
<223> Any amino acid

<220>
<221> MOD_RES
<222> (19519)..(19519)
<223> Any amino acid

<220>
<221> MOD_RES
<222> (19649)..(19650)
<223> Any amino acid

<220>
<221> MOD_RES
<222> (19652)..(19652)
<223> Any amino acid

<220>
<221> MOD_RES
<222> (19659)..(19659)
<223> Any amino acid

<220>
<221> MOD_RES
<222> (19662)..(19663)
<223> Any amino acid

<220>
<221> MOD_RES
<222> (19665)..(19665)
<223> Any amino acid

<220>
<221> MOD_RES
<222> (19675)..(19675)
<223> Any amino acid

<220>
<221> MOD_RES
<222> (19685)..(19686)
<223> Any amino acid

<220>
<221> MOD_RES
<222> (19688)..(19688)
<223> Any amino acid

<220>
<221> MOD_RES
<222> (19700)..(19701)
<223> Any amino acid

<220>
<221> MOD_RES
<222> (19710)..(19710)

<223> Any amino acid

<220>
<221> MOD_RES
<222> (19712)..(19713)
<223> Any amino acid

<220>
<221> MOD_RES
<222> (19715)..(19717)
<223> Any amino acid

<220>
<221> MOD_RES
<222> (19721)..(19721)
<223> Any amino acid

<220>
<221> MOD_RES
<222> (19725)..(19725)
<223> Any amino acid

<220>
<221> MOD_RES
<222> (19727)..(19727)
<223> Any amino acid

<220>
<221> MOD_RES
<222> (19733)..(19737)
<223> Any amino acid

<220>
<221> MOD_RES
<222> (19739)..(19740)
<223> Any amino acid

<220>
<221> MOD_RES
<222> (19751)..(19751)
<223> Any amino acid

<220>
<221> MOD_RES
<222> (19758)..(19758)
<223> Any amino acid

<220>
<221> MOD_RES
<222> (19760)..(19760)
<223> Any amino acid

<220>
<221> MOD_RES
<222> (19764)..(19764)
<223> Any amino acid

<220>

<221> MOD_RES
<222> (19768)..(19768)
<223> Any amino acid

<220>
<221> MOD_RES
<222> (19777)..(19777)
<223> Any amino acid

<220>
<221> MOD_RES
<222> (19780)..(19780)
<223> Any amino acid

<220>
<221> MOD_RES
<222> (19783)..(19783)
<223> Any amino acid

<220>
<221> MOD_RES
<222> (19789)..(19789)
<223> Any amino acid

<220>
<221> MOD_RES
<222> (19794)..(19794)
<223> Any amino acid

<220>
<221> MOD_RES
<222> (19796)..(19798)
<223> Any amino acid

<220>
<221> MOD_RES
<222> (19801)..(19801)
<223> Any amino acid

<220>
<221> MOD_RES
<222> (19805)..(19806)
<223> Any amino acid

<220>
<221> MOD_RES
<222> (19808)..(19808)
<223> Any amino acid

<220>
<221> MOD_RES
<222> (19815)..(19815)
<223> Any amino acid

<220>
<221> MOD_RES
<222> (19818)..(19819)
<223> Any amino acid

<220>
<221> MOD_RES
<222> (19821)..(19821)
<223> Any amino acid

<220>
<221> MOD_RES
<222> (19831)..(19831)
<223> Any amino acid

<220>
<221> MOD_RES
<222> (19960)..(19961)
<223> Any amino acid

<220>
<221> MOD_RES
<222> (19963)..(19963)
<223> Any amino acid

<220>
<221> MOD_RES
<222> (19970)..(19970)
<223> Any amino acid

<220>
<221> MOD_RES
<222> (19973)..(19974)
<223> Any amino acid

<220>
<221> MOD_RES
<222> (19976)..(19976)
<223> Any amino acid

<220>
<221> MOD_RES
<222> (19986)..(19986)
<223> Any amino acid

<220>
<221> MOD_RES
<222> (19996)..(19997)
<223> Any amino acid

<220>
<221> MOD_RES
<222> (19999)..(19999)
<223> Any amino acid

<220>
<221> MOD_RES
<222> (20011)..(20012)
<223> Any amino acid

<220>
<221> MOD_RES
<222> (20021)..(20021)

<223> Any amino acid

<220>
<221> MOD_RES
<222> (20023)..(20024)
<223> Any amino acid

<220>
<221> MOD_RES
<222> (20026)..(20028)
<223> Any amino acid

<220>
<221> MOD_RES
<222> (20032)..(20032)
<223> Any amino acid

<220>
<221> MOD_RES
<222> (20036)..(20036)
<223> Any amino acid

<220>
<221> MOD_RES
<222> (20038)..(20038)
<223> Any amino acid

<220>
<221> MOD_RES
<222> (20044)..(20048)
<223> Any amino acid

<220>
<221> MOD_RES
<222> (20050)..(20051)
<223> Any amino acid

<220>
<221> MOD_RES
<222> (20062)..(20062)
<223> Any amino acid

<220>
<221> MOD_RES
<222> (20069)..(20069)
<223> Any amino acid

<220>
<221> MOD_RES
<222> (20071)..(20071)
<223> Any amino acid

<220>
<221> MOD_RES
<222> (20075)..(20075)
<223> Any amino acid

<220>

<221> MOD_RES
<222> (20079)..(20079)
<223> Any amino acid

<220>
<221> MOD_RES
<222> (20088)..(20088)
<223> Any amino acid

<220>
<221> MOD_RES
<222> (20091)..(20091)
<223> Any amino acid

<220>
<221> MOD_RES
<222> (20094)..(20094)
<223> Any amino acid

<220>
<221> MOD_RES
<222> (20100)..(20100)
<223> Any amino acid

<220>
<221> MOD_RES
<222> (20105)..(20105)
<223> Any amino acid

<220>
<221> MOD_RES
<222> (20107)..(20109)
<223> Any amino acid

<220>
<221> MOD_RES
<222> (20112)..(20112)
<223> Any amino acid

<220>
<221> MOD_RES
<222> (20116)..(20117)
<223> Any amino acid

<220>
<221> MOD_RES
<222> (20119)..(20119)
<223> Any amino acid

<220>
<221> MOD_RES
<222> (20126)..(20126)
<223> Any amino acid

<220>
<221> MOD_RES
<222> (20129)..(20130)
<223> Any amino acid

<220>
<221> MOD_RES
<222> (20132)..(20132)
<223> Any amino acid

<220>
<221> MOD_RES
<222> (20142)..(20142)
<223> Any amino acid

<220>
<221> MOD_RES
<222> (20272)..(20273)
<223> Any amino acid

<220>
<221> MOD_RES
<222> (20275)..(20275)
<223> Any amino acid

<220>
<221> MOD_RES
<222> (20282)..(20282)
<223> Any amino acid

<220>
<221> MOD_RES
<222> (20285)..(20286)
<223> Any amino acid

<220>
<221> MOD_RES
<222> (20288)..(20288)
<223> Any amino acid

<220>
<221> MOD_RES
<222> (20298)..(20298)
<223> Any amino acid

<220>
<221> MOD_RES
<222> (20308)..(20309)
<223> Any amino acid

<220>
<221> MOD_RES
<222> (20311)..(20311)
<223> Any amino acid

<220>
<221> MOD_RES
<222> (20323)..(20324)
<223> Any amino acid

<220>
<221> MOD_RES
<222> (20333)..(20333)

<223> Any amino acid

<220>
<221> MOD_RES
<222> (20335)..(20336)
<223> Any amino acid

<220>
<221> MOD_RES
<222> (20806)..(20808)
<223> Any amino acid

<220>
<221> MOD_RES
<222> (20812)..(20812)
<223> Any amino acid

<220>
<221> MOD_RES
<222> (20816).. (20816)
<223> Any amino acid

<220>
<221> MOD_RES
<222> (20818)..(20818)
<223> Any amino acid

<220>
<221> MOD_RES
<222> (20824)..(20828)
<223> Any amino acid

<220>
<221> MOD_RES
<222> (20830)..(20831)
<223> Any amino acid

<400> 12

Met Leu Lys Pro Ser Gly Leu Pro Gly Ser Ser Ser Pro Thr Arg Ser
1                   5                   10                  15

Leu Met Thr Gly Ser Arg Ser Thr Lys Ala Thr Pro Glu Met Asp Ser
            20                  25                  30

Gly Leu Thr Gly Ala Thr Leu Ser Pro Lys Thr Ser Thr Gly Ala Ile
            35                  40                  45

Val Val Thr Glu His Thr Leu Pro Phe Thr Ser Pro Asp Lys Thr Leu
        50                  55                  60

Ala Ser Pro Thr Ser Ser Val Val Gly Arg Thr Thr Gln Ser Leu Gly
65                  70                  75                  80

Val Met Ser Ser Ala Leu Pro Glu Ser Thr Ser Arg Gly Met Thr His
                85                  90                  95

Ser Glu Gln Arg Thr Ser Pro Ser Leu Ser Pro Gln Val Asn Gly Thr
            100                 105                 110

Pro Ser Arg Asn Tyr Pro Ala Thr Ser Met Val Ser Gly Leu Ser Ser
            115                 120                 125

Pro Arg Thr Arg Thr Ser Ser Thr Glu Gly Asn Phe Thr Lys Glu Ala
        130                 135                 140

Ser Thr Tyr Thr Leu Thr Val Glu Thr Thr Ser Gly Pro Val Thr Glu
145                 150                 155                 160

Lys Tyr Thr Val Pro Thr Glu Thr Ser Thr Thr Glu Gly Asp Ser Thr
                165                 170                 175

Glu Thr Pro Trp Asp Thr Arg Tyr Ile Pro Val Lys Ile Thr Ser Pro
            180                 185                 190

Met Lys Thr Phe Ala Asp Ser Thr Ala Ser Lys Glu Asn Ala Pro Val
            195                 200                 205

Ser Met Thr Pro Ala Glu Thr Thr Val Thr Asp Ser His Thr Pro Gly
        210                 215                 220

Arg Thr Asn Pro Ser Phe Gly Thr Leu Tyr Ser Ser Phe Leu Asp Leu
225                 230                 235                 240

288

Ser Pro Lys Gly Thr Pro Asn Ser Arg Gly Glu Thr Ser Leu Glu Leu
              245                 250                 255

Ile Leu Ser Thr Thr Gly Tyr Pro Phe Ser Ser Pro Glu Pro Gly Ser
              260                 265                 270

Ala Gly His Ser Arg Ile Ser Thr Ser Ala Pro Leu Ser Ser Ser Ala
              275                 280                 285

Ser Val Leu Asp Asn Lys Ile Ser Glu Thr Ser Ile Phe Ser Gly Gln
    290                 295                 300

Ser Leu Thr Ser Pro Leu Ser Pro Gly Val Pro Glu Ala Arg Ala Ser
305                 310                 315                 320

Thr Met Pro Asn Ser Ala Ile Pro Phe Ser Met Thr Leu Ser Asn Ala
              325                 330                 335

Glu Thr Ser Ala Glu Arg Val Arg Ser Thr Ile Ser Ser Leu Gly Thr
              340                 345                 350

Pro Ser Ile Ser Thr Lys Gln Thr Ala Glu Thr Ile Leu Thr Phe His
              355                 360                 365

Ala Phe Ala Glu Thr Met Asp Ile Pro Ser Thr His Ile Ala Lys Thr
    370                 375                 380

Leu Ala Ser Glu Trp Leu Gly Ser Pro Gly Thr Leu Gly Gly Thr Ser
385                 390                 395                 400

Thr Ser Ala Leu Thr Thr Thr Ser Pro Ser Thr Thr Leu Val Ser Glu
              405                 410                 415

Glu Thr Asn Thr His His Ser Thr Ser Gly Lys Glu Thr Glu Gly Thr
              420                 425                 430

Leu Asn Thr Ser Met Thr Pro Leu Glu Thr Ser Ala Pro Gly Glu Glu
              435                 440                 445

Ser Glu Met Thr Ala Thr Leu Val Pro Thr Leu Gly Phe Thr Thr Leu
    450                 455                 460

Asp Ser Lys Ile Arg Ser Pro Ser Gln Val Ser Ser Ser His Pro Thr
465                 470                 475                 480

Arg Glu Leu Arg Thr Thr Gly Ser Thr Ser Gly Arg Gln Ser Ser Ser
              485                 490                 495

```
Thr Ala Ala His Gly Ser Ser Asp Ile Leu Arg Ala Thr Thr Ser Ser
            500                 505             510

Thr Ser Lys Ala Ser Ser Trp Thr Ser Glu Ser Thr Ala Gln Gln Phe
            515                 520             525

Ser Glu Pro Gln His Thr Gln Trp Val Glu Thr Ser Pro Ser Met Lys
            530                 535             540

Thr Glu Arg Pro Pro Ala Ser Thr Ser Val Ala Ala Pro Ile Thr Thr
545                 550             555             560

Ser Val Pro Ser Val Val Ser Gly Phe Thr Thr Leu Lys Thr Ser Ser
            565                 570             575

Thr Lys Gly Ile Trp Leu Glu Glu Thr Ser Ala Asp Thr Leu Ile Gly
            580                 585             590

Glu Ser Thr Ala Gly Pro Thr Thr His Gln Phe Ala Val Pro Thr Gly
            595                 600             605

Ile Ser Met Thr Gly Gly Ser Ser Thr Arg Gly Ser Gln Gly Thr Thr
            610                 615             620

His Leu Leu Thr Arg Ala Thr Ala Ser Ser Glu Thr Ser Ala Asp Leu
625                 630             635             640

Thr Leu Ala Thr Asn Gly Val Pro Val Ser Val Ser Pro Ala Val Ser
            645                 650             655

Lys Thr Ala Ala Gly Ser Ser Pro Pro Gly Gly Thr Lys Pro Ser Tyr
            660                 665             670

Thr Met Val Ser Ser Val Ile Pro Glu Thr Ser Ser Leu Gln Ser Ser
            675                 680             685

Ala Phe Arg Glu Gly Thr Ser Leu Gly Leu Thr Pro Leu Asn Thr Arg
            690                 695             700

His Pro Phe Ser Ser Pro Glu Pro Asp Ser Ala Gly His Thr Lys Ile
705                 710             715             720

Ser Thr Ser Ile Pro Leu Leu Ser Ser Ala Ser Val Leu Glu Asp Lys
            725                 730             735

Val Ser Ala Thr Ser Thr Phe Ser His His Lys Ala Thr Ser Ser Ile
            740                 745             750
```

290

```
Thr Thr Gly Thr Pro Glu Ile Ser Thr Lys Thr Lys Pro Ser Ser Ala
        755             760         765

Val Leu Ser Ser Met Thr Leu Ser Asn Ala Ala Thr Ser Pro Glu Arg
    770             775             780

Val Arg Asn Ala Thr Ser Pro Leu Thr His Pro Ser Pro Ser Gly Glu
785             790             795             800

Glu Thr Ala Gly Ser Val Leu Thr Leu Ser Thr Ser Ala Glu Thr Thr
            805             810             815

Asp Ser Pro Asn Ile His Pro Thr Gly Thr Leu Thr Ser Glu Ser Ser
            820             825             830

Glu Ser Pro Ser Thr Leu Ser Leu Pro Ser Val Ser Gly Val Lys Thr
        835             840             845

Thr Phe Ser Ser Ser Thr Pro Ser Thr His Leu Phe Thr Ser Gly Glu
    850             855             860

Glu Thr Glu Glu Thr Ser Asn Pro Ser Val Ser Gln Pro Glu Thr Ser
865             870             875             880

Val Ser Arg Val Arg Thr Thr Leu Ala Ser Thr Ser Val Pro Thr Pro
            885             890             895

Val Phe Pro Thr Met Asp Thr Trp Pro Thr Arg Ser Ala Gln Phe Ser
            900             905             910

Ser Ser His Leu Val Ser Glu Leu Arg Ala Thr Ser Ser Thr Ser Val
    915             920             925

Thr Asn Ser Thr Gly Ser Ala Leu Pro Lys Ile Ser His Leu Thr Gly
    930             935             940

Thr Ala Thr Met Ser Gln Thr Asn Arg Asp Thr Phe Asn Asp Ser Ala
945             950             955             960

Ala Pro Gln Ser Thr Thr Trp Pro Glu Thr Ser Pro Arg Phe Lys Thr
            965             970             975

Gly Leu Pro Ser Ala Thr Thr Thr Val Ser Thr Ser Ala Thr Ser Leu
        980             985             990

Ser Ala Thr Val Met Val Ser Lys  Phe Thr Ser Pro Ala  Thr Ser Ser
```

| | 995 | | | | | | 1000 | | | | | | 1005 | | |

Met Glu Ala Thr Ser Ile Arg Glu Pro Ser Thr Thr Ile Leu Thr
1010 1015 1020

Thr Glu Thr Thr Asn Gly Pro Gly Ser Met Ala Val Ala Ser Thr
1025 1030 1035

Asn Ile Pro Ile Gly Lys Gly Tyr Ile Thr Glu Gly Arg Leu Asp
1040 1045 1050

Thr Ser His Leu Pro Ile Gly Thr Thr Ala Ser Ser Glu Thr Ser
1055 1060 1065

Met Asp Phe Thr Met Ala Lys Glu Ser Val Ser Met Ser Val Ser
1070 1075 1080

Pro Ser Gln Ser Met Asp Ala Ala Gly Ser Ser Thr Pro Gly Arg
1085 1090 1095

Thr Ser Gln Phe Val Asp Thr Phe Ser Asp Asp Val Tyr His Leu
1100 1105 1110

Thr Ser Arg Glu Ile Thr Ile Pro Arg Asp Gly Thr Ser Ser Ala
1115 1120 1125

Leu Thr Pro Gln Met Thr Ala Thr His Pro Pro Ser Pro Asp Pro
1130 1135 1140

Gly Ser Ala Arg Ser Thr Trp Leu Gly Ile Leu Ser Ser Ser Pro
1145 1150 1155

Ser Ser Pro Thr Pro Lys Val Thr Met Ser Ser Thr Phe Ser Thr
1160 1165 1170

Gln Arg Val Thr Thr Ser Met Ile Met Asp Thr Val Glu Thr Ser
1175 1180 1185

Arg Trp Asn Met Pro Asn Leu Pro Ser Thr Thr Ser Leu Thr Pro
1190 1195 1200

Ser Asn Ile Pro Thr Ser Gly Ala Ile Gly Lys Ser Thr Leu Val
1205 1210 1215

Pro Leu Asp Thr Pro Ser Pro Ala Thr Ser Leu Glu Ala Ser Glu
1220 1225 1230

Gly Gly Leu Pro Thr Leu Ser Thr Tyr Pro Glu Ser Thr Asn Thr
    1235                1240            1245

Pro Ser Ile His Leu Gly Ala His Ala Ser Ser Glu Ser Pro Ser
    1250                1255            1260

Thr Ile Lys Leu Thr Met Ala Ser Val Val Lys Pro Gly Ser Tyr
    1265                1270            1275

Thr Pro Leu Thr Phe Pro Ser Ile Glu Thr His Ile His Val Ser
    1280                1285            1290

Thr Ala Arg Met Ala Tyr Ser Ser Gly Ser Ser Pro Glu Met Thr
    1295                1300            1305

Ala Pro Gly Glu Thr Asn Thr Gly Ser Thr Trp Asp Pro Thr Thr
    1310                1315            1320

Tyr Ile Thr Thr Thr Asp Pro Lys Asp Thr Ser Ser Ala Gln Val
    1325                1330            1335

Ser Thr Pro His Ser Val Arg Thr Leu Arg Thr Thr Glu Asn His
    1340                1345            1350

Pro Lys Thr Glu Ser Ala Thr Pro Ala Ala Tyr Ser Gly Ser Pro
    1355                1360            1365

Lys Ile Ser Ser Ser Pro Asn Leu Thr Ser Pro Ala Thr Lys Ala
    1370                1375            1380

Trp Thr Ile Thr Asp Thr Thr Glu His Ser Thr Gln Leu His Tyr
    1385                1390            1395

Thr Lys Leu Ala Glu Lys Ser Ser Gly Phe Glu Thr Gln Ser Ala
    1400                1405            1410

Pro Gly Pro Val Ser Val Val Ile Pro Thr Ser Pro Thr Ile Gly
    1415                1420            1425

Ser Ser Thr Leu Glu Leu Thr Ser Asp Val Pro Gly Glu Pro Leu
    1430                1435            1440

Val Leu Ala Pro Ser Glu Gln Thr Thr Ile Thr Leu Pro Met Ala
    1445                1450            1455

Thr Trp Leu Ser Thr Ser Leu Thr Glu Glu Met Ala Ser Thr Asp
    1460                1465            1470

```
Leu Asp Ile Ser Ser Pro Ser  Ser Pro Met Ser Thr  Phe Ala Ile
    1475            1480                1485

Phe Pro Pro Met Ser Thr Pro  Ser His Glu Leu Ser  Lys Ser Glu
    1490            1495                1500

Ala Asp Thr Ser Ala Ile Arg  Asn Thr Asp Ser Thr  Thr Leu Asp
    1505            1510                1515

Gln His Leu Gly Ile Arg Ser  Leu Gly Arg Thr Gly  Asp Leu Thr
    1520            1525                1530

Thr Val Pro Ile Thr Pro Leu  Thr Thr Thr Trp Thr  Ser Val Ile
    1535            1540                1545

Glu His Ser Thr Gln Ala Gln  Asp Thr Leu Ser Ala  Thr Met Ser
    1550            1555                1560

Pro Thr His Val Thr Gln Ser  Leu Lys Asp Gln Thr  Ser Ile Pro
    1565            1570                1575

Ala Ser Ala Ser Pro Ser His  Leu Thr Glu Val Tyr  Pro Glu Leu
    1580            1585                1590

Gly Thr Gln Gly Arg Ser Ser  Ser Glu Ala Thr Thr  Phe Trp Lys
    1595            1600                1605

Pro Ser Thr Asp Thr Leu Ser  Arg Glu Ile Glu Thr  Gly Pro Thr
    1610            1615                1620

Asn Ile Gln Ser Thr Pro Pro  Met Asp Asn Thr Thr  Thr Gly Ser
    1625            1630                1635

Ser Ser Ser Gly Val Thr Leu  Gly Ile Ala His Leu  Pro Ile Gly
    1640            1645                1650

Thr Ser Ser Pro Ala Glu Thr  Ser Thr Asn Met Ala  Leu Glu Arg
    1655            1660                1665

Arg Ser Ser Thr Ala Thr Val  Ser Met Ala Gly Thr  Met Gly Leu
    1670            1675                1680

Leu Val Thr Ser Ala Pro Gly  Arg Ser Ile Ser Gln  Ser Leu Gly
    1685            1690                1695

Arg Val Ser Ser Val Leu Ser  Glu Ser Thr Thr Glu  Gly Val Thr
    1700            1705                1710
```

294

```
Asp Ser  Ser Lys Gly Ser Ser  Pro Arg Leu Asn Thr  Gln Gly Asn
    1715             1720             1725

Thr Ala  Leu Ser Ser Ser Leu  Glu Pro Ser Tyr Ala  Glu Gly Ser
    1730             1735              1740

Gln Met  Ser Thr Ser Ile Pro  Leu Thr Ser Ser Pro  Thr Thr Pro
    1745             1750             1755

Asp Val  Glu Phe Ile Gly Gly  Ser Thr Phe Trp Thr  Lys Glu Val
    1760             1765             1770

Thr Thr  Val Met Thr Ser Asp  Ile Ser Lys Ser Ser  Ala Arg Thr
    1775             1780             1785

Glu Ser  Ser Ser Ala Thr Leu  Met Ser Thr Ala Leu  Gly Ser Thr
    1790             1795             1800

Glu Asn  Thr Gly Lys Glu Lys  Leu Arg Thr Ala Ser  Met Asp Leu
    1805             1810             1815

Pro Ser  Pro Thr Pro Ser Met  Glu Val Thr Pro Trp  Ile Ser Leu
    1820             1825             1830

Thr Leu  Ser Asn Ala Pro Asn  Thr Thr Asp Ser Leu  Asp Leu Ser
    1835             1840             1845

His Gly  Val His Thr Ser Ser  Ala Gly Thr Leu Ala  Thr Asp Arg
    1850             1855             1860

Ser Leu  Asn Thr Gly Val Thr  Arg Ala Ser Arg Leu  Glu Asn Gly
    1865             1870             1875

Ser Asp  Thr Ser Ser Lys Ser  Leu Ser Met Gly Asn  Ser Thr His
    1880             1885             1890

Thr Ser  Met Thr Asp Thr Glu  Lys Ser Glu Val Ser  Ser Ser Ile
    1895             1900             1905

His Pro  Arg Pro Glu Thr Ser  Ala Pro Gly Ala Glu  Thr Thr Leu
    1910             1915             1920

Thr Ser  Thr Pro Gly Asn Arg  Ala Ile Ser Leu Thr  Leu Pro Phe
    1925             1930             1935

Ser Ser  Ile Pro Val Glu Glu  Val Ile Ser Thr Gly  Ile Thr Ser
```

|  | 1940 | | | | | 1945 | | | | | 1950 | | |
|---|---|---|---|---|---|---|---|---|---|---|---|---|---|---|
| Gly | Pro | Asp | Ile | Asn | Ser | Ala | Pro | Met | Thr | His | Ser | Pro | Ile | Thr |
|  | 1955 | | | | | 1960 | | | | | 1965 | | | |

| Pro | Pro | Thr | Ile | Val | Trp | Thr | Ser | Thr | Gly | Thr | Ile | Glu | Gln | Ser |
|---|---|---|---|---|---|---|---|---|---|---|---|---|---|---|
|  | 1970 | | | | | 1975 | | | | | 1980 | | | |

| Thr | Gln | Pro | Leu | His | Ala | Val | Ser | Ser | Glu | Lys | Val | Ser | Val | Gln |
|---|---|---|---|---|---|---|---|---|---|---|---|---|---|---|
|  | 1985 | | | | | 1990 | | | | | 1995 | | | |

| Thr | Gln | Ser | Thr | Pro | Tyr | Val | Asn | Ser | Val | Ala | Val | Ser | Ala | Ser |
|---|---|---|---|---|---|---|---|---|---|---|---|---|---|---|
|  | 2000 | | | | | 2005 | | | | | 2010 | | | |

| Pro | Thr | His | Glu | Asn | Ser | Val | Ser | Ser | Gly | Ser | Ser | Thr | Ser | Ser |
|---|---|---|---|---|---|---|---|---|---|---|---|---|---|---|
|  | 2015 | | | | | 2020 | | | | | 2025 | | | |

| Pro | Tyr | Ser | Ser | Ala | Ser | Leu | Glu | Ser | Leu | Asp | Ser | Thr | Ile | Ser |
|---|---|---|---|---|---|---|---|---|---|---|---|---|---|---|
|  | 2030 | | | | | 2035 | | | | | 2040 | | | |

| Arg | Arg | Asn | Ala | Ile | Thr | Ser | Trp | Leu | Trp | Asp | Leu | Thr | Thr | Ser |
|---|---|---|---|---|---|---|---|---|---|---|---|---|---|---|
|  | 2045 | | | | | 2050 | | | | | 2055 | | | |

| Leu | Pro | Thr | Thr | Thr | Trp | Pro | Ser | Thr | Ser | Leu | Ser | Glu | Ala | Leu |
|---|---|---|---|---|---|---|---|---|---|---|---|---|---|---|
|  | 2060 | | | | | 2065 | | | | | 2070 | | | |

| Ser | Ser | Gly | His | Ser | Gly | Val | Ser | Asn | Pro | Ser | Ser | Thr | Thr | Thr |
|---|---|---|---|---|---|---|---|---|---|---|---|---|---|---|
|  | 2075 | | | | | 2080 | | | | | 2085 | | | |

| Glu | Phe | Pro | Leu | Phe | Ser | Ala | Ala | Ser | Thr | Ser | Ala | Ala | Lys | Gln |
|---|---|---|---|---|---|---|---|---|---|---|---|---|---|---|
|  | 2090 | | | | | 2095 | | | | | 2100 | | | |

| Arg | Asn | Pro | Glu | Thr | Glu | Thr | His | Gly | Pro | Gln | Asn | Thr | Ala | Ala |
|---|---|---|---|---|---|---|---|---|---|---|---|---|---|---|
|  | 2105 | | | | | 2110 | | | | | 2115 | | | |

| Ser | Thr | Leu | Asn | Thr | Asp | Ala | Ser | Ser | Val | Thr | Gly | Leu | Ser | Glu |
|---|---|---|---|---|---|---|---|---|---|---|---|---|---|---|
|  | 2120 | | | | | 2125 | | | | | 2130 | | | |

| Thr | Pro | Val | Gly | Ala | Ser | Ile | Ser | Ser | Glu | Val | Pro | Leu | Pro | Met |
|---|---|---|---|---|---|---|---|---|---|---|---|---|---|---|
|  | 2135 | | | | | 2140 | | | | | 2145 | | | |

| Ala | Ile | Thr | Ser | Arg | Ser | Asp | Val | Ser | Gly | Leu | Thr | Ser | Glu | Ser |
|---|---|---|---|---|---|---|---|---|---|---|---|---|---|---|
|  | 2150 | | | | | 2155 | | | | | 2160 | | | |

| Thr | Ala | Asn | Pro | Ser | Leu | Gly | Thr | Ala | Ser | Ser | Ala | Gly | Thr | Lys |
|---|---|---|---|---|---|---|---|---|---|---|---|---|---|---|
|  | 2165 | | | | | 2170 | | | | | 2175 | | | |

Leu Thr Arg Thr Ile Ser Leu Pro Thr Ser Glu Ser Leu Val Ser
2180 2185 2190

Phe Arg Met Asn Lys Asp Pro Trp Thr Val Ser Ile Pro Leu Gly
2195 2200 2205

Ser His Pro Thr Thr Asn Thr Glu Thr Ser Ile Pro Val Asn Ser
2210 2215 2220

Ala Gly Pro Pro Gly Leu Ser Thr Val Ala Ser Asp Val Ile Asp
2225 2230 2235

Thr Pro Ser Asp Gly Ala Glu Ser Ile Pro Thr Val Ser Phe Ser
2240 2245 2250

Pro Ser Pro Asp Thr Glu Val Thr Thr Ile Ser His Phe Pro Glu
2255 2260 2265

Lys Thr Thr His Ser Phe Arg Thr Ile Ser Ser Leu Thr His Glu
2270 2275 2280

Leu Thr Ser Arg Val Thr Pro Ile Pro Gly Asp Trp Met Ser Ser
2285 2290 2295

Ala Met Ser Thr Lys Pro Thr Gly Ala Ser Pro Ser Ile Thr Leu
2300 2305 2310

Gly Glu Arg Arg Thr Ile Thr Ser Ala Ala Pro Thr Thr Ser Pro
2315 2320 2325

Ile Val Leu Thr Ala Ser Phe Thr Glu Thr Ser Thr Val Ser Leu
2330 2335 2340

Asp Asn Glu Thr Thr Val Lys Thr Ser Asp Ile Leu Asp Ala Arg
2345 2350 2355

Lys Thr Asn Glu Leu Pro Ser Asp Ser Ser Ser Ser Ser Asp Leu
2360 2365 2370

Ile Asn Thr Ser Ile Ala Ser Ser Thr Met Asp Val Thr Lys Thr
2375 2380 2385

Ala Ser Ile Ser Pro Thr Ser Ile Ser Gly Met Thr Ala Ser Ser
2390 2395 2400

Ser Pro Ser Leu Phe Ser Ser Asp Arg Pro Gln Val Pro Thr Ser
2405 2410 2415

297

```
Thr Thr Glu Thr Asn Thr Ala  Thr Ser Pro Ser Val  Ser Ser Asn
    2420            2425           2430

Thr Tyr Ser Leu Asp Gly Gly  Ser Asn Val Gly Gly  Thr Pro Ser
    2435            2440           2445

Thr Leu Pro Pro Phe Thr Ile  Thr His Pro Val Glu  Thr Ser Ser
    2450            2455           2460

Ala Leu Leu Ala Trp Ser Arg  Pro Val Arg Thr Phe  Ser Thr Met
    2465            2470           2475

Val Ser Thr Asp Thr Ala Ser  Gly Glu Asn Pro Thr  Ser Ser Asn
    2480            2485           2490

Ser Val Val Thr Ser Val Pro  Ala Pro Gly Thr Trp  Ala Ser Val
    2495            2500           2505

Gly Ser Thr Thr Asp Leu Pro  Ala Met Gly Phe Leu  Lys Thr Ser
    2510            2515           2520

Pro Ala Gly Glu Ala His Ser  Leu Leu Ala Ser Thr  Ile Glu Pro
    2525            2530           2535

Ala Thr Ala Phe Thr Pro His  Leu Ser Ala Ala Val  Val Thr Gly
    2540            2545           2550

Ser Ser Ala Thr Ser Glu Ala  Ser Leu Leu Thr Thr  Ser Glu Ser
    2555            2560           2565

Lys Ala Ile His Ser Ser Pro  Gln Thr Pro Thr Thr  Pro Thr Ser
    2570            2575           2580

Gly Ala Asn Trp Glu Thr Ser  Ala Thr Pro Glu Ser  Leu Leu Val
    2585            2590           2595

Val Thr Glu Thr Ser Asp Thr  Thr Leu Thr Ser Lys  Ile Leu Val
    2600            2605           2610

Thr Asp Thr Ile Leu Phe Ser  Thr Val Ser Thr Pro  Pro Ser Lys
    2615            2620           2625

Phe Pro Ser Thr Gly Thr Leu  Ser Gly Ala Ser Phe  Pro Thr Leu
    2630            2635           2640

Leu Pro Asp Thr Pro Ala Ile  Pro Leu Thr Ala Thr  Glu Pro Thr
    2645            2650           2655
```

298

```
Ser Ser Leu Ala Thr Ser Phe Asp Ser Thr Pro Leu Val Thr Ile
    2660              2665          2670

Ala Ser Asp Ser Leu Gly Thr Val Pro Glu Thr Thr Leu Thr Met
    2675              2680          2685

Ser Glu Thr Ser Asn Gly Asp Ala Leu Val Leu Lys Thr Val Ser
    2690              2695          2700

Asn Pro Asp Arg Ser Ile Pro Gly Ile Thr Ile Gln Gly Val Thr
    2705              2710          2715

Glu Ser Pro Leu His Pro Ser Ser Thr Ser Pro Ser Lys Ile Val
    2720              2725          2730

Ala Pro Arg Asn Thr Thr Tyr Glu Gly Ser Ile Thr Val Ala Leu
    2735              2740          2745

Ser Thr Leu Pro Ala Gly Thr Thr Gly Ser Leu Val Phe Ser Gln
    2750              2755          2760

Ser Ser Glu Asn Ser Glu Thr Thr Ala Leu Val Asp Ser Ser Ala
    2765              2770          2775

Gly Leu Glu Arg Ala Ser Val Met Pro Leu Thr Thr Gly Ser Gln
    2780              2785          2790

Gly Met Ala Ser Ser Gly Gly Ile Arg Ser Gly Ser Thr His Ser
    2795              2800          2805

Thr Gly Thr Lys Thr Phe Ser Ser Leu Pro Leu Thr Met Asn Pro
    2810              2815          2820

Gly Glu Val Thr Ala Met Ser Glu Ile Thr Thr Asn Arg Leu Thr
    2825              2830          2835

Ala Thr Gln Ser Thr Ala Pro Lys Gly Ile Pro Val Lys Pro Thr
    2840              2845          2850

Ser Ala Glu Ser Gly Leu Leu Thr Pro Val Ser Ala Ser Ser Ser
    2855              2860          2865

Pro Ser Lys Ala Phe Ala Ser Leu Thr Thr Ala Pro Pro Ser Thr
    2870              2875          2880

Trp Gly Ile Pro Gln Ser Thr Leu Thr Phe Glu Phe Ser Glu Val
```

```
                2885                          2890                          2895


        Pro Ser  Leu Asp Thr Lys Ser  Ala Ser Leu Pro Thr  Pro Gly Gln
            2900                 2905             2910


        Ser Leu  Asn Thr Ile Pro Asp  Ser Asp Ala Ser Thr  Ala Ser Ser
            2915                 2920             2925


        Ser Leu  Ser Lys Ser Pro Glu  Lys Asn Pro Arg Ala  Arg Met Met
            2930                 2935             2940


        Thr Ser  Thr Lys Ala Ile Ser  Ala Ser Ser Phe Gln  Ser Thr Gly
            2945                 2950             2955


        Phe Thr  Glu Thr Pro Glu Gly  Ser Ala Ser Pro Ser  Met Ala Gly
            2960                 2965             2970


        His Glu  Pro Arg Val Pro Thr  Ser Gly Thr Gly Asp  Pro Arg Tyr
            2975                 2980             2985


        Ala Ser  Glu Ser Met Ser Tyr  Pro Asp Pro Ser Lys  Ala Ser Ser
            2990                 2995             3000


        Ala Met  Thr Ser Thr Ser Leu  Ala Ser Lys Leu Thr  Thr Leu Phe
            3005                 3010             3015


        Ser Thr  Gly Gln Ala Ala Arg  Ser Gly Ser Ser Ser  Ser Pro Ile
            3020                 3025             3030


        Ser Leu  Ser Thr Glu Lys Glu  Thr Ser Phe Leu Ser  Pro Thr Ala
            3035                 3040             3045


        Ser Thr  Ser Arg Lys Thr Ser  Leu Phe Leu Gly Pro  Ser Met Ala
            3050                 3055             3060


        Arg Gln  Pro Asn Ile Leu Val  His Leu Gln Thr Ser  Ala Leu Thr
            3065                 3070             3075


        Leu Ser  Pro Thr Ser Thr Leu  Asn Met Ser Gln Glu  Glu Pro Pro
            3080                 3085             3090


        Glu Leu  Thr Ser Ser Gln Thr  Ile Ala Glu Glu Glu  Gly Thr Thr
            3095                 3100             3105


        Ala Glu  Thr Gln Thr Leu Thr  Phe Thr Pro Ser Glu  Thr Pro Thr
            3110                 3115             3120
```

```
Ser Leu Leu Pro Val Ser Ser Pro Thr Glu Pro Thr Ala Arg Arg
    3125            3130            3135

Lys Ser Ser Pro Glu Thr Trp Ala Ser Ser Ile Ser Val Pro Ala
    3140            3145            3150

Lys Thr Ser Leu Val Glu Thr Thr Asp Gly Thr Leu Val Thr Thr
    3155            3160            3165

Ile Lys Met Ser Ser Gln Ala Ala Gln Gly Asn Ser Thr Trp Pro
    3170            3175            3180

Ala Pro Ala Glu Glu Thr Gly Thr Ser Pro Ala Gly Thr Ser Pro
    3185            3190            3195

Gly Ser Pro Glu Val Ser Thr Thr Leu Lys Ile Met Ser Ser Lys
    3200            3205            3210

Glu Pro Ser Ile Ser Pro Glu Ile Arg Ser Thr Val Arg Asn Ser
    3215            3220            3225

Pro Trp Lys Thr Pro Glu Thr Thr Val Pro Met Glu Thr Thr Val
    3230            3235            3240

Glu Pro Val Thr Leu Gln Ser Thr Ala Leu Gly Ser Gly Ser Thr
    3245            3250            3255

Ser Ile Ser His Leu Pro Thr Gly Thr Thr Ser Pro Thr Lys Ser
    3260            3265            3270

Pro Thr Glu Asn Met Leu Ala Thr Glu Arg Val Ser Leu Ser Pro
    3275            3280            3285

Ser Pro Pro Glu Ala Trp Thr Asn Leu Tyr Ser Gly Thr Pro Gly
    3290            3295            3300

Gly Thr Arg Gln Ser Leu Ala Thr Met Ser Ser Val Ser Leu Glu
    3305            3310            3315

Ser Pro Thr Ala Arg Ser Ile Thr Gly Thr Gly Gln Gln Ser Ser
    3320            3325            3330

Pro Glu Leu Val Ser Lys Thr Thr Gly Met Glu Phe Ser Met Trp
    3335            3340            3345

His Gly Ser Thr Gly Gly Thr Thr Gly Asp Thr His Val Ser Leu
    3350            3355            3360
```

301

Ser Thr Ser Ser Asn Ile Leu Glu Asp Pro Val Thr Ser Pro Asn
    3365                3370            3375

Ser Val Ser Ser Leu Thr Asp Lys Ser Lys His Lys Thr Glu Thr
    3380                3385            3390

Trp Val Ser Thr Thr Ala Ile Pro Ser Thr Val Leu Asn Asn Lys
    3395                3400            3405

Ile Met Ala Ala Glu Gln Gln Thr Ser Arg Ser Val Asp Glu Ala
    3410                3415            3420

Tyr Ser Ser Thr Ser Ser Trp Ser Asp Gln Thr Ser Gly Ser Asp
    3425                3430            3435

Ile Thr Leu Gly Ala Ser Pro Asp Val Thr Asn Thr Leu Tyr Ile
    3440                3445            3450

Thr Ser Thr Ala Gln Thr Thr Ser Leu Val Ser Leu Pro Ser Gly
    3455                3460            3465

Asp Gln Gly Ile Thr Ser Leu Thr Asn Pro Ser Gly Gly Lys Thr
    3470                3475            3480

Ser Ser Ala Ser Ser Val Thr Ser Pro Ser Ile Gly Leu Glu Thr
    3485                3490            3495

Leu Arg Ala Asn Val Ser Ala Val Lys Ser Asp Ile Ala Pro Thr
    3500                3505            3510

Ala Gly His Leu Ser Gln Thr Ser Ser Pro Ala Glu Val Ser Ile
    3515                3520            3525

Leu Asp Val Thr Thr Ala Pro Thr Pro Gly Ile Ser Thr Thr Ile
    3530                3535            3540

Thr Thr Met Gly Thr Asn Ser Ile Ser Thr Thr Thr Pro Asn Pro
    3545                3550            3555

Glu Val Gly Met Ser Thr Met Asp Ser Thr Pro Ala Thr Glu Arg
    3560                3565            3570

Arg Thr Thr Ser Thr Glu His Pro Ser Thr Trp Ser Ser Thr Ala
    3575                3580            3585

Ala Ser Asp Ser Trp Thr Val Thr Asp Met Thr Ser Asn Leu Lys
    3590                3595            3600

```
Val Ala  Arg Ser Pro Gly Thr  Ile Ser Thr Met His  Thr Thr Ser
    3605                 3610                 3615

Phe Leu  Ala Ser Ser Thr Glu  Leu Asp Ser Met Ser  Thr Pro His
    3620                 3625                 3630

Gly Arg  Ile Thr Val Ile Gly  Thr Ser Leu Val Thr  Pro Ser Ser
    3635                 3640                 3645

Asp Ala  Ser Ala Val Lys Thr  Glu Thr Ser Thr Ser  Glu Arg Thr
    3650                 3655                 3660

Leu Ser  Pro Ser Asp Thr Thr  Ala Ser Thr Pro Ile  Ser Thr Phe
    3665                 3670                 3675

Ser Arg  Val Gln Arg Met Ser  Ile Ser Val Pro Asp  Ile Leu Ser
    3680                 3685                 3690

Thr Ser  Trp Thr Pro Ser Ser  Thr Glu Ala Glu Asp  Val Pro Val
    3695                 3700                 3705

Ser Met  Val Ser Thr Asp His  Ala Ser Thr Lys Thr  Asp Pro Asn
    3710                 3715                 3720

Thr Pro  Leu Ser Thr Phe Leu  Phe Asp Ser Leu Ser  Thr Leu Asp
    3725                 3730                 3735

Trp Asp  Thr Gly Arg Ser Leu  Ser Ser Ala Thr Ala  Thr Thr Ser
    3740                 3745                 3750

Ala Pro  Gln Gly Ala Thr Thr  Pro Gln Glu Leu Thr  Leu Glu Thr
    3755                 3760                 3765

Met Ile  Ser Pro Ala Thr Ser  Gln Leu Pro Phe Ser  Ile Gly His
    3770                 3775                 3780

Ile Thr  Ser Ala Val Thr Pro  Ala Ala Met Ala Arg  Ser Ser Gly
    3785                 3790                 3795

Val Thr  Phe Ser Arg Pro Asp  Pro Thr Ser Lys Lys  Ala Glu Gln
    3800                 3805                 3810

Thr Ser  Thr Gln Leu Pro Thr  Thr Thr Ser Ala His  Pro Gly Gln
    3815                 3820                 3825

Val Pro  Arg Ser Ala Ala Thr  Thr Leu Asp Val Ile  Pro His Thr
```

303

```
            3830                    3835                        3840

Ala Lys  Thr Pro Asp Ala Thr  Phe Gln Arg Gln Gly  Gln Thr Ala
    3845                 3850                 3855

Leu Thr  Thr Glu Ala Arg Ala  Thr Ser Asp Ser Trp  Asn Glu Lys
    3860                 3865                 3870

Glu Lys  Ser Thr Pro Ser Ala  Pro Trp Ile Thr Glu  Met Met Asn
    3875                 3880                 3885

Ser Val  Ser Glu Asp Thr Ile  Lys Glu Val Thr Ser  Ser Ser Ser
    3890                 3895                 3900

Val Leu  Lys Asp Pro Glu Tyr  Ala Gly His Lys Leu  Gly Ile Trp
    3905                 3910                 3915

Asp Asp  Phe Ile Pro Lys Phe  Gly Lys Ala Ala His  Met Arg Glu
    3920                 3925                 3930

Leu Pro  Leu Leu Ser Pro Pro  Gln Asp Lys Glu Ala  Ile His Pro
    3935                 3940                 3945

Ser Thr  Asn Thr Val Glu Thr  Thr Gly Trp Val Thr  Ser Ser Glu
    3950                 3955                 3960

His Ala  Ser His Ser Thr Ile  Pro Ala His Ser Ala  Ser Ser Lys
    3965                 3970                 3975

Leu Thr  Ser Pro Val Val Thr  Thr Ser Thr Arg Glu  Gln Ala Ile
    3980                 3985                 3990

Val Ser  Met Ser Thr Thr Thr  Trp Pro Glu Ser Thr  Arg Ala Arg
    3995                 4000                 4005

Thr Glu  Pro Asn Ser Phe Leu  Thr Ile Glu Leu Arg  Asp Val Ser
    4010                 4015                 4020

Pro Tyr  Met Asp Thr Ser Ser  Thr Thr Gln Thr Ser  Ile Ile Ser
    4025                 4030                 4035

Ser Pro  Gly Ser Thr Ala Ile  Thr Lys Gly Pro Arg  Thr Glu Ile
    4040                 4045                 4050

Thr Ser  Ser Lys Arg Ile Ser  Ser Ser Phe Leu Ala  Gln Ser Met
    4055                 4060                 4065
```

```
Arg Ser  Ser Asp Ser Pro Ser  Glu Ala Ile Thr Arg  Leu Ser Asn
    4070                4075               4080

Phe Pro  Ala Met Thr Glu Ser  Gly Gly Met Ile Leu  Ala Met Gln
    4085                4090               4095

Thr Ser  Pro Pro Gly Ala Thr  Ser Leu Ser Ala Pro  Thr Leu Asp
    4100                4105               4110

Thr Ser  Ala Thr Ala Ser Trp  Thr Gly Thr Pro Leu  Ala Thr Thr
    4115                4120               4125

Gln Arg  Phe Thr Tyr Ser Glu  Lys Thr Thr Leu Phe  Ser Lys Gly
    4130                4135               4140

Pro Glu  Asp Thr Ser Gln Pro  Ser Pro Pro Ser Val  Glu Glu Thr
    4145                4150               4155

Ser Ser  Ser Ser Ser Leu Val  Pro Ile His Ala Thr  Thr Ser Pro
    4160                4165               4170

Ser Asn  Ile Leu Leu Thr Ser  Gln Gly His Ser Pro  Ser Ser Thr
    4175                4180               4185

Pro Pro  Val Thr Ser Val Phe  Leu Ser Glu Thr Ser  Gly Leu Gly
    4190                4195               4200

Lys Thr  Thr Asp Met Ser Arg  Ile Ser Leu Glu Pro  Gly Thr Ser
    4205                4210               4215

Leu Pro  Pro Asn Leu Ser Ser  Thr Ala Gly Glu Ala  Leu Ser Thr
    4220                4225               4230

Tyr Glu  Ala Ser Arg Asp Thr  Lys Ala Ile His His  Ser Ala Asp
    4235                4240               4245

Thr Ala  Val Thr Asn Met Glu  Ala Thr Ser Ser Glu  Tyr Ser Pro
    4250                4255               4260

Ile Pro  Gly His Thr Lys Pro  Ser Lys Ala Thr Ser  Pro Leu Val
    4265                4270               4275

Thr Ser  His Ile Met Gly Asp  Ile Thr Ser Ser Thr  Ser Val Phe
    4280                4285               4290

Gly Ser  Ser Glu Thr Thr Glu  Ile Glu Thr Val Ser  Ser Val Asn
    4295                4300               4305
```

```
Gln Gly  Leu Gln Glu Arg Ser  Thr Ser Gln Val Ala  Ser Ser Ala
    4310                 4315             4320

Thr Glu  Thr Ser Thr Val Ile  Thr His Val Ser Ser  Gly Asp Ala
    4325                 4330             4335

Thr Thr  His Val Thr Lys Thr  Gln Ala Thr Phe Ser  Ser Gly Thr
    4340                 4345             4350

Ser Ile  Ser Ser Pro His Gln  Phe Ile Thr Ser Thr  Asn Thr Phe
    4355                 4360             4365

Thr Asp  Val Ser Thr Asn Pro  Ser Thr Ser Leu Ile  Met Thr Glu
    4370                 4375             4380

Ser Ser  Gly Val Thr Ile Thr  Thr Gln Thr Gly Pro  Thr Gly Ala
    4385                 4390             4395

Ala Thr  Gln Gly Pro Tyr Leu  Leu Asp Thr Ser Thr  Met Pro Tyr
    4400                 4405             4410

Leu Thr  Glu Thr Pro Leu Ala  Val Thr Pro Asp Phe  Met Gln Ser
    4415                 4420             4425

Glu Lys  Thr Thr Leu Ile Ser  Lys Gly Pro Lys Asp  Val Thr Trp
    4430                 4435             4440

Thr Ser  Pro Pro Ser Val Ala  Glu Thr Ser Tyr Pro  Ser Ser Leu
    4445                 4450             4455

Thr Pro  Phe Leu Val Thr Thr  Ile Pro Pro Ala Thr  Ser Thr Leu
    4460                 4465             4470

Gln Gly  Gln His Thr Ser Ser  Pro Val Ser Ala Thr  Ser Val Leu
    4475                 4480             4485

Thr Ser  Gly Leu Val Lys Thr  Thr Asp Met Leu Asn  Thr Ser Met
    4490                 4495             4500

Glu Pro  Val Thr Asn Ser Pro  Gln Asn Leu Asn Asn  Pro Ser Asn
    4505                 4510             4515

Glu Ile  Leu Ala Thr Leu Ala  Ala Thr Thr Asp Ile  Glu Thr Ile
    4520                 4525             4530

His Pro  Ser Ile Asn Lys Ala  Val Thr Asn Met Gly  Thr Ala Ser
    4535                 4540             4545
```

```
Ser Ala  His Val Leu His Ser  Thr Leu Pro Val Ser  Ser Glu Pro
    4550             4555              4560

Ser Thr  Ala Thr Ser Pro Met  Val Pro Ala Ser Ser  Met Gly Asp
    4565             4570              4575

Ala Leu  Ala Ser Ile Ser Ile  Pro Gly Ser Glu Thr  Thr Asp Ile
    4580             4585              4590

Glu Gly  Glu Pro Thr Ser Ser  Leu Thr Ala Gly Arg  Lys Glu Asn
    4595             4600              4605

Ser Thr  Leu Gln Glu Met Asn  Ser Thr Thr Glu Ser  Asn Ile Ile
    4610             4615              4620

Leu Ser  Asn Val Ser Val Gly  Ala Ile Thr Glu Ala  Thr Lys Met
    4625             4630              4635

Glu Val  Pro Ser Phe Asp Ala  Thr Phe Ile Pro Thr  Pro Ala Gln
    4640             4645              4650

Ser Thr  Lys Phe Pro Asp Ile  Phe Ser Val Ala Ser  Ser Arg Leu
    4655             4660              4665

Ser Asn  Ser Pro Pro Met Thr  Ile Ser Thr His Met  Thr Thr Thr
    4670             4675              4680

Gln Thr  Gly Ser Ser Gly Ala  Thr Ser Lys Ile Pro  Leu Ala Leu
    4685             4690              4695

Asp Thr  Ser Thr Leu Glu Thr  Ser Ala Gly Thr Pro  Ser Val Val
    4700             4705              4710

Thr Glu  Gly Phe Ala His Ser  Lys Ile Thr Thr Ala  Met Asn Asn
    4715             4720              4725

Asp Val  Lys Asp Val Ser Gln  Thr Asn Pro Pro Phe  Gln Asp Glu
    4730             4735              4740

Ala Ser  Ser Pro Ser Ser Gln  Ala Pro Val Leu Val  Thr Thr Leu
    4745             4750              4755

Pro Ser  Ser Val Ala Phe Thr  Pro Gln Trp His Ser  Thr Ser Ser
    4760             4765              4770

Pro Val  Ser Met Ser Ser Val  Leu Thr Ser Ser Leu  Val Lys Thr
```

307

|      | 4775 |     |     |     |     | 4780 |     |     |     |     | 4785 |     |     |     |
|------|------|-----|-----|-----|-----|------|-----|-----|-----|-----|------|-----|-----|-----|
| Ala  | Gly  | Lys | Val | Asp | Thr | Ser  | Leu | Glu | Thr | Val | Thr  | Ser | Ser | Pro |
|      | 4790 |     |     |     |     | 4795 |     |     |     |     | 4800 |     |     |     |
| Gln  | Ser  | Met | Ser | Asn | Thr | Leu  | Asp | Asp | Ile | Ser | Val  | Thr | Ser | Ala |
|      | 4805 |     |     |     |     | 4810 |     |     |     |     | 4815 |     |     |     |
| Ala  | Thr  | Thr | Asp | Ile | Glu | Thr  | Thr | His | Pro | Ser | Ile  | Asn | Thr | Val |
|      | 4820 |     |     |     |     | 4825 |     |     |     |     | 4830 |     |     |     |
| Val  | Thr  | Asn | Val | Gly | Thr | Thr  | Gly | Ser | Ala | Phe | Glu  | Ser | His | Ser |
|      | 4835 |     |     |     |     | 4840 |     |     |     |     | 4845 |     |     |     |
| Thr  | Val  | Ser | Ala | Tyr | Pro | Glu  | Pro | Ser | Lys | Val | Thr  | Ser | Pro | Asn |
|      | 4850 |     |     |     |     | 4855 |     |     |     |     | 4860 |     |     |     |
| Val  | Thr  | Thr | Ser | Thr | Met | Glu  | Asp | Thr | Thr | Ile | Ser  | Arg | Ser | Ile |
|      | 4865 |     |     |     |     | 4870 |     |     |     |     | 4875 |     |     |     |
| Pro  | Lys  | Ser | Ser | Lys | Thr | Thr  | Arg | Thr | Glu | Thr | Glu  | Thr | Thr | Ser |
|      | 4880 |     |     |     |     | 4885 |     |     |     |     | 4890 |     |     |     |
| Ser  | Leu  | Thr | Pro | Lys | Leu | Arg  | Glu | Thr | Ser | Ile | Ser  | Gln | Glu | Ile |
|      | 4895 |     |     |     |     | 4900 |     |     |     |     | 4905 |     |     |     |
| Thr  | Ser  | Ser | Thr | Glu | Thr | Ser  | Thr | Val | Pro | Tyr | Lys  | Glu | Leu | Thr |
|      | 4910 |     |     |     |     | 4915 |     |     |     |     | 4920 |     |     |     |
| Gly  | Ala  | Thr | Thr | Glu | Val | Ser  | Arg | Thr | Asp | Val | Thr  | Ser | Ser | Ser |
|      | 4925 |     |     |     |     | 4930 |     |     |     |     | 4935 |     |     |     |
| Ser  | Thr  | Ser | Phe | Pro | Gly | Pro  | Asp | Gln | Ser | Thr | Val  | Ser | Leu | Asp |
|      | 4940 |     |     |     |     | 4945 |     |     |     |     | 4950 |     |     |     |
| Ile  | Ser  | Thr | Glu | Thr | Asn | Thr  | Arg | Leu | Ser | Thr | Ser  | Pro | Ile | Met |
|      | 4955 |     |     |     |     | 4960 |     |     |     |     | 4965 |     |     |     |
| Thr  | Glu  | Ser | Ala | Glu | Ile | Thr  | Ile | Thr | Thr | Gln | Thr  | Gly | Pro | His |
|      | 4970 |     |     |     |     | 4975 |     |     |     |     | 4980 |     |     |     |
| Gly  | Ala  | Thr | Ser | Gln | Asp | Thr  | Phe | Thr | Met | Asp | Pro  | Ser | Asn | Thr |
|      | 4985 |     |     |     |     | 4990 |     |     |     |     | 4995 |     |     |     |
| Thr  | Pro  | Gln | Ala | Gly | Ile | His  | Ser | Ala | Met | Thr | His  | Gly | Phe | Ser |
|      | 5000 |     |     |     |     | 5005 |     |     |     |     | 5010 |     |     |     |

```
Gln Leu  Asp Val Thr Thr Leu  Met Ser Arg Ile Pro  Gln Asp Val
    5015                 5020             5025

Ser Trp  Thr Ser Pro Pro Ser  Val Asp Lys Thr Ser  Ser Pro Ser
    5030                 5035             5040

Ser Phe  Leu Ser Ser Pro Ala  Met Thr Thr Pro Ser  Leu Ile Ser
    5045                 5050             5055

Ser Thr  Leu Pro Glu Asp Lys  Leu Ser Ser Pro Met  Thr Ser Leu
    5060                 5065             5070

Leu Thr  Ser Gly Leu Val Lys  Ile Thr Asp Ile Leu  Arg Thr Arg
    5075                 5080             5085

Leu Glu  Pro Val Thr Ser Ser  Leu Pro Asn Phe Ser  Ser Thr Ser
    5090                 5095             5100

Asp Lys  Ile Leu Ala Thr Ser  Lys Asp Ser Lys Asp  Thr Lys Glu
    5105                 5110             5115

Ile Phe  Pro Ser Ile Asn Thr  Glu Glu Thr Asn Val  Lys Ala Asn
    5120                 5125             5130

Asn Ser  Gly His Glu Ser His  Ser Pro Ala Leu Ala  Asp Ser Glu
    5135                 5140             5145

Thr Pro  Lys Ala Thr Thr Gln  Met Val Ile Thr Thr  Thr Val Gly
    5150                 5155             5160

Asp Pro  Ala Pro Ser Thr Ser  Met Pro Val His Gly  Ser Ser Glu
    5165                 5170             5175

Thr Thr  Asn Ile Lys Arg Glu  Pro Thr Tyr Phe Leu  Thr Pro Arg
    5180                 5185             5190

Leu Arg  Glu Thr Ser Thr Ser  Gln Glu Ser Ser Phe  Pro Thr Asp
    5195                 5200             5205

Thr Ser  Phe Leu Leu Ser Lys  Val Pro Thr Gly Thr  Ile Thr Glu
    5210                 5215             5220

Val Ser  Ser Thr Gly Val Asn  Ser Ser Ser Lys Ile  Ser Thr Pro
    5225                 5230             5235

Asp His  Asp Lys Ser Thr Val  Pro Pro Asp Thr Phe  Thr Gly Glu
    5240                 5245             5250
```

309

Ile Pro Arg Val Phe Thr Ser   Ser Ile Lys Thr Lys   Ser Ala Glu
    5255                5260                5265

Met Thr Ile Thr Thr Gln Ala   Ser Pro Pro Glu Ser   Ala Ser His
    5270                5275                5280

Ser Thr Leu Pro Leu Asp Thr   Ser Thr Thr Leu Ser   Gln Gly Gly
    5285                5290                5295

Thr His Ser Thr Val Thr Gln   Gly Phe Pro Tyr Ser   Glu Val Thr
    5300                5305                5310

Thr Leu Met Gly Met Gly Pro   Gly Asn Val Ser Trp   Met Thr Thr
    5315                5320                5325

Pro Pro Val Glu Glu Thr Ser   Ser Val Ser Ser Leu   Met Ser Ser
    5330                5335                5340

Pro Ala Met Thr Ser Pro Ser   Pro Val Ser Ser Thr   Ser Pro Gln
    5345                5350                5355

Ser Ile Pro Ser Ser Pro Leu   Pro Val Thr Ala Leu   Pro Thr Ser
    5360                5365                5370

Val Leu Val Thr Thr Thr Asp   Val Leu Gly Thr Thr   Ser Pro Glu
    5375                5380                5385

Ser Val Thr Ser Ser Pro Pro   Asn Leu Ser Ser Ile   Thr His Glu
    5390                5395                5400

Arg Pro Ala Thr Tyr Lys Asp   Thr Ala His Thr Glu   Ala Ala Met
    5405                5410                5415

His His Ser Thr Asn Thr Ala   Val Thr Asn Val Gly   Thr Ser Gly
    5420                5425                5430

Ser Gly His Lys Ser Gln Ser   Ser Val Leu Ala Asp   Ser Glu Thr
    5435                5440                5445

Ser Lys Ala Thr Pro Leu Met   Ser Thr Thr Ser Thr   Leu Gly Asp
    5450                5455                5460

Thr Ser Val Ser Thr Ser Thr   Pro Asn Ile Ser Gln   Thr Asn Gln
    5465                5470                5475

Ile Gln Thr Glu Pro Thr Ala   Ser Leu Ser Pro Arg   Leu Arg Glu
    5480                5485                5490

Ser Ser Thr Ser Glu Lys Thr   Ser Ser Thr Thr Glu   Thr Asn Thr
    5495                5500                5505

Ala Phe Ser Tyr Val Pro Thr   Gly Ala Ile Thr Gln   Ala Ser Arg
    5510                5515                5520

Thr Glu Ile Ser Ser Ser Arg   Thr Ser Ile Ser Asp   Leu Asp Arg
    5525                5530                5535

Pro Thr Ile Ala Pro Asp Ile   Ser Thr Gly Met Ile   Thr Arg Leu
    5540                5545                5550

Phe Thr Ser Pro Ile Met Thr   Lys Ser Ala Glu Met   Thr Val Thr
    5555                5560                5565

Thr Gln Thr Thr Thr Pro Gly   Ala Thr Ser Gln Gly   Ile Leu Pro
    5570                5575                5580

Trp Asp Thr Ser Thr Thr Leu   Phe Gln Gly Gly Thr   His Ser Thr
    5585                5590                5595

Val Ser Gln Gly Phe Pro His   Ser Glu Ile Thr Thr   Leu Arg Ser
    5600                5605                5610

Arg Thr Pro Gly Asp Val Ser   Trp Met Thr Thr Pro   Pro Val Glu
    5615                5620                5625

Glu Thr Ser Ser Gly Phe Ser   Leu Met Ser Pro Ser   Met Thr Ser
    5630                5635                5640

Pro Ser Pro Val Ser Ser Thr   Ser Pro Glu Ser Ile   Pro Ser Ser
    5645                5650                5655

Pro Leu Pro Val Thr Ala Leu   Leu Thr Ser Val Leu   Val Thr Thr
    5660                5665                5670

Thr Asn Val Leu Gly Thr Thr   Ser Pro Glu Thr Val   Thr Ser Ser
    5675                5680                5685

Pro Pro Asn Leu Ser Ser Pro   Thr Gln Glu Arg Leu   Thr Thr Tyr
    5690                5695                5700

Lys Asp Thr Ala His Thr Glu   Ala Met His Ala Ser   Met His Thr
    5705                5710                5715

Asn Thr Ala Val Ala Asn Val   Gly Thr Ser Ile Ser   Gly His Glu

```
                5720                        5725                        5730


           Ser Gln Ser Ser Val Pro Ala  Asp Ser His Thr Ser  Lys Ala Thr
               5735                    5740                    5745


           Ser Pro Met Gly Ile Thr Phe  Ala Met Gly Asp Thr  Ser Val Ser
               5750                    5755                    5760


           Thr Ser Thr Pro Ala Phe Phe  Glu Thr Arg Ile Gln  Thr Glu Ser
               5765                    5770                    5775


           Thr Ser Ser Leu Ile Pro Gly  Leu Arg Asp Thr Arg  Thr Ser Glu
               5780                    5785                    5790


           Glu Ile Asn Thr Val Thr Glu  Thr Ser Thr Val Leu  Ser Glu Val
               5795                    5800                    5805


           Pro Thr Thr Thr Thr Thr Glu  Val Ser Arg Thr Glu  Val Ile Thr
               5810                    5815                    5820


           Ser Ser Arg Thr Thr Ile Ser  Gly Pro Asp His Ser  Lys Met Ser
               5825                    5830                    5835


           Pro Tyr Ile Ser Thr Glu Thr  Ile Thr Arg Leu Ser  Thr Phe Pro
               5840                    5845                    5850


           Phe Val Thr Gly Ser Thr Glu  Met Ala Ile Thr Asn  Gln Thr Gly
               5855                    5860                    5865


           Pro Ile Gly Thr Ile Ser Gln  Ala Thr Leu Thr Leu  Asp Thr Ser
               5870                    5875                    5880


           Ser Thr Ala Ser Trp Glu Gly  Thr His Ser Pro Val  Thr Gln Arg
               5885                    5890                    5895


           Phe Pro His Ser Glu Glu Thr  Thr Thr Met Ser Arg  Ser Thr Lys
               5900                    5905                    5910


           Gly Val Ser Trp Gln Ser Pro  Pro Ser Val Glu Glu  Thr Ser Ser
               5915                    5920                    5925


           Pro Ser Ser Pro Val Pro Leu  Pro Ala Ile Thr Ser  His Ser Ser
               5930                    5935                    5940


           Leu Tyr Ser Ala Val Ser Gly  Ser Ser Pro Thr Ser  Ala Leu Pro
               5945                    5950                    5955
```

```
Val Thr  Ser Leu Leu Thr Ser  Gly Arg Arg Lys Thr  Ile Asp Met
    5960              5965             5970


Leu Asp  Thr His Ser Glu Leu  Val Thr Ser Ser Leu  Pro Ser Ala
    5975              5980             5985


Ser Ser  Phe Ser Gly Glu Ile  Leu Thr Ser Glu Ala  Ser Thr Asn
    5990              5995             6000


Thr Glu  Thr Ile His Phe Ser  Glu Asn Thr Ala Glu  Thr Asn Met
    6005              6010             6015


Gly Thr  Thr Asn Ser Met His  Lys Leu His Ser Ser  Val Ser Ile
    6020              6025             6030


His Ser  Gln Pro Ser Gly His  Thr Pro Pro Lys Val  Thr Gly Ser
    6035              6040             6045


Met Met  Glu Asp Ala Ile Val  Ser Thr Ser Thr Pro  Gly Ser Pro
    6050              6055             6060


Glu Thr  Lys Asn Val Asp Arg  Asp Ser Thr Ser Pro  Leu Thr Pro
    6065              6070             6075


Glu Leu  Lys Glu Asp Ser Thr  Ala Leu Val Met Asn  Ser Thr Thr
    6080              6085             6090


Glu Ser  Asn Thr Val Phe Ser  Ser Val Ser Leu Asp  Ala Ala Thr
    6095              6100             6105


Glu Val  Ser Arg Ala Glu Val  Thr Tyr Tyr Asp Pro  Thr Phe Met
    6110              6115             6120


Pro Ala  Ser Ala Gln Ser Thr  Lys Ser Pro Asp Ile  Ser Pro Glu
    6125              6130             6135


Ala Ser  Ser Ser His Ser Asn  Ser Pro Pro Leu Thr  Ile Ser Thr
    6140              6145             6150


His Lys  Thr Ile Ala Thr Gln  Thr Gly Pro Ser Gly  Val Thr Ser
    6155              6160             6165


Leu Gly  Gln Leu Thr Leu Asp  Thr Ser Thr Ile Ala  Thr Ser Ala
    6170              6175             6180


Gly Thr  Pro Ser Ala Arg Thr  Gln Asp Phe Val Asp  Ser Glu Thr
    6185              6190             6195
```

```
Thr Ser Val Met Asn Asn Asp  Leu Asn Asp Val Leu  Lys Thr Ser
    6200                6205                6210

Pro Phe Ser Ala Glu Glu Ala  Asn Ser Leu Ser Ser  Gln Ala Pro
    6215                6220                6225

Leu Leu Val Thr Thr Ser Pro  Ser Pro Val Thr Ser  Thr Leu Gln
    6230                6235                6240

Glu His Ser Thr Ser Ser Leu  Val Ser Val Thr Ser  Val Pro Thr
    6245                6250                6255

Pro Thr Leu Ala Lys Ile Thr  Asp Met Asp Thr Asn  Leu Glu Pro
    6260                6265                6270

Val Thr Arg Ser Pro Gln Asn  Leu Arg Asn Thr Leu  Ala Thr Ser
    6275                6280                6285

Glu Ala Thr Thr Asp Thr His  Thr Met His Pro Ser  Ile Asn Thr
    6290                6295                6300

Ala Met Ala Asn Val Gly Thr  Thr Ser Ser Pro Asn  Glu Phe Tyr
    6305                6310                6315

Phe Thr Val Ser Pro Asp Ser  Asp Pro Tyr Lys Ala  Thr Ser Ala
    6320                6325                6330

Val Val Ile Thr Ser Thr Ser  Gly Asp Ser Ile Val  Ser Thr Ser
    6335                6340                6345

Met Pro Arg Ser Ser Ala Met  Lys Lys Ile Glu Ser  Glu Thr Thr
    6350                6355                6360

Phe Ser Leu Ile Phe Arg Leu  Arg Glu Thr Ser Thr  Ser Gln Lys
    6365                6370                6375

Ile Gly Ser Ser Ser Asp Thr  Ser Thr Val Phe Asp  Lys Ala Phe
    6380                6385                6390

Thr Ala Ala Thr Thr Glu Val  Ser Arg Thr Glu Leu  Thr Ser Ser
    6395                6400                6405

Ser Arg Thr Ser Ile Gln Gly  Thr Glu Lys Pro Thr  Met Ser Pro
    6410                6415                6420

Asp Thr Ser Thr Arg Ser Val  Thr Met Leu Ser Thr  Phe Ala Gly
    6425                6430                6435
```

314

```
Leu Thr Lys Ser Glu Glu Arg  Thr Ile Ala Thr Gln  Thr Gly Pro
    6440              6445              6450

His Arg Ala Thr Ser Gln Gly  Thr Leu Thr Trp Asp  Thr Ser Ile
    6455              6460              6465

Thr Thr Ser Gln Ala Gly Thr  His Ser Ala Met Thr  His Gly Phe
    6470              6475              6480

Ser Gln Leu Asp Leu Ser Thr  Leu Thr Ser Arg Val  Pro Glu Tyr
    6485              6490              6495

Ile Ser Gly Thr Ser Pro Pro  Ser Val Glu Lys Thr  Ser Ser Ser
    6500              6505              6510

Ser Ser Leu Leu Ser Leu Pro  Ala Ile Thr Ser Pro  Ser Pro Val
    6515              6520              6525

Pro Thr Thr Leu Pro Glu Ser  Arg Pro Ser Ser Pro  Val His Leu
    6530              6535              6540

Thr Ser Leu Pro Thr Ser Gly  Leu Val Lys Thr Thr  Asp Met Leu
    6545              6550              6555

Ala Ser Val Ala Ser Leu Pro  Pro Asn Leu Gly Ser  Thr Ser His
    6560              6565              6570

Lys Ile Pro Thr Thr Ser Glu  Asp Ile Lys Asp Thr  Glu Lys Met
    6575              6580              6585

Tyr Pro Ser Thr Asn Ile Ala  Val Thr Asn Val Gly  Thr Thr Thr
    6590              6595              6600

Ser Glu Lys Glu Ser Tyr Ser  Ser Val Pro Ala Tyr  Ser Glu Pro
    6605              6610              6615

Pro Lys Val Thr Ser Pro Met  Val Thr Ser Phe Asn  Ile Arg Asp
    6620              6625              6630

Thr Ile Val Ser Thr Ser Met  Pro Gly Ser Ser Glu  Ile Thr Arg
    6635              6640              6645

Ile Glu Met Glu Ser Thr Phe  Ser Val Ala His Gly  Leu Lys Gly
    6650              6655              6660

Thr Ser  Thr Ser Gln Asp Pro  Ile Val Ser Thr Glu  Lys Ser Ala
```

6665            6670            6675

Val Leu His Lys Leu Thr Thr Gly Ala Thr Glu Thr Ser Arg Thr
    6680            6685            6690

Glu Val Ala Ser Ser Arg Arg Thr Ser Ile Pro Gly Pro Asp His
    6695            6700            6705

Ser Thr Glu Ser Pro Asp Ile Ser Thr Glu Val Ile Pro Ser Leu
    6710            6715            6720

Pro Ile Ser Leu Gly Ile Thr Glu Ser Ser Asn Met Thr Ile Ile
    6725            6730            6735

Thr Arg Thr Gly Pro Pro Leu Gly Ser Thr Ser Gln Gly Thr Phe
    6740            6745            6750

Thr Leu Asp Thr Pro Thr Thr Ser Ser Arg Ala Gly Thr His Ser
    6755            6760            6765

Met Ala Thr Gln Glu Phe Pro His Ser Glu Met Thr Thr Val Met
    6770            6775            6780

Asn Lys Asp Pro Glu Ile Leu Ser Trp Thr Ile Pro Pro Ser Ile
    6785            6790            6795

Glu Lys Thr Ser Phe Ser Ser Ser Leu Met Pro Ser Pro Ala Met
    6800            6805            6810

Thr Ser Pro Pro Val Ser Ser Thr Leu Pro Lys Thr Ile His Thr
    6815            6820            6825

Thr Pro Ser Pro Met Thr Ser Leu Leu Thr Pro Ser Leu Val Met
    6830            6835            6840

Thr Thr Asp Thr Leu Gly Thr Ser Pro Glu Pro Thr Thr Ser Ser
    6845            6850            6855

Pro Pro Asn Leu Ser Ser Thr Ser His Val Ile Leu Thr Thr Asp
    6860            6865            6870

Glu Asp Thr Thr Ala Ile Glu Ala Met His Pro Ser Thr Ser Thr
    6875            6880            6885

Ala Ala Thr Asn Val Glu Thr Thr Cys Ser Gly His Gly Ser Gln
    6890            6895            6900

```
Ser Ser  Val Leu Thr Asp Ser  Glu Lys Thr Lys Ala  Thr Ala Pro
    6905                 6910             6915

Met Asp  Thr Thr Ser Thr Met  Gly His Thr Thr Val  Ser Thr Ser
    6920                 6925             6930

Met Ser  Val Ser Ser Glu Thr  Thr Lys Ile Lys Arg  Glu Ser Thr
    6935                 6940             6945

Tyr Ser  Leu Thr Pro Gly Leu  Arg Glu Thr Ser Ile  Ser Gln Asn
    6950                 6955             6960

Ala Ser  Phe Ser Thr Asp Thr  Ser Ile Val Leu Ser  Glu Val Pro
    6965                 6970             6975

Thr Gly  Thr Thr Ala Glu Val  Ser Arg Thr Glu Val  Thr Ser Ser
    6980                 6985             6990

Gly Arg  Thr Ser Ile Pro Gly  Pro Ser Gln Ser Thr  Val Leu Pro
    6995                 7000             7005

Glu Ile  Ser Thr Arg Thr Met  Thr Arg Leu Phe Ala  Ser Pro Thr
    7010                 7015             7020

Met Thr  Glu Ser Ala Glu Met  Thr Ile Pro Thr Gln  Thr Gly Pro
    7025                 7030             7035

Ser Gly  Ser Thr Ser Gln Asp  Thr Leu Thr Leu Asp  Thr Ser Thr
    7040                 7045             7050

Thr Lys  Ser Gln Ala Lys Thr  His Ser Thr Leu Thr  Gln Arg Phe
    7055                 7060             7065

Pro His  Ser Glu Met Thr Thr  Leu Met Ser Arg Gly  Pro Gly Asp
    7070                 7075             7080

Met Ser  Trp Gln Ser Ser Pro  Ser Leu Glu Asn Pro  Ser Ser Leu
    7085                 7090             7095

Pro Ser  Leu Leu Ser Leu Pro  Ala Thr Thr Ser Pro  Pro Pro Ile
    7100                 7105             7110

Ser Ser  Thr Leu Pro Val Thr  Ile Ser Ser Ser Pro  Leu Pro Val
    7115                 7120             7125

Thr Ser  Leu Leu Thr Ser Ser  Pro Val Thr Thr Thr  Asp Met Leu
    7130                 7135             7140
```

317

```
His Thr Ser Pro Glu Leu Val  Thr Ser Ser Pro Pro  Lys Leu Ser
    7145             7150             7155

His Thr Ser Asp Glu Arg Leu  Thr Thr Gly Lys Asp  Thr Thr Asn
    7160             7165             7170

Thr Glu Ala Val His Pro Ser  Thr Asn Thr Ala Ala  Ser Asn Val
    7175             7180             7185

Glu Ile Pro Ser Phe Gly His  Glu Ser Pro Ser Ser  Ala Leu Ala
    7190             7195             7200

Asp Ser Glu Thr Ser Lys Ala  Thr Ser Pro Met Phe  Ile Thr Ser
    7205             7210             7215

Thr Gln Glu Asp Thr Thr Val  Ala Ile Ser Thr Pro  His Phe Leu
    7220             7225             7230

Glu Thr Ser Arg Ile Gln Lys  Glu Ser Ile Ser Ser  Leu Ser Pro
    7235             7240             7245

Lys Leu Arg Glu Thr Gly Ser  Ser Val Glu Thr Ser  Ser Ala Ile
    7250             7255             7260

Glu Thr Ser Ala Val Leu Ser  Glu Val Ser Ile Gly  Ala Thr Thr
    7265             7270             7275

Glu Ile Ser Arg Thr Glu Val  Thr Ser Ser Ser Arg  Thr Ser Ile
    7280             7285             7290

Ser Gly Ser Ala Glu Ser Thr  Met Leu Pro Glu Ile  Ser Thr Thr
    7295             7300             7305

Arg Lys Ile Ile Lys Phe Pro  Thr Ser Pro Ile Leu  Ala Glu Ser
    7310             7315             7320

Ser Glu Met Thr Ile Lys Thr  Gln Thr Ser Pro Pro  Gly Ser Thr
    7325             7330             7335

Ser Glu Ser Thr Phe Thr Leu  Asp Thr Ser Thr Thr  Pro Ser Leu
    7340             7345             7350

Val Ile Thr His Ser Thr Met  Thr Gln Arg Leu Pro  His Ser Glu
    7355             7360             7365

Ile Thr Thr Leu Val Ser Arg  Gly Ala Gly Asp Val  Pro Arg Pro
    7370             7375             7380
```

318

```
Ser Ser   Leu Pro Val Glu Glu   Thr Ser Pro Pro Ser   Ser Gln Leu
    7385                7390              7395

Ser Leu   Ser Ala Met Ile Ser   Pro Ser Pro Val Ser   Ser Thr Leu
    7400                7405              7410

Pro Ala   Ser Ser His Ser Ser   Ser Ala Ser Val Thr   Ser Pro Leu
    7415                7420              7425

Thr Pro   Gly Gln Val Lys Thr   Thr Glu Val Leu Asp   Ala Ser Ala
    7430                7435              7440

Glu Pro   Glu Thr Ser Ser Pro   Pro Ser Leu Ser Ser   Thr Ser Val
    7445                7450              7455

Glu Ile   Leu Ala Thr Ser Glu   Val Thr Thr Asp Thr   Glu Lys Ile
    7460                7465              7470

His Pro   Phe Pro Asn Thr Ala   Val Thr Lys Val Gly   Thr Ser Ser
    7475                7480              7485

Ser Gly   His Glu Ser Pro Ser   Ser Val Leu Pro Asp   Ser Glu Thr
    7490                7495              7500

Thr Lys   Ala Thr Ser Ala Met   Gly Thr Ile Ser Ile   Met Gly Asp
    7505                7510              7515

Thr Ser   Val Ser Thr Leu Thr   Pro Ala Leu Ser Asn   Thr Arg Lys
    7520                7525              7530

Ile Gln   Ser Glu Pro Ala Ser   Ser Leu Thr Thr Arg   Leu Arg Glu
    7535                7540              7545

Thr Ser   Thr Ser Glu Glu Thr   Ser Leu Ala Thr Glu   Ala Asn Thr
    7550                7555              7560

Val Leu   Ser Lys Val Ser Thr   Gly Ala Thr Thr Glu   Val Ser Arg
    7565                7570              7575

Thr Glu   Ala Ile Ser Phe Ser   Arg Thr Ser Met Ser   Gly Pro Glu
    7580                7585              7590

Gln Ser   Thr Met Ser Gln Asp   Ile Ser Ile Gly Thr   Ile Pro Arg
    7595                7600              7605

Ile Ser   Ala Ser Ser Val Leu   Thr Glu Ser Ala Lys   Met Thr Ile
```

```
          7610                    7615                    7620


       Thr Thr Gln Thr Gly Pro Ser Glu Ser Thr Leu Glu Ser Thr Leu
          7625                    7630                    7635


       Asn Leu Asn Thr Ala Thr Thr Pro Ser Trp Val Glu Thr His Ser
          7640                    7645                    7650


       Ile Val Ile Gln Gly Phe Pro His Pro Glu Met Thr Thr Ser Met
          7655                    7660                    7665


       Gly Arg Gly Pro Gly Gly Val Ser Trp Pro Ser Pro Pro Phe Val
          7670                    7675                    7680


       Lys Glu Thr Ser Pro Pro Ser Ser Pro Leu Ser Leu Pro Ala Val
          7685                    7690                    7695


       Thr Ser Pro His Pro Val Ser Thr Thr Phe Leu Ala His Ile Pro
          7700                    7705                    7710


       Pro Ser Pro Leu Pro Val Thr Ser Leu Leu Thr Ser Gly Pro Ala
          7715                    7720                    7725


       Thr Thr Thr Asp Ile Leu Gly Thr Ser Thr Glu Pro Gly Thr Ser
          7730                    7735                    7740


       Ser Ser Ser Ser Leu Ser Thr Thr Ser His Glu Arg Leu Thr Thr
          7745                    7750                    7755


       Tyr Lys Asp Thr Ala His Thr Glu Ala Val His Pro Ser Thr Asn
          7760                    7765                    7770


       Thr Gly Gly Thr Asn Val Ala Thr Thr Ser Ser Gly Tyr Lys Ser
          7775                    7780                    7785


       Gln Ser Ser Val Leu Ala Asp Ser Ser Pro Met Cys Thr Thr Ser
          7790                    7795                    7800


       Thr Met Gly Asp Thr Ser Val Leu Thr Ser Thr Pro Ala Phe Leu
          7805                    7810                    7815


       Glu Thr Arg Arg Ile Gln Thr Glu Leu Ala Ser Ser Leu Thr Pro
          7820                    7825                    7830


       Gly Leu Arg Glu Ser Ser Gly Ser Glu Gly Thr Ser Ser Gly Thr
          7835                    7840                    7845
```

```
Lys Met  Ser Thr Val Leu Ser  Lys Val Pro Thr Gly  Ala Thr Thr
    7850                 7855              7860

Glu Ile  Ser Lys Glu Asp Val  Thr Ser Ile Pro Gly  Pro Ala Gln
    7865                 7870              7875

Ser Thr  Ile Ser Pro Asp Ile  Ser Thr Arg Thr Val  Ser Trp Phe
    7880                 7885              7890

Ser Thr  Ser Pro Val Met Thr  Glu Ser Ala Glu Ile  Thr Met Asn
    7895                 7900              7905

Thr His  Thr Ser Pro Leu Gly  Ala Thr Thr Gln Gly  Thr Ser Thr
    7910                 7915              7920

Leu Ala  Thr Ser Ser Thr Thr  Ser Leu Thr Met Thr  His Ser Thr
    7925                 7930              7935

Ile Ser  Gln Gly Phe Ser His  Ser Gln Met Ser Thr  Leu Met Arg
    7940                 7945              7950

Arg Gly  Pro Glu Asp Val Ser  Trp Met Ser Pro Pro  Leu Leu Glu
    7955                 7960              7965

Lys Thr  Arg Pro Ser Phe Ser  Leu Met Ser Ser Pro  Ala Thr Thr
    7970                 7975              7980

Ser Pro  Ser Pro Val Ser Ser  Thr Leu Pro Glu Ser  Ile Ser Ser
    7985                 7990              7995

Ser Pro  Leu Pro Val Thr Ser  Leu Leu Thr Ser Gly  Leu Ala Lys
    8000                 8005              8010

Thr Thr  Asp Met Leu His Lys  Ser Ser Glu Pro Val  Thr Asn Ser
    8015                 8020              8025

Pro Ala  Asn Leu Ser Ser Thr  Ser Val Glu Ile Leu  Ala Thr Ser
    8030                 8035              8040

Glu Val  Thr Thr Asp Thr Glu  Lys Thr His Pro Ser  Ser Asn Arg
    8045                 8050              8055

Thr Val  Thr Asp Val Gly Thr  Ser Ser Ser Gly His  Glu Ser Thr
    8060                 8065              8070

Ser Phe  Val Leu Ala Asp Ser  Gln Thr Ser Lys Val  Thr Ser Pro
    8075                 8080              8085
```

321

Met Val Ile Thr Ser Thr Met Glu Asp Thr Ser Val Ser Thr Ser
    8090                8095            8100

Thr Pro Gly Phe Phe Glu Thr Ser Arg Ile Gln Thr Glu Pro Thr
    8105                8110            8115

Ser Ser Leu Thr Leu Gly Leu Arg Lys Thr Ser Ser Ser Glu Gly
    8120                8125            8130

Thr Ser Leu Ala Thr Glu Met Ser Thr Val Leu Ser Gly Val Pro
    8135                8140            8145

Thr Gly Ala Thr Ala Glu Val Ser Arg Thr Glu Val Thr Ser Ser
    8150                8155            8160

Ser Arg Thr Ser Ile Ser Gly Phe Ala Gln Leu Thr Val Ser Pro
    8165                8170            8175

Glu Thr Ser Thr Glu Thr Ile Thr Arg Leu Pro Thr Ser Ser Ile
    8180                8185            8190

Met Thr Glu Ser Ala Glu Met Met Ile Lys Thr Gln Thr Asp Pro
    8195                8200            8205

Pro Gly Ser Thr Pro Glu Ser Thr His Thr Val Asp Ile Ser Thr
    8210                8215            8220

Thr Pro Asn Trp Val Glu Thr His Ser Thr Val Thr Gln Arg Phe
    8225                8230            8235

Ser His Ser Glu Met Thr Thr Leu Val Ser Arg Ser Pro Gly Asp
    8240                8245            8250

Met Leu Trp Pro Ser Gln Ser Ser Val Glu Glu Thr Ser Ser Ala
    8255                8260            8265

Ser Ser Leu Leu Ser Leu Pro Ala Thr Thr Ser Pro Ser Pro Val
    8270                8275            8280

Ser Ser Thr Leu Val Glu Asp Phe Pro Ser Ala Ser Leu Pro Val
    8285                8290            8295

Thr Ser Leu Leu Thr Pro Gly Leu Val Ile Thr Thr Asp Arg Met
    8300                8305            8310

Gly Ile Ser Arg Glu Pro Gly Thr Ser Ser Thr Ser Asn Leu Ser
    8315                8320            8325

```
Ser Thr  Ser His Glu Arg Leu  Thr Thr Leu Glu Asp  Thr Val Asp
    8330             8335             8340

Thr Glu  Asp Met Gln Pro Ser  Thr His Thr Ala Val  Thr Asn Val
    8345             8350             8355

Arg Thr  Ser Ile Ser Gly His  Glu Ser Gln Ser Ser  Val Leu Ser
    8360             8365             8370

Asp Ser  Glu Thr Pro Lys Ala  Thr Ser Pro Met Gly  Thr Thr Tyr
    8375             8380             8385

Thr Met  Gly Glu Thr Ser Val  Ser Ile Ser Thr Ser  Asp Phe Phe
    8390             8395             8400

Glu Thr  Ser Arg Ile Gln Ile  Glu Pro Thr Ser Ser  Leu Thr Ser
    8405             8410             8415

Gly Leu  Arg Glu Thr Ser Ser  Ser Glu Arg Ile Ser  Ser Ala Thr
    8420             8425             8430

Glu Gly  Ser Thr Val Leu Ser  Glu Val Pro Ser Gly  Ala Thr Thr
    8435             8440             8445

Glu Val  Ser Arg Thr Glu Val  Ile Ser Ser Arg Gly  Thr Ser Met
    8450             8455             8460

Ser Gly  Pro Asp Gln Phe Thr  Ile Ser Pro Asp Ile  Ser Thr Glu
    8465             8470             8475

Ala Ile  Thr Arg Leu Ser Thr  Ser Pro Ile Met Thr  Glu Ser Ala
    8480             8485             8490

Glu Ser  Ala Ile Thr Ile Glu  Thr Gly Ser Pro Gly  Ala Thr Ser
    8495             8500             8505

Glu Gly  Thr Leu Thr Leu Asp  Thr Ser Thr Thr Thr  Phe Trp Ser
    8510             8515             8520

Gly Thr  His Ser Thr Ala Ser  Pro Gly Phe Ser His  Ser Glu Met
    8525             8530             8535

Thr Thr  Leu Met Ser Arg Thr  Pro Gly Asp Val Pro  Trp Pro Ser
    8540             8545             8550

Leu Pro  Ser Val Glu Glu Ala  Ser Ser Val Ser Ser  Ser Leu Ser
```

```
         8555                    8560                    8565

Ser Pro Ala Met Thr Ser Thr  Ser Phe Phe Ser Ala  Leu Pro Glu
    8570                8575                8580

Ser Ile Ser Ser Ser Pro His  Pro Val Thr Ala Leu  Leu Thr Leu
    8585                8590                8595

Gly Pro Val Lys Thr Thr Asp  Met Leu Arg Thr Ser  Ser Glu Pro
    8600                8605                8610

Glu Thr Ser Ser Pro Pro Asn  Leu Ser Ser Thr Ser  Ala Glu Ile
    8615                8620                8625

Leu Ala Thr Ser Glu Val Thr  Lys Asp Arg Glu Lys  Ile His Pro
    8630                8635                8640

Ser Ser Asn Thr Pro Val Val  Asn Val Gly Thr Val  Ile Tyr Lys
    8645                8650                8655

His Leu Ser Pro Ser Ser Val  Leu Ala Asp Leu Val  Thr Thr Lys
    8660                8665                8670

Pro Thr Ser Pro Met Ala Thr  Thr Ser Thr Leu Gly  Asn Thr Ser
    8675                8680                8685

Val Ser Thr Ser Thr Pro Ala  Phe Pro Glu Thr Met  Met Thr Gln
    8690                8695                8700

Pro Thr Ser Ser Leu Thr Ser  Gly Leu Arg Glu Ile  Ser Thr Ser
    8705                8710                8715

Gln Glu Thr Ser Ser Ala Thr  Glu Arg Ser Ala Ser  Leu Ser Gly
    8720                8725                8730

Met Pro Thr Gly Ala Thr Thr  Lys Val Ser Arg Thr  Glu Ala Leu
    8735                8740                8745

Ser Leu Gly Arg Thr Ser Thr  Pro Gly Pro Ala Gln  Ser Thr Ile
    8750                8755                8760

Ser Pro Glu Ile Ser Thr Glu  Thr Ile Thr Arg Ile  Ser Thr Pro
    8765                8770                8775

Leu Thr Thr Thr Gly Ser Ala  Glu Met Thr Ile Thr  Pro Lys Thr
    8780                8785                8790
```

324

```
Gly His  Ser Gly Ala Ser Ser  Gln Gly Thr Phe Thr  Leu Asp Thr
    8795            8800            8805

Ser Ser  Arg Ala Ser Trp Pro  Gly Thr His Ser Ala  Ala Thr His
    8810            8815            8820

Arg Ser  Pro His Ser Gly Met  Thr Thr Pro Met Ser  Arg Gly Pro
    8825            8830            8835

Glu Asp  Val Ser Trp Pro Ser  Arg Pro Ser Val Glu  Lys Thr Ser
    8840            8845            8850

Pro Pro  Ser Ser Leu Val Ser  Leu Ser Ala Val Thr  Ser Pro Ser
    8855            8860            8865

Pro Leu  Tyr Ser Thr Pro Ser  Glu Ser Ser His Ser  Ser Pro Leu
    8870            8875            8880

Arg Val  Thr Ser Leu Phe Thr  Pro Val Met Met Lys  Thr Thr Asp
    8885            8890            8895

Met Leu  Asp Thr Ser Leu Glu  Pro Val Thr Thr Ser  Pro Pro Ser
    8900            8905            8910

Met Asn  Ile Thr Ser Asp Glu  Ser Leu Ala Thr Ser  Lys Ala Thr
    8915            8920            8925

Met Glu  Thr Glu Ala Ile Gln  Leu Ser Glu Asn Thr  Ala Val Thr
    8930            8935            8940

Gln Met  Gly Thr Ile Ser Ala  Arg Gln Glu Phe Tyr  Ser Ser Tyr
    8945            8950            8955

Pro Gly  Leu Pro Glu Pro Ser  Lys Val Thr Ser Pro  Val Val Thr
    8960            8965            8970

Ser Ser  Thr Ile Lys Asp Ile  Val Ser Thr Thr Ile  Pro Ala Ser
    8975            8980            8985

Ser Glu  Ile Thr Arg Ile Glu  Met Glu Ser Thr Ser  Thr Leu Thr
    8990            8995            9000

Pro Thr  Pro Arg Glu Thr Ser  Thr Ser Gln Glu Ile  His Ser Ala
    9005            9010            9015

Thr Lys  Pro Ser Thr Val Pro  Tyr Lys Ala Leu Thr  Ser Ala Thr
    9020            9025            9030
```

325

Ile Glu Asp Ser Met Thr Gln  Val Met Ser Ser Ser  Arg Gly Pro
    9035              9040              9045

Ser Pro Asp Gln Ser Thr Met  Ser Gln Asp Ile Ser  Ser Glu Val
    9050              9055              9060

Ile Thr Arg Leu Ser Thr Ser  Pro Ile Lys Ala Glu  Ser Thr Glu
    9065              9070              9075

Met Thr Ile Thr Thr Gln Thr  Gly Ser Pro Gly Ala  Thr Ser Arg
    9080              9085              9090

Gly Thr Leu Thr Leu Asp Thr  Ser Thr Thr Phe Met  Ser Gly Thr
    9095              9100              9105

His Ser Thr Ala Ser Gln Gly  Phe Ser His Ser Gln  Met Thr Ala
    9110              9115              9120

Leu Met Ser Arg Thr Pro Gly  Asp Val Pro Trp Leu  Ser His Pro
    9125              9130              9135

Ser Val Glu Glu Ala Ser Ser  Ala Ser Phe Ser Leu  Ser Ser Pro
    9140              9145              9150

Val Met Thr Ser Ser Ser Pro  Val Ser Ser Thr Leu  Pro Asp Ser
    9155              9160              9165

Ile His Ser Ser Ser Leu Pro  Val Thr Ser Leu Leu  Thr Ser Gly
    9170              9175              9180

Leu Val Lys Thr Thr Glu Leu  Leu Gly Thr Ser Ser  Glu Pro Glu
    9185              9190              9195

Thr Ser Ser Pro Pro Asn Leu  Ser Ser Thr Ser Ala  Glu Ile Leu
    9200              9205              9210

Ala Thr Thr Glu Val Thr Thr  Asp Thr Glu Lys Leu  Glu Met Thr
    9215              9220              9225

Asn Val Val Thr Ser Gly Tyr  Thr His Glu Ser Pro  Ser Ser Val
    9230              9235              9240

Leu Ala Asp Ser Val Thr Thr  Lys Ala Thr Ser Ser  Met Gly Ile
    9245              9250              9255

Thr Tyr Pro Thr Gly Asp Thr  Asn Val Leu Thr Ser  Thr Pro Ala
    9260              9265              9270

326

```
Phe Ser  Asp Thr Ser Arg Ile  Gln Thr Lys Ser Lys  Leu Ser Leu
    9275             9280          9285

Thr Pro  Gly Leu Met Glu Thr  Ser Ile Ser Glu Glu  Thr Ser Ser
    9290             9295          9300

Ala Thr  Glu Lys Ser Thr Val  Leu Ser Ser Val Pro  Thr Gly Ala
    9305             9310          9315

Thr Thr  Glu Val Ser Arg Thr  Glu Ala Ile Ser Ser  Ser Arg Thr
    9320             9325          9330

Ser Ile  Pro Gly Pro Ala Gln  Ser Thr Met Ser Ser  Asp Thr Ser
    9335             9340          9345

Met Glu  Thr Ile Thr Arg Ile  Ser Thr Pro Leu Thr  Arg Lys Glu
    9350             9355          9360

Ser Thr  Asp Met Ala Ile Thr  Pro Lys Thr Gly Pro  Ser Gly Ala
    9365             9370          9375

Thr Ser  Gln Gly Thr Phe Thr  Leu Asp Ser Ser Ser  Thr Ala Ser
    9380             9385          9390

Trp Pro  Gly Thr His Ser Ala  Thr Thr Gln Arg Phe  Pro Gln Ser
    9395             9400          9405

Val Val  Thr Thr Pro Met Ser  Arg Gly Pro Glu Asp  Val Ser Trp
    9410             9415          9420

Pro Ser  Pro Leu Ser Val Glu  Lys Asn Ser Pro Pro  Ser Ser Leu
    9425             9430          9435

Val Ser  Ser Ser Ser Val Thr  Ser Pro Ser Pro Leu  Tyr Ser Thr
    9440             9445          9450

Pro Ser  Gly Ser Ser His Ser  Ser Pro Val Pro Val  Thr Ser Leu
    9455             9460          9465

Phe Thr  Ser Ile Met Met Lys  Ala Thr Asp Met Leu  Asp Ala Ser
    9470             9475          9480

Leu Glu  Pro Glu Thr Thr Ser  Ala Pro Asn Met Asn  Ile Thr Ser
    9485             9490          9495

Asp Glu  Ser Leu Ala Thr Ser  Lys Ala Thr Thr Glu  Thr Glu Ala
```

```
            9500                    9505                    9510


    Ile His  Val Phe Glu Asn Thr  Ala Ala Ser His Val  Glu Thr Thr
        9515                    9520                    9525


    Ser Ala  Thr Glu Glu Leu Tyr  Ser Ser Ser Pro Gly  Phe Ser Glu
        9530                    9535                    9540


    Pro Thr  Lys Val Ile Ser Pro  Val Val Thr Ser Ser  Ser Ile Arg
        9545                    9550                    9555


    Asp Asn  Met Val Ser Thr Thr  Met Pro Gly Ser Ser  Gly Ile Thr
        9560                    9565                    9570


    Arg Ile  Glu Ile Glu Ser Met  Ser Ser Leu Thr Pro  Gly Leu Arg
        9575                    9580                    9585


    Glu Thr  Arg Thr Ser Gln Asp  Ile Thr Ser Ser Thr  Glu Thr Ser
        9590                    9595                    9600


    Thr Val  Leu Tyr Lys Met Ser  Ser Gly Ala Thr Pro  Glu Val Ser
        9605                    9610                    9615


    Arg Thr  Glu Val Met Pro Ser  Ser Arg Thr Ser Ile  Pro Gly Pro
        9620                    9625                    9630


    Ala Gln  Ser Thr Met Ser Leu  Asp Ile Ser Asp Glu  Val Val Thr
        9635                    9640                    9645


    Arg Leu  Ser Thr Ser Pro Ile  Met Thr Glu Ser Ala  Glu Ile Thr
        9650                    9655                    9660


    Ile Thr  Thr Gln Thr Gly Tyr  Ser Leu Ala Thr Ser  Gln Val Thr
        9665                    9670                    9675


    Leu Pro  Leu Gly Thr Ser Met  Thr Phe Leu Ser Gly  Thr His Ser
        9680                    9685                    9690


    Thr Met  Ser Gln Gly Leu Ser  His Ser Glu Met Thr  Asn Leu Met
        9695                    9700                    9705


    Ser Arg  Gly Pro Glu Ser Leu  Ser Trp Thr Ser Pro  Arg Phe Val
        9710                    9715                    9720


    Glu Thr  Thr Arg Ser Ser Ser  Ser Leu Thr Ser Leu  Pro Leu Thr
        9725                    9730                    9735
```

```
Thr Ser  Leu Ser Pro Val Ser  Ser Thr Leu Leu Asp  Ser Ser Pro
    9740             9745             9750

Ser Ser  Pro Leu Pro Val Thr  Ser Leu Ile Leu Pro  Gly Leu Val
    9755             9760             9765

Lys Thr  Thr Glu Val Leu Asp  Thr Ser Ser Glu Pro  Lys Thr Ser
    9770             9775             9780

Ser Ser  Pro Asn Leu Ser Ser  Thr Ser Val Glu Ile  Pro Ala Thr
    9785             9790             9795

Ser Glu  Ile Met Thr Asp Thr  Glu Lys Ile His Pro  Ser Ser Asn
    9800             9805             9810

Thr Ala  Val Ala Lys Val Arg  Thr Ser Ser Ser Val  His Glu Ser
    9815             9820             9825

His Ser  Ser Val Leu Ala Asp  Ser Glu Thr Thr Ile  Thr Ile Pro
    9830             9835             9840

Ser Met  Gly Ile Thr Ser Ala  Val Asp Asp Thr Thr  Val Phe Thr
    9845             9850             9855

Ser Asn  Pro Ala Phe Ser Glu  Thr Arg Arg Ile Pro  Thr Glu Pro
    9860             9865             9870

Thr Phe  Ser Leu Thr Pro Gly  Phe Arg Glu Thr Ser  Thr Ser Glu
    9875             9880             9885

Glu Thr  Thr Ser Ile Thr Glu  Thr Ser Ala Val Leu  Tyr Gly Val
    9890             9895             9900

Pro Thr  Ser Ala Thr Thr Glu  Val Ser Met Thr Glu  Ile Met Ser
    9905             9910             9915

Ser Asn  Arg Thr His Ile Pro  Asp Ser Asp Gln Ser  Thr Met Ser
    9920             9925             9930

Pro Asp  Ile Ile Thr Glu Val  Ile Thr Arg Leu Ser  Ser Ser Ser
    9935             9940             9945

Met Met  Ser Glu Ser Thr Gln  Met Thr Ile Thr Thr  Gln Lys Ser
    9950             9955             9960

Ser Pro  Gly Ala Thr Ala Gln  Ser Thr Leu Thr Leu  Ala Thr Thr
    9965             9970             9975
```

329

```
Thr Ala  Pro Leu Ala Arg Thr  His Ser Thr Val Pro  Pro Arg Phe
    9980                9985                9990

Leu His  Ser Glu Met Thr Thr  Leu Met Ser Arg Ser  Pro Glu Asn
    9995                10000               10005

Pro Ser  Trp Lys Ser Ser Pro  Phe Val Glu Lys Thr  Ser Ser Ser
    10010               10015               10020

Ser Ser  Leu Leu Ser Leu Pro  Val Thr Thr Ser Pro  Ser Val Ser
    10025               10030               10035

Ser Thr  Leu Pro Gln Ser Ile  Pro Ser Ser Ser Phe  Ser Val Thr
    10040               10045               10050

Ser Leu  Leu Thr Pro Gly Met  Val Lys Thr Thr Asp  Thr Ser Thr
    10055               10060               10065

Glu Pro  Gly Thr Ser Leu Ser  Pro Asn Leu Ser Gly  Thr Ser Val
    10070               10075               10080

Glu Ile  Leu Ala Ala Ser Glu  Val Thr Thr Asp Thr  Glu Lys Ile
    10085               10090               10095

His Pro  Ser Ser Ser Met Ala  Val Thr Asn Val Gly  Thr Thr Ser
    10100               10105               10110

Ser Gly  His Glu Leu Tyr Ser  Ser Val Ser Ile His  Ser Glu Pro
    10115               10120               10125

Ser Lys  Ala Thr Tyr Pro Val  Gly Thr Pro Ser Ser  Met Ala Glu
    10130               10135               10140

Thr Ser  Ile Ser Thr Ser Met  Pro Ala Asn Phe Glu  Thr Thr Gly
    10145               10150               10155

Phe Glu  Ala Glu Pro Phe Ser  His Leu Thr Ser Gly  Phe Arg Lys
    10160               10165               10170

Thr Asn  Met Ser Leu Asp Thr  Ser Ser Val Thr Pro  Thr Asn Thr
    10175               10180               10185

Pro Ser  Ser Pro Gly Ser Thr  His Leu Leu Gln Ser  Ser Lys Thr
    10190               10195               10200

Asp Phe  Thr Ser Ser Ala Lys  Thr Ser Ser Pro Asp  Trp Pro Pro
    10205               10210               10215
```

```
Ala Ser   Gln Tyr Thr Glu Ile   Pro Val Asp Ile Ile   Thr Pro Phe
    10220                 10225                 10230

Asn Ala   Ser Pro Ser Ile Thr   Glu Ser Thr Gly Ile   Thr Ser Phe
    10235                 10240                 10245

Pro Glu   Ser Arg Phe Thr Met   Ser Val Thr Glu Ser   Thr His His
    10250                 10255                 10260

Leu Ser   Thr Asp Leu Leu Pro   Ser Ala Glu Thr Ile   Ser Thr Gly
    10265                 10270                 10275

Thr Val   Met Pro Ser Leu Ser   Glu Ala Met Thr Ser   Phe Ala Thr
    10280                 10285                 10290

Thr Gly   Val Pro Arg Ala Ile   Ser Gly Ser Gly Ser   Pro Phe Ser
    10295                 10300                 10305

Arg Thr   Glu Ser Gly Pro Gly   Asp Ala Thr Leu Ser   Thr Ile Ala
    10310                 10315                 10320

Glu Ser   Leu Pro Ser Ser Thr   Pro Val Pro Phe Ser   Ser Ser Thr
    10325                 10330                 10335

Phe Thr   Thr Thr Asp Ser Ser   Thr Ile Pro Ala Leu   His Glu Ile
    10340                 10345                 10350

Thr Ser   Ser Ser Ala Thr Pro   Tyr Arg Val Asp Thr   Ser Leu Gly
    10355                 10360                 10365

Thr Glu   Ser Ser Thr Thr Glu   Gly Arg Leu Val Met   Val Ser Thr
    10370                 10375                 10380

Leu Asp   Thr Ser Ser Gln Pro   Gly Arg Thr Ser Ser   Thr Pro Ile
    10385                 10390                 10395

Leu Asp   Thr Arg Met Thr Glu   Ser Val Glu Leu Gly   Thr Val Thr
    10400                 10405                 10410

Ser Ala   Tyr Gln Val Pro Ser   Leu Ser Thr Arg Leu   Thr Arg Thr
    10415                 10420                 10425

Asp Gly   Ile Met Glu His Ile   Thr Lys Ile Pro Asn   Glu Ala Ala
    10430                 10435                 10440

His Arg   Gly Thr Ile Arg Pro   Val Lys Gly Pro Gln   Thr Ser Thr
```

331

```
              10445                    10450                      10455


          Ser Pro  Ala Ser Pro Lys Gly  Leu His Thr Gly Gly  Thr Lys Arg
              10460                10465                10470


          Met Glu  Thr Thr Thr Thr Ala  Leu Lys Thr Thr Thr  Thr Ala Leu
              10475                10480                10485


          Lys Thr  Thr Ser Arg Ala Thr  Leu Thr Thr Ser Val  Tyr Thr Pro
              10490                10495                10500


          Thr Leu  Gly Thr Leu Thr Pro  Leu Asn Ala Ser Arg  Gln Met Ala
              10505                10510                10515


          Ser Thr  Ile Leu Thr Glu Met  Met Ile Thr Thr Pro  Tyr Val Phe
              10520                10525                10530


          Pro Asp  Val Pro Glu Thr Thr  Ser Ser Leu Ala Thr  Ser Leu Gly
              10535                10540                10545


          Ala Glu  Thr Ser Thr Ala Leu  Pro Arg Thr Thr Pro  Ser Val Leu
              10550                10555                10560


          Asn Arg  Glu Ser Glu Thr Thr  Ala Ser Leu Val Ser  Arg Ser Gly
              10565                10570                10575


          Ala Glu  Arg Ser Pro Val Ile  Gln Thr Leu Asp Val  Ser Ser Ser
              10580                10585                10590


          Glu Pro  Asp Thr Thr Ala Ser  Trp Val Ile His Pro  Ala Glu Thr
              10595                10600                10605


          Ile Pro  Thr Val Ser Lys Thr  Thr Pro Asn Phe Phe  His Ser Glu
              10610                10615                10620


          Leu Asp  Thr Val Ser Ser Thr  Ala Thr Ser His Gly  Ala Asp Val
              10625                10630                10635


          Ser Ser  Ala Ile Pro Thr Asn  Ile Ser Pro Ser Glu  Leu Asp Ala
              10640                10645                10650


          Leu Thr  Pro Leu Val Thr Ile  Ser Gly Thr Asp Thr  Ser Thr Thr
              10655                10660                10665


          Phe Pro  Thr Leu Thr Lys Ser  Pro His Glu Thr Glu  Thr Arg Thr
              10670                10675                10680
```

332

```
Thr Trp  Leu Thr His Pro Ala   Glu Thr Ser Ser Thr   Ile Pro Arg
    10685                10690                10695

Thr Ile  Pro Asn Phe Ser His   His Glu Ser Asp Ala   Thr Pro Ser
    10700                10705                10710

Ile Ala  Thr Ser Pro Gly Ala   Glu Thr Ser Ser Ala   Ile Pro Ile
    10715                10720                10725

Met Thr  Val Ser Pro Gly Ala   Glu Asp Leu Val Thr   Ser Gln Val
    10730                10735                10740

Thr Ser  Ser Gly Thr Asp Arg   Asn Met Thr Ile Pro   Thr Leu Thr
    10745                10750                10755

Leu Ser  Pro Gly Glu Pro Lys   Thr Ile Ala Ser Leu   Val Thr His
    10760                10765                10770

Pro Glu  Ala Gln Thr Ser Ser   Ala Ile Pro Thr Ser   Thr Ile Ser
    10775                10780                10785

Pro Ala  Val Ser Arg Leu Val   Thr Ser Met Val Thr   Ser Leu Ala
    10790                10795                10800

Ala Lys  Thr Ser Thr Thr Asn   Arg Ala Leu Thr Asn   Ser Pro Gly
    10805                10810                10815

Glu Pro  Ala Thr Thr Val Ser   Leu Val Thr His Pro   Ala Gln Thr
    10820                10825                10830

Ser Pro  Thr Val Pro Trp Thr   Thr Ser Ile Phe Phe   His Ser Lys
    10835                10840                10845

Ser Asp  Thr Thr Pro Ser Met   Thr Thr Ser His Gly   Ala Glu Ser
    10850                10855                10860

Ser Ser  Ala Val Pro Thr Pro   Thr Val Ser Thr Glu   Val Pro Gly
    10865                10870                10875

Val Val  Thr Pro Leu Val Thr   Ser Ser Arg Ala Val   Ile Ser Thr
    10880                10885                10890

Thr Ile  Pro Ile Leu Thr Leu   Ser Pro Gly Glu Pro   Glu Thr Thr
    10895                10900                10905

Pro Ser  Met Ala Thr Ser His   Gly Glu Glu Ala Ser   Ser Ala Ile
    10910                10915                10920
```

333

```
Pro Thr   Pro Thr Val Ser Pro   Gly Val Pro Gly Val   Val Thr Ser
    10925                 10930                 10935

Leu Val   Thr Ser Ser Arg Ala   Val Thr Ser Thr Thr   Ile Pro Ile
    10940                 10945                 10950

Leu Thr   Phe Ser Leu Gly Glu   Pro Glu Thr Thr Pro   Ser Met Ala
    10955                 10960                 10965

Thr Ser   His Gly Thr Glu Ala   Gly Ser Ala Val Pro   Thr Val Leu
    10970                 10975                 10980

Pro Glu   Val Pro Gly Met Val   Thr Ser Leu Val Ala   Ser Ser Arg
    10985                 10990                 10995

Ala Val   Thr Ser Thr Thr Leu   Pro Thr Leu Thr Leu   Ser Pro Gly
    11000                 11005                 11010

Glu Pro   Glu Thr Thr Pro Ser   Met Ala Thr Ser His   Gly Ala Glu
    11015                 11020                 11025

Ala Ser   Ser Thr Val Pro Thr   Val Ser Pro Glu Val   Pro Gly Val
    11030                 11035                 11040

Val Thr   Ser Leu Val Thr Ser   Ser Ser Gly Val Asn   Ser Thr Ser
    11045                 11050                 11055

Ile Pro   Thr Leu Ile Leu Ser   Pro Gly Glu Leu Glu   Thr Thr Pro
    11060                 11065                 11070

Ser Met   Ala Thr Ser His Gly   Ala Glu Ala Ser Ser   Ala Val Pro
    11075                 11080                 11085

Thr Pro   Thr Val Ser Pro Gly   Val Ser Gly Val Val   Thr Pro Leu
    11090                 11095                 11100

Val Thr   Ser Ser Arg Ala Val   Thr Ser Thr Thr Ile   Pro Ile Leu
    11105                 11110                 11115

Thr Leu   Ser Ser Ser Glu Pro   Glu Thr Thr Pro Ser   Met Ala Thr
    11120                 11125                 11130

Ser His   Gly Val Glu Ala Ser   Ser Ala Val Leu Thr   Val Ser Pro
    11135                 11140                 11145

Glu Val   Pro Gly Met Val Thr   Ser Leu Val Thr Ser   Ser Arg Ala
    11150                 11155                 11160
```

334

```
Val Thr   Ser Thr Thr Ile Pro   Thr Leu Thr Ile Ser   Ser Asp Glu
    11165             11170              11175

Pro Glu   Thr Thr Thr Ser Leu   Val Thr His Ser Glu   Ala Lys Met
    11180             11185              11190

Ile Ser   Ala Ile Pro Thr Leu   Ala Val Ser Pro Thr   Val Gln Gly
    11195             11200              11205

Leu Val   Thr Ser Leu Val Thr   Ser Ser Gly Ser Glu   Thr Ser Ala
    11210             11215              11220

Phe Ser   Asn Leu Thr Val Ala   Ser Ser Gln Pro Glu   Thr Ile Asp
    11225             11230              11235

Ser Trp   Val Ala His Pro Gly   Thr Glu Ala Ser Ser   Val Val Pro
    11240             11245              11250

Thr Leu   Thr Val Ser Thr Gly   Glu Pro Phe Thr Asn   Ile Ser Leu
    11255             11260              11265

Val Thr   His Pro Ala Glu Ser   Ser Ser Thr Leu Pro   Arg Thr Thr
    11270             11275              11280

Ser Arg   Phe Ser His Ser Glu   Leu Asp Thr Met Pro   Ser Thr Val
    11285             11290              11295

Thr Ser   Pro Glu Ala Glu Ser   Ser Ser Ala Ile Ser   Thr Thr Ile
    11300             11305              11310

Ser Pro   Gly Ile Pro Gly Val   Leu Thr Ser Leu Val   Thr Ser Ser
    11315             11320              11325

Gly Arg   Asp Ile Ser Ala Thr   Phe Pro Thr Val Pro   Glu Ser Pro
    11330             11335              11340

His Glu   Ser Glu Ala Thr Ala   Ser Trp Val Thr His   Pro Ala Val
    11345             11350              11355

Thr Ser   Thr Thr Val Pro Arg   Thr Thr Pro Asn Tyr   Ser His Ser
    11360             11365              11370

Glu Pro   Asp Thr Thr Pro Ser   Ile Ala Thr Ser Pro   Gly Ala Glu
    11375             11380              11385

Ala Thr   Ser Asp Phe Pro Thr   Ile Thr Val Ser Pro   Asp Val Pro
```

|  | 11390 |  |  | 11395 |  |  | 11400 |  |  |

Asp Met Val Thr Ser Gln Val Thr Ser Ser Gly Thr Asp Thr Ser
    11405           11410           11415

Ile Thr Ile Pro Thr Leu Thr Leu Ser Ser Gly Glu Pro Glu Thr
    11420           11425           11430

Thr Thr Ser Phe Ile Thr Tyr Ser Glu Thr His Thr Ser Ser Ala
    11435           11440           11445

Ile Pro Thr Leu Pro Val Ser Pro Gly Ala Ser Lys Met Leu Thr
    11450           11455           11460

Ser Leu Val Ile Ser Ser Gly Thr Asp Ser Thr Thr Thr Phe Pro
    11465           11470           11475

Thr Leu Thr Glu Thr Pro Tyr Glu Pro Glu Thr Thr Ala Ile Gln
    11480           11485           11490

Leu Ile His Pro Ala Glu Thr Asn Thr Met Val Pro Lys Thr Thr
    11495           11500           11505

Pro Lys Phe Ser His Ser Lys Ser Asp Thr Thr Leu Pro Val Ala
    11510           11515           11520

Ile Thr Ser Pro Gly Pro Glu Ala Ser Ser Ala Val Ser Thr Thr
    11525           11530           11535

Thr Ile Ser Pro Asp Met Ser Asp Leu Val Thr Ser Leu Val Pro
    11540           11545           11550

Ser Ser Gly Thr Asp Thr Ser Thr Thr Phe Pro Thr Leu Ser Glu
    11555           11560           11565

Thr Pro Tyr Glu Pro Glu Thr Thr Val Thr Trp Leu Thr His Pro
    11570           11575           11580

Ala Glu Thr Ser Thr Thr Val Ser Gly Thr Ile Pro Asn Phe Ser
    11585           11590           11595

His Arg Gly Ser Asp Thr Ala Pro Ser Met Val Thr Ser Pro Gly
    11600           11605           11610

Val Asp Thr Arg Ser Gly Val Pro Thr Thr Thr Ile Pro Pro Ser
    11615           11620           11625

```
Ile Pro   Gly Val Val Thr Ser   Gln Val Thr Ser Ser   Ala Thr Asp
    11630                 11635                 11640

Thr Ser   Thr Ala Ile Pro Thr   Leu Thr Pro Ser Pro   Gly Glu Pro
    11645                 11650                 11655

Glu Thr   Thr Ala Ser Ser Ala   Thr His Pro Gly Thr   Gln Thr Gly
    11660                 11665                 11670

Phe Thr   Val Pro Ile Arg Thr   Val Pro Ser Ser Glu   Pro Asp Thr
    11675                 11680                 11685

Met Ala   Ser Trp Val Thr His   Pro Pro Gln Thr Ser   Thr Pro Val
    11690                 11695                 11700

Ser Arg   Thr Thr Ser Ser Phe   Ser His Ser Ser Pro   Asp Ala Thr
    11705                 11710                 11715

Pro Val   Met Ala Thr Ser Pro   Arg Thr Glu Ala Ser   Ser Ala Val
    11720                 11725                 11730

Leu Thr   Thr Ile Ser Pro Gly   Ala Pro Glu Met Val   Thr Ser Gln
    11735                 11740                 11745

Ile Thr   Ser Ser Gly Ala Ala   Thr Ser Thr Thr Val   Pro Thr Leu
    11750                 11755                 11760

Thr His   Ser Pro Gly Met Pro   Glu Thr Thr Ala Leu   Leu Ser Thr
    11765                 11770                 11775

His Pro   Arg Thr Gly Thr Ser   Lys Thr Phe Pro Ala   Ser Thr Val
    11780                 11785                 11790

Phe Pro   Gln Val Ser Glu Thr   Thr Ala Ser Leu Thr   Ile Arg Pro
    11795                 11800                 11805

Gly Ala   Glu Thr Ser Thr Ala   Leu Pro Thr Gln Thr   Thr Ser Ser
    11810                 11815                 11820

Leu Phe   Thr Leu Leu Val Thr   Gly Thr Ser Arg Val   Asp Leu Ser
    11825                 11830                 11835

Pro Thr   Ala Ser Pro Gly Val   Ser Ala Lys Thr Ala   Pro Leu Ser
    11840                 11845                 11850

Thr His   Pro Gly Thr Glu Thr   Ser Thr Met Ile Pro   Thr Ser Thr
    11855                 11860                 11865
```

337

```
Leu Ser  Leu Gly Leu Leu Glu  Thr Thr Gly Leu Leu  Ala Thr Ser
    11870              11875          11880

Ser Ser  Ala Glu Thr Ser Thr  Ser Thr Leu Thr Leu  Thr Val Ser
    11885              11890          11895

Pro Ala  Val Ser Gly Leu Ser  Ser Ala Ser Ile Thr  Thr Asp Lys
    11900              11905          11910

Pro Gln  Thr Val Thr Ser Trp  Asn Thr Glu Thr Ser  Pro Ser Val
    11915              11920          11925

Thr Ser  Val Gly Pro Pro Glu  Phe Ser Arg Thr Val  Thr Gly Thr
    11930              11935          11940

Thr Met  Thr Leu Ile Pro Ser  Glu Met Pro Thr Pro  Pro Lys Thr
    11945              11950          11955

Ser His  Gly Glu Gly Val Ser  Pro Thr Thr Ile Leu  Arg Thr Thr
    11960              11965          11970

Met Val  Glu Ala Thr Asn Leu  Ala Thr Thr Gly Ser  Ser Pro Thr
    11975              11980          11985

Val Ala  Lys Thr Thr Thr Thr  Phe Asn Thr Leu Ala  Gly Ser Leu
    11990              11995          12000

Phe Thr  Pro Leu Thr Thr Pro  Gly Met Ser Thr Leu  Ala Ser Glu
    12005              12010          12015

Ser Val  Thr Ser Arg Thr Ser  Tyr Asn His Arg Ser  Trp Ile Ser
    12020              12025          12030

Thr Thr  Ser Ser Tyr Asn Arg  Arg Tyr Trp Thr Pro  Ala Thr Ser
    12035              12040          12045

Thr Pro  Val Thr Ser Thr Phe  Ser Pro Gly Ile Ser  Thr Ser Ser
    12050              12055          12060

Ile Pro  Ser Ser Thr Ala Ala  Thr Val Pro Phe Met  Val Pro Phe
    12065              12070          12075

Thr Leu  Asn Phe Thr Ile Thr  Asn Leu Gln Tyr Glu  Glu Asp Met
    12080              12085          12090

Arg His  Pro Gly Ser Arg Lys  Phe Asn Ala Thr Glu  Arg Glu Leu
    12095              12100          12105
```

338

```
Gln Gly   Leu Leu Lys Pro Leu   Phe Arg Asn Ser Ser   Leu Glu Tyr
    12110             12115                 12120


Leu Tyr   Ser Gly Cys Arg Leu   Ala Ser Leu Arg Pro   Glu Lys Asp
    12125             12130                 12135


Ser Ser   Ala Met Ala Val Asp   Ala Ile Cys Thr His   Arg Pro Asp
    12140             12145                 12150


Pro Glu   Asp Leu Gly Leu Asp   Arg Glu Arg Leu Tyr   Trp Glu Leu
    12155             12160                 12165


Ser Asn   Leu Thr Asn Gly Ile   Gln Glu Leu Gly Pro   Tyr Thr Leu
    12170             12175                 12180


Asp Arg   Asn Ser Leu Tyr Val   Asn Gly Phe Thr His   Arg Ser Ser
    12185             12190                 12195


Met Pro   Thr Thr Ser Thr Pro   Gly Thr Ser Thr Val   Asp Val Gly
    12200             12205                 12210


Thr Ser   Gly Thr Pro Ser Ser   Ser Pro Ser Pro Thr   Ala Ala Gly
    12215             12220                 12225


Pro Leu   Leu Met Pro Phe Thr   Leu Asn Phe Thr Ile   Thr Asn Leu
    12230             12235                 12240


Gln Tyr   Glu Glu Asp Met Arg   Arg Thr Gly Ser Arg   Lys Phe Asn
    12245             12250                 12255


Thr Met   Glu Ser Val Leu Gln   Gly Leu Leu Lys Pro   Leu Phe Lys
    12260             12265                 12270


Asn Thr   Ser Val Gly Pro Leu   Tyr Ser Gly Cys Arg   Leu Thr Leu
    12275             12280                 12285


Leu Arg   Pro Glu Lys Asp Gly   Ala Ala Thr Gly Val   Asp Ala Ile
    12290             12295                 12300


Cys Thr   His Arg Leu Asp Pro   Lys Ser Pro Gly Leu   Asn Arg Glu
    12305             12310                 12315


Gln Leu   Tyr Trp Glu Leu Ser   Lys Leu Thr Asn Asp   Ile Glu Glu
    12320             12325                 12330


Leu Gly   Pro Tyr Thr Leu Asp   Arg Asn Ser Leu Tyr   Val Asn Gly
```

```
              12335                    12340                         12345


        Phe Thr   His Gln Ser Ser Val   Ser Thr Thr Ser Thr   Pro Gly Thr
            12350               12355            12360


        Ser Thr   Val Asp Leu Arg Thr   Ser Gly Thr Pro Ser   Ser Leu Ser
            12365               12370            12375


        Ser Pro   Thr Ile Met Ala Ala   Gly Pro Leu Leu Val   Pro Phe Thr
            12380               12385            12390


        Leu Asn   Phe Thr Ile Thr Asn   Leu Gln Tyr Gly Glu   Asp Met Gly
            12395               12400            12405


        His Pro   Gly Ser Arg Lys Phe   Asn Thr Thr Glu Arg   Val Leu Gln
            12410               12415            12420


        Gly Leu   Leu Gly Pro Ile Phe   Lys Asn Thr Ser Val   Gly Pro Leu
            12425               12430            12435


        Tyr Ser   Gly Cys Arg Leu Thr   Ser Leu Arg Ser Glu   Lys Asp Gly
            12440               12445            12450


        Ala Ala   Thr Gly Val Asp Ala   Ile Cys Ile His His   Leu Asp Pro
            12455               12460            12465


        Lys Ser   Pro Gly Leu Asn Arg   Glu Arg Leu Tyr Trp   Glu Leu Ser
            12470               12475            12480


        Gln Leu   Thr Asn Gly Ile Lys   Glu Leu Gly Pro Tyr   Thr Leu Asp
            12485               12490            12495


        Arg Asn   Ser Leu Tyr Val Asn   Gly Phe Thr His Arg   Thr Ser Val
            12500               12505            12510


        Pro Thr   Thr Ser Thr Pro Gly   Thr Ser Thr Val Asp   Leu Gly Thr
            12515               12520            12525


        Ser Gly   Thr Pro Phe Ser Leu   Pro Ser Pro Ala Thr   Ala Gly Pro
            12530               12535            12540


        Leu Leu   Val Leu Phe Thr Leu   Asn Phe Thr Ile Thr   Asn Leu Lys
            12545               12550            12555


        Tyr Glu   Glu Asp Met His Arg   Pro Gly Ser Arg Lys   Phe Asn Thr
            12560               12565            12570
```

```
Thr Glu   Arg Val Leu Gln Thr   Leu Leu Gly Pro Met   Phe Lys Asn
    12575             12580             12585

Thr Ser   Val Gly Leu Leu Tyr   Ser Gly Cys Arg Leu   Thr Leu Leu
    12590             12595             12600

Arg Ser   Glu Lys Asp Gly Ala   Ala Thr Gly Val Asp   Ala Ile Cys
    12605             12610             12615

Thr His   Arg Leu Asp Pro Lys   Ser Pro Gly Leu Asp   Arg Glu Gln
    12620             12625             12630

Leu Tyr   Trp Glu Leu Ser Gln   Leu Thr Asn Gly Ile   Lys Glu Leu
    12635             12640             12645

Gly Pro   Tyr Thr Leu Asp Arg   Asn Ser Leu Tyr Val   Asn Gly Phe
    12650             12655             12660

Thr His   Trp Ile Pro Val Pro   Thr Ser Ser Thr Pro   Gly Thr Ser
    12665             12670             12675

Thr Val   Asp Leu Gly Ser Gly   Thr Pro Ser Ser Leu   Pro Ser Pro
    12680             12685             12690

Thr Ala   Ala Gly Pro Leu Leu   Val Pro Phe Thr Leu   Asn Phe Thr
    12695             12700             12705

Ile Thr   Asn Leu Gln Tyr Glu   Glu Asp Met His His   Pro Gly Ser
    12710             12715             12720

Arg Lys   Phe Asn Thr Thr Glu   Arg Val Leu Gln Gly   Leu Leu Gly
    12725             12730             12735

Pro Met   Phe Lys Asn Thr Ser   Val Gly Leu Leu Tyr   Ser Gly Cys
    12740             12745             12750

Arg Leu   Thr Leu Leu Arg Ser   Glu Lys Asp Gly Ala   Ala Thr Gly
    12755             12760             12765

Val Asp   Ala Ile Cys Thr His   Arg Leu Asp Pro Lys   Ser Pro Gly
    12770             12775             12780

Val Asp   Arg Glu Gln Leu Tyr   Trp Glu Leu Ser Gln   Leu Thr Asn
    12785             12790             12795

Gly Ile   Lys Glu Leu Gly Pro   Tyr Thr Leu Asp Arg   Asn Ser Leu
    12800             12805             12810
```

341

```
Tyr Val   Asn Gly Phe Thr His   Gln Thr Ser Ala Pro   Asn Thr Ser
    12815                 12820                 12825

Thr Pro   Gly Thr Ser Thr Val   Asp Leu Gly Thr Ser   Gly Thr Pro
    12830                 12835                 12840

Ser Ser   Leu Pro Ser Pro Thr   Ser Ala Gly Pro Leu   Leu Val Pro
    12845                 12850                 12855

Phe Thr   Leu Asn Phe Thr Ile   Thr Asn Leu Gln Tyr   Glu Glu Asp
    12860                 12865                 12870

Met Arg   His Pro Gly Ser Arg   Lys Phe Asn Thr Thr   Glu Arg Val
    12875                 12880                 12885

Leu Gln   Gly Leu Leu Lys Pro   Leu Phe Lys Ser Thr   Ser Val Gly
    12890                 12895                 12900

Pro Leu   Tyr Ser Gly Cys Arg   Leu Thr Leu Leu Arg   Ser Glu Lys
    12905                 12910                 12915

Asp Gly   Ala Ala Thr Gly Val   Asp Ala Ile Cys Thr   His Arg Leu
    12920                 12925                 12930

Asp Pro   Lys Ser Pro Gly Val   Asp Arg Glu Gln Leu   Tyr Trp Glu
    12935                 12940                 12945

Leu Ser   Gln Leu Thr Asn Gly   Ile Lys Glu Leu Gly   Pro Tyr Thr
    12950                 12955                 12960

Leu Asp   Arg Asn Ser Leu Tyr   Val Asn Gly Phe Thr   His Gln Thr
    12965                 12970                 12975

Ser Ala   Pro Asn Thr Ser Thr   Pro Gly Thr Ser Thr   Val Asp Leu
    12980                 12985                 12990

Gly Thr   Ser Gly Thr Pro Ser   Ser Leu Pro Ser Pro   Thr Ser Ala
    12995                 13000                 13005

Gly Pro   Leu Leu Val Pro Phe   Thr Leu Asn Phe Thr   Ile Thr Asn
    13010                 13015                 13020

Leu Gln   Tyr Glu Glu Asp Met   His His Pro Gly Ser   Arg Lys Phe
    13025                 13030                 13035

Asn Thr   Thr Glu Arg Val Leu   Gln Gly Leu Leu Gly   Pro Met Phe
    13040                 13045                 13050
```

```
Lys Asn     Thr Ser Val Gly Leu     Leu Tyr Ser Gly Cys     Arg Leu Thr
    13055               13060               13065

Leu Leu     Arg Pro Glu Lys Asn     Gly Ala Ala Thr Gly     Met Asp Ala
    13070               13075               13080

Ile Cys     Ser His Arg Leu Asp     Pro Lys Ser Pro Gly     Leu Asn Arg
    13085               13090               13095

Glu Gln     Leu Tyr Trp Glu Leu     Ser Gln Leu Thr His     Gly Ile Lys
    13100               13105               13110

Glu Leu     Gly Pro Tyr Thr Leu     Asp Arg Asn Ser Leu     Tyr Val Asn
    13115               13120               13125

Gly Phe     Thr His Arg Ser Ser     Val Ala Pro Thr Ser     Thr Pro Gly
    13130               13135               13140

Thr Ser     Thr Val Asp Leu Gly     Thr Ser Gly Thr Pro     Ser Ser Leu
    13145               13150               13155

Pro Ser     Pro Thr Thr Ala Val     Pro Leu Leu Val Pro     Phe Thr Leu
    13160               13165               13170

Asn Phe     Thr Ile Thr Asn Leu     Gln Tyr Gly Glu Asp     Met Arg His
    13175               13180               13185

Pro Gly     Ser Arg Lys Phe Asn     Thr Thr Glu Arg Val     Leu Gln Gly
    13190               13195               13200

Leu Leu     Gly Pro Leu Phe Lys     Asn Ser Ser Val Gly     Pro Leu Tyr
    13205               13210               13215

Ser Gly     Cys Arg Leu Ile Ser     Leu Arg Ser Glu Lys     Asp Gly Ala
    13220               13225               13230

Ala Thr     Gly Val Asp Ala Ile     Cys Thr His His Leu     Asn Pro Gln
    13235               13240               13245

Ser Pro     Gly Leu Asp Arg Glu     Gln Leu Tyr Trp Gln     Leu Ser Gln
    13250               13255               13260

Met Thr     Asn Gly Ile Lys Glu     Leu Gly Pro Tyr Thr     Leu Asp Arg
    13265               13270               13275

Asn Ser     Leu Tyr Val Asn Gly     Phe Thr His Arg Ser     Ser Gly Leu
```

```
            13280                    13285                        13290

Thr Thr   Ser Thr Pro Trp Thr   Ser Thr Val Asp Leu   Gly Thr Ser
    13295                    13300                    13305

Gly Thr   Pro Ser Pro Val Pro   Ser Pro Thr Thr Ala   Gly Pro Leu
    13310                    13315                    13320

Leu Val   Pro Phe Thr Leu Asn   Phe Thr Ile Thr Asn   Leu Gln Tyr
    13325                    13330                    13335

Glu Glu   Asp Met His Arg Pro   Gly Ser Arg Lys Phe   Asn Thr Thr
    13340                    13345                    13350

Glu Arg   Val Leu Gln Gly Leu   Leu Ser Pro Ile Phe   Lys Asn Ser
    13355                    13360                    13365

Ser Val   Gly Pro Leu Tyr Ser   Gly Cys Arg Leu Thr   Ser Leu Arg
    13370                    13375                    13380

Pro Glu   Lys Asp Gly Ala Ala   Thr Gly Met Asp Ala   Val Cys Leu
    13385                    13390                    13395

Tyr His   Pro Asn Pro Lys Arg   Pro Gly Leu Asp Arg   Glu Gln Leu
    13400                    13405                    13410

Tyr Trp   Glu Leu Ser Gln Leu   Thr His Asn Ile Thr   Glu Leu Gly
    13415                    13420                    13425

Pro Tyr   Ser Leu Asp Arg Asp   Ser Leu Tyr Val Asn   Gly Phe Thr
    13430                    13435                    13440

His Gln   Asn Ser Val Pro Thr   Thr Ser Thr Pro Gly   Thr Ser Thr
    13445                    13450                    13455

Val Tyr   Trp Ala Thr Thr Gly   Thr Pro Ser Ser Phe   Pro Gly His
    13460                    13465                    13470

Thr Glu   Pro Gly Pro Leu Leu   Ile Pro Phe Thr Phe   Asn Phe Thr
    13475                    13480                    13485

Ile Thr   Asn Leu His Tyr Glu   Glu Asn Met Gln His   Pro Gly Ser
    13490                    13495                    13500

Arg Lys   Phe Asn Thr Thr Glu   Arg Val Leu Gln Gly   Leu Leu Lys
    13505                    13510                    13515
```

Pro Leu Phe Lys Asn Thr Ser Val Gly Pro Leu Tyr Ser Gly Cys
    13520             13525             13530

Arg Leu Thr Ser Leu Arg Pro Glu Lys Asp Gly Ala Ala Thr Gly
    13535             13540             13545

Met Asp Ala Val Cys Leu Tyr His Pro Asn Pro Lys Arg Pro Gly
    13550             13555             13560

Leu Asp Arg Glu Gln Leu Tyr Trp Glu Leu Ser Gln Leu Thr His
    13565             13570             13575

Asn Ile Thr Glu Leu Gly Pro Tyr Ser Leu Asp Arg Asp Ser Leu
    13580             13585             13590

Tyr Val Asn Gly Phe Thr His Gln Asn Ser Val Pro Thr Thr Ser
    13595             13600             13605

Thr Pro Gly Thr Ser Thr Val Tyr Trp Ala Thr Thr Gly Thr Pro
    13610             13615             13620

Ser Ser Phe Pro Gly His Thr Glu Pro Gly Pro Leu Leu Ile Pro
    13625             13630             13635

Phe Thr Phe Asn Phe Thr Ile Thr Asn Leu His Tyr Glu Glu Asn
    13640             13645             13650

Met Gln His Pro Gly Ser Arg Lys Phe Asn Thr Thr Glu Arg Val
    13655             13660             13665

Leu Gln Gly Leu Leu Lys Pro Leu Phe Lys Asn Thr Ser Val Gly
    13670             13675             13680

Pro Leu Tyr Ser Gly Cys Arg Leu Thr Leu Leu Arg Pro Glu Lys
    13685             13690             13695

His Glu Ala Ala Thr Gly Val Asp Thr Ile Cys Thr His Arg Val
    13700             13705             13710

Asp Pro Ile Gly Pro Gly Leu Asp Arg Glu Arg Leu Tyr Trp Glu
    13715             13720             13725

Leu Ser Gln Leu Thr Asn Ser Ile Thr Glu Leu Gly Pro Tyr Thr
    13730             13735             13740

Leu Asp Arg Asp Ser Leu Tyr Val Asn Gly Phe Asn Pro Arg Ser
    13745             13750             13755

```
Ser Val Pro Thr Thr Ser Thr Pro Gly Thr Ser Thr Val His Leu
    13760           13765           13770

Ala Thr Ser Gly Thr Pro Ser Ser Leu Pro Gly His Thr Ala Pro
    13775           13780           13785

Val Pro Leu Leu Ile Pro Phe Thr Leu Asn Phe Thr Ile Thr Asn
    13790           13795           13800

Leu His Tyr Glu Glu Asn Met Gln His Pro Gly Ser Arg Lys Phe
    13805           13810           13815

Asn Thr Thr Glu Arg Val Leu Gln Gly Leu Leu Lys Pro Leu Phe
    13820           13825           13830

Lys Asn Thr Ser Val Gly Pro Leu Tyr Ser Gly Cys Arg Leu Thr
    13835           13840           13845

Leu Leu Arg Pro Glu Lys His Glu Ala Ala Thr Gly Val Asp Thr
    13850           13855           13860

Ile Cys Thr His Arg Val Asp Pro Ile Gly Pro Gly Leu Xaa Xaa
    13865           13870           13875

Glu Xaa Leu Tyr Trp Glu Leu Ser Xaa Leu Thr Xaa Xaa Ile Xaa
    13880           13885           13890

Glu Leu Gly Pro Tyr Thr Leu Asp Arg Xaa Ser Leu Tyr Val Asn
    13895           13900           13905

Gly Phe Thr His Xaa Xaa Ser Xaa Pro Thr Thr Ser Thr Pro Gly
    13910           13915           13920

Thr Ser Thr Val Xaa Xaa Gly Thr Ser Gly Thr Pro Ser Ser Xaa
    13925           13930           13935

Pro Xaa Xaa Thr Ser Ala Gly Pro Leu Leu Val Pro Phe Thr Leu
    13940           13945           13950

Asn Phe Thr Ile Thr Asn Leu Gln Tyr Glu Glu Asp Met His His
    13955           13960           13965

Pro Gly Ser Arg Lys Phe Asn Thr Thr Glu Arg Val Leu Gln Gly
    13970           13975           13980

Leu Leu Gly Pro Met Phe Lys Asn Thr Ser Val Gly Leu Leu Tyr
    13985           13990           13995
```

346

```
Ser Gly   Cys Arg Leu Thr Leu   Leu Arg Pro Glu Lys   Asn Gly Ala
    14000                14005             14010

Ala Thr   Gly Met Asp Ala Ile   Cys Ser His Arg Leu   Asp Pro Lys
    14015                14020             14025

Ser Pro   Gly Leu Asp Arg Glu   Gln Leu Tyr Trp Glu   Leu Ser Gln
    14030                14035             14040

Leu Thr   His Gly Ile Lys Glu   Leu Gly Pro Tyr Thr   Leu Asp Arg
    14045                14050             14055

Asn Ser   Leu Tyr Val Asn Gly   Phe Thr His Arg Ser   Ser Val Ala
    14060                14065             14070

Pro Thr   Ser Thr Pro Gly Thr   Ser Thr Val Asp Leu   Gly Thr Ser
    14075                14080             14085

Gly Thr   Pro Ser Ser Leu Pro   Ser Pro Thr Thr Ala   Val Pro Leu
    14090                14095             14100

Leu Val   Pro Phe Thr Leu Asn   Phe Thr Ile Thr Asn   Leu Gln Tyr
    14105                14110             14115

Gly Glu   Asp Met Arg His Pro   Gly Ser Arg Lys Phe   Asn Thr Thr
    14120                14125             14130

Glu Arg   Val Leu Gln Gly Leu   Leu Gly Pro Leu Phe   Lys Asn Ser
    14135                14140             14145

Ser Val   Gly Pro Leu Tyr Ser   Gly Cys Arg Leu Ile   Ser Leu Arg
    14150                14155             14160

Ser Glu   Lys Asp Gly Ala Ala   Thr Gly Val Asp Ala   Ile Cys Thr
    14165                14170             14175

His His   Leu Asn Pro Gln Ser   Pro Gly Leu Asp Arg   Glu Gln Leu
    14180                14185             14190

Tyr Trp   Gln Leu Ser Gln Met   Thr Asn Gly Ile Lys   Glu Leu Gly
    14195                14200             14205

Pro Tyr   Thr Leu Asp Arg Asn   Ser Leu Tyr Val Asn   Gly Phe Thr
    14210                14215             14220

His Arg   Ser Ser Gly Leu Thr   Thr Ser Thr Pro Trp   Thr Ser Thr
```

|  | 14225 |  |  |  |  | 14230 |  |  |  |  | 14235 |  |  |  |

```
Val Asp   Leu Gly Thr Ser Gly   Thr Pro Ser Pro Val   Pro Ser Pro
    14240                 14245                 14250

Thr Thr   Ala Gly Pro Leu Leu   Val Pro Phe Thr Leu   Asn Phe Thr
    14255                 14260                 14265

Ile Thr   Asn Leu Gln Tyr Glu   Glu Asp Met His Arg   Pro Gly Ser
    14270                 14275                 14280

Arg Lys   Phe Asn Ala Thr Glu   Arg Val Leu Gln Gly   Leu Leu Ser
    14285                 14290                 14295

Pro Ile   Phe Lys Asn Ser Ser   Val Gly Pro Leu Tyr   Ser Gly Cys
    14300                 14305                 14310

Arg Leu   Thr Ser Leu Arg Pro   Glu Lys Asp Gly Ala   Ala Thr Gly
    14315                 14320                 14325

Met Asp   Ala Val Cys Leu Tyr   His Pro Asn Pro Lys   Arg Pro Gly
    14330                 14335                 14340

Leu Asp   Arg Glu Gln Leu Tyr   Trp Glu Leu Ser Gln   Leu Thr His
    14345                 14350                 14355

Asn Ile   Thr Glu Leu Gly Pro   Tyr Ser Leu Asp Arg   Asp Ser Leu
    14360                 14365                 14370

Tyr Val   Asn Gly Phe Thr His   Gln Ser Ser Met Thr   Thr Thr Arg
    14375                 14380                 14385

Thr Pro   Asp Thr Ser Thr Met   His Leu Ala Thr Ser   Arg Thr Pro
    14390                 14395                 14400

Ala Ser   Leu Ser Gly Pro Thr   Thr Ala Ser Pro Leu   Leu Val Leu
    14405                 14410                 14415

Phe Thr   Ile Asn Cys Thr Ile   Thr Asn Leu Gln Tyr   Glu Glu Asp
    14420                 14425                 14430

Met Arg   Arg Thr Gly Ser Arg   Lys Phe Asn Thr Met   Glu Ser Val
    14435                 14440                 14445

Leu Gln   Gly Leu Leu Lys Pro   Leu Phe Lys Asn Thr   Ser Val Gly
    14450                 14455                 14460
```

```
Pro Leu Tyr Ser Gly Cys Arg   Leu Thr Leu Leu Arg   Pro Lys Lys
    14465               14470               14475

Asp Gly Ala Ala Thr Gly Val   Asp Ala Ile Cys Thr   His Arg Leu
    14480               14485               14490

Asp Pro Lys Ser Pro Gly Leu   Asn Arg Glu Gln Leu   Tyr Trp Glu
    14495               14500               14505

Leu Ser Lys Leu Thr Asn Asp   Ile Glu Glu Leu Gly   Pro Tyr Thr
    14510               14515               14520

Leu Asp Arg Asn Ser Leu Tyr   Val Asn Gly Phe Thr   His Gln Ser
    14525               14530               14535

Ser Val Ser Thr Thr Ser Thr   Pro Gly Thr Ser Thr   Val Asp Leu
    14540               14545               14550

Arg Thr Ser Gly Thr Pro Ser   Ser Leu Ser Ser Pro   Thr Ile Met
    14555               14560               14565

Xaa Xaa Xaa Pro Leu Leu Xaa   Pro Phe Thr Xaa Asn   Xaa Thr Ile
    14570               14575               14580

Thr Asn Leu Xaa Xaa Xaa Xaa   Xaa Met Xaa Xaa Pro   Gly Ser Arg
    14585               14590               14595

Lys Phe Asn Thr Thr Glu Arg   Val Leu Gln Gly Leu   Leu Arg Pro
    14600               14605               14610

Leu Phe Lys Asn Thr Ser Val   Ser Ser Leu Tyr Ser   Gly Cys Arg
    14615               14620               14625

Leu Thr Leu Leu Arg Pro Glu   Lys Asp Gly Ala Ala   Thr Arg Val
    14630               14635               14640

Asp Ala Ala Cys Thr Tyr Arg   Pro Asp Pro Lys Ser   Pro Gly Leu
    14645               14650               14655

Asp Arg Glu Gln Leu Tyr Trp   Glu Leu Ser Gln Leu   Thr His Ser
    14660               14665               14670

Ile Thr Glu Leu Gly Pro Tyr   Thr Leu Asp Arg Val   Ser Leu Tyr
    14675               14680               14685

Val Asn Gly Phe Asn Pro Arg   Ser Ser Val Pro Thr   Thr Ser Thr
    14690               14695               14700
```

349

```
Pro Gly   Thr Ser Thr Val His   Leu Ala Thr Ser Gly   Thr Pro Ser
    14705             14710                   14715

Ser Leu   Pro Gly His Thr Xaa   Xaa Xaa Pro Leu Leu   Xaa Pro Phe
    14720             14725                   14730

Thr Xaa   Asn Xaa Thr Ile Thr   Asn Leu Xaa Xaa Xaa   Xaa Xaa Met
    14735             14740                   14745

Xaa Xaa   Pro Gly Ser Arg Lys   Phe Asn Thr Thr Glu   Arg Val Leu
    14750             14755                   14760

Gln Gly   Leu Leu Lys Pro Leu   Phe Arg Asn Ser Ser   Leu Glu Tyr
    14765             14770                   14775

Leu Tyr   Ser Gly Cys Arg Leu   Ala Ser Leu Arg Pro   Glu Lys Asp
    14780             14785                   14790

Ser Ser   Ala Met Ala Val Asp   Ala Ile Cys Thr His   Arg Pro Asp
    14795             14800                   14805

Pro Glu   Asp Leu Gly Leu Asp   Arg Glu Arg Leu Tyr   Trp Glu Leu
    14810             14815                   14820

Ser Asn   Leu Thr Asn Gly Ile   Gln Glu Leu Gly Pro   Tyr Thr Leu
    14825             14830                   14835

Asp Arg   Asn Ser Leu Tyr Val   Asn Gly Phe Thr His   Arg Ser Ser
    14840             14845                   14850

Gly Leu   Thr Thr Ser Thr Pro   Trp Thr Ser Thr Val   Asp Leu Gly
    14855             14860                   14865

Thr Ser   Gly Thr Pro Ser Pro   Val Pro Ser Pro Thr   Thr Ala Gly
    14870             14875                   14880

Pro Leu   Leu Val Pro Phe Thr   Leu Asn Phe Thr Ile   Thr Asn Leu
    14885             14890                   14895

Gln Tyr   Glu Glu Asp Met His   Arg Pro Gly Ser Arg   Arg Phe Asn
    14900             14905                   14910

Thr Thr   Glu Arg Val Leu Gln   Gly Leu Leu Thr Pro   Leu Phe Lys
    14915             14920                   14925

Asn Thr   Ser Val Gly Pro Leu   Tyr Ser Gly Cys Arg   Leu Thr Leu
    14930             14935                   14940
```

```
Leu Arg   Pro Glu Lys Gln Glu   Ala Ala Thr Gly Val   Asp Thr Ile
    14945               14950            14955

Cys Thr   His Arg Val Asp Pro   Ile Gly Pro Gly Leu   Asp Arg Glu
    14960               14965            14970

Arg Leu   Tyr Trp Glu Leu Ser   Gln Leu Thr Asn Ser   Ile Thr Glu
    14975               14980            14985

Leu Gly   Pro Tyr Thr Leu Asp   Arg Asp Ser Leu Tyr   Val Asn Gly
    14990               14995            15000

Phe Asn   Pro Trp Ser Ser Val   Pro Thr Thr Ser Thr   Pro Gly Thr
    15005               15010            15015

Ser Thr   Val His Leu Ala Thr   Ser Gly Thr Pro Ser   Ser Leu Pro
    15020               15025            15030

Gly His   Thr Ala Pro Val Pro   Leu Leu Ile Pro Phe   Thr Leu Asn
    15035               15040            15045

Phe Thr   Ile Thr Asp Leu His   Tyr Glu Glu Asn Met   Gln His Pro
    15050               15055            15060

Gly Ser   Arg Lys Phe Asn Thr   Thr Glu Arg Val Leu   Gln Gly Leu
    15065               15070            15075

Leu Lys   Pro Leu Phe Lys Ser   Thr Ser Val Gly Pro   Leu Tyr Ser
    15080               15085            15090

Gly Cys   Arg Leu Thr Leu Leu   Arg Pro Glu Lys His   Gly Ala Ala
    15095               15100            15105

Thr Gly   Val Asp Ala Ile Cys   Thr Leu Arg Leu Asp   Pro Thr Gly
    15110               15115            15120

Pro Gly   Leu Asp Arg Glu Arg   Leu Tyr Trp Glu Leu   Ser Gln Leu
    15125               15130            15135

Thr Asn   Ser Val Thr Glu Leu   Gly Pro Tyr Thr Leu   Asp Arg Asp
    15140               15145            15150

Ser Leu   Tyr Val Asn Gly Phe   Thr His Arg Ser Ser   Val Pro Thr
    15155               15160            15165

Thr Ser   Ile Pro Gly Thr Ser   Ala Val His Leu Glu   Thr Ser Gly
```

351

```
        15170                    15175                        15180

     Thr Pro  Ala Ser Leu Pro Gly   His Thr Ala Pro Gly   Pro Leu Leu
         15185                  15190                15195

     Val Pro  Phe Thr Leu Asn Phe   Thr Ile Thr Asn Leu   Gln Tyr Glu
         15200                  15205                15210

     Glu Asp  Met Arg His Pro Gly   Ser Arg Lys Phe Ser   Thr Thr Glu
         15215                  15220                15225

     Arg Val  Leu Gln Gly Leu Leu   Lys Pro Leu Phe Lys   Asn Thr Ser
         15230                  15235                15240

     Val Ser  Ser Leu Tyr Ser Gly   Cys Arg Leu Thr Leu   Leu Arg Pro
         15245                  15250                15255

     Glu Lys  Asp Gly Ala Ala Thr   Arg Val Asp Ala Val   Cys Thr His
         15260                  15265                15270

     Arg Pro  Asp Pro Lys Ser Pro   Gly Leu Asp Arg Glu   Arg Leu Tyr
         15275                  15280                15285

     Trp Lys  Leu Ser Gln Leu Thr   His Gly Ile Thr Glu   Leu Gly Pro
         15290                  15295                15300

     Tyr Thr  Leu Asp Arg His Ser   Leu Tyr Val Asn Gly   Phe Thr His
         15305                  15310                15315

     Gln Ser  Ser Met Thr Thr Thr   Arg Thr Pro Asp Thr   Ser Thr Met
         15320                  15325                15330

     His Leu  Ala Thr Ser Arg Thr   Pro Ala Ser Leu Ser   Gly Pro Thr
         15335                  15340                15345

     Thr Ala  Ser Pro Leu Leu Val   Leu Phe Thr Ile Asn   Phe Thr Ile
         15350                  15355                15360

     Thr Asn  Leu Arg Tyr Glu Glu   Asn Met His His Pro   Gly Ser Arg
         15365                  15370                15375

     Lys Phe  Asn Thr Thr Glu Arg   Val Leu Gln Gly Leu   Leu Arg Pro
         15380                  15385                15390

     Val Phe  Lys Asn Thr Ser Val   Gly Pro Leu Tyr Ser   Gly Cys Arg
         15395                  15400                15405
```

352

Leu Thr Thr Leu Arg Pro Lys      Lys Asp Gly Ala Ala      Thr Lys Val
    15410           15415                     15420

Asp Ala Ile Cys Thr Tyr Arg      Pro Asp Pro Lys Ser      Pro Gly Leu
    15425           15430                     15435

Asp Arg Glu Gln Leu Tyr Trp      Glu Leu Ser Gln Leu      Thr His Ser
    15440           15445                     15450

Ile Thr Glu Leu Gly Pro Tyr      Thr Gln Asp Arg Asp      Ser Leu Tyr
    15455           15460                     15465

Val Asn Gly Phe Thr His Arg      Ser Ser Val Pro Thr      Thr Ser Ile
    15470           15475                     15480

Pro Gly Thr Ser Ala Val His      Leu Glu Thr Ser Gly      Thr Pro Ala
    15485           15490                     15495

Ser Leu Pro Gly His Thr Ala      Pro Gly Pro Leu Leu      Val Pro Phe
    15500           15505                     15510

Thr Leu Asn Phe Thr Ile Thr      Asn Leu Gln Tyr Glu      Glu Asp Met
    15515           15520                     15525

Arg His Pro Gly Ser Arg Lys      Phe Asn Thr Thr Glu      Arg Val Leu
    15530           15535                     15540

Gln Gly Leu Leu Lys Pro Leu      Phe Lys Ser Thr Ser      Val Gly Pro
    15545           15550                     15555

Leu Tyr Ser Gly Cys Arg Leu      Thr Leu Leu Arg Pro      Glu Lys Arg
    15560           15565                     15570

Gly Ala Ala Thr Gly Val Asp      Thr Ile Cys Thr His      Arg Leu Asp
    15575           15580                     15585

Pro Leu Asn Pro Gly Leu Asp      Arg Glu Gln Leu Tyr      Trp Glu Leu
    15590           15595                     15600

Ser Lys Leu Thr Arg Gly Ile      Ile Glu Leu Gly Pro      Tyr Leu Leu
    15605           15610                     15615

Asp Arg Gly Ser Leu Tyr Val      Asn Gly Phe Thr His      Arg Thr Ser
    15620           15625                     15630

Val Pro Thr Thr Ser Thr Pro      Gly Thr Ser Thr Val      Asp Leu Gly
    15635           15640                     15645

```
Thr Ser  Gly Thr Pro Phe Ser  Leu Pro Ser Pro Ala  Xaa Xaa Xaa
    15650             15655             15660

Pro Leu  Leu Xaa Pro Phe Thr  Xaa Asn Xaa Thr Ile  Thr Asn Leu
    15665             15670             15675

Xaa Xaa  Xaa Xaa Xaa Met Xaa  Xaa Pro Gly Ser Arg  Lys Phe Asn
    15680             15685             15690

Thr Thr  Glu Arg Val Leu Gln  Thr Leu Leu Gly Pro  Met Phe Lys
    15695             15700             15705

Asn Thr  Ser Val Gly Leu Leu  Tyr Ser Gly Cys Arg  Leu Thr Leu
    15710             15715             15720

Leu Arg  Ser Glu Lys Asp Gly  Ala Ala Thr Gly Val  Asp Ala Ile
    15725             15730             15735

Cys Thr  His Arg Leu Asp Pro  Lys Ser Pro Gly Val  Asp Arg Glu
    15740             15745             15750

Gln Leu  Tyr Trp Glu Leu Ser  Gln Leu Thr Asn Gly  Ile Lys Glu
    15755             15760             15765

Leu Gly  Pro Tyr Thr Leu Asp  Arg Asn Ser Leu Tyr  Val Asn Gly
    15770             15775             15780

Phe Thr  His Trp Ile Pro Val  Pro Thr Ser Ser Thr  Pro Gly Thr
    15785             15790             15795

Ser Thr  Val Asp Leu Gly Ser  Gly Thr Pro Ser Ser  Leu Pro Ser
    15800             15805             15810

Pro Thr  Thr Ala Gly Pro Leu  Leu Val Pro Phe Thr  Leu Asn Phe
    15815             15820             15825

Thr Ile  Thr Asn Leu Lys Tyr  Glu Glu Asp Met His  Cys Pro Gly
    15830             15835             15840

Ser Arg  Lys Phe Asn Thr Thr  Glu Arg Val Leu Gln  Ser Leu Leu
    15845             15850             15855

Gly Pro  Met Phe Lys Asn Thr  Ser Val Gly Pro Leu  Tyr Ser Gly
    15860             15865             15870

Cys Arg  Leu Thr Leu Leu Arg  Ser Glu Lys Asp Gly  Ala Ala Thr
    15875             15880             15885
```

354

```
Gly Val   Asp Ala Ile Cys Thr   His Arg Leu Asp Pro   Lys Ser Pro
    15890               15895           15900

Gly Val   Asp Arg Glu Gln Leu   Tyr Trp Glu Leu Ser   Gln Leu Thr
    15905               15910           15915

Asn Gly   Ile Lys Glu Leu Gly   Pro Tyr Thr Leu Asp   Arg Asn Ser
    15920               15925           15930

Leu Tyr   Val Asn Gly Phe Thr   His Gln Thr Ser Ala   Pro Asn Thr
    15935               15940           15945

Ser Thr   Pro Gly Thr Ser Thr   Val Asp Leu Gly Thr   Ser Gly Thr
    15950               15955           15960

Pro Ser   Ser Leu Pro Ser Pro   Thr Xaa Xaa Xaa Pro   Leu Leu Xaa
    15965               15970           15975

Pro Phe   Thr Xaa Asn Xaa Thr   Ile Thr Asn Leu Xaa   Xaa Xaa Xaa
    15980               15985           15990

Xaa Met   Xaa Xaa Pro Gly Ser   Arg Lys Phe Asn Thr   Thr Glu Xaa
    15995               16000           16005

Val Leu   Gln Gly Leu Leu Xaa   Pro Xaa Phe Lys Asn   Xaa Ser Val
    16010               16015           16020

Gly Xaa   Leu Tyr Ser Gly Cys   Arg Leu Thr Xaa Leu   Arg Xaa Glu
    16025               16030           16035

Lys Xaa   Gly Ala Ala Thr Gly   Xaa Asp Ala Ile Cys   Xaa His Xaa
    16040               16045           16050

Xaa Xaa   Pro Lys Xaa Pro Gly   Leu Xaa Xaa Glu Xaa   Leu Tyr Trp
    16055               16060           16065

Glu Leu   Ser Xaa Leu Thr Xaa   Xaa Ile Xaa Glu Leu   Gly Pro Tyr
    16070               16075           16080

Thr Leu   Asp Arg Xaa Ser Leu   Tyr Val Asn Gly Phe   Thr His Trp
    16085               16090           16095

Ile Pro   Val Pro Thr Ser Ser   Thr Pro Gly Thr Ser   Thr Val Asp
    16100               16105           16110

Leu Gly   Ser Gly Thr Pro Ser   Ser Leu Pro Ser Pro   Thr Thr Ala
```

16115     16120     16125

Gly Pro Leu Leu Val Pro Phe  Thr Leu Asn Phe Thr  Ile Thr Asn
  16130     16135     16140

Leu Lys Tyr Glu Glu Asp Met  His Cys Pro Gly Ser  Arg Lys Phe
  16145     16150     16155

Asn Thr Thr Glu Arg Val Leu  Gln Ser Leu Leu Gly  Pro Met Phe
  16160     16165     16170

Lys Asn Thr Ser Val Gly Pro  Leu Tyr Ser Gly Cys  Arg Leu Thr
  16175     16180     16185

Ser Leu Arg Ser Glu Lys Asp  Gly Ala Ala Thr Gly  Val Asp Ala
  16190     16195     16200

Ile Cys Thr His Arg Val Asp  Pro Lys Ser Pro Gly  Val Asp Arg
  16205     16210     16215

Glu Gln Leu Tyr Trp Glu Leu  Ser Gln Leu Thr Asn  Gly Ile Lys
  16220     16225     16230

Glu Leu Gly Pro Tyr Thr Leu  Asp Arg Asn Ser Leu  Tyr Val Asn
  16235     16240     16245

Gly Phe Thr His Gln Thr Ser  Ala Pro Asn Thr Ser  Thr Pro Gly
  16250     16255     16260

Thr Ser Thr Val Xaa Xaa Gly  Thr Ser Gly Thr Pro  Ser Ser Xaa
  16265     16270     16275

Pro Xaa Xaa Thr Ser Ala Gly  Pro Leu Leu Val Pro  Phe Thr Leu
  16280     16285     16290

Asn Phe Thr Ile Thr Asn Leu  Gln Tyr Glu Glu Asp  Met His His
  16295     16300     16305

Pro Gly Ser Arg Lys Phe Asn  Thr Thr Glu Arg Val  Leu Gln Gly
  16310     16315     16320

Leu Leu Gly Pro Met Phe Lys  Asn Thr Ser Val Gly  Leu Leu Tyr
  16325     16330     16335

Ser Gly Cys Arg Leu Thr Leu  Leu Arg Pro Glu Lys  Asn Gly Ala
  16340     16345     16350

```
Thr Thr   Gly Met Asp Ala Ile   Cys Thr His Arg Leu   Asp Pro Lys
    16355                 16360               16365

Ser Pro   Gly Leu Xaa Xaa Glu   Xaa Leu Tyr Trp Glu   Leu Ser Xaa
    16370                 16375               16380

Leu Thr   Xaa Xaa Ile Xaa Glu   Leu Gly Pro Tyr Thr   Leu Asp Arg
    16385                 16390               16395

Xaa Ser   Leu Tyr Val Asn Gly   Phe Thr His Xaa Xaa   Ser Xaa Pro
    16400                 16405               16410

Thr Thr   Ser Thr Pro Gly Thr   Ser Thr Val Xaa Xaa   Gly Thr Ser
    16415                 16420               16425

Gly Thr   Pro Ser Ser Xaa Pro   Xaa Xaa Thr Xaa Xaa   Xaa Pro Leu
    16430                 16435               16440

Leu Xaa   Pro Phe Thr Xaa Asn   Xaa Thr Ile Thr Asn   Leu Xaa Xaa
    16445                 16450               16455

Xaa Xaa   Xaa Met Xaa Xaa Pro   Gly Ser Arg Lys Phe   Asn Thr Thr
    16460                 16465               16470

Glu Arg   Val Leu Gln Gly Leu   Leu Lys Pro Leu Phe   Arg Asn Ser
    16475                 16480               16485

Ser Leu   Glu Tyr Leu Tyr Ser   Gly Cys Arg Leu Ala   Ser Leu Arg
    16490                 16495               16500

Pro Glu   Lys Asp Ser Ser Ala   Met Ala Val Asp Ala   Ile Cys Thr
    16505                 16510               16515

His Arg   Pro Asp Pro Glu Asp   Leu Gly Leu Asp Arg   Glu Arg Leu
    16520                 16525               16530

Tyr Trp   Glu Leu Ser Asn Leu   Thr Asn Gly Ile Gln   Glu Leu Gly
    16535                 16540               16545

Pro Tyr   Thr Leu Asp Arg Asn   Ser Leu Tyr Val Asn   Gly Phe Thr
    16550                 16555               16560

His Arg   Ser Ser Met Pro Thr   Thr Ser Thr Pro Gly   Thr Ser Thr
    16565                 16570               16575

Val Asp   Val Gly Thr Ser Gly   Thr Pro Ser Ser Ser   Pro Ser Pro
    16580                 16585               16590
```

357

```
Thr Thr   Ala Gly Pro Leu Leu   Ile Pro Phe Thr Leu   Asn Phe Thr
    16595                 16600                 16605

Ile Thr   Asn Leu Gln Tyr Gly   Glu Asp Met Gly His   Pro Gly Ser
    16610                 16615                 16620

Arg Lys   Phe Asn Thr Thr Glu   Arg Val Leu Gln Gly   Leu Leu Gly
    16625                 16630                 16635

Pro Ile   Phe Lys Asn Thr Ser   Val Gly Pro Leu Tyr   Ser Gly Cys
    16640                 16645                 16650

Arg Leu   Thr Ser Leu Arg Ser   Glu Lys Asp Gly Ala   Ala Thr Gly
    16655                 16660                 16665

Val Asp   Ala Ile Cys Ile His   His Leu Asp Pro Lys   Ser Pro Gly
    16670                 16675                 16680

Leu Asn   Arg Glu Arg Leu Tyr   Trp Glu Leu Ser Gln   Leu Thr Asn
    16685                 16690                 16695

Gly Ile   Lys Glu Leu Gly Pro   Tyr Thr Leu Asp Arg   Asn Ser Leu
    16700                 16705                 16710

Tyr Val   Asn Gly Phe Thr His   Arg Thr Ser Val Pro   Thr Thr Ser
    16715                 16720                 16725

Thr Pro   Gly Thr Ser Thr Val   Asp Leu Gly Thr Ser   Gly Thr Pro
    16730                 16735                 16740

Phe Ser   Leu Pro Ser Pro Ala   Thr Ala Gly Pro Leu   Leu Val Leu
    16745                 16750                 16755

Phe Thr   Leu Asn Phe Thr Ile   Thr Asn Leu Lys Tyr   Glu Glu Asp
    16760                 16765                 16770

Met His   Arg Pro Gly Ser Arg   Lys Phe Asn Thr Thr   Glu Arg Val
    16775                 16780                 16785

Leu Gln   Thr Leu Leu Gly Pro   Met Phe Lys Asn Thr   Ser Val Gly
    16790                 16795                 16800

Leu Leu   Tyr Ser Gly Cys Arg   Leu Thr Leu Leu Arg   Ser Glu Lys
    16805                 16810                 16815

Asp Gly   Ala Ala Thr Gly Val   Asp Ala Ile Cys Thr   His Arg Leu
    16820                 16825                 16830
```

358

```
Asp Pro  Lys Ser Pro Gly Leu   Xaa Xaa Glu Xaa Leu   Tyr Trp Glu
    16835               16840           16845

Leu Ser  Xaa Leu Thr Xaa Xaa   Ile Xaa Glu Leu Gly   Pro Tyr Thr
    16850               16855           16860

Leu Asp  Arg Xaa Ser Leu Tyr   Val Asn Gly Phe Thr   His Xaa Xaa
    16865               16870           16875

Ser Xaa  Pro Thr Thr Ser Thr   Pro Gly Thr Ser Thr   Val Xaa Xaa
    16880               16885           16890

Gly Thr  Ser Gly Thr Pro Ser   Ser Xaa Pro Xaa Xaa   Thr Xaa Xaa
    16895               16900           16905

Xaa Pro  Leu Leu Xaa Pro Phe   Thr Xaa Asn Xaa Thr   Ile Thr Asn
    16910               16915           16920

Leu Xaa  Xaa Xaa Xaa Xaa Met   Xaa Xaa Pro Gly Ser   Arg Lys Phe
    16925               16930           16935

Asn Thr  Thr Glu Arg Val Leu   Gln Gly Leu Leu Arg   Pro Val Phe
    16940               16945           16950

Lys Asn  Thr Ser Val Gly Pro   Leu Tyr Ser Gly Cys   Arg Leu Thr
    16955               16960           16965

Leu Leu  Arg Pro Lys Lys Asp   Gly Ala Ala Thr Lys   Val Asp Ala
    16970               16975           16980

Ile Cys  Thr Tyr Arg Pro Asp   Pro Lys Ser Pro Gly   Leu Asp Arg
    16985               16990           16995

Glu Gln  Leu Tyr Trp Glu Leu   Ser Gln Leu Thr His   Ser Ile Thr
    17000               17005           17010

Glu Leu  Gly Pro Tyr Thr Gln   Asp Arg Asp Ser Leu   Tyr Val Asn
    17015               17020           17025

Gly Phe  Thr His Arg Ser Ser   Val Pro Thr Thr Ser   Ile Pro Gly
    17030               17035           17040

Thr Ser  Ala Val His Leu Glu   Thr Thr Gly Thr Pro   Ser Ser Phe
    17045               17050           17055

Pro Gly  His Thr Glu Pro Gly   Pro Leu Leu Ile Pro   Phe Thr Phe
```

17060　　　　　　　　　　17065　　　　　　　　　　17070

Asn Phe　Thr Ile Thr Asn Leu　Arg Tyr Glu Glu Asn　Met Gln His
　　17075　　　　　　　　17080　　　　　　　　17085

Pro Gly　Ser Arg Lys Phe Asn　Thr Thr Glu Arg Val　Leu Gln Gly
　　17090　　　　　　　　17095　　　　　　　　17100

Leu Leu　Thr Pro Leu Phe Lys　Asn Thr Ser Val Gly　Pro Leu Tyr
　　17105　　　　　　　　17110　　　　　　　　17115

Ser Gly　Cys Arg Leu Thr Leu　Leu Arg Pro Glu Lys　Gln Glu Ala
　　17120　　　　　　　　17125　　　　　　　　17130

Ala Thr　Gly Val Asp Thr Ile　Cys Thr His Arg Val　Asp Pro Ile
　　17135　　　　　　　　17140　　　　　　　　17145

Gly Pro　Gly Leu Asp Arg Glu　Arg Leu Tyr Trp Glu　Leu Ser Gln
　　17150　　　　　　　　17155　　　　　　　　17160

Leu Thr　Asn Ser Ile Thr Glu　Leu Gly Pro Tyr Thr　Leu Asp Arg
　　17165　　　　　　　　17170　　　　　　　　17175

Asp Ser　Leu Tyr Val Asp Gly　Phe Asn Pro Trp Ser　Ser Val Pro
　　17180　　　　　　　　17185　　　　　　　　17190

Thr Thr　Ser Thr Pro Gly Thr　Ser Thr Val His Leu　Ala Thr Ser
　　17195　　　　　　　　17200　　　　　　　　17205

Gly Thr　Pro Ser Pro Leu Pro　Gly His Thr Ala Pro　Val Pro Leu
　　17210　　　　　　　　17215　　　　　　　　17220

Leu Ile　Pro Phe Thr Leu Asn　Phe Thr Ile Thr Asp　Leu His Tyr
　　17225　　　　　　　　17230　　　　　　　　17235

Glu Glu　Asn Met Gln His Pro　Gly Ser Arg Lys Phe　Asn Thr Thr
　　17240　　　　　　　　17245　　　　　　　　17250

Glu Arg　Val Leu Gln Gly Leu　Leu Lys Pro Leu Phe　Lys Ser Thr
　　17255　　　　　　　　17260　　　　　　　　17265

Ser Val　Gly Pro Leu Tyr Ser　Gly Cys Arg Leu Thr　Leu Leu Arg
　　17270　　　　　　　　17275　　　　　　　　17280

Pro Glu　Lys His Gly Ala Ala　Thr Gly Val Asp Ala　Ile Cys Thr
　　17285　　　　　　　　17290　　　　　　　　17295

360

```
Leu Arg   Leu Asp Pro Thr Gly   Pro Gly Leu Asp Arg   Glu Arg Leu
    17300                17305                17310

Tyr Trp   Glu Leu Ser Gln Leu   Thr Asn Ser Ile Thr   Glu Leu Gly
    17315                17320                17325

Pro Tyr   Thr Leu Asp Arg Asp   Ser Leu Tyr Val Asn   Gly Phe Asn
    17330                17335                17340

Pro Trp   Ser Ser Val Pro Thr   Thr Ser Thr Pro Gly   Thr Ser Thr
    17345                17350                17355

Val His   Leu Ala Thr Ser Gly   Thr Pro Ser Ser Leu   Pro Gly His
    17360                17365                17370

Thr Thr   Ala Gly Pro Leu Leu   Val Pro Phe Thr Leu   Asn Phe Thr
    17375                17380                17385

Ile Thr   Asn Leu Lys Tyr Glu   Glu Asp Met His Cys   Pro Gly Ser
    17390                17395                17400

Arg Lys   Phe Asn Thr Thr Glu   Arg Val Leu Gln Ser   Leu His Gly
    17405                17410                17415

Pro Met   Phe Lys Asn Thr Ser   Val Gly Pro Leu Tyr   Ser Gly Cys
    17420                17425                17430

Arg Leu   Thr Leu Leu Arg Ser   Glu Lys Asp Gly Ala   Ala Thr Gly
    17435                17440                17445

Val Asp   Ala Ile Cys Thr His   Arg Leu Asp Pro Lys   Ser Pro Gly
    17450                17455                17460

Leu Xaa   Xaa Glu Xaa Leu Tyr   Trp Glu Leu Ser Xaa   Leu Thr Xaa
    17465                17470                17475

Xaa Ile   Xaa Glu Leu Gly Pro   Tyr Thr Leu Asp Arg   Xaa Ser Leu
    17480                17485                17490

Tyr Val   Asn Gly Phe Thr His   Xaa Xaa Ser Xaa Pro   Thr Thr Ser
    17495                17500                17505

Thr Pro   Gly Thr Ser Thr Val   Xaa Xaa Gly Thr Ser   Gly Thr Pro
    17510                17515                17520

Ser Ser   Xaa Pro Xaa Xaa Thr   Xaa Xaa Xaa Pro Leu   Leu Xaa Pro
    17525                17530                17535
```

361

```
Phe Thr    Xaa Asn Xaa Thr Ile    Thr Asn Leu Xaa Xaa    Xaa Xaa Xaa
    17540               17545                17550

Met Xaa    Xaa Pro Gly Ser Arg    Lys Phe Asn Thr Thr    Glu Xaa Val
    17555               17560                17565

Leu Gln    Gly Leu Leu Xaa Pro    Xaa Phe Lys Asn Xaa    Ser Val Gly
    17570               17575                17580

Xaa Leu    Tyr Ser Gly Cys Arg    Leu Thr Xaa Leu Arg    Xaa Glu Lys
    17585               17590                17595

Xaa Gly    Ala Ala Thr Gly Xaa    Asp Ala Ile Cys Xaa    His Xaa Xaa
    17600               17605                17610

Xaa Pro    Lys Xaa Pro Gly Leu    Xaa Xaa Glu Xaa Leu    Tyr Trp Glu
    17615               17620                17625

Leu Ser    Xaa Leu Thr Asn Ser    Ile Thr Glu Leu Gly    Pro Tyr Thr
    17630               17635                17640

Leu Asp    Arg Asp Ser Leu Tyr    Val Asn Gly Phe Thr    His Arg Ser
    17645               17650                17655

Ser Met    Pro Thr Thr Ser Ile    Pro Gly Thr Ser Ala    Val His Leu
    17660               17665                17670

Glu Thr    Ser Gly Thr Pro Ala    Ser Leu Pro Gly His    Thr Ala Pro
    17675               17680                17685

Gly Pro    Leu Leu Val Pro Phe    Thr Leu Asn Phe Thr    Ile Thr Asn
    17690               17695                17700

Leu Gln    Tyr Glu Glu Asp Met    Arg His Pro Gly Ser    Arg Lys Phe
    17705               17710                17715

Asn Thr    Thr Glu Arg Val Leu    Gln Gly Leu Leu Lys    Pro Leu Phe
    17720               17725                17730

Lys Ser    Thr Ser Val Gly Pro    Leu Tyr Ser Gly Cys    Arg Leu Thr
    17735               17740                17745

Leu Leu    Arg Pro Glu Lys Arg    Gly Ala Ala Thr Gly    Val Asp Thr
    17750               17755                17760

Ile Cys    Thr His Arg Leu Asp    Pro Leu Asn Pro Gly    Leu Xaa Xaa
    17765               17770                17775
```

Glu Xaa   Leu Tyr Trp Glu Leu   Ser Xaa Leu Thr Xaa   Xaa Ile Xaa
    17780                 17785             17790

Glu Leu   Gly Pro Tyr Thr Leu   Asp Arg Xaa Ser Leu   Tyr Val Asn
    17795                 17800             17805

Gly Phe   Thr His Xaa Xaa Ser   Xaa Pro Thr Thr Ser   Thr Pro Gly
    17810                 17815             17820

Thr Ser   Thr Val Xaa Xaa Gly   Thr Ser Gly Thr Pro   Ser Ser Xaa
    17825                 17830             17835

Pro Xaa   Xaa Thr Xaa Xaa Xaa   Pro Leu Leu Xaa Pro   Phe Thr Xaa
    17840                 17845             17850

Asn Xaa   Thr Ile Thr Asn Leu   Xaa Xaa Xaa Xaa Xaa   Met Xaa Xaa
    17855                 17860             17865

Pro Gly   Ser Arg Lys Phe Asn   Thr Thr Glu Xaa Val   Leu Gln Gly
    17870                 17875             17880

Leu Leu   Xaa Pro Xaa Phe Lys   Asn Xaa Ser Val Gly   Xaa Leu Tyr
    17885                 17890             17895

Ser Gly   Cys Arg Leu Thr Xaa   Leu Arg Xaa Glu Lys   Xaa Gly Ala
    17900                 17905             17910

Ala Thr   Gly Xaa Asp Ala Ile   Cys Xaa His Xaa Xaa   Xaa Pro Lys
    17915                 17920             17925

Xaa Pro   Gly Leu Xaa Xaa Glu   Xaa Leu Tyr Trp Glu   Leu Ser Xaa
    17930                 17935             17940

Leu Thr   Xaa Xaa Ile Xaa Glu   Leu Gly Pro Tyr Thr   Leu Asp Arg
    17945                 17950             17955

Xaa Ser   Leu Tyr Val Asn Gly   Phe His Pro Arg Ser   Ser Val Pro
    17960                 17965             17970

Thr Thr   Ser Thr Pro Gly Thr   Ser Thr Val His Leu   Ala Thr Ser
    17975                 17980             17985

Gly Thr   Pro Ser Ser Leu Pro   Gly His Thr Ala Pro   Val Pro Leu
    17990                 17995             18000

Leu Ile   Pro Phe Thr Leu Asn   Phe Thr Ile Thr Asn   Leu His Tyr

363

18005                          18010                          18015

Glu Glu   Asn Met Gln His Pro   Gly Ser Arg Lys Phe   Asn Thr Thr
    18020                 18025                 18030

Glu Arg   Val Leu Gln Gly Leu   Leu Gly Pro Met Phe   Lys Asn Thr
    18035                 18040                 18045

Ser Val   Gly Leu Leu Tyr Ser   Gly Cys Arg Leu Thr   Leu Leu Arg
    18050                 18055                 18060

Pro Glu   Lys Asn Gly Ala Ala   Thr Gly Met Asp Ala   Ile Cys Ser
    18065                 18070                 18075

His Arg   Leu Asp Pro Lys Ser   Pro Gly Leu Xaa Xaa   Glu Xaa Leu
    18080                 18085                 18090

Tyr Trp   Glu Leu Ser Xaa Leu   Thr Xaa Xaa Ile Xaa   Glu Leu Gly
    18095                 18100                 18105

Pro Tyr   Thr Leu Asp Arg Xaa   Ser Leu Tyr Val Asn   Gly Phe Thr
    18110                 18115                 18120

His Xaa   Xaa Ser Xaa Pro Thr   Thr Ser Thr Pro Gly   Thr Ser Thr
    18125                 18130                 18135

Val Xaa   Xaa Gly Thr Ser Gly   Thr Pro Ser Ser Xaa   Pro Xaa Xaa
    18140                 18145                 18150

Thr Xaa   Xaa Xaa Pro Leu Leu   Xaa Pro Phe Thr Xaa   Asn Xaa Thr
    18155                 18160                 18165

Ile Thr   Asn Leu Xaa Xaa Xaa   Xaa Xaa Met Xaa Xaa   Pro Gly Ser
    18170                 18175                 18180

Arg Lys   Phe Asn Thr Thr Glu   Xaa Val Leu Gln Gly   Leu Leu Xaa
    18185                 18190                 18195

Pro Xaa   Phe Lys Asn Xaa Ser   Val Gly Xaa Leu Tyr   Ser Gly Cys
    18200                 18205                 18210

Arg Leu   Thr Xaa Leu Arg Xaa   Glu Lys Xaa Gly Ala   Ala Thr Gly
    18215                 18220                 18225

Xaa Asp   Ala Ile Cys Xaa His   Xaa Xaa Xaa Pro Lys   Xaa Pro Gly
    18230                 18235                 18240

Leu Xaa  Xaa Glu Xaa Leu Tyr  Trp Glu Leu Ser Xaa  Leu Thr Xaa
    18245                18250            18255

Xaa Ile  Xaa Glu Leu Gly Pro  Tyr Thr Leu Asp Arg  Xaa Ser Leu
    18260                18265            18270

Tyr Val  Asn Gly Phe Thr His  Gln Asn Ser Val Pro  Thr Thr Ser
    18275                18280            18285

Thr Pro  Gly Thr Ser Thr Val  Tyr Trp Ala Thr Thr  Gly Thr Pro
    18290                18295            18300

Ser Ser  Phe Pro Gly His Thr  Glu Pro Gly Pro Leu  Leu Ile Pro
    18305                18310            18315

Phe Thr  Phe Asn Phe Thr Ile  Thr Asn Leu His Tyr  Glu Glu Asn
    18320                18325            18330

Met Gln  His Pro Gly Ser Arg  Lys Phe Asn Thr Thr  Glu Arg Val
    18335                18340            18345

Leu Gln  Gly Leu Leu Thr Pro  Leu Phe Lys Asn Thr  Ser Val Gly
    18350                18355            18360

Pro Leu  Tyr Ser Gly Cys Arg  Leu Thr Leu Leu Arg  Pro Glu Lys
    18365                18370            18375

Gln Glu  Ala Ala Thr Gly Val  Asp Thr Ile Cys Thr  His Arg Val
    18380                18385            18390

Asp Pro  Ile Gly Pro Gly Leu  Xaa Xaa Glu Xaa Leu  Tyr Trp Glu
    18395                18400            18405

Leu Ser  Xaa Leu Thr Xaa Xaa  Ile Xaa Glu Leu Gly  Pro Tyr Thr
    18410                18415            18420

Leu Asp  Arg Xaa Ser Leu Tyr  Val Asn Gly Phe Thr  His Xaa Xaa
    18425                18430            18435

Ser Xaa  Pro Thr Thr Ser Thr  Pro Gly Thr Ser Thr  Val Xaa Xaa
    18440                18445            18450

Gly Thr  Ser Gly Thr Pro Ser  Ser Xaa Pro Xaa Xaa  Thr Xaa Xaa
    18455                18460            18465

Xaa Pro  Leu Leu Xaa Pro Phe  Thr Xaa Asn Xaa Thr  Ile Thr Asn
    18470                18475            18480

```
Leu Xaa   Xaa Xaa Xaa Xaa Met   Xaa Xaa Pro Gly Ser   Arg Lys Phe
    18485               18490               18495

Asn Thr   Thr Glu Xaa Val Leu   Gln Gly Leu Leu Xaa   Pro Xaa Phe
    18500               18505               18510

Lys Asn   Xaa Ser Val Gly Xaa   Leu Tyr Ser Gly Cys   Arg Leu Thr
    18515               18520               18525

Xaa Leu   Arg Xaa Glu Lys Xaa   Gly Ala Ala Thr Gly   Xaa Asp Ala
    18530               18535               18540

Ile Cys   Xaa His Xaa Xaa Xaa   Pro Lys Xaa Pro Gly   Leu Xaa Xaa
    18545               18550               18555

Glu Xaa   Leu Tyr Trp Glu Leu   Ser Xaa Leu Thr Xaa   Xaa Ile Xaa
    18560               18565               18570

Glu Leu   Gly Pro Tyr Thr Leu   Asp Arg Xaa Ser Leu   Tyr Val Asn
    18575               18580               18585

Gly Phe   Thr His Arg Ser Ser   Val Pro Thr Thr Ser   Ser Pro Gly
    18590               18595               18600

Thr Ser   Thr Val His Leu Ala   Thr Ser Gly Thr Pro   Ser Ser Leu
    18605               18610               18615

Pro Gly   His Thr Ala Pro Val   Pro Leu Leu Ile Pro   Phe Thr Leu
    18620               18625               18630

Asn Phe   Thr Ile Thr Asn Leu   His Tyr Glu Glu Asn   Met Gln His
    18635               18640               18645

Pro Gly   Ser Arg Lys Phe Asn   Thr Thr Glu Arg Val   Leu Gln Gly
    18650               18655               18660

Leu Leu   Lys Pro Leu Phe Lys   Ser Thr Ser Val Gly   Pro Leu Tyr
    18665               18670               18675

Ser Gly   Cys Arg Leu Thr Leu   Leu Arg Pro Glu Lys   His Gly Ala
    18680               18685               18690

Ala Thr   Gly Val Asp Ala Ile   Cys Thr Leu Arg Leu   Asp Pro Thr
    18695               18700               18705

Gly Pro   Gly Leu Xaa Xaa Glu   Xaa Leu Tyr Trp Glu   Leu Ser Xaa
    18710               18715               18720
```

366

```
Leu Thr   Xaa Xaa Ile Xaa Glu   Leu Gly Pro Tyr Thr   Leu Asp Arg
    18725                 18730              18735

Xaa Ser   Leu Tyr Val Asn Gly   Phe Thr His Xaa Xaa   Ser Xaa Pro
    18740                 18745              18750

Thr Thr   Ser Thr Pro Gly Thr   Ser Thr Val Xaa Xaa   Gly Thr Ser
    18755                 18760              18765

Gly Thr   Pro Ser Ser Xaa Pro   Xaa Xaa Thr Xaa Xaa   Xaa Pro Leu
    18770                 18775              18780

Leu Xaa   Pro Phe Thr Xaa Asn   Xaa Thr Ile Thr Asn   Leu Xaa Xaa
    18785                 18790              18795

Xaa Xaa   Xaa Met Xaa Xaa Pro   Gly Ser Arg Lys Phe   Asn Thr Thr
    18800                 18805              18810

Glu Xaa   Val Leu Gln Gly Leu   Leu Xaa Pro Xaa Phe   Lys Asn Xaa
    18815                 18820              18825

Ser Val   Gly Xaa Leu Tyr Ser   Gly Cys Arg Leu Thr   Xaa Leu Arg
    18830                 18835              18840

Xaa Glu   Lys Xaa Gly Ala Ala   Thr Gly Xaa Asp Ala   Ile Cys Xaa
    18845                 18850              18855

His Xaa   Xaa Xaa Pro Lys Xaa   Pro Gly Leu Xaa Xaa   Glu Xaa Leu
    18860                 18865              18870

Tyr Trp   Glu Leu Ser Xaa Leu   Thr Xaa Xaa Ile Xaa   Glu Leu Gly
    18875                 18880              18885

Pro Tyr   Thr Leu Asp Arg Xaa   Ser Leu Tyr Val Asn   Gly Phe Thr
    18890                 18895              18900

His Arg   Thr Ser Val Pro Thr   Thr Ser Thr Pro Gly   Thr Ser Thr
    18905                 18910              18915

Val His   Leu Ala Thr Ser Gly   Thr Pro Ser Ser Leu   Pro Gly His
    18920                 18925              18930

Thr Ala   Pro Val Pro Leu Leu   Ile Pro Phe Thr Leu   Asn Phe Thr
    18935                 18940              18945

Ile Thr   Asn Leu Gln Tyr Glu   Glu Asp Met His Arg   Pro Gly Ser
```

```
              18950                    18955                    18960


Arg Lys    Phe Asn Thr Thr Glu    Arg Val Leu Gln Gly    Leu Leu Ser
    18965                    18970                    18975


Pro Ile    Phe Lys Asn Ser Ser    Val Gly Pro Leu Tyr    Ser Gly Cys
    18980                    18985                    18990


Arg Leu    Thr Ser Leu Arg Pro    Glu Lys Asp Gly Ala    Ala Thr Gly
    18995                    19000                    19005


Met Asp    Ala Val Cys Leu Tyr    His Pro Asn Pro Lys    Arg Pro Gly
    19010                    19015                    19020


Leu Asp    Arg Glu Gln Leu Tyr    Cys Glu Leu Ser Gln    Leu Thr His
    19025                    19030                    19035


Asn Ile    Thr Glu Leu Gly Pro    Tyr Ser Leu Asp Arg    Asp Ser Leu
    19040                    19045                    19050


Tyr Val    Asn Gly Phe Thr His    Gln Asn Ser Val Pro    Thr Thr Ser
    19055                    19060                    19065


Thr Pro    Gly Thr Ser Thr Val    Tyr Trp Ala Thr Thr    Gly Thr Pro
    19070                    19075                    19080


Ser Ser    Phe Pro Gly His Thr    Xaa Xaa Xaa Pro Leu    Leu Xaa Pro
    19085                    19090                    19095


Phe Thr    Xaa Asn Xaa Thr Ile    Thr Asn Leu Xaa Xaa    Xaa Xaa Xaa
    19100                    19105                    19110


Met Xaa    Xaa Pro Gly Ser Arg    Lys Phe Asn Thr Thr    Glu Xaa Val
    19115                    19120                    19125


Leu Gln    Gly Leu Leu Xaa Pro    Xaa Phe Lys Asn Xaa    Ser Val Gly
    19130                    19135                    19140


Xaa Leu    Tyr Ser Gly Cys Arg    Leu Thr Xaa Leu Arg    Xaa Glu Lys
    19145                    19150                    19155


Xaa Gly    Ala Ala Thr Gly Xaa    Asp Ala Ile Cys Xaa    His Xaa Xaa
    19160                    19165                    19170


Xaa Pro    Lys Xaa Pro Gly Leu    Xaa Xaa Glu Xaa Leu    Tyr Trp Glu
    19175                    19180                    19185
```

```
Leu Ser Xaa Leu Thr Xaa Xaa    Ile Xaa Glu Leu Gly    Pro Tyr Thr
    19190           19195               19200


Leu Asp Arg Xaa Ser Leu Tyr    Val Asn Gly Phe Thr    His Trp Ser
    19205           19210               19215


Ser Gly Leu Thr Thr Ser Thr    Pro Trp Thr Ser Thr    Val Asp Leu
    19220           19225               19230


Gly Thr Ser Gly Thr Pro Ser    Pro Val Pro Ser Pro    Thr Thr Ala
    19235           19240               19245


Gly Pro Leu Leu Val Pro Phe    Thr Leu Asn Phe Thr    Ile Thr Asn
    19250           19255               19260


Leu Gln Tyr Glu Glu Asp Met    His Arg Pro Gly Ser    Arg Lys Phe
    19265           19270               19275


Asn Ala Thr Glu Arg Val Leu    Gln Gly Leu Leu Ser    Pro Ile Phe
    19280           19285               19290


Lys Asn Thr Ser Val Gly Pro    Leu Tyr Ser Gly Cys    Arg Leu Thr
    19295           19300               19305


Leu Leu Arg Pro Glu Lys Gln    Glu Ala Ala Thr Gly    Val Asp Thr
    19310           19315               19320


Ile Cys Thr His Arg Val Asp    Pro Ile Gly Pro Gly    Leu Xaa Xaa
    19325           19330               19335


Glu Xaa Leu Tyr Trp Glu Leu    Ser Xaa Leu Thr Xaa    Xaa Ile Xaa
    19340           19345               19350


Glu Leu Gly Pro Tyr Thr Leu    Asp Arg Xaa Ser Leu    Tyr Val Asn
    19355           19360               19365


Gly Phe Thr His Xaa Xaa Ser    Xaa Pro Thr Thr Ser    Thr Pro Gly
    19370           19375               19380


Thr Ser Thr Val Xaa Xaa Gly    Thr Ser Gly Thr Pro    Ser Ser Xaa
    19385           19390               19395


Pro Xaa Xaa Thr Xaa Xaa Xaa    Pro Leu Leu Xaa Pro    Phe Thr Xaa
    19400           19405               19410


Asn Xaa Thr Ile Thr Asn Leu    Xaa Xaa Xaa Xaa Xaa    Met Xaa Xaa
    19415           19420               19425
```

```
Pro Gly  Ser Arg Lys Phe Asn   Thr Thr Glu Xaa Val   Leu Gln Gly
    19430                19435              19440

Leu Leu  Xaa Pro Xaa Phe Lys   Asn Xaa Ser Val Gly   Xaa Leu Tyr
    19445                19450              19455

Ser Gly  Cys Arg Leu Thr Xaa   Leu Arg Xaa Glu Lys   Xaa Gly Ala
    19460                19465              19470

Ala Thr  Gly Xaa Asp Ala Ile   Cys Xaa His Xaa Xaa   Xaa Pro Lys
    19475                19480              19485

Xaa Pro  Gly Leu Xaa Xaa Glu   Xaa Leu Tyr Trp Glu   Leu Ser Xaa
    19490                19495              19500

Leu Thr  Xaa Xaa Ile Xaa Glu   Leu Gly Pro Tyr Thr   Leu Asp Arg
    19505                19510              19515

Xaa Ser  Leu Tyr Val Asn Gly   Phe Thr His Arg Ser   Phe Gly Leu
    19520                19525              19530

Thr Thr  Ser Thr Pro Trp Thr   Ser Thr Val Asp Leu   Gly Thr Ser
    19535                19540              19545

Gly Thr  Pro Ser Pro Val Pro   Ser Pro Thr Thr Ala   Gly Pro Leu
    19550                19555              19560

Leu Val  Pro Phe Thr Leu Asn   Phe Thr Ile Thr Asn   Leu Gln Tyr
    19565                19570              19575

Glu Glu  Asp Met His Arg Pro   Gly Ser Arg Lys Phe   Asn Thr Thr
    19580                19585              19590

Glu Arg  Val Leu Gln Gly Leu   Leu Thr Pro Leu Phe   Arg Asn Thr
    19595                19600              19605

Ser Val  Ser Ser Leu Tyr Ser   Gly Cys Arg Leu Thr   Leu Leu Arg
    19610                19615              19620

Pro Glu  Lys Asp Gly Ala Ala   Thr Arg Val Asp Ala   Val Cys Thr
    19625                19630              19635

His Arg  Pro Asp Pro Lys Ser   Pro Gly Leu Xaa Xaa   Glu Xaa Leu
    19640                19645              19650

Tyr Trp  Glu Leu Ser Xaa Leu   Thr Xaa Xaa Ile Xaa   Glu Leu Gly
    19655                19660              19665
```

EP 2 894 473 B1

Pro Tyr     Thr Leu Asp Arg Xaa     Ser Leu Tyr Val Asn     Gly Phe Thr
    19670                   19675                   19680

His Xaa     Xaa Ser Xaa Pro Thr     Thr Ser Thr Pro Gly     Thr Ser Thr
    19685                   19690                   19695

Val Xaa     Xaa Gly Thr Ser Gly     Thr Pro Ser Ser Xaa     Pro Xaa Xaa
    19700                   19705                   19710

Thr Xaa     Xaa Xaa Pro Leu Leu     Xaa Pro Phe Thr Xaa     Asn Xaa Thr
    19715                   19720                   19725

Ile Thr     Asn Leu Xaa Xaa Xaa     Xaa Xaa Met Xaa Xaa     Pro Gly Ser
    19730                   19735                   19740

Arg Lys     Phe Asn Thr Thr Glu     Xaa Val Leu Gln Gly     Leu Leu Xaa
    19745                   19750                   19755

Pro Xaa     Phe Lys Asn Xaa Ser     Val Gly Xaa Leu Tyr     Ser Gly Cys
    19760                   19765                   19770

Arg Leu     Thr Xaa Leu Arg Xaa     Glu Lys Xaa Gly Ala     Ala Thr Gly
    19775                   19780                   19785

Xaa Asp     Ala Ile Cys Xaa His     Xaa Xaa Xaa Pro Lys     Xaa Pro Gly
    19790                   19795                   19800

Leu Xaa     Xaa Glu Xaa Leu Tyr     Trp Glu Leu Ser Xaa     Leu Thr Xaa
    19805                   19810                   19815

Xaa Ile     Xaa Glu Leu Gly Pro     Tyr Thr Leu Asp Arg     Xaa Ser Leu
    19820                   19825                   19830

Tyr Val     Asn Gly Phe Thr His     Trp Ile Pro Val Pro     Thr Ser Ser
    19835                   19840                   19845

Thr Pro     Gly Thr Ser Thr Val     Asp Leu Gly Ser Gly     Thr Pro Ser
    19850                   19855                   19860

Ser Leu     Pro Ser Pro Thr Thr     Ala Gly Pro Leu Leu     Val Pro Phe
    19865                   19870                   19875

Thr Leu     Asn Phe Thr Ile Thr     Asn Leu Gln Tyr Gly     Glu Asp Met
    19880                   19885                   19890

Gly His     Pro Gly Ser Arg Lys     Phe Asn Thr Thr Glu     Arg Val Leu

371

```
        19895                 19900                 19905

Gln Gly   Leu Leu Gly Pro Ile   Phe Lys Asn Thr Ser   Val Gly Pro
    19910                 19915                 19920

Leu Tyr   Ser Gly Cys Arg Leu   Thr Ser Leu Arg Ser   Glu Lys Asp
    19925                 19930                 19935

Gly Ala   Ala Thr Gly Val Asp   Ala Ile Cys Ile His   His Leu Asp
    19940                 19945                 19950

Pro Lys   Ser Pro Gly Leu Xaa   Xaa Glu Xaa Leu Tyr   Trp Glu Leu
    19955                 19960                 19965

Ser Xaa   Leu Thr Xaa Xaa Ile   Xaa Glu Leu Gly Pro   Tyr Thr Leu
    19970                 19975                 19980

Asp Arg   Xaa Ser Leu Tyr Val   Asn Gly Phe Thr His   Xaa Xaa Ser
    19985                 19990                 19995

Xaa Pro   Thr Thr Ser Thr Pro   Gly Thr Ser Thr Val   Xaa Xaa Gly
    20000                 20005                 20010

Thr Ser   Gly Thr Pro Ser Ser   Xaa Pro Xaa Xaa Thr   Xaa Xaa Xaa
    20015                 20020                 20025

Pro Leu   Leu Xaa Pro Phe Thr   Xaa Asn Xaa Thr Ile   Thr Asn Leu
    20030                 20035                 20040

Xaa Xaa   Xaa Xaa Xaa Met Xaa   Xaa Pro Gly Ser Arg   Lys Phe Asn
    20045                 20050                 20055

Thr Thr   Glu Xaa Val Leu Gln   Gly Leu Leu Xaa Pro   Xaa Phe Lys
    20060                 20065                 20070

Asn Xaa   Ser Val Gly Xaa Leu   Tyr Ser Gly Cys Arg   Leu Thr Xaa
    20075                 20080                 20085

Leu Arg   Xaa Glu Lys Xaa Gly   Ala Ala Thr Gly Xaa   Asp Ala Ile
    20090                 20095                 20100

Cys Xaa   His Xaa Xaa Xaa Pro   Lys Xaa Pro Gly Leu   Xaa Xaa Glu
    20105                 20110                 20115

Xaa Leu   Tyr Trp Glu Leu Ser   Xaa Leu Thr Xaa Xaa   Ile Xaa Glu
    20120                 20125                 20130
```

Leu Gly  Pro Tyr Thr Leu Asp  Arg Xaa Ser Leu Tyr  Val Asn Gly
    20135                20140               20145

Phe Thr  His Gln Thr Phe Ala  Pro Asn Thr Ser Thr  Pro Gly Thr
    20150                20155               20160

Ser Thr  Val Asp Leu Gly Thr  Ser Gly Thr Pro Ser  Ser Leu Pro
    20165                20170               20175

Ser Pro  Thr Ser Ala Gly Pro  Leu Leu Val Pro Phe  Thr Leu Asn
    20180                20185               20190

Phe Thr  Ile Thr Asn Leu Gln  Tyr Glu Glu Asp Met  His His Pro
    20195                20200               20205

Gly Ser  Arg Lys Phe Asn Thr  Thr Glu Arg Val Leu  Gln Gly Leu
    20210                20215               20220

Leu Gly  Pro Met Phe Lys Asn  Thr Ser Val Gly Leu  Leu Tyr Ser
    20225                20230               20235

Gly Cys  Arg Leu Thr Leu Leu  Arg Pro Glu Lys Asn  Gly Ala Ala
    20240                20245               20250

Thr Arg  Val Asp Ala Val Cys  Thr His Arg Pro Asp  Pro Lys Ser
    20255                20260               20265

Pro Gly  Leu Xaa Xaa Glu Xaa  Leu Tyr Trp Glu Leu  Ser Xaa Leu
    20270                20275               20280

Thr Xaa  Xaa Ile Xaa Glu Leu  Gly Pro Tyr Thr Leu  Asp Arg Xaa
    20285                20290               20295

Ser Leu  Tyr Val Asn Gly Phe  Thr His Xaa Xaa Ser  Xaa Pro Thr
    20300                20305               20310

Thr Ser  Thr Pro Gly Thr Ser  Thr Val Xaa Xaa Gly  Thr Ser Gly
    20315                20320               20325

Thr Pro  Ser Ser Xaa Pro Xaa  Xaa Thr Ala Pro Val  Pro Leu Leu
    20330                20335               20340

Ile Pro  Phe Thr Leu Asn Phe  Thr Ile Thr Asn Leu  His Tyr Glu
    20345                20350               20355

Glu Asn  Met Gln His Pro Gly  Ser Arg Lys Phe Asn  Thr Thr Glu
    20360                20365               20370

Arg Val Leu Gln Gly Leu Leu Lys Pro Leu Phe Lys Ser Thr Ser
    20375             20380             20385

Val Gly Pro Leu Tyr Ser Gly Cys Arg Leu Thr Leu Leu Arg Pro
    20390             20395             20400

Glu Lys His Gly Ala Ala Thr Gly Val Asp Ala Ile Cys Thr Leu
    20405             20410             20415

Arg Leu Asp Pro Thr Gly Pro Gly Leu Asp Arg Glu Arg Leu Tyr
    20420             20425             20430

Trp Glu Leu Ser Gln Leu Thr Asn Ser Val Thr Glu Leu Gly Pro
    20435             20440             20445

Tyr Thr Leu Asp Arg Asp Ser Leu Tyr Val Asn Gly Phe Thr Gln
    20450             20455             20460

Arg Ser Ser Val Pro Thr Thr Ser Ile Pro Gly Thr Ser Ala Val
    20465             20470             20475

His Leu Glu Thr Ser Gly Thr Pro Ala Ser Leu Pro Gly His Thr
    20480             20485             20490

Ala Pro Gly Pro Leu Leu Val Pro Phe Thr Leu Asn Phe Thr Ile
    20495             20500             20505

Thr Asn Leu Gln Tyr Glu Val Asp Met Arg His Pro Gly Ser Arg
    20510             20515             20520

Lys Phe Asn Thr Thr Glu Arg Val Leu Gln Gly Leu Leu Lys Pro
    20525             20530             20535

Leu Phe Lys Ser Thr Ser Val Gly Pro Leu Tyr Ser Gly Cys Arg
    20540             20545             20550

Leu Thr Leu Leu Arg Pro Glu Lys Arg Gly Ala Ala Thr Gly Val
    20555             20560             20565

Asp Thr Ile Cys Thr His Arg Leu Asp Pro Leu Asn Pro Gly Leu
    20570             20575             20580

Asp Arg Glu Gln Leu Tyr Trp Glu Leu Ser Lys Leu Thr Arg Gly
    20585             20590             20595

Ile Ile Glu Leu Gly Pro Tyr Leu Leu Asp Arg Gly Ser Leu Tyr
    20600             20605             20610

374

```
Val Asn    Gly Phe Thr His Arg    Asn Phe Val Pro Ile    Thr Ser Thr
    20615                20620            20625

Pro Gly    Thr Ser Thr Val His    Leu Gly Thr Ser Glu    Thr Pro Ser
    20630                20635            20640

Ser Leu    Pro Arg Pro Ile Val    Pro Gly Pro Leu Leu    Val Pro Phe
    20645                20650            20655

Thr Leu    Asn Phe Thr Ile Thr    Asn Leu Gln Tyr Glu    Glu Ala Met
    20660                20665            20670

Arg His    Pro Gly Ser Arg Lys    Phe Asn Thr Thr Glu    Arg Val Leu
    20675                20680            20685

Gln Gly    Leu Leu Arg Pro Leu    Phe Lys Asn Thr Ser    Ile Gly Pro
    20690                20695            20700

Leu Tyr    Ser Ser Cys Arg Leu    Thr Leu Leu Arg Pro    Glu Lys Asp
    20705                20710            20715

Lys Ala    Ala Thr Arg Val Asp    Ala Ile Cys Thr His    His Pro Asp
    20720                20725            20730

Pro Gln    Ser Pro Gly Leu Asn    Arg Glu Gln Leu Tyr    Trp Glu Leu
    20735                20740            20745

Ser Gln    Leu Thr His Gly Ile    Thr Glu Leu Gly Pro    Tyr Thr Leu
    20750                20755            20760

Asp Arg    Asp Ser Leu Tyr Val    Asp Gly Phe Thr His    Trp Ser Pro
    20765                20770            20775

Ile Pro    Thr Thr Ser Thr Pro    Gly Thr Ser Ile Val    Asn Leu Gly
    20780                20785            20790

Thr Ser    Gly Ile Pro Pro Ser    Leu Pro Glu Thr Thr    Xaa Xaa Xaa
    20795                20800            20805

Pro Leu    Leu Xaa Pro Phe Thr    Xaa Asn Xaa Thr Ile    Thr Asn Leu
    20810                20815            20820

Xaa Xaa    Xaa Xaa Xaa Met Xaa    Xaa Pro Gly Ser Arg    Lys Phe Asn
    20825                20830            20835

Thr Thr    Glu Arg Val Leu Gln    Gly Leu Leu Lys Pro    Leu Phe Lys
```

20840                    20845                    20850


Ser Thr   Ser Val Gly Pro Leu   Tyr Ser Gly Cys Arg   Leu Thr Leu
    20855                 20860                 20865


Leu Arg   Pro Glu Lys Asp Gly   Val Ala Thr Arg Val   Asp Ala Ile
    20870                 20875                 20880


Cys Thr   His Arg Pro Asp Pro   Lys Ile Pro Gly Leu   Asp Arg Gln
    20885                 20890                 20895


Gln Leu   Tyr Trp Glu Leu Ser   Gln Leu Thr His Ser   Ile Thr Glu
    20900                 20905                 20910


Leu Gly   Pro Tyr Thr Leu Asp   Arg Asp Ser Leu Tyr   Val Asn Gly
    20915                 20920                 20925


Phe Thr   Gln Arg Ser Ser Val   Pro Thr Thr Ser Thr   Pro Gly Thr
    20930                 20935                 20940


Phe Thr   Val Gln Pro Glu Thr   Ser Glu Thr Pro Ser   Ser Leu Pro
    20945                 20950                 20955


Gly Pro   Thr Ala Thr Gly Pro   Val Leu Leu Pro Phe   Thr Leu Asn
    20960                 20965                 20970


Phe Thr   Ile Thr Asn Leu Gln   Tyr Glu Glu Asp Met   His Arg Pro
    20975                 20980                 20985


Gly Ser   Arg Lys Phe Asn Thr   Thr Glu Arg Val Leu   Gln Gly Leu
    20990                 20995                 21000


Leu Met   Pro Leu Phe Lys Asn   Thr Ser Val Ser Ser   Leu Tyr Ser
    21005                 21010                 21015


Gly Cys   Arg Leu Thr Leu Leu   Arg Pro Glu Lys Asp   Gly Ala Ala
    21020                 21025                 21030


Thr Arg   Val Asp Ala Val Cys   Thr His Arg Pro Asp   Pro Lys Ser
    21035                 21040                 21045


Pro Gly   Leu Asp Arg Glu Arg   Leu Tyr Trp Lys Leu   Ser Gln Leu
    21050                 21055                 21060


Thr His   Gly Ile Thr Glu Leu   Gly Pro Tyr Thr Leu   Asp Arg His
    21065                 21070                 21075

```
Ser Leu    Tyr Val Asn Gly Phe    Thr His Gln Ser Ser    Met Thr Thr
    21080                21085                21090

Thr Arg    Thr Pro Asp Thr Ser    Thr Met His Leu Ala    Thr Ser Arg
    21095                21100                21105

Thr Pro    Ala Ser Leu Ser Gly    Pro Thr Thr Ala Ser    Pro Leu Leu
    21110                21115                21120

Val Leu    Phe Thr Ile Asn Phe    Thr Ile Thr Asn Leu    Arg Tyr Glu
    21125                21130                21135

Glu Asn    Met His His Pro Gly    Ser Arg Lys Phe Asn    Thr Thr Glu
    21140                21145                21150

Arg Val    Leu Gln Gly Leu Leu    Arg Pro Val Phe Lys    Asn Thr Ser
    21155                21160                21165

Val Gly    Pro Leu Tyr Ser Gly    Cys Arg Leu Thr Leu    Leu Arg Pro
    21170                21175                21180

Lys Lys    Asp Gly Ala Ala Thr    Lys Val Asp Ala Ile    Cys Thr Tyr
    21185                21190                21195

Arg Pro    Asp Pro Lys Ser Pro    Gly Leu Asp Arg Glu    Gln Leu Tyr
    21200                21205                21210

Trp Glu    Leu Ser Gln Leu Thr    His Ser Ile Thr Glu    Leu Gly Pro
    21215                21220                21225

Tyr Thr    Leu Asp Arg Asp Ser    Leu Tyr Val Asn Gly    Phe Thr Gln
    21230                21235                21240

Arg Ser    Ser Val Pro Thr Thr    Ser Ile Pro Gly Thr    Pro Thr Val
    21245                21250                21255

Asp Leu    Gly Thr Ser Gly Thr    Pro Val Ser Lys Pro    Gly Pro Ser
    21260                21265                21270

Ala Ala    Ser Pro Leu Leu Val    Leu Phe Thr Leu Asn    Phe Thr Ile
    21275                21280                21285

Thr Asn    Leu Arg Tyr Glu Glu    Asn Met Gln His Pro    Gly Ser Arg
    21290                21295                21300

Lys Phe    Asn Thr Thr Glu Arg    Val Leu Gln Gly Leu    Leu Arg Ser
    21305                21310                21315
```

377

```
Leu Phe  Lys Ser Thr Ser Val  Gly Pro Leu Tyr Ser  Gly Cys Arg
    21320            21325            21330

Leu Thr  Leu Leu Arg Pro Glu  Lys Asp Gly Thr Ala  Thr Gly Val
    21335            21340            21345

Asp Ala  Ile Cys Thr His His  Pro Asp Pro Lys Ser  Pro Arg Leu
    21350            21355            21360

Asp Arg  Glu Gln Leu Tyr Trp  Glu Leu Ser Gln Leu  Thr His Asn
    21365            21370            21375

Ile Thr  Glu Leu Gly His Tyr  Ala Leu Asp Asn Asp  Ser Leu Phe
    21380            21385            21390

Val Asn  Gly Phe Thr His Arg  Ser Ser Val Ser Thr  Thr Ser Thr
    21395            21400            21405

Pro Gly  Thr Pro Thr Val Tyr  Leu Gly Ala Ser Lys  Thr Pro Ala
    21410            21415            21420

Ser Ile  Phe Gly Pro Ser Ala  Ala Ser His Leu Leu  Ile Leu Phe
    21425            21430            21435

Thr Leu  Asn Phe Thr Ile Thr  Asn Leu Arg Tyr Glu  Glu Asn Met
    21440            21445            21450

Trp Pro  Gly Ser Arg Lys Phe  Asn Thr Thr Glu Arg  Val Leu Gln
    21455            21460            21465

Gly Leu  Leu Arg Pro Leu Phe  Lys Asn Thr Ser Val  Gly Pro Leu
    21470            21475            21480

Tyr Ser  Gly Ser Arg Leu Thr  Leu Leu Arg Pro Glu  Lys Asp Gly
    21485            21490            21495

Glu Ala  Thr Gly Val Asp Ala  Ile Cys Thr His Arg  Pro Asp Pro
    21500            21505            21510

Thr Gly  Pro Gly Leu Asp Arg  Glu Gln Leu Tyr Leu  Glu Leu Ser
    21515            21520            21525

Gln Leu  Thr His Ser Ile Thr  Glu Leu Gly Pro Tyr  Thr Leu Asp
    21530            21535            21540

Arg Asp  Ser Leu Tyr Val Asn  Gly Phe Thr His Arg  Ser Ser Val
    21545            21550            21555
```

```
Pro Thr    Thr Ser Thr Gly Val    Val Ser Glu Glu Pro    Phe Thr Leu
    21560                  21565             21570

Asn Phe    Thr Ile Asn Asn Leu    Arg Tyr Met Ala Asp    Met Gly Gln
    21575                  21580             21585

Pro Gly    Ser Leu Lys Phe Asn    Ile Thr Asp Asn Val    Met Lys His
    21590                  21595             21600

Leu Leu    Ser Pro Leu Phe Gln    Arg Ser Ser Leu Gly    Ala Arg Tyr
    21605                  21610             21615

Thr Gly    Cys Arg Val Ile Ala    Leu Arg Ser Val Lys    Asn Gly Ala
    21620                  21625             21630

Glu Thr    Arg Val Asp Leu Leu    Cys Thr Tyr Leu Gln    Pro Leu Ser
    21635                  21640             21645

Gly Pro    Gly Leu Pro Ile Lys    Gln Val Phe His Glu    Leu Ser Gln
    21650                  21655             21660

Gln Thr    His Gly Ile Thr Arg    Leu Gly Pro Tyr Ser    Leu Asp Lys
    21665                  21670             21675

Asp Ser    Leu Tyr Leu Asn Gly    Tyr Asn Glu Pro Gly    Leu Asp Glu
    21680                  21685             21690

Pro Pro    Thr Thr Pro Lys Pro    Ala Thr Thr Phe Leu    Pro Pro Leu
    21695                  21700             21705

Ser Glu    Ala Thr Thr Ala Met    Gly Tyr His Leu Lys    Thr Leu Thr
    21710                  21715             21720

Leu Asn    Phe Thr Ile Ser Asn    Leu Gln Tyr Ser Pro    Asp Met Gly
    21725                  21730             21735

Lys Gly    Ser Ala Thr Phe Asn    Ser Thr Glu Gly Val    Leu Gln His
    21740                  21745             21750

Leu Leu    Arg Pro Leu Phe Gln    Lys Ser Ser Met Gly    Pro Phe Tyr
    21755                  21760             21765

Leu Gly    Cys Gln Leu Ile Ser    Leu Arg Pro Glu Lys    Asp Gly Ala
    21770                  21775             21780

Ala Thr    Gly Val Asp Thr Thr    Cys Thr Tyr His Pro    Asp Pro Val
```

```
        21785                      21790                      21795

Gly Pro Gly Leu Asp Ile Gln   Gln Leu Tyr Trp Glu   Leu Ser Gln
    21800                 21805                 21810

Leu Thr His Gly Val Thr Gln   Leu Gly Phe Tyr Val   Leu Asp Arg
    21815                 21820                 21825

Asp Ser Leu Phe Ile Asn Gly   Tyr Ala Pro Gln Asn   Leu Ser Ile
    21830                 21835                 21840

Arg Gly Glu Tyr Gln Ile Asn   Phe His Ile Val Asn   Trp Asn Leu
    21845                 21850                 21855

Ser Asn Pro Asp Pro Thr Ser   Ser Glu Tyr Ile Thr   Leu Leu Arg
    21860                 21865                 21870

Asp Ile Gln Asp Lys Val Thr   Thr Leu Tyr Lys Gly   Ser Gln Leu
    21875                 21880                 21885

His Asp Thr Phe Arg Phe Cys   Leu Val Thr Asn Leu   Thr Met Asp
    21890                 21895                 21900

Ser Val Leu Val Thr Val Lys   Ala Leu Phe Ser Ser   Asn Leu Asp
    21905                 21910                 21915

Pro Ser Leu Val Glu Gln Val   Phe Leu Asp Lys Thr   Leu Asn Ala
    21920                 21925                 21930

Ser Phe His Trp Leu Gly Ser   Thr Tyr Gln Leu Val   Asp Ile His
    21935                 21940                 21945

Val Thr Glu Met Glu Ser Ser   Val Tyr Gln Pro Thr   Ser Ser Ser
    21950                 21955                 21960

Ser Thr Gln His Phe Tyr Leu   Asn Phe Thr Ile Thr   Asn Leu Pro
    21965                 21970                 21975

Tyr Ser Gln Asp Lys Ala Gln   Pro Gly Thr Thr Asn   Tyr Gln Arg
    21980                 21985                 21990

Asn Lys Arg Asn Ile Glu Asp   Ala Leu Asn Gln Leu   Phe Arg Asn
    21995                 22000                 22005

Ser Ser Ile Lys Ser Tyr Phe   Ser Asp Cys Gln Val   Ser Thr Phe
    22010                 22015                 22020
```

```
Arg Ser   Val Pro Asn Arg His   His Thr Gly Val Asp   Ser Leu Cys
    22025             22030             22035

Asn Phe   Ser Pro Leu Ala Arg   Arg Val Asp Arg Val   Ala Ile Tyr
    22040             22045             22050

Glu Glu   Phe Leu Arg Met Thr   Arg Asn Gly Thr Gln   Leu Gln Asn
    22055             22060             22065

Phe Thr   Leu Asp Arg Ser Ser   Val Leu Val Asp Gly   Tyr Ser Pro
    22070             22075             22080

Asn Arg   Asn Glu Pro Leu Thr   Gly Asn Ser Asp Leu   Pro Phe Trp
    22085             22090             22095

Ala Val   Ile Leu Ile Gly Leu   Ala Gly Leu Leu Gly   Leu Ile Thr
    22100             22105             22110

Cys Leu   Ile Cys Gly Val Leu   Val Thr Thr Arg Arg   Arg Lys Lys
    22115             22120             22125

Glu Gly   Glu Tyr Asn Val Gln   Gln Gln Cys Pro Gly   Tyr Tyr Gln
    22130             22135             22140

Ser His   Leu Asp Leu Glu Asp   Leu Gln
    22145             22150
```

<210> 13
<211> 702
<212> PRT
<213> Homo sapiens

<400> 13

Met Glu Ser Pro Ser Ala Pro Pro His Arg Trp Cys Ile Pro Trp Gln
1               5                   10              15

Arg Leu Leu Leu Thr Ala Ser Leu Leu Thr Phe Trp Asn Pro Pro Thr
        20              25              30

Thr Ala Lys Leu Thr Ile Glu Ser Thr Pro Phe Asn Val Ala Glu Gly
        35              40              45

Lys Glu Val Leu Leu Leu Val His Asn Leu Pro Gln His Leu Phe Gly
    50              55              60

Tyr Ser Trp Tyr Lys Gly Glu Arg Val Asp Gly Asn Arg Gln Ile Ile
65              70              75              80

Gly Tyr Val Ile Gly Thr Gln Gln Ala Thr Pro Gly Pro Ala Tyr Ser

```
                    85                      90                      95


   Gly Arg Glu Ile Ile Tyr Pro Asn Ala Ser Leu Leu Ile Gln Asn Ile
               100                 105                 110


   Ile Gln Asn Asp Thr Gly Phe Tyr Thr Leu His Val Ile Lys Ser Asp
               115                 120                 125


   Leu Val Asn Glu Glu Ala Thr Gly Gln Phe Arg Val Tyr Pro Glu Leu
       130                 135                 140


   Pro Lys Pro Ser Ile Ser Ser Asn Asn Ser Lys Pro Val Glu Asp Lys
   145                 150                 155                 160


   Asp Ala Val Ala Phe Thr Cys Glu Pro Glu Thr Gln Asp Ala Thr Tyr
                   165                 170                 175


   Leu Trp Trp Val Asn Asn Gln Ser Leu Pro Val Ser Pro Arg Leu Gln
               180                 185                 190


   Leu Ser Asn Gly Asn Arg Thr Leu Thr Leu Phe Asn Val Thr Arg Asn
               195                 200                 205


   Asp Thr Ala Ser Tyr Lys Cys Glu Thr Gln Asn Pro Val Ser Ala Arg
       210                 215                 220


   Arg Ser Asp Ser Val Ile Leu Asn Val Leu Tyr Gly Pro Asp Ala Pro
   225                 230                 235                 240


   Thr Ile Ser Pro Leu Asn Thr Ser Tyr Arg Ser Gly Glu Asn Leu Asn
               245                 250                 255


   Leu Ser Cys His Ala Ala Ser Asn Pro Pro Ala Gln Tyr Ser Trp Phe
               260                 265                 270


   Val Asn Gly Thr Phe Gln Gln Ser Thr Gln Glu Leu Phe Ile Pro Asn
               275                 280                 285


   Ile Thr Val Asn Asn Ser Gly Ser Tyr Thr Cys Gln Ala His Asn Ser
       290                 295                 300


   Asp Thr Gly Leu Asn Arg Thr Thr Val Thr Thr Ile Thr Val Tyr Ala
   305                 310                 315                 320


   Glu Pro Pro Lys Pro Phe Ile Thr Ser Asn Asn Ser Asn Pro Val Glu
               325                 330                 335
```

```
Asp Glu Asp Ala Val Ala Leu Thr Cys Glu Pro Glu Ile Gln Asn Thr
            340                 345                 350

Thr Tyr Leu Trp Trp Val Asn Asn Gln Ser Leu Pro Val Ser Pro Arg
            355                 360                 365

Leu Gln Leu Ser Asn Asp Asn Arg Thr Leu Thr Leu Leu Ser Val Thr
    370                 375                 380

Arg Asn Asp Val Gly Pro Tyr Glu Cys Gly Ile Gln Asn Glu Leu Ser
385                 390                 395                 400

Val Asp His Ser Asp Pro Val Ile Leu Asn Val Leu Tyr Gly Pro Asp
            405                 410                 415

Asp Pro Thr Ile Ser Pro Ser Tyr Thr Tyr Tyr Arg Pro Gly Val Asn
            420                 425                 430

Leu Ser Leu Ser Cys His Ala Ala Ser Asn Pro Pro Ala Gln Tyr Ser
            435                 440                 445

Trp Leu Ile Asp Gly Asn Ile Gln Gln His Thr Gln Glu Leu Phe Ile
    450                 455                 460

Ser Asn Ile Thr Glu Lys Asn Ser Gly Leu Tyr Thr Cys Gln Ala Asn
465                 470                 475                 480

Asn Ser Ala Ser Gly His Ser Arg Thr Thr Val Lys Thr Ile Thr Val
            485                 490                 495

Ser Ala Glu Leu Pro Lys Pro Ser Ile Ser Ser Asn Asn Ser Lys Pro
            500                 505                 510

Val Glu Asp Lys Asp Ala Val Ala Phe Thr Cys Glu Pro Glu Ala Gln
    515                 520                 525

Asn Thr Thr Tyr Leu Trp Trp Val Asn Gly Gln Ser Leu Pro Val Ser
    530                 535                 540

Pro Arg Leu Gln Leu Ser Asn Gly Asn Arg Thr Leu Thr Leu Phe Asn
545                 550                 555                 560

Val Thr Arg Asn Asp Ala Arg Ala Tyr Val Cys Gly Ile Gln Asn Ser
            565                 570                 575

Val Ser Ala Asn Arg Ser Asp Pro Val Thr Leu Asp Val Leu Tyr Gly
            580                 585                 590
```

```
Pro Asp Thr Pro Ile Ile Ser Pro Pro Asp Ser Ser Tyr Leu Ser Gly
        595             600             605

Ala Asn Leu Asn Leu Ser Cys His Ser Ala Ser Asn Pro Ser Pro Gln
        610             615             620

Tyr Ser Trp Arg Ile Asn Gly Ile Pro Gln Gln His Thr Gln Val Leu
625             630             635             640

Phe Ile Ala Lys Ile Thr Pro Asn Asn Asn Gly Thr Tyr Ala Cys Phe
        645             650             655

Val Ser Asn Leu Ala Thr Gly Arg Asn Asn Ser Ile Val Lys Ser Ile
        660             665             670

Thr Val Ser Ala Ser Gly Thr Ser Pro Gly Leu Ser Ala Gly Ala Thr
        675             680             685

Val Gly Ile Met Ile Gly Val Leu Val Gly Val Ala Leu Ile
        690             695             700
```

<210> 14
<211> 393
<212> PRT
<213> Homo sapiens

<400> 14

```
Met Glu Glu Pro Gln Ser Asp Pro Ser Val Glu Pro Pro Leu Ser Gln
1               5               10              15

Glu Thr Phe Ser Asp Leu Trp Lys Leu Leu Pro Glu Asn Asn Val Leu
        20              25              30

Ser Pro Leu Pro Ser Gln Ala Met Asp Asp Leu Met Leu Ser Pro Asp
        35              40              45

Asp Ile Glu Gln Trp Phe Thr Glu Asp Pro Gly Pro Asp Glu Ala Pro
        50              55              60

Arg Met Pro Glu Ala Ala Pro Arg Val Ala Pro Ala Pro Ala Ala Pro
65              70              75              80

Thr Pro Ala Ala Pro Ala Pro Ala Pro Ser Trp Pro Leu Ser Ser Ser
        85              90              95

Val Pro Ser Gln Lys Thr Tyr Gln Gly Ser Tyr Gly Phe Arg Leu Gly
        100             105             110
```

```
Phe Leu His Ser Gly Thr Ala Lys Ser Val Thr Cys Thr Tyr Ser Pro
        115                 120             125

Ala Leu Asn Lys Met Phe Cys Gln Leu Ala Lys Thr Cys Pro Val Gln
        130                 135             140

Leu Trp Val Asp Ser Thr Pro Pro Gly Thr Arg Val Arg Ala Met
145                 150             155                 160

Ala Ile Tyr Lys Gln Ser Gln His Met Thr Glu Val Val Arg Arg Cys
            165                 170             175

Pro His His Glu Arg Cys Ser Asp Ser Asp Gly Leu Ala Pro Pro Gln
        180                 185             190

His Leu Ile Arg Val Glu Gly Asn Leu Arg Val Glu Tyr Leu Asp Asp
        195                 200             205

Arg Asn Thr Phe Arg His Ser Val Val Val Pro Tyr Glu Pro Pro Glu
    210                 215             220

Val Gly Ser Asp Cys Thr Thr Ile His Tyr Asn Tyr Met Cys Asn Ser
225                 230             235                 240

Ser Cys Met Gly Gly Met Asn Arg Arg Pro Ile Leu Thr Ile Ile Thr
            245                 250             255

Leu Glu Asp Ser Ser Gly Asn Leu Leu Gly Arg Asn Ser Phe Glu Val
        260                 265             270

Arg Val Cys Ala Cys Pro Gly Arg Asp Arg Arg Thr Glu Glu Glu Asn
        275                 280             285

Leu Arg Lys Lys Gly Glu Pro His His Glu Leu Pro Pro Gly Ser Thr
    290                 295             300

Lys Arg Ala Leu Pro Asn Asn Thr Ser Ser Ser Pro Gln Pro Lys Lys
305                 310             315                 320

Lys Pro Leu Asp Gly Glu Tyr Phe Thr Leu Gln Ile Arg Gly Arg Glu
            325                 330             335

Arg Phe Glu Met Phe Arg Glu Leu Asn Glu Ala Leu Glu Leu Lys Asp
        340                 345             350

Ala Gln Ala Gly Lys Glu Pro Gly Gly Ser Arg Ala His Ser Ser His
        355                 360             365
```

```
Leu Lys Ser Lys Lys Gly Gln Ser Thr Ser Arg His Lys Lys Leu Met
    370             375             380

Phe Lys Thr Glu Gly Pro Asp Ser Asp
    385             390
```

<210> 15
<211> 10
<212> PRT
<213> Homo sapiens

<400> 15

```
Ile Arg Gly Arg Glu Arg Phe Glu Met Phe
1           5                   10
```

<210> 16
<211> 10
<212> PRT
<213> Artificial Sequence

<220>
<223> Description of Artificial Sequence: Synthetic peptide

<400> 16

```
Asp Gly Thr Ser Phe Gln Lys Glu Asn Cys
1           5                   10
```